# EUROPEAN PATENT SPECIFICATION

(11) **EP 0 518 558 B1**
(45) Date of publication and mention of the grant of the patent: **02.09.1998**
(21) Application number: 92305130.4
(22) Date of filing: 04.06.1992
(51) Int. Cl.: C07D 477/00, A61K 31/40

(54) **1-Methylcarbapenem derivatives, their preparation and their use as antibiotics**
1-Methyl-carbapenemderivate, deren Herstellung und Verwendung als Antibiotika
Dérivés de méthyl-1-carbapénème, leur préparation et leur application comme antibiotiques

(30) Priority: 04.06.1991 JP 131545/91; 27.12.1991 JP 345737/91; 18.02.1992 JP 30521/92; 10.04.1992 JP 91283/92
(43) Date of publication of application: 16.12.1992
(73) Proprietor: Sankyo Company Limited, Tokyo (JP)
(72) Inventor: Kawamoto, Isao, c/o Sankyo Company Limited, Tokyo 140 (JP); Endo, Rokuro, c/o Sankyo Company Limited, Tokyo 140 (JP); Miyauchi, Masao, c/o Sankyo Company Limited, Tokyo 140 (JP); Watanabe, Katsuhiko, c/o Sankyo Company Limited, Tokyo 140 (JP); Nakayama, Eiji, c/o Sankyo Company Limited, Tokyo 140 (JP); Yasuda, Hiroshi, c/o Sankyo Company Limited, Tokyo 140 (JP); Ohya, Satoshi, c/o Sankyo Company Limited, Tokyo 140 (JP); Utsui, Yukio, c/o Sankyo Company Limited, Tokyo 140 (JP)
(74) Representative: Gibson, Christian John Robert

(56) References cited:
- EP-A- 126 587
- EP-A- 243 686
- EP-A- 442 497
- EP-A- 443 883
- EP-A- 472 062
- CHEMICAL ABSTRACTS, vol. 117, 1992, Columbus, Ohio, US; abstract no. 69658X, 'Preparation of carbapenem derivatives and their salts as antibiotics.'

## Description

The present invention relates to a series of new 1-methylcarbapenem derivatives which have excellent antibiotic activity and outstanding stability in vivo. The invention also provides methods and compositions using these derivatives for the treatment and prophylaxis of infections, as well as processes for their preparation.

The carbapenem compounds are a well known series of compounds, related to the penicillins, which have been used or have been proposed for use as antibiotics. They have in common a basic structure which may be represented by the formula (A):

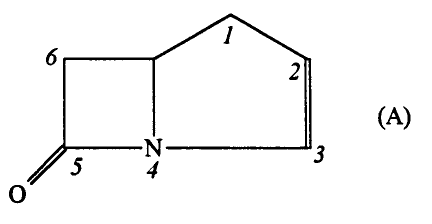

In this formula, we have indicated the numbering of those positions of importance to the carbapenem compounds, using the numbering scheme commonly used in the art and as employed in the nomenclature of the compounds of the present invention. In accordance with the recommendations of the International Union of Pure and Applied Chemistry (IUPAC), Commission on Nomenclature of Organic Chemistry, the compounds referred to herein are named semi-systematically, using the above carbapenem structure as the parent name.

Those carbapenem antibiotics having no substituent at the 1-position are potentially a very useful series of compounds which have extraordinarily potent antibacterial activity. Unfortunately, however, they are chemically unstable and, moreover, are sensitive to dehydropeptidase I in vivo. Dehydropeptidase I is an enzyme which hydrolyses the β-lactam ring in carbapenem antibiotics and which exists in mammalian tissue, for example in the renal cortex. It is responsible for the extensive metabolisation of many otherwise valuable β-lactam antibiotics in animals, including humans, thus greatly reducing their value. Despite these disadvantages, these carbapenem antibiotics are finding increasing use in the treatment of bacterial infections. A typical and common antibiotic of this type is thienamycin, which has the formula (B):

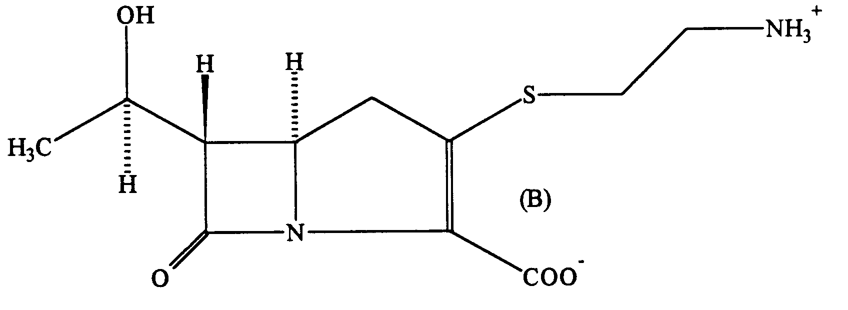

Metabolism of the antibiotic in vivo may be demonstrated by a low recovery of the compound itself (as opposed to its metabolic products) in the urine, and this has been demonstrated for thienamycin [H. Kropp et al., Antimicrob. Agents, Chemother., 22, 62 (1982); and S. R. Norrby et al., ibid., 23, 300 (1983)].

Although it has been found that carbapenem compounds having a substituent at the 1-position (commonly a 1-methyl group) do not have this susceptibility to dehydropeptidase I in vivo, many of the compounds of this type discovered to date lack sufficient activity. It is, therefore, considered highly desirable to find a carbapenem antibiotic which combines the good activity of thienamycin with a resistance to dehydropeptidase I in vivo.

Many carbapenem compounds are now known. Some are described, for example, in European Patent Publications No. 126 587, 182 213, 243 686, 333 175, 442 497 and 443 883. EP 182 213 and EP 333 175 disclose compounds in which a thio-pyrrolidinyl group and its ring carbon atom substituent are linked by an alkylene group, and thus differ from the compounds of the present invention in that there is no linking carbonyl group. EP 442 497 and EP 443 883 disclose carboxylic thio-pyrrolidinyl beta-lactam compounds having a terminal quaternary ammonium group which is taught to provide resistance to dehydropeptidase I. The compounds disclosed in EP 126 587 and EP 243 686, on the other hand, are carboxylic thio-pyrrolidinyl beta-lactam compounds, and are thus thought to represent the closest prior art to the compounds of the present invention. However, the present compounds have demonstrated significantly better activity than the prior art compounds.

Thus, in a first aspect, the present invention provides compounds of formula (I): 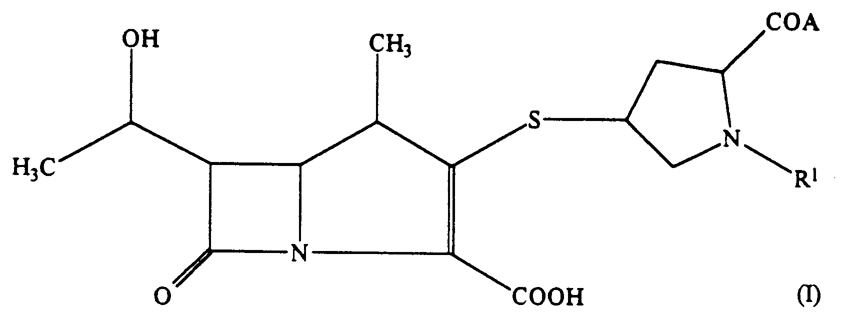 wherein:
R¹ represents:
   a hydrogen atom,
   an unsubstituted alkyl group having from 1 to 6 carbon atoms,
   a substituted alkyl group which has from 1 to 6 carbon atoms and which is substituted by at least one substituent selected from substituents (a), defined below,
   an alkenyl group having from 2 to 6 carbon atoms,
   an alkynyl group having from 2 to 6 carbon atoms, or
   a group of formula -C(=NH)R°, where R° represents a hydrogen atom or an alkyl group having from 1 to 6 carbon atoms; and
A represents a group of formula (A1), (A2), (A3), (A4), (A5), (A6), (A7) or (A8): 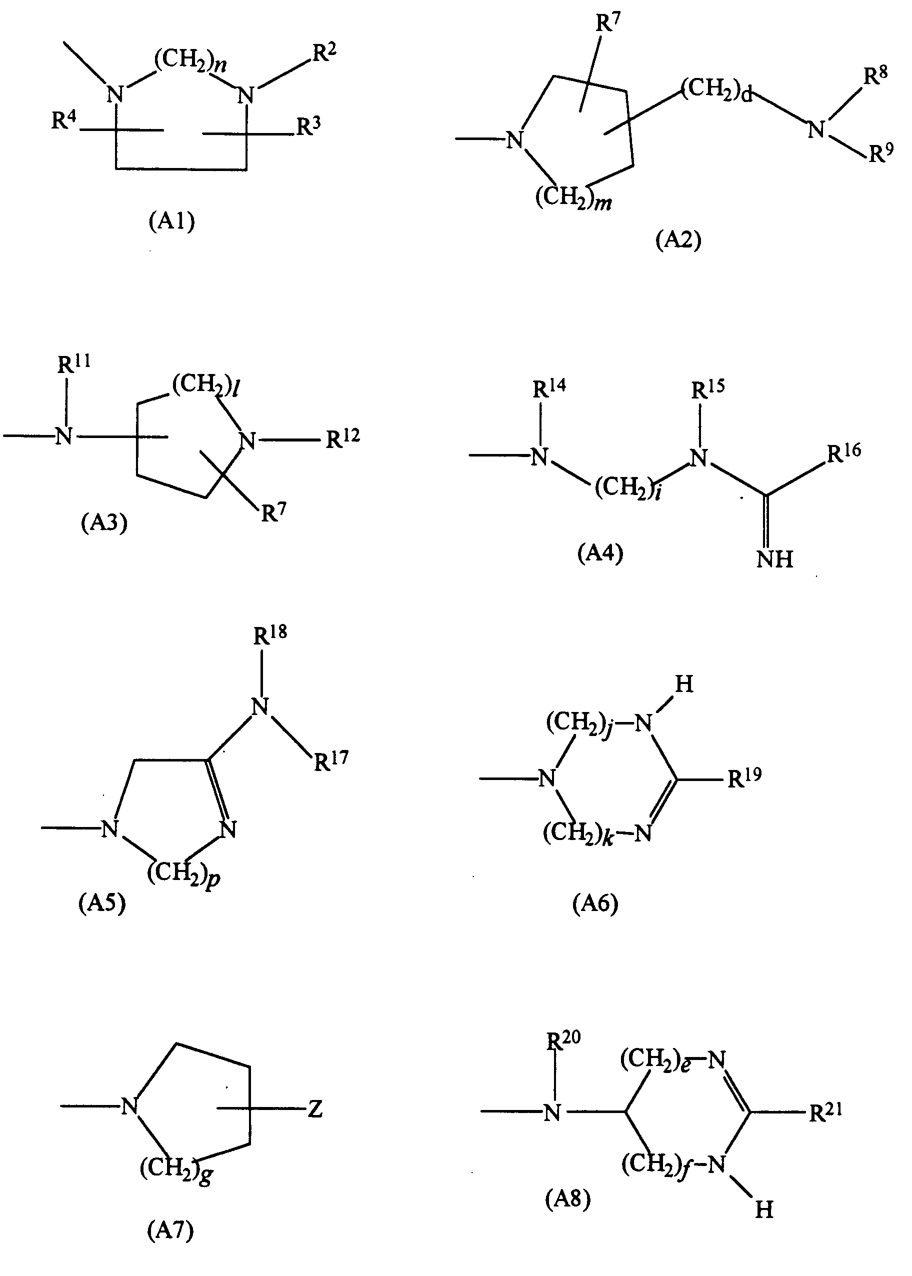 wherein:
R² represents:
   a hydrogen atom,
   an unsubstituted alkyl group having from 1 to 6 carbon atoms,
   a substituted alkyl group which has from 1 to 6 carbon atoms and which is substituted by at least one substituent selected from substituents (b), defined below,
   an alkenyl group having from 2 to 6 carbon atoms,
   an alkynyl group having from 2 to 6 carbon atoms, or
   a group of formula -C(=NH)R⁶,
      where R⁶ represents a hydrogen atom, an unsubstituted alkyl group having from 1 to 6 carbon atoms, a substituted alkyl group which has from 1 to 6 carbon atoms and which is substituted by at least one substituent selected from substituents (c), defined below, or a cycloalkyl group having from 3 to 7 ring carbon atoms;
R³, R⁴ and R⁷ are independently selected from:
   hydrogen atoms,
   unsubstituted alkyl groups having from 1 to 6 carbon atoms,
   substituted alkyl groups which have from 1 to 6 carbon atoms and which are substituted by at least one substituent selected from substituents (d), defined below,
   halogen atoms,
   hydroxy groups,
   carboxy groups,
   groups of formula -CO.NR^{a}R^{b}, -OCO.NR^{a}R^{b} and -NR^{a}R^{b},
      wherein R^{a} and R^{b} are independently selected from hydrogen atoms and alkyl groups having from 1 to 4 carbon atoms, and cyano groups;
R⁸ represents:
   a hydrogen atom,
   an unsubstituted alkyl group having from 1 to 6 carbon atoms,
   a substituted alkyl group which has from 1 to 6 carbon atoms and which is substituted by at least one substituent selected from substituents (a), defined below,
   an alkenyl group having from 2 to 6 carbon atoms, or
   an alkynyl group having from 2 to 6 carbon atoms;
R⁹ represents:
   a hydrogen atom,
   an unsubstituted alkyl group having from 1 to 6 carbon atoms,
   a substituted alkyl group which has from 1 to 6 carbon atoms and which is substituted by at least one substituent selected from substituents (a), defined below, or
   a group of formula -C(=NH)R¹⁰,
      where R¹⁰ represents a hydrogen atom, an unsubstituted alkyl group having from 1 to 6 carbon atoms, a substituted alkyl group which has from 1 to 6 carbon atoms and which is substituted by at least one substituent selected from substituents (c), defined below, or a cycloalkyl group having from 3 to 7 ring carbon atoms;
   or
R⁸ and R⁹ together represent a group of formula -(CH₂)ₛ-W-(CH₂)ₜ-
   wherein W represents a carbon-carbon single bond, an oxygen atom, a sulphur atom or a group of formula >NR²², wherein R²² represents a hydrogen atom or an alkyl group having from 1 to 6 carbon atoms, and
   s and t are independently 1, 2 or 3;
R¹¹ represents a hydrogen atom or an alkyl group having from 1 to 6 carbon atoms;
R¹² represents:
   a hydrogen atom,
   an unsubstituted alkyl group having from 1 to 6 carbon atoms,
   a substituted alkyl group which has from 1 to 6 carbon atoms and which is substituted by at least one substituent selected from substituents (a), defined below,
   an alkenyl group having from 2 to 6 carbon atoms,
   an alkynyl group having from 2 to 6 carbon atoms, or
   a group of formula -C(=NH)R¹³,
      where R¹³ represents a hydrogen atom, an unsubstituted alkyl group having from 1 to 6 carbon atoms, a substituted alkyl group which has from 1 to 6 carbon atoms and which is substituted by at least one substituent selected from substituents (c), defined below, or a cycloalkyl group having from 3 to 7 ring carbon atoms;
R¹⁴ and R¹⁵ are independently selected from hydrogen atoms and alkyl groups having from 1 to 6 carbon atoms;
R¹⁶ represents a hydrogen atom, an unsubstituted alkyl group having from 1 to 6 carbon atoms, a substituted alkyl group which has from 1 to 6 carbon atoms and which is substituted by at least one substituent selected from substituents (c), defined below, or a cycloalkyl group having from 3 to 7 ring carbon atoms;
R¹⁷ and R¹⁸ are independently selected from:
   hydrogen atoms,
   unsubstituted alkyl groups having from 1 to 6 carbon atoms, and
   substituted alkyl groups which have from 1 to 6 carbon atoms and which are substituted by at least one substituent selected from substituents (a), defined below;
   or
R¹⁷ and R¹⁸ together represent a group of formula -(CH₂)_{q}-Y-(CH₂)ᵣ-
   wherein Y represents a carbon-carbon single bond, an oxygen atom, a sulphur atom or a group of formula >NR²³, wherein R²³ represents a hydrogen atom or an alkyl group having from 1 to 6 carbon atoms, and
   q and r are independently 1, 2 or 3;
R¹⁹, R²⁰ and R²¹ are independently selected from hydrogen atoms and alkyl groups having from 1 to 6 carbon atoms;
Z represents an imidazolyl, triazolyl or tetrazolyl group;
d is 0 or 1;
e, f, i, j and k are independently 1 or 2;
q, ℓ and m are independently 0, 1 or 2; and
n and p are independently 1, 2 or 3;
   PROVIDED THAT, where A represents a group of formula (A1):
   R², R³ and R⁴ do not all represent hydrogen atoms when R¹ represents a hydrogen atom; and
   R¹, R³ and R⁴ do not all represent hydrogen atoms when R² represents an alkyl group;
said substituents (a) are selected from hydroxy groups, carboxy groups, cyano groups, halogen atoms, oxygen atoms (to form an oxo group), alkoxy groups having from 1 to 6 carbon atoms, and groups of formula -CO.NR^{a}R^{b}, -OCO.NR^{a}R^{b} and -NR^{a}R^{b}, wherein R^{a} and R^{b} are as defined above;
said substituents (b) are selected from:
   hydroxy groups,
   carboxy groups,
   cyano groups,
   halogen atoms,
   alkoxy groups having from 1 to 6 carbon atoms, groups of formula -CO.NR^{a}R^{b}, -OCO.NR^{a}R^{b} and -NR^{a}R^{b}, wherein R^{a} and R^{b} are as defined above,
   sulphamoyl groups,
   ureido groups,
   sulpho groups,
   alkanoyl groups having from 1 to 6 carbon atoms,
   alkanoylamino groups having from 1 to 6 carbon atoms,
   alkanoyloxy groups having from 1 to 6 carbon atoms,
   alkylthio groups having from 1 to 6 carbon atoms,
   alkylsulphinyl groups having from 1 to 6 carbon atoms, and
   alkylsulphonyl groups having from 1 to 6 carbon atoms;
said substituents (c) are selected from:
   halogen atoms,
   alkoxy groups having from 1 to 6 carbon atoms,
   cycloalkyl groups having from 3 to 7 ring carbon atoms; and
said substituents (d) are selected from:
   hydroxy groups,
   cyano groups,
   groups of formula -CO.NR^{a}R^{b}, -OCO.NR^{a}R^{b} and -NR^{a}R^{b}, wherein R^{a} and R^{b} are as defined above,
   carboxy groups,
   halogen atoms, and
   alkoxy groups having from 1 to 6 carbon atoms;
and pharmaceutically acceptable salts and esters thereof.

The present invention also provides a pharmaceutical composition comprising a pharmaceutically acceptable carrier, diluent or adjuvant in admixture with an effective amount of an antibiotic, wherein the antibiotic is selected from compounds of formula (I) and pharmaceutically acceptable salts and esters thereof.

The present invention further provides the use of a compound of formula (I) for the manufacture of a medicament for the treatment or prophylaxis of bacterial infections.

The present invention also provides processes for preparing these compounds, which are described in greater detail hereafter.

It is, therefore, an advantage of the present invention that it provides a series of new 1-methylcarbapenem compounds having antibiotic activity.

It is a further advantage of the present invention that such compounds have a good resistance to dehydropeptidase I in vivo.

Other objects and advantages will become apparent as the description proceeds.

In the compounds of the present invention, where R¹, R², R³, R⁴, R⁶, R⁷, R⁸, R⁹, R¹⁰, R¹¹, R¹², R¹³, R¹⁴, R¹⁵, R¹⁶, R¹⁷, R¹⁸, R¹⁹, R²⁰, R²¹, R²² or R²³ represents an alkyl group having from 1 to 6 carbon atoms, this may be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4 carbon atoms, preferably the methyl, ethyl, propyl, isopropyl, butyl, isobutyl and t-butyl groups, and most preferably the methyl group.

Where R° represents an alkyl group having from 1 to 6 carbon atoms, this may likewise be a straight or branched chain group having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, 2-methylbutyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, hexyl and isohexyl groups. Of these, we prefer those alkyl groups having from 1 to 4, more preferably from 1 to 3, carbon atoms, preferably the methyl, ethyl, propyl, isopropyl, butyl, isobutyl and t-butyl groups, more preferably the methyl, ethyl and propyl groups, and most preferably the methyl group.

Where R¹, R², R³, R⁴, R⁶, R⁷, R⁸, R⁹, R¹⁰, R¹², R¹³, R¹⁶, R¹⁷ or R¹⁸, represents a substituted alkyl group having from 1 to 6 carbon atoms, this may be a straight or branched chain group, preferably a straight chain group, having from 1 to 6, preferably from 1 to 4, carbon atoms, and examples include the methyl, ethyl, propyl, isopropyl, butyl, isobutyl, sec-butyl, t-butyl, pentyl, isopentyl, neopentyl, hexyl and isohexyl groups. Of these, we prefer those alkyl groups having from 1 to 3 carbon atoms, preferably the methyl, ethyl and propyl groups, and most preferably the methyl and ethyl groups. The substituents may be selected from the appropriate one of substituents (a), (b), (c) and (d), as defined above and exemplified below. There is no particular limitation on the number of substituents, except such as may be imposed by the number of substitutable positions, and possibly by steric constraints. However, in general, from 1 to 3 substituents are preferred, a single substituent being normally most preferred.

Where R¹, R², R⁸ or R¹² represents an alkenyl group having from 2 to 6 carbon atoms, this may be a straight or branched chain group having from 2 to 6, preferably 3 or 4, carbon atoms, and examples include the vinyl, allyl, 2-methylallyl, 1-propenyl, isopropenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl and 5-hexenyl groups, of which the vinyl, allyl, 2-methylallyl, 1-propenyl, isopropenyl and butenyl groups are preferred, the allyl and 2-methylallyl groups being most preferred.

Where R¹, R², R⁸ or R¹² represents an alkynyl group having from 2 to 6 carbon atoms, this may be a straight or branched chain group having from 2 to 6, preferably 3 or 4, carbon atoms, and examples include the ethynyl, propargyl (2-propynyl), 1-propynyl, 1-butynyl, 2-butynyl, 3-butynyl, 2-methyl-2-propynyl, 1-pentynyl, 2-pentynyl, 3-pentynyl and 4-pentynyl groups, of which the propynyl and butynyl groups are preferred, the propargyl and 2-methyl-2-propynyl groups being most preferred.

Where R⁶, R¹⁰, R¹³, R¹⁶ or substituent (c) represents a cycloalkyl group, this may have from 3 to 7 ring carbon atoms, and examples include the cyclopropyl, cyclobutyl, cyclopentyl, cyclohexyl and cycloheptyl groups, of which the cyclopropyl, cyclobutyl and cyclopentyl groups are preferred, the cyclopropyl group being most preferred.

Where R³, R⁴, R⁷, substituent (a), substituent (b), substituent (c) or substituent (d) represents a halogen atom, this may be a fluorine, chlorine, bromine or iodine atom, more preferably a fluorine, chlorine or bromine atom, and most preferably a fluorine or chlorine atom.

Where R^{a} or R^{b} represents an alkyl group, this may be a straight or branched chain alkyl group having from 1 to 4 carbon atoms, and examples include the methyl, ethyl, propyl, isopropyl, butyl, isobutyl, sec-butyl and t-butyl groups. Of these, we prefer those alkyl groups having 1 or 2 carbon atoms, most preferably the methyl group. However R^{a} and R^{b} preferably both represent hydrogen atoms. Preferred groups of formula -CO.NR^{a}R^{b}, -OCO.NR^{a}R^{b} and -NR^{a}R^{b} are the amino, methylamino, ethylamino, propylamino, butylamino, dimethylamino, diethylamino, methylethylamino, methylbutylamino, carbamoyl, methylcarbamoyl, ethylcarbamoyl, propylcarbamoyl, butylcarbamoyl, dimethylcarbamoyl, diethylcarbamoyl, methylethylcarbamoyl, methylbutylcarbamoyl, carbamoyloxy, methylcarbamoyloxy, ethylcarbamoyloxy, propylcarbamoyloxy, butylcarbamoyloxy, dimethylcarbamoyloxy, diethylcarbamoyloxy, methylethylcarbamoyloxy and methylbutylcarbamoyloxy groups, of which the amino, carbamoyl and carbamoyloxy groups are preferred.

Where R⁸ and R⁹ together represent a group of formula -(CH₂)ₛ-W-(CH₂)ₜ-, W represents a carbon-carbon single bond, an oxygen atom, a sulphur atom or a group of formula >NR²², wherein R²² represents a hydrogen atom or an alkyl group having from 1 to 6 carbon atoms, and s and t are independently 1, 2 or 3. In this case, R⁸ and R⁹ together with the nitrogen atom to which they are attached form a nitrogen-containing heterocyclic group. Preferably, where W represents a carbon-carbon single bond, (s + t) is an integer from 3 to 6, more preferably from 3 to 5 and most preferably 4 or 5. Where W represents an oxygen atom, a sulphur atom or a group of formula >NR²², (s + t) is preferably an integer from 2 to 5, more preferably from 2 to 4 and most preferably 3 or 4. Within these preferred constraints, s and t are preferably each 1 or 2. Examples of such groups of formula -(CH₂)ₛ-W-(CH₂)ₜ- include:
-(CH₂)₄-;
-(CH₂)₅-;
-(CH₂)-O-(CH₂)₂-;
-(CH₂)₂-O-(CH₂)₂-;
-(CH₂)-S-(CH₂)₂-;
-(CH₂)₂-S-(CH₂)₂-;
-(CH₂)-NH-(CH₂)₂-;
-(CH₂)₂-NH-(CH₂)₂-;
-(CH₂)-NMe-(CH₂)₂-; and
-(CH₂)₂-NMe-(CH₂)₂-;
where Me represents a methyl group.

Where R¹⁷ and R¹⁸ together represent a group of formula -(CH₂)_{q}-Y-(CH₂)ᵣ-, Y represents a carbon-carbon single bond, an oxygen atom, a sulphur atom or a group of formula >NR²³, wherein R²³ represents a hydrogen atom or an alkyl group having from 1 to 6 carbon atoms, and q and r are independently 1, 2 or 3. In this case, R¹⁷ and R¹⁸ together with the nitrogen atom to which they are attached form a nitrogen-containing heterocyclic group. Preferably, where Y represents a carbon-carbon single bond, (q + r) is an integer from 3 to 6, more preferably from 3 to 5 and most preferably 4 or 5. Where Y represents an oxygen atom, a sulphur atom or a group of formula >NR²³, (q + r) is preferably an integer from 2 to 5, more preferably from 2 to 4 and most preferably 3 or 4. Within these preferred constraints, q and r are preferably each 1 or 2. Examples of such groups of formula -(CH₂)_{q}-Y-(CH₂)ᵣ- include:
-(CH₂)₄-;
-(CH₂)₅-;
-(CH₂)-O-(CH₂)₂-;
-(CH₂)₂-O-(CH₂)₂-;
-(CH₂)-S-(CH₂)₂-;
-(CH₂)₂-S-(CH₂)₂-;
-(CH₂)-NH-(CH₂)₂-;
-(CH₂)₂-NH-(CH₂)₂-;
-(CH₂)-NMe-(CH₂)₂-; and
-(CH₂)₂-NMe-(CH₂)₂-;
where Me represents a methyl group.

Where substituent (a), substituent (b), substituent (c) or substituent (d) represents an alkoxy group having from 1 to 6 carbon atoms, this may likewise be a straight or branched chain group having from 1 to 6, preferably from 1 to 4, carbon atoms, and examples include the methoxy, ethoxy, propoxy, isopropoxy, butoxy, isobutoxy, sec-butoxy, t-butoxy, pentyloxy, isopentyloxy, neopentyloxy, 2-methylbutoxy, 1-ethylpropoxy, 4-methylpentyloxy, 3-methylpentyloxy, 2-methylpentyloxy, 1-methylpentyloxy, 3,3-dimethylbutoxy, 2,2-dimethylbutoxy, 1,1-dimethylbutoxy, 1,2-dimethylbutoxy, 1,3-dimethylbutoxy, 2,3-dimethylbutoxy, 2-ethylbutoxy, hexyloxy and isohexyloxy groups. Of these, we prefer those alkoxy groups having from 1 to 3 carbon atoms, preferably the methoxy, ethoxy, propoxy, isopropoxy, butoxy, isobutoxy and t-butoxy groups, more preferably the methoxy, ethoxy and propoxy groups, and most preferably the methoxy group.

Where substituent (b) represents an alkanoyl group, this has from 1 to 6 carbon atoms, preferably from 1 to 4 carbon atoms, and examples include the formyl, acetyl, propionyl, butyryl, isobutyryl, pivaloyl, valeryl, isovaleryl and hexanoyl groups, of which the acetyl and propionyl groups are more preferred, the acetyl group being most preferred.

Where substituent (b) represents an alkanoylamino group, this has from 1 to 6 carbon atoms, preferably from 1 to 4 carbon atoms, and examples include the formylamino, acetylamino, propionylamino, butyrylamino, isobutyrylamino, pivaloylamino, valerylamino, isovalerylamino and hexanoylamino groups, of which the acetylamino and propionylamino groups are more preferred, the acetylamino group being most preferred.

Where substituent (b) represents an alkanoyloxy group, this has from 1 to 6 carbon atoms, preferably from 1 to 4 carbon atoms, and examples include the formyloxy, acetyloxy, propionyloxy, butyryloxy, isobutyryloxy, pivaloyloxy, valeryloxy, isovaleryloxy and hexanoyloxy groups, of which the acetyloxy and propionyloxy groups are more preferred, the acetyloxy group being most preferred.

Where substituent (b) represents an alkylthio group having from 1 to 6 carbon atoms, this may likewise be a straight or branched chain group having from 1 to 6, preferably from 1 to 4, carbon atoms, and examples include the methylthio, ethylthio, propylthio, isopropylthio, butylthio, isobutylthio, sec-butylthio, t-butylthio, pentylthio, isopentylthio, neopentylthio, 2-methylbutylthio, 1-ethylpropylthio, 4-methylpentylthio, 3-methylpentylthio, 2-methylpentylthio, 1-methylpentylthio, 3,3-dimethylbutylthio, 2,2-dimethylbutylthio, 1,1-dimethylbutylthio, 1,2-dimethylbutylthio, 1,3-dimethylbutylthio, 2,3-dimethylbutylthio, 2-ethylbutylthio, hexylthio and isohexylthio groups. Of these, we prefer those alkylthio groups having from 1 to 4, more preferably from 1 to 3, carbon atoms, preferably the methylthio, ethylthio, propylthio, isopropylthio, butylthio, isobutylthio and t-butylthio groups, more preferably the methylthio, ethylthio and propylthio groups, and most preferably the methylthio group.

Where substituent (b) represents an alkylsulphinyl group having from 1 to 6 carbon atoms, this may likewise be a straight or branched chain group having from 1 to 6, preferably from 1 to 4, carbon atoms, and examples include the methylsulphinyl, ethylsulphinyl, propylsulphinyl, isopropylsulphinyl, butylsulphinyl, isobutylsulphinyl, sec-butylsulphinyl, t-butylsulphinyl, pentylsulphinyl, isopentylsulphinyl, neopentylsulphinyl, 2-methylbutylsulphinyl, 1-ethylpropylsulphinyl, 4-methylpentylsulphinyl, 3-methylpentylsulphinyl, 2-methylpentylsulphinyl, 1-methylpentylsulphinyl, 3,3-dimethylbutylsulphinyl, 2,2-dimethylbutylsulphinyl, 1,1-dimethylbutylsulphinyl, 1,2-dimethylbutylsulphinyl, 1,3-dimethylbutylsulphinyl, 2,3-dimethylbutylsulphinyl, 2-ethylbutylsulphinyl, hexylsulphinyl and isohexylsulphinyl groups. Of these, we prefer those alkylsulphinyl groups having from 1 to 4, more preferably from 1 to 3, carbon atoms, preferably the methylsulphinyl, ethylsulphinyl, propylsulphinyl, isopropylsulphinyl, butylsulphinyl, isobutylsulphinyl and t-butylsulphinyl groups, more preferably the methylsulphinyl, ethylsulphinyl and propylsulphinyl groups, and most preferably the methylsulphinyl group.

Where substituent (b) represents an alkylsulphonyl group having from 1 to 6 carbon atoms, this may likewise be a straight or branched chain group having from 1 to 6, preferably from 1 to 4, carbon atoms, and examples include the methylsulphonyl, ethylsulphonyl, propylsulphonyl, isopropylsulphonyl, butylsulphonyl, isobutylsulphonyl, sec-butylsulphonyl, t-butylsulphonyl, pentylsulphonyl, isopentylsulphonyl, neopentylsulphonyl, 2-methylbutylsulphonyl, 1-ethylpropylsulphonyl, 4-methylpentylsulphonyl, 3-methylpentylsulphonyl, 2-methylpentylsulphonyl, 1-methylpentylsulphonyl, 3,3-dimethylbutylsulphonyl, 2,2-dimethylbutylsulphonyl, 1,1-dimethylbutylsulphonyl, 1,2-dimethylbutylsulphonyl, 1,3-dimethylbutylsulphonyl, 2,3-dimethylbutylsulphonyl, 2-ethylbutylsulphonyl, hexylsulphonyl and isohexylsulphonyl groups. Of these, we prefer those alkylsulphonyl groups having from 1 to 4, more preferably from 1 to 3, carbon atoms, preferably the methylsulphonyl, ethylsulphonyl, propylsulphonyl, isopropylsulphonyl, butylsulphonyl, isobutylsulphonyl and t-butylsulphonyl groups, more preferably the methylsulphonyl, ethylsulphonyl and propylsulphonyl groups, and most preferably the methylsulphonyl group.

The compounds of formula (I) have a carboxy group at the carbapenem 3-position, and the compounds may also contain one or more additional carboxy groups depending upon the meanings of R³, R⁴, R⁷, substituent (a), substituent (b) and substituent (d). Such carboxy groups can, of course form salts and esters, and such salts and esters also form part of the present invention. There is no particular restriction on the nature of these salts and esters, provided that, where they are intended for therapeutic use, they are pharmaceutically acceptable. Where they are intended for non-therapeutic uses, e.g. as intermediates in the preparation of other, and possibly more active, compounds, even this restriction does not apply. In the case of the esters, we prefer, in general, an ester residue which is capable of hydrolysis in the mammalian body, as is well known in the art. However, any ester residue can be used, provided that, as explained above, if the compound is intended for therapeutic use, it is pharmaceutically acceptable. Examples of suitable ester groups include:
C₁ - C₂₀ alkyl groups, more preferably C₁ - C₆ alkyl groups, such as those exemplified in relation to R¹ etc. and higher alkyl groups as are well known in the art, such as the heptyl, octyl, nonyl, decyl, dodecyl, tridecyl, pentadecyl, octadecyl, nonadecyl and icosyl groups, but most preferably the methyl, ethyl and t-butyl groups;
C₃ - C₇ cycloalkyl groups, for example as illustrated herein in relation to R⁶ etc.;
aralkyl groups, in which the alkyl part is a C₁ - C₃ alkyl group and the aryl part is a C₆ - C₁₄ carbocyclic aromatic group which may be substituted or unsubstituted and, if substituted, has at least one substituent selected from substituents (e) defined and exemplified below, although the unsubstituted groups are preferred; examples of such aralkyl groups include the benzyl, phenethyl, 1-phenylethyl, 3-phenylpropyl, 2-phenylpropyl, 1-naphthylmethyl, 2-naphthylmethyl, 2-(1-naphthyl)ethyl, 2-(2-naphthyl)ethyl, benzhydryl (i.e. diphenylmethyl), triphenylmethyl, bis(o-nitrophenyl)methyl, 9-anthrylmethyl, 2,4,6-trimethylbenzyl, 4-bromobenzyl, 2-nitrobenzyl, 4-nitrobenzyl, 3-nitrobenzyl, 4-methoxybenzyl and piperonyl groups;
alkenyl groups such as those defined and exemplified above in relation to R¹ etc., but which may be substituted or unsubstituted and, if substituted have at least one substituent selected from substituents (a) defined above; examples of the unsubstituted groups are given above in relation to R¹ etc., and preferred groups include the allyl, 2-chloroallyl and 2-methylallyl groups;
halogenated C₁ - C₆, preferably C₁ - C₄, alkyl groups in which the alkyl part is as defined and exemplified in relation to the alkyl groups which may be represented by R¹ etc., and the halogen atom is chlorine, fluorine, bromine or iodine, such as the 2,2,2-trichloroethyl, 2-haloethyl (e.g. 2-chloroethyl, 2-fluoroethyl, 2-bromoethyl or 2-iodoethyl), 2,2-dibromoethyl and 2,2,2-tribromoethyl group;
substituted silylalkyl groups, in which the alkyl part is as defined and exemplified in relation to the alkyl groups which may be represented by R¹ etc., and the silyl group has up to 3 substituents selected from C₁ - C₆ alkyl groups and phenyl groups which are unsubstituted or have at least one substituent selected from substituents (e) defined and exemplified below, for example a 2-trimethylsilylethyl group;
phenyl groups, in which the phenyl group is unsubstituted or substituted, preferably with at least one C₁-C₄ alkyl or acylamino group, for example the phenyl, tolyl and benzamidophenyl groups;
phenacyl groups, which may be unsubstituted or have at least one substituent selected from substituents (e) defined and exemplified below, for example the phenacyl group itself or the p-bromophenacyl group;
cyclic and acyclic terpenyl groups, for example the geranyl, neryl, linalyl, phytyl, menthyl (especially m- and p- menthyl), thujyl, caryl, pinanyl, bornyl, notcaryl, norpinanyl, norbornyl, menthenyl, camphenyl and norbornenyl groups;
alkoxymethyl groups, in which the alkoxy part is C₁ - C₆, preferably C₁ - C₄, and may itself be substituted by a single unsubstituted alkoxy group, such as the methoxymethyl, ethoxymethyl, propoxymethyl, isopropoxymethyl, butoxymethyl and methoxyethoxymethyl groups;
aliphatic acyloxyalkyl groups, in which the acyl group is preferably an alkanoyl group and is more preferably a C₂ - C₆ alkanoyl group, and the alkyl part is a C₂ - C₆, and preferably C₂ - C₄, alkyl group, such as the acetoxymethyl, propionyloxymethyl, butyryloxymethyl, isobutyryloxymethyl, pivaloyloxymethyl, 1-pivaloyloxyethyl, 1-acetoxyethyl, 1-isobutyryloxyethyl, 1-pivaloyloxypropyl, 2-methyl-1-pivaloyloxypropyl, 2-pivaloyloxypropyl, 1-isobutyryloxyethyl, 1-isobutyryloxypropyl, 1-acetoxypropyl, 1-acetoxy-2-methylpropyl, 1-propionyloxyethyl, 1-propionyloxypropyl, 2-acetoxypropyl and 1-butyryloxyethyl groups;
cycloalkyl-substituted aliphatic acyloxyalkyl groups, in which the acyl group is preferably an alkanoyl group and is more preferably a C₂ - C₆ alkanoyl group, the cycloalkyl substituent is C₃ - C₇, and the alkyl part is a C₁ - C₆ alkyl group, preferably a C₁ - C₄ alkyl group, such as the (cyclohexylacetoxy)methyl, 1-(cyclohexylacetoxy)ethyl, 1-(cyclohexylacetoxy)propyl, 2-methyl-1-(cyclohexylacetoxy)propyl, (cyclopentylacetoxy)methyl, 1-(cyclopentylacetoxy)ethyl, 1-(cyclopentylacetoxy)propyl and 2-methyl-1-(cyclopentylacetoxy)propyl, groups;
alkoxycarbonyloxyalkyl groups, especially 1-(alkoxycarbonyloxy)ethyl groups, in which the alkoxy part is C₁ - C₁₀, preferably C₁ - C₆, and more preferably C₁ - C₄, and the alkyl part is C₁ - C₆, preferably C₁ - C₄, such as the 1-methoxycarbonyloxyethyl, 1-ethoxycarbonyloxyethyl, 1-propoxycarbonyloxyethyl, 1-isopropoxycarbonyloxyethyl, 1-butoxycarbonyloxyethyl, 1-isobutoxycarbonyloxyethyl, 1-sec-butoxycarbonyloxyethyl, 1-t-butoxycarbonyloxyethyl, 1-(1-ethylpropoxycarbonyloxy)ethyl and 1-(1,1-dipropylbutoxycarbonyloxy)ethyl groups, and other alkoxycarbonylalkyl groups, in which both the alkoxy and alkyl groups are C₁ - C₆, preferably C₁ - C₄, such as the 2-methyl-1-(isopropoxycarbonyloxy)propyl, 2-(isopropoxycarbonyloxy)propyl, isopropoxycarbonyloxymethyl, t-butoxycarbonyloxymethyl, methoxycarbonyloxymethyl and ethoxycarbonyloxymethyl groups;
cycloalkylcarbonyloxyalkyl and cycloalkyloxycarbonyloxyalkyl groups, in which the cycloalkyl group is C₃ - C₁₀, preferably C₃ - C₇, is mono- or poly- cyclic and is optionally substituted by at least one (and preferably only one) C₁ - C₄ alkyl group (e.g. selected from those alkyl groups exemplified above) and the alkyl group is a C₁ - C₆, more preferably C₁ - C₄, alkyl group (e.g. selected from those alkyl groups exemplified above) and is most preferably methyl, ethyl or propyl, for example the 1-methylcyclohexylcarbonyloxymethyl, 1-methylcyclohexyloxycarbonyloxymethyl, cyclopentyloxycarbonyloxymethyl, cyclopentylcarbonyloxymethyl, 1-cyclohexyloxycarbonyloxyethyl, 1-cyclohexylcarbonyloxyethyl, 1-cyclopentyloxycarbonyloxyethyl, 1-cyclopentylcarbonyloxyethyl, 1-cycloheptyloxycarbonyloxyethyl, 1-cycloheptylcarbonyloxyethyl, 1-methylcyclopentylcarbonyloxymethyl, 1-methylcyclopentyloxycarbonyloxymethyl, 2-methyl-1-(1-methylcyclohexylcarbonyloxy)propyl, 1-(1-methylcyclohexylcarbonyloxy)propyl, 2-(1-methylcyclohexylcarbonyloxy)propyl, 1-(cyclohexylcarbonyloxy)propyl, 2-(cyclohexylcarbonyloxy)propyl, 2-methyl-1-(1-methylcyclopentylcarbonyloxy)propyl, 1-(1-methylcyclopentylcarbonyloxy)propyl, 2-(1-methylcyclopentylcarbonyloxy)propyl, 1-(cyclopentylcarbonyloxy)propyl, 2-(cyclopentylcarbonyloxy)propyl, 1-(1-methylcyclopentylcarbonyloxy)ethyl, 1-(1-methylcyclopentylcarbonyloxy)propyl, adamantyloxycarbonyloxymethyl, adamantylcarbonyloxymethyl, 1-adamantyloxycarbonyloxyethyl and 1-adamantylcarbonyloxyethyl groups;
cycloalkylalkoxycarbonyloxyalkyl groups in which the alkoxy group has a single cycloalkyl substituent, the cycloalkyl substituent being C₃ - C₁₀, preferably C₃ - C₇, and mono- or poly- cyclic, for example the cyclopropylmethoxycarbonyloxymethyl, cyclobutylmethoxycarbonyloxymethyl, cyclopentylmethoxycarbonyloxymethyl, cyclohexylmethoxycarbonyloxymethyl, 1-(cyclopropylmethoxycarbonyloxy)ethyl, 1-(cyclobutylmethoxycarbonyloxy)ethyl, 1-(cyclopentylmethoxycarbonyloxy)ethyl and 1-(cyclohexylmethoxycarbonyloxy)ethyl groups;
terpenylcarbonyloxyalkyl and terpenyloxycarbonyloxyalkyl groups, in which the terpenyl group is as exemplified above, and is preferably a cyclic terpenyl group, for example the 1-(menthyloxycarbonyloxy)ethyl, 1-(menthylcarbonyloxy)ethyl, menthyloxycarbonyloxymethyl, menthylcarbonyloxymethyl, 1-(3-pinanyloxycarbonyloxy)ethyl, 1-(3-pinanylcarbonyloxy)ethyl, 3-pinanyloxycarbonyloxymethyl and 3-pinanylcarbonyloxymethyl groups;
5-alkyl or 5-phenyl [which may be substituted by at least one substituent selected from substituents (e)] (2-oxo-1,3-dioxolen-4-yl)alkyl groups in which each alkyl group (which may be the same or different) is C₁ - C₆, preferably C₁ - C₄, for example the (5-methyl-2-oxo-1,3-dioxolen-4-yl)methyl, (5-phenyl-2-oxo-1,3-dioxolen-4-yl)methyl, (5-isopropyl-2-oxo-1,3-dioxolen-4-yl)methyl, (5-t-butyl-2-oxo-1,3-dioxolen-4-yl)methyl and 1-(5-methyl-2-oxo-1,3-dioxolen-4-yl)ethyl groups; and
other groups, especially groups which are easily removed in vivo such as the phthalidyl, indanyl and 2-oxo-4,5,6,7-tetrahydro-1,3-benzodioxolen-4-yl groups.

Of the above groups, we especially prefer those groups which can be removed easily in vivo, and most preferably the aliphatic acyloxyalkyl groups, alkoxycarbonyloxyalkyl groups, cycloalkylcarbonyloxyalkyl groups, phthalidyl groups and (5-substituted 2-oxo-1,3-dioxolen-4-yl)methyl groups.

In the case of the carboxy groups represented by R³, R⁴, R⁷, substituent (a), substituent (b) and substituent (d), preferred ester groups are the alkyl groups, i.e. the carboxy group is replaced by an alkoxycarbonyl group, such as a methoxycarbonyl, ethoxycarbonyl or propoxycarbonyl group.

The substituents (e), referred to above include:
C₁ - C₄ alkyl groups, such as those exemplified above in relation to R^{a} and R^{b};
C₁ - C₄ alkoxy groups, such as the methoxy, ethoxy, propoxy, isopropoxy, butoxy, isobutoxy, sec-butoxy and t-butoxy groups;
C₁ - C₄ haloalkyl groups, in which the alkyl part is as exemplified above in relation to R^{a} and R^{b} and the halogen atom is as exemplified above in relation to R³ etc., such as the chloromethyl, fluoromethyl, bromomethyl, iodomethyl, 2-chloroethyl, 2-fluoroethyl, 2-bromoethyl, 2-iodoethyl, trifluoromethyl, 2,2,2-trifluoroethyl, 2,2,2-trichloroethyl, 3-fluoropropyl and 4-chlorobutyl groups;
C₁ -C₃ alkylenedioxy groups, such as the methylenedioxy, ethylenedioxy, propylenedioxy and trimethylenedioxy groups;
halogen atoms, such as those exemplified above in relation to R³ etc.;
cyano groups and nitro groups.

Thus, preferred compounds of the present invention are those compounds of formula (Ia): 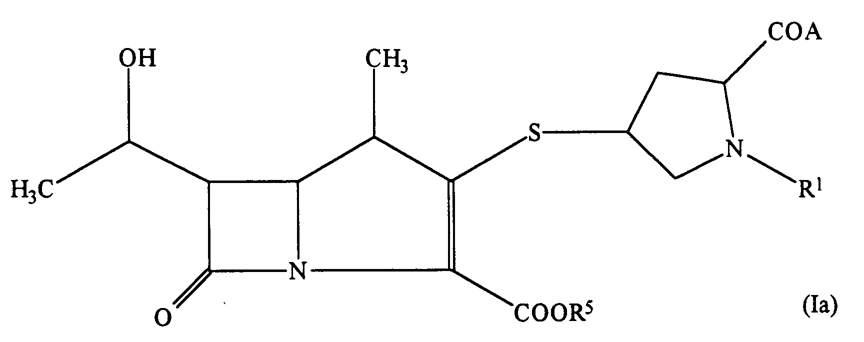 wherein R¹ and A are as defined above, and R⁵ represents a hydrogen atom or an ester group, preferably an ester group capable of hydrolysis in vivo. and more preferably an aliphatic acyloxyalkyl group, an alkoxycarbonyloxyalkyl group, a cycloalkylcarbonyloxyalkyl group, a phthalidyl group or a (5-substituted 2-oxo-1,3-dioxolen-4-yl)methyl group.

In particular, we prefer that R⁵ should represent a hydrogen atom, a (5-substituted 2-oxo-1,3-dioxolen-4-yl)methyl group, a 1-methylcyclohexylcarbonyloxymethyl group, a 1-isopropoxycarbonyloxyethyl group or a 1-cyclohexylcarbonyloxyethyl group.

The compounds of the present invention can also form salts with bases. Examples of such salts include: salts with an alkali metal, such as sodium, potassium or lithium; salts with an alkaline earth metal, such as barium or calcium; salts with another metal, such as magnesium or aluminum; organic base salts, such as a salt with triethylamine, diisopropylamine, cyclohexylamine or dicyclohexylamine; and salts with a basic amino acid, such as lysine or arginine. Also, where the compound of the present invention contains a basic group in its molecule, it can form acid addition salts. Examples of such acid addition salts include: salts with mineral acids, especially hydrohalic acids (such as hydrofluoric acid, hydrobromic acid, hydroiodic acid or hydrochloric acid), nitric acid, carbonic acid, sulphuric acid or phosphoric acid; salts with lower alkylsulphonic acids, such as methanesulphonic acid, trifluoromethanesulphonic acid or ethanesulphonic acid; salts with arylsulphonic acids, such as benzenesulphonic acid or p-toluenesulphonic acid; salts with organic carboxylic acids, such as acetic acid, fumaric acid, tartaric acid, oxalic acid, maleic acid, malic acid, succinic acid, benzoic acid, mandelic acid, ascorbic acid, lactic acid, gluconic acid or citric acid; and salts with amino acids, such as glutamic acid or aspartic acid.

Preferred groups and atoms which may be represented by R¹ include: the hydrogen atom; alkyl groups having from 1 to 3 carbon atoms (such as the methyl, ethyl and propyl groups); substituted alkyl groups having from 1 to 3 carbon atoms, in which the substituent is selected from substituents (a′), defined below; alkenyl groups having 3 or 4 carbon atoms (such as the allyl group); alkynyl groups having 3 or 4 carbon atoms (such as the propargyl group); and the formimidoyl and acetimidoyl groups.

Substituents (a′) are selected from hydroxy groups, carboxy groups, carbamoyl groups, carbamoyloxy groups, cyano groups, halogen atoms (such as fluorine atoms), alkoxy groups having from 1 to 3 carbon atoms (such as methoxy groups or ethoxy groups), amino groups, and mono- and di- alkylamino groups in which the or each alkyl group has from 1 to 3 carbon atoms (such as methylamino groups or dimethylamino groups).

In the compounds of formula (I) and (Ia) and pharmaceutically acceptable salts and esters thereof, where A represents a group of formula (A1), we prefer that n should be 2 or 3.

Also, in this case, R² preferably represents:
a hydrogen atom;
an alkyl group having from 1 to 3 carbon atoms, such as a methyl, ethyl or propyl group;
a substituted alkyl group having from 1 to 3 carbon atoms, in which the substituent is selected from substituents (b'), defined below, such as a substituted methyl, ethyl or propyl group;
an alkenyl group having 3 or 4 carbon atoms (such as the allyl group);
an alkynyl group having 3 or 4 carbon atoms (such as the propargyl group); or
a group of formula -C(=NH)R⁶,
   where R⁶ represents
   a hydrogen atom,
   an unsubstituted alkyl group having from 1 to 3 carbon atoms, such as a methyl or ethyl group,
   a substituted alkyl group which has from 1 to 3 carbon atoms and which is substituted by at least one substituent selected from halogen atoms,
   alkoxy groups having from 1 to 3 carbon atoms and cycloalkyl groups having from 3 to 6 carbon atoms, such as a chloromethyl group, a methoxymethyl group or a cyclopropylmethyl group, or
   cycloalkyl group having from 3 to 6 ring carbon atoms, such as a cyclopropyl group.

Substituents (b'), as mentioned above, include:
hydroxy groups;
carboxy groups;
carbamoyl groups;
carbamoyloxy groups;
cyano groups;
sulphamoyl groups;
ureido groups;
sulpho groups;
alkoxy groups having from 1 to 3 carbon atoms, such as the methoxy group;
alkoxycarbonyl groups having from 2 to 4 carbon atoms, such as the methoxycarbonyl group;
alkanoyl groups having from 2 to 4 carbon atoms, such as the acetyl group;
alkanoylamino groups having from 2 to 4 carbon atoms, such as the acetamido group;
alkanoyloxy groups having from 2 to 4 carbon atoms, such as the acetoxy group;
amino groups;
mono- and di- alkylamino groups in which the or each alkyl group has from 1 to 3 carbon atoms, such as the methylamino and dimethylamino groups;
alkylthio groups having from 1 to 3 carbon atoms, such as the methylthio group;
alkylsulphinyl groups having from 1 to 3 carbon atoms, such as the methylsulphinyl group;
alkylsulphonyl groups having from 1 to 3 carbon atoms, such as the methylsulphonyl group;
mono- and di- alkylcarbamoyl groups in which the or each alkyl group has from 1 to 3 carbon atoms, such as the methylcarbamoyl and dimethylcarbamoyl groups; and
mono- and di- alkylcarbamoyloxy groups in which the or each alkyl group has from 1 to 3 carbon atoms, such as the methylcarbamoyloxy and dimethylcarbamoyloxy groups.

In addition, where A represents a group of formula (A1), R³ and R⁴, which may be the same or different from each other, each represents a hydrogen atom, an alkyl group having from 1 to 3 carbon atoms (such as a methyl or ethyl group), a hydroxy group, a carboxy group, a carbamoyl group or a substituted alkyl group which has from 1 to 3 carbon atoms and which is substituted by at least one substituent selected from hydroxy groups, alkoxy groups having from 1 to 3 carbon atoms, amino groups, carbamoyl groups and halogen atoms (such as the hydroxymethyl, methoxymethyl, aminomethyl, carbamoylmethyl and fluoromethyl groups).

More preferred compounds of formula (I) and (Ia) and pharmaceutically acceptable salts and esters thereof, in which A represents a group of formula (A1) are those in which:
n is 2;
R¹ represents a hydrogen atom, a methyl group, a fluoromethyl group, a formimidoyl group or an acetimidoyl group;
R² represents a hydrogen atom, a 2-hydroxyethyl group, a 2-carbamoylethyl group, a carboxymethyl group, a carbamoylmethyl group, a 2-fluoroethyl group, a formimidoyl group or an acetimidoyl group; and
R³ and R⁴ are the same or different and each represents a hydrogen atom, a methyl group, a carbamoyl group, a cyano group, a carboxy group, a hydroxymethyl group, a fluoromethyl group or an aminomethyl group.

An alternative preferred class of compounds of formula (I) and (Ia) and pharmaceutically acceptable salts and esters thereof, in which A represents a group of formula (A1) are those in which:
n is 3;
R¹ represents a hydrogen atom, a methyl group, a fluoromethyl group, a formimidoyl group or an acetimidoyl group;
R² represents a hydrogen atom, a methyl group, a formimidoyl group, an acetimidoyl group, a carboxymethyl group, a carbamoylmethyl group, a 2-hydroxyethyl group or a 2-fluoroethyl group; and
R³ and R⁴ are the same or different and each represents a hydrogen atom, a methyl group, a hydroxy group, an amino group, a cyano group, a carboxy group, a carbamoyl group, a carbamoyloxy group, a hydroxymethyl group, a fluoromethyl group or an aminomethyl group.

A most preferred class of compounds of formula (I) and (Ia) and pharmaceutically acceptable salts and esters thereof, in which A represents a group of formula (A1) are those in which:
n is 2;
R¹ represents a hydrogen atom, a methyl group, a formimidoyl group or an acetimidoyl group;
R² represents a hydrogen atom, a 2-hydroxyethyl group, a carboxymethyl group, a formimidoyl group or an acetimidoyl group;
R³ represents a hydrogen atom; and
R⁴ represents a methyl group, a carbamoyl group, a cyano group, a hydroxymethyl group, a fluoromethyl group or an aminomethyl group.

An alternative most preferred class of compounds of formula (I) and (Ia) and pharmaceutically acceptable salts and esters thereof, in which A represents a group of formula (A1), are those in which:
n is 3;
R¹ represents a hydrogen atom, a methyl group, a formimidoyl group or an acetimidoyl group;
R² represents a formimidoyl group, an acetimidoyl group, a carboxymethyl group, a 2-hydroxyethyl group or a 2-fluoroethyl group; and
R³ and R⁴ are the same or different and each represents a hydrogen atom, a hydroxy group, an amino group or a cyano group.

Where A in the compound of formula (I) or (Ia) and pharmaceutically acceptable salts and esters thereof represents a group of formula (A2), d is 0 or 1, and m is 0, 1 or 2. In this case, we prefer that R⁷ should represent:
a hydrogen atom;
a carboxy group;
a carbamoyl group;
an alkyl group having from 1 to 3 carbon atoms, such as a methyl, ethyl or propyl group; or
a substituted alkyl group having from 1 to 3 carbon atoms, in which the substituent is selected from hydroxy groups, alkoxy groups having from 1 to 3 carbon atoms (such as the methoxy or ethoxy groups), carbamoyl groups, carboxy groups and cyano groups, such as a substituted methyl, ethyl or propyl group.

Where A represents a group of formula (A2), we also prefer that R⁸ should represent:
a hydrogen atom;
an alkyl group having from 1 to 3 carbon atoms, such as a methyl, ethyl or propyl group;
a substituted alkyl group having from 1 to 3 carbon atoms, such as a substituted methyl, ethyl or propyl group, in which the substituent is selected from hydroxy groups, alkoxy groups having from 1 to 3 carbon atoms (such as the methoxy or ethoxy groups), carbamoyl groups, carbamoyloxy groups, carboxy groups, cyano groups, amino groups and halogen atoms (such as the fluorine and chlorine atoms);
an alkenyl group having 3 or 4 carbon atoms (such as the allyl group); or
an alkynyl group having 3 or 4 carbon atoms (such as the propargyl group).

In such a case, R⁹ preferably represents:
a hydrogen atom;
an alkyl group having from 1 to 3 carbon atoms, such as a methyl, ethyl or propyl group;
a substituted alkyl group having from 1 to 3 carbon atoms, such as a substituted methyl, ethyl or propyl group, in which the substituent is selected from hydroxy groups, alkoxy groups having from 1 to 3 carbon atoms (such as the methoxy or ethoxy groups), carbamoyl groups, carbamoyloxy groups, carboxy groups, cyano groups, amino groups and halogen atoms (such as the fluorine and chlorine atoms); or
a group of formula -C(=NH)R¹⁰, in which R¹⁰ represents:
   a hydrogen atom;
   an alkyl group having from 1 to 3 carbon atoms, such as a methyl, ethyl or propyl group;
   a substituted alkyl group having from 1 to 3 carbon atoms, such as a substituted methyl, ethyl or propyl group, in which the substituent is selected from alkoxy groups having from 1 to 3 carbon atoms (such as the methoxy or ethoxy groups) and halogen atoms (such as the fluorine and chlorine atoms);
   a cycloalkyl group having from 3 to 6 carbon atoms, such as a cyclopropyl group or a cyclobutyl group; or
   an alkyl group having from 1 to 3 carbon atoms, such as a methyl or ethyl group, which is substituted by a single cycloalkyl group having from 3 to 6 carbon atoms, such as a cyclopropylmethyl group, a cyclopropylethyl group or a cyclobutylmethyl group.

Alternatively, R⁸ and R⁹ may together represent a group of formula -(CH₂)ₛ-W-(CH₂)ₜ-, wherein W represents a carbon-carbon single bond, an oxygen atom, a sulphur atom or a group of formula >NR²², wherein R²² represents a hydrogen atom or an alkyl group having from 1 to 3 carbon atoms (such as a methyl or ethyl group), s is 1, 2 or 3 and t is 2.

In particular, a preferred class of compounds of the present invention in which A represents a group of formula (A2) are those compounds of formula (I) or (Ia), and pharmaceutically acceptable salts and esters thereof, in which:
d is 0 or 1;
m is 0, 1 or 2;
R¹ represents a hydrogen atom, a methyl group, a fluoromethyl group, a formimidoyl group or an acetimidoyl group;
R⁷ represents a hydrogen atom, an alkyl group having from 1 to 3 carbon atoms (such as a methyl group or an ethyl group), a hydroxy group, an amino group, a cyano group, a halogen atom (such as a fluorine atom or a chlorine atom), a carboxy group, a carbamoyl group or a hydroxymethyl group;
R⁸ represents a hydrogen atom, an alkyl group having from 1 to 3 carbon atoms (such as a methyl group or an ethyl group), a fluoromethyl group, a carbamoylmethyl group, a carboxymethyl group, an alkenyl group having 3 or 4 carbon atoms (such as an allyl group), an alkynyl group having 3 or 4 carbon atoms (such as a propargyl group), a 2-haloethyl group (such as a 2-fluoroethyl group), a 2-hydroxyethyl group, a 2-alkoxyethyl group, in which the alkoxy part has from 1 to 3 carbon atoms (such as a 2-methoxyethyl group) or a 2-aminoethyl group;
R⁹ represents a hydrogen atom, an alkyl group having from 1 to 3 carbon atoms (such as a methyl group or an ethyl group), a fluoromethyl group, a carbamoylmethyl group, a carboxymethyl group, a formimidoyl group, an acetimidoyl group, a 2-haloethyl group (such as a 2-fluoroethyl group), a 2-hydroxyethyl group, a 2-alkoxyethyl group, in which the alkoxy part has from 1 to 3 carbon atoms (such as a 2-methoxyethyl group) or a 2-aminoethyl group;
   or
R⁸ and R⁹ together represent a group of formula
   -(CH₂)₄-,
   -(CH₂)₅-,
   -(CH₂)₂O(CH₂)₂-,
   -(CH₂)₂S(CH₂)₂-,
   -(CH₂)₂NH(CH₂)₂- or
-(CH₂)₂NCH₃(CH₂)₂-.

The most preferred class of compounds of the present invention in which A represents a group of formula (A2) are those compounds of formula (I) and (Ia) and pharmaceutically acceptable salts and esters thereof, in which:
d is 0;
m is 1 or 2;
R¹ represents a hydrogen atom, a methyl group, a fluoromethyl group, a formimidoyl group or an acetimidoyl group;
R⁷ represents a hydrogen atom;
R⁸ represents a hydrogen atom, an alkyl group having from 1 to 3 carbon atoms (such as a methyl group or an ethyl group), a carbamoylmethyl group, a carboxymethyl group, a 2-fluoroethyl group or a 2-hydroxyethyl group; and
R⁹ represents a hydrogen atom, an alkyl group having from 1 to 3 carbon atoms (such as a methyl group or an ethyl group), a formimidoyl group, an acetimidoyl group or a 2-fluoroethyl group.

In the case of those compounds of the present invention in which A represents a group of formula (A3), ℓ is 0, 1 or 2. R⁷ preferably represents a hydrogen atom or an alkyl group having from 1 to 3 carbon atoms, such as a methyl, ethyl or propyl group.

R¹¹ preferably represents a hydrogen atom or an alkyl group having from 1 to 3 carbon atoms, such as a methyl, ethyl or propyl group.

R¹² also preferably represents:
a hydrogen atom;
an alkyl group having from 1 to 3 carbon atoms, such as a methyl, ethyl or propyl group;
a substituted alkyl group having from 1 to 3 carbon atoms, such as a substituted methyl, ethyl or propyl group, in which the substituent is selected from hydroxy groups, alkoxy groups having from 1 to 3 carbon atoms (such as the methoxy or ethoxy groups), carbamoyl groups, carbamoyloxy groups, carboxy groups, cyano groups, amino groups and halogen atoms (such as the fluorine and chlorine atoms);
an alkenyl group having 3 or 4 carbon atoms (such as the allyl group);
an alkynyl group having 3 or 4 carbon atoms (such as the propargyl group) or;
a group of formula -C(=NH)R¹³, in which R¹³ represents:
   a hydrogen atom;
   an alkyl group having from 1 to 3 carbon atoms, such as a methyl, ethyl or propyl group;
   a substituted alkyl group having from 1 to 3 carbon atoms, such as a substituted methyl, ethyl or propyl group, in which the substituent is selected from alkoxy groups having from 1 to 3 carbon atoms (such as the methoxy or ethoxy groups) and halogen atoms (such as the fluorine and chlorine atoms);
   a cycloalkyl group having from 3 to 6 carbon atoms, such as a cyclopropyl group or a cyclobutyl group; or
   an alkyl group having from 1 to 3 carbon atoms, such as a methyl or ethyl group, which is substituted by a single cycloalkyl group having from 3 to 6 carbon atoms, such as a cyclopropylmethyl group, a cyclopropylethyl group or a cyclobutylmethyl group.

In particular, a preferred class of compounds of the present invention in which A represents a group of formula (A3) are those compounds of formula (I) or (Ia), and pharmaceutically acceptable salts and esters thereof, in which:
ℓ is 0, 1 or 2;
R¹ represents a hydrogen atom, a methyl group, a fluoromethyl group, a formimidoyl group or an acetimidoyl group;
R⁷ represents a hydrogen atom;
R¹¹ represents a hydrogen atom or an alkyl group having from 1 to 3 carbon atoms, such as a methyl group or an ethyl group; and
R¹² represents a hydrogen atom, an alkyl group having from 1 to 3 carbon atoms (such as a methyl group or an ethyl group), a fluoromethyl group, a carbamoylmethyl group, a carboxymethyl group, an alkenyl group having 3 or 4 carbon atoms (such as the allyl group), an alkynyl group having 3 or 4 carbon atoms (such as the propargyl group), a formimidoyl group, an acetimidoyl group, a 2-haloethyl group (such as a 2-fluoroethyl group), a 2-hydroxyethyl group, a 2-alkoxyethyl group, in which the alkoxy part has from 1 to 3 carbon atoms (such as a 2-methoxyethyl group) or a 2-aminoethyl group.

The most preferred class of compounds of the present invention in which A represents a group of formula (A3) are those compounds of formula (I) and (Ia) and pharmaceutically acceptable salts and esters thereof, in which:
ℓ is 1 or 2;
R¹ represents a hydrogen atom, a methyl group, a fluoromethyl group, a formimidoyl group or an acetimidoyl group;
R⁷ represents a hydrogen atom;
R¹¹ represents a hydrogen atom or a methyl group; and
R¹² represents a hydrogen atom, an alkyl group having from 1 to 3 carbon atoms (such as a methyl group or an ethyl group), a fluoromethyl group, a carbamoylmethyl group, a carboxymethyl group, a formimidoyl group, an acetimidoyl group, a 2-fluoroethyl group or a 2-hydroxyethyl group.

In the case of those compounds of the present invention in which A represents a group of formula (A4), i is 1 or 2. R¹⁴ and R¹⁵, which may be the same or different, preferably each represents a hydrogen atom or an alkyl group having from 1 to 3 carbon atoms, such as a methyl, ethyl or propyl group.

Additionally, we prefer that R¹⁶ should represent:
a hydrogen atom;
an alkyl group having from 1 to 3 carbon atoms, such as a methyl, ethyl or propyl group;
a substituted alkyl group having from 1 to 3 carbon atoms, such as a substituted methyl, ethyl or propyl group, in which the substituent is selected from alkoxy groups having from 1 to 3 carbon atoms (such as the methoxy or ethoxy groups) and halogen atoms (such as the fluorine and chlorine atoms);
a cycloalkyl group having from 3 to 6 carbon atoms, such as a cyclopropyl group or a cyclobutyl group; or
an alkyl group having from 1 to 3 carbon atoms, such as a methyl or ethyl group, which is substituted by a single cycloalkyl group having from 3 to 6 carbon atoms, such as a cyclopropylmethyl group, a cyclopropylethyl group or a cyclobutylmethyl group.

In particular, a preferred class of compounds of the present invention in which A represents a group of formula (A4) are those compounds of formula (I) or (Ia), in which:
i is 1 or 2; and
R¹, R¹⁴, R¹⁵ and R¹⁶ are independently selected from hydrogen atoms and alkyl groups having from 1 to 3 carbon atoms (such as the methyl group or the ethyl group, especially the methyl group).

The most preferred class of compounds of the present invention in which A represents a group of formula (A4) are those compounds of formula (I) and (Ia) and pharmaceutically acceptable salts and esters thereof, in which:
i is 1; and
R¹, R¹⁴, R¹⁵ and R¹⁶ are independently selected from hydrogen atoms and methyl groups.

In the case of those compounds of the present invention in which A represents a group of formula (A5), p is 1, 2 or 3, preferably 2. R¹⁷ and R¹⁸, which may be the same or different, preferably each represents:
a hydrogen atom;
an alkyl group having from 1 to 3 carbon atoms, such as a methyl, ethyl or propyl group;
a substituted alkyl group having from 1 to 3 carbon atoms, such as a substituted methyl, ethyl or propyl group, in which the substituent is selected from hydroxy groups, alkoxy groups having from 1 to 3 carbon atoms (such as the methoxy or ethoxy groups) and halogen atoms (such as the fluorine and chlorine atoms).

Alternatively, R¹⁷ and R¹⁸ may together preferably represent a group of formula -(CH₂)_{q}-Y-(CH₂)ᵣ-, wherein Y represents a carbon-carbon single bond, an oxygen atom or a group of formula >NR²³, wherein R²³ represents a hydrogen atom or an alkyl group having from 1 to 3 carbon atoms (such as a methyl or ethyl group), and q and r are each 2 or 3.

In particular, a preferred class of compounds of the present invention in which A represents a group of formula (A5) are those compounds of formula (I) or (Ia), and pharmaceutically acceptable salts and esters thereof, in which:
p is 2;
R¹ represents a hydrogen atom, a methyl group, a fluoromethyl group, a formimidoyl group or an acetimidoyl group;
R¹⁷ and R¹⁸ are the same or different and each represents a hydrogen atom, an alkyl group having from 1 to 3 carbon atoms (such as a methyl group or an ethyl group), a 2-haloethyl group (such as a 2-fluoroethyl group) or a 2-hydroxyethyl group;
   or
R¹⁷ and R¹⁸ together represent a group of formula
   -(CH₂)₄-,
   -(CH₂)₅-,
   -(CH₂)₂O(CH₂)₂-,
   -(CH₂)₂S(CH₂)₂-,
   -(CH₂)₂NH(CH₂)₂- or
   -(CH₂)₂NCH₃(CH₂)₂-.

The most preferred class of compounds of the present invention in which A represents a group of formula (A5) are those compounds of formula (I) and (Ia) and pharmaceutically acceptable salts and esters thereof, in which:
p is 2;
R¹ represents a hydrogen atom, a methyl group, a fluoromethyl group, a formimidoyl group or an acetimidoyl group; and
R¹⁷ and R¹⁸ are the same or different and each represents a hydrogen atom or a methyl group.

In the case of those compounds of the present invention in which A represents a group of formula (A6), j and k are independently 1 or 2, and preferably each is 2.

R¹⁹ preferably represents a hydrogen atom or an alkyl group having from 1 to 3 carbon atoms (such as a methyl group or an ethyl group).

In particular, a preferred class of compounds of the present invention in which A represents a group of formula (A6) are those compounds of formula (I) or (Ia), and pharmaceutically acceptable salts and esters thereof, in which:
j and k are both 2;
R¹ represents a hydrogen atom, a methyl group, a fluoromethyl group, a formimidoyl group or an acetimidoyl group; and
R¹⁹ preferably represents a hydrogen atom or an alkyl group having from 1 to 3 carbon atoms (such as a methyl group or an ethyl group).

The most preferred class of compounds of the present invention in which A represents a group of formula (A6) are those compounds of formula (I) and (Ia) and pharmaceutically acceptable salts and esters thereof, in which:
j and k are both 2; and
R¹ and R¹⁹ are independently selected from hydrogen atoms and methyl groups.

In the case of those compounds of the present invention in which A represents a group of formula (A7), q is 0, 1 or 2, and preferably 1 or 2.

In these compounds, Z preferably represents a 1-imidazolyl group, a 1,2,4-triazol-1-yl group or a 1,2,3-triazol-1-yl group.

In particular, a preferred class of compounds of the present invention in which A represents a group of formula (A7) are those compounds of formula (I) or (Ia), and pharmaceutically acceptable salts and esters thereof, in which:
q is 0, 1 or 2;
R¹ represents a hydrogen atom or a methyl group; and
Z represents a 1-imidazolyl group, a 1,2,4-triazol-1-yl group or a 1,2,3-triazol-1-yl group.

The most preferred class of compounds of the present invention in which A represents a group of formula (A7) are those compounds of formula (I) and (Ia) and pharmaceutically acceptable salts and esters thereof, in which:
q is 1 or 2;
R¹ represents a hydrogen atom or a methyl group; and
Z represents a 1-imidazolyl group, a 1,2,4-triazol-1-yl group or a 1,2,3-triazol-1-yl group.

In the case of those compounds of the present invention in which A represents a group of formula (A8), e and f are independently 1 or 2, and preferably each is 1.

R²⁰ and R²¹, which may be the same or different, preferably each represents a hydrogen atom or an alkyl group having from 1 to 3 carbon atoms, such as a methyl, ethyl or propyl group.

In particular, a preferred class of compounds of the present invention in which A represents a group of formula (A8) are those compounds of formula (I) or (Ia), and pharmaceutically acceptable salts and esters thereof, in which:
e and f are both 1;
R¹ represents a hydrogen atom, a methyl group, a fluoromethyl group, a formimidoyl group or an acetimidoyl group; and
R²⁰ and R²¹, which may be the same or different, each represents a hydrogen atom or an alkyl group having from 1 to 3 carbon atoms, such as a methyl, ethyl or propyl group.

The most preferred class of compounds of the present invention in which A represents a group of formula (A8) are those compounds of formula (I) and (Ia) and pharmaceutically acceptable salts and esters thereof, in which:
e and f are both 1;
R¹ represents a hydrogen atom or a methyl group;
R²⁰ represents a hydrogen atom; and
R²¹ represents a hydrogen atom or a methyl group.

In the case of all of the compounds, including the preferred compounds and most preferred compounds, referred to above, we prefer those compounds in which R⁵ represents a hydrogen atom, that is to say compounds of formula (I).

The compounds of the present invention necessarily contain several asymmetric carbon atoms in their molecules, and can thus form optical isomers. Although these are all represented herein by a single molecular formula, the present invention includes both the individual, isolated isomers and mixtures, including racemates thereof. Where stereospecific synthesis techniques are employed or optically active compounds are employed as starting materials, individual isomers may be prepared directly; on the other hand, if a mixture of isomers is prepared, the individual isomers may be obtained by conventional resolution techniques.

Of the isomers, we especially prefer those in which the carbon atoms are in the same configurations as those of thienamycin, that is: in the R configuration at position 1, in the (5S, 6S) configuration at positions 5 and 6, and in the R configuration at the hydroxy-substituted α-position of the side chain at position 6.

Specific examples of compounds of the present invention are those compounds of formula (I), in which the various substituent groups are as defined in Tables 1 to 8. In the Tables, the following abbreviations are used:
- Ac: acetyl
- Acim: acetimidoyl
- All: allyl
- Azp: perhydroazepinyl (= homopiperazinyl)
- Azt: azetidinyl
- Car: carbamoyl
- Et: ethyl
- Foim: formimidoyl
- Imaz: imidazolidinyl
- Imid: imidazolyl
- Me: methyl
- Mec: methoxycarbonyl
- Mor: morpholino
- Pip: piperidyl
- Piz: piperazinyl
- Prg: propargyl (= 2-propynyl)
- Pyrd: pyrrolidinyl
- Sam: sulphamoyl
- Thz: perhydro-1,4-thiazin-4-yl (= thiomorpholino)
- Ur: ureido

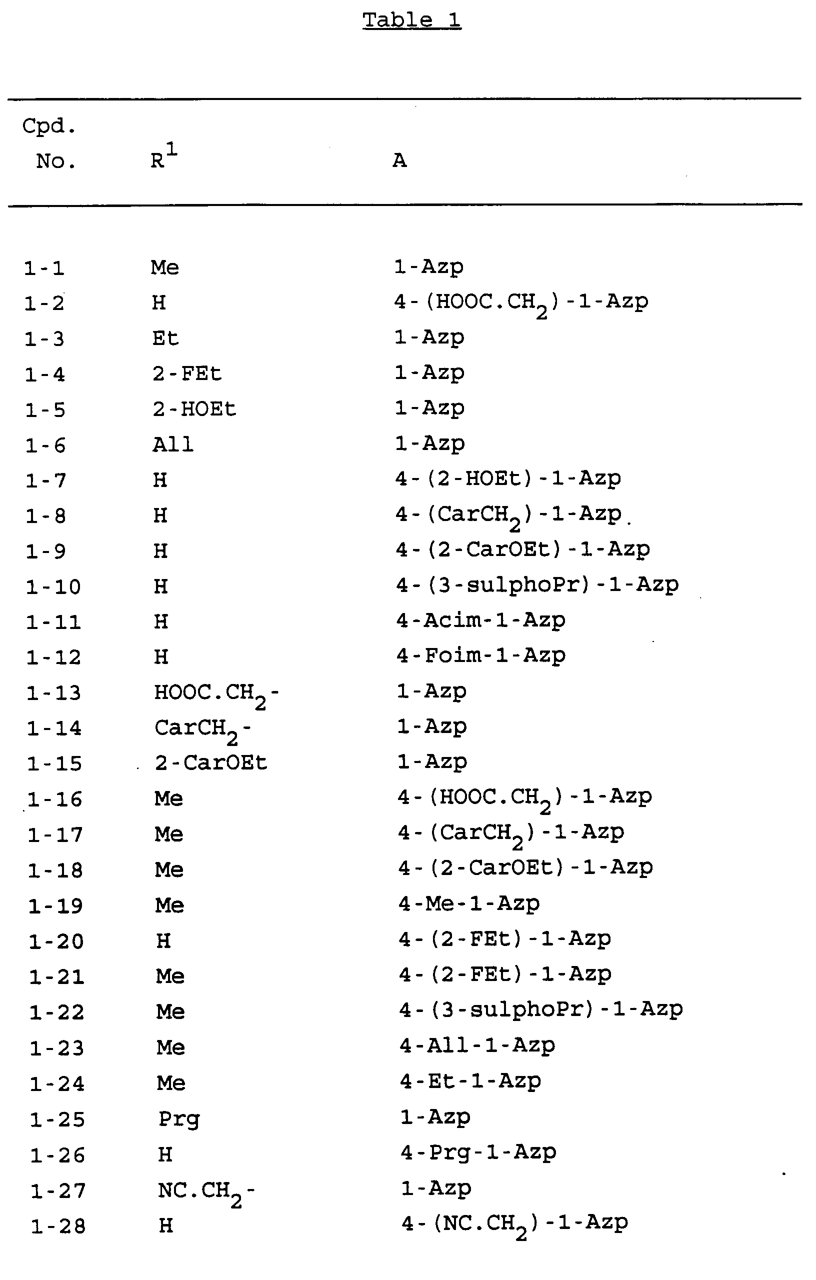

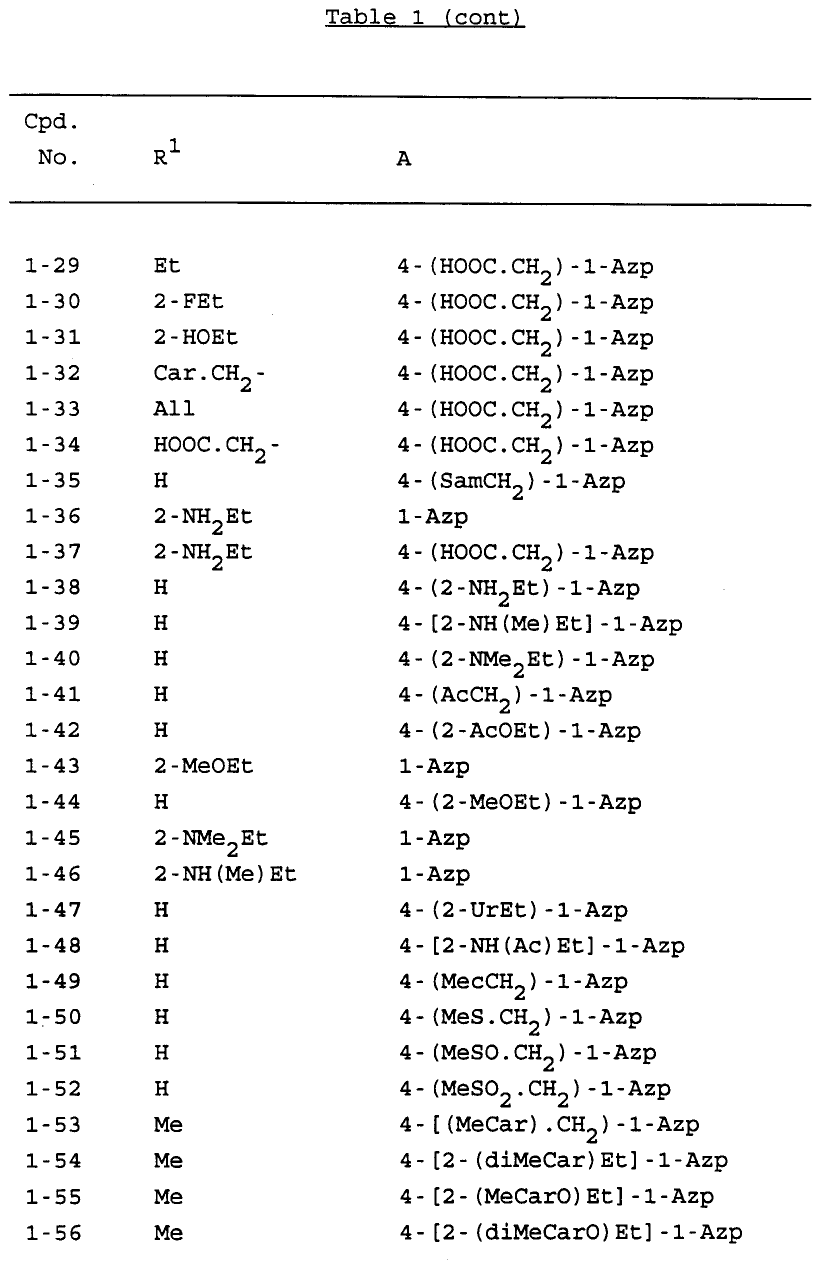

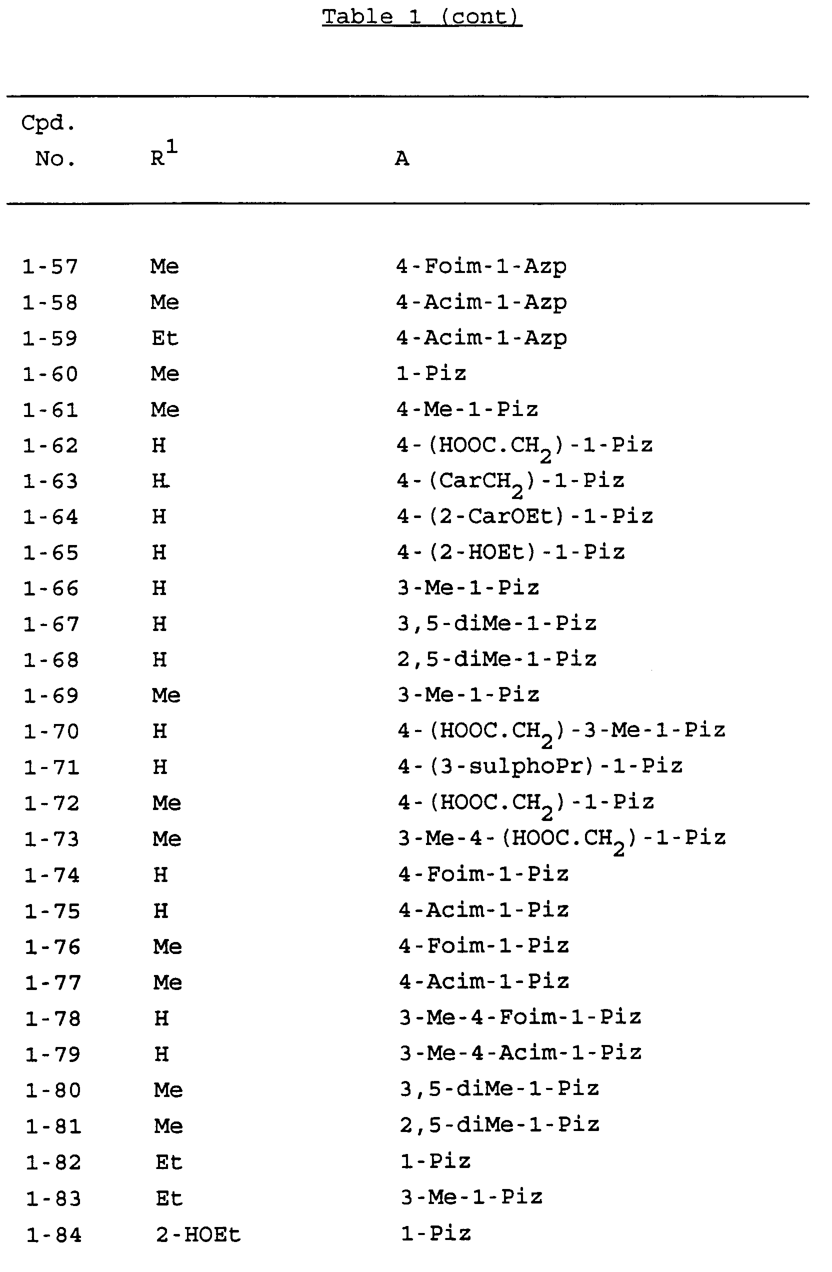

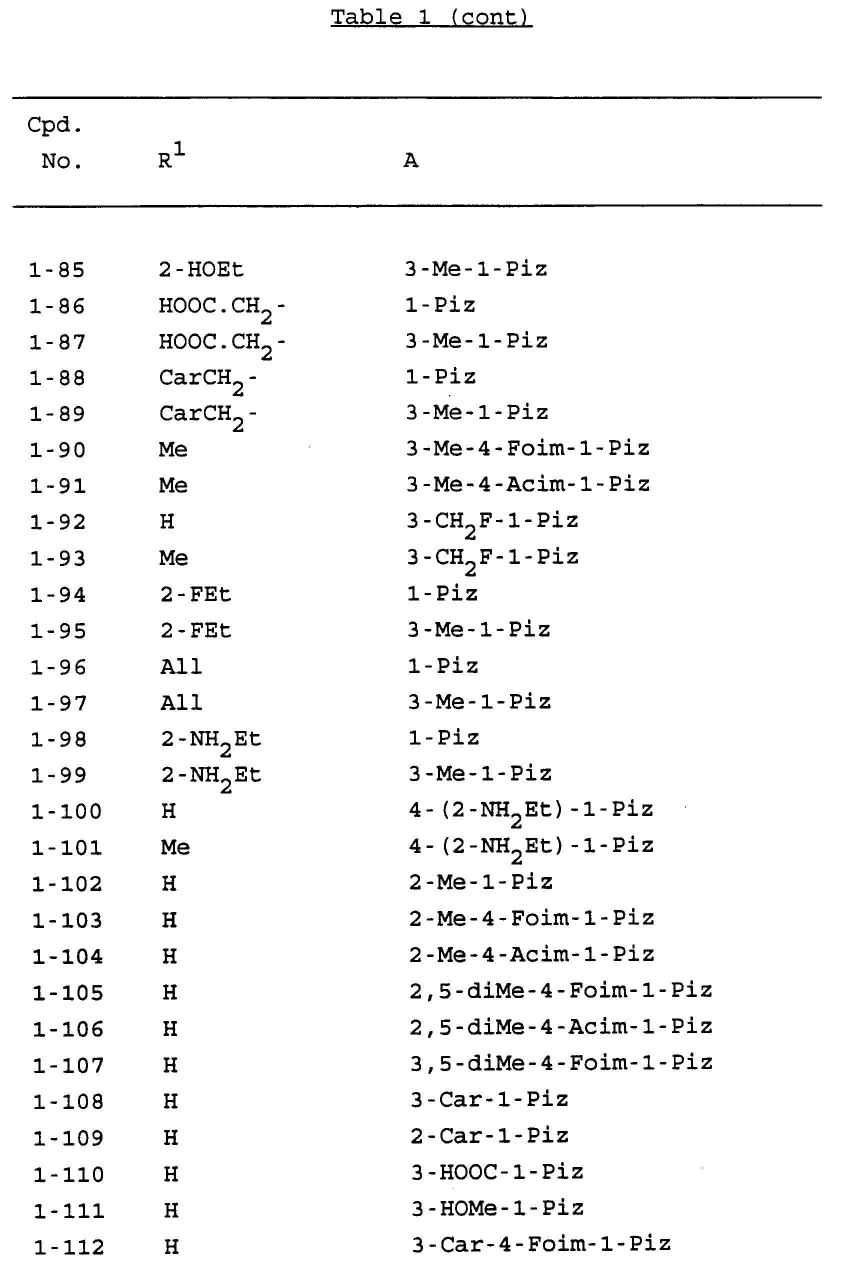

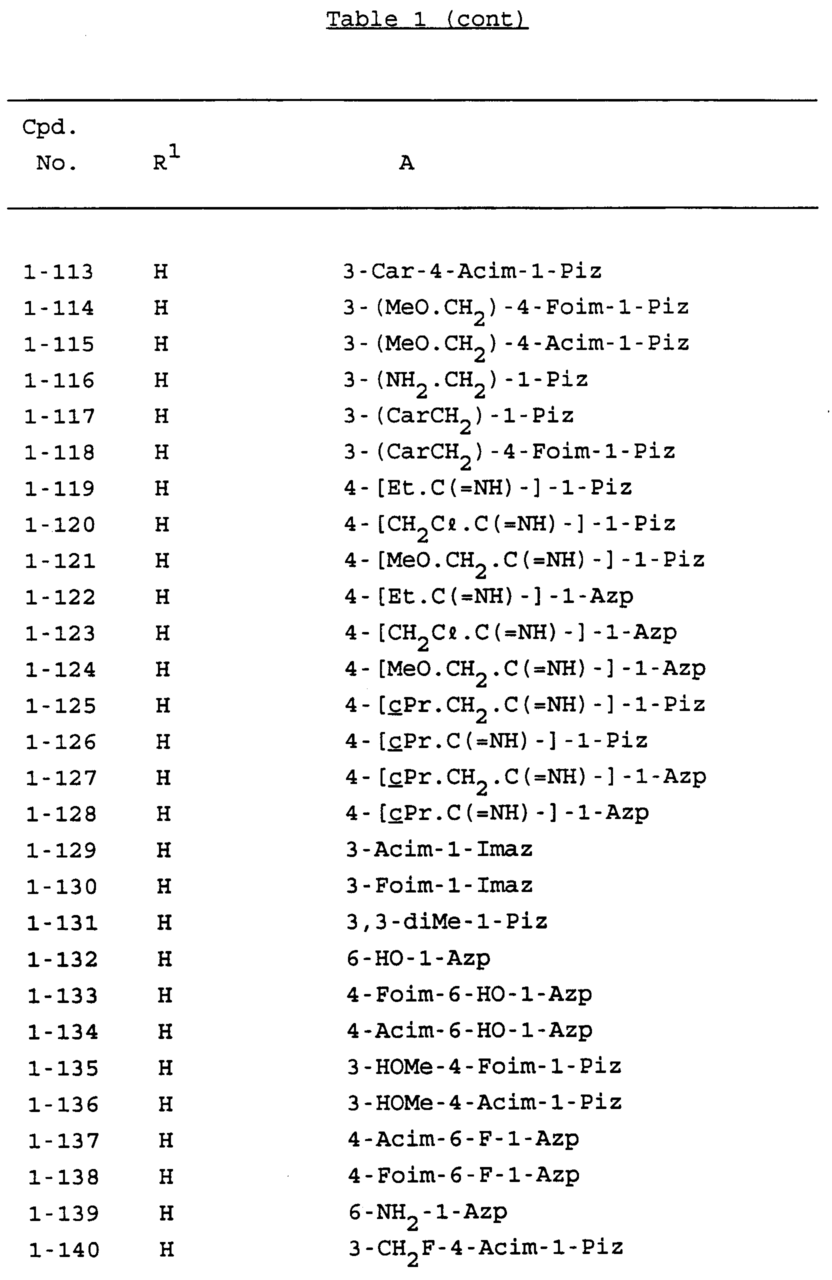

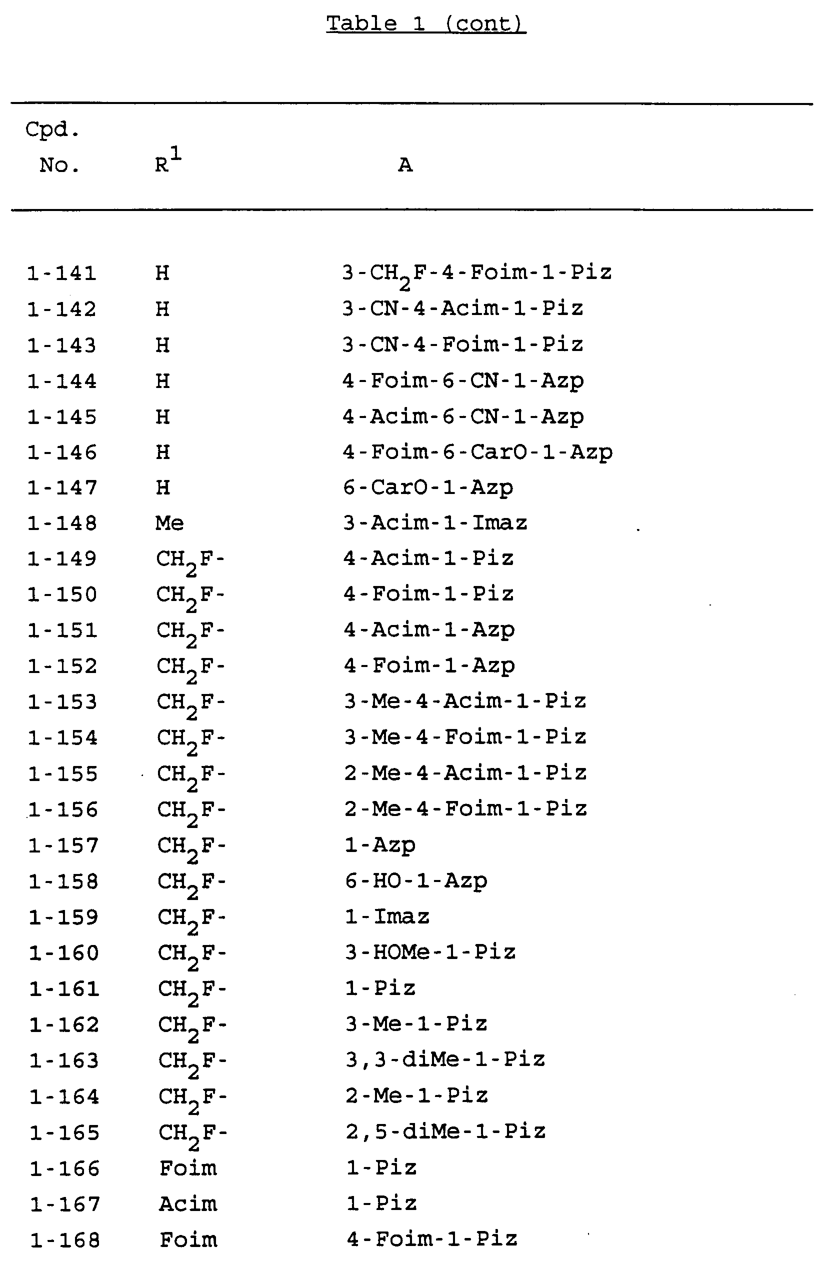

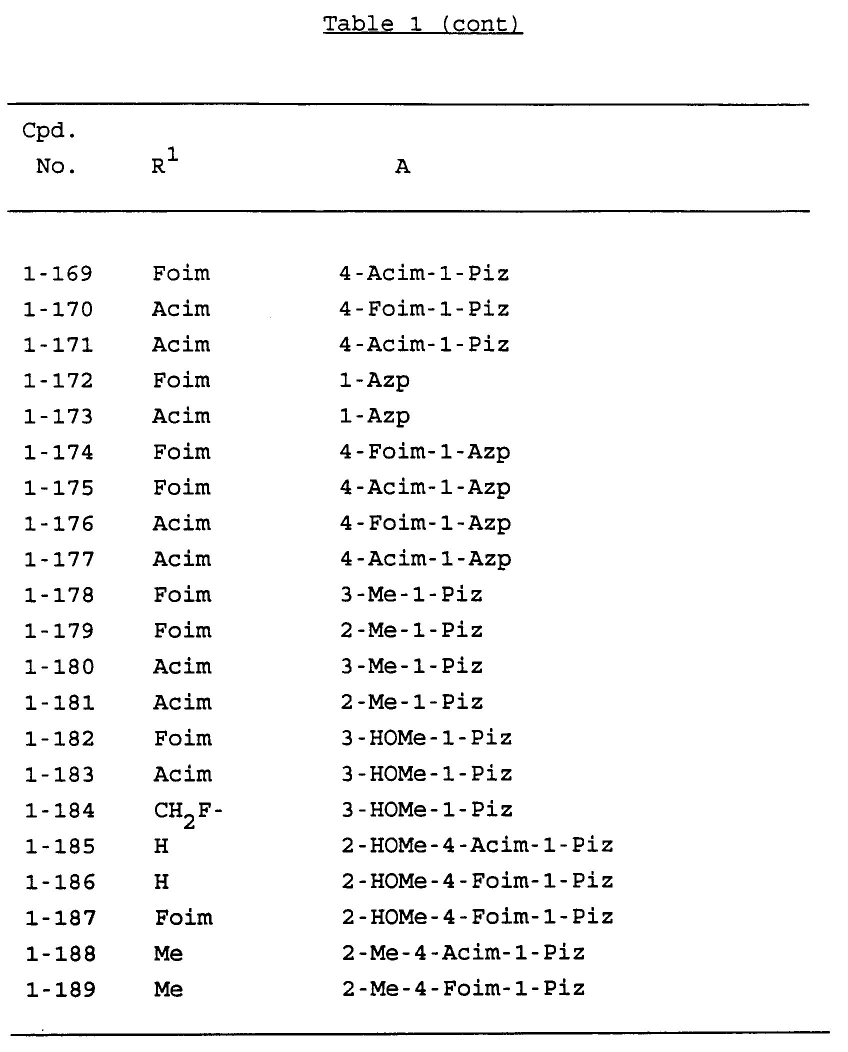

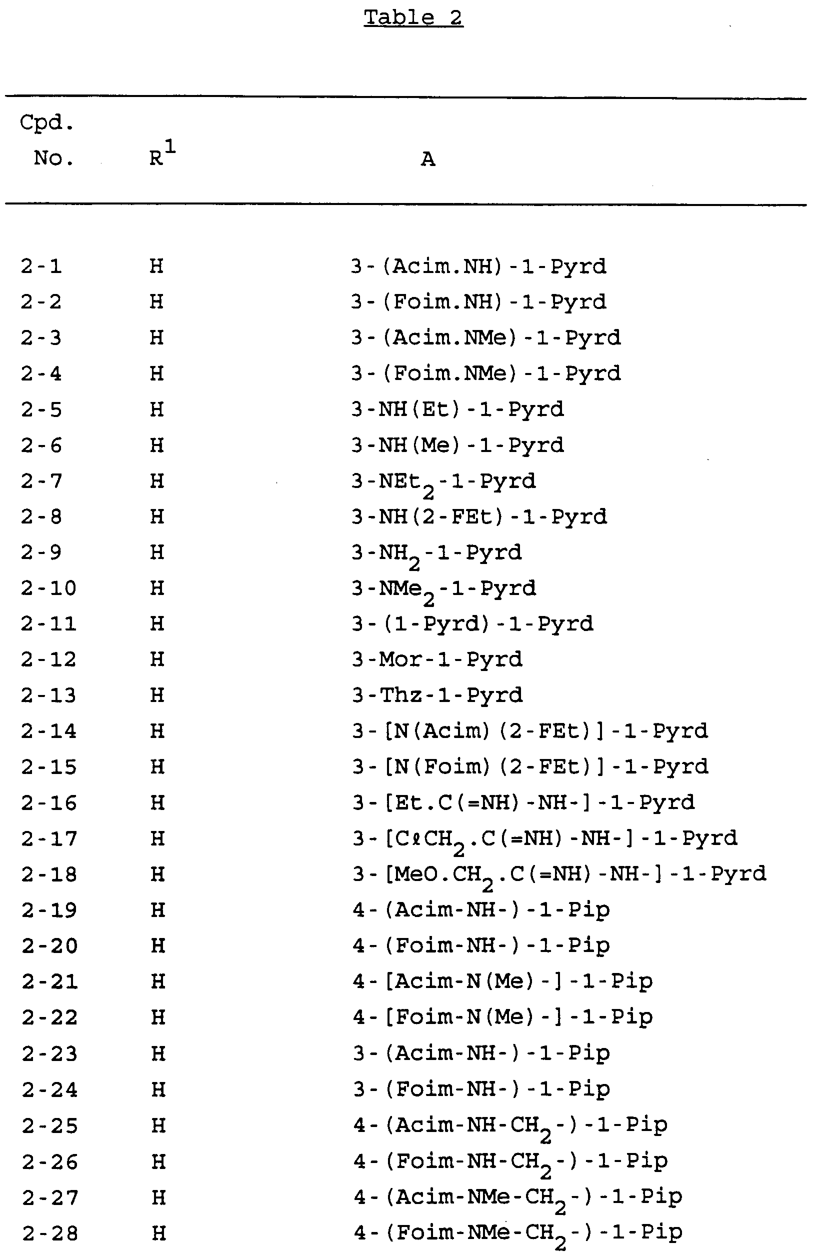

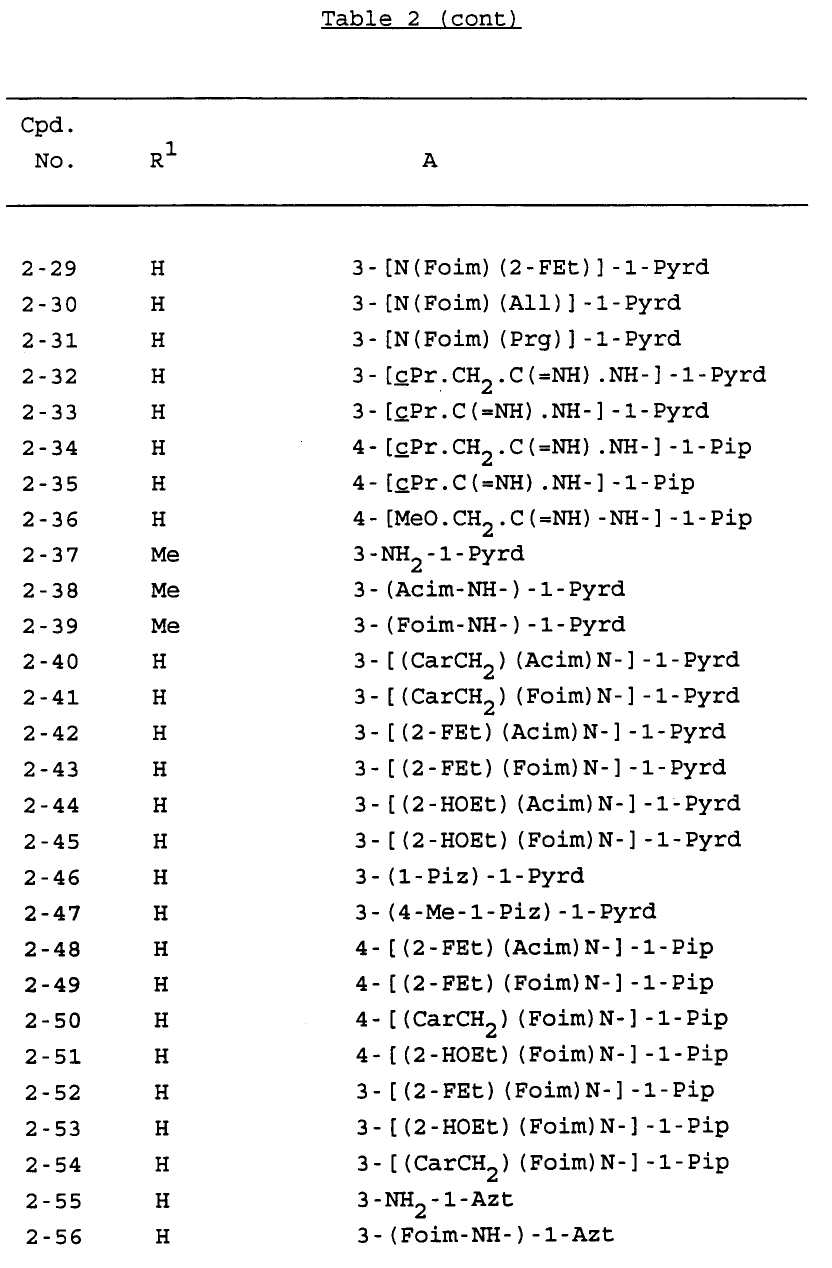

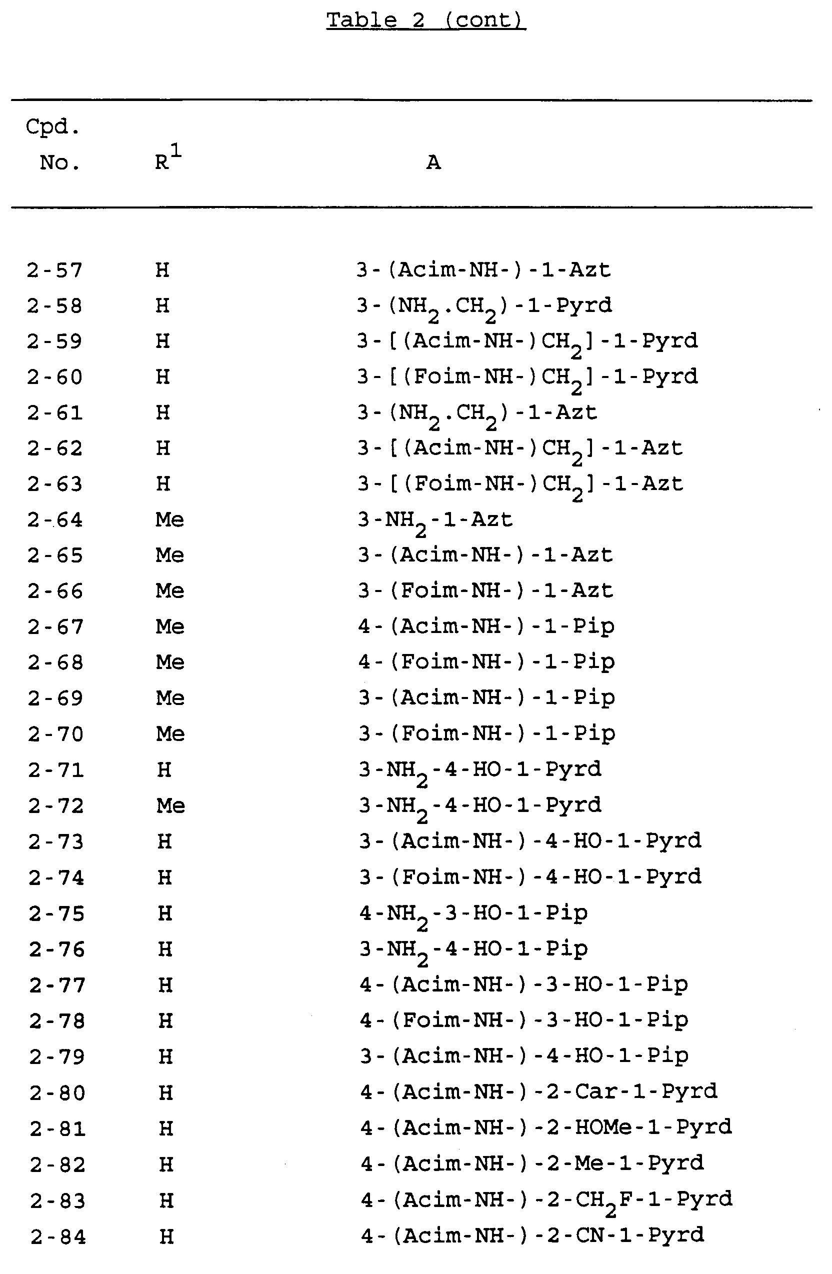

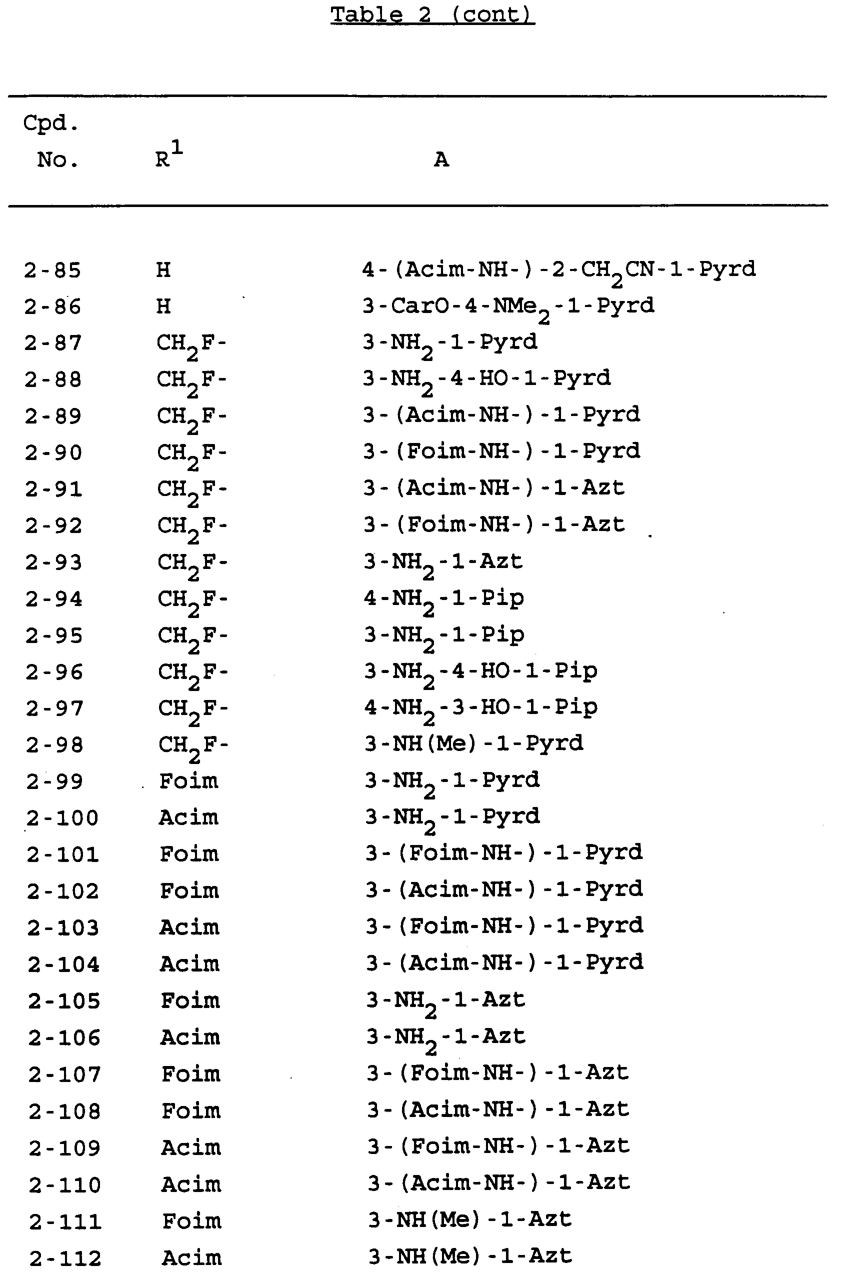

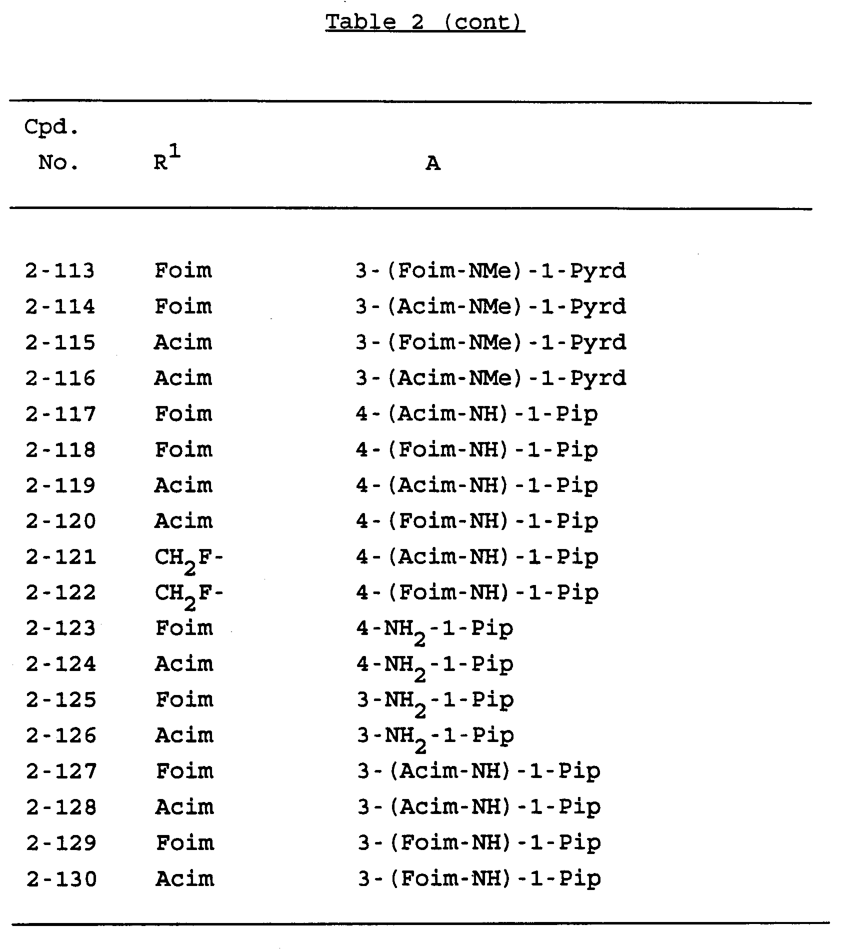

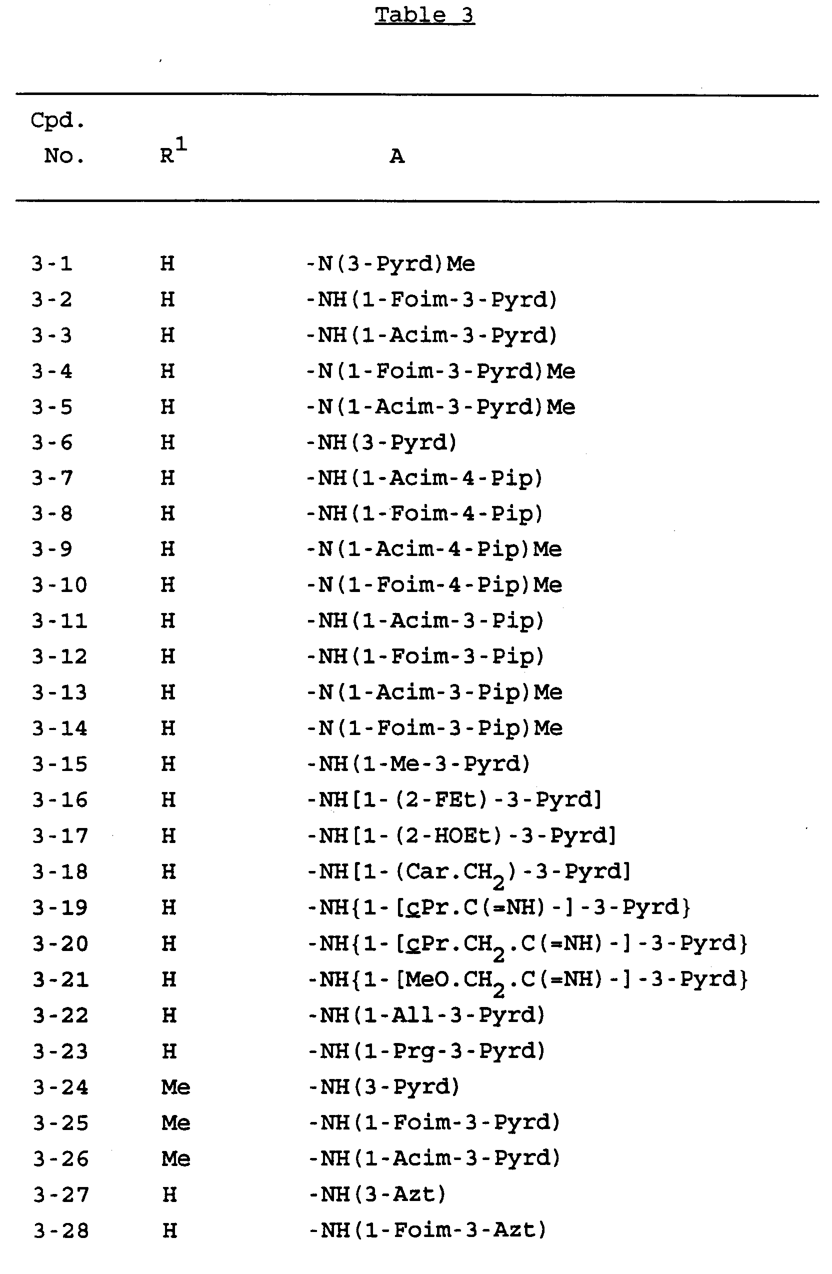

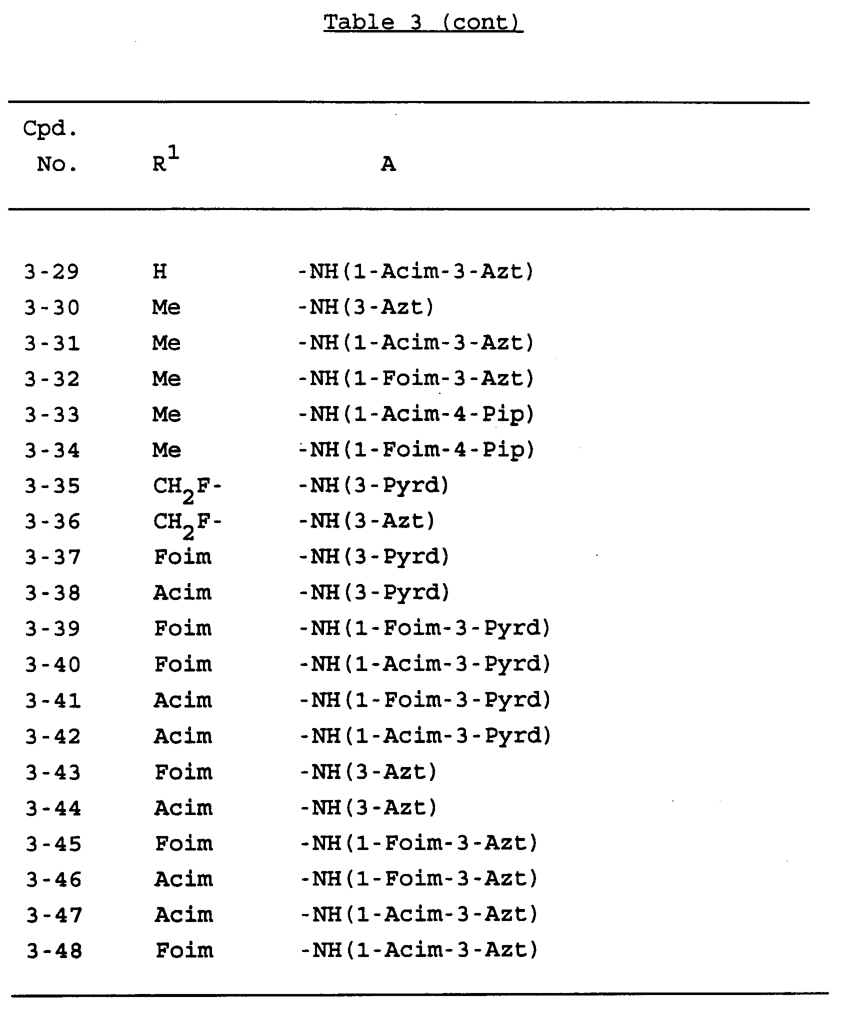

**Table 4**

| Cpd. No. | R¹ | A |
|---|---|---|
| 4-1 | H | -NH-CH₂CH₂-NH-CH=NH |
| 4-2 | H | -NH-CH₂CH₂-NH-C(=NH)-Me |
| 4-3 | H | -NH-CH₂CH₂-NMe-CH=NH |
| 4-4 | H | -NH-CH₂CH₂-NMe-C(=NH)-Me |
| 4-5 | H | -NMe-CH₂CH₂-NMe-CH=NH |
| 4-6 | H | -NMe-CH₂CH₂-NMe-C(=NH)-Me |
| 4-7 | Me | -NH-CH₂CH₂-NH-CH=NH |
| 4-8 | Me | -NH-CH₂CH₂-NH-C(=NH)-Me |

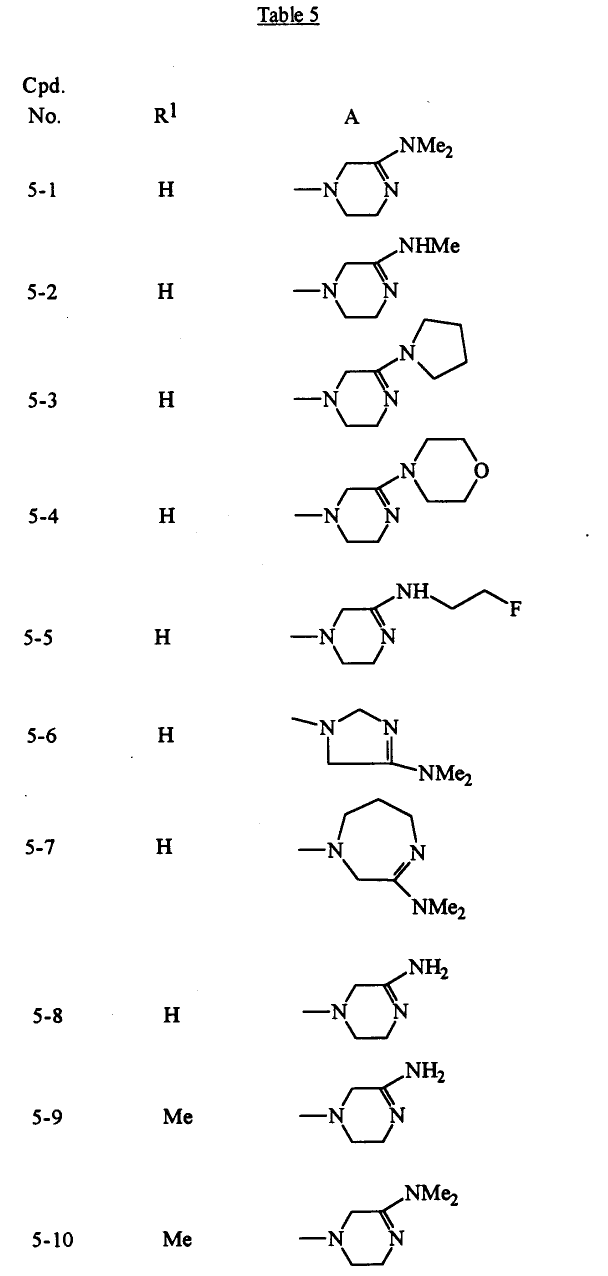

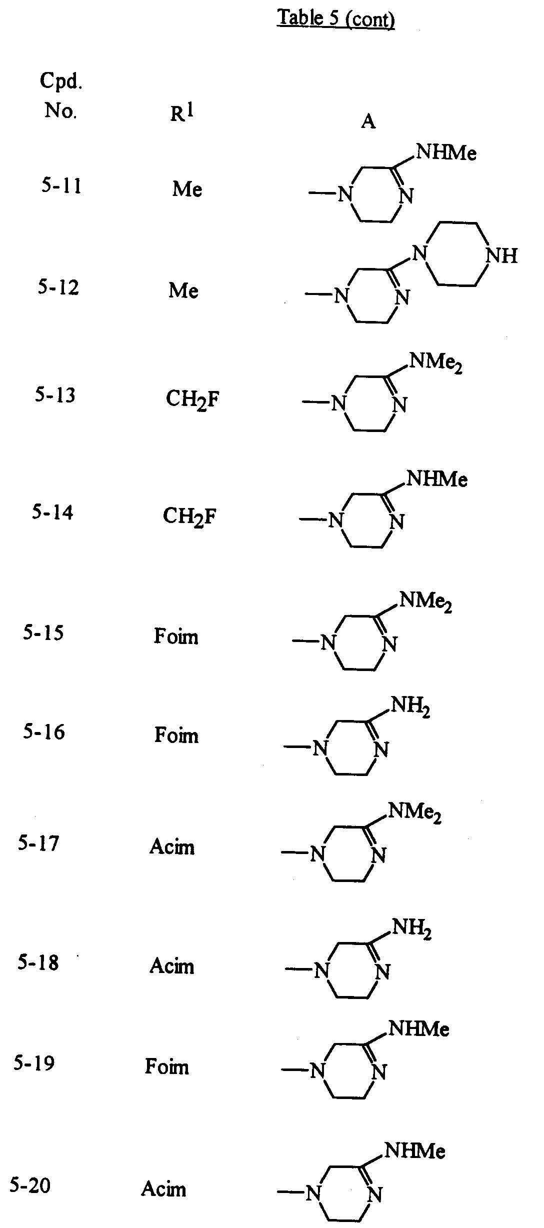

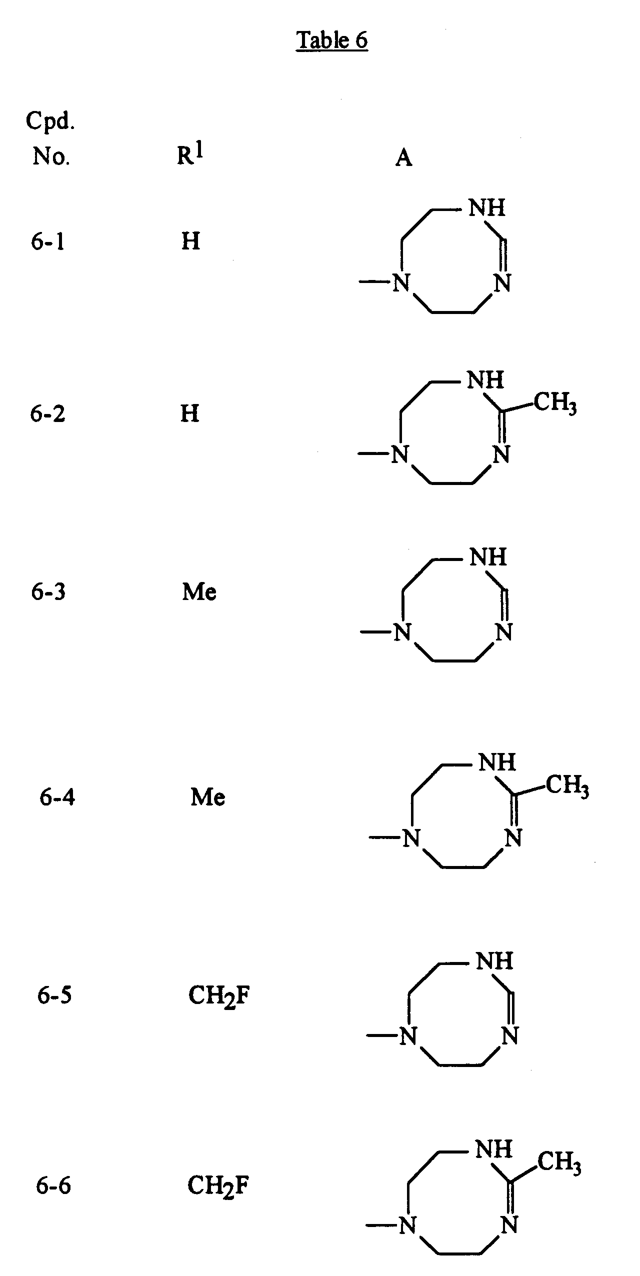

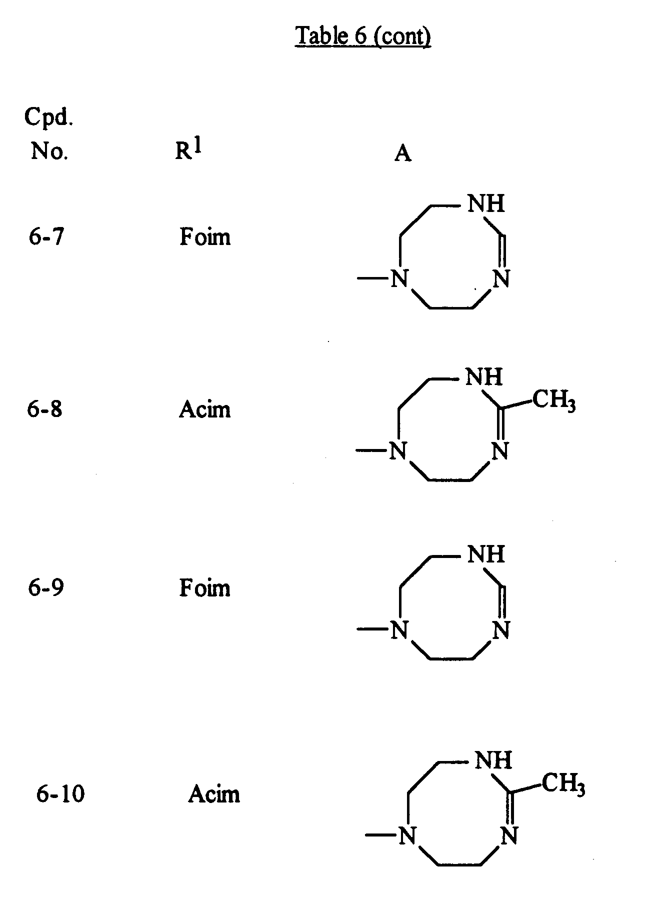

**Table 7**

| Cpd. No. | R¹ | A |
|---|---|---|
| 7-1 | H | 3-(1-Imid)-1-Pyrd |
| 7-2 | H | 3-(1,2,4-triazol-1-yl)-1-Pyrd |
| 7-3 | H | 3-(1,2,3-triazol-1-yl)-1-Pyrd |
| 7-4 | H | 4-(1-Imid)-1-Pip |
| 7-5 | H | 4-(1,2,4-triazol-1-yl)-1-Pip |
| 7-6 | H | 4-(1,2,3-triazol-1-yl)-1-Pip |
| 7-7 | Me | 3-(1-Imid)-1-Pyrd |
| 7-8 | Me | 4-(1-Imid)-1-Pip |
| 7-9 | Me | 4-(1,2,4-triazol-1-yl)-1-Pip |
| 7-10 | Me | 3-(1,2,4-triazol-1-yl)-1-Pyrd |
| 7-11 | H | 3-(1-Imid)-1-Azt |
| 7-12 | H | 3-(1,2,4-triazol-1-yl)-1-Azt |
| 7-13 | H | 3-(1-Imid)-1-Pip |
| 7-14 | H | 3-(1,2,4-triazol-1-yl)-1-Pip |

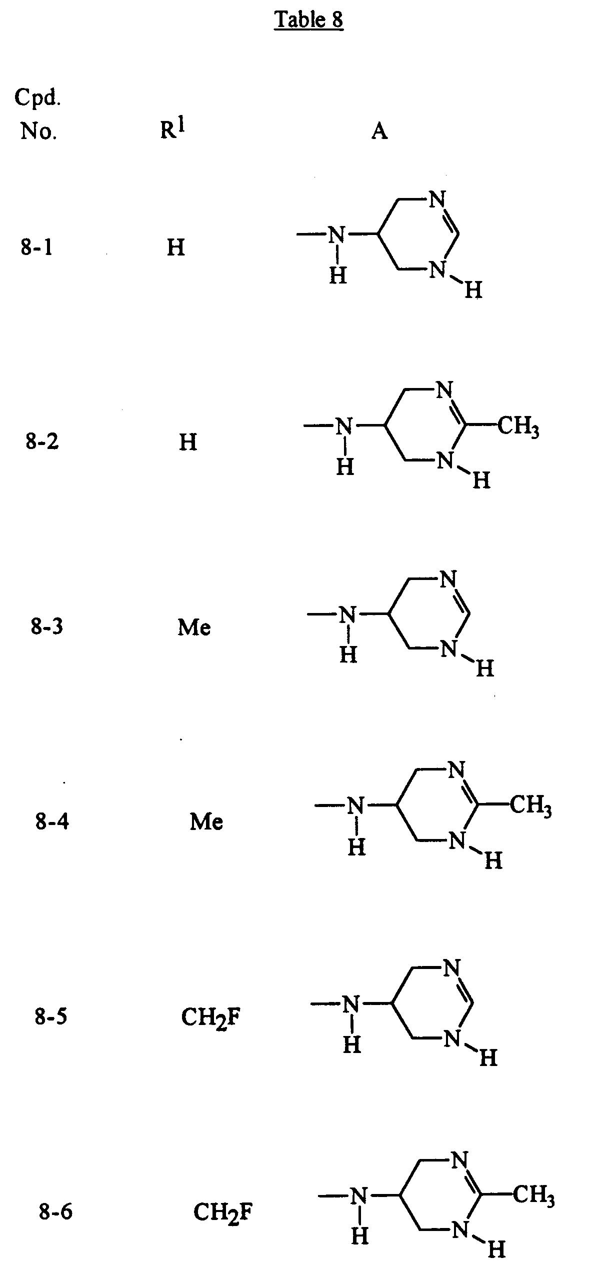

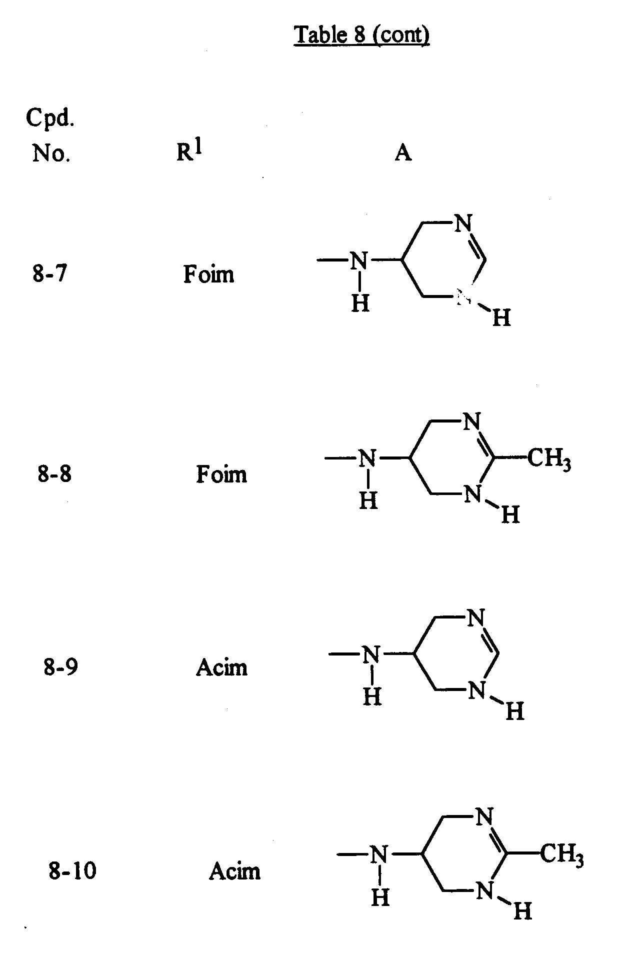

Of the compounds listed above, the following are preferred, that is to say Compounds No. 1-1, 1-2, 1-7, 1-8, 1-11, 1-12, 1-20, 1-57, 1-58, 1-60, 1-62, 1-64, 1-65, 1-66, 1-68, 1-69, 1-74, 1-75, 1-76, 1-77, 1-78, 1-79, 1-82, 1-90, 1-102, 1-103, 1-104, 1-111, 1-132, 1-166, 1-168, 1-172, 2-1, 2-2, 2-3, 2-4, 2-9, 2-10, 2-19, 2-20, 2-23, 2-37, 2-38, 2-39, 2-67, 2-99, 2-102, 3-2, 3-6, 4-1, 4-2, 5-1, 6-1, 7-1, 7-2 and 8-1, and the following are more preferred, that is to say Compounds No. 1-1, 1-2, 1-7, 1-11, 1-12, 1-20, 1-57, 1-60, 1-65, 1-66, 1-74, 1-75, 1-76, 1-77, 1-78, 1-79, 1-102, 1-103, 1-104, 1-111, 1-132, 1-168, 2-1, 2-2, 2-3, 2-4, 2-9, 2-10, 2-19, 2-20, 2-23, 2-37, 2-38, 2-39, 2-67, 2-99, 3-2, 3-6, 4-1, 5-1, 7-1 and 7-2. The most preferred specific compounds of the present invention are Compounds No.:
1-1. 2-[2-(1-Homopiperazinylcarbonyl)-1-methylpyrrolidin-4-ylthio]-6-(1-hydroxyethyl)-1-methyl-1-carbapen-2-em-3-carboxylic acid;
1-2. 2-[2-(4-Carboxymethylhomopiperazin-1-ylcarbonyl) pyrrolidin-4-ylthio]-6-(1-hydroxyethyl)-1-methyl-1-carbapen-2-em-3-carboxylic acid;
1-7. 2-{2-[4-(2-Hydroxyethyl)homopiperazin-1-yl-carbonyl]pyrrolidin-4-ylthio}-6-(1-hydroxyethyl)-1-methyl-1-carbapen-2-em-3-carboxylic acid;
1-11. 2-[2-(4-Acetimidoylhomopiperazin-1-ylcarbonyl)pyrrolidin-4-ylthio]-6-(1-hydroxyethyl)-1-methyl-1-carbapen-2-em-3-carboxylic acid;
1-12. 2-[2-(4-Formimidoylhomopiperazin-1-ylcarbonyl)pyrrolidin-4-ylthio]-6-(1-hydroxyethyl)-1-methyl-1-carbapen-2-em-3-carboxylic acid;
1-57. 2-[2-(4-Formimidoylhomopiperazin-1-ylcarbonyl)-1-methylpyrrolidin-4-ylthio]-6-(1-hydroxyethyl)-1-methyl-1-carbapen-2-em-3-carboxylic acid;
1-60. 2-[1-Methyl-(2-piperazin-1-ylcarbonyl)pyrrolidin-4-ylthio]-6-(1-hydroxyethyl)-1-methyl-1-carbapen-2-em-3-carboxylic acid;
1-65. 2-{2-[4-(2-Hydroxyethyl)piperazin-1-ylcarbonyl]pyrrolidin-4-ylthio}-6-(1-hydroxyethyl)-1-methyl-1-carbapen-2-em-3-carboxylic acid;
1-66. 2-[2-(3-Methylpiperazin-1-ylcarbonyl)pyrrolidin-4-ylthio]-6-(1-hydroxyethyl)-1-methyl-1-carbapen-2-em-3-carboxylic acid;
1-74. 2-[2-(4-Formimidoylpiperazin-1-ylcarbonyl)pyrrolidin-4-ylthio]-6-(1-hydroxyethyl)-1-methyl-1-carbapen-2-em-3-carboxylic acid;
1-75. 2-[2-(4-Acetimidoylpiperazin-1-ylcarbonyl)pyrrolidin-4-ylthio]-6-(1-hydroxyethyl)-1-methyl-1-carbapen-2-em-3-carboxylic acid;
1-76. 2-[2-(4-Formimidoylpiperazin-1-ylcarbonyl)-1-methylpyrrolidin-4-ylthio]-6-(1-hydroxyethyl)-1-methyl-1-carbapen-2-em-3-carboxylic acid;
1-77. 2-[2-(4-Acetimidoylpiperazin-1-ylcarbonyl)-1-methylpyrrolidin-4-ylthio]-6-(1-hydroxyethyl)-1-methyl-1-carbapen-2-em-3-carboxylic acid;
1-78. 2-[2-(4-Formimidoyl-3-methylpiperazin-1-yl-carbonyl)pyrrolidin-4-ylthio]-6-(1-hydroxyethyl)-1-methyl-1-carbapen-2-em-3-carboxylic acid;
1-79. 2-[2-(4-Acetimidoyl-3-methylpiperazin-1-yl-carbonyl)pyrrolidin-4-ylthio]-6-(1-hydroxyethyl)-1-methyl-1-carbapen-2-em-3-carboxylic acid;
1-102. 2-[2-(2-Methylpiperazin-1-ylcarbonyl)pyrrolidin-4-ylthio]-6-(1-hydroxyethyl)-1-methyl-1-carbapen-2-em-3-carboxylic acid;
1-103. 2-[2-(4-Formimidoyl-2-methylpiperazin-1-yl-carbonyl)pyrrolidin-4-ylthio]-6-(1-hydroxyethyl)-1-methyl-1-carbapen-2-em-3-carboxylic acid;
1-104. 2-[2-(4-Acetimidoyl-2-methylpiperazin-1-yl-carbonyl)pyrrolidin-4-ylthio]-6-(1-hydroxyethyl)-1-methyl-1-carbapen-2-em-3-carboxylic acid;
1-111. 2-[2-(3-Hydroxymethylpiperazin-1-ylcarbonyl)pyrrolidin-4-ylthio]-6-(1-hydroxyethyl)-1-methyl-1-carbapen-2-em-3-carboxylic acid;
1-168. 2-[1-Formimidoyl-2-(4-formimidoylpiperazin-1-ylcarbonyl)pyrrolidin-4-ylthio]-6-(1-hydroxyethyl)-1-methyl-1-carbapen-2-em-3-carboxylic acid;
2-1. 2-[2-(3-Acetimidoylaminopyrrolidin-1-ylcarbonyl)pyrrolidin-4-ylthio]-6-(1-hydroxyethyl)-1-methyl-1-carbapen-2-em-3-carboxylic acid;
2-2. 2-[2-(3-Formimidoylaminopyrrolidin-1-ylcarbonyl)pyrrolidin-4-ylthio]-6-(1-hydroxyethyl)-1-methyl-1-carbapen-2-em-3-carboxylic acid;
2-9. 2-[2-(3-Aminopyrrolidin-1-ylcarbonyl)pyrrolidin-4-ylthio]-6-(1-hydroxyethyl)-1-methyl-1-carbapen-2-em-3-carboxylic acid;
2-19. 2-[2-(4-Acetimidoylaminopiperidin-1-ylcarbonyl)pyrrolidin-4-ylthio]-6-(1-hydroxyethyl)-1-methyl-1-carbapen-2-em-3-carboxylic acid;
2-37. 2-[2-(3-Aminopyrrolidin-1-ylcarbonyl)-1-methylpyrrolidin-4-ylthio]-6-(1-hydroxyethyl)-1-methyl-1-carbapen-2-em-3-carboxylic acid;
2-38. 2-[2-(3-Acetimidoylaminopyrrolidin-1-ylcarbonyl)-1-methylpyrrolidin-4-ylthio]-6-(1-hydroxyethyl)-1-methyl-1-carbapen-2-em-3-carboxylic acid;
2-39. 2-[2-(3-Formimidoylaminopyrrolidin-1-ylcarbonyl)-1-methylpyrrolidin-4-ylthio]-6-(1-hydroxyethyl)-1-methyl-1-carbapen-2-em-3-carboxylic acid;
2-67. 2-[2-(4-Acetimidoylaminopiperidin-1-ylcarbonyl)-1-methylpyrrolidin-4-ylthio]-6-(1-hydroxyethyl)-1-methyl-1-carbapen-2-em-3-carboxylic acid;
3-2. 2-[2-(1-Formimidoylpyrrolidin-3-ylcarbamoyl)pyrrolidin-4-ylthio]-6-(1-hydroxyethyl)-1-methyl-1-carbapen-2-em-3-carboxylic acid;
5-1. 2-[2-(3-Dimethylamino-1,2,5,6-tetrahydropyrazin-1-ylcarbonyl)pyrrolidin-4-ylthio]-6-(1-hydroxyethyl)-1-methyl-1-carbapen-2-em-3-carboxylic acid.

The compounds of the present invention can be prepared by a variety of methods, whose general techniques are known in the art for the preparation of compounds of this type. For example, they may be prepared by reacting a compound of formula (II) : 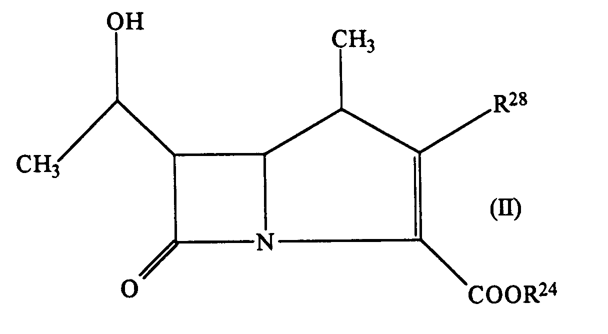 [in which R²⁴ represents a carboxy-protecting group, as exemplified below, and R²⁸ represents an alkanesulphonyloxy group, an arylsulphonyloxy group, a dialkylphosphoryloxy group, a diarylphosphoryloxy group (as exemplified hereafter in relation to R²⁵) or a group of formula -S(→O)R²⁷, where R²⁷ represents an alkyl group, a haloalkyl group, an acetamidoalkyl group, an acetamidoalkenyl group, an aryl group, or an aromatic heterocyclic group] with a compound of formula (III) : 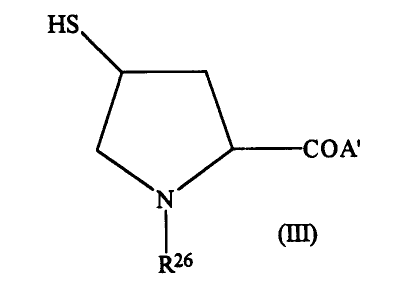 (in which R²⁶ represents any of the groups or atoms represented by R¹ or any such group or atom in which any active group is protected, and A' represents any of the groups or atoms represented by A or any such group or atom in which any active group is protected) and then, if necessary removing any protecting group.

In the above formulae, R²⁴ represents a carboxy-protecting group. Examples of such groups include: alkyl groups, such as the methyl, ethyl and t-butyl groups; aralkyl groups, such as the benzyl, benzhydryl idiphenylmethyl), 4-nitrobenzyl and 2-nitrobenzyl groups; alkenyl groups, such as the allyl, 2-chloroallyl and 2-methylallyl group; halogenated alkyl groups, such as the 2,2,2-trichloroethyl, 2,2-dibromoethyl and 2,2,2-tribromoethyl groups; and the 2-trimethylsilylethyl group.

A' and R²⁶ have the same meaning as defined for A and R¹, respectively, or, if A' or R²⁶ requires a protecting group, A' or R²⁶ includes such a protecting group. Examples of such protecting groups include: normal hydroxy-, imino-, amino- and carboxy-protecting group; and examples include: the p-nitrobenzyloxycarbonyl and p-nitrobenzyl groups. However, many such groups are well known to those skilled in the art and any group capable of protecting an active group in this type of compound may equally be used here.

R²⁷ represents:
an alkyl group, preferably having from 1 to 4 carbon atom, such as a methyl, ethyl, propyl or isopropyl group; a halogenated alkyl group, preferably having from 1 to 4 carbon atom, such as a fluoromethyl, chloromethyl, fluoroethyl, chloroethyl, fluoropropyl, difluoromethyl, difluoroethyl, dichloroethyl, trifluoromethyl or trifluoroethyl group;
an acetamidoalkyl group, such as a 2-acetamidoethyl group;
an acetamidoalkenyl group, such as a 2-acetamidovinyl group;
an aryl group, such as an optionally substituted phenyl or naphthyl group, which may optionally have from one to three of the following substituents, which may be the same or different: fluorine, chlorine and bromine atoms, methyl, ethyl, propyl, isoproyl, methoxy, ethoxy, propoxy, isopropoxy, methoxycarbonyl, ethoxycarbonyl, t-butoxycarbonyl, carbamoyl, mono- and di- alkylcarbamoyl (wherein examples of alkyl groups include, for example, the methyl, ethyl and propyl groups), nitro, hydroxy and cyano groups; or
an aromatic heterocyclic group, such as an optionally substituted pyridyl or pyrimidinyl group, which may optionally have from one to three of the following substituents, which may be the same or different: fluorine, chlorine and bromine atoms, methyl, ethyl, propyl and isopropyl groups.

In more detail, the compounds of the present invention may preferably be prepared as illustrated in the following Reaction Schemes A and B:

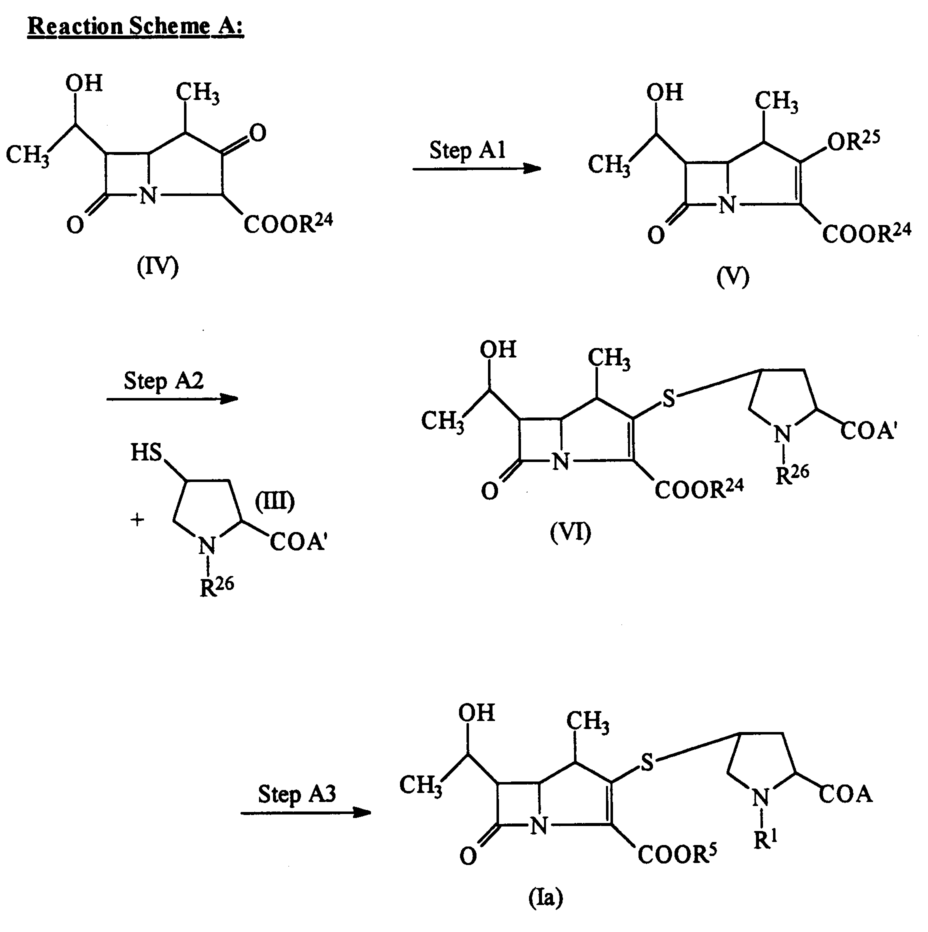

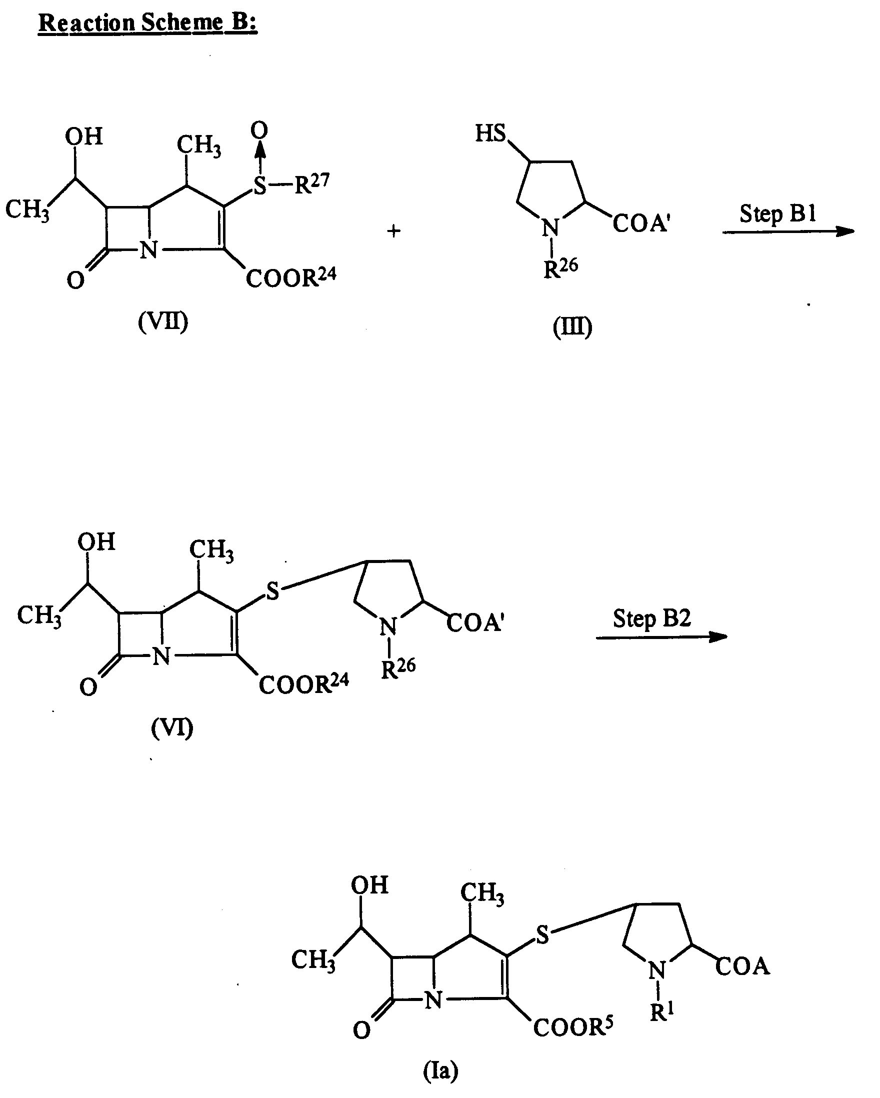

In the above formulae, A', R¹, R⁵, R²⁴, R²⁶ and R²⁷ are as defined above and R²⁵ is defined below.

R²⁵ represents a sulphonyl or phosphoryl group, for example: an alkanesulphonyl group, such as a methanesulphonyl, trifluoromethanesulphonyl, ethanesulphonyl, propanesulphonyl, isopropanesulphonyl or butanesulphonyl group; an arylsulphonyl group, such as a phenylsulphonyl, tolylsulphonyl or naphthylsulphonyl group; a dialkylphosphoryl group, such as a dimethylphosphoryl, diethylphosphoryl, dipropylphosphoryl, diisopropylphosphoryl, dibutylphosphoryl or dipentylphosphoryl group; or a diarylphosphoryl group, such as a diphenylphosphoryl or ditolylphosphoryl group.

In Step A1 of Reaction Scheme A, a compound of formula (V) is prepared by reacting a compound of formula (IV) with an alkanesulphonic acid anhydride, an arylsulphonic acid anhydride, a dialkylphosphoryl halide or a diarylphosphoryl halide in the presence of a base to produce a compound of formula (V).

Examples of reagents which may be used in this Step include: alkanesulphonic acid anhydrides, such as methanesulphonic acid anhydride, trifluoromethanesulphonic acid anhydride or ethanesulphonic acid anhydride; anhydrous arylsulphonic acid anhydrides, such as anhydrous benzenesulphonic acid anhydride or p-toluenesulphonic acid anhydride; dialkylphosphoryl halides, such as dimethylphosphoryl chloride or diethylphosphoryl chloride; and diarylphosphoryl chlorides, such as diphenylphosphoryl chloride or diphenylphosphoryl bromide. Of these, we especially prefer p-toluenesulphonic acid anhydride or diphenylphosphoryl chloride.

The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: halogenated hydrocarbons, especially halogenated aliphatic hydrocarbons, such as methylene chloride, 1,2-dichloroethane or chloroform; nitriles, such as acetonitrile; and amides, especially fatty acid amides, such as N,N-dimethylformamide or N,N-dimethylacetamide.

There is likewise no particular limitation on the nature of the base used for the reaction, provided that it has no adverse effect on other parts of the molecule, especially the β-lactam ring. Preferred examples of such bases include: organic bases, especially tertiary amines, such as triethylamine, diisopropylethylamine or 4-dimethylaminopyridine.

The reaction can take place over a wide range of temperatures, and the precise reaction temperature is not critical to the present invention. In general, we find it convenient to carry out the reaction at a relatively low temperature in order to control side reactions, for example at a temperature of from -20°C to about 40°C. The time required for the reaction may also vary widely, depending on many factors, notably the reaction temperature and the nature of the reagents and solvent employed. However, provided that the reaction is effected under the preferred conditions outlined above, a period of from 10 minutes to 5 hours will usually suffice.

The reaction mixture thus obtained can be treated, in Step A2 of the Reaction Scheme, with a compound of formula (III) in the presence of a base and without any intermediate isolation or purification of the compound of formula (V). There is no particular limitation on the nature of the base used for this step and preferred examples include: organic bases, especially tertiary amines, such as triethylamine or diisopropylethylamine; and inorganic bases, such as potassium carbonate or sodium carbonate.

The reaction can take place over a wide range of temperatures, and the precise reaction temperature is not critical to the present invention. In general, we find it convenient to carry out the reaction at a temperature of from -20°C to room temperature. The time required for the reaction may also vary widely, depending on many factors, notably the reaction temperature and the nature of the reagents and solvent employed. However, provided that the reaction is effected under the preferred conditions outlined above, a period of from 30 minutes to 108 hours will usually suffice.

After completion of the reaction, the desired compound of formula (VI) can be recovered from the reaction mixture by conventional means. An example of one such technique comprises: concentrating the reaction mixture by distilling off the solvent, preferably under reduced pressure; adding a water-immiscible organic solvent to the residue; washing the organic phase with water; and finally distilling off the organic solvent. If necessary, the desired compound thus obtained can be further purified by conventional means, for example, by recrystallisation, reprecipitation or the various chromatography techniques, notably column chromatography or preparative thin layer chromatography. Alternatively, if desired, the reaction mixture obtained in this Step can also be used as a starting material for subsequent Steps without isolation of the compound of formula (VI).

In Step A3, if necessary, the compound of formula (VI) obtained in Step A2 can be converted to a free carboxylic acid derivative by eliminating the carboxy-protecting group represented by R²⁴ according to conventional means. The reaction used to remove the protecting group represented by R²⁴ will, of course, vary depending upon the nature of the protecting group, but any reaction known in the art for the deprotection of compounds of this type may equally be used here.

For example, where the substituent on the compound of formula (VI) represented by R²⁴ is a protecting group capable of removal by reduction, for example, a halogenated alkyl, aralkyl or benzhydryl group, the reaction can preferably be carried out by contacting the compound of formula (VI) with a reducing reagent. Preferred examples of reducing reagents which may be used for this reaction include: where the carboxy-protecting is, for example, a halogenated alkyl group (such as a 2,2-dibromoethyl or 2,2,2-trichloroethyl group), zinc and acetic acid; and where it is, for example, an aralkyl group (such as a benzyl, 4-nitrobenzyl group or a benzhydryl group), hydrogen and a catalyst for use in catalytic reduction, such as palladium on charcoal or an alkali metal sulphide, such as sodium sulphide or potassium sulphide. The reaction is normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: alcohols, such as methanol or ethanol; ethers, such as tetrahydrofuran or dioxane; aliphatic acids, such as acetic acid; and mixtures of water with one or more of these organic solvents.

The reaction can take place over a wide range of temperatures, and the precise reaction temperature is not critical to the present invention. In general, we find it convenient to carry out the reaction at a temperature of from 0°C to about room temperature. The time required for the reaction may also vary widely, depending on many factors, notably the reaction temperature and the nature of the reagents and solvent employed. However, provided that the reaction is effected under the preferred conditions outlined above, a period of from 5 minutes to 12 hours will usually suffice.

After completion of the reaction, the desired compound can be recovered from the reaction mixture by conventional means. An example of one such technique comprises: filtering off insoluble materials deposited in the reaction mixture and distilling off the solvent from the filtrate.

The compound thus obtained can, if necessary, be further purified by conventional means, for example, by recrystallisation, or the various chromatography techniques, notably column chromatography or preparative thin layer chromatography.

If necessary, the carboxy group deprotected as described above can be converted to an ester group capable of hydrolyzing in vivo and the ester compound can be purified as a pharmacologically acceptable salt by conventional means.

When A' or R²⁶ contains a hydroxy-, imino-, amino-or carboxy-protecting group (for example, a p-nitrobenzyloxycarbonyl group or a p-nitrobenzyl group), the protecting group can be removed at the same time as the aforementioned removal of the carboxy-protecting group (when R²⁴ is a p-nitrobenzyl group.).

Alternatively compounds of formula (Ia) can also be prepared by Reaction Scheme B, as shown above.

The compound of formula (VII) used as a starting compound in this reaction scheme is disclosed, for example, in Japanese Patent Kokai Publication No. Sho 62-30781.

In Step B1 of this Reaction Scheme, a compound of formula (VI) is prepared by reacting the compound of formula (VII) with a mercaptan of formula (III) in the presence of a base. The reaction is also normally and preferably effected in the presence of a solvent. There is no particular restriction on the nature of the solvent to be employed, provided that it has no adverse effect on the reaction or on the reagents involved and that it can dissolve the reagents, at least to some extent. Examples of suitable solvents include: esters, such as ethyl acetate; ethers, such as tetrahydrofuran; nitriles, such as acetonitrile; amides, especially fatty acid amides, such as N,N-dimethylformamide; sulphoxides, such as dimethyl sulphoxide; water; and mixtures of any two or more of these solvents. There is also no particular limitation on the nature of the base employed for the reaction, provided that it has no adverse effect on other parts of the molecule, especially the β-lactam ring. Examples of such bases include: organic bases, especially tertiary amines, such as diisopropylethylamine, triethylamine, N-methylpiperidine or 4-dimethylaminopyridine; and inorganic bases, especially alkali metal carbonates and hydrogencarbonates, such as potassium carbonate or sodium hydrogencarbonate.

The reaction can take place over a wide range of temperatures, and the precise reaction temperature is not critical to the present invention. In general, we find it convenient to carry out the reaction at a relatively low temperature in order to reduce side reactions, for example at a temperature of from -20°C to 40°C. The time required for the reaction may also vary widely, depending on many factors, notably the reaction temperature and the nature of the reagents and solvent employed. However, provided that the reaction is effected under the preferred conditions outlined above, a period of from 15 minutes to 75 hours will usually suffice.

After completion of the reaction, the desired compound of formula (VI) of the reaction can be recovered from the reaction mixture by conventional means.

Compounds of formula (Ia) can then be prepared from this compound of formula (VI) by removing the protecting groups which may be present in the groups represented by A' and R²⁶ as described in Reaction Scheme A, if any, and by removing any other protecting group or, if necessary, by converting a deprotected carboxy group to an ester group capable of hydrolyzing in vivo.

Compounds of formula (Ia) prepared as described above can be converted to a pharmacologically acceptable salt using procedures and techniques well known in the field of β-lactam antibiotics.

The mercaptan of formula (III) used as a starting material in both of the above Reaction Schemes can be prepared following the procedure described in Japanese Patent Kokai Application No. Hei 2-28180 and No. Hei 2-3687, and Japanese Patent Application No. Hei 3-27059 and No. Hei 3-131545.

The compounds of the present invention exhibit excellent antibacterial activity with a broad antibacterial spectrum, and have the ability to inhibit the activity of β-lactamase, unlike most thienamycin-type compounds, which are liable to be metabolised in the mammalian body. The derivatives of the present invention, in addition, exhibit excellent stability against dehydropeptidase 1, which is also known to catalyze the inactivation of compounds of the thienamycin type. The derivatives of the present invention showed a strong antibacterial activity against a wide range of pathogenic bacteria including Gram-positive bacteria such as Staphylococcus aureus, and Bacillus subtilis, Gram-negative bacteria such as Escherichia coli, Shigella species, Streptococcus pneumoniae, Proteus species, Serratia species, Enterobacter species, Klebsiella species and Pseudomonas species, and anaerobic bacteria such as Bacteroides fragilis.

The antibacterial activity was determined by the agar plate dilution method, and the minimal inhibitory concentrations of the compounds of the present invention against a variety of common pathogenic bacteria were determined. The compounds of the present invention are identified by reference to the one of the following Examples which illustrates their preparation. The compounds of Examples 1, 4, 5, 6, 11, 15, 23, 37, 42, 43, 44, 45, 50, 54, 55, 61 and 66 were all found to be more active than imipenem against strains of Escherichia coli NIHJ, Klebsiella pneumoniae 846 and Pseudomonas aeruginosa 1001. The compounds also had a generally superior activity against Staphylococcus aureus 209P, similar to that of imipenem.

These results demonstrate that the compounds of the present invention have activities which are, in general, better than that of imipenem: moreover, they are, unlike imipenem, resistant to dehydropeptidase I and β-lactamase.

The carbapenem-3-carboxylic acid derivatives of the present invention, therefore, are useful as therapeutic agents for the treatment and prophylaxis of infections caused by these pathogenic bacteria. The compounds may be administered in any conventional form for this purpose, and the exact formulation used will depend on the disease to be treated, the age and condition of the patient and other factors, which are well known in the art. For example, for oral administration, the compounds may be formulated as tablets, capsules, granules, powders or syrups; and for parenteral administration, they may be formulated for intravenous injection or intramuscular injection. The dosage will vary widely, depending upon the age, body weight, symptoms and condition of the patient, as well as the mode of administration and times and routine of administration; however, for an adult human patient, a daily dosage of from about 100 mg to 3000 mg is recommended, and this may be administered as a single dose or in divided doses.

In the following Examples and Preparations, unless otherwise indicated, nuclear magnetic resonance spectrum measurements in deuterium oxide were carried out using tetramethylsilane as an external standard and those in other solvents were carried out using tetramethylsilane as an internal standard. Also, in measurement of partical sizes, the mesh sizes used are in accordance with the Tyler standard.

### EXAMPLE 1

### (1R,5S,6S)-2-[(2S,4S)-2-(4-Acetimidoylpiperazin-1-yl-carbonyl)pyrrolidin-4-ylthio]-6-[(1R)-1-hydroxyethyl]-1-methyl-1-carbapen-2-em-3-carboxylic acid

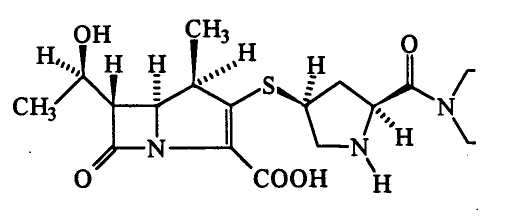

### 1(a) 4-Nitrobenzyl [(1R,5S,6S)-2-[(2S,4S)-2-[4-(N-4-nitrobenzyloxycarbonylacetimidoyl) piperazin-1-yl-carbonyl]-1-(4-nitrobenzyloxycarbonyl)pyrrolidin-4-ylthio]-6-[(1R)-1-hydroxyethyl]-1-methyl-1-carbapen-2-em-3-carboxylate

127 µℓ of diphenylphosphoryl chloride and 108 µℓ of diisopropylethylamine were added dropwise, whilst ice-cooling, to a solution of 210 mg of 4-nitrobenzyl (1R,5R,6S)-6-[(1R)-1-hydroxyethyl]-1-methyl-2-oxo-1-carbapenam-3-carboxylate in 2.6 ml of dry acetonitrile, and the resulting mixture was stirred at the same temperature for 1 hour. At the end of this time, a solution of 450 mg of (2S,4S)-4-mercapto-2-[4-(N-4-nitrobenzyloxycarbonylacetimidoyl)piperazin-1-yl-carbonyl]-1-(4-nitrobenzyloxycarbonyl) pyrrolidine trifluoromethanesulphonate (prepared as described in Preparation 1) in 2.4 ml of dry acetonitrile and 274 µℓ of diisopropylethylamine were simultaneously added dropwise to the mixture, whilst ice-cooling, and the mixture was allowed to stand overnight at the same temperature. The reaction mixture was then concentrated by evaporation under reduced pressure, and the concentrate was diluted with ethyl acetate. The diluted solution was washed, in turn, with water and with an aqueous solution of sodium chloride, after which it was dried over anhydrous magnesium sulphate. The solvent was then removed by distillation under reduced pressure, and the resulting residue was purified by column chromatography through 100 ml of silica gel 60 (Merck Art No. 9385), using a 10 : 5 : 2 by volume mixture of ethyl acetate, methylene chloride and methanol as the eluent. Those fractions containing the title compound were combined and concentrated by evaporation under reduced pressure, to give 120 mg of the title compound, as an amorphous solid.
Infrared Absorption Spectrum (KBr), νₘₐₓ cm⁻¹:
   1772, 1709, 1663, 1607, 1562, 1521, 1495, 1431, 1405, 1346, 1291, 1261, 1210.
Nuclear Magnetic Resonance Spectrum (CDCℓ₃, 270 MHz), δ ppm:
   1.27 & 1.28 (together 3H, two doublets, J = 7.33 & 6.84 Hz);
   1.37 (3H, doublet, J = 5.86 Hz);
   1.60 (1H, broad singlet);
   1.86 - 2.08 (1H, multiplet);
   2.26 & 2.31 (together 3H, two singlets);
   2.60 - 2.78 (1H, multiplet);
   3.25 - 4.28 (15H, multiplet);
   4.65 - 4.80 (1H, multiplet);
   5.04 - 5.52 (6H, multiplet);
   7.41 - 7.67 (6H, multiplet);
   8.17 - 8.24 (6H, multiplet).

### 1(b) (1R,5S,6S)-2-[(2S,4S)-2-(4-Acetimidoylpiperazin-1-ylcarbonyl)pyrrolidin-4-ylthio]-6-[(1R)-1-hydroxyethyl]-1-methyl-1-carbapen-2-em-3-carboxylic acid

114 mg of 4-nitrobenzyl (1R,5S,6S)-2-[(2S,4S)-2-[4-(N-4-nitrobenzyloxycarbonylacetimidoyl)piperazin-1-yl-carbonyl]-1-(4-nitrobenzyloxycarbonyl)pyrrolidin-4-ylthio]-6-[(1R)-1-hydroxyethyl]-1-methyl-1-carbapen-2-em-3-carboxylate [prepared as described in step (a) above] were dissolved in 6 ml of a 2 : 1 by volume mixture of tetrahydrofuran and water, and were hydrogenated by bubbling hydrogen through it at room temperature for 2 hours in the presence of 300 mg of 10% w/w palladium-on-charcoal. At the end of this time, the catalyst was filtered off and the filtrate was washed with diethyl ether. The resulting solution was concentrated by evaporation under reduced pressure, and the residue was purified by reverse phase column chromatography through 5 ml of Cosmo Sil 75C₁₈-PREP (a trade mark for a product of Nacalai Tesque), using 20% v/v aqueous methanol as the eluent. Those fractions containing the title compound were combined, concentrated by evaporation under reduced pressure, and lyophilised, to give 13 mg of the title compound as a powder.
Ultraviolet absorption spectrum (H₂O) λₘₐₓ nm:
   300.
Infrared Absorption Spectrum (KBr), νₘₐₓ cm⁻¹:
   1753, 1603, 1447, 1385, 1284, 1251.
Nuclear Magnetic Resonance Spectrum (270 MHz, D₂O, using sodium tetradeuterated trimethylsilylpropionate as an internal standard), δ ppm:
   1.22 (3H, doublet, J = 6.84 Hz);
   1.30 (3H, doublet, J = 6.35 Hz);
   1.58 - 1.68 (1H, multiplet);
   2.36 (3H, singlet);
   2.66 - 2.78 (1H, multiplet);
   3.02 - 3.18 (2H, multiplet);
   3.34 - 3.45 (2H, multiplet);
   3.65 - 3.93 (9H, multiplet);
   4.10 (1H, triplet, J = 8.06 Hz);
   4.19 - 4.30 (2H, multiplet).

### EXAMPLE 2

### (1R,5S,6S)-2-[(2S,4S)-2-(4-Acetimidoylhomopiperazin-1-ylcarbonyl)pyrrolidin-4-ylthiol-6-[(1R)-1-hydroxyethyl]-1-methyl-1-carbapen-2-em-3-carboxylic acid

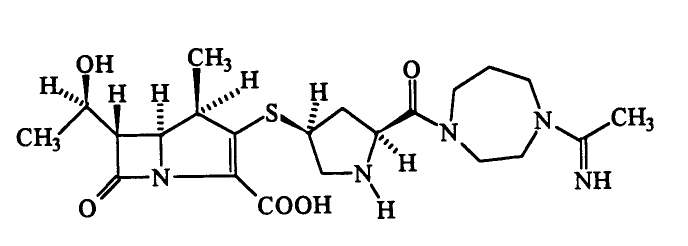

### 2(a) 4-Nitrobenzyl (1R,5S,6S)-2-[(2S,4S)-2-[4-(N-4-nitrobenzyloxycarbonylacetimidoyl)homopiperazin-1-yl-carbonyl]-1-(4-nitrobenzyloxycarbonyl) pyrrolidin-4-yl-thio]-6-[(1R)-1-hydroxyethyl]-1-methyl-1-carbapen-2-em-3-carboxylate

82 µℓ of diphenylphosphoryl chloride and 70 µℓ of diisopropylethylamine were added dropwise, whilst ice-cooling, to a solution of 136 mg of 4-nitrobenzyl (1R,5R,6S)-6-[(1R)-1-hydroxyethyl]-1-methyl-2-oxo-1-carbapenam-3-carboxylate in 1.7 ml of dry acetonitrile, and the resulting mixture was stirred at the same temperature for 1 hour. At the end of this time, a solution of 296 mg of (2S,4S)-4-mercapto-2-[4-(N-4-nitrobenzyloxycarbonylacetimidoyl)homopiperazin-1-yl-carbonyl]-1-(4-nitrobenzyloxycarbonyl) pyrrolidine trifluoromethanesulphonate (prepared as described in Preparation 2) in 1.5 ml of dry acetonitrile and 179 µℓ of diisopropylethylamine were simultaneously added dropwise to the mixture, whilst ice-cooling, and the mixture thus obtained was stirred at the same temperature for 3 hours. The reaction mixture was then concentrated by evaporation under reduced pressure, and the resulting residue was worked up and purified in the same manner as described in Example 1(a), to give 148 mg of the title compound, as a powder.
Infrared Absorption Spectrum (KBr), νₘₐₓ cm⁻¹:
   1773, 1709, 1657, 1607, 1561, 1521, 1496, 1429, 1405, 1346, 1305, 1278, 1210.
Nuclear Magnetic Resonance Spectrum (CDCℓ₃, 270 MHz), δ ppm:
   1.27 (3H, doublet, J = 7.33 Hz);
   1.37 (3H, doublet, J = 6.35 Hz);
   1.42 - 2.20 (4H, multiplet);
   1.28 & 2.39 (together 3H, two singlets);
   2.60 - 2.79 (1H, multiplet);
   3.25 - 4.35 (15H, multiplet);
   4.62 - 4.83 (1H, multiplet);
   5.05 - 5.52 (6H, multiplet);
   7.42 - 7.67 (6H, multiplet);
   8.15 - 8.24 (6H, multiplet).

### 2(b) (1R,5S,6S)-2-[(2S,4S)-2-(4-Acetimidoylhomopiperazin-1-ylcarbonyl)pyrrolidin-4-ylthio]-6-[(1R)-1-hydroxyethyl]-1-methyl-1-carbapen-2-em-3-carboxylic acid

144 mg of 4-nitrobenzyl (1R,5S,6S)-2-[(2S,4S)-2-[4-(N-4-nitrobenzyloxycarbonylacetimidoyl)homopiperazin-1-ylcarbonyl]-1-(4-nitrobenzyloxycarbonyl)pyrrolidin-4-yl-thio]-6-[(1R)-1-hydroxyethyl]-1-methyl-1-carbapen-2-em-3-carboxylate [prepared as described in step (a) above] were dissolved in 7.5 ml of a 2 : 1 by volume mixture of tetrahydrofuran and water, and were hydrogenated by bubbling hydrogen through the solution at room temperature for 1.5 hours in the presence of 375 mg of 10% w/w palladium-on-charcoal. At the end of this time, the reaction mixture was worked up and purified by the same procedure as described in Example 1(b), to give 24 mg of the title compound as a powder.
Ultraviolet absorption spectrum (H₂O) λₘₐₓ nm:
   298.
Infrared Absorption Spectrum (KBr), νₘₐₓ cm⁻¹:
   1754, 1597, 1450, 1383, 1288, 1259.
Nuclear Magnetic Resonance Spectrum (270 MHz, D₂O, using tetradeuterated sodium trimethylsilylpropionate as an internal standard), δ ppm:
   1.22 (3H, doublet, J = 6.84 Hz);
   1.30 (3H, doublet, J = 6.35 Hz);
   1.38 - 1.63 (1H, multiplet);
   1.85 - 2.04 (2H, multiplet);
   2.31 & 2.36 (together 3H, two singlets);
   2.67 - 2.82 (1H, multiplet);
   3.03 - 3.18 (2H, multiplet);
   3.34 - 3.47 (2H, multiplet);
   3.64 - 3.96 (9H, multiplet);
   4.09 (1H, triplet, J = 8.06 Hz);
   4.19 - 4.30 (2H, multiplet).

### EXAMPLE 3

### (1R,5S,6S)-2-[(2S,4S)-2-[(3S)-4-Acetimidoyl-3-methylpiperazin-1-ylcarbonyl]pyrrolidin-4-ylthio]-6-[(1R)-1-hydroxyethyl]-1-methyl-1-carbapen-2-em-3-carboxylic acid.

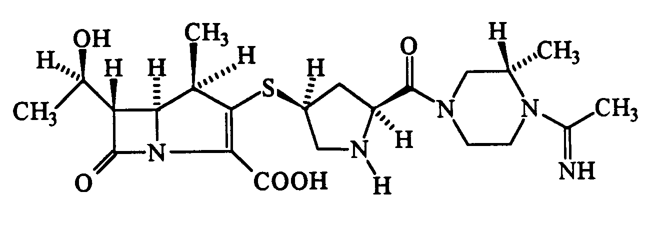

### 3(a) 4-Nitrobenzyl (1R,5S,6S)-2-[(2S,4S)-2-[(3S)-4-(N-4-nitrobenzyloxycarbonylacetimidoyl)-3-methylpiperazin-1-ylcarbonyl]-1-(4-nitrobenzyloxycarbonyl)pyrrolidin-4-ylthio]-6-[(1R)-1-hydroxyethyl]-1-methyl-1-carbapen-2-em-3-carboxylate

85 µℓ of diphenylphosphoryl chloride and 72 µℓ of diisopropylethylamine were added dropwise, whilst ice-cooling, to a solution of 140 mg of 4-nitrobenzyl (1R,5R,6S)-6-[(1R)-1-hydroxyethyl]-1-methyl-2-oxo-1-carbapenam-3-carboxylate in 1.8 ml of dry acetonitrile, and the resulting mixture was stirred at the same temperature for 1 hour. A solution of 290 mg of (2S,4S)-4-mercapto-2-[(3S)-4-(N-4-nitrobenzyloxycarbonylacetimidoyl)-3-methylpiperazin-1-ylcarbonyl]-1-(4-nitrobenzyloxycarbonyl) pyrrolidine trifluoromethanesulphonate (prepared as described in Preparation 3) in 1.6 ml of dry acetonitrile and 182 µℓ of diisopropylethylamine were simultaneously added dropwise to the mixture, whilst ice-cooling, and the mixture thus obtained was allowed to stand overnight at the same temperature. At the end of this time, the reaction mixture was worked up and purified by the same procedure as described in Example 1(a), to give 118 mg of the title compound, as a powder.

### 3(b) (1R,5S,6S)-2-[(2S,4S)-2-[(3S)-4-Acetimidoyl-3-methylpiperazin-1-ylcarbonyl]pyrrolidin-4-ylthio]-6-[(1R)-1-hydroxyethyl]-1-methyl-1-carbapen-2-em-3-carboxylic acid

85 mg of 4-nitrobenzyl (1R,5S,6S)-2-[(2S,4S)-2-[(3S)-4-(N-4-nitrobenzyloxycarbonylacetimidoyl)-3-methylpiperazin-1-ylcarbonyl]-1-(4-nitrobenzyloxycarbonyl)pyrrolidin-4-ylthio]-6-[(1R)-1-hydroxyethyl]-1-methyl-1-carbapen-2-em-3-carboxylate [prepared as described in step (a) above] were dissolved in 4.1 ml of a 2 : 1 by volume mixture of tetrahydrofuran and water, and were hydrogenated by bubbling hydrogen through the solution at room temperature for 2 hours in the presence of 210 mg of 10% w/w palladium-on-charcoal. At the end of this time, the reaction mixture was worked up and purified by the same procedure as described in Exmaple 1(b), to give 14 mg of the title compound as a powder.
Ultraviolet absorption spectrum (H₂O) λₘₐₓ nm:
   299.
Infrared Absorption Spectrum (KBr), νₘₐₓ cm⁻¹:
   1755, 1642, 1607, 1448, 1385, 1283, 1249.
Nuclear Magnetic Resonance Spectrum (270 MHz, D₂O, using tetradeuterated sodium trimethylsilylpropionate as an internal standard), δ ppm: 1.20 - 1.35 (9H, multiplet);
   1.51 - 1.72 (1H, multiplet);
   2.34 (3H, broad singlet);
   2.66 - 2.86 (1H, multiplet);
   3.02 - 3.20 (2H, multiplet);
   3.25 - 3.47 (2H, multiplet);
   3.48 - 4.38 (11H, multiplet).

### EXAMPLE 4

### (1R,5S,6S)-2-[(2S,4S)-2-(4-Formimidoylpiperazin-1-ylcarbonyl)pyrrolidin-4-ylthio]-6-[(1R)-1-hydroxyethyl]-1-methyl-1-carbapen-2-em-3-carboxylic acid

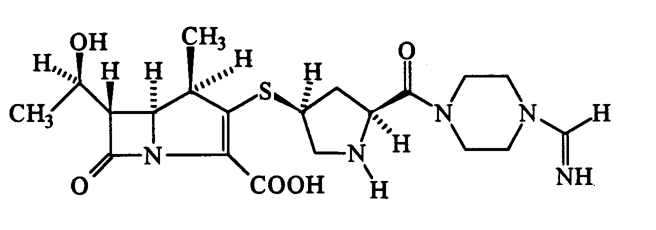

### 4(a) 4-Nitrobenzyl (1R,5S,6S)-2-{2-[4-(N-4-nitrobenzyloxycarbonylformimidoyl)piperazin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

98 µℓ of diphenylphosphoryl chloride and 84 µℓ of diisopropylethylamine were added dropwise, whilst ice-cooling, to a solution of 162 mg of 4-nitrobenzyl (1R,5R,6S)-6-[(1R)-1-hydroxyethyl]-1-methyl-2-oxo-1-carbapenam-3-carboxylate in 2 ml of dry acetonitrile, and the resulting mixture was stirred at the same temperature for 1 hour. A solution of 337 mg of (2S,4S)-4-mercapto-2-[4-(N-4-nitrobenzyloxycarbonylformimidoyl)piperazin-1-ylcarbonyl]-1-(4-nitrobenzyloxycarbonyl)pyrrolidine trifluoromethanesulphonate [prepared as described in Preparation 4(2)] in 1.8 ml of dry acetonitrile and 212 µℓ of diisopropylethylamine were then simultaneously added dropwise to the mixture, whilst ice-cooling, and the mixture was allowed to stand overnight at the same temperature. The reaction mixture was then worked up and purified by the same procedure as described in Example 1(a), to give 110 mg of the title compound, as a powder.
Infrared Absorption Spectrum (KBr), νₘₐₓ cm⁻¹:
   1774, 1709, 1660, 1604, 1520, 1345.
Nuclear Magnetic Resonance Spectrum (CDCℓ₃, 270 MHz), δ ppm:
   1.26 & 1.28 (together 3H, two doublets, J = 7.3 Hz);
   1.36 (3H, doublet, J = 6.4 Hz);
   1.80 - 2.10 (1H, multiplet);
   2.60 - 2.80 (1H, multiplet);
   3.25 - 4.30 (15H, multiplet);
   4.70 - 4.85 (1H, multiplet);
   5.15 - 5.53 (6H, multiplet);
   7.40 - 7.70 (6H, multiplet);
   8.15 - 8.26 (6H, multiplet);
   8.48 & 8.53 (together 1H, two singlets).

### 4(b) (1R,5S,6S)-2-[(2S,4S)-2-(4-Formimidoylpiperazin-1-ylcarbonyl)pyrrolidin-4-ylthio]-6-[(1R)-1-hydroxyethyl]-1-methyl-1-carbapen-2-em-3-carboxylic acid

98 mg of 4-nitrobenzyl (1R,5S,6S)-2-{2-[4-(N-4-nitrobenzyloxycarbonylformimidoyl)piperazin-1-yl-carbonyl]-1-(4-nitrobenzyloxycarbonyl) pyrrolidin-4-yl-thio}-6-[(1R)-1-hydroxyethyl]-1-methyl-1-carbapen-2-em-3-carboxylate [prepared as described in step (a) above] were dissolved in 5 ml of a 2 : 1 by volume mixture of tetrahydrofuran and water, and were hydrogenated by bubbling hydrogen through it at room temperature for 2 hours in the presence of 250 mg of 10% w/w palladiumon-charcoal. At the end of this time, the reaction mixture was worked up and purified by the same procedure as described in Example 1(b), to give 12 mg of the title compound as a powder.
Ultraviolet absorption spectrum (H₂O) λₘₐₓ nm:
   299.
Infrared Absorption Spectrum (KBr), νₘₐₓ cm⁻¹:
   1756, 1711, 1647, 1589, 1448, 1383, 1248.
Nuclear Magnetic Resonance Spectrum (270 MHz, D₂O, using tetradeuterated sodium trimethylsilylpropionate as an internal standard), δ ppm:
   1.21 (3H, doublet, J = 7.3 Hz);
   1.30 (3H, doublet, J = 6.4 Hz);
   1.63 - 1.74 (1H, multiplet);
   2.70 - 2.82 (1H, multiplet);
   3.06 - 3.26 (2H, multiplet);
   3.34 - 3.46 (2H, multiplet);
   3.55 - 3.90 (9H, multiplet);
   4.14 - 4.31 (3H, multiplet);
   7.92 (1H, singlet).

### EXAMPLE 5

### (1R,5S,6S)-2-[(2S,4S)-2-(4-Formimidoylhomopiperazin-1-ylcarbonyl)pyrrolidin-4-ylthio]-6-[(1R)-1-hydroxyethyl]-1-methyl-1-carbapen-2-em-3-carboxylic acid

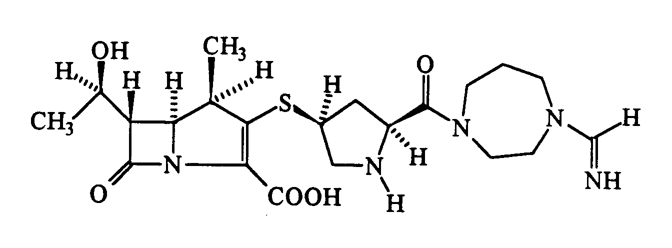

### 5(a) 4-Nitrobenzyl (1R,5S,6S)-2-[(2S,4S)-2-[4-(N-4-nitrobenzyloxycarbonylformimidoyl)homopiperazin-1-yl-carbonyl]-1-(4-nitrobenzyloxycarbonyl) pyrrolidin-4-ylthio]-6-[(1R)-1-hydroxyethyl]-1-methyl-1-carbapen-2-em-3-carboxylate

90 µℓ of diphenylphosphoryl chloride and 77 µℓ of diisopropylethylamine were added dropwise, whilst ice-cooling, to a solution of 150 mg of 4-nitrobenzyl (1R,5R,6S)-6-[(1R)-1-hydroxyethyl]-1-methyl-2-oxo-1-carbapenam-3-carboxylate in 1.9 ml of dry acetonitrile, and the resulting mixture was stirred at the same temperature for 1 hour. A solution of 316 mg of (2S,4S)-4-mercapto-2-[4-(N-4-nitrobenzyloxycarbonylformimidoyl)homopiperazin-1-ylcarbonyl]-1-(4-nitrobenzyloxycarbonyl)pyrrolidine trifluoromethanesulphonate [prepared as described in Preparation 5(1)] in 1.7 ml of dry acetonitrile and 195 µℓ of diisopropylethylamine were then simultaneously added dropwise to the mixture, whilst ice-cooling and the mixture was allowed to stand overnight at the same temperature. At the end of this time, the reaction mixture was worked up and purified by the same procedure as described in Example 1(b), to give 105 mg of the title compound, as a powder.
Infrared Absorption Spectrum (KBr), νₘₐₓ cm⁻¹:
   1773, 1709, 1657, 1601, 1521, 1346, 1209, 1161.
Nuclear Magnetic Resonance Spectrum (CDCℓ₃, 270 MHz), δ ppm:
   1.22 - 1.38 (6H, multiplet);
   1.80 - 2.18 (3H, multiplet);
   2.60 - 2.81 (1H, multiplet);
   3.22 - 4.30 (15H, multiplet);
   4.60 - 4.72 (1H, multiplet);
   5.10 - 5.53 (6H, multiplet);
   7.39 - 7.68 (6H, multiplet);
   8.14 - 8.25 (6H, multiplet);
   8.32 - 8.57 (1H, multiplet).

### 5(b) (1R,5S,6S)-2-[(2S,4S)-2-(4-Formimidoylhomopiperazin-1-ylcarbonyl)pyrrolidin-4-ylthio]-6-[(1R)-1-hydroxyethyl]-1-methyl-1-carbapen-2-em-3-carboxylic acid

89 mg of 4-nitrobenzyl (1R,5S,6S)-2-[(2S,4S)-2-[4-(N-4-nitrobenzyloxycarbonylformimidoyl)homopiperazin-1-ylcarbonyl]-1-(4-nitrobenzyloxycarbonyl)pyrrolidin-4-ylthio]-6-[(1R)-1-hydroxyethyl]-1-methyl-1-carbapen-2-em-3-carboxylate [prepared as described in step (a) above] were dissolved in 4.5 ml of a 2 : 1 by volume mixture of tetrahydrofuran and water, and were hydrogenated by bubbling hydrogen through the solution at room temperature for 2 hours in the presence of 230 mg of 10% w/w palladium-on-charcoal. At the end of this time, the reaction mixture was worked up and purified by the same procedure as described in Example 1(b), to give 13 mg of the title compound as a powder.
Ultraviolet absorption spectrum (H₂O) λₘₐₓ nm.
   298.
Infrared Absorption Spectrum (KBr), νₘₐₓ cm⁻¹:
   1754, 1706, 1638, 1588, 1383.
Nuclear Magnetic Resonance Spectrum (270 MHz, D₂O, using tetradeuterated sodium trimethylsilylpropionate as an internal standard), δ ppm:
   1.21 (3H, doublet, J = 7.3 Hz);
   1.30 (3H, doublet, J = 6.4 Hz);
   1.45 - 1.62 (1H, multiplet);
   1.90 - 2.02 (2H, multiplet);
   2.67 - 2.83 (1H, multiplet);
   3.03 - 3.20 (2H, multiplet);
   3.30 - 3.47 (2H, multiplet);
   3.60 - 3.97 (9H, multiplet);
   4.02 - 4.16 (1H, multiplet);
   4.20 - 4.31 (2H, multiplet);
   7.84, 7.93 & 7.96 (together 1H, three singlets).

### EXAMPLE 6

### (1R,5S,6S)-2-{(2S,4S)-2-[(3S)-4-Formimidoyl-3-methylpiperazin-1-ylcarbonyl]pyrrolidin-4-ylthio}-6-[(1R)-1-hydroxyethyl]-1-methyl-1-carbapen-2-em-3-carboxylic acid

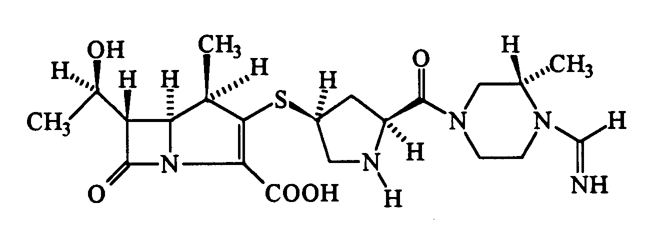

### 6(a) 4-Nitrobenzyl (1R,5S,6S)-2-{(2S,4S)-2-[(3S)-4-(N-4-nitrobenzyloxycarbonylformimidoyl)-3-methylpiperazin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

83 µℓ of diphenylphosphoryl chloride and 17 µℓ of diisopropylethylamine were added dropwise, whilst ice-cooling, to a solution of 138 mg of 4-nitrobenzyl (1R,5R,6S)-6-[(1R)-1-hydroxyethyl]-1-methyl-2-oxo-1-carbapenam-3-carboxylate in 1.7 ml of dry acetonitrile, and the resulting mixture was stirred at the same temperature for 1 hour. A solution of 290 mg of (2S,4S)-4-mercapto-2-[(3S)-4-(N-4-nitrobenzyloxycarbonylformimidoyl)-3-methylpiperazin-1-ylcarbonyl]-1-(4-nitrobenzyloxycarbonyl) pyrrolidine trifluoromethanesulphonate (prepared as described in Preparation 6) in 1.5 ml of dry acetonitrile and 179 µℓ of diisopropylethylamine were then simultaneously added dropwise to the resulting solution, whilst ice-cooling, and the mixture thus obtained was allowed to stand overnight at the same temperature. At the end of this time, the reaction mixture was worked up and purified by the same procedure as described in Example 1(a), to give 108 mg of the title compound, as a powder.

### 6(b) (1R,5S,6S)-2-{(2S,4S)-2-[(3S)-4-Formimidoyl-3-methylpiperazin-1-ylcarbonyl]pyrrolidin-4-ylthio}-6-[(1R)-1-hydroxyethyl]-1-methyl-1-carbapen-2-em-3-carboxylic acid

87 mg of 4-nitrobenzyl (1R,5S,6S)-2-{(2S,4S)-2-[(3S)-4-(N-4-nitrobenzyloxycarbonylformimidoyl)-3-methylpiperazin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 4.5 ml of a 2 : 1 by volume mixture of tetrahydrofuran and water, and were hydrogenated by bubbling hydrogen through the solution at room temperature for 2 hours in the presence of 220 mg of 10% w/w palladium-on-charcoal. The reaction mixture was then worked up and purified by the same procedure as that described in Example 1(b), to give 16 mg of the title compound, as a powder.
Ultraviolet absorption spectrum (H₂O) λₘₐₓ nm:
   299.
Infrared Absorption Spectrum (KBr), νₘₐₓ cm⁻¹:
   1754, 1705, 1645, 1591, 1447, 1386, 1258.
Nuclear Magnetic Resonance Spectrum (270 MHz, D₂O, using tetradeuterated sodium trimethylsilylpropionate as an internal standard), δ ppm:
   1.17 - 1.43 (9H, multiplet);
   1.52 - 1.73 (1H, multiplet);
   2.66 - 2.87 (1H, multiplet);
   3.01 - 3.26 (2H, multiplet);
   3.33 - 3.48 (2H, multiplet);
   3.48 - 4.43 (11H, multiplet);
   7.84 & 7.95 (together 1H, two singlets).

### EXAMPLE 7

### (1R,5S,6S)-2-[(2S,4S)-2-(2-Methyl-1-piperazinylcarbonyl)pyrrolidin-4-ylthio]-6-[(1R)-1-hydroxyethyl]-1-methyl-1-carbapen-2-em-3-carboxylic acid hydrochloride

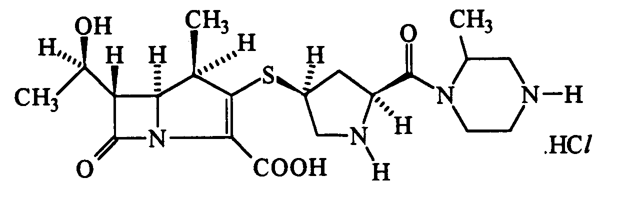

### 7(a) 4-Nitrobenzyl (1R,5S,6S)-2-{(2S,4S)-2-[2-methyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

0.67 ml of diphenylphosphoryl chloride and 0.56 ml of diisopropylethylamine were added dropwise, whilst ice-cooling, to a solution of 1.05 g of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred at the same temperature for 1 hour. A solution of 1.98 g of (2S,4S)-4-mercapto-2-[2-methyl-4-(4-nitrobenzyloxycarbonyl)-1-piperazinylcarbonyl]-1-(4-nitrobenzyloxycarbonyl)pyrrolidine (prepared as described in Preparation 7) in 10 ml of dry acetonitrile and 0.56 ml of diisopropylethylamine were then simultaneously added dropwise to the mixture at a temperature of between 2°C and 10°C, and the mixture thus obtained was stirred for 90 minutes, whilst ice-cooling; it was then allowed to stand overnight at the same temperature. At the end of this time, the reaction mixture was concentrated by evaporation under reduced pressure, and the residue was diluted with ethyl acetate. The diluted solution was washed, in turn, with water and with an aqueous solution of sodium chloride. The ethyl acetate solution was then dried over anhydrous magnesium sulphate, and the solvent was removed by distillation under reduced pressure. The resulting residue was purified by column chromatography through 250 ml of silica gel 60 (Merck Art No. 9385), using a 95 : 5 by volume mixture of ethyl acetate and methanol as the eluent, to give 2.48 g of the title compound, as an amorphous solid.
Infrared Absorption Spectrum (KBr), νₘₐₓ cm⁻¹:
   1775, 1708, 1655, 1606, 1522, 1434, 1346.
Nuclear Magnetic Resonance Spectrum (CDCℓ₃, 270 MHz), δ ppm:
   1.28 (3H, doublet, J = 7.32 Hz);
   1.36 (3H, doublet, J = 6.35 Hz);
   1.06 - 1.47 (3H, multiplet);
   1.66 - 2.06 (1H, multiplet);
   2.47 - 5.15 (17H, multiplet);
   5.20 - 5.52 (6H, multiplet);
   7.43 - 7.52 (4H, multiplet);
   7.65 (2H, doublet, J = 8.79 Hz);
   8.23 (4H, two doublets, J = 8.29 Hz);
   8.17 - 8.25 (2H, multiplet).

### 7(b) (1R,5S,6S)-2-[(2S,4S)-2-(2-Methyl-1-piperazinylcarbonyl)pyrrolidin-4-ylthio]-6-[(1R)-1-hydroxyethyl]-1-methyl-1-carbapen-2-em-3-carboxylic acid hydrochloride

2.48 g of 4-nitrobenzyl (1R,5S,6S)-2-{(2S,4S)-2-[2-methyl-4-(4-nitrobenzyloxycarbonyl)-1-piperazinylcarbonyl]-1-(4-nitrobenzyloxycarbonyl) pyrrolidin-4-yl-thio}-6-[(1R)-1-hydroxyethyl]-1-methyl-1-carbapen-2-em-3-carboxylate [prepared as described in step (a) above] were dissolved in 30 ml of a 1 : 1 by volume mixture of tetrahydrofuran and water, and then 2.9 ml of 1N aqueous hydrochloric acid were added, and the mixture was hydrogenated by bubbling hydrogen through it at room temperature for 2.5 hours in the presence of 2.5 g of 10% w/w palladium-on-charcoal. The catalyst was filtered off, and the filtrate was washed with diethyl ether. The resulting aqueous solution was concentrated by evaporation under reduced pressure, and the residue was purified by reverse phase column chromatography through 125 ml of Cosmo Sil 75C₁₈-PREP (a trade mark for a product of Nacalai Tesque), using water as the eluent. Those fractions containing the title compound were combined, concentrated by evaporation under reduced pressure and lyophilised, to give 440 mg of the title compound as a powder.
Infrared Absorption Spectrum (KBr), νₘₐₓ cm⁻¹:
   1760, 1655, 1593, 1447, 1384, 1287.
Nuclear Magnetic Resonance Spectrum (270 MHz, D₂O, using tetradeuterated sodium trimethylsilylpropionate as an internal standard), δ ppm:
   1.21 (3H, doublet, J = 7.32 Hz);
   1.28 (3H, doublet, J = 6.35 Hz);
   1.34 - 1.51 (3H, multiplet);
   1.92 - 2.07 (1H, multiplet);
   2.99 - 3.55 (9H, multiplet);
   3.69 - 4.65 (6H, multiplet);
   4.72 - 4.95 (1H, multiplet).
Ultraviolet absorption spectrum (H₂O) λₘₐₓ nm:
   296.

### EXAMPLE 8

### (1R,5S,6S)-2-[(2S,4S)-2-(3-Methyl-1-piperazinylcarbonyl]pyrrolidin-4-ylthio]-6-[(1R)-1-hydroxyethyl]-1-methyl-1-carbapen-2-em-3-carboxylic acid hydrochloride

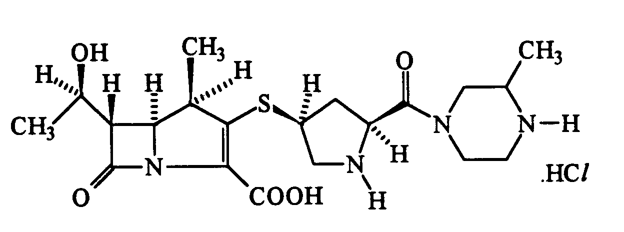

### 8(a) 4-Nitrobenzyl (1R,5S,6S)-2-{(2S,4S)-2-[3-methyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

0.60 ml of diphenylphosphoryl chloride and 0.51 ml of diisopropylethylamine were added dropwise, whilst ice-cooling, to a solution of 0.96 g of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred at the same temperature for 40 minutes. A solution of 1.55 g of (2S,4S)-4-mercapto-2-[3-methyl-4-(4-nitrobenzyloxycarbonyl)-1-piperazinylcarbonyl]-1-(4-nitrobenzyloxycarbonyl)pyrrolidine (prepared as described in Preparation 8) in 20 ml of dry acetonitrile and 0.46 ml of diisopropylethylamine were then simultaneously added dropwise to the mixture at a temperature of between 2°C and 7°C and the mixture was allowed to stand overnight at the same temperature. At the end of this time, the reaction mixture was concentrated by evaporation under reduced pressure, and the residue was diluted with ethyl acetate. The diluted solution was washed, in turn, with water and with an aqueous solution of sodium chloride, after which it was dried over anhydrous magnesium sulphate, and the solvent was removed by distillation under reduced pressure. The resulting residue was purified by column chromatography through 250 ml of silica gel 60 (Merck Art No. 9385), using a 95 : 5 by volume mixture of ethyl acetate and methanol as the eluent, to give 1.98 g of the title compound, as an amorphous solid.
Infrared Absorption Spectrum (KBr), νₘₐₓ cm⁻¹:
   1775, 1706, 1659, 1607, 1522, 1430, 1406, 1346.
Nuclear Magnetic Resonance Spectrum (CDCℓ₃, 270 MHz), δ ppm:
   1.16 - 1.38 (6H, multiplet);
   1.36 (3H, doublet, J = 6.35 Hz);
   1.80 - 2.07 (1H, multiplet);
   2.63 - 4.81 (17H, multiplet);
   5.09 - 5.52 (6H, multiplet);
   7.27 - 7.52 (4H, multiplet);
   7.65 (2H, doublet, J = 8.30 Hz);
   8.22 (4H, doublet, J = 8.79 Hz);
   8.16 - 8.25 (2H, multiplet).

### 8(b) (1R,5S,6S)-2-[(2S,4S)-2-(3-Methyl-1-piperazinylcarbonyl]pyrrolidin-4-ylthio]-6-[(1R)-1-hydroxyethyl]-1-methyl-1-carbapen-2-em-3-carboxylic acid hydrochloride

1.97 g of 4-nitrobenzyl (1R,5S,6S)-2-{(2S,4S)-2-[3-methyl-4-(4-nitrobenzyloxycarbonyl)-1-piperazinyl-carbonyl]-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] was dissolved in 40 ml of a 1 : 1 by volume mixture of tetrahydrofuran and water, and then 2.3 ml of 1N aqueous hydrochloric acid were added, and the mixture was hydrogenated by bubbling hydrogen through it at room temperature for 2 hours in the presence of 2.0 g of 10% w/w palladium-on-charcoal. The catalyst was filtered off, and the filtrate was washed with diethyl ether. It was then concentrated by evaporation under reduced pressure, and the resulting residue was purified by reverse phase column chromatography through 100 ml of Cosmo Sil 75C₁₈-PREP (a trade mark for a product of Nacalai Tesque), using water as the eluent. Those fractions containing the title compound were combined, concentrated by evaporation under reduced pressure and lyophilised, to give 320 mg of the title compound as a powder.
Infrared Absorption Spectrum (KBr), νₘₐₓ cm⁻¹:
   1760, 1660, 1594, 1453, 1386, 1263.
Nuclear Magnetic Resonance Spectrum (270 MHz, D₂O, using tetradeuterated sodium trimethylsilylpropionate as an internal standard), δ ppm: 1.21 (3H, doublet, J = 7.33 Hz);
   1.28 (3H, doublet, J = 6.35 Hz);
   1.36 - 1.40 (3H, multiplet);
   1.97 - 2.07 (1H, multiplet);
   3.01 - 3.67 (9H, multiplet);
   3.77 - 4.53 (6H, multiplet);
   4.72 - 4.95 (1H, multiplet).
Ultraviolet absorption spectrum (H₂O) λₘₐₓ nm:
   296.

### EXAMPLE 9

### (1R,5S,6S)-2-{(2S,4S)-2-[(2S)-2-Methyl-1-piperazinylcarbonyl]pyrrolidin-4-ylthio}-6-[(1R)-1-hydroxyethyl]-1-methyl-1-carbapen-2-em-3-carboxylic acid hydrochloride

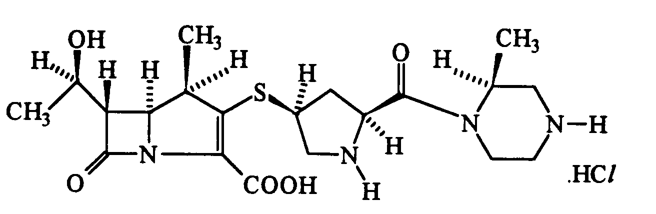

### 9(a) 4-Nitrobenzyl (1R,5S,6S)-2-{(2S,4S)-2-[(2S)-2-methyl-4-(4-nitrobenzyloxycarbonyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

57 µℓ of diphenylphosphoryl chloride and 48 µℓ of diisopropylethylamine were added dropwise, whilst ice-cooling, to a solution of 89 mg of 4-nitrobenzyl (1R,5R,6S)-6-[(1R)-1-hydroxyethyl]-1-methyl-2-oxo-1-carbapenam-3-carboxylate in 1.0 ml of dry acetonitrile, and the resulting mixture was stirred at the same temperature for 1 hour. A solution of 168 mg of (2S,4S)-4-mercapto-2-[(2S)-2-methyl-4-(4-nitrobenzyloxycarbonyl)-1-piperazinylcarbonyl]-1-(4-nitrobenzyloxycarbonyl)pyrrolidine (prepared as described in Preparation 9) in 0.5 ml of dry acetonitrile and 48 µℓ of diisopropylethylamine were simultaneously added dropwise to the mixture, whilst ice-cooling. The mixture was then stirred at the same temperature for 1 hour, after which it was allowed to stand overnight at the same temperature. At the end of this time, the reaction mixture was worked up and purified by the same procedure as described in Example 7(a) to give 205 mg of the title compound, as a powder.

### 9(b) (1R,5S,6S)-2-{(2S,4S)-2-[(2S)-2-Methyl-1-piperazinylcarbonyl]pyrrolidin-4-ylthio}-6-[(1R)-1-hydroxyethyl]-1-methyl-1-carbapen-2-em-3-carboxylic acid hydrochloride

205 mg of 4-nitrobenzyl (1R,5S,6S)-2-{(2S,4S)-2**-**[(2S)-2-methyl-4-(4-nitrobenzyloxycarbonyl)-1-piperazinylcarbonyl] -1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 3 ml of a 1 : 1 by volume mixture of tetrahydrofuran and water, after which 240 µℓ of 1N aqueous hydrochloric acid were added, and the mixture was hydrogenated by bubbling hydrogen through it at room temperature for 2 hours in the presence of 250 mg of 10% w/w palladium-on-charcoal. The reaction mixture was then worked up and purified by the same procedure as described in Example 7(b), to give 30 mg of the title compound as a powder.
Ultraviolet absorption spectrum (H₂O) λₘₐₓ nm:
   296.
Infrared Absorption Spectrum (KBr), νₘₐₓ cm⁻¹:
   1759, 1653, 1591, 1447, 1384, 1287.
Nuclear Magnetic Resonance Spectrum (270 MHz, D₂O, using tetradeuterated sodium trimethylsilylpropionate as an internal standard), δ ppm:
   1.21 (3H, doublet, J = 6.83 Hz);
   1.28 (3H, doublet, J = 6.35 Hz);
   1.35 & 1.50 (together 3H, two doublets, J = 6.83 Hz);
   1.88 - 2.11 (1H, multiplet);
   2.97 - 3.60 (8H, multiplet);
   3.47 (1H, doublet of doublets, J = 6.35 & 2.93 Hz);
   3.63 - 3.90 (2H, multiplet);
   4.03 - 4.13 (1H, multiplet);
   4.20 - 4.29 (2H, multiplet);
   4.33 - 4.98 (2H, multiplet).

### EXAMPLE 10

### (1R,5S,6S)-2-{(2S,4S)-2-[(2R)-2-Methyl-1-piperazinylcarbonyl]pyrrolidin-4-ylthio}-6-[(1R)-1-hydroxyethyl]-1-methyl-1-carbapen-2-em-3-carboxylic acid hydrochloride

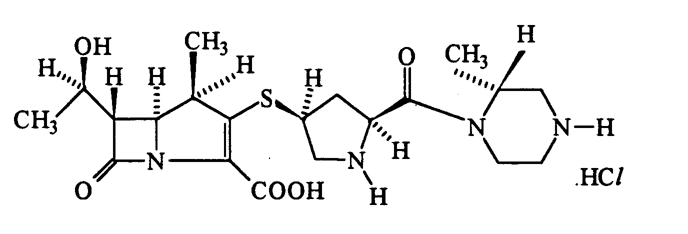

### 10(a) 4-Nitrobenzyl (1R,5S,6S)-2-{(2S,4S)-2-[(2R)-2-methyl-4-(4-nitrobenzyloxycarbonyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

37 µℓ of diphenylphosphoryl chloride and 31 µℓ of diisopropylethylamine were added dropwise, whilst ice-cooling, to a solution of 58 mg of 4-nitrobenzyl (1R,5R,6S)-6-[(1R)-1-hydroxyethyl]-1-methyl-2-oxo-1-carbapenam-3-carboxylate in 1.0 ml of dry acetonitrile, and the resulting mixture was stirred at the same temperature for 1 hour. A solution of 109 mg of (2S,4S)-4-mercapto-2-[(2R)-2-methyl-4-(4-nitrobenzyloxycarbonyl)-1-piperazinylcarbonyl]-1-(4-nitrobenzyloxycarbonyl)pyrrolidine (prepared as described in Preparation 10) in 0.5 ml of dry acetonitrile and 31 µℓ of diisopropylethylamine were then simultaneously added dropwise to the mixture at a temperature of 5°C to 10°C and the mixture thus obtained was stirred for 30 minutes, whilst ice-cooling, after which it was allowed to stand overnight at the same temperature. At the end of this time, the reaction mixture was worked up and purified by the same procedure as described in Example 7(a), to give 132 mg of the title compound, as a powder.

### 10(b) (1R,5S,6S)-2-{(2S,4S)-2-[(2R)-2-Methyl-1-piperazinylcarbonyl]pyrrolidin-4-ylthio}-6-[(1R)-1-hydroxyethyl]-1-methyl-1-carbapen-2-em-3-carboxylic acid hydrochloride

132 mg of 4-nitrobenzyl (1R,5S,6S)-2-{(2S,4S)-2-[(2R)-2-methyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] was dissolved in 3 ml of a 1 : 1 by volume mixture of tetrahydrofuran and water, after which 160 µℓ of 1N aqueous hydrochloric acid were added, and the mixture was hydrogenated by bubbling hydrogen through it at room temperature for 2 hours in the presence of 150 mg of 10% w/w palladium-on-charcoal. At the end of this time, the reaction mixture was worked up and purified by the same procedure as described in Example 7(b), to give 23 mg of the title compound as a powder.
Ultraviolet absorption spectrum (H₂O) λₘₐₓ nm:
   296.

### EXAMPLE 11

### (1R,5S,6S)-2-{(2S,4S)-2-[(3S)-3-Methyl-1-piperazinylcarbonyl]pyrrolidin-4-ylthio}-6-[(1R)-1-hydroxyethyl]-1-methyl-1-carbapen-2-em-3-carboxylic acid hydrochloride

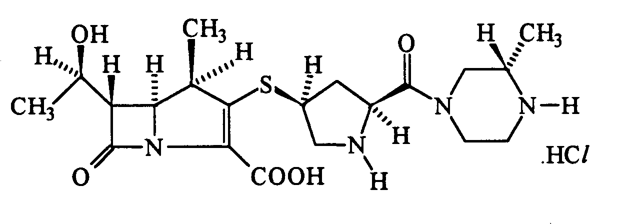

### 11(a) 4-Nitrobenzyl (1R,5S,6S)-2-{(2S,4S)-2-[(3S)-3-methyl-4-(4-nitrobenzyloxycarbonyl)-1-piperazinyl-carbonyl]-1-(4-nitrobenzyloxycarbonyl)pyrrolidin-4-ylthio]-6-[(1R)-1-hydroxyethyl]-1-methyl-1-carbapen-2-em-3-carboxylate

50 µℓ of diphenylphosphoryl chloride and 43 µℓ of diisopropylethylamine were added dropwise, whilst ice-cooling, to a solution of 80 mg of 4-nitrobenzyl (1R,5R,6S)-6-[(1R)-1-hydroxyethyl]-1-methyl-2-oxo-1-carbapenam-3-carboxylate in 1.0 ml of dry acetonitrile, and the resulting mixture was stirred at the same temperature for 50 minutes. A solution of 130 mg of (2S,4S)-4-mercapto-2-[(3S)-3-methyl-4-(4-nitrobenzyloxycarbonyl)-1-piperazinylcarbonyl]-1-(4-nitrobenzyloxycarbonyl)pyrrolidine (prepared as described in Preparation 11) in 1.5 ml of dry acetonitrile and 38 µℓ of diisopropylethylamine were then simultaneously added dropwise to the mixture, whilst ice-cooling, and the mixture was stirred at the same temperature for 2 hours, after which it was allowed to stand overnight at the same temperature. At the end of this time, the reaction mixture was worked up and purified by the same procedure as described in Example 8(b), to give 170 mg of the title compound, as a powder.

### 11(b) (1R,5S,6S)-2-{(2S,4S)-2-[(3S)-3-Methyl-1-piperazinylcarbonyl]pyrrolidin-4-ylthio}-6-[(1R)-1-hydroxyethyl]-1-methyl-1-carbapen-2-em-3-carboxylic acid hydrochloride

170 mg of 4-nitrobenzyl (1R,5S,6S)-2-{(2S,4S)-2-[(3S)-3-methyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 3.5 ml of a 1 : 1 by volume mixture of tetrahydrofuran and water, after which 0.19 ml of 1N aqueous hydrochloric acid was added, and the mixture was hydrogenated by bubbling hydrogen through it at room temperature for 2.5 hours in the presence of 170 mg of 10% w/w palladium-on-charcoal. At the end of this time, the reaction mixture was worked up and purified by the same procedure as described in Example 8(b), to give 25.0 mg of the title compound as a powder.
Ultraviolet absorption spectrum (H₂O) λₘₐₓ nm:
   296.
Infrared Absorption Spectrum (KBr), νₘₐₓ cm⁻¹:
   1761, 1660, 1594, 1453, 1385, 1262.
Nuclear Magnetic Resonance Spectrum (270 MHz, D₂O, using tetradeuterated sodium trimethylsilylpropionate as an internal standard), δ ppm:
   1.20 (3H, doublet, J = 7.32 Hz);
   1.28 (3H, doublet, J = 6.35 Hz);
   1.36 - 1.40 (3H, multiplet);
   1.99 - 2.07 (1H, multiplet);
   3.04 - 3.63 (9H, multiplet);
   3.76 - 4.50 (6H, multiplet);
   4.72 - 4.93 (1H, multiplet).

### EXAMPLE 12

### (1R,5S,6S)-2-{(2S,4S)-2-[(3R)-3-Methyl-1-piperazinylcarbonyl]pyrrolidin-4-ylthio}-6-[(1R)-1-hydroxyethyl]-1-methyl-1-carbapen-2-em-3-carboxylic acid hydrochloride

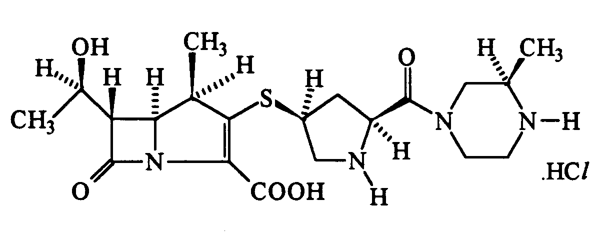

### 12(a) 4-Nitrobenzyl (1R,5S,6S)-2-{(2S,4S)-2-[(3R)-3-methyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

55 µℓ of diphenylphosphoryl chloride and 47 µℓ of diisopropylethylamine were added dropwise, whilst ice-cooling, to a solution of 87 mg of 4-nitrobenzyl (1R,5R,6S)-6-[(1R)-1-hydroxyethyl]-1-methyl-2-oxo-1-carbapenam-3-carboxylate in 1.0 ml of dry acetonitrile, and the resulting mixture was stirred at the same temperature for 1 hour. A solution of 145 mg of (2S,4S)-4-mercapto-2-[(3R)-3-methyl-4-(4-nitrobenzyloxycarbonyl)-1-piperazinylcarbonyl]-1-(4-nitrobenzyloxycarbonyl)pyrrolidine (prepared as described in Preparation 12) in 2.0 ml of dry acetonitrile and 42 µℓ of diisopropylethylamine were then simultaneously added dropwise to the mixture, whilst ice-cooling, and the mixture was stirred at the same temperature for 1 hour, after which it was allowed to stand overnight at the same temperature. At the end of this time, the reaction mixture was worked up and purified by the same procedure as described in Example 8(a), to give 175 mg of the title compound, as an amorphous solid.

### 12(b) (1R,5S,6S)-2-{(2S,4S)-2-[(3R)-3-Methyl-1-piperazinylcarbonyl]pyrrolidin-4-ylthio}-6-[(1R)-1-hydroxyethyl]-1-methyl-1-carbapen-2-em-3-carboxylic acid hydrochloride

175 mg of 4-nitrobenzyl (1R,5S,6S)-2-{(2S,4S)-2-[(3R)-3-methyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 35 ml of a 1 : 1 by volume mixture of tetrahydrofuran and water, after which 0.20 ml of 1N aqueous hydrochloric acid was added, and the mixture was hydrogenated by bubbling hydrogen through it at room temperature for 2 hours in the presence of 175 mg of 10% w/w palladium-on-charcoal. At the end of this time, the reaction mixture was worked up and purified by the same procedure as described in Example 8(b), to give 28.0 mg of the title compound as a powder.
Ultraviolet absorption spectrum (H₂O) λₘₐₓ nm:
   296.

### EXAMPLE 13

### (1R,5S,6S)-2-[(2S,4S)-2-(trans-2,5-Dimethyl-1-piperazinylcarbonyl)pyrrolidin-4-ylthio]-6-[(1R)-1-hydroxyethyl]-1-methyl-1-carbapen-2-em-3-carboxylic acid hydrochloride

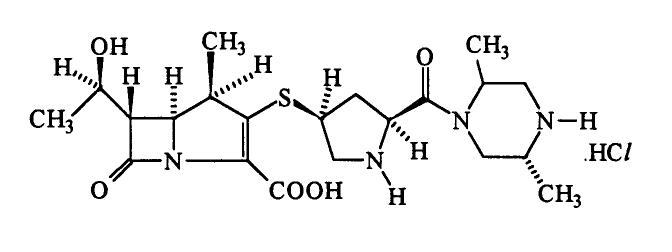

### 13(a) 4-Nitrobenzyl (1R,5S,6S)-2-{(2S,4S)-2-[trans-2,5-dimethyl-4-(4-nitrobenzyloxycarbonyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

150 µℓ of diphenylphosphoryl chloride and 127 µℓ of diisopropylethylamine were added dropwise, whilst ice-cooling, to a solution of 248 mg of (1R,5R,6S)-6-[(1R)-1-hydroxyethyl]-1-methyl-2-oxo-1-carbapenam-3-carboxylic acid in 3 ml of dry acetonitrile, and the resulting mixture was stirred at the same temperature for 1 hour. A solution of 533 mg of (2S,4S)-4-mercapto-[2-(trans-2,5-dimethyl-4-(4-nitrobenzyloxycarbonyl)-1-piperazinylcarbonyl] -1-(4-nitrobenzyloxycarbonyl)pyrrolidine (prepared as described in Preparation 13) in 2.5 ml of dry acetonitrile and 132 µℓ of diisopropylethylamine were then simultaneously added dropwise to the mixture at a temperature of between 0 and 5°C, and the mixture was stirred for 2 hours, whilst ice-cooling, after which it was allowed to stand overnight in a refrigerator. At the end of this time, the reaction mixture was worked up and purified by the same procedure as described in Example 7(a), to give 281 mg of the title compound, as a powder.

### 13(b) (1R,5S,6S)-2-[(2S,4S)-2-(trans-2,5-Dimethyl-1-piperazinylcarbonyl)pyrrolidin-4-ylthio]-6-[(1R)-1-hydroxyethyl]-1-methyl-1-carbapen-2-em-3-carboxylic acid hydrochloride

281 mg of 4-nitrobenzyl (1R,5S,6S)-2-{(2S,4S)-2-[trans-2,5-dimethyl-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 6 ml of a 1 : 1 by volume mixture of tetrahydrofuran and water, after which 328 µℓ of 1N aqueous hydrochloric acid were added, and the mixture was hydrogenated by bubbling hydrogen through it at room temperature for 2 hours in the presence of 0.3 g of 10% w/w palladium-on-charcoal. At the end of this time, the reaction mixture was worked up and purified by the same procedure as described in Example 7(b), to give 29 mg of the title compound as a powder.
Ultraviolet absorption spectrum (H₂O) λₘₐₓ nm:
   296.5.
Infrared Absorption Spectrum (KBr), νₘₐₓ cm⁻¹:
   1762, 1656, 1592, 1455, 1386, 1139.
Nuclear Magnetic Resonance Spectrum (270 MHz, D₂O, using tetradeuterated sodium trimethylsilylpropionate as an internal standard), δ ppm:
   1.20 (3H, doublet, J = 7.32 Hz);
   1.28 (3H, doublet, J = 6.35 Hz);
   1.35 (3H, doublet, J = 7.32 Hz);
   1.41 & 1.46 (together 3H, two doublets, J = 6.84 Hz);
   1.92 - 2.08 (1H, multiplet);
   2.96 - 3.65 (6H, multiplet);
   3.47 (1H, doublet of doublets, J = 6.35 & 2.93 Hz);
   3.76 - 3.90 (3H, multiplet);
   4.00 - 4.15 (1H, multiplet);
   4.20 - 4.41 (3H, multiplet);
   4.75 - 4.88 (1H, multiplet).

### EXAMPLE 14

### (1R,5S,6S)-2-[(2S,4S)-2-(cis-3,5-Dimethyl-1-piperazinylcarbonyl)pyrrolidin-4-ylthio]-6-[(1R)-1-hydroxyethyl]-1-methyl-1-carbapen-2-em-3-carboxylic acid hydrochloride

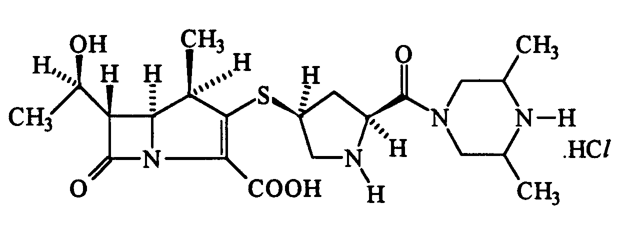

### 14(a) 4-Nitrobenzyl (1R,5S,6S)-2-[(2S,4S)-2-(cis-3,5-dimethyl-1-piperazinylcarbonyl)-1-(4-nitrobenzyloxycarbonyl)pyrrolidin-4-ylthio]-6-[(1R)-1-hydroxyethyl]-1-methyl-1-carbapen-2-em-3-carboxylate

92 µℓ of diphenylphosphoryl chloride and 78 µℓ of diisopropylethylamine were added dropwise, whilst ice-cooling, to a solution of 152 mg of 4-nitrobenzyl (1R,5R,6S)-6-[(1R)-1-hydroxyethyl]-1-methyl-2-oxo-1-carbapenam-3-carboxylate in 2 ml of dry acetonitrile, and the resulting mixture was stirred at the same temperature for 1 hour. At the end of this time, 176 µℓ of diisopropylethylamine and a solution of 230 mg of (2S,4S)-4-mercapto-2-(cis-3,5-dimethyl-1-piperazinylcarbonyl)-1-(4-nitrobenzyloxycarbonyl)pyrrolidine (prepared as described in Preparation 14) in 1.8 ml of dry acetonitrile were simultaneously added dropwise to the mixture, whilst ice-cooling, and the mixture was stirred at the same temperature for 5 hours, after which it was allowed to stand overnight in a refrigerator. The reaction mixture was then freed from the solvent by distillation under reduced pressure, and the resulting residue was mixed with an aqueous solution of sodium hydrogencarbonate and then extracted with ethyl acetate. The extract was dried over anhydrous magnesium sulphate and the solvent was removed by distillation under reduced pressure. The resulting residue was worked up and purified by the same procedure as described in Example 8(a), to give 224 mg of the title compound, as a powder.

### 14(b) (1R,5S,6S)-2-[(2S,4S)-2-(cis-3,5-Dimethyl-1-piperazinylcarbonyl)pyrrolidin-4-ylthio]-6-[(1R)-1-hydroxyethyl]-1-methyl-1-carbapen-2-em-3-carboxylic acid hydrochloride

The whole of the 4-nitrobenzyl (1R,5S,6S)-2-[(2S,4S)-2-(cis-3,5-dimethyl-1-piperazinylcarbonyl]-1-(4-nitrobenzyloxycarbonyl)pyrrolidin-4-ylthio]-6-[(1R)-1-hydroxyethyl]-1-methyl-1-carbapen-2-em-3-carboxylate prepared as described in step (a) above was dissolved in 4 ml of a 1 : 1 by volume mixture of tetrahydrofuran and water, after which 261 µℓ of 1N aqueous hydrochloric acid were added, and the mixture was hydrogenated by bubbling hydrogen through it at room temperature for 2 hours in the presence of 0.2 g of 10% w/w palladium-on-charcoal. At the end of this time, the reaction mixture was worked up and purified by the same procedure as described in Example 8(b), to give 27 mg of the title compound as a powder.
Ultraviolet absorption spectrum (H₂O) λₘₐₓ nm:
   296.5.
Infrared Absorption Spectrum (KBr), νₘₐₓ cm⁻¹:
   1761, 1661, 1599, 1459, 1386, 1268.
Nuclear Magnetic Resonance Spectrum (270 MHz, D₂O, using tetradeuterated sodium trimethylsilylpropionate as an internal standard), δ ppm:
   1.20 (3H, doublet, J = 7.32 Hz);
   1.28 (3H, doublet, J = 6.35 Hz);
   1.35 - 1.39 (6H, multiplet);
   1.96 - 2.06 (1H, multiplet);
   2.72 - 3.53 (9H, multiplet);
   3.75 - 4.29 (5H, multiplet);
   4.53 - 4.94 (1H, multiplet).

### EXAMPLE 15

### (1R,5S,6S)-2-{(2S,4S)-2-[4-(2-Hydroxyethyl)-1-homopiperazinylcarbonyl] pyrrolidin-4-ylthio}-6-[(1R)-1-hydroxyethyl]-1-methyl-1-carbapen-2-em-3-carboxylic acid hydrochloride

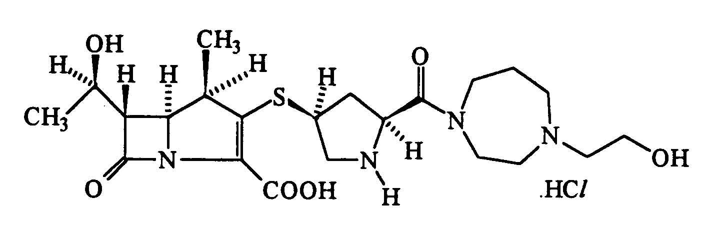

### 15(a) 4-Nitrobenzyl (1R,5S,6S)-2-{(2S,4S)-2-[4-(2-4'-nitrobenzyloxycarbonyloxyethyl)-1-homopiperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

460 µℓ of diphenylphosphoryl chloride and 390 µℓ of diisopropylethylamine were added dropwise, whilst ice-cooling, to a solution of 761 mg of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred at the same temperature for 1 hour. At the end of this time, 780 µℓ of diisopropylethylamine and a solution of 2.2 g of (2S,4S)-4-mercapto-2-{4-[2-(4-nitrobenzyloxycarbonyl)oxyethyl]-1-homopiperazinylcarbonyl}-1-(4-nitrobenzyloxycarbonyl)pyrrolidine trifluoromethanesulphonate (prepared as described in Preparation 15) in 5 ml of dry acetonitrile were simultaneously added dropwise to the mixture, whilst ice-cooling, and the mixture was stirred overnight at the same temperature. The solvent was then removed by distillation under reduced pressure, and the resulting residue was mixed with an aqueous solution of sodium hydrogencarbonate. The mixture was then extracted with ethyl acetate. The extract was dried over anhydrous sodium sulphate and the solvent was removed by distillation under reduced pressure. The resulting residue was purified by column chromatography through 50 g of silica gel (a product of Merck, 230 to 400 mesh), using a 9 : 1 by volume mixture of ethyl acetate and methanol as the eluent. Those fractions containing the title compound were combined and concentrated by evaporation under reduced pressure, to give 857 mg of the title compound, as a powder.
Infrared Absorption Spectrum (KBr), νₘₐₓ cm⁻¹:
   1768, 1750, 1710, 1649, 1522, 1347, 1260.
Nuclear Magnetic Resonance Spectrum (CDCℓ₃, 270 MHz), δ ppm:
   1.27 (3H, doublet, J = 7.3 Hz);
   1.36 (3H, doublet, J = 6.0 Hz);
   1.80 - 2.05 (3H, multiplet);
   2.40 - 3.00 (7H, multiplet);
   3.23 - 3.78 (7H, multiplet);
   4.00 - 4.29 (5H, multiplet);
   4.61 - 4.77 (1H, multiplet);
   5.03 - 5.53 (6H, multiplet);
   7.42 - 7.67 (6H, multiplet);
   8.16 - 8.25 (6H, multiplet).

### 15(b) (1R,5S,6S)-2-{(2S,4S)-2-[4-(2-Hydroxyethyl)-1-homopiperazinylcarbonyl]pyrrolidin-4-ylthio}-6-[(1R)-1-hydroxyethyl]-1-methyl-1-carbapen-2-em-3-carboxylic acid hydrochloride

350 mg of 4-nitrobenzyl (1R,5S,6S)-2-{(2S,4S)-2-[4-(2-4'-nitrobenzyloxycarbonyloxyethyl)-1-homopiperazinylcarbonyl] -1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 20 ml of a 3 : 2 by volume mixture of tetrahydrofuran and water, after which 0.33 ml of 1N aqueous hydrochloric acid was added, and the mixture was hydrogenated by bubbling hydrogen through it at room temperature for 2 hours in the presence of 0.5 g of 10% w/w palladium-on-charcoal. At the end of this time, the catalyst was filtered off, and the filtrate was extracted with diethyl ether. The remaining aqueous layer was concentrated by evaporation under reduced pressure, and the resulting residue was purified by Lobar column chromatography (a product of Merck, LiChroprep RP-8, size B), using water as the eluent. Those fractions containing the title compound were combined, concentrated by evaporation under reduced pressure and lyophilised, to give 105 mg of the title compound as a colourless powder.
Infrared Absorption Spectrum (KBr), νₘₐₓ cm⁻¹:
   1759, 1652, 1595, 1460, 1378, 1286.
Nuclear Magnetic Resonance Spectrum (270 MHz, D₂O, using tetradeuterated sodium trimethylsilylpropionate as an internal standard), δ ppm:
   1.21 (3H, doublet, J = 7.3 Hz);
   1.28 (3H, doublet, J = 6.4 Hz);
   1.96 - 2.10 (1H, multiplet);
   2.25 - 2.34 (2H, multiplet);
   3.00 - 3.15 (1H, multiplet);
   3.33 - 3.54 (6H, multiplet);
   3.66 - 3.84 (7H, multiplet);
   3.91 - 3.96 (2H, multiplet);
   4.04 - 4.13 (1H, multiplet);
   4.20 - 4.29 (2H, multiplet);
   4.81 - 4.91 (1H, multiplet).

### EXAMPLE 16

### (1R,5S,6S)-2-[(2S,4S)-2-(4-Carbamoylmethyl-1-homopiperazinylcarbonyl)pyrrolidin-4-ylthio]-6-[(1R)-1-hydroxyethyl]-1-methyl-1-carbapen-2-em-3-carboxylic acid hydrochloride

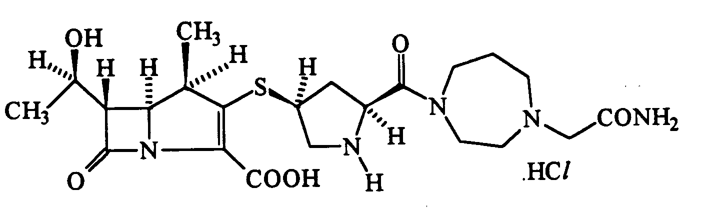

### 16(a) 4-Nitrobenzyl (1R,5S,6S)-2-[(2S,4S)-2-(4-carbamoylmethyl-1-homopiperazinylcarbonyl)-1-(4-nitrobenzyloxycarbonyl)pyrrolidin-4-ylthio]-6-[(1R)-1-hydroxyethyl]-1-methyl-1-carbapen-2-em-3-carboxylate

2.45 ml of diphenylphosphoryl chloride and 2.05 ml of diisopropylethylamine were added dropwise, whilst ice-cooling, to a solution of 3.98 g of 4-nitrobenzyl (1R,5R,6S)-6-[(1R)-1-hydroxyethyl]-1-methyl-2-oxo-1-carbapenam-3-carboxylate in 40 ml of dry acetonitrile, and the resulting mixture was stirred at the same temperature for 1 hour. At the end of this time, 1.85 ml of diisopropylethylamine and a solution of 4.88 g of (2S,4S)-4-mercapto-2-(4-carbamoylmethyl-1-homopiperazinylcarbonyl]-1-(4-nitrobenzyloxycarbonyl)pyrrolidine (prepared as described in Preparation 16) in 30 ml of dry acetonitrile were simultaneously added dropwise to the mixture, whilst ice-cooling, and the mixture was stirred at the same temperature for 1 hour. The reaction mixture was then diluted with 130 ml of acetonitrile and 200 ml of water, after which 1.85 g of sodium hydrogencarbonate was added. The mixture thus obtained was purified by reverse phase column chromatography through 500 g of Cosmo Sil 75C₁₈-PREP (a trade mark for a product of Nacalai Tesque), using 50% v/v aqueous acetonitrile as the eluent. Those fractions containing the title compound were combined and concentrated to give 6.05 g of the title compound, as a powder.
Infrared Absorption Spectrum (KBr), νₘₐₓ cm⁻¹:
   1772, 1708, 1521, 1346.
Nuclear Magnetic Resonance Spectrum (hexadeuterated dimethyl sulphoxide, 270 MHz), δ ppm:
   1.14 - 1.20 (6H, multiplet);
   1.59 - 1.75 (3H, multiplet);
   2.30 - 3.02 (6H, multiplet);
   3.13 - 3.38 (2H, multiplet);
   3.42 - 3.70 (5H, multiplet);
   3.75 - 4.00 (2H, multiplet);
   4.10 - 4.28 (2H, multiplet);
   4.68 - 4.83 (1H, multiplet);
   5.05 - 5.49 (6H, multiplet);
   7.08 - 7.22 (2H, multiplet);
   7.54 - 7.74 (4H, multiplet);
   8.20 - 8.25 (4H, multiplet).

### 16(b) (1R,5S,6S)-2-[(2S,4S)-2-(4-Carbamoylmethyl-1-homopiperazinylcarbonyl)pyrrolidin-4-ylthio]-6-[(1R)-1-hydroxyethyl]-1-methyl-1-carbapen-2-em-3-carboxylic acid hydrochloride

200 mg of 4-nitrobenzyl (1R,5S,6S)-2-[(2S,4S)-2-(4-carbamoylmethyl-1-homopiperazinylcarbonyl)-1-(4-nitrobenzyloxycarbonyl)pyrrolidin-4-ylthio]-6-[(1R)-1-hydroxyethyl]-1-methyl-1-carbapen-2-em-3-carboxylate [prepared as described in step (a) above] were dissolved in 20 ml of a 1 : 1 by volume mixture of tetrahydrofuran and water, after which 0.18 ml of 1N aqueous hydrochloric acid were added to the mixture, which was then hydrogenated by bubbling hydrogen through it at room temperature for 2 hours in the presence of 0.3 g of 10% w/w palladium-on-charcoal. The catalyst was then filtered off, and the filtrate was extracted with diethyl ether. The aqueous layer was concentrated by evaporation under reduced pressure, and the resulting residue was purified by column chromatography through a Lobar column (a product of Merck, LiChroprep RP-8, size B), using water as the eluent. Those fractions containing the title compound were combined, concentrated by evaporation under reduced pressure and lyophilised, to give 30 mg of the title compound as a colourless powder.
Ultraviolet absorption spectrum (H₂O) λₘₐₓ nm:
   297.

### EXAMPLE 17

### (1R,5S,6S)-2-[(2S,4S)-2-(4-Acetimidoylpiperazin-1-yl-carbonyl)pyrrolidin-4-ylthio]-6-[(1R)-1-hydroxyethyl]-1-methyl-1-carbapen-2-em-3-carboxylic acid hydrochloride

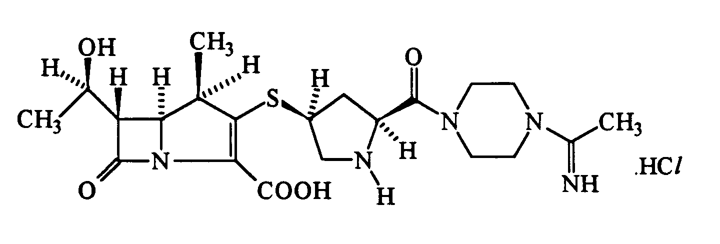

### 17(a) 4-Nitrobenzyl (1R,5S,6S)-2-{(2S,4S)-2-[4-(N-4-nitrobenzyloxycarbonylacetimidoyl)piperazin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

1.45 ml of diphenylphosphoryl chloride and 1.22 ml of diisopropylethylamine were added dropwise, whilst ice-cooling, to a solution of 2.43 g of 4-nitrobenzyl (1R,5R,6S)-6-[(1R)-1-hydroxyethyl]-1-methyl-2-oxo-1-carbapenam-3-carboxylate in 25 ml of dry acetonitrile, and the resulting mixture was stirred at the same temperature for 30 minutes. A solution of 3.99 g of (2S,4S)-4-mercapto-2-[4-(N-4-nitrobenzyloxycarbonylacetimidoyl)piperazin-1-ylcarbonyl]-1-(4-nitrobenzyloxycarbonyl)pyrrolidine (prepared as described in Preparation 17) in 15 ml of dry acetonitrile and 1.22 ml of diisopropylethylamine were then simultaneously added dropwise to the mixture, whilst ice-cooling, and the mixture was stirred overnight at the same temperature. At the end of this time, the reaction mixture was concentrated by evaporation under reduced pressure, and the resulting residue was diluted with ethyl acetate. The diluted solution was washed, in turn, with an aqueous solution of sodium hydrogencarbonate, with water and with an aqueous solution of sodium chloride. The ethyl acetate layer was then dried over anhydrous sodium sulphate, and the solvent was removed by distillation under reduced pressure. The residue was purified by column chromatography through silica gel, using a 6 : 4 by volume mixture of ethyl acetate and acetonitrile as the eluent. Those fractions containing the title compound were combined and concentrated to give 5.17 g of the title compound, as a powder.

The spectral data of this compound are identical with those of the compound prepared as described in Example 1(a).

### 17(b) (1R,5S,6S)-2-[(2S,4S)-2-(4-Acetimidoylpiperazin-1-ylcarbonyl)pyrrolidin-4-ylthio]-6-[(1R)-1-hydroxyethyl]-1-methyl-1-carbapen-2-em-3-carboxylic acid

5.10 g of 4-nitrobenzyl (1R,5S,6S)-2-{(2S,4S)-2-[4-(N-4-nitrobenzyloxycarbonylacetimidoyl)piperazin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 100 ml of a 6 : 4 by volume mixture of tetrahydrofuran and water, and the mixture was hydrogenated by bubbling hydrogen through it at room temperature for 2.5 hours in the presence of 5.0 g of 10% w/w palladium-on-charcoal. The catalyst was filtered off, and the filtrate was washed with diethyl ether and then concentrated by evaporation under reduced pressure. The resulting residue was purified by reverse phase column chromatography through 250 ml of Cosmo Sil 75C₁₈-PREP (a trade mark for a product of Nacalai Tesque), using water, 5% v/v aqueous acetonitrile and 7% v/v aqueous acetonitrile, in that order, as the eluent. Those fractions containing the title compound were combined, concentrated by evaporation under reduced pressure and lyophilised to give 940 mg of the title compound, as a powder.

The spectral data of this compound are identical with those of the compound prepared as described in Example 1(b).

### 17(c) (1R,5S,6S)-2-[(2S,4S)-2-(4-Acetimidoylpiperazin-1-ylcarbonyl)pyrrolidin-4-ylthio]-6-[(1R)-1-hydroxyethyl]-1-methyl-1-carbapen-2-em-3-carboxylic acid hydrochloride

200 mg of (1R,5S,6S)-2-[(2S,4S)-2-(4-acetimidoylpiperazin-1-ylcarbonyl)pyrrolidin-4-ylthio]-6-[(1R)-1-hydroxyethyl] -1-methyl-1-carbapen-2-em-3-carboxylic acid [prepared as described in step (b) above] were dissolved in 5 ml of water, after which 0.43 ml of 1N aqueous hydrochloric acid was added. The resulting mixture was worked up and purified by reverse phase column chromatography through 30 ml of Cosmo Sil 75C₁₈-PREP (a trade mark for a product of Nacalai Tesque), using water as the eluent. Those fractions containing the title compound were combined and lyophilised to give 149 mg of the title compound as a powder.
Infrared Absorption Spectrum (KBr), νₘₐₓ cm⁻¹:
   1756, 1656, 1620, 1450, 1382, 1252.
Ultraviolet absorption spectrum (H₂O) λₘₐₓ nm:
   297.
Nuclear Magnetic Resonance Spectrum (D₂O, 270 MHz), δ ppm:
   1.21 (3H, doublet, J = 7.3 Hz);
   1.29 (3H, doublet, J = 6.4 Hz);
   1.97 - 2.07 (1H, multiplet);
   2.35 & 2.36 (together 3H, two singlets);
   3.02 - 3.14 (1H, multiplet);
   3.31 - 3.43 (1H, multiplet);
   3.45 - 3.53 (2H, multiplet);
   3.68 - 3.88 (9H, multiplet);
   4.03 - 4.12 (1H, multiplet);
   4.20 - 4.30 (2H, multiplet);
   4.82 - 4.90 (1H, multiplet).

### EXAMPLE 18

### (1R,5S,6S)-2-{(2S,4S)-2-[(3S)-3-Aminopyrrolidin-1-ylcarbonyl]pyrrolidin-4-ylthio}-6-[(1R)-1-hydroxyethyl]-1-methyl-1-carbapen-2-em-3-carboxylic acid hydrochloride

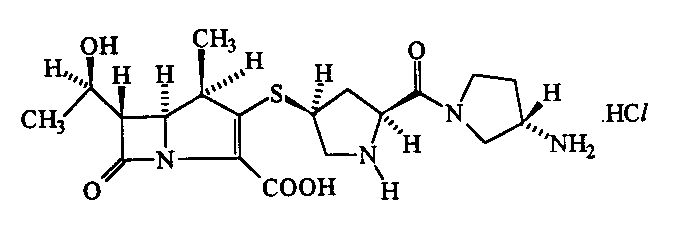

### 18(a) 4-Nitrobenzyl (1R,5S,6S)-2-{(2S,4S)-2-[(3S)-3-(4-nitrobenzyloxycarbonyl)aminopyrrolidin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

453 µℓ of diphenylphosphoryl chloride and 381 µℓ of diisopropylethylamine were added dropwise, whilst ice-cooling, to a solution of 760 mg of 4-nitrobenzyl (1R,5R,6S)-6-[(1R)-1-hydroxyethyl]-1-methyl-2-oxo-1-carbapenam-3-carboxylate in 6 ml of dry acetonitrile, and the resulting mixture was stirred at the same temperature for 1 hour. A solution of 1.26 g of (2S,4S)-4-mercapto-2-[(3S)-3-(4-nitrobenzyloxycarbonyl)-aminopyrrolidin-1-ylcarbonyl]-1-(4-nitrobenzyloxycarbonyl)pyrrolidine (prepared as described in Preparation 18) in 5 ml of dry acetonitrile and 364 µℓ of diisopropylethylamine were then simultaneously added dropwise to the mixture, whilst ice-cooling and the mixture was stirred at the same temperature for 3 hours. At the end of this time, the solvent was removed by distillation under reduced pressure, and the resulting residue was diluted with 70 ml of ethyl acetate. The diluted solution was then washed, in turn, with water, with a saturated aqueous solution of sodium hydrogencarbonate, with water and with a saturated aqueous solution of sodium chloride, in that order. The washed organic solution was then dried over anhydrous magnesium sulphate, and the solvent was removed by distillation under reduced pressure. The resulting residue was purified by column chromatography through silica gel, using a 4 : 96 by volume mixture of methanol and ethyl acetate as the eluent, to give 1.01 g of the title compound, as a powder.
Infrared Absorption Spectrum (KBr), νₘₐₓ cm⁻¹:
   3377, 1774, 1713, 1648, 1607, 1521, 1346, 852, 736.
Nuclear Magnetic Resonance Spectrum (hexadeuterated dimethyl sulphoxide, 270 MHz), δ ppm:
   1.15 - 1.18 (6H, multiplet);
   1.70 - 2.20 (2H, multiplet);
   2.77 - 2.85 (1H, multiplet);
   3.12 - 4.27 (14H, multiplet);
   4.49 - 4.64 (1H, multiplet);
   5.05 - 5.49 (7H, multiplet);
   7.48 - 7.82 (6H, multiplet);
   8.17 - 8.25 (6H, multiplet).

### 18(b) (1R,5S,6S)-2-{(2S,4S)-2-[(3S)-3-Aminopyrrolidin-1-ylcarbonyl]pyrrolidin-4-ylthio}-6-[(1R)-1-hydroxyethyl]-1-methyl-1-carbapen-2-em-3-carboxylic acid hydrochloride

1.0 g of 4-nitrobenzyl (1R,5S,6S)-2-{(2S,4S)-2-[(3S)-3-(4-nitrobenzyloxycarbonyl)aminopyrrolidin-1-ylcarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 30 ml of a 2 : 1 by volume mixture of tetrahydrofuran and water, after which 1.0 ml of 1N aqueous hydrochloric acid was added, and the mixture was hydrogenated by bubbling hydrogen through it at room temperature for 2 hours in the presence of 1.5 g of 10% w/w palladium-on-charcoal. The catalyst was filtered off, and the filtrate was washed with diethyl ether. The washed aqueous solution was concentrated by evaporation under reduced pressure, and the residue was purified by reverse phase column chromatography through 19 ml of Cosmo Sil 75C₁₈-PREP (a trade mark for a product of Nacalai Tesque), using water as the eluent. Those fractions containing the title compound were combined, concentrated by evaporation under reduced pressure and lyophilised to give 169 mg of the title compound as a powder.
Infrared Absorption Spectrum (KBr), νₘₐₓ cm⁻¹:
   3397, 1758, 1653, 1587, 1465, 1386.
Nuclear Magnetic Resonance Spectrum (270 MHz, D₂O, using tetradeuterated sodium trimethylsilylpropionate as an internal standard), δ ppm:
   1.21 (3H, doublet, J = 7.32 Hz);
   1.29 (3H, doublet, J = 6.35 Hz);
   1.97 - 2.19 (1H, multiplet);
   2.21 - 2.29 (1H, multiplet);
   2.36 - 2.60 (1H, multiplet);
   3.02 - 3.14 (1H, multiplet);
   3.32 - 3.43 (1H, multiplet);
   3.45 - 3.53 (2H, multiplet);
   3.57 - 3.90 (5H, multiplet);
   3.98 - 4.17 (2H, multiplet);
   4.20 - 4.29 (2H, multiplet);
   4.63 - 4.82 (1H, multiplet).

### EXAMPLE 19

### (1R,5S,6S)-2-{(2S,4S)-2-[(3S)-Pyrrolidin-3-ylaminocarbonyl]pyrrolidin-4-ylthio}-6-[(1R)-1-hydroxyethyl]-1-methyl-1-carbapen-2-em-3-carboxylic acid hydrochloride

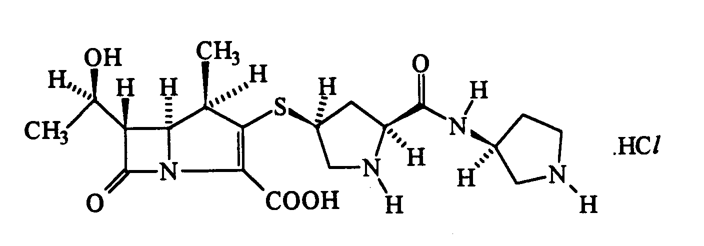

### 19(a) 4-Nitrobenzyl (1R,5S,6S)-2-{(2S,4S)-2-[(3S)-1-(4-nitrobenzyloxycarbonyl)pyrrolidin-3-ylaminocarbonyl]-1-(4-nitrobenzyloxycarbonyl) pyrrolidin-4-ylthio}-6-[(1R)-1-hydroxyethyl]-1-methyl-1-carbapen-2-em-3-carboxylate

253 µℓ of diphenylphosphoryl chloride and 212 µℓ of diisopropylethylamine were added dropwise, whilst ice-cooling, to a solution of 420 mg of 4-nitrobenzyl (1R,5R,6S)-6-[(1R)-1-hydroxyethyl]-1-methyl-2-oxo-1-carbapenam-3-carboxylate in 4.2 ml of dry acetonitrile, and the resulting mixture was stirred at the same temperature for 1 hour. A solution of 913 mg of (2S,4S)-4-mercapto-2-[(3S)-1-(4-nitrobenzyloxycarbonyl)pyrrolidin-3-ylaminocarbonyl]-1-(4-nitrobenzyloxycarbonyl)pyrrolidine (prepared as described in Preparation 19) in 5 ml of dry acetonitrile and 404 µℓ of diisopropylethylamine were then simultaneously added dropwise to the mixture, whilst ice-cooling, and the mixture was stirred at the same temperature for 7 hours and then allowed to stand overnight at the same temperature. At the end of this time, 101 µℓ of diisopropylethylamine were added, and the mixture was stirred at the same temperature for 1 hour. The reaction mixture was then worked up and purified by the same procedure as described in Example 18(a), to give 504 mg of the title compound, as a powder.
Infrared Absorption Spectrum (KBr), νₘₐₓ cm⁻¹:
   3385, 1775, 1709, 1607, 1522, 1346, 853, 737.
Nuclear Magnetic Resonance Spectrum (hexadeuterated dimethyl sulphoxide, 270 MHz), δ ppm:
   1.15 - 1.18 (6H, multiplet);
   1.69 - 2.07 (3H, multiplet);
   2.60 - 2.80 (1H, multiplet);
   3.10 - 3.70 (9H, multiplet);
   3.80 - 4.35 (6H, multiplet);
   5.15 - 5.48 (6H, multiplet);
   7.57 - 7.73 (6H, multiplet);
   8.21 - 8.33 (6H, multiplet).

### 19(b) (1R,5S,6S)-2-{(2S,4S)-2-[(3S)-Pyrrolidin-3-ylaminocarbonyl]pyrrolidi-4-ylthio}-6-[(1R)-1-hydroxyethyl]-1-methyl-1-carbapen-2-em-3-carboxylic acid hydrochloride

475 mg of 4-nitrobenzyl (1R,5S,6S)-2-{(2S,4S)-2-[(3S)-1-(4-nitrobenzyloxycarbonyl)pyrrolidin-3-ylaminocarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 15 ml of a 2 : 1 by volume mixture of tetrahydrofuran and water, after which 0.45 ml of 1N aqueous hydrochloric acid was added, and the mixture hydrogenated by bubbling hydrogen through it at room temperature for 2 hours in the presence of 1 g of 10% w/w palladium-on-charcoal. At the end of this time, the reaction mixture was worked up and purified by the same procedure as described in Example 18(b), to give 46 mg of the title compound as a powder.
Infrared Absorption Spectrum (KBr), νₘₐₓ cm⁻¹:
   3367, 1760, 1683, 1558, 1390, 1281.
Nuclear Magnetic Resonance Spectrum (270 MHz, D₂O, using tetradeuterated sodium trimethylsilylpropionate as an internal standard), δ ppm:
   1.21 (3H, doublet, J = 7.33 Hz);
   1.28 (3H, doublet, J = 6.35 Hz);
   2.04 - 2.24 (2H, multiplet);
   2.33 - 2.46 (1H, multiplet);
   2.88 - 3.00 (1H, multiplet);
   3.31 - 3.56 (6H, multiplet);
   3.59 - 3.67 (1H, multiplet);
   3.77 - 3.84 (1H, multiplet);
   4.04 - 4.13 (1H, multiplet);
   4.20 - 4.29 (2H, multiplet);
   4.47 - 4.56 (2H, multiplet).

### EXAMPLE 20

### (1R,5S,6S)-2-{(2S,4S)-2-[(3R)-Pyrrolidin-3-ylaminocarbonyl]pyrrolidin-4-ylthio}-6-[(1R)-1-hydroxyethyl]-1-methyl-1-carbapen-2-em-3-carboxylic acid hydrochloride

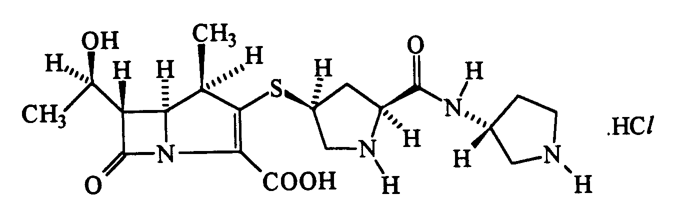

### 20(a) 4-Nitrobenzyl (1R,5S,6S)-2-{(2S,4S)-2-[(3R)-1-(4-nitrobenzyloxycarbonyl)pyrrolidin-3-ylaminocarbonyl]-1-(4-nitrobenzyloxycarbonyl)pyrrolidin-4-ylthio}-6-[(1R)-1-hydroxyethyl]-1-methyl-1-carbapen-2-em-3-carboxylate

Following a procedure similar to that described in Example 19(a), but using 1.06 g of (2S,4S)-4-mercapto-2-[(3R)-1-(4-nitrobenzyloxycarbonyl)pyrrolidin-3-ylaminocarbonyl]-1-(4-nitrobenzyloxycarbonyl) pyrrolidine (prepared as described in Preparation 20) instead of the (2S,4S)-4-mercapto-2-[(3S)-1-(4-nitrobenzyloxycarbonyl)pyrrolidin-3-ylaminocarbonyl]-1-(4-nitrobenzyloxycarbonyl)pyrrolidine, 540 mg of the title compound were obtained as a powder.

### (20b) (1R,5S,6S)-2-{(2S,4S)-2-[(3R)-Pyrrolidin-3-ylaminocarbonyl]pyrrolidin-4-ylthio}-6-[(1R)-1-hydroxyethyl]-1-methyl-1-carbapen-2-em-3-carboxylic acid hydrochloride

500 mg of 4-nitrobenzyl (1R,5S,6S)-2-{(2S,4S)-2-[(3R)-1-(4-nitrobenzyloxycarbonyl)pyrrolidin-3-ylaminocarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 20 ml of a 1 : 1 by volume mixture of tetrahydrofuran and water, after which 0.47 ml of 1N aqueous hydrochloric acid was added, and the mixture was hydrogenated by bubbling hydrogen through it at room temperature for 2 hours in the presence of 1 g of 10% w/w palladium-on-charcoal. The reaction mixture was then worked up and purified by the same procedure as described in Exmaple 18(b), to give 43 mg of the title compound as a powder.
Ultraviolet absorption spectrum (H₂O) λₘₐₓ nm:
   297.

### EXAMPLE 21

### (1R,5S,6S)-2-{(2S,4S)-2-[(3S)-3-Dimethylaminopyrrolidin-1-ylcarbonyl]pyrrolidin-4-ylthio}-6-[(1R)-1-hydroxyethyl]-1-methyl-1-carbapen-2-em-3-carboxylic acid hydrochloride

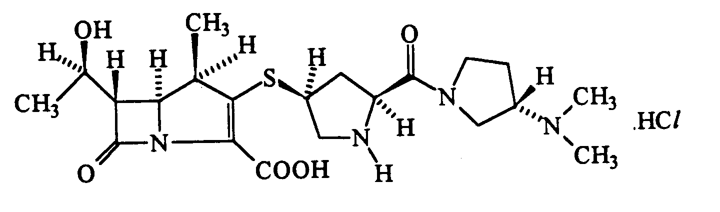

### 21(a) 4-Nitrobenzyl (1R,5S,6S)-2-{(2S,4S)-2-[(3S)-3-dimethylaminopyrrolidin-1-ylcarbonyl]-1-(4-nitrobenzyloxycarbonyl]pyrrolidin-4-ylthio] -6-[(1R)-1-hydroxyethyl]-1-methyl-1-carbapen-2-em-3-carboxylate

880 µℓ of diphenylphosphoryl chloride and 740 µℓ of diisopropylethylamine, whilst ice-cooling, were added dropwise to a solution of 1.46 g of 4-nitrobenzyl (1R,5R,6S)-6-[(1R)-1-hydroxyethyl]-1-methyl-2-oxo-1-carbapenam-3-carboxylate in 12 ml of dry acetonitrile, and the resulting mixture was stirred at the same temperature for 0.5 hours. A solution of 1.70 g of (2S,4S)-4-mercapto-2-[(3S)-3-dimethylaminopyrrolidin-1-ylcarbonyl]-1-(4-nitrobenzyloxycarbonyl)pyrrolidine trifluorosulphonate (prepared as described in Preparation 21) in 8 ml of dry acetonitrile and 700 µℓ of diisopropylethylamine were then simultaneously added dropwise to the mixture, whilst ice-cooling, and the mixture was stirred at the same temperature for 2.5 hours. At the end of this time, the reaction mixture was worked up and purified by the same procedure as described in Example 18(a), to give 1.65 g of the title compound, as a powder.
Infrared Absorption Spectrum (KBr), νₘₐₓ cm⁻¹:
   1773, 1711, 1650, 1607, 1522, 1346, 854, 738.
Nuclear Magnetic Resonance Spectrum (hexadeuterated dimethyl sulphoxide, 270 MHz), δ ppm:
   1.15 (3H, doublet, J = 3.42 Hz);
   1.18 (3H, doublet, J = 3.90 Hz);
   2.09 (2H, doublet, J = 8.3 Hz);
   2.17 (1H, singlet);
   2.70 - 3.93 (17H, multiplet);
   3.95 - 4.08 (1H, multiplet);
   4.11 - 4.20 (1H, multiplet);
   4.23 - 4.29 (1H, multiplet);
   4.55 - 4.66 (1H, multiplet);
   5.06 - 5.75 (4H, multiplet);
   7.53 - 7.74 (4H, multiplet);
   8.21 - 8.25 (4H, multiplet).

### 21(b) (1R,5S,6S)-2-{(2S,4S)-2-[(3S)-3-Dimethylaminopyrrolidin-1-ylcarbonyl]pyrrolidin-4-ylthio}-6-[(1R)-1-hydroxyethyl]-1-methyl-1-carbapen-2-em-3-carboxylic acid hydrochloride

306 mg of 4-nitrobenzyl (1R,5S,6S)-2-{(2S,4S)-2-[(3S)-3-dimethylaminopyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 12 ml of a 2 : 1 by volume mixture of tetrahydrofuran and water, after which 0.38 ml of 1N aqueous hydrochloric acid was added, and the mixture was hydrogenated by bubbling hydrogen through it at room temperature for 2 hours in the presence of 600 mg of 10% w/w palladium-on-charcoal. The reaction mixture was then worked up and purified by the same procedure as described in Example 18(b), to give 19 mg of the title compound as a powder.
Infrared Absorption Spectrum (KBr), νₘₐₓ cm⁻¹:
   3385, 1764, 1656, 1553, 1466, 1375.
Nuclear Magnetic Resonance Spectrum (270 MHz, D₂O, using tetradeuterated sodium trimethylsilylpropionate as an internal standard), δ ppm:
   1.22 (3H, doublet, J = 7.32 Hz);
   1.28 (3H, doublet, J = 6.35 Hz);
   1.95 - 2.10 (1H, multiplet);
   2.15 - 2.35 (3H, multiplet);
   2.5 - 2.7 (1H, multiplet);
   2.96 - 2.97 (6H, multiplet);
   3.00 - 3.15 (1H, multiplet);
   3.37 - 3.43 (1H, multiplet);
   3.46 - 3.52 (2H, multiplet);
   3.56 - 3.70 (2H, multiplet);
   3.73 - 4.11 (6H, multiplet);
   4.15 - 4.30 (2H, multiplet).

### EXAMPLE 22

### (1R,5S,6S)-2-{(2S,4S)-2-[(3S)-3-Methylaminopyrrolidin-1-ylcarbonyl]pyrrolidin-4-ylthio}-6-[(1R)-1-hydroxyethyl]-1-methyl-1-carbapen-2-em-3-carboxylic acid hydrochloride

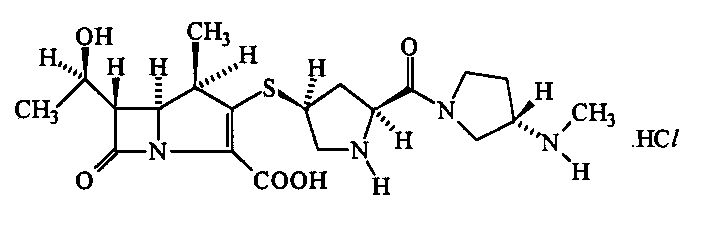

### 22(a) 4-Nitrobenzyl (1R,5S,6S)-2-{(2S,4S)-2-[(3S)-3-N-methyl-N-(4-nitrobenzyloxycarbonyl)aminopyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 18(a), but using 820 mg of (2S,4S)-4-mercapto-2-[(3S)-3-N-methyl-N-(4-nitrobenzyloxycarbonyl)aminopyrrolidin-1-ylcarbonyl]-1-(4-nitrobenzyloxycarbonyl)-pyrrolidine (prepared as described in Preparation 22) instead of the (2S,4S)-4-mercapto-2-[(3S)-3-(4-nitrobenzyloxycarbonyl)aminopyrrolidin-1-ylcarbonyl]-1-(4-nitrobenzyloxycarbonyl)pyrrolidine, 630 mg of the title compound were obtained.

### 22(b) (1R,5S,6S)-2-{(2S,4S)-2-[(3S)-3-Methylaminopyrrolidin-1-ylcarbonyl]pyrrolidin-4-ylthio}-6-[(1R)-1-hydroxyethyl]-1-methyl-1-carbapen-2-em-3-carboxylic acid hydrochloride

580 mg of 4-nitrobenzyl (1R,5S,6S)-2-{(2S,4S)-2-[(3S)-3-N-methyl-N-(4-nitrobenzyloxycarbonyl) aminopyrrolidin-1-ylcarbonyl]-1-(4-nitrobenzyloxycarbonyl)-pyrrolidin-4-ylthio}-6-[(1R)-1-hydroxyethyl]-1-methyl-1-carbapen-2-em-3-carboxylate [prepared as described in step (a) above] were dissolved in 12 ml of a 1 : 1 by volume mixture of tetrahydrofuran and water, after which 0.55 ml of 1N aqueous hydrochloric acid was added, and the mixture was hydrogenated by bubbling hydrogen through it at room temperature for 2 hours in the presence of 700 mg of 10% w/w palladium-on-charcoal. At the end of this time, the reaction mixture was worked up and purified by the same procedure as described in Example 18(b), to give 53 mg of the title compound as a powder.
Ultraviolet absorption spectrum (H₂O) λₘₐₓ nm:
   297.

### EXAMPLE 23

### (1R,5S,6S)-1-{(2S,4S)-2-[(3S)-Acetimidoylaminopyrrolidin-1-ylcarbonyl]pyrrolidin-4-ylthio}-6-[(1R)-1-hydroxyethyl]-1-methyl-1-carbapen-2-em-3-carboxylic acid

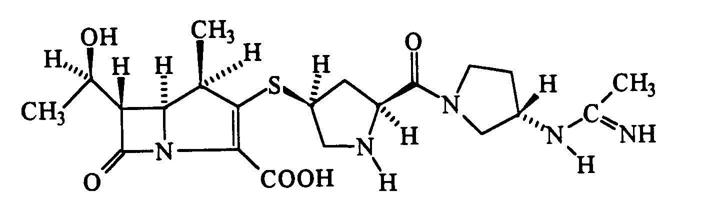

### 23(a) 4-Nitrobenzyl (1R,5S,6S)-2-{(2S,4S)-2-[(3S)-3-(N-4-nitrobenzyloxycarbonylacetimidoylamino)pyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

231 µℓ of diphenylphosphoryl chloride and 194 µℓ of diisopropylethylamine, whilst ice-cooling, were added dropwise to a solution of 384 mg of 4-nitrobenzyl (1R,5R,6S)-6-[(1R)-1-hydroxyethyl]-1-methyl-2-oxo-1-carbapenam-3-carboxylate in 6 ml of dry acetonitrile, and the resulting mixture was stirred at the same temperature for 45 minutes. A solution of 652 mg of (2S,4S)-4-mercapto-2-[(3S)-3-(N-4-nitrobenzyloxycarbonylacetimidoylamino)pyrrolidin-1-ylcarbonyl]-1-(4-nitrobenzyloxycarbonyl) pyrrolidine (prepared as described in Preparation 23) in 4 ml of acetonitrile and 185 µℓ of diisopropylethylamine were then simultaneously added dropwise to the mixture, and the mixture was stirred at the same temperature for 3 hours. At the end of this time, the reaction mixture was concentrated by evaporation under reduced pressure. The resulting residue was diluted with ethyl acetate and the diluted solution was washed with water and with an aqueous solution of sodium chloride. The ethyl acetate solution was dried over anhydrous magnesium sulphate and the solvent was removed by distillation under reduced pressure. The resulting residue was purified by column chromatography through silica gel, using a 9 : 9 : 1 by volume mixture of methylene chloride, ethyl acetate and methanol as the eluent. Those fractions containing the title compound were combined and concentrated to give 510 mg of the title compound, as a powder.
Infrared Absorption Spectrum (KBr), νₘₐₓ cm⁻¹:
   1774, 1709, 1654, 1607, 1551, 1521, 1441, 1404, 1346.
Nuclear Magnetic Resonance Spectrum (hexadeuterated dimethyl sulphoxide + D₂O, 270 MHz), δ ppm:
   1.15 (3H, doublet, J = 6.35 Hz);
   1.16 (3H, doublet, J = 7.32 Hz);
   1.60 - 2.28 (2H, multiplet);
   2.10 (3H, singlet);
   2.69 - 2.93 (1H, multiplet);
   3.10 - 4.72 (14H, multiplet);
   5.04 - 5.51 (6H, multiplet);
   7.46 - 7.76 (6H, multiplet);
   8.14 - 8.28 (6H, multiplet).

### 23(b) (1R,5S,6S)-1-{(2S,4S)-2-[(3S)-Acetimidoylaminopyrrolidin-1-ylcarbonyl]pyrrolidin-4-ylthio}-6-[(1R)-1-hydroxyethyl]-1-methyl-1-carbapen-2-em-3-carboxylic acid

500 mg of 4-nitrobenzyl (1R,5S,6S)-2-{(2S,4S)-2-[(3S)-3-(N-4-nitrobenzyloxycarbonylacetimidoylamino) pyrrolidin-1-ylcarbonyl]-1-(4-nitrobenzyloxycarbonyl)-pyrrolidin-4-ylthio}-6-[(1R)-1-hydroxyethyl]-1-methyl-1-carbapen-2-em-3-carboxylate [prepared as described in step (a) above] were dissolved in 16 ml of a 1 : 1 by volume mixture of tetrahydrofuran and water, and the mixture was hydrogenated by bubbling hydrogen through it at room temperature for 2 hours in the presence of 400 mg of 10% w/w palladium-on-charcoal. The catalyst was then filtered off, and the filtrate was washed with diethyl ether. The aqueous layer was concentrated by evaporation under reduced pressure, and the concentrate was purified by reverse phase column chromatography through 20 ml of Cosmo Sil 75C₁₈-PREP (a product of Nacalai Tesque), using 6% v/v aqueous acetonitrile as the eluent. Those fractions containing the title compound were combined, concentrated by evaporation under reduced pressure and lyophilised, to give 136 mg of the title compound as a powder.
Ultraviolet absorption spectrum (H₂O) λₘₐₓ nm:
   298.
Infrared Absorption Spectrum (KBr), νₘₐₓ cm⁻¹:
   1756, 1632, 1590, 1451, 1386, 1283, 1259.
Nuclear Magnetic Resonance Spectrum (270 MHz, D₂O, using tetradeuterated sodium trimethylsilylpropionate as an internal standard), δ ppm:
   1.22 (3H, doublet, J = 7.32 Hz);
   1.30 (3H, doublet, J = 6.35 Hz);
   1.57 - 1.71 (1H, multiplet);
   1.97 - 2.51 (2H, multiplet);
   2.23 & 2.25 (together 3H, two singlets);
   2.64 - 2.81 (1H, multiplet);
   3.05 (1H, doublet of doublets, J = 12.21 & 3.91 Hz);
   3.18 (1H, doublet of doublets, J = 12.21 & 5.86 Hz);
   3.43 (1H, doublet of doublets, J = 6.35 & 2.24 Hz);
   3.32 - 4.06 (7H, multiplet);
   4.22 (1H, doublet of doublets, J = 9.28 & 2.44 Hz);
   4.18 - 4.43 (2H, multiplet).

### EXEMPLE 24

### (1R,5S,6S)-2-{(2S,4S)-2-[(3S)-Formimidoylaminopyrrolidin-1-ylcarbonyl]pyrrolidin-4-ylthio}-6-[(1R)-1-hydroxyethyl]-1-methyl-1-carbapen-2-em-3-carboxylic acid

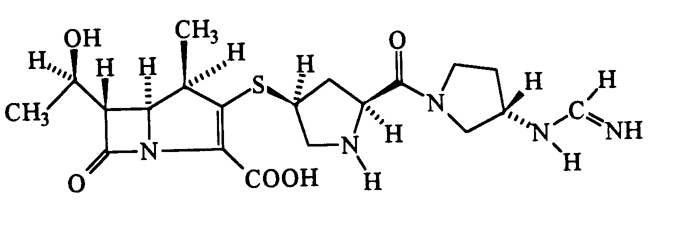

### 24(a) 4-Nitrobenzyl (1R,5S,6S)-2-{(2S,4S)-2-[(3S)-3-(N-4-nitrobenzyloxycarbonylformimidoylamino)pyrrolidin1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

250 µℓ of diphenylphosphoryl chloride and 210 µℓ of diisopropylethylamine were added dropwise, whilst ice-cooling, to a solution of 417 mg of 4-nitrobenzyl (1R,5R,6S)-6-[(1R)-1-hydroxyethyl]-1-methyl-2-oxo-1-carbapenam-3-carboxylate in 8 ml of dry acetonitrile, and the resulting mixture was stirred at the same temperature for 1 hour. A solution of 659 mg of (2S,4S)-4-mercapto-2-[(3S)-3-(N-4-nitrobenzyloxycarbonylformimidoylamino)pyrrolidin-1-ylcarbonyl]-1-(4-nitrobenzyloxycarbonyl) pyrrolidine (prepared as described in Preparation 24) in 7 ml of dry acetonitrile and 210 µℓ of diisopropylethylamine were then simultaneously added dropwise to the mixture, whilst ice-cooling, and the mixture was stirred at the same temperature for 1 hour and then allowed to stand overnight at the same temperature. At the end of this time, the reaction mixture was worked up and purified by the same procedure as described in Example 23(a), to give 593 mg of the title compound, as a powder.
Infrared Absorption Spectrum (KBr), νₘₐₓ cm⁻¹:
   1772, 1707, 1655, 1605, 1521, 1444, 1346, 1210, 1136, 1111.
Nuclear Magnetic Resonance Spectrum (CDCℓ₃ + D₂O, 270 MHz), δ ppm:
   1.25 (3H, doublet, J = 7.32 Hz);
   1.36 (3H, doublet, J = 6.35 Hz);
   1.75 - 2.80 (4H, multiplet);
   3.22 - 4.32 (12H, multiplet);
   4.40 - 4.65 (1H, multiplet);
   5.12 - 5.55 (6H, multiplet);
   7.38 - 7.69 (6H, multiplet);
   8.06 - 8.31 (6H, multiplet);
   8.42 (1H, singlet).

### 24(b) (1R,5S,6S)-2-{(2S,4S)-2-[(3S)-Formimidoylaminopyrrolidin-1-ylcarbonyl]pyrrolidin-4-ylthio}-6-[(1R)-1-hydroxyethyl]-1-methyl-1-carbapen-2-em-3-carboxylic acid

570 mg of 4-nitrobenzyl (1R,5S,6S)-2-{(2S,4S)-2-[(3S)-3-(N-4-nitrobenzyloxycarbonylformimidoylamino)pyrrolidin-1-ylcarbonyl]-1-(4-nitrobenzyloxycarbonyl)-pyrrolidin-4-ylthio}-6-[(1R)-1-hydroxyethyl]-1-methyl-1-carbapen-2-em-3-carboxylate [prepared as described in step (a) above] were dissolved in 20 ml of a 1 : 1 by volume mixture of tetrahydrofuran and water, and the mixture was hydrogenated by bubbling hydrogen through it at room temperature for 2 hours in the presence of 450 mg of 10% w/w palladium-on-charcoal. At the end of this time, the reaction mixture was worked up and purified by the same procedure as described in Example 23(b) to give 125 mg of the title compound as a powder.
Ultraviolet absorption spectrum (H₂O) λₘₐₓ nm:
   300.
Infrared Absorption Spectrum (KBr), νₘₐₓ cm⁻¹:
   1755, 1634, 1592, 1455, 1388, 1286, 1260, 1182, 1148.
Nuclear Magnetic Resonance Spectrum (270 MHz, D₂O, using sodium tetradeuterated trimethylsilylpropionate as an internal standard), δ ppm:
   1.22 (3H, doublet, J = 7.32 Hz);
   1.30 (3H, doublet, J = 6.35 Hz);
   1.58 - 1.75 (1H, multiplet);
   1.98 - 2.53 (2H, multiplet);
   2.64 - 2.86 (1H, multiplet);
   3.07 (1H, doublet of doublets, J = 12.21 & 3.91 Hz);
   3.21 (1H, doublet of doublets, J = 12.21 & 5.86 Hz);
   3.32 - 4.12 (8H, multiplet);
   4.17 - 4.53 (3H, multiplet);
   7.80, 7.82, 7.95 & 7.96 (together 1H, four singlets).

### EXAMPLE 25

### (1R,5S,6S)-2-{(2S,4S)-2-[(3R)-1-Formimidoylpyrrolidin-3-ylaminocarbonyl]pyrrolidin-4-ylthio}-6-[(1R)-1-hydroxyethyl]-1-methyl-1-carbapen-2-em-3-carboxylic acid

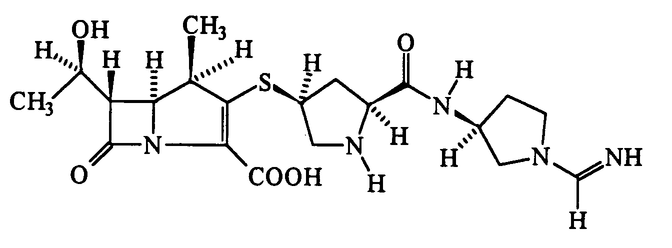

### 25(a) 4-Nitrobenzyl (1R,5S,6S)-2-{(2S,4S)-2-[(3S)-1-(N-4-nitrobenzyloxycarbonylformimidoyl)pyrrolidin-3-yl-aminocarbonyl]-1-(4-nitrobenzyloxycarbonyl) pyrrolidin-4-ylthio}-6-[(1R)-1-hydroxyethyl]-1-methyl-1-carbapen-2em-3-carboxylate

225 µℓ of diphenylphosphoryl chloride and 189 µℓ of diisopropylethylamine were added dropwise, whilst ice-cooling, to a solution of 374 mg of 4-nitrobenzyl (1R,5R,6S)-6-[(1R)-1-hydroxyethyl]-1-methyl-2-oxo-1-carbapenam-3-carboxylate in 5 ml of dry acetonitrile, and the resulting mixture was stirred at the same temperature for 1 hour. A solution of 620 mg of (2S,4S)-4-mercapto-2-[(3S)-1-(N-4-nitrobenzyloxycarbonylformimidoyl)pyrrolidin-3-ylaminocarbonyl]-1-(4-nitrobenzyloxycarbonyl) pyrrolidine (prepared as described in Preparation 25) in 5 ml of dry acetonitrile and 189 µℓ of diisopropylethylamine were then simultaneously added dropwise to the mixture, whilst ice-cooling, and the mixture was stirred at the same temperature for 1 hour, after which it was allowed to stand overnight at the same temperature. At the end of this time, the reaction mixture was worked up and purified by the same procedure as described in Example 23(a), to give 250 mg of the title compound, as a powder.
Infrared Absorption Spectrum (KBr), νₘₐₓ cm⁻¹:
   1774, 1710, 1605, 1520, 1450, 1346, 1220, 1157, 1108.
Nuclear Magnetic Resonance Spectrum (CDCℓ₃ + D₂O, 270 MHz), δ ppm:
   1.27 (3H, doublet, J = 7.32 Hz);
   1.36 (3H, doublet, J = 6.35 Hz);
   2.01 - 2.38 (2H, multiplet);
   3.23 - 4.62 (14H, multiplet);
   5.10 - 5.36 (6H, multiplet);
   5.37 - 5.52 (1H, multiplet);
   7.42 - 7.68 (6H, multiplet);
   8.11 - 8.28 (6H, multiplet);
   8.63 (1H, singlet).

### 25(b) (1R,5S,6S)-2-{(2S,4S)-2-[(3R)-1-Formimidoylpyrrolidin-3-ylaminocarbonyl]pyrrolidin-4-ylthio}-6-[(1R)-1-hydroxyethyl]-1-methyl-1-carbapen-2-em-3-carboxylic acid

205 mg of 4-nitrobenzyl (1R,5S,6S)-2-{(2S,4S)-2-[(3S)-1-(N-4-nitrobenzyloxycarbonylformimidoyl)-pyrrolidin-3-ylaminocarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 12 ml of 1 : 1 by volume mixture of tetrahydrofuran and water, and the mixture was hydrogenated by bubbling hydrogen through it at room temperature for 2 hours in the presence of 160 mg of 10% w/w palladium-on-charcoal. At the end of this time, the reaction mixture was worked up and purified by the same procedure as described in Example 23(b), to give 50 mg of the title compound as a powder.
Ultraviolet absorption spectrum (H₂O) λₘₐₓ nm:
   299.
Infrared Absorption Spectrum (KBr), νₘₐₓ cm⁻¹:
   1754, 1710, 1657, 1586, 1449, 1427, 1386, 1285, 1262, 1182, 1146.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.30 (3H, doublet, J = 6.35 Hz);
   1.75 - 3.04 (5H, multiplet);
   3.31 - 4.95 (12H, multiplet);
   8.01 & 8.03 (together 1H, two singlets).

### EXAMPLE 26

### (1R,5S,6S)-2-{(2S,4S)-2-[(3S)-1-Acetimidoylpyrrolidin-3-ylaminocarbonyl]pyrrolidin-4-ylthio}-6-[(1R)-1-hydroxyethyl]-1-methyl-1-carbapen-2-em-3-carboxylic acid

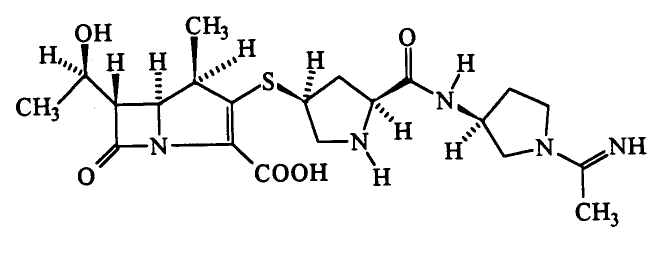

### 26(a) 4-Nitrobenzyl (1R,5S,6S)-2-{(2S,4S)-2-[(3S)-1-(N-4-nitrobenzyloxycarbonylacetimidoyl)pyrrolidin-3-yl-aminocarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 23(a), but using 422 mg of 4-nitrobenzyl (1R,5R,6S)-6-[(1R)-1-hydroxyethyl]-1-methyl-2-oxo-1-carbapenam-3-carboxylate and 717 mg of (2S,4S)-4-mercapto-2-[(3S)-1-(N-4-nitrobenzyloxycarbonylacetimidoyl)pyrrolidin-3-ylaminocarbonyl]-1-(4-nitrobenzyloxycarbonyl) pyrrolidine (prepared as described in Preparation 26), 535 mg of the title compound were obtained as a powder.

### 26(b) (1R,5S,6S)-2-{(2S,4S)-2-[(3S)-1-Acetimidoylpyrrolidin-3-ylaminocarbonyl]pyrrolidin-4-ylthio}-6-[(1R)-1-hydroxyethyl]-1-methyl-1-carbapen-2-em-3-carboxylic acid

Following a procedure similar to that described in Example 23(b), but using 523 mg of 4-nitrobenzyl (1R,5S,6S)-2-{(2S,4S)-2-[(3S)-1-(N-4-nitrobenzyloxycarbonylacetimidoyl)pyrrolidin-3-ylaminocarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above], 128 mg of the title compound were obtained as a powder.
Ultraviolet absorption spectrum (H₂O λₘₐₓ nm:
   299.

### EXAMPLE 27

### (1R,5S,6S)-2-{(2S,4S)-2-[(3R)-1-Formimidoylpyrrolidin-3-ylaminocarbonyl]pyrrolidin-4-ylthio}-6-[(1R)-1-hydroxyethyl]-1-methyl-1-carbapen-2-em-3-carboxylic acid

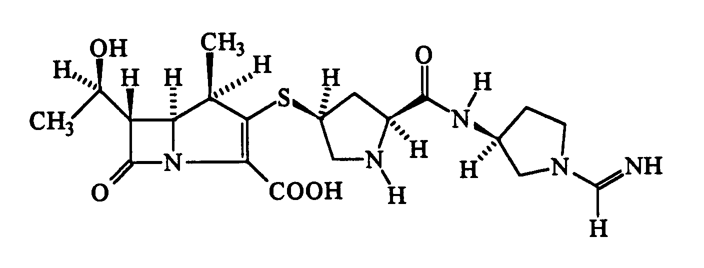

### 27(a) 4-Nitrobenzyl (1R,5S,6S)-2-{(2S,4S)-2-[(3R)-1-(N-4-nitrobenzyloxycarbonylformimidoyl)pyrrolidin-3-yl-aminocarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 23(a), but using 464 mg of 4-nitrobenzyl (1R,5R,6S)-6-[(1R)-1-hydroxyethyl]-1-methyl-2-oxo-1-carbapenam-3-carboxylate and 788 mg of (2S,4S)-4-mercapto-2-[(3R)-1-(N-4-nitrobenzyloxycarbonylformimidoyl)pyrrolidin-3-ylaminocarbonyl]-1-(4-nitrobenzyloxycarbonyl) pyrrolidine (prepared as described in Preparation 27), 548 mg of the title compound were obtained, as a powder.

### 27(b) (1R,5S,6S)-2-{(2S,4S)-2-[(3R)-1-Formimidoylpyrrolidin-3-ylaminocarbonyl]pyrrolidin-4-ylthio}-6-[(1R)-1-hydroxyethyl]-1-methyl-1-carbapen-2-em-3-carboxylic acid

Following a procedure similar to that described in Example 23(b), but using 530 mg of 4-nitrobenzyl (1R,5S,6S)-2-[(2S,4S)-2-[(3R)-1-(N-4-nitrobenzyloxycarbonylformimidoyl)pyrrolidin-3-ylaminocarbonyl]-1-(4-nitrobenzyloxycarbonyl)pyrrolidin-4-ylthio]-6-[(1R)-1-hydroxyethyl]-1-methyl-1-carbapen-2-em-3-carboxylate [prepared as described in step (a) above], 133 mg of the title compound were obtained as a powder.
Ultraviolet absorption spectrum (H₂O) λₘₐₓ nm:
   300.

### EXAMPLE 28

### (1R,5S,6S)-2-{(2S,4S)-2-[N-Methyl-N-((3S)-3-pyrrolidinyl)carbamoyl]pyrrolidin-4-ylthio}-6-[(1R)-1-hydroxyethyl]-1-methyl-1-carbapen-2-em-3-carboxylic acid hydrochloride

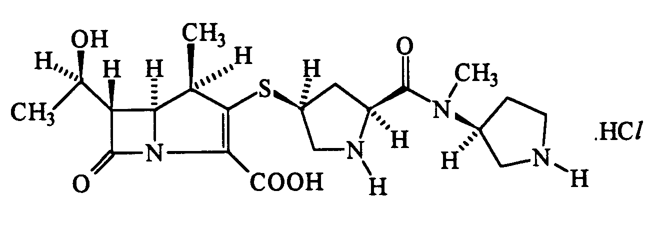

### 28(a) 4-Nitrobenzyl (1R,5S,6S)-2-{(2S,4S)-2-{N-methyl-N-[(3S)-1-(4-nitrobenzyloxycarbonyl)pyrrolidin-3-yl]carbamoyl}-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 19(b), but using 1.09 g of (2S,4S)-4-mercapto-2-{(N-methyl-N-[(3S)-1-(4-nitrobenzyloxycarbonyl)-pyrrolidin-3-yl]carbamoyl}-1-(4-nitrobenzyloxycarbonyl)pyrrolidine instead of the (2S,4S)-4-mercapto-2-[(3S)-1-(4-nitrobenzyloxycarbonyl)pyrrolidin-3-ylaminocarbonyl]-1-(4-nitrobenzyloxycarbonyl)pyrrolidine, 550 mg of the title compound were obtained as a powder.

### 28(b) (1R,5S,6S)-2-{(2S,4S)-2-[N-Methyl-N-((3S)-3-pyrrolidinyl)carbamoyl]pyrrolidin-4-ylthio}-6-[(1R)-1-hydroxyethyl]-1-methyl-1-carbapen-2-em-3-carboxylic acid hydrochloride

500 mg of 4-nitrobenzyl (1R,5S,6S)-2-{(2S,4S)-2-{N-methyl-N-[(3S)-1-(4-nitrobenzyloxycarbonyl) -pyrrolidin-3-yl]carbamoyl}-1-(4-nitrobenzyloxycarbonyl)-pyrrolidin-4-ylthio}-6-[(1R)-1-hydroxyethyl]-1-methyl-1-carbapen-2-em-3-carboxylate [prepared as described in step (a) above] were dissolved in 20 ml of a 1 : 1 by volume mixture of tetrahydrofuran and water, after which 0.45 ml of 1N aqueous hydrochloric acid was added, and the mixture was hydrogenated by bubbling hydrogen through it at room temperature for 2 hours in the presence of 700 mg of 10% w/w palladium-on-charcoal. At the end of this time, the reaction mixture was worked up and purified by the same procedure as described in Example 18(b), to give 30 mg of the title compound as a powder.
Ultraviolet absorption spectrum (H₂O) λₘₐₓ nm:
   297.

### EXAMPLE 29

### (1R,5S,6S)-2-{(2S,4S)-2-[N-Methyl-N-[(3S)-1-formimidoylpyrrolidin-3-yl)carbamoyl]pyrrolidin-4-ylthio}-6-[(1R)-1-hydroxyethyl]-1-methyl-1-carbapen-2-em-3-carboxylic acid

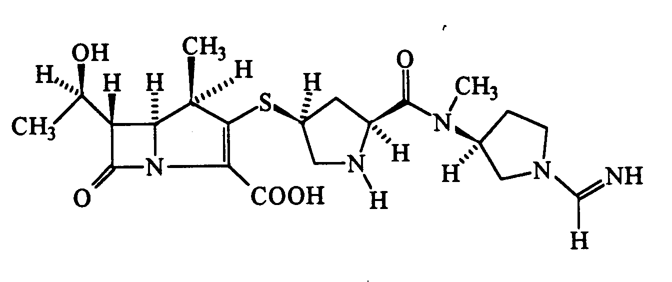

### 29(a) 4-Nitrobenzyl (1R,5S,6S)-2-{(2S,4S)-2-{N-methyl-N-((3S)-1-(N-4-nitrobenzyloxycarbonylformimidoyl)-pyrrolidin-3-yl]carbamoyl}pyrrolidin-4-ylthio}-6-[(1R)-1-hydroxyethyl]-1-methyl-1-carbapen-2-em-3-carboxylate

Following a procedure similar to that described in Example 23(a), but using 400 mg of 4-nitrobenzyl (1R,5R,6S)-6-[(1R)-1-hydroxyethyl]-1-methyl-2-oxo-1-carbapenam-3-carboxylate and 679 mg of (2S,4S)-4-mercapto-2-{N-methyl-N-[(3S)-(N-4-nitrobenzyloxycarbonylformimidoyl)pyrrolidin-3-yl] carbamoyl}-1-(4-nitrobenzyloxycarbonyl)pyrrolidine (prepared as described in Preparation 29), 420 mg of the title compound were obtained as a powder.

### 29(b) (1R,5S,6S)-2-{(2S,4S)-2-[N-Methyl-N-((3S)-1-formimidoylpyrrolidin-3-yl)carbamoyl]pyrrolidin-4-ylthio}-6-[(1R)-1-hydroxethyl]-1-methyl-1-carbapen-2-em-3-carboxylic acid

Following a procedure similar to that described in Example 23(b), but using 410 mg of 4-nitrobenzyl (1R,5S,6S)-2-{(2S,4S)-2-{N-methyl-N-[(3S)-1-(N-4-nitrobenzyloxycarbonylformimidoyl)pyrrolidin-3-yl]-carbamoyl}pyrrolidin-4-ylthio}-6-[(1R)-1-hydroxyethyl]-1-methyl-1-carbapen-2-em-3-carboxylate [prepared as described in step (a) above], 104 mg of the title compound were obtained as a powder.
Ultraviolet absorption spectrum (H₂O) λₘₐₓ nm:
   299.

### EXAMPLE 30

### (1R,5S,6S)-2-[(2S,4S)-2-(3-Carbamoyl-1-piperazinylcarbonyl)pyrrolidin-4-ylthio]-6-[(1R)-1-hydroxyethyl]-1-methyl-1-carbapen-2-em-3-carboxylic acid hydrochloride

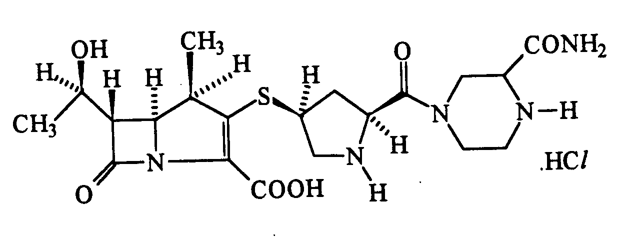

### 30(a) 4-Nitrobenzyl (1R,5S,6S)-2-{(2S,4S)-2-[3-carbamoyl-4-(4-nitrobenzyloxycarbonyl)-1-piperazinyl-carbonyl]-1-(4-nitrobenzyloxycarbonyl) pyrrolidin-4-ylthio}-6-[(1R)-1-hydroxyethyl]-1-methyl-1-carbapen-2-em-3-carboxylate

0.78 ml of diphenylphosphoryl chloride and 0.65 ml of diisopropylethylamine were added dropwise, whilst ice-cooling, to a solution of 1.23 g of 4-nitrobenzyl (1R,5R,6S)-6-[(1R)-1-hydroxyethyl]-1-methyl-2-oxo-1-carbapenam-3-carboxylate in 20 ml of dry acetonitrile, and the resulting mixture was stirred at the same temperature for 1 hour. A solution of 2.11 g of (2S,4S)-4-mercapto-2-[3-carbamoyl-4-(4-nitrobenzyloxycarbonyl]-1-piperazinylcarbonyl]-1-(4-nitrobenzyloxycarbonyl)pyrrolidine (prepared as described in Preparation 30) in 20 ml of dry acetonitrile and 0.59 ml of diisopropylethylamine were then simultaneously added to the mixture, whilst ice-cooling, and the mixture was stirred at the temperature of ice-cooling for 90 minutes and then stirred at room temperature for 90 minutes. At the end of this time, the reaction mixture was worked up and purified by the same procedure as described in Example 7(a), to give 2.38 g of the title compound, as a powder.

### 30(b) (1R,5S,6S)-2-[(2S,4S)-2-(3-Carbamoyl-1-piperazinylcarbonyl)pyrrolidin-4-ylthio]-6-[(1R)-1-hydroxyethyl]-1-methyl-1-carbapen-2-em-3-carboxylic acid hydrochloride

2.33 g of 4-nitrobenzyl (1R,5S,6S)-2-{(2S,4S)-2-[3-carbamoyl-4-(4-nitrobenzyloxycarbonyl)-1-piperazinylcarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 25 ml of a 1 : 1 by volume mixture of tetrahydrofuran and water, after which 2.7 ml of 1N aqueous hydrochloric acid were added, and the mixture was hydrogenated by bubbling hydrogen through it at room temperature for 2 hours in the presence of 2.33 g of 10% w/w palladium-on-charcoal. At the end of this time, the reaction mixture was worked up and purified by the same procedure as described in Example 7(b), to give 160 mg of the title compound, as a powder.
Infrared Absorption Spectrum (KBr), νₘₐₓ cm⁻¹:
   1759, 1698, 1662, 1450, 1383, 1266.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.28 (3H, doublet, J = 6.35 Hz);
   2.04 - 2.10 (1H, multiplet);
   3.01 - 5.08 (16H, multiplet).
Ultraviolet absorption spectrum (H₂O λₘₐₓ nm:
   297.

### EXAMPLE 31

### (1R,5S,6S)-2-{(2S,4S)-2-[4-(2-Fluoroethyl)-1-homopiperazinylcarbonyl]pyrrolidin-4-ylthio}-6-[(1R)-1-hydroxyethyl]-1-methyl-1-carbapen-2-em-3-carboxylic acid hydrochloride

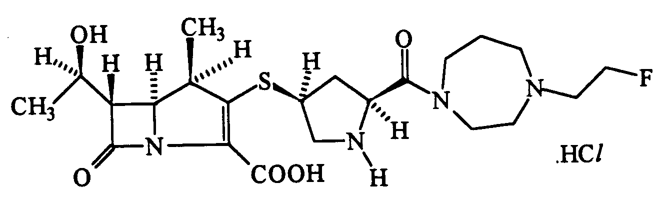

### 31(a) 4-Nitrobenzyl (1R,5S,6S)-2-{(2S,4S)-2-[4-(2-fluoroethyl)-1-homopiperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

1.37 ml of diphenylphosphoryl chloride and 1.15 ml of diisopropylethylamine were added dropwise, whilst ice-cooling, to a solution of 2.23 g of 4-nitrobenzyl (1R,5R,6S)-6-[(1R)-1-hydroxyethyl]-1-methyl-2-oxo-1-carbapenam-3-carboxylate in 23 ml of dry acetonitrile, and the resulting mixture was stirred at the same temperature for 1 hour. A solution of 3.99 g of (2S,4S)-4-mercapto-2-[4-(2-fluoroethyl)-1-homopiperazinylcarbonyl]-1-(4-nitrobenzyloxycarbonyl)pyrrolidine trifluoromethansulphonate (prepared as described in Preparation 31) in 57 ml of dry acetonitrile and 3.40 ml of diisopropylethylamine were then simultaneously added dropwise to the mixture, whilst ice-cooling, and the mixture was stirred at the same temperature for 1 hour, after which it was allowed to stand overnight at the same temperature. At the end of this time, the reaction mixture was worked up and purified by the same procedure as described in Example 15(a), to give 2.96 g of the title compound, as a powder.
Infrared Absorption Spectrum (KBr), νₘₐₓ cm⁻¹:
   1773, 1710, 1647, 1522, 1346, 1206.
Nuclear Magnetic Resonance Spectrum (CDCℓ₃, 270 MHz), δ ppm:
   1.25 - 1.40 (6H, multiplet);
   1.73 - 2.02 (3H, multiplet);
   2.45 - 3.05 (7H, multiplet);
   3.24 - 3.88 (8H, multiplet);
   4.01 - 4.78 (6H, multiplet);
   5.03 - 5.54 (4H, multiplet);
   7.42 - 7.67 (4H, multiplet);
   8.17 - 8.25 (4H, multiplet).

### 31(b) (1R,5S,6S)-2-{(2S,4S)-2-[4-(2-Fluoroethyl)-1-homopiperazinylcarbonyl]pyrrolidin-4-ylthio}-6-[(1R)-1-hydroxyethyl]-1-methyl-1-carbapen-2-em-3-carboxylic acid hydrochloride

500 mg of 4-nitrobenzyl (1R,5S,6S)-2-{(2S,4S)-2-[4-(2-fluoroethyl)-1-homopiperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 12 ml of a 1 : 1 by volume mixture of tetrahydrofuran and water, after which 0.62 ml of 1N aqueous hydrochloric acid was added, and the mixture was hydrogenated by bubbling hydrogen through it at room temperature for 2 hours in the presence of 500 mg of 10% w/w palladium-on-charcoal. At the end of this time, the reaction mixture was worked up and purified by the same procedure as described in Example 15(b), to give 96 mg of the title compound, as a powder.
Infrared Absorption Spectrum (KBr), νₘₐₓ cm⁻¹:
   1763, 1653, 1460, 1384, 1284.
Ultraviolet absorption spectrum (H₂O) λₘₐₓ nm:
   297.
Nuclear Magnetic Resonance Spectrum (D₂O, 270 MHz), δ ppm:
   1.22 (3H, doublet, J = 7.3 Hz);
   1.28 (3H, doublet, J = 6.4 Hz);
   2.00 - 2.12 (1H, multiplet);
   2.22 - 2.38 (2H, multiplet);
   3.02 - 3.16 (1H, multiplet);
   3.36 - 4.16 (15H, multiplet);
   4.20 - 4.30 (2H, multiplet);
   4.76 - 4.98 (3H, multiplet).

### EXAMPLE 32

### (1R,5S,6S)-2-{(2S,4S)-2-[(3S)-3-(Imidazol-1-yl)-pyrrolidin-1-ylcarbonyl]pyrrolidin-4-ylthio}-6-[(1R)-1-hydroxyethyl]-1-methyl-1-carbapen-2-em-3-carboxylic acid hydrochloride

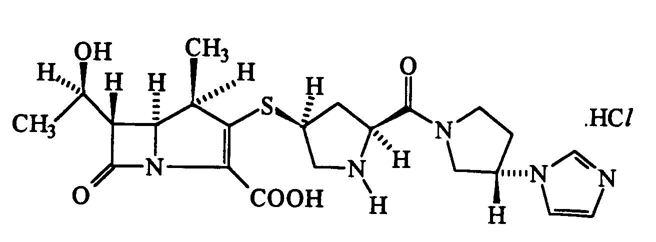

### 32(a) 4-Nitrobenzyl (1R,5S,6S)-2-[(2S,4S)-2-[(3S)-3-(imidazol-1-yl)pyrrolidin-1-ylcarbonyl]-1-(4-nitrobenzyloxycarbonyl)pyrrolidin-4-ylthio] -6-[(1R)-1-hydroxyethyl]-1-methyl-1-carbapen-2-em-3-carboxylate

970 µℓ of diphenylphosphoryl chloride and 810 µℓ of diisopropylethylamine were added dropwise, whilst ice-cooling, to a solution of 1.60 g of 4-nitrobenzyl (1R,5R,6S)-6-[(1R)-1-hydroxyethyl]-1-methyl-2-oxo-1-carbapenam-3-carboxylate in 16 ml of dry acetonitrile, and the resulting mixture was stirred for 30 minutes under the same conditions. A solution of 1.9 g of (2S,4S)-4-mercapto-2-[(3S)-3-(imidazol-1-yl)pyrrolidin-1-ylcarbonyl]-1-(4-nitrobenzyloxycarbonyl)pyrrolidine (prepared as described in Preparation 32) in 22 ml of dry acetonitrile and 770 µℓ of diisopropylethylamine were then simultaneously added dropwise to the mixture, and the mixture was stirred for 2 hours, whilst ice-cooling,, and then allowed to stand overnight at 4°C. At the end of this time, the reaction mixture was diluted with an equivalent amount of water and mixed with 800 mg of sodium hydrogencarbonate. The mixture thus obtained was purified by reverse phase column chromatography through 300 g of Cosmo Sil 75C₁₈-PREP (a trade mark for a product of Nacalai Tesque), using a 1 : 1 by volume mixture of acetonitrile and water as the eluent. Those fractions containing the title compound were combined and concentrated by evaporation under reduced pressure, to give 2.31 g of the title compound, as a powder.
Infrared Absorption Spectrum (KBr), νₘₐₓ cm⁻¹:
   1773, 1709, 1656, 1521, 1346.
Nuclear Magnetic Resonance Spectrum (CDCℓ₃, 270 MHz), δ ppm:
   1.25 - 1.39 (6H, multiplet);
   2.00 - 2.80 (4H, multiplet);
   3.25 - 4.96 (13H, multiplet);
   5.05 - 5.53 (4H, multiplet);
   6.82 - 8.29 (11H, multiplet).

### 32(b) (1R,5S,6S)-2-{(2S,4S)-2-[(3S)-3-(Imidazol-1-yl)pyrrolidin-1-ylcarbonyl]pyrrolidin-4-ylthio}-6-[(1R)-1-hydroxyethyl]-1-methyl-1-carbapen-2-em-3-carboxylic acid hydrochloride

200 mg of 4-nitrobenzyl (1R,5S,6S)-2-{(2S,4S)-2-[(3S)-3-(imidazol-1-yl)pyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in a mixture of 15 ml of tetrahydrofuran and 10 ml of water, and the solution was vigorously stirred for 1.7 hours at a temperature of between 28°C and 30°C in an atmosphere of hydrogen and in the presence of 0.3 g of 10% w/w palladium-on-charcoal. At the end of this time, the catalyst was removed by filtration, and the filtrate was washed three times, each time with 20 ml of diethyl ether. The resulting aqueous solution was concentrated by evaporation under reduced pressure, and the residue was purified by reverse phase column chromatography through 20 ml of Cosmo Sil 75C₁₈-PREP, using water as the eluent. Those fractions containing the title compound were combined and lyophilised, to give 17 mg of the title compound, as a colourless powder.
Ultraviolet absorption spectrum (H₂O) λₘₐₓ nm:
   297.

### EXAMPLE 33

### (1R,5S,6S)-2-{(2S,4S)-2-[(3S)-3-(1,2,4-Triazol-1-yl)pyrrolidin-1-ylcarbonyl]pyrrolidin-4-ylthio]-6-[(1R)-1-hydroxyethyl]-1-methyl-1-carbapen-2-em-3-carboxylic acid hydrochloride

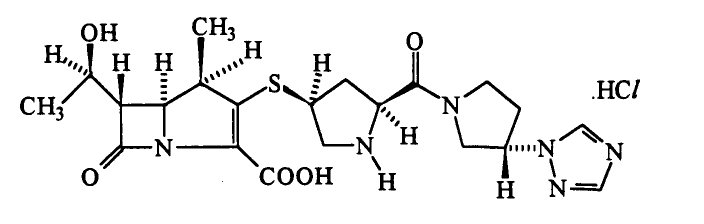

### 33(a) 4-Nitrobenzyl (1R,5S,6S)-2-{(2S,4S)-2-[(3S)-3-(1,2,4-triazol-1-yl)pyrrolidin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

290 µℓ of diphenylphosphoryl chloride and 250 µℓ of diisopropylethylamine were added dropwise, whilst ice-cooling, to a solution of 486 mg of 4-nitrobenzyl (1R,5R,6S)-6-[(1R)-1-hydroxyethyl]-1-methyl-2-oxo-1-carbapenam-3-carboxylate in 5 ml of dry acetonitrile, and the resulting mixture was stirred at the same temperature for 1 hour. A solution of 690 mg of (2S,4S)-4-mercapto-2-[(3S)-3-(1,2,4-triazol-1-yl)-pyrrolidin-1-ylcarbonyl]-1-(4-nitrobenzyloxycarbonyl)pyrrolidine (prepared as described in Preparation 33) in 4 ml of dry acetonitrile and 230 µℓ of diisopropylethylamine were then simultaneously added dropwise to the mixture, and the mixture was stirred at the same temperature for 4 hours. At the end of this time, the reaction mixture was worked up and purified by the same procedure as described in Example 18(a), to give 744 mg of the title compound, as a powder.
Infrared Absorption Spectrum (KBr), νₘₐₓ cm⁻¹:
   3401, 1772, 1709, 1655, 1607, 1522, 1346, 854, 738.
Nuclear Magnetic Resonance Spectrum (hexadeuterated dimethyl sulphoxide, 270 MHz), δ ppm:
   1.14 - 1.20 (6H, multiplet);
   1.50 - 1.70 (1H, multiplet);
   2.30 - 2.50 (1H, multiplet);
   2.70 - 2.95 (1H, multiplet);
   3.10 - 4.30 (14H, multiplet);
   4.51 - 4.70 (1H, multiplet);
   5.05 - 5.49 (5H, multiplet);
   7.48 - 7.74 (4H, multiplet);
   8.15 - 8.27 (4H, multiplet);
   8.52 - 8.63 (1H, multiplet).

### 33(b) (1R,5S,6S)-2-{(2S,4S)-2-[(3s)-3-(1,2,a-Triazol-1-yl)pyrrolidin-1-ylcarbonyl]pyrrolidin-4-ylthio]-6-[(1R)-1-hydroxyethyl]-1-methyl-1-carbapen-2-em-3-carboxylic acid hydrochloride

528 mg of 4-nitrobenzyl (1R,5S,6S)-2-{(2S,4S)-2-[(3S)-3-(1,2,4-triazol-1-yl)pyrrolidin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 10 ml of a 1 : 1 by volume mixture of tetrahydrofuran and water, after which 0.63 ml of 1N aqueous hydrochloric acid was added, and the mixture was hydrogenated by bubbling hydrogen through it at room temperature for 2 hours in the presence of 1 g of 10% w/w palladium-on-charcoal. At the end of this time, the reaction mixture was worked up and purified by the same procedure as described in Example 18(b), to give 85 mg of the title compound, as a powder.
Ultraviolet absorption spectrum (H₂O) λₘₐₓ nm:
   297.

### EXAMPLE 34

### (1R,5S,6S)-2-{(2S,4S)-2-[(3R)-3-Aminopyrrolidin-1-ylcarbonyl]pyrrolidin-4-ylthio}-6-[(1R)-1-hydroxyethyl]-1-methyl-1-carbapen-2-em-3-carboxylic acid hydrochloride

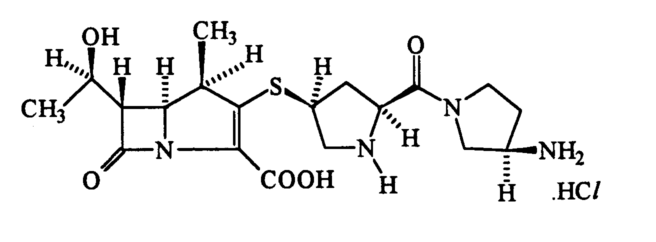

### 34(a) 4-Nitrobenzyl (1R,5S,6S)-2-{(2S,4S)-2-[(3R)-3-(4-nitrobenzyloxycarbonyl)aminopyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 18(a), but using 950 mg of (2S,4S)-4-mercapto-2-[(3R)-3-(4-nitrobenzyloxycarbonyl)aminopyrrolidin-1-ylcarbonyl]-1-(4-nitrobenzyloxycarbonyl) pyrrolidine (prepared as described in Preparation 34) instead of the (2S,4S)-4-mercapto-2-[(3S)-3-(4-nitrobenzyloxycarbonyl)aminopyrrolidin-1-ylcarbonyl]-1-(4-nitrobenzyloxycarbonyl)pyrrolidine, 760 mg of the title compound were obtained, as a powder.

### 34(b) (1R,5S,6S)-2-{(2S,4S)-2-[(3R)-3-aminopyrrolidin-1-ylcarbonyl]pyrrolidin-4-ylthio}-6-[(1R)-1-hydroxyethyl]-1-methyl-1-carbapen-2-em-3-carboxylic acid hydrochloride

730 mg of 4-nitrobenzyl (1R,5S,6S)-2-{(2S,4S)-2-[(3R)-3-(4-nitrobenzyloxycarbonyl)aminopyrrolidin-1-ylcarbonyl] -1-(4-nitrobenzyloxycarbonyl) pyrrolidin-4-yl-thio)-6-[(1R)-1-hydroxyethyl]-1-methyl-1-carbapen-2-em-3-carboxylate [prepared as described in step (a) above] were dissolved in 20 ml of a 1 : 1 by volume mixture of tetrahydrofuran and water, after which 0.75 ml of 1N aqueous hydrochloric acid was added, and the mixture was hydrogenated by bubbling hydrogen through it at room temperature for 2 hours in the presence of 1.0 g of 10% w/w palladium-on-charcoal. At the end of this time, the reaction mixture was worked up and purified by the same procedure as described in Example 18(b), to give 120 mg of the title compound, as a powder.
Ultraviolet absorption spectrum (H₂O) λₘₐₓ nm:
   297.

### EXAMPLE 35

### (1R,5S,6S)-2-{(2S,4S)-2-[(3R)-3-Acetimidoylpyrrolidin-1-ylcarbonyl]pyrrolidin-4-ylthiol}-6-[(1R)-1-hydroxyethyl]-1-methyl-1-carbapen-2-em-3-carboxylic acid

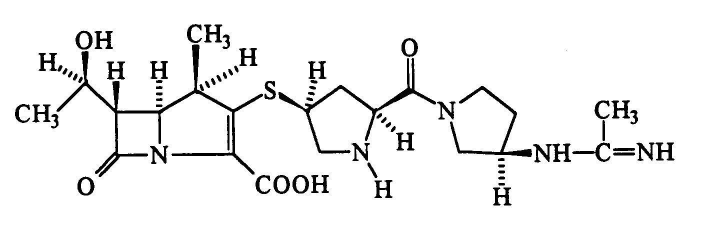

### 35(a) 4-Nitrobenzyl (1R,5S,6S)-2-{(2S,4S)-2-[(3R)-3-(N-4-nitrobenzyloxycarbonylacetimidoylamino)pyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 23(a), but using 92 mg of 4-nitrobenzyl (1R,5R,6S)-6-[(1R)-1-hydroxyethyl]-1-methyl-2-oxo-1-carbapenam-3-carboxylate and 156 mg of (2S,4S)-4-mercapto-2-[(3R)-3-(N-4-nitrobenzyloxycarbonylacetimidoylamino)pyrrolidin-1-ylcarbonyl]-1-(4-nitrobenzyloxycarbonyl) pyrrolidine (prepared as described in Preparation 35), were obtained 125 mg of the title compound, as a powder.

### 35(b) (1R,5S,6S)-2-{(2S,4S)-2-[(3R)-3-Acetimidoylpyrrolidin-1-ylcarbonyl]pyrrolidin-4-ylthio}-6-[(1R)-1-hydroxyethyl]-1-methyl-1-carbapen-2-em-3-carboxylic acid

Following a procedure similar to that described in Example 23(b), but using 120 mg of 4-nitrobenzyl (1R,5S,6S)-2-{(2S,4S)-2-[(3R)-3-(N-4-nitrobenzyloxycarbonylacetimidoylamino)pyrrolidin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above], 31 mg of the title compound were obtained as a powder.
Ultraviolet absorption spectrum (H₂O) λₘₐₓ nm:
   299.
Infrared Absorption Spectrum (KBr), νₘₐₓ cm⁻¹:
   1755, 1631, 1590, 1452, 1386, 1284, 1260.
Nuclear Magnetic Resonance Spectrum (270 MHz, D₂O, using sodium tetradeuterated trimethylsilylpropionate as an internal standard), δ ppm:
   1.22 (3H, doublet, J = 7.33 Hz);
   1.30 (3H, doublet, J = 6.35 Hz);
   1.57 - 1.73 (1H, multiplet);
   2.06 - 2.46 (2H, multiplet);
   2.24 (3H, singlet);
   2.64 - 2.84 (1H, multiplet);
   3.00 - 3.25 (2H, multiplet);
   3.33 - 3.90 (7H, multiplet);
   3.95 - 4.09 (1H, multiplet);
   4.17 - 4.42 (3H, multiplet).

### EXAMPLE 36

### (1R,5S,6S)-2-{(2S,4S)-2-[(3R)-3-Formimidoylaminopyrrolidin-1-ylcarbonyl]pyrrolidin-4-ylthio}-6-[(1R)-1-hydroxyethyl]-1-methyl-1-carbapen-2-em-3-carboxylic acid

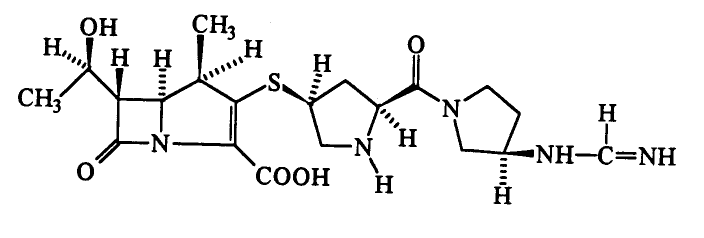

### 36(a) 4-Nitrobenzyl (1R,5S,6S)-2-{(2S,4S)-2-[(3R)-3-(N-4-nitrobenzyloxycarbonylformimidoylamino)pyrrolidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 23(a), but using 120 mg of 4-nitrobenzyl (1R,5R,6S)-6-[(1R)-1-hydroxyethyl]-1-methyl-2-oxo-1-carbapenam-3-carboxylate and 192 mg of (2S,4S)-4-mercapto-2-[(3R)-3-(N-4-nitrobenzyloxycarbonylformimidoylamino)pyrrolidin-1-ylcarbonyl]-1-(4-nitrobenzyloxycarbonyl) pyrrolidine (prepared as described in Preparation 36), were obtained 172 mg of the title compound, as a powder.

### 36(b) (1R,5S,6S)-2-{(2S,4S)-2-[(3R)-3-Formimidoylaminopyrrolidin-1-ylcarbonyl]pyrrolidin-4-ylthio}-6-[(1R)-1-hydroxyethyl]-1-methyl-1-carbapen-2-em-3-carboxylic acid

Following a procedure similar to that described in Example 23(b), but using 165 mg of 4-nitrobenzyl (1R,5S,6S)-2-{(2S,4S)-2-[(3R)-3-(N-4-nitrobenzyloxycarbonylformimidoylamino)pyrrolidin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above], 38 mg of the title compound were obtained as a powder.
Ultraviolet absorption spectrum (H₂O) λₘₐₓ nm:
   300.

### EXAMPLE 37

### (1R,5S,6S)-2-[(2S,4S)-2-(3-Hydroxymethyl-1-piperazinylcarbonyl)pyrrolidin-4-ylthio]-6-[(1R)-1-hydroxyethyl]-1-methyl-1-carbapen-2-em-3-carboxylic acid hydrochloride

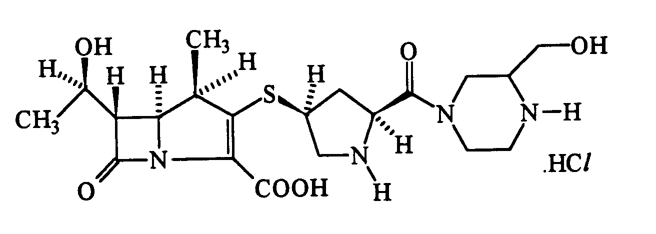

### 37(a) 4-Nitrobenzyl (1R,5S,6S)-2-{(2S,4S)-2-(3-(4-nitrobenzylcarbonyloxymethyl)-4-(4-nitrobenzyloxycarbonyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

46 µℓ of diphenylphosphoryl chloride and 38 λl of diisopropylethylamine were added dropwise, whilst ice-cooling, to a solution of 73 mg of 4-nitrobenzyl (1R,5R,6S)-6-[(1R)-1-hydroxyethyl]-1-methyl-2-oxo-1-carbapenam-3-carboxylate in 1.0 ml of dry acetonitrile, and the resulting mixture was stirred at the same temperature for 1 hour. A solution of 157 mg of (2S,4S)-4-mercapto-2-[3-(4-nitrobenzylcarbonyloxymethyl)4-(4-nitrobenzyloxycarbonyl)-1-piperazinylcarbonyl]-1-(4-nitrobenzyloxycarbonyl) pyrrolidine (prepared as described in Preparation 37) in 1.0 ml of dry acetonitrile and 35 µℓ of diisopropylethylamine were then simultaneously added to the mixture, whilst ice-cooling, and the mixture was stirred at the same temperature for 1 hour, after which it was allowed to stand overnight at the same temperature. At the end of this time, the reaction mixture was worked up and purified by the same procedure as described in Example 7(a) to give 147 mg of the title compound, as a powder.
Infrared Absorption Spectrum (KBr), νₘₐₓ cm⁻¹:
   1758, 1706, 1660, 1608, 1522, 1432, 1347, 1268.
Nuclear Magnetic Resonance Spectrum (CDCℓ₃, 270 MHz), δ ppm:
   1.27 (3H, doublet, J = 7.33 Hz);
   1.37 (3H, doublet, J = 5.86 Hz);
   2.53 - 3.02 (2H, multiplet);
   3.10 - 3.78 (7H, multiplet);
   3.81 - 4.37 (8H, multiplet);
   4.47 - 4.85 (3H, multiplet);
   5.09 - 5.52 (8H, multiplet);
   7.47 (6H, doublet, J = 8.30 Hz);
   7.65 (2H, doublet, J = 8.79 Hz);
   8.15 - 8.23 (8H, multiplet).

### 37(b) (1R,5S,6S)-2-[(2S,4S)-2-(3-Hydroxymethyl-1-piperazinylcarbonyl)pyrrolidin-4-ylthio]-6-[(1R)-1-hydroxyethyl]-1-methyl-1-carbapen-2-em-3-carboxylic acid hydrochloride

140 mg of 4-nitrobenzyl (1R,5S,6S)-2-{(2S,4S)-2-[3-(4-nitrobenzylcarbonyloxymethyl)-4-(4-nitrobenzyloxycarbonyl) -1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 2 ml of a 1 : 1 by volume mixture of tetrahydrofuran and water, after which 0.13 ml of 1N aqueous hydrochloric acid was added, and the mixture was hydrogenated by bubbling hydrogen through it at room temperature for 2 hours in the presence of 140 mg of 10% w/w palladium-on-charcoal. At the end of this time, the reaction mixture was worked up and purified by the same procedure as described in Example 7(b), to give 20 mg of the title compound, as a powder.
Ultraviolet absorption spectrum (H₂O) λₘₐₓ nm:
   297.
Infrared Absorption Spectrum (KBr), νₘₐₓ cm⁻¹:
   1759, 1660, 1594, 1451, 1387, 1266.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.28 (3H, doublet, J = 6.35 Hz);
   1.97 - 2.08 (1H, multiplet);
   3.01 - 3.15 (1H, multiplet);
   3.19 - 3.41 (3H, multiplet);
   3.44 - 3.68 (4H, multiplet);
   3.48 (1H, doublet of doublets, J = 6.35 & 2.93 Hz);
   3.73 - 4.15 (5H, multiplet);
   4.20 - 4.29 (2H, multiplet);
   4.42 - 4.60 (1H, multiplet);
   4.75 - 4.96 (1H, multiplet).

### EXAMPLE 38

### (1R,5S,6S)-2-[(2S,4S)-2-(3-Carboxy-1-piperazinyl carbonyl)pyrrolidin-4-ylthio]-6-[(1R)-1-hydroxyethyl]-1-methyl-1-carbapen-2-em-3-carboxylic acid

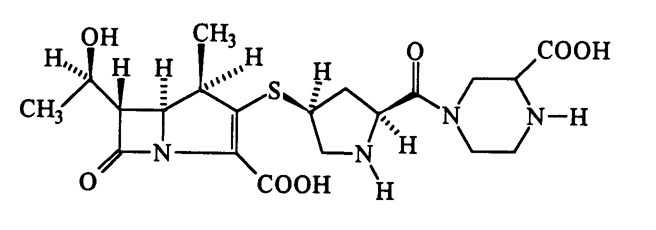

### 38(a) 4-Nitrobenzyl (1R,5S,6S)-2-{(2S,4S)-2-[3-(4-nitrobenzyloxycarbonyl)-4-(4-nitrobenzyloxycarbonyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

55 µℓ of diphenylphosphoryl chloride and 46 µℓ of diisopropylethylamine were added dropwise, whilst ice-cooling, to a solution of 88 mg of 4-nitrobenzyl (1R,5R,6S)-6-[(1R)-1-hydroxyethyl]-1-methyl-2-oxo-1-carbapenam-3-carboxylate in 1.5 ml of dry acetonitrile, and the resulting mixture was stirred at the same temperature for 1 hour. A solution of 184 mg of (2S,4S)-4-mercapto-2-[3-(4-nitrobenzyloxycarbonyl)-4-(4-nitrobenzyloxycarbonyl)-1-piperazinylcarbonyl]-1(4-nitrobenzyloxycarbonyl)pyrrolidine (prepared as described in Preparation 38) in 1.5 ml of dry acetonitrile and 42 µℓ of diisopropylethylamine were then simultaneously added dropwise to the mixture, whilst ice-cooling, and the mixture was allowed to stand overnight at the same temperature. At the end of this time, the reaction mixture was worked up and purified by the same procedure as described in Example 7(a) to give 160 mg of the title compound, as a powder.

### 38(b) (1R,5S,6S)-2-[(2S,4S)-2-(3-Carboxy-1-piperazinylcarbonyl)pyrrolidin-4-ylthio]-6-[(1R)-1-hydroxyethyl]-1-methyl-1-carbapen-2-em-3-carboxylic acid

160 mg of 4-nitrobenzyl (1R,5S,6S)-2-{(2S,4S)-2-[3-(4-nitrobenzyloxycarbonyl)-4-(4-nitrobenzyloxycarbonyl)-1-piperazinylcarbonyl] -1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 3 ml of a 1 : 1 by volume mixture of tetrahydrofuran and water, and the mixture was hydrogenated by bubbling hydrogen through it at room temperature for 2 hours in the presence of 160 mg of 10% w/w palladium-on-charcoal. At the end of this time, the reaction mixture was worked up and purified by the same procedure as described in Example 7(b), to give 35 mg of the title compound, as a powder.
Ultraviolet absorption spectrum (H₂O) λₘₐₓ nm:
   297.

### EXAMPLE 39

### (1R,5S,6S)-2-{(2S,4S)-2-[(3R)-1-Acetimidoylpyrrolidin-3-ylaminocarbonyl]pyrrolidin-4-ylthio}-6-[(1R)-1-hydroxyethyl]-1-methyl-1-carbapen-2-em-3-carboxylic acid

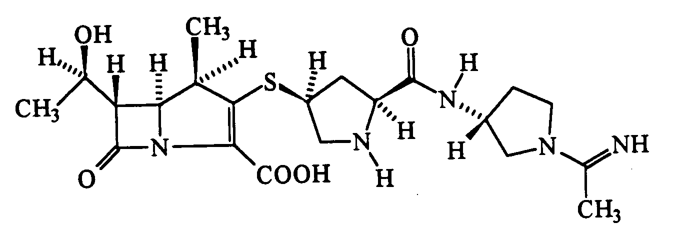

### 39(a) 4-Nitrobenzyl (1R,5S,6S)-2-{(2S,4S)-2-[(3R)-1-(N-4-nitrobenzyloxycarbonylacetimidoyl)pyrrolidin-3-yl-aminocarbonyl]-1-(4-nitrobenzyloxycarbonyl) pyrrolidin-4-ylthio}-6-[(1R)-1-hydroxyethyl]-1-methyl-1-carbapen-2-em-3-carboxylate

Following a procedure similar to that described in Example 23(a), but using 300 mg of 4-nitrobenzyl (1R,5R,6S)-6-[(1R)-1-hydroxyethyl]-1-methyl-2-oxo-1-carbapenam-3-carboxylate and 510 mg of (2S,4S)-4-mercapto-2-[(3R)-1-(N-4-nitrobenzyloxycarbonylacetimidoyl)pyrrolidin-3-ylaminocarbonyl]-1-(4-nitrobenzyloxycarbonyl) pyrrolidine (prepared as described in Preparation 39), 206 mg of the title compound were obtained as a powder.

### 39(b) (1R,5S,6S)-2-{(2S,4S)-2-[(3R)-1-Acetimidoylpyrrolidin-3-ylaminocarbonyl]pyrrolidin-4-ylthio}-6-[(1R)-1-hydroxyethyl]-1-methyl-1-carbapen-2-em-3-carboxylic acid

Following a procedure similar to that described in Example 23(b), but using 198 mg of 4-nitrobenzyl (1R,5S,6S)-2-[(2S,4S)-2-[(3R)-1-(N-4-nitrobenzyloxycarbonylacetimidoyl)pyrrolidin-3-ylaminocarbonyl]-1-(4-nitrobenzyloxycarbonyl)pyrrolidin-4-ylthio]-6-[(1R)-1-hydroxyethyl]-1-methyl-1-carbapen-2-em-3-carboxylate [prepared as described in step (a) above], 48 mg of the title compound were obtained as a powder.
Ultraviolet absorption spectrum (H₂O λₘₐₓ nm:
   298.

### EXAMPLE 40

### (1R,5S,6S)-2-{(2S,4S)-2-[N-(2-Acetimidoylaminoethyl)-carbamoyl]pyrrolidin-4-ylthio}-6-[(1R)-1-hydroxyethyl-1-methyl-1-carbapen-2-em-3-carboxylic acid

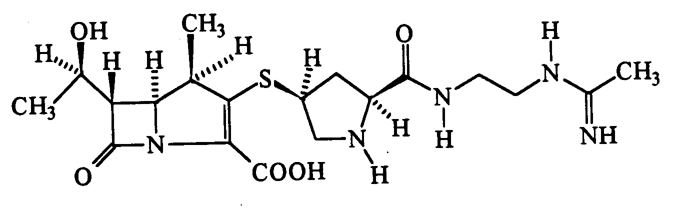

### 40(a) 4-Nitrobenzyl (1R,5S,6S)-2-{2-N-[2-(N-4-nitrobenzyloxycarbonylacetimidoyl)aminoethyl]carbamoyl-1-(4-nitrobenzyloxycarbonyl)pyrrolidin-4-ylthio}-6-[(1R)-1-hydroxyethyl]-1-methy1-1-carbapen-2-em-3-carboxylate

105 µℓ of diphenylphosphoryl chloride and 88 µℓ of diisopropylethylamine were added dropwise, whilst ice-cooling, to a solution of 170 mg of 4-nitrobenzyl (1R,5R,6S)-6-[(1R)-1-hydroxyethyl]-1-methyl-2-oxo-1-carbapenam-3-carboxylate in 2 ml of dry acetonitrile, and the resulting mixture was stirred at the same temperature for 1 hour. A solution of 290 mg of (2S,4S)-4-mercapto-2-{N-[2-(N-4-nitrobenzyloxycarbonylacetimidoyl)aminoethyl]carbamoyl}-1-(4-nitrobenzyloxycarbonyl)pyrrolidine (prepared as described in Preparation 40) in 10 ml of dry acetonitrile and 92 µℓ of diisopropylethylamine were then simultaneously added dropwise to the mixture, and the mixture was allowed to stand overnight at the same temperature. At the end of this time, the reaction mixture was worked up and purified by the same procedure as described in Example 1(a) to give 270 mg of the title compound, as a powder.

### 40(b) (1R,5S,6S)-2-{(2S,4S)-2-[N-(2-Acetimidoylaminoethyl)carbamoyl]pyrrolidin-4-ylthio}-6-[(1R)-1-hydroxyethyl]-1-methyl-1-carbapen-2-em-3-carboxylic acid

265 mg of 4-nitrobenzyl (1R,5S,6S)-2-{N-[2-(N-4-nitrobenzyloxycarbonylacetimidoyl)aminoethyl]carbamoyl-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 15 ml of a 2 : 1 by volume mixture of tetrahydrofuran and water, and the mixture was hydrogenated by bubbling hydrogen through it at room temperature for 2 hours in the presence of 270 mg of 10% w/w palladium-on-charcoal. At the end of this time, the reaction mixture was worked up and purified by the same procedure as described in Example 1(b), to give 60 mg of the title compound, as a powder.
Ultraviolet absorption spectrum (H₂O) λₘₐₓ nm:
   299.
Nuclear Magnetic Resonance Spectrum (D₂O, 270 MHz), δ ppm:
   1.21 (3H, doublet, J = 7.3 Hz);
   1.30 (3H, doublet, J = 6.6 Hz);
   1.68 - 1.70 (1H, multiplet);
   2.23 (3H, singlet);
   2.60 - 2.73 (1H, multiplet);
   2.89 (1H, doublet of doublets, J = 11.2 & 4.0 Hz);
   3.32 - 3.66 (7H, multiplet);
   3.69 - 3.80 (1H, multiplet);
   3.85 (1H, doublet of doublets, J = 9.2 & 5.3 Hz);
   4.18 - 4.32 (2H, multiplet).

### EXAMPLE 41

### (1R,5S,6S)-2-{(2S,4S)-2-[N-(2-Formimidoylaminoethyl)carbamoyl]pyrrolidin-4-ylthio}-6-[(1R)-1-hydroxyethyl]-1-methyl-1-carbapen-2-em-3-carboxylic acid

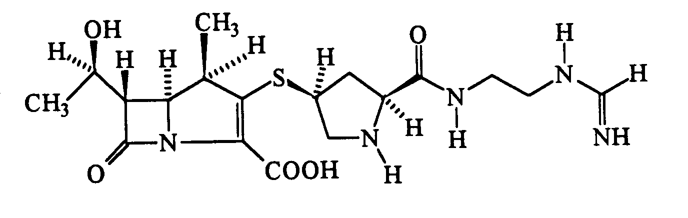

### 41(a) 4-Nitrobenzyl (1R,5S,6S)-2-{2-N-[2-(4-nitrobenzyloxycarbonylformimidoyl)aminoethyl]carbamoyl-1-(4-nitrobenzyloxycarbonyl)pyrrolidin-4-ylthio}-6-[(1R)-1-hydroxyethyl]-1-methyl-1-carbapen-2-em-3-carboxylate

53 µℓ of diphenylphosphoryl chloride and 44 µℓ of diisopropylethylamine were added dropwise, whilst ice-cooling, to a solution of 90 mg of 4-nitrobenzyl (1R,5R,6S)-6-[(1R)-1-hydroxyethyl]-1-methyl-2-oxo-1-carbapenam-3-carboxylate in 1 ml of dry acetonitrile, and the resulting mixture was stirred at the same temperature for 1 hour. A solution of 150 mg of (2S,4S)-4-mercapto-2-{N-[2-(N-4-nitrobenzyloxycarbonylformimidoyl)aminoethyl]carbamoyl}-1-(4-nitrobenzyloxycarbonyl)pyrrolidine (prepared as described in Preparation 41) in 10 ml of dry acetonitrile and 50 µℓ of diisopropylethylamine were then simultaneously added dropwise to the mixture, and the mixture was allowed to stand overnight at the same temperature. At the end of this time, the reaction mixture was worked up and purified by the same procedure as described in Example 1(a), to give 155 mg of the title compound, as a powder.

### 41(b) (1R,5S,6S)-2-{(2S,4S)-2-[N-(2-Formimidoylaminoethyl)carbamoyl]pyrrolidin-4-ylthio}-6-[(1R)-1-hydroxyethyl]-1-methyl-1-carbapen-2-em-3-carboxylic acid

150 mg of 4-nitrobenzyl (1R,5S,6S)-2-{N-[2-(4-nitrobenzyloxycarbonylformimidoyl)aminoethyl]carbamoyl-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 10 ml of a 2 : 1 by volume mixture of tetrahydrofuran and water, and the mixture was hydrogenated by bubbling hydrogen through it at room temperature for 2 hours in the presence of 150 mg of 10% w/w palladium-on-charcoal. At the end of this time, the reaction mixture was worked up and purified by the same procedure as described in Example 1(b), to give 20 mg of the title compound as a powder.
Ultraviolet absorption spectrum (H₂O) λₘₐₓ nm:
   299.
Nuclear Magnetic Resonance Spectrum (270 MHz, D₂O, using sodium tetradeuterated trimethylsilylpropionate as an internal standard), δ ppm:
   1.21 (3H, doublet, J = 7.3 Hz);
   1.30 (3H, doublet, J = 6.4 Hz);
   1.70 - 1.84 (1H, multiplet);
   2.60 - 2.76 (1H, multiplet);
   2.89 - 2.97 (1H, multiplet);
   3.32 - 3.62 (7H, multiplet);
   3.70 - 3.81 (1H, multiplet);
   3.85 - 3.92 (1H, multiplet);
   4.19 - 4.29 (2H, multiplet);
   7.84 & 7.85 (1H, two singlets).
Infrared Absorption Spectrum (KBr), νₘₐₓ cm⁻¹:
   1755, 1717, 1660, 1590, 1388.

### EXAMPLE 42

### (1R,5S,6S)-2-[(2S,4S)-2-(3-Dimethylamino-1,2,5,6-tetrahydropyrazin-1-ylcarbonyl)pyrrolidin-4-ylthio]-6-[(1R)-1-hydroxyethyl]-1-methyl-1-carbapen-2-em-3-carboxylic acid

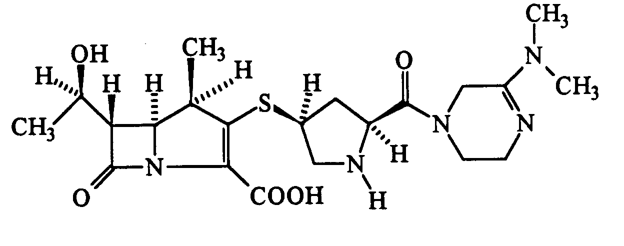

### 42(a) 4-Nitrobenzyl (1R,5S,6S)-2-[(2S,4S)-2-(3-dimethylamino-1,2,5,6-tetrahydropyrazin-1-ylcarbonyl)-1-(4-nitrobenzyloxycarbonyl)pyrrolidin-4-ylthio]-6-[(1R)-1-hydroxyethyl]-1-methyl-1-carbapen-2-em-3-carboxylate

126 µℓ of diphenylphosphoryl chloride and 106 µℓ of diisopropylethylamine were added dropwise, whilst ice-cooling, to a solution of 208 mg of 4-nitrobenzyl (1R,5R,6S)-6-[(1R)-1-hydroxyethyl]-1-methyl-2-oxo-1-carbapenam-3-carboxylate in 2.6 ml of dry acetonitrile, and the resulting mixture was stirred at the same temperature for 1 hour. A solution of 445 mg of (2S,4S)-4-mercapto-2-(3-dimethylamino-1,2,5,6-tetrahydropyrazin-1-ylcarbonyl)-1-(4-nitrobenzyloxycarbonyl)-pyrrolidine trifluoromethanesulphonate (prepared as described in Preparation 42) in 2.4 ml of dry acetonitrile and 271 µℓ of diisopropylethylamine were then simultaneously added dropwise to the mixture, and the mixture was allowed to stand overnight at the same temperature. At the end of this time, the reaction mixture was worked up and purified by the same procesure as described in Example 1(a), to give 98 mg of the title compound, as a powder.
Infrared Absorption Spectrum (KBr), νₘₐₓ cm ⁻¹:
   1771, 1709, 1659, 1608, 1522, 1495, 1441, 1405, 1346, 1277, 1209.
Nuclear Magnetic Resonance Spectrum (CDCℓ₃ + hexadeuterated dimethyl sulphoxide, 270 MHz), δ ppm:
   1.28 (3H, doublet, J = 7.32 Hz);
   1.34 (3H, doublet, J = 6.34 Hz);
   1.85 - 2.08 (1H, multiplet);
   2.71 - 2.86 (1H, multiplet);
   3.08 - 4.85 (20H, multiplet);
   5.05 - 5.53 (4H, multiplet);
   7.42 - 7.58 (2H, multiplet);
   7.66 (2H, doublet, J = 8.79 Hz);
   8.16 - 8.26 (4H, multiplet).

### 42(b) (1R,5S,6S)-2-[(2S,4S)-2-(3-Dimethylamino-1,2,5,6-tetrahydropyrazin-1-ylcarbonyl)pyrrolidin-4-ylthio]-6-[(1R)-1-hydroxyethyl]-1-methyl-1-carbapen-2-em-3-carboxylic acid

95 mg of 4-nitrobenzyl (1R,5S,6S)-2-[(2S,4S)-2-(3-dimethylamino-1,2,5,6-tetrahydropyrazin-1-ylcarbonyl)-1-(4-nitrobenzyloxycarbonyl) pyrrolidin-4-ylthio]-6-(1R)-1-hydroxyethyl]-1-methyl-1-carbapen-2-em-3-carboxylate [prepared as described in step (a) above] were dissolved in 5 ml of a 2 : 1 by volume mixture of tetrahydrofuran and water, and the mixture was hydrogenated by bubbling hydrogen through it at room temperature for 2 hours in the presence of 250 mg of 10% w/w palladium-on-charcoal. At the end of this time, the reaction mixture was worked up and purified by the same procedure as described in Example 1(b), to give 8 mg of the title compound as a powder.
Ultraviolet absorption spectrum (H₂O) λₘₐₓ nm:
   299.
Infrared Absorption Spectrum (KBr), νₘₐₓ cm⁻¹:
   1751, 1659, 1598, 1455, 1391, 1346, 1247.
Nuclear Magnetic Resonance Spectrum (270 MHz, D₂O, using sodium tetradeuterated trimethylsilylpropionate as an internal standard), δ ppm:
   1.22 (3H, doublet, J = 7.33 Hz);
   1.30 (3H, doublet, J = 6.35 Hz);
   1.60 - 1.83 (1H, multiplet);
   2.68 - 2.92 (1H, multiplet);
   3.03 - 4.43 (20H, multiplet).

### EXAMPLE 43

### (1R,5S,6S)-2-[(2S,4S)-2-(4-Acetimidoylaminopiperidin-1-ylcarbonyl)pyrrolidin-4-ylthio]-6-[(1R)-1-hydroxyethyl]-1-methyl-1-carbapen-2-em-3-carboxylic acid

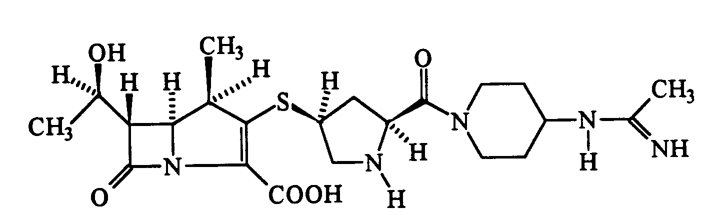

Following a procedure similar to that described in Example 1, but using 181 mg of 4-nitrobenzyl (1R,5R,6S)-6-[(1R)-1-hydroxyethyl]-1-methyl-2-oxo-1-carbapenam-3-carboxylate and 300 mg of (2S,4S)-4-mercapto-2-(4-nitrobenzyloxycarbonylacetimidoyl)aminopiperidin-1-ylcarbonyl]-1-(4-nitrobenzyloxycarbonyl)pyrrolidine (prepared as described in Preparation 43), 21 mg of the title compound were obtained, as a powder.
Ultraviolet absorption spectrum (H₂O) λₘₐₓ nm:
   299.
Nuclear Magnetic Resonance Spectrum (270 MHz, D₂O, using sodium tetradeuterated trimethylsilylpropionate as an internal standard), δ ppm:
   1.21 (3H, doublet, J = 7.3 Hz);
   1.30 (3H, doublet, J = 6.4 Hz);
   1.46 - 1.70 (3H, multiplet);
   2.02 - 2.18 (2H, multiplet);
   2.22 (3H, singlet);
   2.69 - 2.83 (1H, multiplet);
   2.90 - 3.04 (1H, multiplet);
   3.06 - 3.46 (5H, multiplet);
   3.78 - 3.99 (3H, multiplet);
   4.12 - 4.44 (4H, multiplet).

### EXAMPLE 44

### (1R,5S,6S)-2-[(2S,4S)-2-(4-Acetimidoylpiperazin-1-ylcarbonyl)-1-methylpyrolidin-4-ylthio]-6-[(1R)-1-hydroxyethyl]-1-methyl-1-carbapen-2-em-3-carboxylic acid

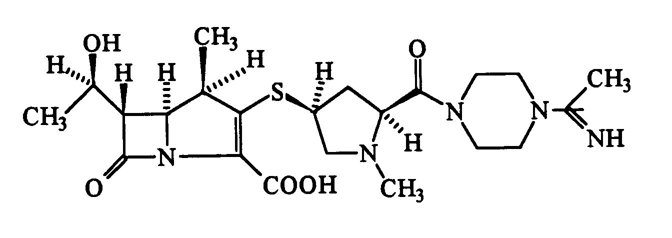

Following a procedure similar to that described in Example 1, but using 181 mg of 4-nitrobenzyl (1R,5R,6S) 6-[(1R)-1-hydroxyethyl]-1-methyl-2-oxo-1-carbapenam-3-carboxylate and 220 mg of (2S,4S)-4-mercapto-2-[4-(N-4-nitrobenzyloxycarbonylacetimidoyl)piperazin-1-ylcarbonyl]-1-methylpyrrolidine (prepared as described in Preparation 44), 20 mg of the title compound were obtained, as a powder.
Ultraviolet absorption spectrum (H₂O) λₘₐₓ nm:
   299.
Nuclear Magnetic Resonance Spectrum (270 MHz, D₂O, using sodium tetradeuterated trimethylsilylpropionate as an internal standard), δ ppm:
   1.21 (3H, doublet, J = 7.3 Hz);
   1.30 (3H, doublet, J = 6.4 Hz);
   1.66 (1H, doubled doublet of doublets, J = 13.7, 8.8 & 5.4 Hz);
   2.29 (3H, singlet);
   2.35 (3H, singlet);
   2.75 - 2.88 (2H, multiplet);
   3.10 (1H, doublet of doublets, J = 12.2 & 1.4 Hz);
   3.31 - 3.43 (2H, multiplet);
   3.52 (1H, triplet, J = 8.3 Hz);
   3.60 - 4.00 (9H, multiplet);
   4.18 - 4.30 (2H, multiplet).
Infrared Absorption Spectrum (KBr), νₘₐₓ cm⁻¹:
   1754, 1608, 1594, 1448, 1384, 1285.

### EXAMPLE 45

### (1R,5S,6S)-2-[(2S,4S)-2-[(3S)-3-Aminopyrrolidin-1-ylcarbonyl]-1-methylpyrrolidin-4-ylthio]-6-[(1R)-1-hydroxyethyl]-1-methyl-1-carbapen-2-em-3-carboxylic acid hydrochloride

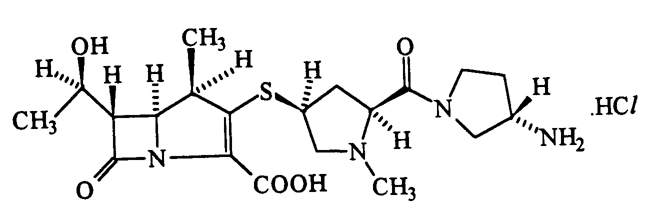

### 45(a) 4-Nitrobenzyl (1R,5S,6S)-2-{(2S,4S)-2-[(3S)-3-(4-nitrobenzyloxycarbonyl)aminopyrrolidin-1-ylcarbonyl]-1-methylpyrrolidin-4-ylthio}-6-[(1R)-1-hydroxyethyl]-1-methyl-1-carbapen-2-em-3-carboxylate

Following a procedure similar to that described in Example 18(a), but using 1.05 g of (2S,4S)-4-mercapto-2-[(3S)-3-(4-nitrobenzyloxycarbonyl)aminopyrrolidin-1-ylcarbonyl]-1-methylpyrrolidine instead of the (2S,4S)-4-mercapto-2-[(3S)-3-(4-nitrobenzyloxycarbonyl)amino-pyrrolidin-1-ylcarbonyl]-1-(4-nitrobenzyloxycarbonyl) pyrrolidine, 1.20 g of the title compound was obtained as a powder.

### 45(b) (1R,5S,6S)-2-[(2S,4S)-2-[(3S)-3-Aminopyrrolidin-1-ylcarbonyl]-1-methylpyrrolidin-4-ylthio]-6-[(1R)-1-hydroxyethyl]-1-methyl-1-carbapen-2-em-3-carboxylic acid hydrochloride

1.0 g of 4-nitrobenzyl (1R,5S,6S)-2-{(2S,4S)-2-[(3S)-3-(4-nitrobenzyloxycarbonyl)aminopyrrolidin-1-ylcarbonyl] -1-methylpyrrolidin-4-ylthio}-6-[(1R)-1-hydroxyethyl]-1-methyl-1-carbapen-2-em-3-carboxylate [prepared as described in step (a) above] was dissolved in 30 ml of a 2 : 1 by volume mixture of tetrahydrofuran and water, after which 1.04 ml of 1N aqueous hydrochloric acid was added, and the mixture was hydrogenated by bubbling hydrogen through it at room temperature for 2 hours in the presence of 1.5 g of 10% w/w palladium-on-charcoal. At the end of this time, the reaction mixture was worked up and purified by the same procedure as described in Example 18(b), to give 175 mg of the title compound as a powder.
Ultraviolet absorption spectrum (H₂O) λₘₐₓ nm:
   297.
Infrared Absorption Spectrum (KBr), νₘₐₓ cm⁻¹:
   3390, 1760, 1655, 1599, 1467, 1374.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.29 (3H, doublet, J = 6.35 Hz);
   1.95 - 2.30 (1H, multiplet);
   2.30 - 2.70 (2H, multiplet);
   2.96 (3H, doublet, J = 2.93 Hz);
   3.15 - 3.27 (1H, multiplet);
   3.27 - 3.40 (1H, multiplet);
   3.46 - 3.49 (1H, multiplet);
   3.50 - 4.35 (10H, multiplet);
   4.45 - 4.65 (1H, multiplet).

### EXAMPLE 46

### (1R,5S,6S)-2-{(2S,4S)-2-[(3S)-3-Formimidoylaminopyrrolidin-1-ylcarbonyl]-1-methylpyrrolidin-4-ylthio}-6-[(1R)-1-hydroxyethyl]-1-methyl-1-carbapen-2-em-3-carboxylic acid

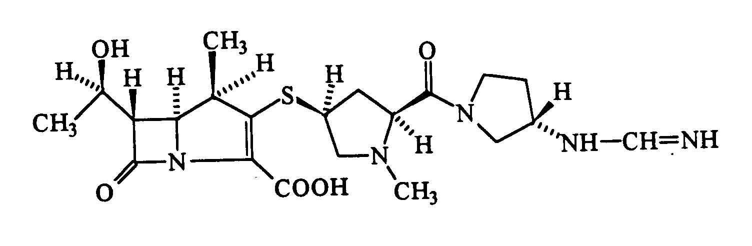

### 46(a) 4-Nitrobenzyl (1R,5S,6S)-2-{(2S,4S)-2-[(3S)-3-(N-4-nitrobenzyloxycarbonylformimidoylamino)pyrrolidin-1-ylcarbonyl]-1-methylpyrrolidin-4-ylthio}-6-[(1R)-1-hydroxyethyl]-1-methyl-1-carbapen-2-em-3-carboxylate

Following a procedure similar to that described in Example 23(a), but using 124 mg of 4-nitrobenzyl (1R,5R,6S)-6-[(1R)-1-hydroxyethyl]-1-methyl-2-oxo-1-carbapenam-3-carboxylate and 190 mg of (2S,4S)-4-mercapto-2-[(3S)-3-(N-4-nitrobenzyloxycarbonylformimidoylamino)pyrrolidin-1-ylcarbonyl]-1-methylpyrrolidine (prepared as described in Preparation 46), 178 mg of the title compound were obtained as a powder.

### 46(b) (1R,5S,6S)-2-{(2S,4S)-2-[(3S)-3-Formimidoylaminopyrrolidin-1-ylcarbonyl]-1-methylpyrrolidin-4-ylthio}-6-[(1R)-1-hydroxyethyl]-1-methyl-1-carbapen-2-em-3-carboxylic acid

Following a procedure similar to that described in Example 23(b), but using 170 mg of 4-nitrobenzyl (1R,5S,6S)-2-{(2S,4S)-2-[(3S)-3-(N-4-nitrobenzyloxycarbonylformimidoylamino)pyrrolidin-1-ylcarbonyl]-1-methylpyrrolidin-4-ylthio}-6-[(1R)-1-hydroxyethyl]-1-methyl-1-carbapen-2-em-3-carboxylate [prepared as described in step (a) above], 35 mg of the title compound were obtained as a powder.
Ultraviolet absorption spectrum (H₂O) λₘₐₓ nm: 298
Infrared Absorption Spectrum (KBr), νₘₐₓ cm⁻¹:
   3255, 1755, 1634, 1595, 1455, 1386.
Nuclear Magnetic Resonance Spectrum (400 MHz, D₂O, using sodium tetradeuterated trimethylsilylpropionate as an internal standard), δ ppm:
   1.21 (3H, doublet, J = 7.32 Hz);
   1.31 (3H, doublet, J = 6.60 Hz);
   1.60 - 1.75 (1H, multiplet);
   2.30 (3H, doublet, J = 5.86 Hz);
   2.05 - 2.50 (2H, multiplet);
   2.75 - 2.95 (2H, multiplet);
   3.05 - 3.15 (1H, multiplet);
   3.30 - 3.50 (3H, multiplet);
   3.50 - 3.95 (4H, multiplet);
   3.96 - 4.05 (1H, multiplet);
   4.20 (1H, doublet of doublets, J = 2.57 & 9.17 Hz);
   4.26 (1H, quartet, J = 6.40 Hz);
   4.30 - 4.47 (1H, multiplet);
   7.80, 7.81 & 7.94 (together 1H, three singlets).

### EXAMPLE 47

### (1R,5S.6S)-2-{(2S,4S)-2-[4-(Imidazol-1-yl)piperidin-1-ylcarbonyl]pyrrolidin-4-ylthio}-6-[(1R)-1-hydroxyethyl]-1-methyl-1-carbapen-2-em-3-carboxylic acid hydrochloride

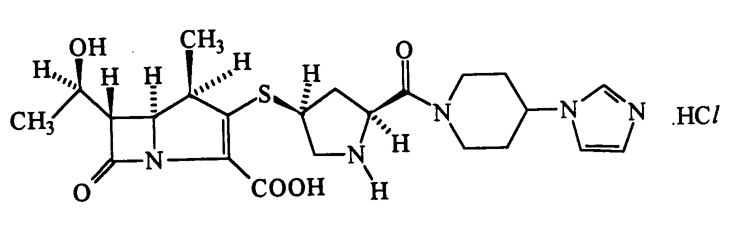

### 47(a) 4-Nitrobenzyl (1R,5S,6S)-2-{(2S,4S)-2-[4-(imidazol-1-yl)piperidin-1-ylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate

560 µℓ of diphenylphosphoryl chloride and 470 µℓ of diisopropylethylamine were added dropwise, whilst ice-cooling, to a solution of 910 mg of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred for 30 minutes under the same conditions. A solution of 1140 mg of (2S,4S)-4-mercapto-2-[4-(imidazol-1-yl)piperidin-1-ylcarbonyl]-1-(4-nitrobenzyloxycarbonyl)pyrrolidine (prepared as described in Preparation 47) in 10 ml of dry acetonitrile and 435 µℓ of diisopropylethylamine were then simultaneously added dropwise to the mixture, and the mixture was stirred for 2 hours, whilst ice-cooling, after which it was allowed to stand overnight at 4°C. At the end of this time, the reaction mixture was diluted with an equivalent amount of water and mixed with 800 mg of sodium hydrogencarbonate. The mixture thus obtained was purified by reverse phase column chromatography through 200 ml of Cosmo Sil 75C₁₈-PREP (a trade mark for a product of Nacalai Tesque) using a 1 : 1 by volume mixture of acetonitrile and water as the eluent. Those fractions containing the title compound were combined and concentrated to give 1.40 g of the title compound, as a powder.
Nuclear Magnetic Resonance Spectrum (hexadeuterated dimethyl sulphoxide, 270 MHz), δ ppm:
   1.12 - 1.20 (6H, multiplet);
   1.58 - 1.90 (3H, multiplet);
   1.91 - 2.06 (2H, multiplet);
   2.62 - 2.79 (1H, multiplet);
   2.80 - 2.97 (1H, multiplet);
   3.06 - 3.37 (4H, multiplet);
   3.55 - 3.70 (1H, multiplet);
   3.71 - 3.93 (1H, multiplet);
   3.94 - 4.56 (5H, multiplet);
   4.74 - 4.97 (1H, multiplet);
   5.04 - 5.49 (5H, multiplet);
   6.81 - 8.28 (11H, multiplet).
Infrared Absorption Spectrum (KBr), νₘₐₓ cm⁻¹:
   1773, 1710, 1656, 1522, 1346, 1208.

### 47(b) (1R,5S,6S)-2-{(2S,4S)-2-[4-(Imidazol-1-yl)piperidin-1-ylcarbony]pyrrolidin-4-ylthio}-6-[(1R)-1-hydroxyethyl]-1-methyl-1-carbapen-2-em-3-carboxylic acid hydrochloride

200 mg of 4-nitrobenzyl (1R,5S,6S)-2-{(2S,4S)-2-[4-(imidazol-1-yl)piperidin-1-ylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in a mixture of 15 ml of tetrahydrofuran and 10 ml of water, and the solution was vigorously stirred at a temperature of between 28°C and 30°C for 1.7 hours in an atmosphere of hydrogen and in the presence of 0.3 g of 10% w/w palladium-on-charcoal. At the end of this time, the catalyst was removed by filtration, and the filtrate was washed three times, each time with 20 ml of diethyl ether. The resulting aqueous solution was concentrated by evaporation under reduced pressure. The concentrate was purified by reverse phase column chromatography through 20 ml of Cosmo Sil 75C₁₈-PREP (a trade mark for a product of Nacalai Tesque), using water as the eluent. Those fractions containing the title compound were combined and lyophilised, to give 18 mg of the title compound as a colourless powder.
Ultraviolet absorption spectrum (H₂O) λₘₐₓ nm:
   297.

### EXAMPLE 48

### (1R,5S,6S)-2-[(2S,4S)-2-(3,3-Dimethyl-1-piperazinylcarbonyl)pyrrolidin-4-ylthio]-6-[(1R)-1-hydroxyethyl]-1-methyl-1-carbapen-2-em-3-carboxylic acid hydrochloride

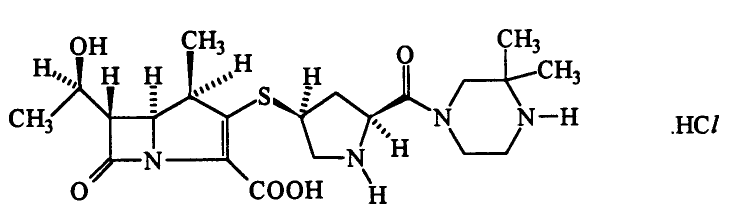

### 48(a) 4-Nitrobenzyl (1R,5S,6S)-2-{(2S,4S)-2-[3,3-dimethyl-4-(4-nitrobenzyloxycarbonyl)-1-piperazinyl-carbonyl]-1-(4-nitrobenzyloxycarbonyl) pyrrolidin-4-ylthio}-6-[(1R)-1-hydroxyethyl]-1-methyl-1-carbapen-2-em-3-carboxylate

67 µℓ of diphenylphosphoryl chloride and 56 µℓ of diisopropylethylamine were added dropwise, whilst ice-cooling, to a solution of 100 mg of 4-nitrobenzyl (1R,5R,6S)-6-[(1R)-1-hydroxyethyl]-1-methyl-2-oxo-1-carbapenam-3-carboxylate in 1.5 ml of dry acetonitrile, and the resulting mixture was stirred at the same temperature for 1 hour. A solution of 200 mg of (2S,4S)-4-mercapto-2-[3,3-dimethyl-4-(4-nitrobenzyloxycarbonyl)-1-piperazinylcarbonyl]-1-(4-nitrobenzyloxycarbonyl)pyrrolidine (prepared as described in Preparation 48) in 1.5 ml of dry acetonitrile and 56 µℓ of diisopropylethylamine were then simultaneously added dropwise to the mixture, and the mixture was stirred at the same temperature for 1 hour, after which it was allowed to stand overnight at the same temperature. At the end of this time, the reaction mixture was worked up and purified by the same procedure as described in Example 7(a), to give 205 mg of the title compound, as a powder.

### 48(b) (1R,5S,6S)-2-[(2S,4S)-2-(3,3-Dimethyl-1-piperazinylcarbonyl)pyrrolidin-4-ylthio]-6-[(1R)-1-hydroxyethyl]-1-methyl-1-carbapen-2-em-3-carboxylic acid hydrochloride

205 mg of 4-nitrobenzyl (1R,5S,6S)-2-{(2S,4S)-2-[3,3-dimethyl-4-(4-nitrobenzyloxycarbonyl)-1-piperazinylcarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 5 ml of a 1 : 1 by volume mixture of tetrahydrofuran and water, after which 0.23 ml of 1N aqueous hydrochloric acid was added, and the mixture was hydrogenated by bubbling hydrogen through it at room temperature for 2 hours in the presence of 200 mg of 10% w/w palladium-on-charcoal. At the end of this time, the reaction mixture was worked up and purified by the same procedure as described in Example 7(b), to give 35 mg of the title compound as a powder.
Ultraviolet absorption spectrum (H₂O) λₘₐₓ nm:
   296.

### EXAMPLE 49

### (1R,5S,6S)-2-[(2S,4S)-2-(1-Homopiperazinylcarbonyl)-1-methylpyrrolidin-4-ylthio]-6-[(1R)-1-hydroxyethyl]-1-methyl-1-carbapen-2-em-3-carboxylic acid hydrochloride

### 49(a) 4-Nitrobenzyl (1R,5S,6S)-2-[(2S,4S)-2-[4-(4-nitrobenzyloxycarbonyl)-1-homopiperazinylcarbonyl]-1-methylpyrrolidin-4-ylthio]-6-[(1R) -1-hydroxyethyl]-1-methyl-1-carbapen-2-em-3-carboxylate

0.89 ml of diphenylphosphoryl chloride and 0.75 ml of diisopropylethylamine were simultaneously added dropwise, whilst ice-cooling, to a solution of 1.4 g of 4-nitrobenzyl (1R,5R,6S)-6-[(1R)-1-hydroxyethyl]-1-methyl-2-oxo-1-carbapenam-3-carboxylate in 14 ml of dry acetonitrile, and the resulting mixture was stirred at the same temperature for 40 minutes. A solution of 2.68 g of (2S,4S)-4-mercapto-1-methyl-2-[4-(4-nitrobenzyloxycarbonyl)-1-homopiperazinylcarbonyl]pyrrolidine trifluoromethanesulphonate (prepared as described in Preparation 49) in 14 ml of dry acetonitrile and 1.64 ml of diisopropylethylamine were then simultaneously added dropwise to the mixture, whilst ice-cooling, and the resulting mixture was stirred at the same temperature for 5 hours, after which it was allowed to stand overnight at room temperature. At the end of this time, the reaction mixture was concentrated by evaporation under reduced pressure, and the residue was diluted with ethyl acetate. The dilute solution was then washed with an aqueous solution of sodium hydrogencarbonate, with a phosphate buffer solution (pH 6.86), with water and with an aqueous solution of sodium chloride, in that order, after which it was dried over anhydrous magnesium sulphate. The solvent was then removed by distillation under reduced pressure, and the resulting residue was purified by column chromatography through silica gel (Merck Art No. 7734), using a gradient elution method with mixtures of ethyl acetate and methanol ranging from 85 : 15 to 80 : 20 by volume as the eluent, to give 1.7 g of the title compound, as a powder.
Infrared Absorption Spectrum (KBr), νₘₐₓ cm⁻¹:
   1769, 1702, 1642, 1521, 1346.
Nuclear Magnetic Resonance Spectrum (CDCℓ₃, 270 MHz), δ ppm:
   1.27 (3H, doublet, J = 7.32 Hz);
   1.35 (3H, doublet, J = 6.35 Hz);
   1.40 - 2.05 (6H, multiplet);
   2.32 - 2.85 (3H, multiplet);
   3.15 - 4.05 (13H, multiplet);
   4.20 - 4.37 (2H, multiplet);
   5.20 - 5.52 (4H, multiplet);
   7.47 - 7.53 (2H, multiplet);
   7.67 (2H, doublet, J = 8.79 Hz);
   8.23 (4H, doublet, J = 8.79 Hz).

### 49(b) (1R,5S,6S)-2-[(2S,4S)-2-(1-Homopiperazinylcarbonyl)-1-methylpyrrolidin-4-ylthio]-6-[(1R)-1-hydroxyethyl]-1-methyl-1-carbapen-2-em-3-carboxylic acid hydrochloride

A solution of 1.3 g of 4-nitrobenzyl (1R,5S,6S)-2-[(2S,4S)-2-[4-(4-nitrobenzyloxycarbonyl)-1-homopiperazinylcarbonyl] -1-methylpyrrolidin-4-ylthio]-6-[(1R)-1-hydroxyethyl]-1-methyl-1-carbapen-2-em-3-carboxylate [prepared as described in step (a) above] in 20 ml of a 1 : 1 by volume mixture of tetrahydrofuran and water was mixed with 2.6 ml of 1N aqueous hydrochloric acid, and the mixture was hydrogenated by bubbling hydrogen through it at room temperature for 2 hours in the presence of 10% w/w palladium-on-charcoal. The catalyst was then removed by filtration, and the filtrate was washed with diethyl ether. The aqueous solution was concentrated by evaporation under reduced pressure, after which the residue was purified by reverse phase column chromatography through a Lobar column (Merck, LiChroprep RP-8, size B), using water as the eluent. Those fractions containing the title compound were collected, concentrated by evaporation under reduced pressure and lyophilised, to give 260 mg of the title compound as a powder.
Infrared Absorption Spectrum (KBr), νₘₐₓ cm⁻¹:
   1759, 1650, 1598, 1458, 1389.
Ultraviolet Absorption Spectrum (H₂O) λₘₐₓ nm:
   297.6
Nuclear Magnetic Resonance Spectrum (270 MHz, D₂O, using tetradeuterated sodium trimethylsilylpropionate as an internal standard) δ ppm:
   1.21 (3H, doublet, J = 7.32 Hz);
   1.29 (3H, doublet, J = 6.35 Hz);
   1.99 - 2.32 (3H, multiplet);
   2.97 (3H, singlet);
   3.18 - 4.05 (13H, multiplet);
   4.12 - 4.35 (3H, multiplet);
   4.66 - 4.82 (1H, multiplet).

### EXAMPLE 50

### (1R,5S,6S)-2-[(2S,4S)-2-(4-Carboxymethyl-1-homopiperazinylcarbonyl)pyrrolidin-4-ylthio]-6-[(1R)-1-hydroxyethyl]-1-methyl-1-carbapen-2-em-3-carboxylic acid

### 50(a) 4-Nitrobenzyl (1R,5S,6S)-2-[(2S,4S)-2-[4-(4-nitrobenzyloxycarbonylmethyl)-1-homopiperazinylcarbonyl]-1-(4-nitrobenzyloxycarbonyl)pyrrolidin-4-ylthio]-6-[(1R)-1-hydroxyethyl]-1-methyl-1-carbapen-2-em-3-carboxylate

0.37 ml of diphenylphosphoryl chloride and 0.31 ml of diisopropylethylamine were added dropwise, whilst ice-cooling, to a solution of 580 mg of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred at the same temperature for 1 hour. 0.99 ml of diisopropylethylamine and a solution of 1.43 g of (2S,4S)-4-mercapto-2[4-(4-nitrobenzyloxycarbonylmethyl)-1-homopiperazinylcarbonyl]-1-(4-nitrobenzyloxycarbonyl) pyrrolidine bis(trifluoromethanesulphonate) (prepared as described in Preparation 50) in 10 ml of dry acetonitrile were then simultaneously added dropwise to the mixture, whilst ice-cooling, and the resulting mixture was stirred overnight at the same temperature. At the end of this time, the solvent was removed by distillation under reduced pressure, and the residue was mixed with an aqueous solution of sodium hydrogencarbonate. It was then extracted with ethyl acetate. The extract was dried over anhydrous magnesium sulphate and the solvent was removed by distillation under reduced pressure. The residue was subjected to column chromatography through a Lobar column (Merck, LiChroprep Si60, size B), using a 5 : 1 by volume mixture of ethyl acetate and methanol as the eluent to give 1.1 g of the title compound, as a powder.
Infrared Absorption Spectrum (KBr), νₘₐₓ cm⁻¹:
   1771, 1709, 1647, 1606, 1521, 1346.
Nuclear Magnetic Resonance Spectrum (270 MHz, CDCℓ₃), δ ppm:
   1.28 (3H, doublet, J = 6.84 Hz);
   1.37 (3H, doublet, J = 6.35 Hz);
   1.74 - 2.04 (4H, multiplet);
   2.62 - 2.96 (6H, multiplet);
   3.27 (1H, doublet of doublets, J = 6.83 & 2.44 Hz);
   3.34 - 3.79 (9H, multiplet);
   4.19 - 4.27 (2H, multiplet);
   4.66 - 4.77 (1H, multiplet);
   5.06 - 5.52 (6H, multiplet);
   7.45 - 7.52 (4H, multiplet);
   7.65 (2H, doublet, J = 8.79 Hz);
   8.23 (6H, doublet, J = 8.79 Hz).

### 50(b) (1R,5S,6S)-2-[(2S,4S)-2-(4-Carboxymethyl-1-homopiperazinylcarbonyl)pyrrolidin-4-ylthio]-6-[(1R)-1-hydroxyethyl]-1-methyl-1-carbapen-2-em-3-carboxylic acid

A solution of 0.5 g of 4-nitrobenzyl (1R,5S,6S)-2-[(2S,4S)-2-[4-(4-nitrobenzyloxycarbonylmethyl)-1-homopiperazinylcarbonyl] -1-(4-nitrobenzyloxycarbonyl)-pyrrolidin-4-ylthio]-6-[(1R)-1-hydroxyethyl]-1-methyl-1-carbapen-2-em-3-carboxylate [prepared as described in step (a) above] in 15 ml of a 1 : 1 by volume mixture of tetrahydrofuran and water was mixed with 0.26 ml of 1N aqueous hydrochloric acid, and the mixture was hydrogenated by bubbling hydrogen through it at room temperature for 90 minutes in the presence of 0.5 g of 10% w/w palladium-on-charcoal. The catalyst was then removed by filtration, and the filtrate was washed with diethyl ether. The washed aqueous solution was then concentrated by evaporation under reduced pressure, and the concentrate was purified by reverse phase column chromatography through a Lobar column (Merck, LiChroprep RP-8, size B), using 1% v/v aqueous methanol as the eluent. Those fractions containing the title compound were collected, concentrated by evaporation under reduced pressure and lyophilised, to give 170 mg of the title compound as a powder.
Infrared Absorption Spectrum (KBr), νₘₐₓ cm⁻¹:
   1754, 1638, 1460, 1374.
Ultraviolet Absorption Spectrum (H₂O) λₘₐₓ nm:
   296.6.
Nuclear Magnetic Resonance Spectrum (270 MHz, D₂O, using tetradeuterated sodium trimethylsilylpropionate as an internal standard) δ ppm:
   1.22 (3H, doublet, J = 7.33 Hz);
   1.30 (3H, doublet, J = 6.34 Hz);
   1.93 - 2.10 (1H, multiplet);
   2.13 - 2.42 (2H, multiplet);
   2.95 - 3.17 (1H, multiplet);
   3.27 - 3.98 (13H, multiplet);
   3.99 - 4.15 (2H, multiplet);
   4.18 - 4.35 (2H, multiplet);
   4.43 - 4.65 (1H, multiplet).

### EXAMPLE 51

### (1R,5S,6S)-2-[(2S,4S)-2-(4-Methyl-1-piperazinylcarbonyl)-1-methylpyrrolidin-4-ylthio]-6-[(1R)-1-hydroxyethyl]-1-methyl-1-carbapen-2-em-3-carboxylic acid hydrochloride

### 51(a) 4-Nitrobenzyl (1R,5S,6S)-2-[(2S,4S)-2-(4-methyl-1-piperazinylcarbonyl)-1-methylpyrrolidin-4-ylthio]-6-[(1R)-1-hydroxyethyl]-1-methyl-1-carbapen-2-em-3-carboxylate

540 µℓ of diphenylphosophoryl chloride and 470 µℓ of diisopropylethylamine were added dropwise, whilst ice-cooling, to a solution of 920 mg of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred at the same temperature for 1 hour. 1400 µℓ of diisopropylethylamine and a solution of 1350 mg of (2S,4S)-4-mercapto-2-(4-methyl-1-piperazinylcarbonyl)-1-methylpyrrolidine bis(trifluoromethanesulphonate) (prepared as described in Preparation 51) in 5 ml of dry acetonitrile were then simultaneously added dropwise to the mixture, whilst ice-cooling, and the resulting mixture was stirred at the same temperature for 2 hours, after which it was allowed to stand overnight in a refrigerator. At the end of this time, the solvent was removed by distillation under reduced pressure, and the residue was purified by chromatography through a Lobar column (Merck, LiChroprep RP-8, size B). The column was successively eluted with 65% by volume aqueous methanol and with 70% by volume aqueous methanol. Those fractions containing the title compound were combined and concentrated by evaporation under reduced pressure, to give 730 mg of the title compound, as a powder.
Nuclear Magnetic Resonance Spectrum (CDCℓ₃, 270 MHz), δ ppm:
   1.27 (3H, doublet, J = 7.3 Hz);
   1.37 (3H, doublet, J = 6.3 Hz);
   1.83 - 1.93 (1H, multiplet);
   2.30 (3H, singlet);
   2.35 (3H, singlet);
   2.35 - 2.48 (4H, multiplet);
   2.57 - 2.73 (2H, multiplet);
   3.13 - 3.36 (4H, multiplet);
   3.55 - 3.95 (5H, multiplet);
   4.21 - 4.28 (2H, multiplet);
   5.25 (1H, doublet, J = 14.2 Hz);
   5.48 (1H, doublet, J = 14.2 Hz);
   7.66 (2H, doublet, J = 8.8 Hz);
   8.23 (2H, doublet, J = 8.8 Hz).
Infrared Absorption Spectrum (KBr), νₘₐₓ cm⁻¹:
   1769, 1706, 1639, 1606, 1521, 1450, 1346, 1208, 1137.

### 51(b) (1R,5S,6S)-2-[(2S,4S)-2-(4-Methyl-1-piperazinylcarbonyl)-1-methylpyrrolidin-4-ylthio]-6-[(1R)-1-hydroxyethyl]-1-methyl-1-carbapen-2-em-3-carboxylic acid hydrochloride

A solution of 720 mg of 4-nitrobenzyl (1R,5S,6S)-2-[(2S,4S)-2-(4-methyl-1-piperazinylcarbonyl)-1-methyl-pyrrolidin-4-ylthio]-6-[(1R)-1-hydroxyethyl]-1-methyl-1-carbapen-2-em-3-carboxylate [prepared as described in step (a) above] in 60 ml of a 1 : 1 by volume mixture of tetrahydrofuran and water was mixed with 1.2 ml of 1N aqueous hydrochloric acid, and the mixture was hydrogenated by bubbling hydrogen through it at room temperature for 2 hours in the presence of 800 mg of 10% w/w palladium-on-charcoal. The catalyst was then removed by filtration and the filtrate was washed with diethyl ether. The aqueous solution was concentrated by evaporation under reduced pressure, the residue was subjected to column chromatography using a Lobar column (Merck, LiChroprep RP-8, size B) and the column was eluted with 3% by volume aqueous methanol. Those fractions containing the title compound were combined and concentrated by evaporation under reduced pressure, to give 326 mg of the title compound as a colourless powder.
Nuclear Magnetic Resonance Spectrum (270 MHz, D₂O, using tetradeuterated sodium trimethylsilylpropionate as an internal standard) δ ppm:
   1.21 (3H, doublet, J = 6.8 Hz);
   1.29 (3H, doublet, J = 6.3 Hz);
   1.97 - 2.07 (1H, multiplet);
   2.96 (3H, singlet);
   2.97 (3H, singlet);
   3.18 - 3.50 (8H, multiplet);
   3.67 - 3.84 (5H, multiplet);
   4.17 - 4.30 (3H, multiplet);
   4.70 - 4.77 (1H, multiplet).
Infrared Absorption Spectrum (KBr), νₘₐₓ cm⁻¹:
   1754, 1658, 1600, 1456, 1385, 1261.
Ultraviolet Absorption Spectrum (H₂O) λₘₐₓ nm (ε):
   297 (8660).

### EXAMPLE 52

### (1R,5S,6S)-2-{(2S,4S)-2-[4-(2-Hydroxyethyl)-1-piperazinylcarbonyl]-1-methylpyrrolidin-4-ylthio}-6-[(1R)-1-hydroxyethyl]-1-methyl-1-carbapen-2-em-3-carboxylic acid hydrochloride

### 52(a) 4-Nitrobenzyl (1R,5S,6S)-2-{(2S,4S)-2-[4-(2-hydroxyethyl)-1-piperazinylcarbonyl]-1-methylpyrrolidin-4-ylthio}-6-[(1R)-1-hydroxyethyl]-1-methyl-1-carbapen-2-em-3-carboxylate

600 µℓ of diphenylphosphoryl chloride and 510 µℓ of diisopropylethylamine were added dropwise, whilst ice-cooling, to a solution of 1000 mg of 4-nitrobenzyl (1R,5S,6S)-6-[(1R)-1-hydroxyethyl]-1-methyl-2-oxo-1-carbapenam-3-carboxylate in 10 ml of dry acetonitrile, and the resulting mixture was stirred at the same temperature for 1 hour. 1800 µℓ of diisopropylethylamine and a solution of 1680 mg of (2S,4S)-4-mercapto-2-[4-(2-hydroxyethyl)-1-piperazinylcarbonyl] -1-methylpyrrolidine bis(trifluoromethanesulphonate) (prepared as described in Preparation 52) in 5 ml of dry acetonitrile were then simultaneously added dropwise to the mixture, whilst ice-cooling, and the resulting mixture was stirred at the same temperature for 2 hours, after which it was allowed to stand overnight in a refrigerator. At the end of this time, the solvent was removed by distillation under reduced pressure, and the residue was subjected to column chromatography through a Lobar column (Merck, LiChroprep RP-8, size B). The column was eluted first with 55% v/v aqueous methanol and then with 60% v/v aqueous methanol. Those fractions containing the title compound were collected, concentrated by evaporation under reduced pressure and lyophilised, to give 710 mg of the title compound, as a powder.
Nuclear Magnetic Resonance Spectrum (CDCℓ₃, 270 MHz), δ ppm:
   1.27 (3H, doublet, J = 7.3 Hz);
   1.36 (3H, doublet, J = 5.9 Hz);
   1.83 - 1.93 (1H, multiplet);
   2.34 (3H, singlet);
   2.40 - 2.76 (8H, multiplet);
   3.10 - 3.38 (4H, multiplet);
   3.55 - 4.04 (7H, multiplet);
   4.18 - 4.31 (2H, multiplet);
   5.24 (1H, doublet, J = 13.8 Hz);
   5.48 (1H, doublet, J = 13.8 Hz);
   7.66 (2H, doublet, J = 8.6 Hz);
   8.22 (2H, doublet, J = 8.6 Hz).
Infrared Absorption Spectrum (KBr), νₘₐₓ cm⁻¹:
   1756, 1651, 1596, 1464, 1390, 1373, 1286, 1261.

### 52(b) (1R,5S,6S)-2-{(2S,4S)-2-[4-(2-Hydroxyethyl)-1-piperazinylcarbonyl]-1-methylpyrrolidin-4-ylthio}-6-[(1R)-1-hydroxyethyl]-1-methyl-1-carbapen-2-em-3-carboxylic acid hydrochloride

A solution of 700 mg of 4-nitrobenzyl (1R,5S,6S)-2-{(2S,4S)-2-[4-(2-hydroxyethyl)-1-piperazinylcarbonyl]-1-methylpyrrolidin-4-ylthio}-6-[(1R)-1-hydroxyethyl]-1-methyl-1-carbapen-2-em-3-carboxylate [prepared as described in step (a) above] in 60 ml of a 1 : 1 by volume mixture of tetrahydrofuran and water was mixed with 1.15 ml of 1N aqueous hydrochloric acid, and the mixture was hydrogenated by bubbling hydrogen through it at room temperature for 2 hours in the presence of 1000 mg of 10% w/w palladium-on-charcoal. At the end of this time, the catalyst was removed by filtration, and the filtrate was extracted with diethyl ether. The remaining aqueous layer was concentrated by evaporation under reduced pressure, and the residue was subjected to column chromatography through a Lobar column (Merck, LiChroprep RP-8, size B). The column was eluted with 1.5% v/v aqueous methanol and those fractions containing the title compound were combined, concentrated by evaporation under reduced pressure and lyophilised, to give 300 mg of the title compound as a colourless powder.
Nuclear Magnetic Resonance Spectrum (270 MHz, D₂O, using tetradeuterated sodium trimethylsilylpropionate as an internal standard), δ ppm:
   1.21 (3H, doublet, J = 7.3 Hz);
   1.29 (3H, doublet, J = 6.3 Hz);
   1.98 - 2.07 (1H, multiplet);
   2.95 (3H, singlet);
   3.17 - 3.45 (9H, multiplet);
   3.45 - 3.52 (1H, multiplet);
   3.64 - 3.86 (5H, multiplet);
   3.86 - 3.95 (2H, multiplet);
   4.13 - 4.30 (3H, multiplet);
   4.68 - 4.77 (1H, multiplet).
Infrared Absorption Spectrum (KBr), νₘₐₓ cm⁻¹:
   1757, 1658, 1596, 1450, 1391, 1374, 1260.
Ultraviolet Absorption Spectrum (H₂O) λₘₐₓ nm (ε):
   297 (9252).

### EXAMPLE 53

### (1R,5S,6S)-2-{(2S,4S)-2-[4-(2-Carbamoyloxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

### 53(a) 4-Nitrobenzyl (1R,5S,6S)-2-{(2S,4S)-2-[4-(2-carbamoyloxyethyl)-1-piperazinylcarbonyl]-1-(4-nitro-benzyloxycarbonyl)pyrrolidin-4-ylthio}-6-[(1R)-1-hydroxyethyl]-1-methyl-1-carbapen-2-em-3-carboxylate

91 µℓ of diphenylphosphoryl chloride and 77 µℓ of diisopropylethylamine were added dropwise, whilst ice-cooling, to a solution of 152 mg of 4-nitrobenzyl (1R,5R,6S)-6-[(1R)-1-hydroxyethyl]-1-methyl-2-oxo-1-carbapenam-3-carboxylate in 2.0 ml of dry acetonitrile, and the resulting mixture was stirred at the same temperature for 1 hour. 176 µℓ of diisopropylethylamine and a solution of 318 mg of (2S,4S)-4-mercapto-2-[4-(2-carbamoyloxyethyl)-1-piperazinylcarbonyl]-1-(4-nitrobenzyloxycarbonyl)pyrrolidine trifluoromethanesulphonate (prepared as described in Preparation 53) in 2.0 ml of dry acetonitrile were then simultaneously added dropwise to the mixture, whilst ice-cooling, and the resulting mixture was stirred at the same temperature for 2 hours, after which it was allowed to stand overnight in a refrigerator. At the end of this time, the solvent was removed by distillation under reduced pressure, and the residue was mixed with an aqueous solution of sodium hydrogencarbonate. The mixture was then extracted with ethyl acetate, and the extract was dried over anhydrous magnesium sulphate. The solvent was then removed from the extract by distillation under reduced pressure, and the resulting residue was purified by chromatography through a Lobar column (Merck, LiChroprep Si60, size B), using a 5 : 1 by volume mixture of ethyl acetate and methanol as the eluent to give 99 mg of the title compound, as a powder.
Infrared Absorption Spectrum (KBr), νₘₐₓ cm⁻¹:
   1773, 1711, 1650, 1521, 1345.
Nuclear Magnetic Resonance Spectrum (270 MHz, hexadeuterated dimethyl sulphoxide) δ ppm:
   1.15 (3H, doublet, J = 6.35 Hz);
   1.16 (3H, doublet, J = 7.32 Hz);
   1.51 - 1.72 (1H, multiplet);
   2.10 - 2.57 (6H, multiplet);
   2.72 - 2.93 (1H, multiplet);
   3.06 - 4.30 (13H, multiplet);
   4.76 & 4.85 (together 1H, two triplets, J = 7.81 Hz);
   5.03 - 5.27 (3H, multiplet);
   5.30 & 5.46 (2H, AB-quartet, J = 14.16 Hz);
   6.46 (2H, broad singlet);
   7.55 & 7.65 (2H, two doublets, J = 8.79 Hz);
   7.72 (2H, doublet, J = 8.79 Hz);
   8.22 & 8.23 (4H, two doublets, J = 8.79 Hz).

### 53(b) (1R,5S,6S)-2-{(2S,4S)-2-[4-(2-Carbamoyloxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

1.0 g of 4-nitrobenzyl (1R,5S,6S)-2-{(2S,4S)-2-[4-(2-carbamoyloxyethyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] was dissolved in 60 ml of a 1 : 1 by volume mixture of tetrahydrofuran and water, and the mixture was hydrogenated by bubbling hydrogen through it at room temperature for 3 hours in the presence of 1.1 g of 10% w/w palladium-on-charcoal. At the end of this time, the catalyst was removed by filtration, and the filtrate was extracted with diethyl ether. The remaining aqueous layer was concentrated by evaporation under reduced pressure, and the resulting residue was subjected to column chromatography through a Lobar column (Merck, LiChroprep RP-8, size B). The column was eluted with 20% v/v aqueous methanol. Those fractions containing the title compound were combined and the pH was adjusted to a value of 4 by the addition of 1N aqueous hydrochloric acid. The solution was then concentrated by evaporation under reduced pressure, and the concentrate was lyophilised, to give 327 mg of the title compound as a colourless powder.
Infrared Absorption Spectrum (KBr), νₘₐₓ cm⁻¹:
   1728, 1654, 1597, 1392.
Ultraviolet Absorption Spectrum (H₂O) λₘₐₓ nm (ε):
   297 (9706).
Nuclear Magnetic Resonance Spectrum (270 MHz, D₂O, using tetradeuterated sodium trimethylsilylpropionate as an internal standard), δ ppm:
   1.21 (3H, doublet, J = 7.3 Hz);
   1.29 (3H, doublet, J = 6.3 Hz);
   1.95 - 2.06 (1H, multiplet);
   3.01 - 3.25 (7H, multiplet);
   3.31 - 3.43 (1H, multiplet);
   3.46 - 3.52 (2H, multiplet);
   3.72 - 3.90 (5H, multiplet);
   4.02 - 4.11 (1H, multiplet);
   4.21 - 4.30 (2H, multiplet);
   4.32 - 4.36 (2H, multiplet);
   4.82 - 4.89 (1H, multiplet).

### EXAMPLE 54

### (1R,5S,6S)-2-{(2S,4S)-2-[4-(2-Hydroxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

### 54(a) (i) 4-Nitrobenzyl (1R,5S,6S)-2-[(2S,4S)-2-[4-(2-hydroxyethyl)-1-piperazinylcarbonyl]-1-(4-nitrobenzyloxycarbonyl)pyrrolidin-4-ylthio] -6-[(1R)-1-hydroxyethyl]-1-methyl-1-carbapen-2-em-3-carboxylate

90 µℓ of diphenylphosphoryl chloride and 76 µℓ of diisopropylethylamine were added dropwise, whilst ice-cooling, to a solution of 148 mg of 4-nitrobenzyl (1R,5R,6S)-6[(1R)-1-hydroxyethyl]-1-methyl-2-oxo-1-carbapenam-3-carboxylate in 1.9 ml of dry acetonitrile, and the resulting mixture was stirred at the same temperature for 1 hour. 257 µℓ of diisopropylethylamine and 361 mg of (2S,4S)-2-[4-(2-hydroxyethyl)-1-piperazinylcarbonyl]-4-mercapto-1-(4-nitrobenzyloxycarbonyl)pyrrolidine bis(trifluoromethanesulphonate) (prepared as described in Preparation 54) were then simultaneously added dropwise to the mixture, whilst ice-cooling, and the resulting mixture was stirred at the same temperature for 6 hours, after which it was allowed to stand overnight in a refrigerator. At the end of this time, the reaction mixture was worked up in a similar manner to that described in Example 49(b). The crude product thus obtained was purified by chromatography through a Lobar column (Merck, LiChroprep Si60), using a 5 : 1 by volume mixture of acetonitrile and methanol as the eluent, to give 220 mg of the title compound, as a powder.
Ultraviolet Absorption Spectrum (MeOH) λₘₐₓ nm:
   268, 315.
Infrared Absorption Spectrum (KBr), νₘₐₓ cm⁻¹:
   1772, 1710, 1652, 1606, 1521, 1489, 1440, 1405, 1345, 1280, 1207.
Nuclear Magnetic Resonance Spectrum (CDCℓ₃, 270 MHz), δ ppm:
   1.27 & 1.28 (3H, two doublets, J = 7.33 Hz);
   1.37 (3H, doublet, J = 6.35 Hz);
   1.82 - 2.00 (1H, multiplet);
   2.33 - 2.78 (8H, multiplet);
   3.25 - 3.29 (1H, multiplet);
   3.32 - 3.82 (9H, multiplet);
   4.00 - 4.30 (3H, multiplet);
   4.69 & 4.74 (1H, two triplets, J = 7.81 Hz);
   5.05 - 5.52 (5H, multiplet);
   7.44 & 7.51 (2H, two doublets, J = 8.79 Hz);
   7.64 (2H, doublet, J = 8.79 Hz);
   8.23 (4H, doublet, J = 8.79 Hz).

### 54(a) (i') 4-Nitrobenzyl (1R,5S,6S)-2-[(2S,4S)-2-[4-(2-hydroxyethyl)-1-piperazinylcarbonyl]-1-(4-nitrobenzyloxycarbonyl)pyrrolidin-4-ylthio]-6-[(1R)-1-hydroxyethyl]-1-methyl-1-carbapen-2-em-3-carboxylate

1.82 ml of diphenylphosphoryl chloride and 1.54 ml of diisopropylethylamine were added dropwise, whilst ice-cooling, to a solution of 3.0 g of 4-nitrobenzyl (1R,5R,6S)-6-[(1R)-1-hydroxyethyl]-1-methyl-2-oxo-1-carbapenam-3-carboxylate in 38 ml of dry acetonitrile, and the resulting mixture was stirred at the same temperature for 1 hour. 1.54 ml of diisopropylethylamine and a solution of 3.63 g of (2S,4S)-2-[4-(2-hydroxyethyl)-1-piperazinylcarbonyl]-4-mercapto-1-(4-nitrobenzyloxycarbonyl)pyrrolidine (prepared as described in Preparation 54) in 30 ml of dry acetonitrile were then simultaneously added dropwise to the mixture, whilst ice-cooling, and the resulting mixture was stirred at the same temperature for 3 hours, after which it was allowed to stand overnight in a refrigerator. At the end of this time, the reaction mixture was worked up in a similar manner to that described in Example 49(b). The crude product was then purified by column chromatography through silica gel, using a 2 : 1 by volume mixture of ethyl acetate and methanol as the eluent, to give 3.4 g of the title compound, as a powder. The infrared absorption spectrum and nuclear magnetic resonance spectrum of the product were identical with those of the compound prepared as described in step 54(i), above.

### 54(a) (ii) (1R,5S,6S)-2-{(2S,4S)-2-[(4-(2-Hydroxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

1.38 g of 4-nitrobenzyl (1R,5S,6S)-2-[(2S,4S)-2-[4-(2-hydroxyethyl)-1-piperazinylcarbonyl]-1-(4-nitrobenzyloxycarbonyl) pyrrolidin-4-ylthio]-6-[(1R)-1-hydroxyethyl]1-methyl-1-carbapen-2-em-3-carboxylate [prepared as described in step 54(a) (i) or step 54(a) (i') above] was dissolved in 40 ml of a 1 : 1 by volume mixture of tetrahydrofuran and water, and the mixture was hydrogenated by bubbling hydrogen through it at room temperature for 2 hours in the presence of 1.2 g of 10% w/w palladium-on-charcoal. At the end of this time, the catalyst was removed by filtration, and the filtrate was extracted with diethyl ether. The pH of the remaining aqueous layer was adjusted to a value of 4 by the addition of 1N aqueous hydrochloric acid, and then the solution was concentrated by evaporation under reduced pressure. The resulting residue was subjected to column chromatography through a Lobar column (Merck, LiChroprep RP-8, size B) and the column was eluted with 3% v/v aqueous methanol. Those fractions containing the title compound were combined, concentrated by evaporation under reduced pressure and lyophilised, to give 314 mg of the title compound as a colourless powder.
Nuclear Magnetic Resonance Spectrum (270 MHz, D₂O, using sodium tetradeuterated trimethylsilylpropionate as an internal standard), δ ppm:
   1.21 (3H, doublet, J = 7.3 Hz);
   1.28 (3H, doublet, J = 6.4 Hz);
   1.97 - 2.07 (1H, multiplet);
   3.02 - 3.13 (1H, multiplet);
   3.32 - 3.70 (10H, multiplet);
   3.70 - 4.00 (6H, multiplet);
   4.00 - 4.16 (1H, multiplet);
   4.18 - 4.29 (2H, multiplet);
   4.86 - 4.92 (1H, multiplet).
Ultraviolet Absorption Spectrum (H₂O) λₘₐₓ nm (ε):
   297 (8124).
Infrared Absorption Spectrum (KBr), νₘₐₓ cm⁻¹:
   1757, 1659, 1595, 1393, 1376, 1277.

### 54(b) (i) 4-Nitrobenzyl (1R,5S,6S)-2-{(2S,4S)-[4-(2-4'-nitrobenzyloxycarbonyloxyethyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

6.38 g of diphenylphosphoryl chloride and 5.37 ml of diisopropylethylamine were added dropwise, whilst ice-cooling, to a solution of 10.63 g of 4-nitrobenzyl (1R,5R,6S)-6-[(1R)-1-hydroxyethyl]-1-methyl-2-oxo-1-carbapenam-3-carboxylate in 75 ml of dry acetonitrile, and the resulting mixture was stirred at the same temperature for 1 hour. 16.26 ml of diisopropylethylamine and a solution of 32.5 g of (2S,4S)-4-mercapto-2-{4-[2-(4-nitrobenzyloxycarbonyl)oxyethyl]-1-piperazinylcarbonyl}-1-(4-nitrobenzyloxycarbonyl) -pyrrolidine bis (trifluoromethanesulphonate) in 65 ml of dry acetonitrile were then simultaneously added dropwise to the mixture, whilst ice-cooling. The resulting mixture was then stirred at the same temperature for 1 hour, after which it was allowed to stand overnight, whilst ice-cooling. At the end of this time, the solvent was removed by distillation under reduced pressure, and the resulting residue was mixed with an aqueous solution of sodium hydrogencarbonate; it was then extracted with ethyl acetate. The extract was dried over anhydrous magnesium sulphate, and the solvent was removed by distillation under reduced pressure. The residue was purified by column chromatography through silica gel, using a 18 : 1 by volume mixture of ethyl acetate and methanol as the eluent, to give 19.75 g of the title compound, as a powder.
Infrared Absorption Spectrum (KBr), νₘₐₓ cm⁻¹:
   1769, 1751, 1710, 1653, 1607, 1521, 1443, 1347.
Nuclear Magnetic Resonance Spectrum (CDCℓ₃, 270 MHz), δ ppm:
   1.27 & 1.28 (3H, two doublets, J = 7.33 Hz);
   1.37 (3H, doublet, J = 6.35 Hz);
   1.78 - 1.98 (1H, multiplet);
   2.31 - 2.80 (7H, multiplet);
   3.27 (1H, doublet of doublets, J = 6.83 & 2.44 Hz);
   3.31 - 3.76 (8H, multiplet);
   4.01 - 4.33 (5H, multiplet);
   4.68 & 4.74 (1H, two triplets, J = 7.81 Hz);
   5.04 - 5.52 (6H, multiplet);
   7.44 & 7.51 (2H, two doublets, J = 8.79 Hz);
   7.55 & 7.65 (4H, two doublets, J = 8.79 Hz);
   8.17 - 8.25 (6H, multiplet).

### 54 (b)(i′) 4-Nitrobenzyl (1R,5S,6S)-2-{(2S,4S)-[4-(2-4′-nitrobenzyloxycarbonyloxyethyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

675 µℓ of diphenylphosphoryl chloride and 567 µℓ of diisopropylethylamine were added dropwise, whilst ice-cooling, to a solution of 1.12 g of 4-nitrobenzyl (1R,5R,6S)-6-[(1R)-1-hydroxyethyl]-1-methyl-2-oxo-1-carbapenam-3-carboxylate in 10 ml of dry acetonitrile, and the resulting mixture was stirred at the same temperature for 1 hour. A solution of 2.30 g of (2S,4S)-4-mercapto-2-{4-[2-(4-nitrobenzyloxycarbonyl)oxyethyl]-1-piperazinylcarbonyl}-1-(4-nitrobenzyloxycarbonyl)pyrrolidine in 5 ml of dry acetonitrile was then added dropwise to the mixture, whilst ice-cooling, and the resulting mixture was stirred at the same temperature for 1 hour. At the end of this time, the reaction mixture was worked up and purified in a similar manner to that described in step 54(b) (i) above, to give 2.70 g of the title compound, as a powder. The infrared absorption spectrum and nuclear magnetic resonance spectrum of the compound thus obtained were identical with those of the compound prepared as described in step 54(b) (i) above.

### 54(b)(i'') 4-Nitrobenzyl (1R,5S,6S)-2-{(2S,4S)-[4-(2-4'-nitrobenzyloxycarbonyloxyethyl)-1-piperazinylcarbonyl] 1-(4-nitrobenzyloxycarbonyl)pyrrolidin-4-ylthio}-6-[(1R)-1-hydroxyethyl]-1-methyl-1-carbapen-2-em-3-carboxylate

A solution of 28.3 mg of 4-nitrobenzyl (1R,5S,6S)-2-phenylsulphinyl-6-[(1R)-1-hydroxyethyl]-1-methyl-1-carbapen-2-em-3-carboxylate in 1 ml of dry acetonitrile was added dropwise to a solution of 112 mg of (2S,4S)-{4-[2-(4-nitrobenzyloxycarbonyl)oxyethyl]-1-piperazinylcarbonyl}-4-mercapto-1-(4-nitrobenzyloxycarbonyl) -pyrrolidine in 0.5 ml of dry acetonitrile, whilst ice-cooling, and the resulting mixture was stirred at the same temperature for 1 hour. At the end of this time, the reaction mixture was freed from the solvent by distillation under reduced pressure. The resulting residue was purified by column chromatography through silica gel, using a 20 : 1 by volume mixture of ethyl acetate and methanol as the eluent, to give 14 mg of the title compound, as a powder. The infrared absorption spectrum and nuclear magnetic resonance spectrum of the compound thus obtained were identical with those of the compound prepared as described in step 54(b) (i) above.

### 54(b) (i''') 4-Nitrobenzyl (1R,5S,6S)-2-{(2S,4S)-[4-(2-4'-nitrobenzyloxycarbonyloxyethyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

A solution of 50 mg of 4-nitrobenzyl (1R,5S,6S)-2-(4-chlorophenyl)sulphinyl-6-[(1R)-1-hydroxyethyl]-1-methyl-1-carbapen-2-em-3-carboxylate and 19.4 mg of diisopropylethylamine in 0.5 ml of dry acetonitrile was added dropwise to a solution of 93 mg of (2S,4S)-{4-[2-(4-nitrobenzyloxycarbonyl)oxyethyl]-1-piperazinylcarbonyl}-4-mercapto-1-(4-nitrobenzyloxycarbonyl) pyrrolidine in 0.5 ml of dry acetonitrile, whilst ice-cooling, and the resulting mixture was stirred at the same temperature for 1 hour. At the end of this time, the reaction mixture was freed from the solvent by distillation under reduced pressure. The residue was worked up in a similar manner to that described in step 54(a) (i), to give 13 mg of 4-nitrobenzyl (1R,5S,6S)-2-{(2S,4S)-[4-(2-4'-nitrobenzyloxycarbonyloxyethyl)-1-piperazinylcarbonyl] -1-(4-nitrobenzyloxycarbonyl)-pyrrolidin-4-ylthio}-6-[(1R)-1-hydroxyethyl]-1-methyl-1-carbapen-2-em-3-carboxylate as a powder. The infrared absorption spectrum and nuclear magnetic resonance spectrum of the compound thus obtained were identical with those of the compound prepared as described in step 54(b) (i) above.

### 54(b) (ii) (1R,5S,6S)-2-{(2S,4S)-2-[(4-(2-Hydroxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

0.962 g of 4-nitrobenzyl (1R,5S,6S)-2-{(2S,4S)-[4-(2-4'-nitrobenzyloxycarbonyloxyethyl)-1-piperazinylcarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in steps 54(b) (i) to 54(b) (i''')) above] was dissolved in 30 ml of a 1 : 1 by volume mixture of tetrahydrofuran and water, to which 1.2 ml of 1N aqueous hydrochloric acid had been added, and the mixture was hydrogenated by bubbling hydrogen through it in the presence of 1 g of 10% w/w palladium-on-charcoal. At the end of this time, the catalyst was removed by filtration and the filtrate was extracted with ethyl acetate. The aqueous layer was concentrated by evaporation under reduced pressure, and the resulting residue was subjected to column chromatography through a Lobar column (Merck, LiChroprep RP-8, size B), using 3% v/v aqueous methanol as the eluent. Those fractions containing the title compound were combined, concentrated by evaporation under reduced pressure and lyophilised, to give 181 mg of the title compound as a colourless powder.

The infrared absorption spectrum and nuclear magnetic resonance spectrum of the compound thus obtained were identical with those of the compound prepared as described in step 54(a) (ii) above.

### EXAMPLE 55

### (1R,5S,6S)-2-[(2S,4S)-2-(1-Piperazinylcarbonyl)-1-methylpyrrolidin-4-ylthio]-6-[(1R)-1-hydroxyethyl]-1-methyl-1-carbapen-2-em-3-carboxylic acid hydrochloride

### 55(a) 4-Nitrobenzyl (1R,5S,6S)-2-[(2S,4S)-2-[4-(4-nitrobenzyloxycarbonyl)-1-piperazinylcarbonyl]-1-methylpyrrolidin-4-ylthio]-6-[(1R)-1-hydroxyethyl]-1-methyl-1-carbapen-2-em-3-carboxylate

7.0 g of diphenylphosphoryl chloride and 3.4 g of diisopropylethylamine were added dropwise, whilst ice-cooling, to a solution of 8.6 g of 4-nitrobenzyl (1R,5R,6S)-6-[(1R)-1-hydroxyethyl]-1-methyl-2-oxo-1-carbapenam-3-carboxylate in 120 ml of dry acetonitrile, and the resulting mixture was stirred at the same temperature for 1 hour. 6.7 g of diisopropylethylamine and a solution of 13.8 g of (2S,4S)-4-mercapto-2-[4-(4-nitrobenzyloxycarbonyl)-1-piperazinylcarbonyl]-1-methylpyrrolidine trifluoromethanesulphonate in dry acetonitrile were then simultaneously added dropwise to the mixture, whilst ice-cooling, and the resulting mixture was allowed to stand overnight, whilst ice-cooling. At the end of this time, the reaction mixture was worked up and purified in a similar manner to that described in Example 49(a), to give 7.0 g of the title compound, as a powder.
Infrared Absorption Spectrum (KBr), νₘₐₓ cm⁻¹:
   1771, 1706, 1647, 1521, 1436, 1346.
Nuclear Magnetic Resonance Spectrum (CDCℓ₃, 270 MHz), δ ppm:
   1.27 (3H, doublet, J = 6.84 Hz);
   1.37 (3H, doublet, J = 6.35 Hz);
   1.79 - 1.95 (1H, multiplet);
   2.37 (3H, singlet);
   2.60 - 2.81 (2H, multiplet);
   3.10 - 3.92 (13H, multiplet);
   3.97 - 4.33 (3H, multiplet);
   5.21 - 5.52 (4H, multiplet);
   7.53 (2H, doublet, J = 8.79 Hz);
   7.66 (2H, doublet, J = 8.79 Hz);
   8.22 & 8.24 (4H, two doublets, J = 8.79 Hz).

### 55(b) (1R,5S,6S)-2-[(2S,4S)-2-(1-Piperazinylcarbonyl)-1-methylpyrrolidin-4-ylthio]-6-[(1R)-1-hydroxyethyl]-1-methyl-1-carbapen-2-em-3-carboxylic acid hydrochloride

0.22 g of 4-nitrobenzyl (1R,5S,6S)-2-[(2S,4S)-2-[4-(4-nitrobenzyloxycarbonyl)-1-piperazinylcarbonyl]-1-methylpyrrolidin-4-ylthio]-6-[(1R)-1-hydroxyethyl]-1-methyl-1-carbapen-2-em-3-carboxylate [prepared as described in step (a) above] was dissolved in a 1 : 1 by volume mixture of tetrahydrofuran and water, and the mixture was hydrogenated by bubbling hydrogen through it at room temperature for 2 hours in the presence of 0.22 g of 10% w/w palladium-on-charcoal. At the end of this time, the catalyst was removed by filtration, and the filtrate was washed with diethyl ether. The remaining aqueous layer was concentrated by evaporation under reduced pressure, and the resulting residue was mixed with 0.35 ml of 1N aqueous hydrochloric acid. The mixture was purified by reverse phase column chromatography through a Lobar column (Merck, LiChroprep RP-8, size B), using 2% v/v aqueous methanol as the eluent. Those fractions containing the title compound were combined, concentrated by evaporation under reduced pressure and lyophilised, to give 98 mg of the title compound as a powder.
Infrared Absorption Spectrum (KBr), νₘₐₓ cm⁻¹:
   1759, 1657, 1600, 1451, 1383, 1266.
Ultraviolet Absorption Spectrum (H₂O) λₘₐₓ nm:
   296.6.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.29 (3H, doublet, J = 6.35 Hz);
   1.91 - 2.19 (1H, multiplet);
   2.96 (3H, singlet);
   3.15 - 3.43 (6H, multiplet);
   3.48 (1H, doublet of doublets, J = 6.11 & 2.69 Hz);
   3.66 - 3.82 (4H, multiplet);
   3.89 - 3.93 (2H, multiplet);
   4.13 - 4.31 (3H, multiplet);
   4.64 - 4.83 (1H, multiplet).

### EXAMPLE 56

### (1R,5S,6S)-2-[(2S,4S)-2-(4-Carboxymethyl-1-piperazinylcarbonyl)pyrrolidin-4-ylthio]-1-[(1R)-1-hydroxyethyl]-1-methyl-1-carbapen-2-em-3-carboxylic acid

### 56(a) 4-Nitrobenzyl (1R,5S,6S)-2-{(2S,4S)-2-[4-(4-nitrobenzyloxycarbonylmethyl)-1-piperazinylcarbonyl]-pyrrolidin-4-ylthio}-6-[(1R)-1-hydroxyethyl] -1-methyl-1-carbapen-2-em-3-carboxylate

290 µℓ of diphenylphosphoryl chloride and 245 µℓ of diisopropylethylamine were added dropwise, whilst ice-cooling, to a solution of 500 mg of 4-nitrobenzyl (1R,5R,6S)-6-[(1R)-1-hydroxyethyl]-1-methyl-2-oxo-1-carbapenam-3-carboxylate in 5 ml of dry acetonitrile, and the resulting mixture was stirred at the same temperature for 1 hour. 520 µℓ of diisopropylethylamine and a solution of 1.57 g of (2S,4S)-4-mercapto-2-[4-(4-nitrobenzyloxycarbonylmethyl)-1-piperazinylcarbonyl]-1-(4-nitrobenzyloxycarbonyl) pyrrolidine bis(trifluoromethanesulphonate) (prepared as described in Preparation 56) in 5 ml of dry acetonitrile were then simultaneously added dropwise to the mixture, whilst ice-cooling, and the resulting mixture was allowed to stand overnight at the same temperature. At the end of this time, the solvent was removed by distillation under reduced pressure, and the resulting residue was subjected to column chromatography through a Lobar column (Merck, LiChroprep Si60, size B), using with a 5 : 1 by volume mixture of ethyl acetate and methanol as the eluent. Those fractions containing the title compound were combined and concentrated by evaporation under reduced pressure, to give 706 mg of the title compound, as a powder.
Infrared Absorption Spectrum (KBr), νₘₐₓ cm⁻¹:
   1772, 1710, 1654, 1521, 1346.
Nuclear Magnetic Resonance Spectrum (CDCℓ₃, 270 MHz), δ ppm:
   1.27 (3H, doublet, J = 7.3 Hz);
   1.37 (3H, doublet, J = 5.9 Hz);
   1.85 - 2.06 (2H, multiplet);
   2.53 - 2.77 (5H, multiplet);
   3.25 - 3.76 (10H, multiplet);
   4.03 - 4.28 (3H, multiplet);
   4.67 - 4.79 (1H, multiplet);
   5.06 - 5.52 (6H, multiplet);
   7.43 - 7.66 (6H, multiplet);
   8.20 & 8.25 (6H, multiplet).

### 56(b) (1R,5S,6S)-2-[(2S,4S)-2-(4-Carboxymethyl-1-piperazinylcarbonyl)pyrrolidine-4-ylthio]-1-[(1R)-1-hydroxyethyl]-1-methyl-1-carbapen-2-em-3-carboxylic acid

200 mg of 4-nitrobenzyl (1R,5S,6S)-2-{(2S,4S)-2-[4-(4-nitrobenzyloxycarbonylmethyl)-1-piperazinylcarbonyl]pyrrolidin-4-ylthio}-6-[(1R)-1-hydroxyethyl]-1-methyl-1-carbapen-2-em-3-carboxylate [prepared as described in step (a) above] were dissolved in 20 ml of a 1 : 1 by volume mixture of tetrahydrofuran and water, and the mixture was hydrogenated by bubbling hydrogen through it at room temperature for 2 hours in the presence of 0.3 g of 10% w/w palladium-on-charcoal. At the end of this time, the catalyst was removed by filtration, and the filtrate was extracted with 30 ml of ether. The remaining aqueous layer was separated and concentrated by evaporation under reduced pressure. The resulting residue was subjected to column chromatography through a Lobar column (Merck, LiChroprep RP-8, size B), using water as the eluent. Those fractions containing the title compound were combined, concentrated by evaporation under reduced pressure and lyophilised, to give 20 mg of the title compound as a colourless powder.
Infrared Absorption Spectrum (KBr), νₘₐₓ cm⁻¹:
   1755, 1639, 1603, 1386.
Ultraviolet Absorption Spectrum (H₂O) λₘₐₓ nm (ε):
   297 (7753).
Nuclear Magnetic Resonance Spectrum (270 MHz, D₂O) δ ppm:
   1.02 (3H, doublet, J = 7.3 Hz);
   1.10 (3H, doublet, J = 6.3 Hz);
   1.72 - 1.82 (1H, multiplet);
   2.77 - 2.88 (1H, multiplet);
   2.95 - 3.10 (4H, multiplet);
   3.10 - 3.32 (3H, multiplet);
   3.39 (2H, singlet);
   3.44 - 3.75 (6H, multiplet);
   3.79 - 3.88 (1H, multiplet);
   4.01 - 4.10 (2H, multiplet).

### EXAMPLE 57

### (1R,5S,6S)-2-{(2R,4S)-2-[4-(2-Hydroxyethyl)-1-piperazinylcarbonyl] pyrrolidin-4-ylthio}-5-[(1R)-1-hydroxyethyl]-1-methyl-1-carbapen-2-em-3-carboxylic acid hydrochloride.

### 57(a) 4-Nitrobenzyl (1R,5S,6S)-2-?(2R,4S)-2-[4-(2-4'nitrobenzyloxycarbonyloxyethyl)-1-piperazinylcarbonyl]-1-(4-nitrobenzyloxycarbonyl) pyrrolidin-4-ylthio/-6-[(1R)-1-hydroxyethyl]-1-methyl-1-carbapen-2-em-3-carboxylate

109 µℓ of diphenylphosphoryl chloride and 92 µℓ of diisopropylethylamine were added dropwise, whilst ice-cooling, to a solution of 181 mg of 4-nitrobenzyl (1R,5R,6S)-6-[(1R)-1-hydroxyethyl]-1-methyl-2-oxo-1-carbapenam-3-carboxylate in 2 ml of dry acetonitrile, and the resulting mixture was stirred at the same temperature for 50 minutes. 87.1 µℓ of diisopropylethylamine and a solution of 308 mg of (2R,4S)-4-mercapto-2-{4-[2-(4-nitrobenzyloxycarbonyl)oxyethyl]-1-piperazinylcarbonyl}-1-(4-nitrobenzyloxycarbonyl) pyrrolidine (prepared as described in Preparation 57) in 1 ml of dry acetonitrile were added dropwise to the mixture, whilst ice-cooling, and the resulting mixture was stirred at the same temperature for 5 hours. At the end of this time, the solvent was removed by distillation under reduced pressure, and the resulting residue was purified by chromatography through silica gel, using a 5 : 1 by volume mixture of ethyl acetate and methanol as the eluent. Those fractions containing the title compound were combined and concentrated by evaporation under reduced pressure, to give 277 mg of the title compound, as a powder.
Infrared Absorption Spectrum (KBr), νₘₐₓ cm ⁻¹:
   1771, 1750, 1710, 1650, 1607, 1522, 1443, 1347.
Nuclear Magnetic Resonance Spectrum (CDCℓ₃, 270 MHz), δ ppm:
   1.28 (3H, doublet, J = 6.84 Hz);
   1.36 (3H, doublet, J = 6.34 Hz);
   3.31 - 3.96 (8H, multiplet);
   4.01 - 4.33 (5H; multiplet);
   4.77 - 4.90 (1H, multiplet);
   5.02 - 5.55 (6H, multiplet);
   7.41 - 7.66 (4H, multiplet);
   8.19 - 8.25 (4H, multiplet).

### 57(b) (1R,5S,6S)-2-{(2R,4S)-2-[4-(2-Hydroxyethyl)-1-piperazinylcarbonyl]pyrrolidin-4-ylthio}-5-[(1R)-1-hydroxyethyl]-1-methyl-1-carbapen-2-em-3-carboxylic acid hydrochloride

240 mg of 4-nitrobenzyl (1R,5S,6S)-2-{(2R,4S)-2-[4-(2-4'-nitrobenzyloxycarbonyloxyethyl)-1-piperazinylcarbonyl]-1-(4-nitrobenzyloxycarbonyl)pyrrolidin-4-ylthio}-6-[(1R)-1-hydroxyethyl]-1-methyl-1-carbapen-2-em-3-carboxylate [prepared as described in step (a) above] were dissolved in 8 ml of a 1 : 1 by volume mixture of tetrahydrofuran and water and mixed with 0.3 ml of 1N aqueous hydrochloric acid. The mixture was then hydrogenated by bubbling hydrogen through it at room temperature for 2 hours in the presence of 0.3 g of 10% w/w palladium-on-charcoal. At the end of this time, the catalyst was removed by filtration, and the filtrate was extracted with diethyl ether. The remaining aqueous layer was concentrated by evaporation under reduced pressure. The resulting residue was subjected to column chromatography through a Lobar column (Merck, LiChroprep RP-8, size A), using 3% v/v aqueous methanol as the eluent. Those fractions containing the title compound were combined, concentrated by evaporation under reduced pressure and lyophilised, to give 35 mg of the title compound as a colourless powder.
Ultraviolet Absorption Spectrum (H₂O) λₘₐₓ nm:
   297.

### EXAMPLE 58

### (1R,5S,6S)-2-{(2R,4R)-2-[4-(2-Hydroxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

### 58(a) 4-Nitrobenzyl (1R,5S,6S)-2-{(2R,4R)-2-[4-(2-4'-nitrobenzyloxycarbonyloxyethyl)-1-piperazinylcarbonyl]-1-(4-nitrobenzyloxycarbonyl) pyrrolidin-4-ylthio}-6-[(1R)-1-hydroxyethyl]-1-methyl-1-carbapen-2-em-3-carboxylate

164 µℓ of diphenylphosphoryl chloride and 138 µℓ of diisopropylethylamine were added dropwise, whilst ice-cooling, to a solution of 272 mg of 4-nitrobenzyl (1R,5R,6S)-6-[(1R)-1-hydroxyethyl]-1-methyl-2-oxo-1-carbapenam-3-carboxylate in 3 ml of dry acetonitrile, and the resulting mixture was stirred at the same temperature for 1 hour. 131 µℓ of diisopropylethylamine and a solution of 463 mg of (2R,4R)-4-mercapto-2{4-[2-(4-nitrobenzyloxycarbonyl)oxyethyl]-1-piperazinylcarbonyl}-1-(4-nitrobenzyloxycarbonyl)-pyrrolidine in 2 ml of dry acetonitrile were then simultaneously added dropwise to the mixture, whilst ice-cooling, and the resulting mixture was stirred at the same temperature for 1.5 hours. At the end of this time, the solvent was removed by distillation under reduced pressure, and the resulting residue was purified by chromatography through silica gel, using a 9 : 1 by volume mixture of ethyl acetate and methanol as the eluent. Those fractions containing the title compound were combined and concentrated by evaporation under reduced pressure, to give 490 mg of the title compound, as a powder.
Infrared Absorption Spectrum (KBr), νₘₐₓ cm⁻¹:
   1770, 1751, 1711, 1654, 1606, 1522, 1496, 1444, 1404, 1347, 1263, 1208.
Nuclear Magnetic Resonance Spectrum (CDCℓ₃, 270 MHz), δ ppm:
   1.27 (3H, doublet, J = 7.33 Hz);
   1.36 (3H, doublet, J = 5.86 Hz);
   1.82 - 2.05 (1H, multiplet);
   2.25 - 3.10 (7H, multiplet);
   3.25 - 3.85 (9H, multiplet);
   4.05 - 4.86 (6H, multiplet);
   5.05 - 5.51 (6H, multiplet);
   7.43 - 7.67 (6H, multiplet);
   8.18 - 8.25 (6H, multiplet).

### 58(b) (1R,5S,6S)-2-{(2R,4S)-2-[4-(2-Hydroxyethyl)-1-piperazinylcarbonyl]pyrrolidin-4-ylthio}-6-[(1R)-1-hydroxyethyl]-1-methyl-1-carbapen-2-em-3-carboxylic acid hydrochloride

191 mg of 4-nitrobenzyl (1R,5S,6S)-2-{(2R,4R)-2-[4-(2-4'-nitrobenzyloxycarbonyl)oxyethyl]-1-piperazinylcarbonyl] -1-(4-nitrobenzyloxycarbonyl) pyrrolidin-4-ylthio}-6-[(1R)-1-hydroxyethyl]-1-methyl-1-carbapen-2-em-3-carboxylate [prepared as described in step (a) above] were dissolved in 8 ml of a 1 : 1 by volume mixture of tetrahydrofuran and water, and the mixture was hydrogenated by bubbling hydrogen through it at room temperature for 1 hour in the presence of 218 µℓ of 1N aqueous hydrochloric acid and 0.3 g of 10% w/w palladium-on-charcoal. At the end of this time, the catalyst was removed by filtration, and the filtrate was extracted with diethyl ether. The remaining aqueous layer was concentrated by evaporation under reduced pressure, and the resulting residue was subjected to column chromatography through a Lobar column (Merck, LiChroprep RP-8, size A), using 3% v/v aqueous methanol as the eluent. Those fractions containing the title compound were combined, concentrated by evaporation under reduced pressure and lyophilised, to give 26 mg of the title compound as a colourless powder.
Ultraviolet Absorption Spectrum (H₂O) λₘₐₓ nm:
   297.
Infrared Absorption Spectrum (KBr), νₘₐₓ cm⁻¹:
   1758, 1659, 1595, 1451, 1385, 1261, 1181, 1145.
Nuclear Magnetic Resonance Spectrum (270 MHz, D₂O, using tetradeuterated sodium trimethylsilylpropionate as an internal standard), δ ppm:
   1.20 (3H, doublet, J = 7.33 Hz);
   1.28 (3H, doublet, J = 6.35 Hz);
   2.13 - 2.22 (1H, multiplet);
   2.91 - 3.03 (1H, multiplet);
   3.26 - 3.63 (9H, multiplet);
   3.75 - 4.11 (8H, multiplet);
   4.21 - 4.30 (2H, multiplet);
   4.83 - 4.93 (1H, multiplet).

### EXAMPLES 59 TO 88

Following a procedure similar to that described in Example 1 or Example 49, the following compounds were obtained by using the mercaptan shown in the corresponding one of Preparations 59 to 88.

### EXAMPLE 59

### (1R,5S,6S)-2-{(2S,4S)-2-[(2S)-4-Acetimidoyl-2-methylpiperazin-1-ylcarbonyl]pyrrolidin-4-ylthio}-6-[(1R)-1-hydroxyethyl]-1-methyl-1-carbapen-2-em-3-carboxylic acid

Ultraviolet Absorption Spectrum (H₂O), λₘₐₓ nm:
   300.
Infrared Absorption Spectrum (KBr), νₘₐₓ cm⁻¹:
   1755, 1629, 1591, 1448, 1384, 1281.
Nuclear Magnetic Resonance Spectrum (270 MHz, D₂O, using sodium tetradeuterated trimethylsilylpropionate as an internal standard), δ ppm:
   1.21 - 1.36 (6H, multiplet);
   1.30 (3H, doublet, J = 6.35 Hz);
   1.58 - 1.75 (1H, multiplet);
   2.35 & 2.39 (together 3H, two singlets);
   2.63 - 2.85 (1H, multiplet);
   3.06 (1H, doublet of doublets, J = 12.21 & 3.42 Hz);
   3.18 (1H, doublet of doublets, J = 12.21 & 5.86 Hz);
   3.26 - 3.62 (4H, multiplet);
   3.65 - 4.67 (9H, multiplet).

### EXAMPLE 60

### (1R,5S,6S)-2-{(2S,4S)-2-[(2S)-4-Formimidoyl-2-methylpiperazin-1-ylcarbonyl]pyrrolidin-4-ylthio}-6-[(1R)-1-hydroxyethyl]-1-methyl-1-carbapen-2-em-3-carboxylic acid

Ultraviolet Absorption Spectrum (H₂O), λₘₐₓ nm:
   296.
Infrared Absorption Spectrum (KBr), νₘₐₓ cm⁻¹:
   1755, 1711, 1641, 1592, 1452, 1384.
Nuclear Magnetic Resonance Spectrum (270 MHz, D₂O, using sodium tetradeuterated trimethylsilylpropionate as an internal standard), δ ppm:
   1.22 (3H, doublet, J = 7.3 Hz);
   1.58 (3H, doublet, J = 6.3 Hz);
   1.24 & 1.35 (together 3H, two doublets, J = 6.8 Hz);
   1.62 - 1.77 (1H, multiplet);
   2.68 - 2.89 (1H, multiplet);
   3.06 - 4.50 (15H, multiplet);
   7.93, 7.96, 8.03 & 8.19 (together 1H, four singlets).

### EXAMPLE 61

### (1R,5S,6S)-2-{(2S,4S)-1-Methyl-2-[(3S)-3-acetimidoylaminopyrrolidin-1-ylcarbonyl]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   301.
Infrared Absorption Spectrum (KBr), νₘₐₓ cm⁻¹:
   1756, 1682, 1632, 1593, 1453, 1385.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.30 (3H, doublet, J = 6.35 Hz);
   1.60 - 1.75 (1H, multiplet);
   2.24 (3H, doublet, J = 2.93 Hz);
   2.28 (3H, doublet, J = 4.88 Hz);
   2.70 - 2.90 (2H, multiplet);
   3.05 - 3.15 (1H, multiplet);
   3.25 - 3.50 (3H, multiplet);
   3.50 - 4.05 (7H, multiplet);
   4.15 - 4.40 (3H, multiplet).

### EXAMPLE 62

### (1R,5S,6S)-2-[(2S,4S)-2-(3-Acetimidoylaminopiperidin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 63

### (1R,5S,6S)-2-[(2S,4S)-1-Methyl-2-(4-acetimidoylaminopiperidin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 64

### (1R,5S,6S)-2-[(2S,4S)-1-Methyl-2-(4-formimidoylpiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.
Infrared Absorption Spectrum (KBr), νₘₐₓ cm⁻¹:
   1754, 1707, 1651, 1595, 1450, 1385, 1285.
Nuclear Magnetic Resonance Spectrum (270 MHz, D₂O, using sodium tetradeuterated trimethylsilylpropionate as an internal standard), δ ppm:
   1.21 (3H, doublet, J = 7.3 Hz);
   1.30 (3H, doublet, J = 6.4 Hz);
   1.68 (1H, doubled doublet of doublets, J = 13.7,
   8.8 & 5.4 Hz);
   2.34 (3H, singlet);
   2.78 - 2.95 (2H, multiplet);
   3.14 (1H, doublet of doublets, J = 12.2 & 1.4 Hz);
   3.30 - 3.45 (2H, multiplet);
   3.53 - 3.95 (10H, multiplet);
   4.18 - 4.30 (2H, multiplet);
   7.92 (1H, singlet).

### EXAMPLE 65

### (1R,5S,6S)-2-{(2S,4S)-1-Methyl-2-[(2S)-4-acetimidoyl-2-methylpiperazin-1-ylcarbonyl]pyrrolidin-4-ylthio}-6-[(1R)-1-hydroxyethyl]-1-methyl-1-carbapen-2-em-3-carboxylic acid

Ultraviolet Absorption Spectrum (H₂O), λₘₐₓ nm.
   299.

### EXAMPLE 66

### (1R,5S,6S)-2-[(2S,4S)-1-Acetimidoyl-2-(1-piperazinylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   297.
Infrared Absorption Spectrum (KBr), νₘₐₓ cm⁻¹:
   1754, 1663, 1594, 1489, 1455, 1384, 1252, 1209.
Nuclear Magnetic Resonance Spectrum (270 MHz, D₂O, using sodium tetradeuterated trimethylsilylpropionate as an internal standard), δ ppm:
   1.21 (3H, doublet, J = 7.32 Hz);
   1.30 (3H, doublet, J = 6.35 Hz);
   2.09 - 2.24 (1H, multiplet);
   2.16 & 2.38 (together 3H, two singlets);
   2.80 - 3.95 (13H, multiplet);
   3.96 - 4.33 (3H, multiplet);
   5.04 - 5.11 & 5.24 - 5.32 (together 1H, two multiplets).

### EXAMPLE 67

### (1R,5S,6S)-2-[(2S,4S)-1-Formimidoyl-2-(1-piperazinylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   297.
Infrared Absorption Spectrum (KBr), νₘₐₓ cm⁻¹:
   1754, 1708, 1660, 1594, 1489, 1455, 1395, 1251, 1209.
Nuclear Magnetic Resonance Spectrum (400 MHz, D₂O, using sodium tetradeuterated trimethylsilylpropionate as an internal standard), δ ppm:
   1.22 (3H, doublet, J = 7.32 Hz);
   1.29 (3H, doublet, J = 6.40 Hz);
   2.08 - 2.19 (1H, multiplet);
   2.98 - 4.33 (16H, multiplet);
   5.06 - 5.10 & 5.19 - 5.23 (together 1H, two multiplets);
   7.86 & 8.11 (together 1H, two singlets).

### EXAMPLE 68

### (1R,5S,6S)-2-[(2S,4S)-1-Acetimidoyl-2-(4-acetimidoylpiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 69

### (1R,5S,6S)-2-[(2S,4S)-1-Acetimidoyl-2-(4-formimidoylpiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 70

### (1R,5S,6S)-2-[(2S,4S)-1-Formimidoyl-2-(4-formimidoylpiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O)**,** λₘₐₓ nm:
   300.

### EXAMPLE 71

### (1R,5S,6S)-2-[(2S,4S)-1-Formimidoyl-2-(4-acetimidoylpiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 72

### (1R,5S,6S)-2-[(2S,4S)-1-Acetimidoyl-2-[(3S)-3-acetimidoylaminopyrrolidin-1-ylcarbonyl]pyrrolidin-4-ylthio]-6-[(1R)-1-hydroxyethyl]-1-methylcarbapen-2-em-3-carboxylic acid

Ultraviolet Absorption Spectrum (H₂O), λₘₐₓ nm:
   301.
Infrared Absorption Spectrum (KBr), νₘₐₓ cm⁻¹:
   1756, 1633, 1594, 1452, 1385.

### EXAMPLE 73

### (1R,5S,6S)-2-{(2S,4S)-1-Acetimidoyl-2-[(3S)-3-formimidoylaminopyrrolidin-1-ylcarbonyl]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   301.

### EXAMPLE 74

### (1R,5S,6S)-2-{(2S,4S)-1-Acetimidoyl-2-[(3S)-3-aminopyrrolidin-1-ylcarbony]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   297.

### EXAMPLE 75

### (1R,5S,6S)-2-{(2S,4S)-1-Formimidoyl-2-[(3S)-3-acetimidoylaminopyrrolidin-1-ylcarbonyl]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   301.

### EXAMPLE 76

### (1R,5S,6S)-2-{(2S,4S)-1-Formimidoyl-2-[(3S)-3-formimidoylaminopyrrolidin-1-ylcarbonyl]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   301.

### EXAMPLE 77

### (1R,5S,6S)-2-{(2S,4S)-1-formimidoyl-2-[(3S)-3-aminopyrrolidin-1-ylcarbonyl]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   297.

### EXAMPLE 78

### (1R,5S,6S)-2-[(2S,4S)-1-Acetimidoyl-2-(homopiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   297.

### EXAMPLE 79

### (1R,5S,6S)-2-[(2S,4S)-1-Acetimidoyl-2-(4-formimidoylhomopiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 80

### (1R,5S,6S)-2-[(2S,4S)-1-Formimidoyl-1-(homopiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   297.

### EXAMPLE 81

### (1R,5S,6S)-2-[(2S,4S)-1-Formimidoyl-2-(4-formimidoyl-homopiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 82

### (1R,5S,6S)-2-{(2S,4S)-2-[(3S)-3-(N-Methyl-N-acetimidoylaminopyrrolidin-1-ylcarbonyl]pyrrolidin-4-ylthio}-6-[(1R)-1-hydroxyethyl]-1-methylcarbapen-2-em-3-carboxylic acid

Ultraviolet Absorption Spectrum (H₂O), λₘₐₓ nm:
   300.

### EXAMPLE 83

### (1R,5S,6S)-2-[(2S,4S)-2-(2-Hydroxymethylpiperazin-1-ylcarbonyl)pyrrolidin-4-ylthio]-6-[(1R)-1-hydroxyethyl]-1-methyl-1-carbapen-2-em-3-carboxylic acid hydrochloride

Ultraviolet Absorption Spectrum (H₂O), λₘₐₓ nm:
   297.

### EXAMPLE 84

### (1R,5S,6S)-2-[(2S,4S)-2-(4-Acetimidoyl-2-hydroxymethylpiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   301.

### EXAMPLE 85

### (1R,5S,6S)-2-[(2S,4S)-2-(6-Hydroxyhomopiperazin-1-ylcarbonyl)pyrrolidin-4-ylthio]-6-[(1R)-1-hydroxyethyl]-1-methyl-1-carbapen-2-em-3-carboxylic acid hydrochloride

Ultraviolet Absorption Spectrum (H₂O), λₘₐₓ nm:
   296.

### EXAMPLE 86

### (1R,5S,6S)-2-[(2S,4S)-2-(4-Formimidoyl-6-hydroxyhomopiperazin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 87

### (1R,5S,6S)-2-[(2S,4S)-1-Acetimidoyl-2-(4-acetimidoylaminopiperidin-1-ylcarbonyl)pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### EXAMPLE 88

### (1R,5S,6S)-2-[(2S,4S)-1-methyl-2-(4-formimidoylhomopiperazin-1-ylcarbonyl]pyrrolidin-4-ylthio]-6-[(1R)-1-hydroxyethyl]-1-methyl-1-carbapen-2-em-3-carboxylic acid

Ultraviolet Absorption Spectrum (H₂O), λₘₐₓ nm:
   300.

### PREPARATION 1

### (2S,4S)-4-Mercapto-2-[4-(N-4-nitrobenzyloxycarbonylacetimidoyl)piperazin-1-ylcarbonyl]-1-(4-nitrobenzyloxycarbonyl)pyrrolidine trifluoromethanesulfonate

### 1(i) (2S,4S)-4-(4-Methoxybenzylthio)-2-(4-t-butoxycarbonylpiperazin-1-ylcarbonyl)-1-(4-nitrobenzyloxy-carbonyl)pyrrolidine

1.78 g of N,N'-carbonyldiimidazole was added to a solution of 4.46 g of (2S,4S)-4-(4-methoxybenzylthio)-1-(4-nitrobenzyloxycarbonyl)-2-pyrrolidinecarboxylic acid in 45 ml of dry acetonitrile, and the resulting mixture was stirred at room temperature for 1 hour. The reaction mixture was then cooled with ice, and a solution of 2.05 g of 1-t-butoxycarbonylpiperazine in 45 ml of dry acetonitrile was added to the mixture, which was then allowed to stand overnight at room temperature. At the end of this time, the reaction mixture was concentrated by evaporation under reduced pressure, and the concentrate was diluted with ethyl acetate. The ethyl acetate solution was washed with water and with an aqueous solution of sodium chloride and was then dried over anhydrous magnesium sulphate. The solvent was removed by distillation under reduced pressure, and the resulting residue was purified by column chromatography through silica gel, using a 3 : 2 by volume mixture of ethyl acetate and cyclohexane as the eluent, to give 5.4 g of the title compound, as a powder.
Infrared Absorption Spectrum (KBr), νₘₐₓ cm⁻¹:
   1699, 1658, 1609, 1585, 1512, 1456, 1377, 1366, 1344, 1286, 1237, 1205.
Nuclear Magnetic Resonance Spectrum (CDCℓ₃, 270 MHz), δ ppm:
   1.47 (9H, singlet);
   1.73 - 1.87 (1H, multiplet);
   2.40 - 2.52 (1H, multiplet);
   3.03 - 3.17 (1H, multiplet);
   3.25 - 4.09 (10H, multiplet);
   3.73 (2H, singlet);
   3.79 & 3.80 (together 3H, two singlets);
   4.57 & 4.61 (together 1H, two triplets, J = 8.30 Hz);
   5.01 - 5.32 (2H, multiplet);
   6.85 (2H, doublet, J = 8.79 Hz);
   7.23 (2H, doublet, J = 8.79 Hz);
   7.41 & 7.47 (together 2H, two doublets, J = 8.79 Hz);
   8.18 & 8.22 (together 2H, two doublets, J = 8.79 Hz).

### 1(ii) (2S,4S)-4-(4-Methoxybenzylthio)-2-(1-piperazinylcarbonyl)-1-(4-nitrobenzyloxycarbonyl)pyrrolidine hydrochloride

27 ml of a 4N ethyl acetate solution of hydrogen chloride were added to a solution of 5.2 g of (2S,4S)-4-(4-methoxybenzylthio)-2-(4-t-butoxycarbonylpiperazin-1-ylcarbonyl)-1-(4-nitrobenzyloxycarbonyl) pyrrolidine [prepared as described in step (i) above] in 27 ml of ethyl acetate, and the resulting mixture was heated under reflux for 2 hours. At the end of this time, the reaction mixture was concentrated to dryness by evaporation under reduced pressure, and the resulting concentrate was triturated with diethyl ether. The powder thus obtained was collected by filtration and dried to give 4.2 g of the title compound.
Infrared Absorption Spectrum (KBr), νₘₐₓ cm⁻¹:
   1708, 1662, 1609, 1585, 1512, 1434, 1404, 1346, 1319, 1301, 1246, 1209.
Nuclear Magnetic Resonance Spectrum (hexadeuterated dimethyl sulphoxide + D₂O, 270 MHz), δ ppm:
   1.53 - 1.68 (1H, multiplet);
   2.58 - 2.75 (1H, multiplet);
   2.90 - 3.94 (11H, multiplet);
   3.71 & 3.74 (together 3H, two singlets);
   3.78 (2H, singlet);
   4.70 & 4.80 (together 1H, two triplets, J = 8.06 Hz);
   5.03 - 5.23 (2H, multiplet);
   6.89 (2H, doublet, J = 8.30 Hz);
   7.27 (2H, doublet, J = 8.30 Hz);
   7.51 & 7.60 (together 2H, two doublets, J = 8.79 Hz);
   8.23 & 8.25 (together 2H, two doublets, J = 8.79 Hz).

### 1(iii) (2S,4S)-4-(4-Methoxybenzylthio)-2-[4-(N-4-nitrobenzyloxycarbonylacetimidoyl)piperazin-1-ylcarbonyl]-1-(4-nitrobenzyloxycarbonyl)pyrrolidine

11 ml of methylene chloride, followed by 452 mg of N-(4-nitrobenzyloxycarbonyl)acetamidine, were added to a solution of 1.1 g of (2S,4S)-4-(4-methoxybenzylthio)-2-(1-piperazinylcarbonyl)-1-(4-nitrobenzyloxycarbonyl)-pyrrolidine hydrochloride [prepared as described in step (ii) above] in 22 ml of methanol, whilst heating the solution under reflux. The resulting mixture was then heated under reflux for a further 4 hours. At the end of this time, the reaction mixture was freed from the solvent by distillation under reduced pressure, and the resulting residue was purified by column chromatography through silica gel, using a 20 : 1 by volume mixture of ethyl acetate and methanol as the eluent to give 466 mg of the title compound, as a powder.
Infrared Absorption Spectrum (KBr), νₘₐₓ cm⁻¹:
   1709, 1662, 1608, 1570, 1520, 1430, 1405, 1346, 1291, 1254.
Nuclear Magnetic Resonance Spectrum (CDCℓ₃, 270 MHz), δ ppm:
   1.73 - 1.94 (1H, multiplet);
   2.30 & 2.40 (together 3H, two singlets);
   2.38 - 2.52 (1H, multiplet);
   3.03 - 3.18 (1H, multiplet);
   3.11 - 4.05 (10H, multiplet);
   3.73 (2H, singlet);
   3.79 & 3.80 (together 3H, two singlets);
   4.52 - 4.63 (1H, multiplet);
   4.98 - 5.35 (4H, multiplet);
   6.85 (2H, doublet, J = 8.30 Hz);
   7.23 (2H, doublet, J = 8.30 Hz);
   7.40 - 7.63 (4H, multiplet);
   8.16 - 8.25 (4H, multiplet).

### 1(iv) (2S,4S)-4-Mercapto-2-[4-(N-4-nitrobenzyloxycarbonylacetimidoyl)piperazin-1-ylcarbonyl]-1-(4-nitrobenzyloxycarbonyl)pyrrolidine trifluoromethanesulfonate

3.2 ml of trifluoroacetic acid and 103 µℓ of trifluoromethanesulphonic acid were added to a solution of 430 mg of (2S,4S)-4-(4-methoxybenzylthio)-2-[4-(N-4-nitrobenzyloxycarbonylacetimidoyl)piperazin-1-ylcarbonyl]-1-(4-nitrobenzyloxycarbonyl) pyrrolidine [prepared as described in step (iii) above] in 636 µℓ of anisole, and the resulting mixture was stirred for 1 hour, whilst ice-cooling. At the end of this time, the reaction mixture was freed from the solvent by distillation under reduced pressure, and the residue was repeatedly washed with diethyl ether by decantation, to give 450 mg of the title compound, as a powder.
Infrared Absorption Spectrum (KBr), νₘₐₓ cm⁻¹:
   1782, 1705, 1634, 1610, 1522, 1441, 1406, 1348, 1277, 1249, 1224.
Nuclear Magnetic Resonance Spectrum (hexadeuterated dimethyl sulphoxide + D₂O, 270 MHz), δ ppm:
   1.52 - 1.78 (1H, multiplet);
   2.57 - 4.08 (15H, multiplet);
   4.65 - 4.84 (1H, multiplet);
   5.04 - 5.28 (4H, multiplet);
   7.49 - 7.69 (4H, multiplet);
   8.20 - 8.28 (4H, multiplet).

### PREPARATION 2

### (2S,4S)-4-Mercapto-2-[4-(N-4-nitrobenzyloxycarbonylacetimidoyl)homopiperazin-1-ylcarbonyl]-1-(4-nitrobenzyloxycarbonyl)pyrrolidine trifluoromethanesulfonate

### 2(i) (2S,4S)-4-(4-Methoxybenzylthio)-2-(1-homopiperazinylcarbonyl)-1-(4-nitrobenzyloxycarbonyl)pyrrolidine hydrochloride

1.95 g of N,N'-carbonyldiimidazole were added to a solution of 4.5 g of (2S,4S)-4-(4-methoxybenzylthio)-1-(4-nitrobenzyloxycarbonyl)-2-pyrrolidinecarboxylic acid in 45 ml of dry acetonitrile, and the resulting mixture was stirred at room temperature for 1 hour. A solution of 2.0 g of homopiperazine in 10 ml of dry acetonitrile was then added to the reaction mixture, and the mixture thus obtained was stirred at room temperature for 2 hours and at 35°C for 30 minutes. At the end of this time, the reaction mixture was concentrated by evaporation under reduced pressure, and the resulting concentrate was diluted with ethyl acetate. The diluted solution was washed with water and with an aqueous solution of sodium chloride. The ethyl acetate solution was then dried over anhydrous magnesium sulphate and concentrated by evaporation under reduced pressure. The concentrate was dissolved in 44 ml of ethyl acetate, and the solution thus obtained was mixed with 2.5 ml of a 4N solution of hydrogen chloride in ethyl acetate; the mixture was then concentrated by evaporation under reduced pressure. The residue was triturated with diethyl ether, and the resulting powder was collected by filtration and then dried, to give 4.6 g of the title compound.
Infrared Absorption Spectrum (KBr), νₘₐₓ cm⁻¹:
   1706, 1656, 1609, 1585, 1512, 1431, 1405, 1346, 1320, 1301, 1246, 1210.
Nuclear Magnetic Resonance Spectrum (hexadeuterated dimethyl sulphoxide + D₂O, 270 MHz), δ ppm:
   1.54 - 1.72 (1H, multiplet);
   1.86 - 2.14 (2H, multiplet);
   2.60 - 2.72 (1H, multiplet);
   2.94 - 3.96 (11H, multiplet);
   3.72 & 3.74 (together 3H, two singlets);
   3.79 (2H, singlet);
   4.62 - 4.82 (1H, multiplet);
   5.05 - 5.26 (2H, multiplet);
   6.87 (2H, doublet, J = 8.30 Hz);
   7.27 (2H, doublet, J = 8.30 Hz);
   7.52 & 7.60 (together 2H, two doublets, J = 8.79 Hz);
   8.22 & 8.25 (together 2H, two doublets, J = 8.79 Hz).

### 2(ii) (2S,4S)-4-(4-Methoxybenzylthio)-2-[4-(N-4-nitrobenzyloxycarbonylacetimidoyl)homopiperazin-1-ylcarbonyl]-1-(4-nitrobenzyloxycarbonyl) pyrrolidine

25 ml of methylene chloride were added to a solution of 2.5 g of (2S,4S)-4-(4-methoxybenzylthio)-2-(1-homopiperazinylcarbonyl)-1-(4-nitrobenzyloxycarbonyl)pyrrolidine hydrochloride [prepared as described in step (i) above] in 25 ml of methanol, which was being heated under reflux, and then 904 mg of N-(4-nitrobenzyloxycarbonyl)acetamidine were added to the resulting mixture. The reaction mixture was heated under reflux for a further 5 hours, after which it was worked up and purified by the same procedure as described in Preparation 1 (iii), to give 415 mg of the title compound, as a powder.
Infrared Absorption Spectrum (KBr), νₘₐₓ cm ⁻¹:
   1753, 1708, 1657, 1608, 1564, 1520, 1429, 1404, 1346, 1319, 1301, 1274, 1250, 1229.
Nuclear Magnetic Resonance Spectrum (CDCℓ₃, 270 MHz), δ ppm:
   1.70 - 2.60 (7H, multiplet);
   3.02 - 3.17 (1H, multiplet);
   3.22 - 4.60 (11H, multiplet);
   3.716 & 3.723 (together 2H, two singlets);
   3.781 & 3.786 (together 3H, two singlets);
   4.93 - 5.44 (4H, multiplet);
   6.83 & 6.85 (together 2H, two doublets, J = 8.79 Hz);
   7.22 (2H, doublet, J = 8.79 Hz);
   7.41 - 7.58 (4H, multiplet);
   8.16 - 8.26 (4H, multiplet).

### 2(iii) (2S,4S)-4-Mercapto-2-[4-(N-4-nitrobenzyloxycarbonylacetimidoyl)homopiperazin-1-ylcarbonyl]-1-(4-nitrobenzyloxycarbonyl)pyrrolidine trifluoromethanesulfonate

2.1 ml of trifluoroacetic acid and 67 µℓ of trifluoromethanesulphonic acid were added to a solution of 285 mg of (2S,4S)-4-(4-methoxybenzylthio)-2-[4-(N-4-nitrobenzyloxycarbonylacetimidoyl)homopiperazin-1-ylcarbonyl]-1-(4-nitrobenzyloxycarbonyl) pyrrolidine [prepared as described in step (ii) above] in 414 µℓ of anisole, and the resulting mixture was stirred for 1 hour, whilst ice-cooling. At the end of this time, the reaction mixture was worked up by the same procedure as described in Preparation 1(iv), to give 296 mg of the title compound, as a powder.
Infrared Absorption Spectrum (KBr), νₘₐₓ cm⁻¹:
   1781, 1701, 1632, 1609, 1523, 1495, 1437, 1406, 1348, 1279, 1258, 1225, 1213.
Nuclear Magnetic Resonance Spectrum (hexadeuterated dimethyl sulphoxide + D₂O, 270 MHz), δ ppm:
   1.55 - 4.12 (18H, multiplet);
   4.56 - 4.83 (1H, multiplet);
   5.03 - 5.31 (4H, multiplet);
   7.48 - 7.68 (4H, multiplet);
   8.17 - 8.27 (4H, multiplet).

### PREPARATION 3

### (2S,4S)-4-Mercapto-2-[(3S)-4-(N-4-nitrobenzyloxycarbonylacetimidoyl)-3-methylpiperazin-1-ylcarbonyl]1-(4-nitrobenzyloxycarbonyl)pyrrolidine trifluoromethanesulfonate

### 3(i) (2S,4S)-4-(4-Methoxybenzylthio)-2-[(3S)-3-methylpiperazin-1-ylcarbonyl]-1-(4-nitrobenzyloxycarbonyl)pyrrolidine hydrochloride

Following a procedure similar to that described in Preparation 2(i), but using 4.5 g of (2S,4S)-4-(4-methoxybenzylthio)-1-(4-nitrobenzyloxycarbonyl)-2-pyrrolidinecarboxylic acid, 1.78 g of N,N'-carbonyldiimidazole and 1.4 g of (2S)-2-methylpiperazine, 5.3 g of the title compound were obtained, as a powder.

### 3(ii) (2S,4S)-4-(4-Methoxybenzylthio)-2-[(3S)-4-(N-4-nitrobenzyloxycarbonylacetimidoyl)-3-methylpiperazin-1-ylcarbonyl]-1-(4-nitrobenzyloxycarbonyl) pyrrolidine

2.26 g of (2S,4S)-4-(4-methoxybenzylthio)-2-[(3S)-3-methylpiperazin-1-ylcarbonyl]-1-(4-nitrobenzyloxycarbonyl)pyrrolidine hydrochloride [prepared as described in step (i) above] were mixed with 1.14 g of N-(4-nitrobenzyloxycarbonyl)acetamidine and 45 ml of acetonitrile, and the mixture was heated under reflux for 16 hours. At the end of this time, the reaction mixture was worked up and purified by the same procedure as described in Preparation 1(ii), to give 922 mg of the title compound, as a powder.

### 3(iii) (2S,4S)-4-Mercapto-2-[(3S)-4-(N-4-nitrobenzyloxycarbonylacetimidoyl)-3-methylpiperazin-1-ylcarbonyl]-1-(4-nitrobenzyloxycarbonyl) pyrrolidine trifluoromethanesulfonate

Following a procedure similar to that described in Preparation 2(iii), but using 458 mg of (2S,4S)-4-(4-methoxybenzylthio)-2-[(3S)-4-(N-4-nitrobenzyloxycarbonylacetimidoyl)-3-methylpiperazin-1-ylcarbonyl]-1-(4-nitrobenzyloxycarbonyl)pyrrolidine [prepared as described in step (ii) above], 475 mg of the title compound were obtained as a powder.
Infrared Absorption Spectrum (KBr), νₘₐₓ cm⁻¹:
   1782, 1704, 1623, 1523, 1441, 1407, 1348, 1280, 1252, 1225.
Nuclear Magnetic Resonance Spectrum (hexadeuterated dimethyl sulphoxide + D₂O, 270 MHz), δ ppm:
   1.09 - 1.28 (3H, multiplet);
   1.53 - 1.78 (1H, multiplet);
   2.22 - 2.42 (1H, multiplet);
   2.67 - 3.46 (10H, multiplet);
   3.90 - 4.31 (3H, multiplet);
   4.63 - 4.90 (1H, multiplet);
   5.02 - 5.28 (4H, multiplet);
   7.46 - 7.70 (4H, multiplet);
   8.19 - 8.28 (4H, multiplet).

### PREPARATION 4

### (2S,4S)-4-Mercapto-2-[4-(N-4-nitrobenzyloxycarbonylformimidoyl)piperazin-1-ylcarbonyl)-1-(4-nitrobenzyloxycarbonyl)pyrrolidine

### 4(i) (2S,4S)-4-(4-Methoxybenzylthio)-2-[4-(N-4-nitrobenzyloxycarbonylformimidoyl)piperazin-1-ylcarbonyl]-1-(4-nitrobenzyloxycarbonyl)pyrrolidine

A suspension of 5.51 g of (2S,4S)-4-(4-methoxybenzylthio)-2-(1-piperazinylcarbonyl)-1-(4-nitrobenzyloxycarbonyl)pyrrolidine hydrochloride [prepared as described in Preparation 1(ii)] and 2.45 g of N-(4-nitrobenzyloxycarbonyl)formamidine in 10 ml of dry acetonitrile was stirred for 2 hours on a water-bath kept at 50°C. At the end of this time, the reaction mixture was freed from impurities by filtration, and the filtrate was concentrated by evaporation under reduced pressure. The resulting residue was purified by column chromatography through silica gel, using a 6 : 4 by volume mixture of ethyl acetate and acetonitrile as the eluent, to give 5.48 g of the title compound, as a powder.
Infrared Absorption Spectrum (KBr), νₘₐₓ cm⁻¹:
   1716, 1652, 1598, 1516, 1346, 1162, 1007.
Nuclear Magnetic Resonance Spectrum (CDCℓ₃, 270 MHz), δ ppm:
   1.75 - 1.95 (1H, multiplet);
   2.35 - 2.53 (1H, multiplet);
   3.05 - 3.19 (1H, multiplet);
   3.73 (2H, singlet);
   3.79 (3H, singlet);
   3.30 - 4.13 (9H, multiplet);
   4.53 - 4.66 (1H, multiplet);
   5.15 & 5.18 (2H, AB-quartet, J = 13.7 Hz);
   5.29 (2H, singlet);
   6.85 (2H, doublet, J = 8.3 Hz);
   7.23 (2H, doublet, J = 8.3 Hz);
   7.46 (2H, doublet, J = 8.3 Hz);
   7.58 (2H, doublet, J = 8.3 Hz);
   8.21 (2H, doublet, J = 8.3 Hz);
   8.23 (2H, doublet, J = 8.3 Hz);
   8.52 (1H, singlet).

### 4(ii) (2S,4S)-4-Mercapto-2-[4-(N-4-nitrobenzyloxycarbonylformimidoyl)piperazin-1-ylcarbonyl)-1-(4-nitrobenzyloxycarbonyl)pyrrolidine

15 ml of trifluoroacetic acid and 460 µℓ of trifluoromethanesulphonic acid were added to a solution of 2.50 g of (2S,4S)-4-(4-methoxybenzylthio)-2-[4-(N-4-nitrobenzyloxycarbonylformimidoyl)piperazin-1-ylcarbonyl]-1-(4-nitrobenzyloxycarbonyl) pyrrolidine [prepared as described in step (i) above] in 3 ml of anisole, and the resulting mixture was stirred for 1 hour, whilst ice-cooling. The solvent was removed by distillation under reduced pressure, and the resulting residue was washed with diethyl ether, to give 2.55 g of (2S,4S)-4-mercapto-2-[4-(N-4-nitrobenzyloxycarbonylformimidoyl)piperazin-1-ylcarbonyl]-1-(4-nitrobenzyloxycarbonyl)pyrrolidine trifluoromethanesulphonate as a powder. The whole of this product was dissolved in a mixture of ethyl acetate and water, and the solution was made alkaline by adding an aqueous solution of sodium hydrogencarbonate. The ethyl acetate layer was separated and washed with water and with an aqueous solution of sodium chloride, in that order. The solution was dried over anhydrous sodium sulphate, and then the solvent was removed by distillation under reduced pressure, to give 2.0 g of the title compound, as a powder.
Infrared Absorption Spectrum (KBr), νₘₐₓ cm⁻¹:
   1709, 1660, 1603, 1521, 1440, 1346.
Nuclear Magnetic Resonance Spectrum (CDCℓ₃, 270 MHz), δ ppm:
   1.89 (1H, doublet, J = 8.8 Hz);
   1.85 - 2.02 (1H, multiplet);
   2.63 - 2.83 (1H, multiplet);
   3.22 - 4.17 (11H, multiplet);
   4.71 (1H, triplet, J = 8.3 Hz);
   5.19 & 5.22 (together 2H, AB-quartet, J = 13.7 Hz);
   5.27 (2H, singlet);
   7.50 (2H, doublet, J = 8.8 Hz);
   7.57 (2H, doublet, J = 8.8 Hz);
   8.20 (2H, doublet, J = 8.8 Hz);
   8.22 (2H, doublet, J = 8.8 Hz);
   8.54 (1H, singlet).

### PREPARATION 5

### (2S,4S)-4-Mercapto-2-[4-(N-4-nitrobenzyloxycarbonylformimidoyl)homopiperazin-1-ylcarbonyl]-1-(4-nitrobenzyloxycarbonyl)pyrrolidine

Following a procedure similar to that described in Preparation 4, but using 2.10 g of (2S,4S)-4-(4-methoxybenzylthio)-2-(1-homopiperazinylcarbonyl)-1-(4-nitrobenzyloxycarbonyl)pyrrolidine hydrochloride [prepared as described in Preparation 2(i)] and 0.93 g of N-(4-nitrobenzyloxycarbonyl)formamidine, 2.38 g of the trifluoromethanesulphonate of the title compound were obtained. The salt was treated by the same procedure as described in Preparation 4(ii), to give 1.90 g of the title compound.
Infrared Absorption Spectrum (KBr), νₘₐₓ cm⁻¹:
   1709, 1679, 1653, 1600, 1519, 1345, 1159.
Nuclear Magnetic Resonance Spectrum (CDCℓ₃, 270 MHz), δ ppm:
   1.80 - 1.92 (2H, multiplet);
   2.00 - 2.15 (1H, multiplet);
   2.63 - 2.80 (1H, multiplet);
   3.18 - 4.35 (11H, multiplet);
   4.55 - 4.67 (1H, multiplet);
   5.10 - 5.30 (4H, multiplet);
   7.40 - 7.60 (4H, multiplet);
   8.15 - 8.26 (4H, multiplet);
   8.42 - 8.56 (1H, multiplet).

### PREPARATION 6

### (2S,4S)-4-Mercapto-2-[(3S)-4-(N-4-nitrobenzyloxy-carbonylformimidoyl)-3-methylpiperazin-1-ylcarbonyl]-1-(4-nitrobenzyloxycarbonyl)pyrrolidine trifluoromethanesulfonate

Following a procedure similar to that described in Preparation 4, but using 1.13 g of (2S,4S)-4-(4-methoxybenzylthio)-2-[(3S)-3-methylpiperazin-1-ylcarbonyl]-1-(4-nitrobenzyloxycarbonyl) pyrrolidine hydrochloride and 491 mg of N-(4-nitrobenzyloxycarbonyl)formamidine, 1.0 g of the title compound was obtained.
Infrared Absorption Spectrum (KBr), νₘₐₓ cm⁻¹:
   1785, 1688, 1608, 1523, 1444, 1408, 1349, 1248, 1223.
Nuclear Magnetic Resonance Spectrum (hexadeuterated dimethyl sulphoxide + D₂O, 270 MHz), δ ppm:
   1.10 - 1.28 (3H, multiplet);
   1.60 - 1.78 (1H, multiplet);
   2.65 - 3.45 (8H, multiplet);
   3.88 - 4.30 (3H, multiplet);
   3.65 - 3.89 (1H, multiplet);
   5.02 - 5.27 (2H, multiplet);
   5.36 (2H, singlet);
   7.49 - 7.70 (4H, multiplet);
   8.20 - 8.28 (4H, multiplet);
   8.89 (1H, singlet).

### PREPARATION 7

### (2S,4S)-4-Mercapto-2-[2-methyl-4-(4-nitrobenzyloxycarbonyl)piperazin-1-ylcarbonyl]-1-(4-nitrobenzyloxycarbonyl)pyrrolidine

### 7(i) (2S,4S)-4-(4-Methyoxybenzylthio)-2-(4-t-butoxycarbonyl-2-methylpiperazin-1-ylcarbonyl)-1-(4-nitrobenzyloxycarbonyl)pyrrolidine

3.41 ml of triethylamine and 3.03 ml of pivaloyl chloride were added dropwise to a solution of 9.99 g of (2S,4S)-4-(4-methoxybenzylthio)-1-(4-nitrobenzyloxycarbonyl)-2-pyrrolidinecarboxylic acid in 100 ml of dry acetonitrile, whilst ice-cooling, and the resulting mixture was stirred at the same temperature for 20 minutes. A solution of 5.38 g of 1-t-butoxycarbonyl-3-methylpiperazine in 50 ml of dry tetrahydrofuran was then added dropwise to the mixture, and the mixture was stirred at the same temperature for 30 minutes and then at room temperature for 2 hours. At the end of this time, the reaction mixture was concentrated by evaporation under reduced pressure, and the residue was diluted with ethyl acetate. The diluted solution was washed, in turn, with a 1N aqueous solution of oxalic acid, with water and with an aqueous solution of sodium chloride. The ethyl acetate solution was dried over anhydrous magnesium sulphate, and the solvent was removed by distillation under reduced pressure. The residue was purified by column chromatography through silica gel, using a gradient elution method, with mixtures of ethyl acetate and cyclohexane ranging from 1 : 1 to 3 : 2 by volume as the eluent, to give 8.56 g of the title compound, as an amorphous solid.
Infrared Absorption Spectrum (KBr), νₘₐₓ cm⁻¹:
   1701, 1655, 1609, 1523, 1513, 1426, 1405, 1345, 1251, 1168.
Nuclear Magnetic Resonance Spectrum (CDCℓ₃, 270 MHz), δ ppm:
   0.97 - 1.43 (3H, multiplet);
   1.47 (9H, singlet);
   1.46 - 1.89 (3H, multiplet);
   2.47 - 2.50 (1H, multiplet);
   2.71 - 3.67 (4H, multiplet);
   3.73 (2H, singlet);
   3.79 & 3.80 (together 3H, two singlets);
   3.76 - 4.82 (5H, multiplet);
   5.02 - 5.29 (2H, multiplet);
   6.85 (2H, doublet, J = 8.79 Hz);
   7.23 (2H, doublet, J = 8.79 Hz);
   7.41 & 7.46 (together 2H, two doublets, J = 8.79 Hz);
   8.17 & 8.23 (together 2H, two doublets, J = 8.79 Hz).

### 7(ii) (2S,4S)-4-(4-Methoxybenzylthio)-2-(2-methylpiperazin-1-ylcarbonyl)-2-(4-nitrobenzyloxycarbonyl)pyrrolidine

31.6 ml of a 4N solution of hydrogen chloride in ethyl acetate were added dropwise to a solution of 9.55 g of (2S,4S)-4-(4-methoxybenzylthio)-2-(4-t-butoxycarbonyl-2-methylpiperazin-1-ylcarbonyl)-1-(4-nitrobenzyloxycarbonyl)pyrrolidine [prepared as described in step (i) above] in 31.6 ml of ethyl acetate, and the resulting mixture was stirred at the same temperature for 90 minutes. At the end of this time, the reaction mixture was diluted with ethyl acetate, after which it was neutralised by adding an aqueous solution of sodium hydrogencarbonate. The ethyl acetate layer was separated, washed with an aqueous solution of sodium chloride and dried over anhydrous magnesium sulphate. The solvent was then removed by distillation under reduced pressure, and the resulting residue was purified by column chromatography through silica gel using a gradient elution method, with mixtures of ethyl acetate and methanol ranging from a 4 : 1 to 7 : 3 by volume as the eluent, to give 7.0 g of the title compound, as an amorphous solid.
Infrared Absorption Spectrum (KBr), νₘₐₓ cm⁻¹:
   1709, 1648, 1513, 1432, 1404, 1345, 1249.
Nuclear Magnetic Resonance Spectrum (CDCℓ₃, 270 MHz), δ ppm:
   1.10 - 1.46 (3H, multiplet);
   1.70 - 1.87 (1H, multiplet);
   2.18 - 3.15 (8H, multiplet);
   3.30 - 3.58 (2H, multiplet);
   3.73 (2H, singlet);
   3.79 & 3.80 (together 3H, two singlets);
   3.67 - 4.64 (3H, multiplet);
   4.97 - 5.34 (2H, multiplet);
   6.85 (2H, doublet, J = 8.30 Hz);
   7.23 (2H, doublet, J = 8.30 Hz);
   7.42 & 7.47 (together 2H, two doublets, J = 8.30 Hz);
   8.18 & 8.23 (together 2H, two doublets, J = 8.30 Hz).

### 7(iii) (2S,4S)-4-(4-Methoxybenzylthio)-2-[2-methyl-4-(4-nitrobenzyloxycarbonyl)piperazin-1-ylcarbonyl]-1-(4-nitrobenzyloxycarbonyl)pyrrolidine

0.51 g of 4-dimethylaminopyridine was added dropwise at room temperature to a solution of 1.83 g of (2S,4S)-4-(4-methoxybenzylthio)-2-(2-methylpiperazin-1-ylcarbonyl)-2-(4-nitrobenzyloxycarbonyl) pyrrolidine [prepared as described in step (ii) above] in 25 ml of dry acetonitrile, and then a solution of 0.90 g of 4-nitrobenzyl chloroformate in 15 ml of dry acetonitrile was added dropwise to the resulting mixture, whilst ice-cooling. The reaction mixture was stirred at room temperature for 30 minutes, after which it was concentrated by evaporation under reduced pressure, and the residue was diluted with ethyl acetate. The diluted solution was washed with water and with an aqueous solution of sodium chloride, in that order, after which it was dried over anhydrous magnesium sulphate. The solvent was removed by distillation under reduced pressure, and the resulting residue was purified by column chromatography through silica gel, using a 7 : 3 by volume mixture of ethyl acetate and cyclohexane as the eluent, to give 2.28 g of the title compound, as an amorphous solid.
Infrared Absorption Spectrum (KBr), νₘₐₓ cm⁻¹:
   1708, 1656, 1608, 1521, 1433, 1346, 1252.
Nuclear Magnetic Resonance Spectrum (CDCℓ₃, 270 MHz), δ ppm:
   1.02 - 1.36 (3H, multiplet);
   1.59 - 1.80 (3H, multiplet);
   2.30 - 2.58 (1H, multiplet);
   2.61 - 3.59 (5H, multiplet);
   3.73 (2H, singlet);
   3.78 & 3.79 (together 3H, two singlets);
   3.62 - 4.92 (4H, multiplet);
   4.97 - 5.30 (4H, multiplet);
   6.85 (2H, doublet, J = 8.79 Hz);
   7.23 (2H, doublet, J = 8.79 Hz);
   7.41 - 7.52 (4H, multiplet);
   8.17 & 8.23 (together 4H, two doublets, J = 8.79 Hz).

### 7(iv) (2S,4S)-4-Mercapto-2-[2-methyl-4-(4-nitrobenzyloxycarbonyl)piperazin-1-ylcarbonyl]-1-(4-nitrobenzyloxycarbonyl)pyrrolidine

23 ml of trifluoroacetic acid, followed by 0.57 ml of trifluoromethanesulphonic acid, were added dropwise to a solution of 2.26 g of (2S,4S)-4-(4-methoxybenzylthio)-2-[2-methyl-4-(4-nitrobenzyloxycarbonyl)piperazin-1-ylcarbonyl]-1-(4-nitrobenzyloxycarbonyl) pyrrolidine [prepared as described in step (iii) above] in 3.48 ml of anisole, and the resulting mixture was worked up and purified by the same procedure as described in Preparation 4(ii), to give 1.88 g of the title compound, as an amorphous solid.
Infrared Absorption Spectrum (KBr), νₘₐₓ cm⁻¹:
   1707, 1654, 1607, 1521, 1433, 1346.
Nuclear Magnetic Resonance Spectrum (CDCℓ₃, 270 MHz), δ ppm:
   1.05 - 1.40 (3H, multiplet);
   1.59 (1H, singlet);
   1.85 - 2.00 (2H, multiplet);
   2.71 - 3.73 (6H, multiplet);
   3.78 - 5.08 (5H, multiplet);
   5.15 - 5.31 (4H, multiplet);
   7.40 - 7.52 (4H, multiplet);
   8.17 - 8.25 (4H, multiplet).

### PREPARATION 8

### (2S,4S)-4-Mercapto-2-[3-methyl-4-(4-nitrobenzyloxycarbonyl)piperazin-1-ylcarbonyl]-1-(4-nitrobenzyloxycarbonyl)pyrrolidine

### 7(i) (2S,4S)-4-(4-Methoxybenzylthio)-2-[3-methyl-4-(4-nitrobenzyloxycarbonyl)piperazin-1-ylcarbonyl]-1-(4-nitrobenzyloxycarbonyl)pyrrolidine

Following a procedure similar to that described in Preparation 1(i), but using 3.06 g of (2S,4S)-4-(4-methoxybenzylthio)-1-(4-nitrobenzyloxycarbonyl)-2-pyrrolidinecarboxylic acid, 1.34 g of N,N′-carbonyldiimidazole and 2.30 g of 2-methyl-1-(4-nitrobenzyloxycarbonyl)piperazine, 4.07 g of the title compound were obtained.

### 8(ii) (2S,4S)-4-Mercapto-2-[3-methyl-4-(4-nitrobenzyloxycarbonyl)piperazin-1-ylcarbonyl]-1-(4-nitrobenzyloxycarbonyl)pyrrolidine

20 ml of trifluoroacetic acid and subsequently 0.50 ml of trifluoromethanesulphonic acid were added dropwise, whilst ice-cooling, to a solution of 2.0 g of (2S,4S)-4-(4-methoxybenzylthio)-2-[3-methyl-4-(4-nitrobenzyloxycarbonyl)piperazin-1-ylcarbonyl]-1-(4-nitrobenzyloxycarbonyl)pyrrolidine [prepared as described in step (i) above] in 3.08 ml of anisole, and the resulting mixture was stirred at the same temperature for 50 minutes. At the end of this time, the reaction mixture was worked up by the same procedure as described in Preparation 4(ii), to give 1.56 g of the title compound as an amorphous solid.
Infrared Absorption Spectrum (KBr), νₘₐₓ cm⁻¹:
   1705, 1657, 1607, 1521, 1429, 1405, 1346.
Nuclear Magnetic Resonance Spectrum (CDCℓ₃, 270 MHz), δ ppm:
   1.16 - 1.36 (3H, multiplet);
   1.63 - 2.40 (3H, multiplet);
   2.66 - 3.66 (6H, multiplet);
   3.71 - 4.78 (5H, multiplet);
   5.06 - 5.30 (4H, multiplet);
   7.39 - 7.53 (4H, multiplet);
   8.17 - 8.25 (4H, multiplet).

### PREPARATION 9

### (2S,4S)-4-Mercapto-2-[(2S)-2-methyl-4-(4-nitrobenzyloxycarbonyl)piperazin-1-ylcarbonyl]-1-(4-nitrobenzyloxycarbonyl)pyrrolidine

Following a procedure similar to that described in Preparation 7, but using 13.2 g of (2S,4S)-4-(4-methoxybenzylthio)-1-(4-nitrobenzyloxycarbonyl)-2-pyrrolidinecarboxylic acid, 4.0 ml of pivaloyl chloride, 4.5 ml of triethylamine and 6.5 g of (3S)-1-t-butoxycarbonyl-3-methylpiperazine, 1.9 g of the title compound was obtained as an amorphous solid.
Infrared Absorption Spectrum (KBr), νₘₐₓ cm⁻¹:
   1706, 1653, 1607, 1521, 1434, 1406, 1346.
Nuclear Magnetic Resonance Spectrum (CDCℓ₃, 270 MHz), δ ppm:
   1.19 - 1.30 (3H, multiplet);
   1.62 (1H, singlet);
   1.85 - 2.04 (2H, multiplet);
   2.68 - 3.59 (6H, multiplet);
   3.78 - 4.77 (5H, multiplet);
   5.08 - 5.31 (4H, multiplet);
   7.42 - 7.52 (4H, multiplet);
   8.17 - 8.25 (4H, multiplet).

### PREPARATION 10

### (2S,4S)-4-Mercapto-2-[(2R)-2-methyl-4-(4-nitrobenzyloxycarbonyl)piperazin-1-ylcarbonyl]-1-(4-nitrobenzyloxycarbonyl)pyrrolidine

Following a procedure similar to that described in Preparation 7, but using 1.3 g of (2S,4S)-4-(4-methoxybenzylthio)-1-(4-nitrobenzyloxycarbonyl)-2-pyrrolidinecarboxylic acid, 0.40 ml of pivaloyl chloride, 0.45 ml of triethylamine and 0.65 g of (3R)-1-t-butoxycarbonyl-3-methylpiperazine, 0.17 g of the title compound was obtained as an amorphous solid.
Infrared Absorption Spectrum (KBr), νₘₐₓ cm⁻¹:
   1708, 1655, 1607, 1521, 1432, 1435.

### PREPARATION 11

### (2S,4S)-4-Mercapto-2-[(3S)-3-methyl-4-(N-4-nitrobenzyloxycarbonyl)piperazin-1-ylcarbonyl]-1-(4-nitrobenzyloxcarbonyl)pyrrolidine

Following a procedure similar to that described in Preparation 8, but using 4.5 g of (2S,4S)-4-(4-methoxybenzylthio)-1-(4-nitrobenzyloxycarbonyl)-2-pyrrolidinecarboxylic acid, 1.95 g of N,N'-carbonyldiimidazole and 3.34 g of (2S)-2-methyl-1-(4-nitrobenzyloxycarbonyl)-piperazine, 4.32 g of the title compound were obtained as an amorphous solid.
Infrared Absorption Spectrum (KBr), νₘₐₓ cm⁻¹:
   1705, 1657, 1607, 1522, 1429, 1405, 1346.
Nuclear Magnetic Resonance Spectrum (CDCℓ₃, 270 MHz), δ ppm:
   1.16 - 1.36 (3H, multiplet);
   1.62 (1H, singlet);
   1.70 - 2.04 (2H, multiplet);
   2.69 - 2.86 (2H, multiplet);
   2.98 - 4.18 (6H, multiplet);
   4.23 - 4.74 (3H, multiplet);
   5.02 - 5.33 (4H, multiplet);
   7.40 - 7.53 (4H, multiplet);
   8.17 - 8.26 (4H, multiplet).

### PREPARATION 12

### (2S,4S)-4-Mercapto-2-[(3R)-3-methyl-4-(4-nitrobenzyloxycarbonyl)piperazin-1-ylcarbonyl]-1-(4-nitrobenzyloxycarbonyl)pyrrolidine

Following a procedure similar to that described in Preparation 8, but using 0.23 g of (2S,4S)-4-(4-methoxybenzylthio)-1-(4-nitrobenzyloxycarbonyl)-2-pyrrolidinecarboxylic acid, 0.10 g of N,N'-carbonyldiimidazole and 0.17 g of (2R)-2-methyl-1-(4-nitrobenzyloxycarbonyl)piperazine, 0.21 g of the title compound was obtained as an amorphous solid.
Infrared Absorption Spectrum (KBr), νₘₐₓ cm⁻¹:
   1708, 1652, 1607, 1523, 1427, 1346.

### PREPARATION 13

### (2S,4S)-2-[trans-2,5-Dimethyl-4-(4-nitrobenzyloxycarbonyl)piperazin-1-ylcarbonyl]-4-mercapto-1-(4-nitrobenzyloxycarbonyl)pyrrolidine

Following a procedure similar to that described in Preparation 8, but using 1.79 g of (2S,4S)-4-(4-methoxybenzylthio)-1-(4-nitrobenzyloxycarbonyl)-2-pyrrolidinecarboxylic acid, 0.54 ml of pivaloyl chloride, 0.61 ml of triethylamine and 1.29 g of trans-2,5-dimethyl-1-(4-nitrobenzyloxycarbonyl)piperazine, 577 mg of the title compound were obtained as an amorphous solid.
Infrared Absorption Spectrum (KBr), νₘₐₓ cm⁻¹:
   1707, 1653, 1608, 1522, 1425, 1347.
Nuclear Magnetic Resonance Spectrum (CDCℓ₃, 270 MHz), δ ppm:
   0.87 - 1.39 (6H, multiplet);
   1.61 (1H, singlet);
   1.68 - 2.04 (1H, multiplet);
   2.60 - 2.88 (1H, multiplet);
   2.95 - 3.59 (5H, multiplet);
   4.07 - 4.95 (5H, multiplet);
   4.97 - 5.36 (4H, multiplet);
   7.40 - 7.53 (4H, multiplet);
   8.16 - 8.25 (4H, multiplet).

### PREPARATION 14

### (2S,4S)-2-[cis-3,5-Dimethylpiperazin-1-ylcarbonyl)-4-mercapto-1-(4-nitrobenzyloxycarbonyl)pyrrolidine

Following a procedure similar to that described in Preparation 8, but using 3.3 g of (2S,4S)-4-(4-methoxybenzylthio)-1-(4-nitrobenzyloxycarbonyl)-2-pyrrolidinecarboxylic acid, 1.4 g of N,N'-carbonyldiimidazole and 1.0 g of cis-2,6-dimethylpiperazine, 1.56 g of the title compound was obtained as an amorphous solid.
Infrared Absorption Spectrum (KBr), νₘₐₓ cm⁻¹:
   1709, 1651, 1608, 1522, 1439, 1405, 1346.
Nuclear Magnetic Resonance Spectrum (CDCℓ₃, 270 MHz), δ ppm:
   1.05 - 1.29 (6H, multiplet);
   1.85 - 1.96 (2H, multiplet);
   2.12 - 3.72 (9H, multiplet);
   4.04 - 4.17 (1H, multiplet);
   4.40 - 4.74 (2H, multiplet);
   5.03 - 5.36 (2H, multiplet);
   7.40 - 7.52 (2H, multiplet);
   8.17 - 8.23 (2H, multiplet).

### PREPARATION 15

### (2S,4S)-4-Mercapto-2-[4-[2-(4-nitrobenzyloxycarbonyl)oxyethyl]-1-homopiperazinylcarbonyl]-1-(4-nitrobenzyloxycarbonyl)pyrrolidine trifluoromethanesulfonate

Following a procedure similar to that described in Preparation 8, but using 5.0 g of (2S,4S)-4-(4-methoxybenzylthio)-1-(4-nitrobenzyloxycarbonyl)-2-pyrrolidinecarboxylic acid, 2.2 g of N,N'-carbonyldiimidazole and 6:81 g of N-[2-(4-nitrobenzyloxycarbonyl)oxyethyl]homopiperazine bis(trifluoroacetate), 6.50 g of the title compound were obtained.
Nuclear Magnetic Resonance Spectrum (hexadeuterated dimethyl sulphoxide, 270 MHz), δ ppm:
   1.60 - 1.83 (1H, multiplet);
   1.94 - 2.25 (2H, multiplet);
   2.65 - 2.90 (1H, multiplet);
   3.00 - 4.85 (18H, multiplet);
   5.02 - 5.40 (4H, multiplet);
   7.49 - 7.71 (4H, multiplet);
   8.18 - 8.30 (4H, multiplet).

### PREPARATION 16

### (2S,4S)-4-Mercapto-2-(4-carbamoylmethyl-1-homopiperazinylcarbonyl)-1-(4-nitrobenzyloxycarbonyl)-pyrrolidine

Following a procedure similar to that described in Preparation 8, but using 5.69 g of (2S,4S)-4-(4-methoxybenzylthio)-1-(4-nitrobenzyloxycarbonyl)-2-pyrrolidinecarboxylic acid, 2.48 g of N,N'-carbonyldiimidazole and 5.89 g of N-carbamoylmethylhomopiperazine bis(trifluoroacetate), 4.88 g of the title compound were obtained.
Infrared Absorption Spectrum (KBr), νₘₐₓ cm⁻¹:
   1707, 1680, 1647, 1520, 1432, 1404, 1344.
Nuclear Magnetic Resonance Spectrum (hexadeuterated dimethyl sulphoxide + D₂O, 270 MHz), δ ppm:
   1.75 - 2.15 (3H, multiplet);
   2.55 - 2.92 (4H, multiplet);
   2.95 - 3.07 (1H, multiplet);
   3.20 - 3.60 (6H, multiplet);
   3.70 - 3.85 (1H, multiplet);
   3.93 - 4.18 (2H, multiplet);
   4.60 - 4.71 (1H, multiplet);
   5.03 - 5.42 (2H, multiplet);
   7.42 - 7.52 (2H, multiplet);
   8.18 - 8.25 (2H, multiplet).

### PREPARATION 17

### (2S,4S)-4-Mercapto-2-[4-(N-4-nitrobenzyloxycarbonylacetimidoyl)piperazin-1-ylcarbonyl]-1-(4-nitrobenzyloxycarbonyl)pyrrolidine

### 17(i) (2S,4S)-4-(4-Methoxybenzlthio)-2-(1-piperazinylcarbonyl)-1-(4-nitrobenzyloxycarbonyl)pyrrolidine hydrochloride

3.57 g of N,N'-carbonyldiimidazole were added to a solution of 8.93 g of (2S,4S)-4-(4-methoxybenzylthio)-1(4-nitrobenzyloxycarbonyl)-2-pyrrolidinecarboxylic acid in 89 ml of dry acetonitrile, and the resulting mixture was stirred at room temperature for 30 minutes. The reaction mixture thus obtained was then added to a solution of 5.17 g of dry piperazine in 178 ml of dry acetonitrile, whilst ice-cooling, and the mixture was stirred for 4 hours under the same conditions, after which it was concentrated by evaporation under reduced pressure. The resulting residue was dissolved in 500 ml of ethyl acetate, and the solution was washed four times, each time with 300 ml of water, and then once with 300 ml of an aqueous solution of sodium chloride. The ethyl acetate layer was separated and dried over anhydrous sodium sulphate, after which 6 ml of a 4N solution of hydrogen chloride in ethyl acetate were added dropwise to the mixture, whilst stirring, and then 500 ml of diethyl ether were added. The powder thus produced was collected by filtration and dried to give 11.49 g of the title compound.

The spectral data of this product are completely identical with those of the compound prepared as described in Preparation 1(ii).

### 17(ii) (2S,4S)-4-(4-Methoxybenzylthio)-2-[4-(N-4-nitrobenzyloxycarbonyl)pyrrolidine

A suspension of 5.0 g of (2S,4S)-4-(4-methoxybenzylthio)-2-(1-piperazinylcarbonyl)-1-(4-nitrobenzyloxycarbonyl)pyrrolidine hydrochloride [prepared as described in step (i) above] and 2.35 g of N-(4-nitrobenzyloxycarbonyl)acetamidine in 73 ml of dry acetonitrile was stirred on a water-bath kept at 48°C for 3 hours. At the end of this time, the reaction mixture was freed from impurities by filtration, and the filtrate was concentrated by evaporation under reduced pressure. The resulting residue was purified by column chromatography through silica gel, using a 6 : 4 by volume mixture of ethyl acetate and acetonitrile as the eluent, to give 5.41 g of the title compound, as a powder.

The spectral data of this product are completely identical with those of the compound prepared as described in Preparation 1(iii).

### 17(iii) (2S,4S)-4-Mercapto-2-[4-(N-4-nitrobenzyloxycarbonylacetimidoyl)piperazin-1-ylcarbonyl]-1-(4-nitrobenzyloxycarbonyl)pyrrolidine

25 ml of trifluoroacetic acid and 1000 µℓ of trifluoromethanesulphonic acid were added to a solution of 4.90 g of (2S,4S)-4-(4-methoxybenzylthio)-2-[4-(N-4-nitrobenzyloxycarbonyl)pyrrolidine [prepared as described in step (ii) above] in 4.9 ml of anisole, and the resulting mixture was stirred for 1 hour, whilst ice-cooling, after which the solvent was removed by distillation under reduced pressure. The resulting residue was washed with diethyl ether, to produce a powder. This powder was dissolved in a mixture of ethyl acetate and water, and the resulting solution was made alkaline by the addition of an aqueous solution of sodium hydrogencarbonate. The ethyl acetate layer was separated, washed with water and with an aqueous solution of sodium chloride, in that order, and dried over anhydrous sodium sulphate. The solvent was removed by distillation under reduced pressure, to give 4.0 g of the title compound, as a powder.
Infrared Absorption Spectrum (KBr), νₘₐₓ cm⁻¹:
   1709, 1660, 1607, 1570, 1520, 1431, 1346, 1210, 1198, 1162.
Nuclear Magnetic Resonance Spectrum (CDCℓ₃, 270 MHz), δ ppm:
   1.90 (1H, doublet, J = 8.8 Hz);
   1.85 - 2.01 (1H, multiplet);
   2.25 & 2.30 (together 3H, two singlets);
   2.65 - 2.81 (1H, multiplet);
   3.21 - 3.38 (1H, multiplet);
   3.40 - 4.00 (8H, multiplet);
   4.03 - 4.19 (2H, multiplet);
   4.65 & 4.70 (together 1H, two triplets, J = 7.8 Hz);
   5.02 - 5.35 (4H, multiplet);
   7.41 - 7.60 (4H, multiplet);
   8.16 - 8.26 (4H, multiplet).

### PREPARATION 18

### (2S,4S)-4-mercapto-2-[(3S)-3-(4-nitrobenzyloxycarbonyl)aminopyrrolidin-1-ylcarbonyl]-1-(4-nitrobenzyloxycarbonyl)pyrrolidine

### 18(i) (2S,4S)-4-(4-Methoxybenzylthio)-2-[(3S)-3-(4-nitrobenzyloxycarbonyl)aminopyrrolidin-1-ylcarbonyl]-1-(4-nitrobenzyloxycarbonyl)pyrrolidine

A solution of 1.43 g of (2S,4S)-4-(4-methoxybenzylthio)-1-(4-nitrobenzyloxycarbonyl)-2-pyrrolidinecarboxylic acid dissolved in 10 ml of dry tetrahydrofuran was cooled to 0°C. 356 mg of triethylamine, followed by 405 mg of pivaloyl chloride, were added to the cooled solution, and the resulting mixture was stirred at the same temperature for 30 minutes. At the end of this time, a mixture of 1.5 g of (3S)-3-(4-nitrobenzyloxycarbonyl)aminopyridine trifluoroacetate, 830 mg of diisopropylethylamine and 7 ml of dry acetonitrile was added to the solution, and then the temperature was allowed to rise. The reaction mixture was stirred at room temperature for 2.5 hours, after which it was filtered, and the filtrate was freed from the solvent by distillation under reduced pressure. The resulting residue was diluted with ethyl acetate, and the diluted solution was washed with an aqueous solution of sodium hydrogencarbonate and with a saturated aqueous solution of sodium chloride, in that order. The solution was dried over anhydrous magnesium sulphate, and then the solvent was removed by distillation under reduced pressure, and the residue was purified by column chromatography through silica gel, using a 4 : 4 : 1 by volume mixture of ethyl acetate, methylene chloride and acetonitrile as the eluent, to give 1.47 g of the title compound, as a powder.
Infrared Absorption Spectrum (KBr), νₘₐₓ cm⁻¹:
   1716, 1625, 1609, 1519, 1346, 737.
Nuclear Magnetic Resonance Spectrum (hexadeuterated dimethyl sulphoxide, 270 MHz), δ ppm:
   1.54 - 1.65 (1H, multiplet);
   1.72 - 1.86 (1H, multiplet);
   2.57 - 2.69 (1H, multiplet);
   2.99 - 3.13 (1H, multiplet);
   3.72 (3H, doublet, J = 5.37 Hz);
   3.15 - 4.15 (12H, multiplet);
   4.36 - 4.58 (1H, multiplet);
   5.00 - 5.23 (4H, multiplet);
   6.87 (1H, doublet, J = 8.3 Hz);
   7.26 (1H, doublet, J = 8.79 Hz);
   7.46 - 7.62 (4H, multiplet);
   7.70 - 7.80 (1H, multiplet);
   8.15 - 8.25 (4H, multiplet).

### 18(ii) (2S,4S)-4-mercapto-2-[(3S)-3-(4-nitrobenzyloxycarbonyl)aminopyrrolidin-1-ylcarbonyl]-1-(4-nitrobenzyloxycarbonyl)pyrrolidine

12 ml of trifluoroacetic acid and 0.38 ml of trifluoromethanesulphonic acid were added to a suspension of 1.47 g of (2S,4S)-4-(4-methoxybenzylthio)-2-[(3S)-3-(4-nitrobenzyloxycarbonyl)aminopyrrolidin-1-yl-carbonyl]-1-(4-nitrobenzyloxycarbonyl)pyrrolidine [prepared as described in step (i) above] in 2.3 ml of anisole, and the resulting mixture was stirred at room temperature for 2 hours. At the end of this time, the solvent was removed by distillation under reduced pressure, and the residue was washed with hexane to remove the anisole, and then diethyl ether was added. The mixture was then cooled to -78°C, and the solidified product was broken up and separated by decantation. Several repetitions of this procedure yielded a powder and oily materials, which were dissolved in 100 ml of ethyl acetate to give a solution. The resulting solution was washed with an aqueous solution of sodium hydrogencarbonate, and the aqueous washings were extracted with 30 ml of ethyl acetate. The washings were combined with the ethyl acetate solution, and the combined organic phase was washed with an aqueous solution of sodium chloride and dried over anhydrous magnesium sulphate. The solvent was then removed by distillation under reduced pressure, to give 1.26 g of the title compound, as a powder.
Infrared Absorption Spectrum (KBr), νₘₐₓ cm⁻¹:
   1710, 1522, 1347, 854, 738.
Nuclear Magnetic Resonance Spectrum (hexadeuterated dimethyl sulphoxide, 270 MHz), δ ppm:
   1.60 - 2.20 (2H, multiplet);
   2.62 - 2.75 (1H, multiplet);
   3.08 - 4.13 (11H, multiplet);
   4.37 - 4.59 (1H, multiplet);
   5.02 - 5.26 (4H, multiplet);
   7.47 - 7.81 (4H, multiplet);
   8.16 - 8.26 (4H, multiplet).

### PREPARATION 19

### (2S,4S)-4-Mercapto-2-[(3S)-1-(4-nitrobenzyloxycarbonyl)pyrrolidin-3-ylaminocarbonyl]-1-(4-nitrobenzyloxycarbonyl)pyrrolidine

### 19(i) (2S,4S)-4-(4-Methoxybenzylthio)-2-[(3S)-1-(t-butoxycarbonyl)pyrrolidin-3-ylaminocarbonyl]-1-(4-nitrobenzyloxycarbonyl)pyrrolidine

3.05 g of N,N'-carbonyldiimidazole were added to a solution of 7.99 g of (2S,4S)-4-(4-methoxybenzylthio)-1-(4-nitrobenzyloxycarbonyl)-2-pyrrolidinecarboxylic acid in 80 ml of dry acetonitrile, and the resulting mixture was stirred at room temperature for 2 hours. At the end of this time, the reaction mixture was cooled to 0°C, after which a solution of 3.34 g of (3S)-3-amino-1-t-butoxycarbonylpyrrolidine in 30 ml of dry acetonitrile was added, and the mixture was stirred at the same temperature for 20 minutes; it was then stirred at room temperature for a further 1.4 hours and at 32°C for 45 minutes. The reaction mixture was then concentrated by evaporation under reduced pressure, and the concentrate was diluted with 200 ml of ethyl acetate. The resulting ethyl acetate solution was washed twice with water and then once with a saturated aqueous solution of sodium chloride. The solution was then dried over anhydrous magnesium sulphate, after which the solvent was removed by distillation under reduced pressure. The residue was recrystallised from diethyl ether, to give 9.11 g of the title compound, as a powder.

### 19(ii) (2S,4S)-4-(4-Methoxybenzylthio)-2-[(3S)-pyrrolidin-3-ylaminocarbonyl]-1-(4-nitrobenzyloxycarbonyl)pyrrolidine hydrochloride

A mixture of 1.00 g of (2S,4S)-4-(4-methoxybenzylthio)-2-[(3S)-1-(t-butoxycarbonyl)pyrrolidin-3-ylaminocarbonyl]-1-(4-nitrobenzyloxycarbonyl) pyrrolidine [prepared as described in step (i) above] and 10 ml of ethyl acetate was heated to form a solution. 2.5 ml of a 4N solution of hydrogen chloride in ethyl acetate were then added to this solution, and the resulting mixture was heated under reflux for 30 minutes. At the end of this time, the solvent was removed by distillation under reduced pressure, and then, in order to remove the acid, ethyl acetate was added to the residue and the solvent was again removed by distillation under reduced pressure. The residue was triturated with diethyl ether and washed by decantation to give 630 mg of the title compound, as a powder.

### 19(iii) (2S,4S)-4-(4-Methoxybenzylthio)-2-[(3S)-1-(4-nitrobenzyloxycarbonyl)pyrrolidin-3-ylaminocarbonyl]-1-(4-nitrobenzyloxycarbonyl)pyrrolidine

230 mg of diisopropylethylamine were added to a suspension of 1.0 g of (2S,4S)-4-(4-methoxybenzylthio)2-[(3S)-pyrrolidin-3-ylaminocarbonyl]-1-(4-nitrobenzyloxycarbonyl)pyrrolidine hydrochloride [prepared as described in step (ii) above] and 240 mg of 4-dimethylaminopyridine in 10 ml of dry acetonitrile, and the resulting mixture was cooled to 0°C. A solution of 430 mg of 4-nitrobenzyl chloroformate in 4 ml of dry acetonitrile was then added to the mixture. The mixture was then stirred at room temperature for 3 hours, after which a solution of 117 mg of 4-nitrobenzyl chloroformate in 2 ml of dry acetonitrile was added and the mixture was stirred at the same temperature for 1 hour. At the end of this time, the reaction mixture was freed from the solvent by distillation under reduced pressure, and the residue was diluted with 50 ml of methylene chloride. The diluted solution was washed with a saturated aqueous solution of sodium chloride. The aqueous washings were extracted with methylene chloride. The organic extract and the methylene chloride solution were combined and dried over anhydrous magnesium sulphate, and then the solvent was removed by distillation under reduced pressure. The resulting residue was purified by column chromatography through silica gel, using a 4 : 1 by volume mixture of methylene chloride and acetonitrile as the eluent, to give 862 mg of the title compound as a powder.
Infrared Absorption Spectrum (KBr), νₘₐₓ cm⁻¹:
   1709, 1521, 1345, 1109, 854, 738.
Nuclear Magnetic Resonance Spectrum (CDCℓ₃, 270 MHz), δ ppm:
   1.80 - 1.98 (1H, multiplet);
   2.05 - 2.25 (1H, multiplet);
   2.30 - 2.50 (1H, multiplet);
   3.10 - 3.20 (1H, multiplet);
   3.40 - 3.55 (2H, multiplet);
   3.79 (3H, singlet);
   3.20 - 3.90 (8H, multiplet);
   4.20 - 4.30 (1H, multiplet);
   4.42 - 4.48 (1H, multiplet);
   5.13 - 5.26 (4H, multiplet);
   6.82 - 6.87 (2H, multiplet);
   7.19 - 7.26 (2H, multiplet);
   7.45 - 7.53 (4H, multiplet);
   8.19 - 8.24 (4H, multiplet).

### 19(iv) (2S,4S)-4-Mercapto-2-[(3S)-1-(4-nitrobenzyloxycarbonyl)pyrrolidin-3-ylaminocarbonyl]-1-(4-nitrobenzyloxycarbonyl)pyrrolidine

6.5 ml of trifluoroacetic acid and 0.21 ml of trifluoromethanesulphonic acid were added to a suspension of 835 mg of (2S,4S)-4-(4-methoxybenzylthio)-2-[(3S)-1-(4-nitrobenzyloxycarbonyl)pyrrolidin-3-ylaminocarbonyl]-1-(4-nitrobenzyloxycarbonyl) pyrrolidine [prepared as described in step (iii) above] in 1.3 ml of anisole, whilst ice-cooling, and the resulting mixture was stirred at room temperature for 1 hour. At the end of this time, the solvent was removed by distillation under reduced pressure, and the resulting residue was first washed with hexane to remove the anisole and then mixed with diethyl ether. The mixture was cooled to -78°C, and the solidified product was broken up and separated by decantation. Several repetitions of this procedure yielded 940 mg of the title compound as a powder.
Infrared Absorption Spectrum (KBr), νₘₐₓ cm⁻¹:
   1702, 1523, 1347, 856, 739.
Nuclear Magnetic Resonance Spectrum (hexadeuterated dimethyl sulphoxide + D₂O, 270 MHz), δ ppm:
   1.55 - 2.15 (2H, multiplet);
   2.55 - 2.65 (1H, multiplet);
   3.05 - 3.61 (7H, multiplet);
   3.87 - 4.02 (1H, multiplet);
   4.10 - 4.26 (2H, multiplet);
   5.06 - 5.20 (4H, multiplet);
   7.55 - 7.65 (4H, multiplet);
   8.18 - 8.25 (4H, multiplet).

### PREPARATION 20

### (2S,4S)-4-Mercapto-2-[(3R)-1-(4-nitrobenzyloxycarbonyl)pyrrolidin-3-ylaminocarbonyl]-1-(4-nitrobenzyloxycarbonyl)pyrrolidine

Following a procedure similar to that described in Preparation 19, but using 6.4 g of (2S,4S)-4-(4-methoxybenzylthio)-1-(4-nitrobenzyloxycarbonyl)-2-pyrrolidinecarboxylic acid, 2.40 g of N,N'-carbonyldiimidazole and 2.7 g of (3R)-3-amino-1-t-butoxycarbonylpyrrolidine, 750 mg of the title compound were obtained as a powder.
Infrared Absorption Spectrum (KBr), νₘₐₓ cm⁻¹:
   1705, 1522, 1347, 855, 735.

### PREPARATION 21

### (2S,4S)-4-Mercapto-2-[(3S)-3-dimethylaminopyrrolidin-1-ylcarbonyl]-1-(4-nitrobenzyloxycarbonyl)pyrrolidine trifluoromethanesulfonate

### 21(i) (2S,4S)-4-(4-Methoxybenzylthio)-2-[(3S)-3-dimethylaminopyrrolidin-1-ylcarbonyl-1-(4-nitrobenzyloxycarbonyl)pyrrolidine

A solution of 924 mg of (2S,4S)-4-(4-methoxybenzylthio)-1-(4-nitrobenzyloxycarbonyl)-2-pyrrolidinecarboxylic acid in 10 ml of tetrahydrofuran was cooled to -20°C, and 209 mg of triethylamine were added, followed by 250 mg of pivaloyl chloride. The resulting mixture was stirred at the same temperature for 5 minutes, and then a mixture of 651 mg of (3S)-3-dimethylaminopyrrolidine trifluoroacetate, 560 ml of diisopropylethylamine and 7 ml of dry acetonitrile was added to the mixture. The temperature of the reaction mixture was allowed to rise gradually, and the mixture was stirred at 0°C for 1 hour. The solvent was then removed by distillation under reduced pressure. The resulting residue was diluted with ethyl acetate and the solution was washed with an aqueous solution of sodium hydrogencarbonate and with a saturated aqueous solution of sodium chloride, in that order. The organic solution was dried over anhydrous magnesium sulphate, and the solvent was removed by distillation under reduced pressure. The residue was purified by column chromatography through silica gel, using a 3 : 1 by volume mixture of acetonitrile and methanol as the eluent to give 884 mg of the title compound, as a powder.
Infrared Absorption Spectrum (KBr), νₘₐₓ cm⁻¹:
   1710, 1654, 1512, 1345, 1109, 857, 738.
Nuclear Magnetic Resonance Spectrum (hexadeuterated dimethyl sulphoxide, 270 MHz), δ ppm:
   1.49 - 3.31 (15H, multiplet);
   3.35 - 3.57 (2H, multiplet);
   3.71 - 4.00 (6H, multiplet);
   4.44 - 4.56 (1H, multiplet);
   5.00 - 5.21 (2H, multiplet);
   6.88 (2H, doublet, J = 8.79 Hz);
   7.27 (2H, doublet, J = 8.31 Hz);
   7.51 - 7.61 (2H, multiplet);
   8.19 - 8.26 (2H, multiplet).

### 21(ii) (2S,4S)-4-Mercapto-2-[(3S)-3-dimethylaminopyrrolidin-1-ylcarbonyl]-1-(4-nitrobenzyloxycarbonyl)pyrrolidine trifluoromethanesulfonate

8.5 ml of trifluoroacetic acid and 0.28 ml of trifluoromethanesulphonic acid were added, whilst ice-cooling, to a suspension of 845 mg of (2S,4S)-4-(4-methoxybenzylthio)-2-[(3S)-3-dimethylaminopyrrolidin-1-ylcarbonyl-1-(4-nitrobenzyloxycarbonyl) pyrrolidine [prepared as described in step (i) above] in 1.7 ml of anisole, and the resulting mixture was stirred at room temperature for 1 hour. At the end of this time, the solvent was removed by distillation under reduced pressure, and the resulting residue was first washed with hexane to remove the anisole and then mixed with diethyl ether. The mixture was cooled to -78°C, and the solidified product was broken up and separated by decantation. Several repetitions of this procedure yielded 1.14 g of the title compound as a powder.
Infrared Absorption Spectrum (KBr), νₘₐₓ cm⁻¹:
   1705, 1656, 1523, 1348, 857.
Nuclear Magnetic Resonance Spectrum (hexadeuterated dimethyl sulphoxide + D₂O, 270 MHz), δ ppm:
   1.70 - 4.10 (18H, multiplet);
   4.47 - 4.66 (1H, multiplet);
   5.04 - 5.27 (2H, multiplet);
   7.51 - 7.65 (2H, multiplet).

### PREPARATION 22

### (2S,4S)-4-Mercapto-2-{(3S)-3-[N-methyl-N-(4-nitrobenzyloxycarbonyl)amino]pyrrolidin-1-ylcarbonyl}-1-(4-nitrobenzyloxycarbonyl)pyrrolidine

Following a procedure similar to that described in Preparation 18, but using 1.21 g of (2S,4S)-4-(4-methoxybenzylthio)-1-(4-nitrobenzyloxycarbonyl)-2-pyrrolidinecarboxylic acid, 343 mg of pivaloyl chloride and 1.27 g of (3S)-3-[N-methyl-N-(4-nitrobenzyloxycarbonyl)amino]pyrrolidine trifluoroacetate, 1.21 g of the title compound were obtained as a powder.
Infrared Absorption Spectrum (KBr), νₘₐₓ cm⁻¹:
   1709, 1651, 1522, 1346, 856, 737.

### PREPARATION 23

### (2S,4S)-4-Mercapto-2-[(3S)-3-(N-4-nitrobenzyloxy-carbonylacetimidoylamino)pyrrolidin-1-ylcarbonyl]-1-(4-nitrobenzyloxycarbonyl)pyrrolidine

### 23(i) (2S,4S)-4-(4-Methoxybenzylthio)-2-[(3S)-3-aminopyrrolidin-1-ylcarbonyl]-1-(4-nitrobenzyloxycarbonyl)pyrrolidine

2.92 g of N,N'-carbonyldiimidazole were added to a solution of 6.70 g of (2S,4S)-4-(4-methoxybenzylthio)-1-(4-nitrobenzyloxycarbonyl)-2-pyrrolidinecarboxylic acid in 50 ml of dry acetonitrile, and the resulting mixture was stirred at room temperature for 1 hour. A solution of 1.55 g of (3S)-3-aminopyrrolidine in 10 ml of dry acetonitrile was then added to the mixture, whilst ice-cooling, and the mixture was stirred at room temperature for 1 hour. At the end of this time, the reaction mixture was freed from the solvent by distillation under reduced pressure, and the residue was diluted with ethyl acetate. The diluted solution was washed with water and with an aqueous solution of sodium chloride, in that order, after which it was dried over anhydrous magnesium sulphate. The solvent was then removed by distillation under reduced pressure, and the resulting residue was purified by column chromatography through silica gel, using a 1 : 1 by volume mixture of ethyl acetate and methanol as the eluent, to give 4.10 g of the title compound, as a powder.
Infrared Absorption Spectrum (KBr), νₘₐₓ cm⁻¹:
   1708, 1651, 1609, 1512, 1440, 1404, 1346, 1248, 1174.
Nuclear Magnetic Resonance Spectrum (hexadeuterated dimethyl sulphoxide, 270 MHz), δ ppm:
   1.40 - 2.04 (3H, multiplet);
   2.57 - 2.77 (1H, multiplet);
   2.90 - 3.93 (10H, multiplet);
   3.72 & 3.74 (together 3H, two singlets);
   3.78 (2H, singlet);
   4.33 - 4.58 (1H, multiplet);
   4.99 - 5.26 (2H, multiplet);
   6.88 (2H, doublet, J = 8.79 Hz);
   7.27 (2H, doublet, J = 8.79 Hz);
   7.48 - 7.67 (2H, multiplet);
   8.14 - 8.29 (2H, multiplet).

### 23(ii) (2S,4S)-4-(4-Methoxybenzylthio)-2-[(3S)-3-aminopyrrolidin-1-ylcarbonyl]-1-(4-nitrobenzyloxycarbonyl)pyrrolidine hydrochloride

4.37 ml of a 4N solution of hydrogen chloride in ethyl acetate were added, whilst ice-cooling, to a solution of 3.00 g of (2S,4S)-4-(4-methoxybenzylthio)-2-[(3S)-3-aminopyrrolidin-1-ylcarbonyl]-1-(4-nitrobenzyloxycarbonyl)pyrrolidine [prepared as described in step (i) above] in 30 ml of ethyl acetate, and the resulting mixture was stirred at the same temperature for 30 minutes. At the end of this time, it was diluted with ethyl acetate, and the powder which precipitated was collected by filtration and dried, to give 3.20 g of the title compound, as a powder.
Infrared Absorption Spectrum (KBr), νₘₐₓ cm⁻¹:
   1707, 1656, 1609, 1585, 1512, 1440, 1405, 1346, 1249, 1175.
Nuclear Magnetic Resonance Spectrum (hexadeuterated dimethyl sulphoxide, 270 MHz), δ ppm:
   1.49 - 1.78 (1H, multiplet);
   1.88 - 2.33 (2H, multiplet);
   2.59 - 2.75 (1H, multiplet);
   2.96 - 3.12 (1H, multiplet);
   3.12 - 3.97 (7H, multiplet);
   3.72 & 3.74 (together 3H, two singlets);
   3.78 & 3.79 (together 2H, two singlets);
   4.36 - 4.61 (1H, multiplet);
   5.00 - 5.28 (2H, multiplet);
   6.88 (2H, doublet, J = 8.79 Hz);
   7.20 - 7.31 (2H, multiplet);
   7.46 - 7.65 (2H, multiplet);
   8.19 - 8.28 (2H, multiplet);
   8.30 - 8.60 (3H, multiplet).

### 23(iii) (2S,4S)-4-(4-Methoxybenzylthio)-2-[(3S)-3-(N-4-nitrobenzyloxycarbonylacetimidoylamino)pyrrolidin-1-ylcarbonyl]-1-(4-nitrobenzyloxycarbonyl)pyrrolidine

A suspension of 1.00 g of (2S,4S)-4-(4-methoxybenzylthio)-2-[(3S)-3-aminopyrrolidin-1-ylcarbonyl]-1-(4-nitrobenzyloxycarbonyl) pyrrolidine hydrochloride [prepared as described in step (ii) above] and 0.47 g of N-(4-nitrobenzyloxycarbonyl)acetamidine in 20 ml of dry acetonitrile was stirred at 53°C for 2 hours. The reaction mixture was then freed from impurities by filtration, and the filtrate was concentrated by evaporation under reduced pressure. The concentrate was purified by column chromatography through silica gel, using a 45 : 45 : 5 by volume mixture of methylene chloride, ethyl acetate and methanol as the eluent, to give 0.89 g of the title compound, as a powder.
Infrared Absorption Spectrum (KBr), νₘₐₓ cm⁻¹:
   1709, 1643, 1619, 1609, 1557, 1521, 1441, 1402, 1346, 1247, 1226, 1199, 1175.
Nuclear Magnetic Resonance Spectrum (hexadeuterated dimethyl sulphoxide + D₂O, 270 MHz), δ ppm:
   1.49 - 2.25 (3H, multiplet);
   2.09 & 2.10 (together 3H, two singlets);
   2.50 - 2.74 (1H, multiplet);
   3.00 - 3.92 (7H, multiplet);
   3.71 & 3.73 (together 3H, two singlets);
   3.77 (2H, singlet);
   4.15 - 4.63 (2H, multiplet);
   5.00 - 5.31 (4H, multiplet);
   6.87 (1H, doublet, J = 8.79 Hz);
   6.88 (1H, doublet, J = 8.30 Hz);
   7.25 (1H, doublet, J = 8.30 Hz);
   7.27 (1H, doublet, J = 8.79 Hz);
   7.43 - 7.70 (4H, multiplet);
   8.13 - 8.29 (4H, multiplet).

### 23(iv) (2S,4S)-4-Mercapto-2-[(3S)-3-(N-4-nitrobenzyloxycarbonylacetimidoylamino)pyrrolidin-1-ylcarbonyl]-1-(4-nitrobenzyloxycarbonyl)pyrrolidine

4.56 ml of trifluoroacetic acid and 208 µℓ of trifluoromethanesulphonic acid were added, whilst ice-cooling, to a solution of 0.87 g of (2S,4S)-4-(4-methoxybenzylthio)-2-[(3S)-3-(N-4-nitrobenzyloxycarbonylacetimidoylamino) pyrrolidin-1-ylcarbonyl]-1-(4-nitrobenzyloxycarbonyl)pyrrolidine [prepared as described in step (iii) above] in 1.29 ml of anisole, and the resulting mixture was stirred under the same conditions for 1.5 hours. At the end of this time, the solvent was removed by distillation under reduced pressure, and the resulting residue was washed with diethyl ether and dried in vacuo, to give 1.10 g of the trifluoromethanesulphonate of the title compound as a powder. The whole of this salt was dissolved in a mixture of ethyl acetate and water and the solution was made alkaline by adding a 1N aqueous solution of sodium hydroxide. The ethyl acetate layer was separated, washed with water and with an aqueous solution of sodium chloride, in that order, and dried over anhydrous magnesium sulphate. The solvent was removed by distillation under reduced pressure, to give 662 mg of the title compound, as a powder.
Infrared Absorption Spectrum (KBr), νₘₐₓ cm⁻¹:
   1708, 1650, 1607, 1553, 1520, 1440, 1404, 1346, 1217, 1170.
Nuclear Magnetic Resonance Spectrum (hexadeuterated dimethyl sulphoxide + D₂O, 270 MHz), δ ppm:
   1.59 - 2.28 (2H, multiplet);
   2.10 & 2.11 (together 3H, two singlets);
   2.60 - 2.83 (1H, multiplet);
   3.08 - 4.64 (10H, multiplet);
   5.01 - 5.42 (4H, multiplet);
   7.45 - 7.73 (4H, multiplet);
   8.14 - 8.31 (4H, multiplet).

### PREPARATION 24

### (2S,4S)-4-Mercapto-2-[(3S)-3-(N-4-nitrobenzyloxycarbonylformimidoylamino)pyrrolidin-1-ylcarbonyl]-1-(4-nitrobenzyloxycarbonyl)pyrrolidine

Following a procedure similar to that described in Preparations 23(iii) and 23(iv), but using 1.00 g of (2S,4S)-4-(4-methoxybenzylthio)-2-[(3S)-3-aminopyrrolidin-1-ylcarbonyl]-1-(4-nitrobenzyloxycarbonyl)-pyrrolidine hydrochloride [prepared as described in Preparation 23(ii)] and 410 mg of N-(4-nitrobenzyloxycarbonyl)formamidine, 670 mg of the title compound were obtained as a powder.
Infrared Absorption Spectrum (KBr), νₘₐₓ cm⁻¹:
   1707, 1645, 1604, 1520, 1441, 1404, 1346, 1188, 1111.
Nuclear Magnetic Resonance Spectrum (CDCℓ₃ + D₂O, 270 MHz), δ ppm:
   1.71 - 2.32 (3H, multiplet);
   2.60 - 2.84 (1H, multiplet);
   3.19 - 4.18 (8H, multiplet);
   4.36 - 4.57 (1H, multiplet);
   4.93 - 5.40 (4H, multiplet);
   7.40 - 7.61 (4H, multiplet);
   8.12 - 8.30 (4H, multiplet);
   8.42 (1H, singlet).

### PREPARATION 25

### (2S,4S)-4-Mercapto-2-[(3S)-1-(N-4-nitrobenzyloxycarbonylformimidoyl)pyrrolidin-3-ylaminocarbonyl]-1-(4-nitrobenzyloxycarbonyl)pyrrolidine

Following a procedure similar to that described in Preparations 23(iii) and 23(iv), but using 1.20 g of (2S,4S)-4-(4-methoxybenzylthio)-1-(4-nitrobenzyloxycarbonyl)-2-[(3S)-pyrrolidin-3-ylaminocarbonyl]-pyrrolidine hydrochloride and 490 mg of N-(4-nitrobenzyloxycarbonylformamidine, 750 mg of the title compound were obtained as a powder.
Infrared Absorption Spectrum (KBr), νₘₐₓ cm⁻¹:
   1721, 1678, 1664, 1602, 1520, 1449, 1439, 1403, 1346, 1234, 1222.
Nuclear Magnetic Resonance Spectrum (CDCℓ₃ + D₂O, 270 MHz), δ ppm:
   1.87 - 2.83 (4H, multiplet);
   3.24 - 4.08 (7H, multiplet);
   4.20 - 4.63 (2H, multiplet);
   5.14 - 5.38 (4H, multiplet);
   7.45 - 7.60 (4H, multiplet);
   8.15 - 8.29 (4H, multiplet);
   8.60 (1H, singlet).

### PREPARATION 26

### (2S,4S)-4-Mercapto-2-[(3S)-1-(N-4-nitrobenzyloxycarbonylacetimidoyl)pyrrolidin-3-ylaminocarbonyl]-1-(4-nitrobenzyloxycarbonyl)pyrrolidine

Following a procedure similar to that described in Preparations 23(iii) and 23(iv), but using 1.08 g of (2S,4S)-4-(4-methoxybenzylthio)-1-(4-nitrobenzyloxycarbonyl)-2-[(3S)-pyrrolidin-3-ylaminocarbonyl)-pyrrolidine hydrochloride and 440 mg of N-(4-nitrobenzyloxycarbonyl)acetamidine, 608 mg of the title compound were obtained as a powder.
Infrared Absorption Spectrum (KBr), νₘₐₓ cm⁻¹:
   1701, 1655, 1609, 1555.

### PREPARATION 27

### (2S,4S)-4-Mercapto-2-[(3R)-1-(N-4-nitrobenzyloxy, carbonylformimidoyl)pyrrolidin-3-ylaminocarbonyl]-1-(4-nitrobenzyloxycarbonyl)pyrrolidine

Following a procedure similar to that described in Preparations 23(iii) and 23(iv), but using 1.38 g of (2S,4S)-4-(4-methoxybenzylthio)-1-(4-nitrobenzyloxycarbonyl)-2-[(3R)-pyrrolidin-3-ylaminocarbonyl]-pyrrolidine hydrochloride and 564 mg of N-(4-nitrobenzyloxycarbonyl)formamidine, 795 mg of the title compound were obtained as a powder.
Infrared Absorption Spectrum (KBr), νₘₐₓ cm⁻¹:
   1710, 1650, 1587, 1518, 1442, 1346, 1170.

### PREPARATION 28

### (2S,4S)-4-Mercapto-2-{N-methyl-N-[(3S)-1-(N-4-nitrobenzyloxycarbonyl)pyrrolidin-3-yl]carbamoyl}-1-(4-nitrobenzyloxycarbonyl)pyrrolidine

Following a procedure similar to that described in Preparation 19, but using 8.0 g of (2S,4S)-4-(4-methoxybenzylthio)-1-(4-nitrobenzyloxycarbonyl)-2-pyrrolidinecarboxylic acid, 3.05 g of N,N'-carbonyldiimidazole and 3.37 g of (3S)-3-methylamino-1-t-butoxycarbonylpyrrolidine, 504 mg of the title compound were obtained as a powder.
Infrared Absorption Spectrum (KBr), νₘₐₓ cm⁻¹:
   1704, 1522, 1346, 854, 736.

### PREPARATION 29

### (2S,4S)-4-Mercapto-2-{N-methyl-N-[(3S)-1-(N-4-nitrobenzyloxycarbonylformimidoyl)pyrrolidin-3-yl]-carbamoyl}-1-(4-nitrobenzyloxycarbonyl)pyrrolidine

Following a procedure similar to that described in Preparations 23(iii) and 23(iv), but using 1.00 g of (2S,4S)-4-(4-methoxybenzylthio)-1-(4-nitrobenzyloxycarbonyl)-2-[N-methyl-N-[(3S)-pyrrolidin-3-yl]carbamoyl]-pyrrolidine hydrochloride and 405 mg of N-(4-nitrobenzyloxycarbonyl)formamidine, 620 mg of the title compound were obtained as a powder.
Infrared Absorption Spectrum (KBr), νₘₐₓ cm⁻¹:
   1707, 1648, 1514, 1404, 1347, 1173.

### PREPARATION 30

### (2S,4S)-2-(3-Carbamoyl-4-(4-nitrobenzyloxycarbonyl)-1-piperazinylcarbonyl]-4-mercapto-1-(4-nitrobenzyloxycarbonyl)pyrrolidine

Following a procedure similar to that described in Preparation 8, but using 2.18 g of (2S,4S)-4-(4-methoxybenzylthio)-1-(4-nitrobenzyloxycarbonyl)-2-pyrrolidinecarboxylic acid, 0.79 g of N,N'-carbonyldiimidazole and 1.81 g of 2-carbamoyl-1-(4-nitrobenzyloxycarbonyl)-piperazine, 2.13 g of the title compound were obtained as an amorphous solid.
Infrared Absorption Spectrum (KBr), νₘₐₓ cm⁻¹:
   1706, 1607, 1521, 1432, 1405, 1346.
Nuclear Magnetic Resonance Spectrum (CDCℓ₃, 270 MHz), δ ppm:
   1.71 - 2.04 (3H, multiplet);
   2.42 - 3.46 (5H, multiplet);
   3.90 - 4.93 (6H, multiplet);
   5.02 - 5.36 (4H, multiplet);
   5.45 - 6.77 (2H, multiplet);
   7.42 - 7.54 (4H, multiplet);
   8.14 - 8.26 (4H, multiplet).

### PREPARATION 31

### (2S,4S)-4-Mercapto-2-[4-(2-fluoroethyl)-1-homopiperazinylcarbonyl]-1-(4-nitrobenzyloxycarbonyl)pyrrolidine trifluoromethanesulfonate

Following a procedure similar to that described in Preparation 8, but using 3.5 g of (2S,4S)-4-(4-methoxybenzylthio)-1-(4-nitrobenzyloxycarbonyl)-2-pyrrolidinecarboxylic acid, 1.53 g of N,N'-carbonyldiimidazole and 5.83 g of N-(2-fluoroethyl)homopiperazine bis(trifluoroacetate), 4.0 g of the title compound were obtained as a powder.
Infrared Absorption Spectrum (KBr), νₘₐₓ cm⁻¹:
   1784, 1698, 1662, 1524, 1441, 1348, 1286, 1225, 1170, 1030.
Nuclear Magnetic Resonance Spectrum (CDCℓ₃, 270 MHz), δ ppm:
   1.62 - 1.83 (1H, multiplet);
   1.96 - 2.25 (2H, multiplet);
   2.65 - 2.90 (1H, multiplet);
   2.95 - 4.20 (14H, multiplet);
   4.60 - 4.95 (2H, multiplet);
   5.00 - 5.30 (2H, multiplet);
   7.50 - 7.70 (2H, multiplet);
   8.19 - 8.26 (2H, multiplet).

### PREPARATION 32

### (2S,4S)-4-Mercapto-2-[(3S)-3-(imidazol-1-yl)-pyrrolidin-1-ylcarbonyl]-1-(4-nitrobenzyloxycarbonyl)pyrrolidine

### 32(i) (2S,4S)-4-(4-Methoxybenzylthio)-2-[(3S)-3-(imidazol-1-yl)pyrrolidin-1-ylcarbonyl]-1-(4-nitrobenzyloxycarbonyl)pyrrolidine

1.09 g of N,N'-carbonyldiimidazole were added to a solution of 2.5 g of (2S,4S)-4-(4-methoxybenzylthio)-1-(4-nitrobenzyloxycarbonyl)-2-pyrrolidinecarboxylic acid in 25 ml of dry acetonitrile, and the resulting mixture stirred at room temperature for 30 minutes. A solution of 850 mg of (3S)-3-(imidazol-1-yl)pyrrolidine in 5 ml of dry acetonitrile was then added, and the mixture was stirred at room temperature for 2 hours and then at 40°C for a further 4 hours. At the end of this time, the reaction mixture was concentrated by evaporation under reduced pressure, and the resulting residue was purified by reverse phase column chromatography through 200 ml of Cosmo Sil 75C₁₈-PREP (a trade mark for a product of Nacalai Tesque), using a gradient elution method, with mixtures of acetonitrile and water ranging from 50 : 50 to 55 : 45 by volume as the eluent. Those fractions containing the title compound were combined and concentrated by evaporation under reduced pressure, to give 2.54 g of the title compound, as a powder.
Infrared Absorption Spectrum (KBr), νₘₐₓ cm⁻¹:
   1708, 1656, 1609, 1512, 1438, 1404, 1345, 1246, 1173, 1110.
Nuclear Magnetic Resonance Spectrum (CDCℓ₃, 270 MHz), δ ppm:
   1.88 - 2.05 (1H, multiplet);
   2.15 - 2.31 (1H, multiplet);
   2.36 - 2.57 (2H, multiplet);
   3.02 - 3.18 (1H, multiplet);
   3.31 - 3.40 (1H, multiplet);
   3.49 - 3.63 (1H, multiplet);
   3.73 & 3.74 (together 2H, two singlets);
   3.78 & 3.79 (together 3H, two singlets);
   3.80 - 4.08 (3H, multiplet);
   4.26 - 4.48 (2H, multiplet);
   4.71 - 4.89 (1H, multiplet);
   5.00 - 5.34 (2H, multiplet);
   6.76 - 7.60 (9H, multiplet);
   8.15 - 8.27 (2H, multiplet).

### 32(ii) (2S,4S)-4-Mercapto-2-[(3S)-3-(imidazol-1-yl)-pyrrolidin-1-ylcarbonyl]-1-(4-nitrobenzyloxycarbonyl)pyrrolidine

585 µℓ of trifluoromethanesulphonic acid were added, whilst ice-cooling, to a solution of 2.5 g of (2S,4S)-4-(4-methoxybenzylthio)-2-[(3S)-3-(imidazol-1-yl)pyrrolidin-1-ylcarbonyl]-1-(4-nitrobenzyloxycarbonyl)-pyrrolidine [prepared as described in step (i) above] in a mixture of 5 ml of anisole and 15 ml of trifluoroacetic acid, and the resulting mixture was stirred at room temperature for 1 hour and then at 35°C for a further 30 minutes. At the end of this time, the mixture was concentrated by evaporation under reduced pressure, and the resulting residue was washed four times with diethyl ether, to give a colourless powder. This powder was suspended in ethyl acetate, and the suspension was made alkaline by the addition of an aqueous solution of sodium hydrogencarbonate. The ethyl acetate layer was separated and washed with an aqueous solution of sodium chloride, after which it was dried over anhydrous sodium sulphate. The solvent was then removed by distillation under reduced pressure, to give 1.9 g of the title compound, as a colourless powder.
Infrared Absorption Spectrum (KBr), νₘₐₓ cm⁻¹:
   1706, 1655, 1521, 1440, 1405, 1346.
Nuclear Magnetic Resonance Spectrum (CDCℓ₃ + D₂O, 270 MHz), δ ppm:
   1.90 - 2.09 (1H, multiplet);
   2.15 - 2.37 (1H, multiplet);
   2.42 - 2.83 (2H, multiplet);
   3.20 - 3.35 (1H, multiplet);
   3.41 - 4.93 (8H, multiplet);
   5.02 - 5.37 (2H, multiplet);
   6.79 - 8.26 (7H, multiplet).

### PREPARATION 33

### (2S,4S)-4-Mercapto-2-[(3S)-3-(1,2,4-triazol-1-yl)-pyrrolidin-1-yl-1-(4-nitrobenzyloxycarbonyl)pyrrolidine

### 33(i) (2S,4S)-4-(4-Methoxybenzylthio)-2-[(3S)-3-(1,2,4-triazol-1-yl)pyrrolidin-1-ylcarbonyl]-1-(4-nitrobenzyloxycarbonyl)pyrrolidine

Following a procedure similar to that described in Preparation 18(a), but using 768 mg of (2S,4S)-4-(4-methoxybenzylthio)-1-(4-nitrobenzyloxycarbonyl)-2-pyrrolidinecarboxylic aicd, 218 mg of pivaloyl chloride and 238 mg of (3S)-3-(1,2,4-triazol-1-yl)pyrrolidine trifluoroacetate, 803 mg of the title compound were obtained as a powder.
Infrared Absorption Spectrum (KBr), νₘₐₓ cm⁻¹:
   1709, 1656, 1521, 1346, 857, 738.
Nuclear Magnetic Resonance Spectrum (hexadeuterated dimethyl sulphoxide, 270 MHz), δ ppm:
   1.40 - 1.70 (1H, multiplet);
   2.18 - 2.75 (2H, multiplet);
   3.00 - 3.15 (1H, multiplet);
   3.15 - 4.10 (13H, multiplet);
   5.00 - 5.24 (3H, multiplet);
   6.85 - 6.90 (2H, multiplet);
   7.24 - 7.29 (2H, multiplet);
   7.45 - 7.61 (2H, multiplet);
   8.14 - 8.25 (2H, multiplet);
   8.50 - 8.62 (1H, multiplet).

### 33(ii) (2S,4S)-4-Mercapto-2-[(3S)-3-(1,2,4-triazol-1-yl)pyrrolidin-1-yl-1-(4-nitrobenzyloxycarbonyl)pyrrolidine

Following a procedure similar to that described in Preparation 18(b), but using the whole of the (2S,4S)-4-(4-methoxybenzylthio)-2-[(3S)-3-(1,2,4-triazol-1-yl)-pyrrolidin-1-ylcarbonyl] -1-(4-nitrobenzyloxycarbonyl)-pyrrolidine [prepared as described in step (i) above], 803 mg of the title compound were obtained as a powder.
Infrared Absorption Spectrum (KBr), νₘₐₓ cm⁻¹:
   1706, 1652, 1522, 1346, 857, 739.
Nuclear Magnetic Resonance Spectrum (hexadeuterated dimethyl sulphoxide, 270 MHz), δ ppm:
   1.56 - 1.78 (1H, multiplet);
   2.20 - 2.55 (2H, multiplet);
   2.61 - 2.82 (1H, multiplet);
   3.09 - 4.09 (9H, multiplet);
   4.41 - 4.64 (1H, multiplet);
   5.01 - 5.26 (3H, multiplet);
   7.47 - 7.65 (2H, multiplet);
   8.14 - 8.26 (2H, multiplet);
   8.51 - 8.62 (1H, multiplet).

### PREPARATION 34

### (2S,4S)-4-Mercapto-2-[(3R)-3-(4-nitrobenzyloxycarbonyl)aminopyrrolidin-1-ylcarbonyl]-1-(4-nitrobenzyloxycarbonyl)pyrrolidine

Following a procedure similar to that described in Preparation 18, but using 1.29 g of (2S,4S)-4-(4-methoxybenzylthio)-1-(4-nitrobenzyloxycarbonyl)-2-pyrrolidinecarboxylic acid, 365 mg of pivaloyl chloride and 1.14 g of (3R)-3-(4-nitrobenzyloxycarbonyl)aminopyrrolidine trifluoroacetate, 1.09 g of the title compound were obtained as a powder.
Infrared Absorption Spectrum (KBr), νₘₐₓ cm⁻¹:
   1707, 1653, 1523, 1347, 855.

### PREPARATION 35

### (2S,4S)-4-Mercapto-2-[(3R)-3-(N-4-nitrobenzyloxycarbonylacetimidoylamino)pyrrolidin-1-ylcarbonyl]-1-(4-nitrobenzyloxycarbonyl)pyrrolidine

Following a procedure similar to that described in Preparation 23, but using 3.00 g of (2S,4S)-4-(4-methoxybenzylthio)-1-(4-nitrobenzyloxycarbonyl)-2-pyrrolidinecarboxylic acid, 0.70 g of (3R)-3-aminopyrrolidine, 1.31 g of the title compound were obtained as a powder.
Infrared Absorption Spectrum (KBr), νₘₐₓ cm⁻¹:
   1706, 1651, 1552, 1441, 1345, 1171.
Nuclear Magnetic Resonance Spectrum (hexadeuterated dimethyl sulphoxide + D₂O, 270 MHz), δ ppm:
   1.56 - 2.25 (2H, multiplet);
   2.09 & 2.11 (together 3H, two singlets);
   2.62 - 2.83 (1H, multiplet);
   3.04 - 4.09 (8H, multiplet);
   4.14 - 4.62 (2H, multiplet);
   4.98 - 5.37 (4H, multiplet);
   7.43 - 7.70 (4H, multiplet);
   8.15 - 8.30 (4H, multiplet).

### PREPARATION 36

### (2S,4S)-4-mercapto-2-[(3R)-3-(N-4-nitrobenzyloxycarbonylformimidoylamino)pyrrolidin-1-ylcarbonyl]-1-(4-nitrobenzyloxycarbonyl)pyrrolidine

Following a procedure similar to that described in Preparations 23(iii) and 23(iv), but using 0.75 g of (2S,4S)-4-(4-methoxybenzylthio)-2-[(3R)-3-aminopyrrolidin-1-ylcarbonyl]-1-(4-nitrobenzyloxycarbonyl)pyrrolidine hydrochloride and 0.31 g of N-(4-nitrobenzyloxycarbonyl)formamidine, 0.51 g of the title compound were obtained as a powder.
Infrared Absorption Spectrum (KBr), νₘₐₓ cm⁻¹:
   1706, 1644, 1521, 1405, 1345, 1186.

### PREPARATION 37

### (2S,4S)-4-Mercapto-2-[3-(4-nitrobenzyloxycarbonyloxymethyl)-4-(4-nitrobenzyloxycarbonyl)-1-piperazinylcarbonyl]-1-(4-nitrobenzyloxycarbonyl)pyrrolidine

### 37(i) (2S,4S)-2-(3-Hydroxymethyl-1-piperazinylcarbonyl)-4-(4-methoxybenzylthio)-1-(4-nitrobenzyloxycarbonyl)pyrrolidine

Following a procedure similar to that described in Preparation 8, but using 10.0 g of (2S,4S)-4-(4-methoxybenzylthio)-1-(4-nitrobenzyloxycarbonyl)-2-pyrrolidinecarboxylic acid, 4.4 g of N,N'-carbonyldiimidazole and 4.0 g of 2-hydroxymethylpiperazine, 6.9 g of the title compound were obtained, as a powder.

### 37(ii) (2S,4S)-4-(4-Methoxybenzylthio)-2-[3-(4-nitrobenzyloxycarbonyloxymethyl)-4-(nitrobenzyloxycarbonyl)-1-piperazinylcarbonyl]-1-(4-nitrobenzyloxycarbonyl)-pyrrolidine

Following a procedure similar to that described in Preparation 7(iii), but using 3.5 g of (2S,4S)-2-(3-hydroxymethyl-1-piperazinylcarbonyl)-4-(4-methoxybenzylthio)-1-(4-nitrobenzyloxycarbonyl) pyrrolidine [prepared as described in step (i) above], 4.2 g of 4-nitrobenzyl chloroformate and 2.4 g of 4-dimethylaminopyridine, 4.9 g of the title compound were obtained, as an amorphous solid.

### 37(iii) (2S,4S)-4-Mercapto-2-[3-(4-nitrobenzyloxycarbonyloxymethyl)-4-(4-nitrobenzyloxycarbonyl)-1-piperazinylcarbonyl]-1-(4-nitrobenzyloxycarbonyl) -pyrrolidine

Following a procedure similar to that described in Preparation 4(b), but using 0.23 g of (2S,4S)-4-(4-methoxybenzylthio)-2-[3-(4-nitrobenzyloxycarbonyloxymethyl)-4-(nitrobenzyloxycarbonyl) -1-piperazinylcarbonyl]-1-(4-nitrobenzyloxycarbonyl)pyrrolidine [prepared as described in step (ii) above], 0.28 ml of anisole, 2.3 ml of trifluoroacetic acid and 45 µℓ of trifluoromethanesulphonic acid, 190 mg of the title compound were obtained, as an amorphous solid.
Infrared Absorption Spectrum (KBr), νₘₐₓ cm⁻¹:
   1707, 1608, 1521, 1430, 1406, 1345.
Nuclear Magnetic Resonance Spectrum (CDCℓ₃, 270 MHz), δ ppm:
   1.68 (1H, singlet);
   1.86 - 1.99 (1H, multiplet);
   2.62 - 2.69 (1H, multiplet);
   2.93 (1H, doublet of doublets, J = 13.67 & 3.91 Hz);
   3.10 - 3.63 (4H, multiplet);
   3.91 - 4.75 (8H, multiplet);
   5.11 - 5.30 (6H, multiplet);
   7.47 (6H, doublet, J = 8.30 Hz);
   8.14 - 8.26 (6H, multiplet).

### PREPARATION 38

### (2S,4S)-4-Mercapto-2-[3-(4-nitrobenzyloxycarbonyl)-4-(4-nitrobenzyloxycarbonyl)-1-piperazinylcarbonyl]-1-(4-nitrobenzyloxycarbonyl)pyrrolidine

Following a procedure similar to that described in Preparation 8, but using 0.47 g of (2S,4S)-4-(4-methoxybenzylthio)-1-(4-nitrobenzyloxycarbonyl)-2-pyrrolidinecarboxylic acid, 0.20 g of N,N'-carbonyldiimidazole and 0.70 g of 2-(4-nitrobenzyloxycarbonyl)-1-(4-nitrobenzyloxycarbonyl)piperazine, 270 mg of the title compound were obtained as an amorphous solid.
Infrared Absorption Spectrum (KBr), νₘₐₓ cm⁻¹:
   1707, 1653, 1607, 1522, 1430, 1346.

### PREPARATION 39

### (2S,4S)-4-Mercapto-2-[(3R)-1-(N-4-nitrobenzyloxycarbonylacetimidoyl)pyrrolidin-3-ylaminocarbonyl]-1-(4-nitrobenzyloxycarbonyl)pyrrolidine

Following a procedure similar to that described in Preparation 23(iii) and 23(iv), but using 0.76 g of (2S,4S)-4-(4-methoxybenzylthio)-1-(4-nitrobenzyloxycarbonyl)-2-[(3R)-pyrrolidin-3-ylaminocarbonyl]pyrrolidine hydrochloride and 0.33 g of N-(4-nitrobenzyloxycarbonyl)acetamidine, 0.45 g of the title compound was obtained as a powder.
Infrared Absorption Spectrum (KBr), νₘₐₓ cm⁻¹:
   1720, 1665, 1520, 1437, 1345, 1234.

### PREPARATION 40

### (2S,4S)-4-Mercapto-2-{N-[2-(N-4-nitrobenzyloxycarbonylacetimidoyl)aminoethyl]carbamoyl}-1-(4-nitrobenzyloxycarbonyl)pyrrolidine

Following a procedure similar to that described in Preparation 17, but using 850 mg of (2S,4S)-4-(4methoxybenzylthio)-1-(4-nitrobenzyloxycarbonyl)-2-pyrrolidinecarboxylic acid, 450 mg of ethylenediamine and 430 mg of N-(4-nitrobenzyloxycarbonyl)acetamidine, 295 mg of the title compound were obtained as a powder.
Infrared Absorption Spectrum (KBr), νₘₐₓ cm⁻¹:
   1705, 1665, 1568, 1517, 1346, 1225.
Nuclear Magnetic Resonance Spectrum (CDCℓ₃ + D₂O, 270 MHz), δ ppm:
   2.23 (3H, singlet);
   2.1 - 2.4 (1H, broad);
   2.5 - 2.8 (1H, broad);
   3.3 - 3.65 (6H, multiplet);
   3.95 - 4.45 (2H, multiplet);
   5.15 - 5.30 (4H, multiplet);
   7.45 - 7.60 (4H, multiplet);
   8.17 - 8.27 (4H, multiplet).

### PREPARATION 41

### (2S,4S)-4-Mercapto-2-{N-[2-(N-4-nitrobenzyloxycarbonylformimidoyl)aminoethyl]carbamoyl}-1-(4-nitrobenzyloxycarbonyl)pyrrolidine

Following a procedure similar to that described in Preparation 17, but using 850 mg of (2S,4S)-4-(4-methoxybenzylthio)-1-(4-nitrobenzyloxycarbonyl)-2-pyrrolidinecarboxylic acid, 450 mg of ethylenediamine and 400 mg of N-(4-nitrobenzyloxycarbonyl)formamidine, 280 mg of the title compound were obtained as a powder.
Infrared Absorption Spectrum (KBr), νₘₐₓ cm⁻¹:
   1705, 1665, 1568, 1517, 1346, 1225.
Nuclear Magnetic Resonance Spectrum (CDCℓ₃, 270 MHz), δ ppm:
   2.11 - 2.30 (1H, multiplet);
   2.55 - 2.80 (1H, multiplet);
   3.30 - 3.48 (3H, multiplet);
   3.52 - 3.75 (3H, multiplet);
   3.93 - 4.06 (1H, multiplet);
   4.18 - 4.32 (1H, multiplet);
   5.13 - 5.36 (4H, multiplet);
   7.43 - 7.60 (4H, multiplet);
   8.15 - 8.27 (4H, multiplet);
   8.47 & 8.97 (1H, two singlets).

### PREPARATION 42

### (2S,4S)-4-Mercapto-2-(3-dimethylamino-1,2,5,6-tetrahydropyrazin-1-ylcarbonyl)-1-(4-nitrobenzyloxycarbonyl)pyrrolidine trifluoromethanesulfonate

### 42(i) (2S,4S)-4-(4-Methoxybenzylthio)-2-(3-dimethylamino-1,2,5,6-tetrahydropyrazin-1-ylcarbonyl)-1-(4-nitrobenzyloxycarbonyl)pyrrolidine

Following a procedure similar to that described in Preparation 1(i), but using 446 mg of (2S,4S)-4-(4-methoxybenzylthio)-1-(4-nitrobenzyloxycarbonyl)-2-pyrrolidinecarboxylic acid, 178 mg of N,N'-carbonyldiimidazole, 289 mg of 3-dimethylamino-1,2,5,6-tetrahydropyrazine bis(trifluoroacetate) and 289 µℓ of diisopropylethylamine, 460 mg of the title compound were obtained, as a powder.

### 42(ii) (2S,4S)-4-Mercapto-2-(3-dimethylamino-1,2,5,6-tetrahydropyrazin-1-ylcarbonyl)-1-(4-nitrobenzyloxycarbonyl)pyrrolidine trifluoromethanesulfonate

Following a procedure similar to that described in Preparation 1(iv), but using the whole of the (2S,4S)-4-(4-methoxybenzylthio)-2-(3-dimethylamino-1,2,5,6-tetrahydropyrazin-1-ylcarbonyl) -1-(4-nitrobenzyloxycarbonyl)-pyrrolidine [prepared as described in step (i) above], 451 mg of the title compound were obtained as a viscous oil.
Infrared Absorption Spectrum (liquid film), νₘₐₓ
   cm⁻¹: 1705, 1670, 1610, 1525, 1445, 1409, 1348, 1287, 1228.
Nuclear Magnetic Resonance Spectrum (hexadeuterated dimethyl sulphoxide + D₂O, 270 MHz), δ ppm:
   1.58 - 1.78 (1H, multiplet);
   2.71 - 2.86 (1H, multiplet);
   2.88 - 3.86 (12H, multiplet);
   3.90 - 4.10 (1H, multiplet);
   4.48 - 4.68 (2H, multiplet);
   4.75 - 4.92 (1H, multiplet);
   5.02 - 5.23 (2H, multiplet);
   7.52 & 7.63 (together 2H, two doublets, J = 8.79 Hz);
   8.21 & 8.23 (together 2H, two doublets, J = 8.79 Hz).

### PREPARATION 43

### (2S,4S)-4-Mercapto-2-[4-(N-4-nitrobenzyloxycarbonylacetimidoyl)aminopiperidin-1-ylcarbonyl)-1-(4-nitrobenzyloxycarbonyl)pyrrolidine

Following a procedure similar to that described in Preparation 1, but using 446 mg of (2S,4S)-4-(4-methoxybenzylthio)-1-(4-nitrobenzyloxycarbonyl)-2-pyrrolidinecarboxylic acid, 220 mg of 4-t-butoxycarbonylaminopiperidine and 119 mg of N-(4-nitrobenzyloxycarbonyl)acetamidine, 307 mg of the title compound were obtained as a powder.
Infrared Absorption Spectrum (KBr), νₘₐₓ cm⁻¹:
   1781, 1703, 1633, 1610, 1345.
Nuclear Magnetic Resonance Spectrum (CDCℓ₃, 270 MHz), δ ppm:
   1.40 - 2.15 (7H, multiplet);
   2.21 & 2.32 (together 3H, two singlets);
   2.65 - 2.80 (1H, multiplet);
   3.10 - 3.50 (3H, multiplet);
   3.65 - 4.75 (6H, multiplet);
   5.23 (4H, singlet);
   7.48 - 7.60 (4H, multiplet);
   8.18 - 8.26 (4H, multiplet).

### PREPARATION 44

### (2S,4S)-4-Mercapto-2-[4-(N-4-nitrobenzyloxycarbonylacetimidoyl)piperazin-1-ylcarbonyl)-1-methylpyrrolidine

Following a procedure similar to that described in Preparation 1, but using 282 mg of (2S,4S)-4-(4-methoxy benzylthio)-1-methyl-2-pyrrolidinecarboxylic acid, 203 mg of 1-t-butoxycarbonylpiperazine and 258 mg of N-(4-nitrobenzyloxycarbonyl)acetamidine, 225 mg of the title compound were obtained as a viscous oil.

The title compound was also prepared by acylating 1-(N-4-nitrobenzyloxycarbonylacetimidoyl)piperazine with 4-(4-methoxybenzylthio)-1-methylpyrrolidine-2-carboxylic acid and then deprotecting the product.
Infrared Absorption Spectrum (liquid film), νₘₐₓ
   cm⁻¹: 1780, 1705, 1635, 1610, 1346:
Nuclear Magnetic Resonance Spectrum (hexadeuterated dimethyl sulphoxide + D₂O, 270 MHz), δ ppm:
   1.77 - 1.90 (1H, multiplet);
   2.14 (3H, singlet);
   2.82 (3H, singlet);
   2.95 - 3.05 (1H, multiplet);
   3.10 - 3.28 (3H, multiplet);
   3.45 - 3.80 (8H, multiplet);
   4.60 (1H, doublet of doublets, J = 9.3 & 8.3 Hz);
   5.28 (2H, singlet);
   7.66 (2H, doublet, J = 8.8 Hz);
   8.25 (2H, doublet, J = 8.8 Hz).

### PREPARATION 45

### (2S,4S)-4-Mercapto-2-[(3S)-3-(4-nitrobenzyloxycarbonyl)aminopyrrolidin-1-ylcarbonyl]-1-methylpyrrolidine

Following a procedure similar to that described in Preparation 18, but using 1.03 g of (2S,4S)-4-(4-methoxybenzylthio)-1-methyl-2-pyrrolidinecarboxylic acid, 463 mg of pivaloyl chloride and 1.45 g of (3S)-3-(4-nitrobenzyloxycarbonyl)aminopyrrolidine trifluoroacetate, 1.07 g of the title compound were obtained as a powder.
Infrared Absorption Spectrum (KBr), νₘₐₓ cm⁻¹:
   1703, 1655, 1522, 1347, 854, 737.
Nuclear Magnetic Resonance Spectrum (hexadeuterated dimethyl sulphoxide, 270 MHz), δ ppm:
   1.65 - 3.85 (15H, multiplet);
   3.85 - 4.20 (2H, multiplet);
   5.19 (2H, singlet);
   7.62 (2H, doublet, J = 8.30 Hz);
   7.70 - 7.90 (1H, multiplet);
   8.20 - 8.30 (2H; multiplet).

### PREPARATION 46

### (2S,4S)-4-Mercapto-2-[(3S)-3-(N-4-nitrobenzyloxycarbonylformimidoylamino)pyrrolidin-1-ylcarbonyl]-1-methylpyrrolidine

### 46(i) (2S,4S)-4-(4-Methoxybenzylthio)-2-[(3S)-3-aminopyrrolidin-1-ylcarbonyl]-1-(4-nitrobenzyloxycarbonyl)-1-methylpyrrolidine hydrochloride

Following a procedure similar to that described in Preparations 23(i) and 23(ii), but using 2.55 g of (2S,4S)-4-(4-methoxybenzylthio)-1-methyl-2-pyrrolidinecarboxylic acid and 0.94 g of (3S)-3-aminopyrrolidine, 2.91 g of the title compound were obtained as a powder.

### 46(ii) (2S,4S)-4-Mercapto-2-[(3S)-3-(N-4-nitrobenzyloxycarbonylformimidoylamino)pyrrolidin-1-ylcarbonyl]-1-methylpyrrolidine

Following a procedure similar to that described in Preparations 23(iii) and 23(iv), but using N-(4-nitrobenzyloxycarbonyl)formamidine instead of the N-(4-nitrobenzyloxycarbonyl)acetamidine used in Preparation 23(iii), 1.02 g of the title compound was obtained as a powder.
Infrared Absorption Spectrum (liquid film), νₘₐₓ cm⁻¹:
   1705, 1650, 1510, 1440, 1345, 1173.
Nuclear Magnetic Resonance Spectrum (hexadeuterated dimethyl sulphoxide, 270 MHz), δ ppm:
   1.60 - 4.60 (19H, multiplet);
   5.10 - 5.30 (2H, multiplet);
   7.55 - 7.75 (2H, multiplet);
   8.25 - 8.28 (2H, multiplet).

### PREPARATION 47

### (2S,4S)-4-Mercapto-2-[4-(imidazol-1-yl)piperidin-1-ylcarbonyl]-1-(4-nitrobenzyloxycarbonyl)pyrrolidine

### 47(i) (2S,4S)-4-(4-Methoxybenzylthio)-2-[4-(imidazol-1-yl)piperidin-1-ylcarbonyl]-1-(4-nitrobenzyloxycarbonyl)pyrrolidine

660 mg of N,N'-carbonyldiimidazole were added to a solution of 1.52 g of (2S,4S)-4-(4-methoxybenzylthio)-1-(4-nitrobenzyloxycarbonyl)-2-pyrrolidinecarboxylic acid in 15 ml of dry acetonitrile, and the resulting mixture was stirred at room temperature for 30 minutes. A solution of 538 mg of 4-(imidazol-1-yl)piperidine in 5 ml of dry acetonitrile was then added to the solution, and the mixture was stirred at room temperature for 30 minutes and then at 40°C for a further 7 hours. At the end of this time, the reaction mixture was concentrated by evaporation under reduced pressure, and the residue was dissolved in ethyl acetate. The resulting solution was washed with an aqueous solution of sodium hydrogencarbonate, with water and with an aqueous solution of sodium chloride, in that order. The organic solution was then dried over anhydrous sodium sulphate, and the solvent was removed by distillation under reduced pressure. The residue was purified by reverse phase column chromatography through 200 ml of Cosmo Sil 75C₁₈-PREP (a trade mark for a product of Nacalai Tesque), using a gradient elution method, with mixtures of acetonitrile and water ranging from 50 : 50 to 55 : 45 by volume as the eluent. Those fractions containing the title compound were combined and concentrated by evaporation under reduced pressure, to give 1.45 g of the title compound, as a powder.
Infrared Absorption Spectrum (liquid film), νₘₐₓ cm⁻¹:
   1709, 1655, 1609, 1512, 1345, 1246, 1110.
Nuclear Magnetic Resonance Spectrum (CDCℓ₃, 270 MHz), δ ppm:
   1.70 - 1.95 (2H, multiplet);
   2.05 - 2.23 (3H, multiplet);
   2.40 - 2.55 (1H, multiplet);
   2.60 - 2.85 (1H, multiplet);
   3.03 - 3.43 (3H, multiplet);
   3.73 (3H, singlet);
   3.77 - 4.25 (5H, multiplet);
   4.59 - 4.84 (2H, multiplet);
   5.02 - 5.35 (2H, multiplet);
   6.85 (2H, doublet, J = 8.8 Hz);
   6.96 (1H, singlet);
   7.07 & 7.09 (together 1H, two singlets);
   7.23 (2H, doublet, J = 8.8 Hz);
   7.47 (2H, doublet, J = 8.8 Hz);
   7.56 (1H, singlet);
   8.23 (2H, doublet, J = 8.8 Hz).

### 47(ii) (2S,4S)-4-Mercapto-2-[4-(imidazol-1-yl)piperidin-1-ylcarbonyl]-1-(4-nitrobenzyloxycarbonyl)pyrrolidine

350 µℓ of trifluoromethanesulphonic acid were added, whilst ice-cooling, to a solution of 1.44 g of (2S,4S)-4-(4-methoxybenzylthio)-2-[4-(imidazol-1-yl)-piperidin-1-ylcarbonyl]-1-(4-nitrobenzyloxycarbonyl)-pyrrolidine [prepared as described in step (i) above] in a mixture of 1.5 ml of anisole and 7.5 ml of trifluoroacetic acid, and the resulting mixture was stirred at room temperature for 1 hour and then at 35°C for a further 30 minutes. At the end of this time, the reaction mixture was concentrated by evaporation under reduced pressure, and the residue was washed four times with diethyl ether to give a colourless powder. The whole of this powder was suspended in ethyl acetate and the suspension was made alkaline by the addition of an aqueous solution of sodium hydrogencarbonate. The ethyl acetate layer was separated, washed with an aqueous solution of sodium chloride and dried over anhydrous sodium sulphate. The solvent was removed by distillation under reduced pressure, to give 1.15 g of the title compound, as a colourless powder.
Nuclear Magnetic Resonance Spectrum (hexadeuterated dimethyl sulphoxide, 270 MHz), δ ppm:
   1.55 - 1.85 (3H, multiplet);
   2.00 - 2.11 (2H, multiplet);
   2.63 - 2.89 (2H, multiplet);
   3.05 - 3.30 (4H, multiplet);
   3.92 - 4.15 (2H, multiplet);
   4.25 - 4.59 (2H, multiplet);
   4.71 - 4.92 (1H, multiplet);
   5.03 - 5.27 (2H, multiplet);
   6.92 - 8.28 (7H, multiplet).
Infrared Absorption Spectrum (liquid film), νₘₐₓ cm⁻¹:
   1705, 1652, 1523, 1442, 1347, 1268, 1170, 1035.

### PREPARATION 48

### (2S,4S)-2-[3,3-Dimethyl-4-(4-nitrobenzyloxycarbonyl)-1-piperazinylcarbonyl]-4-mercapto-1-(4-nitrobenzyloxycarbonyl)pyrrolidine

Following a procedure similar to that described in Preparation 38, but using 0.35 g of (2S,4S)-4-(4-methoxybenzylthio)-1-(4-nitrobenzyloxycarbonyl)-2-pyrrolidinecarboxylic acid, 0.15 g of N,N'-carbonyldiimidazole and 0.13 g of 2,2-dimethylpiperazine, 250 mg of the title compound were obtained as an amorphous solid.
Infrared Absorption Spectrum (KBr), νₘₐₓ cm⁻¹:
   1707, 1652, 1607, 1521, 1431, 1347.

### PREPARATION 49

### (2S,4S)-4-Mercapto-1-methyl-2-[4-(4-nitrobenzyloxycarbonyl)-1-homopiperazinylcarbonyl]pyrrolidine trifluoromethanesulfonate

### 49(i) (2S,4S)-2-Carbamoyl-4-(4-methoxybenzylthio)-1-methylpyrrolidine

10.86 ml of dimethyl sulphate were added to a solution of 30 g of (2S,4S)-2-carbamoyl-4-(4-methoxybenzylthio)pyrrolidine hydrochloride in a mixture of 36 ml of a 20% w/v aqueous solution of sodium hydroxide and 470 ml of dioxane, and the resulting mixture was stirred at a temperature of between 22°C and 23°C for 1 hour. At the end of this time, the reaction mixture was concentrated by evaporation under reduced pressure, and the resulting residue was extracted with 2 liters of ethyl acetate. The extract was washed with an aqueous solution of sodium chloride and dried over anhydrous magnesium sulphate. The solution was then concentrated by evaporation under reduced pressure, until crystals emerged, and then 400 ml of diisopropyl ether were added to the mixture and the crystals which precipitated were collected by filtration, to give 23 g of the title compound as crystals, melting at 113 - 114°C.
Infrared Absorption Spectrum (KBr), νₘₐₓ cm⁻¹:
   1636, 1609, 1512.
Nuclear Magnetic Resonance Spectrum (60 MHz, CDCℓ₃) δ ppm:
   1.58 - 3.36 (6H, multiplet);
   2.35 (3H, singlet);
   3.68 (2H, singlet);
   3.78 (3H, singlet);
   5.95 (1H, broad singlet);
   6.84 & 7.23 (4H, A₂B₂, J = 9.0 Hz);
   7.20 (1H, broad singlet).

### 49(ii) (2S,4S)-2-Carboxy-4-(4-methoxybenzylthio)-1-methylpyrrolidine

15.16 g of (2S,4S)-2-carbamoyl-4-(4-methoxybenzylthio)-1-methylpyrrolidine [prepared as described in step (i) above] were dissolved in 170 ml of 2N aqueous hydrochloric acid, and the solution was stirred in a bath kept at 110°C for 3.5 hours. At the end of this time, the reaction mixture was cooled to room temperature, and its pH was adjusted to a value of 8.5 by the addition of sodium carbonate. The mixture was then concentrated by evaporation under reduced pressure, after which it was allowed to stand in a refrigerator to precipitate crystals. The precipitated crystals were collected by filtration and washed with a small amount of cold water. They were then dried, to give 10.4 g of the title compound. The mother liquor was then concentrated by evaporation under reduced pressure, and the residue was subjected to column chromatography through CHP20P (75-150 µ, Mitsubishi Chemical Industries, Ltd.), using 50% v/v aqueous methanol as the eluent, to afford a further 3.7 g of the title compound, as crystals melting at 185-187.5°C.
Infrared Absorption Spectrum (KBr), νₘₐₓ cm⁻¹:
   1641, 1623, 1512, 1373, 1311, 1253.
Nuclear Magnetic Resonance Spectrum (270 MHz, D₂O) δ ppm:
   1.83 - 1.93 (1H, multiplet);
   2.59 - 2.75 (1H, multiplet);
   2.72 (3H, singlet);
   3.16 - 3.23 (1H, multiplet);
   3.03 - 3.43 (2H, multiplet);
   3.62 (2H, singlet);
   3.64 (3H, singlet);
   3.74 (1H, doublet of doublets, J = 9.53 & 6.96 Hz);
   6.80 (2H, doublet, J = 8.60 Hz);
   7.15 (2H, doublet, J = 8.60 Hz).

### 49(iii) (2S,4S)-4-(4-Methoxybenzylthio)-1-methyl-2-[4-(4-nitrobenzyloxycarbonyl)-1-homopiperazinylcarbonyl]pyrrolidine

A suspension of 1.8 g of (2S,4S)-2-carboxy-4-(4-methoxybenzylthio)-1-methylpyrrolidine [prepared as described in step (ii) above] and 1.26 g of N,N'-carbonyldiimidazole in 18 ml of dry acetonitrile was stirred at 35°C for 25 minutes. A solution of 3.7 g of 1-(4-nitrobenzyloxycarbonyl)homopiperazine trifluoroacetate in 20 ml of dry acetonitrile and 2.0 ml of N,N-diisopropylethylamine were then simultaneously added dropwise, whilst ice-cooling, to the reaction mixture, and the resulting mixture was stirred at room temperature for 8 hours, after which it was allowed to stand overnight at room temperature. At the end of this time, the reaction mixture was concentrated by evaporation under reduced pressure, and the residue was dissolved in ethyl acetate. The solution was washed with an aqueous solution of sodium hydrogencarbonate, with a phosphate buffer solution (pH 6.86), with water and with an aqueous solution of sodium chloride, in that order. The ethyl acetate layer was then dried over anhydrous magnesium sulphate, and the solvent was removed by distillation under reduced pressure. The resulting residue was purified by column chromatography through silica gel (Merck Art 9385), using a 95 : 5 by volume mixture of acetonitrile and water, to give 2.5 g of the title compound.
Infrared Absorption Spectrum (liquid film), νₘₐₓ cm⁻¹:
   1701, 1646, 1513, 1426, 1346, 1246.
Nuclear Magnetic Resonance Spectrum (CDCℓ₃, 270 MHz), δ ppm:
   1.66 - 1.95 (4H, multiplet);
   2.26 & 2.29 (together 3H, two singlets);
   2.42 - 2.63 (2H, multiplet);
   3.07 - 3.23 (3H, multiplet);
   3.44 - 3.82 (7H, multiplet);
   3.70 (2H, singlet);
   3.79 (3H, singlet);
   5.15 - 5.30 (2H, multiplet);
   6.83 (2H, doublet, J = 8.30 Hz);
   7.21 (2H, doublet, J = 8.30 Hz);
   7.49 & 7.50 (together 2H, two doublets, 8.30 Hz).

### 49(iv) (2S,4S)-4-Mercapto-1-methyl-2-[4-(4-nitrobenzyloxycarbonyl)-1-homopiperazinylcarbonyl]pyrrolidine trifluoromethanesulfonate

25 ml of trifluoroacetic acid and 0.83 ml of trifluoromethanesulphonic acid were added dropwise, whilst ice-cooling, to a solution of 2.5 g of (2S,4S)-4-(4-methoxybenzylthio)-1-methyl-2-[4-(4-nitrobenzyloxycarbonyl)-1-homopiperazinylcarbonyl] -pyrrolidine [prepared as described in step (iii) above] in 5.2 ml of anisole, and the resulting mixture was stirred at the same temperature for 50 minutes. At the end of this time, the reaction mixture was concentrated by evaporation under reduced pressure, and the residue was decanted, in turn, with hexane and with diethyl ether, to give 2.7 g of the title compound as an amorphous solid.
Infrared Absorption Spectrum (liquid film), νₘₐₓ cm⁻¹:
   1688, 1650, 1522, 1483, 1434, 1349:
Nuclear Magnetic Resonance Spectrum (270 MHz, hexadeuterated dimethyl sulphoxide) δ ppm:
   1.58 - 1.92 (2H, multiplet);
   2.67 - 3.08 (3H, multiplet);
   3.28 - 3.82 (13H, multiplet);
   4.20 - 4.85 (2H, multiplet);
   5.16 - 5.28 (2H, multiplet);
   7.53 - 7.67 (2H, multiplet);
   8.24 (2H, doublet, J = 8.31 Hz).

### PREPARATION 50

### (2S,4S)-4-(4-Mercapto)-2-[4-(4-nitrobenzyloxycarbonylmethyl)-1-homopiperazinylcarbonyl]-1-(4-nitrobenzyloxycarbonyl)pyrrolidine bis(trifluoromethanesulfonate)

### 50(i) (2S,4S)-4-(4-Methoxybenzylthio)-2-[4-(4-nitrobenzyloxycarbonylmethyl)-1-homopiperazinylcarbonyl]-1-(4-nitrobenzyloxycarbonyl) pyrrolidine

3.5 g of N,N'-carbonyldiimidazole were added to a solution of 8.0 g of (2S,4S)-4-(4-methoxybenzylthio)6-1-(4-nitrobenzyloxycarbonyl)-2-pyrrolidinecarboxylic acid in 80 ml of dry acetonitrile, and the resulting mixture was stirred at room temperature for 30 minutes. A solution of 14.0 g of 1-(4-nitrobenzyloxycarbonylmethyl)homopiperazine bis(trifluoroacetate) in 80 ml of dry acetonitrile and 14.1 ml of diisopropylethylamine were added, whilst ice-cooling, to the mixture, and the resulting mixture was stirred at room temperature for 1.5 hours and then at 30°C for a further 1.5 hours, after which it was allowed to stand overnight at room temperature. At the end of this time, the reaction mixture was concentrated by evaporation under reduced pressure, and the resulting residue was diluted with ethyl acetate. The dilute solution was washed with water and with an aqueous solution of sodium chloride, in that order, and it was then dried over anhydrous magnesium sulphate. The solvent was then removed by distillation under reduced pressure, and the resulting residue was purified by column chromatography through silica gel (Merck Art 9385), using ethyl acetate as the eluent, to give 7.6 g of the title compound as an amorphous solid.
Infrared Absorption Spectrum (liquid film), νₘₐₓ
   cm⁻¹: 1748, 1709, 1650, 1608, 1520, 1429, 1404, 1346.
Nuclear Magnetic Resonance Spectrum (CDCℓ₃, 270 MHz), δ ppm:
   1.71 - 2.03 (3H, multiplet);
   2.42 - 3.17 (6H, multiplet);
   3.32 - 4.08 (8H, multiplet);
   3.73 (2H, multiplet);
   3.80 & 3.82 (together 3H, two singlets);
   4.49 - 4.63 (1H, multiplet);
   5.02 - 5.35 (4H, multiplet);
   6.85 (2H, doublet, J = 8.30 Hz);
   7.23 (2H, doublet, J = 8.30 Hz);
   7.43 - 7.52 (4H, multiplet);
   8.15 - 8.25 (4H, multiplet).

### 50(ii) (2S,4S)-4-Mercapto-2-[4-(4-nitrobenzyloxycarbonylmethyl)-1-homopiperazinylcarbonyl]-1-(4-nitrobenzyloxycarbonyl)pyrrolidine bis(trifluoromethanesulfonate)

15 ml of trifluoroacetic acid and 0.36 ml of trifluoromethanesulphonic acid were added, whilst ice-cooling, to a solution of 1.47 g of (2S,4S)-4-(4-methoxybenzylthio)-2-[4-(4-nitrobenzyloxycarbonylmethyl)-1-homopiperazinylcarbonyl] -1-(4-nitrobenzyloxycarbonyl)pyrrolidine [prepared as described in step (i) above] in 2.2 ml of anisole, and the resulting mixture was stirred at room temperature for 1 hour. The solvent was then removed by distillation under reduced pressure, and the resulting residue was repeatedly washed with diethyl ether by decantation and dried in vacuo to give 1.8 g of the title compound, as a powder.
Infrared Absorption Spectrum (KBr), νₘₐₓ cm⁻¹:
   1757, 1700, 1608, 1523, 1441, 1408, 1348.
Nuclear Magnetic Resonance Spectrum (270 MHz, hexadeuterated dimethyl sulphoxide) δ ppm:
   1.61 - 1.80 (1H, multiplet);
   2.00 - 2.28 (2H, multiplet);
   2.65 - 2.86 (1H, multiplet);
   3.08 - 4.24 (13H, multiplet);
   4.31 - 4.48 (2H, multiplet);
   5.02 - 5.37 (2H, multiplet);
   5.42 (2H, singlet);
   7.52 & 7.62 (together 2H, two doublets, 8.79 Hz);
   8.23 (2H, doublet, J = 8.79 Hz);
   8.27 (2H, doublet, J = 8.30 Hz).

### PREPARATION 51

### (2S,4S)-4-Mercapto-2-(4-methyl-1-piperazinylcarbonyl)-1-methylpyrrolidine difluoromethanesulfonate

### 51(1) (2S,4S)-4-(4-Methoxybenzylthio)-2-(4-methyl-1-piperazinylcarbonyl)-1-methylpyrrolidine

700 mg of N,N'-carbonyldiimidazole were added to a suspension of 1.0 g of (2S,4S)-2-carboxy-4-(4-methoxybenzylthio)-1-methylpyrrolidine in 15 ml of dry acetonitrile, and the resulting mixture was stirred at 40°C for 30 minutes. At the end of this time, the reaction mixture was ice-cooled, and 440 µℓ of N-methylpiperazine were added to the mixture. The temperature was then allowed to rise to room temperature over a period of 30 minutes. At the end of this time, the reaction mixture was concentrated by evaporation under reduced pressure, and the residue was purified by reverse phase column chromatography using LiChroprep RP-8 (trade mark) as stationary phase and 70% v/v aqueous methanol as the eluent, to give 1060 mg of the title compound as an oil.
Nuclear Magnetic Resonance Spectrum (CDCℓ₃, 270 MHz), δ ppm:
   1.77 - 1.85 (1H, multiplet);
   2.30 (3H, singlet);
   2.32 (3H, multiplet);
   2.35 - 2.56 (5H, multiplet);
   3.05 - 3.17 (3H, multiplet);
   3.70 (2H, singlet);
   3.80 (3H, singlet);
   3.53 - 3.95 (5H, multiplet);
   6.84 (2H, doublet, J = 8.8 Hz);
   7.21 (2H, doublet, J = 8.8 Hz).
Infrared Absorption Spectrum (liquid film), νₘₐₓ cm⁻¹:
   1635, 1611, 1511, 1460, 1445, 1292, 1248, 1033, 833.

### 51(ii) (2S,4S)-4-Mercapto-2-(4-methyl-1-piperazinylcarbonyl)-1-methylpyrrolidine bis(trifluoromethanesulfonate)

10 ml of trifluoroacetic acid, followed by 510 liters of trifluoromethanesulphonic acid were added, whilst ice-cooling, to a solution of 1050 mg of (2S,4S)-4-(4-methoxybenzylthio)-2-(4-methyl-1-piperazinylcarbonyl)-1-methylpyrrolidine [prepared as described in step (i) above] in 3 ml of anisole, and the resulting mixture was stirred at room temperature for 1 hour. At the end of this time, the reaction mixture was concentrated by evaporation under reduced pressure, and the residue was triturated with diethyl ether to cause solidification. The solid was washed with diethyl ether five times and dried, to give 1350 mg of the title compound as a powder.
Nuclear Magnetic Resonance Spectrum (270 MHz, hexadeuterated dimethyl sulphoxide) δ ppm:
   1.74 - 1.86 (1H, multiplet);
   2.81 (3H, singlet);
   2.84 (3H, singlet);
   2.94 - 3.20 (4H, multiplet);
   3.28 - 3.87 (12H, multiplet).
Infrared Absorption Spectrum (KBr), νₘₐₓ cm⁻¹:
   1665, 1480, 1272, 1240, 1226, 1163, 1030, 640.

### PREPARATION 52

### (2S,4S)-2-[4-(2-Hydroxyethyl)-1-piperazinylcarbonyl)-4-mercapto-1-methylpyrrolidine bis(trifluoromethanesulfonate)

### 52(i) (2S,4S)-2-(4-[2-Hydroxyethyl)-1-piperazinylcarbonyl)-4-(4-methoxybenzylthio)-1-methylpyrrolidine

800 mg of N,N'-carbonyldiimidazole were added to a suspension of 1.13 g of (2S,4S)-2-carboxy-4-(4-methoxybenzylthio)-1-methylpyrrolidine in 20 ml of dry acetonitrile, and the resulting mixture was stirred at 40°C for 30 minutes. At the end of this time, the reaction mixture was ice-cooled, and 600 mg of N-hydroxyethylpiperazine were added to the mixture. The temperature was allowed to rise to room temperature over a period of 30 minutes. The reaction mixture was then concentrated by evaporation under reduced pressure, and the residue was purified by reverse phase column chromatography using LiChroprep RP-8 (trade mark) as the stationary phase and 70% v/v aqueous methanol as the eluent, to give 1160 mg of the title compound as a colourless oil.
Nuclear Magnetic Resonance Spectrum (CDCℓ₃, 270 MHz), δ ppm:
   1.76 - 1.87 (1H, multiplet);
   2.32 (3H, singlet);
   2.43 - 2.58 (9H, multiplet);
   3.06 - 3.18 (3H, multiplet);
   3.64 (2H, triplet, J = 5.4 Hz);
   3.70 (2H, singlet);
   3.80 (3H, singlet);
   3.52 - 4.08 (4H, multiplet);
   6.84 (2H, doublet, J = 8.8 Hz);
   7.21 (2H, doublet, J = 8.8 Hz).
Infrared Absorption Spectrum (liquid film), νₘₐₓ cm⁻¹:
   1637, 1611, 1511, 1461, 1444, 1247, 1034, 834.

### 52(ii) (2S,4S)-4-Mercapto-2-[4-(2-hydroxyethyl)-1-piperazinylcarbonyl)-1-methylpyrrolidine bis(trifluoromethanesulfonate)

10 ml of trifluoroacetic acid and 520 µℓ of trifluoromethanesulphonic acid were added, whilst ice-cooling, to a solution of 1150 mg of (2S,4S)-2-[4-(2-hydroxyethyl)-1-piperazinylcarbonyl)-4-(4-methoxybenzylthio)-1-methylpyrrolidine [prepared as described in step (i) above] in 3 ml of anisole, and the resulting mixture was stirred at room temperature for 1 hour. At the end of this time, the reaction mixture was concentrated by evaporation under reduced pressure, to give 1680 mg of the title compound as an oil.
Nuclear Magnetic Resonance Spectrum (270 MHz, D₂O, using sodium tetradeuterated trimethylsilylpropionate as an internal standard), δ ppm:
   1.99 - 2.09 (1H, multiplet);
   2.98 (3H, singlet);
   3.11 - 3.40 (8H, multiplet);
   3.60 - 3.85 (3H, multiplet);
   3.90 - 3.99 (6H, multiplet).

### PREPARATION 53

### (2S,4S)-4-Mercapto-2-[4-(2-carbamoyloxyethyl)-1-piperazinylcarbonyl]-1-(4-nitrobenzyloxycarbonyl)pyrrolidine trifluoromethanesulfonate

### 53(i) (2S,4S)-2-[4-(2-Hydroxyethyl)-1-piperazinylcarbonyl]-4-(4-methoxybenzylthio)-1-(4-nitrobenzyloxycarbonyl)pyrrolidine

10.9 g of N,N'-carbonyldiimdazole were added to a solution of 25.0 g of (2S,4S)-4-(4-methoxybenzylthio)-1-(4-nitrobenzyloxycarbonyl)-2-pyrrolidinecarboxylic acid in 200 ml of dry acetonitrile, and the resulting mixture was stirred at room temperature for 1 hour. A solution of 10.9 g of 1-(2-hydroxyethyl)piperazine in 50 ml of dry acetonitrile was added to the reaction mixture, and the resulting mixture was stirred at room temperature for 45 minutes. At the end of this time, the reaction mixture was concentrated by evaporation under reduced pressure, after which it was diluted with 800 ml of ethyl acetate. The dilute solution was washed, in turn, with water (200 ml, three times) and an aqueous solution of sodium chloride (150 ml, once). The resulting ethyl acetate solution was concentrated by evaporation under reduced pressure to a volume of 100 ml, and the crystals which precipitated were collected by filtration, to give 28.6 g of the title compound as colourless crystals, melting at 140 - 141°C.
Infrared Absorption Spectrum (KBr), νₘₐₓ cm⁻¹:
   1710, 1653, 1670, 1512, 1439, 1404, 1344.
Nuclear Magnetic Resonance Spectrum (CDCℓ₃, 270 MHz), δ ppm:
   1.48 - 1.85 (2H, multiplet);
   2.32 - 2.64 (6H, multiplet);
   2.59 (2H, triplet, J = 5.37 Hz);
   3.03 - 3.16 (1H, multiplet);
   3.30 - 3.71 (5H, multiplet);
   3.65 (2H, triplet, J = 5.37 Hz);
   3.73 (2H, singlet);
   3.79 & 3.80 (together 3H, two singlets);
   3.82 - 4.07 (1H, multiplet);
   4.56 & 4.61 (together 1H, two triplets, J = 8.30 Hz);
   5.02 - 5.31 (2H, multiplet);
   6.85 (2H, doublet, J = 8.79 Hz);
   7.23 (2H, doublet, J = 8.79 Hz);
   7.43 & 7.47 (together 2H, two doublets, J = 8.79 Hz);
   8.18 & 8.23 (together 2H, two doublets, J = 8.79 Hz).

### 53(ii) (2S,4S)-2-[4-(2-Carbamoyloxyethyl)-1-piperazinylcarbonyl]-4-(4-methoxybenzylthio)-1-(4-nitrobenzyloxycarbonyl)pyrrolidine

1.43 ml of trichloroacetyl isocyanate were added, whilst ice-cooling, to a solution of 5.59 g of (2S,4S)-2-[4-(2-hydroxyethyl)-1-piperazinylcarbonyl]-4-(4-methoxybenzylthio)-1-(4-nitrobenzyloxycarbonyl) pyrrolidine [prepared as described in step (i) above] in 50 ml of dry methylene chloride, and the resulting mixture was stirred at the same temperature for 30 minutes. At the end of this time, the solvent was removed by distillation under reduced pressure, and the resulting residue was dissolved in 120 ml of methanol. This solution was then stirred at room temperature for 4.5 hours in the presence of 35 g of silica gel (Merck, silica gel 60, 230 - 400 mesh). The silica gel was removed by filtration and the filtrate was freed from the solvent. The residue was purified by column chromatography through silica gel, using a 8 : 1 by volume mixture of ethyl acetate and methanol as the eluent, to give 5.76 g of the title compound as a colourless powder.
Infrared Absorption Spectrum (KBr), νₘₐₓ cm⁻¹:
   3353, 1711, 1652, 1608, 1513, 1344, 1242.
Nuclear Magnetic Resonance Spectrum (270 MHz, hexadeuterated dimethyl sulphoxide) δ ppm:
   1.38 - 1.57 (1H, multiplet);
   2.14 - 2.70 (7H, multiplet);
   2.98 - 4.08 (9H, multiplet);
   3.72 & 3.74 (together 3H, two singlets);
   3.77 (2H, singlet);
   4.66 & 4.77 (together 1H, two triplets, J = 7.81 Hz);
   5.02 - 5.25 (2H, multiplet);
   6.45 (2H, broad singlet);
   6.88 (2H, doublet, J = 8.79 Hz);
   7.26 (2H, doublet, J = 8.79 Hz);
   7.52 & 7.60 (together 2H, two doublets, J = 8.79 Hz);
   8.20 & 8.24 (together 2H, two doublets, J = 8.79 Hz).

### 53(iii) (2S,4S)-4-Mercapto-2-[4-(2-carbamoyloxyethyl)-1-piperazinylcarbonyl]-1-(4-nitrobenzyloxycarbonyl)pyrrolidine trifluoromethanesulfonate

2.67 ml of trifluoroacetic acid and 122 µℓ of trifluoromethanesulphonic acid were added, whilst ice-cooling, to a solution of 417 mg of (2S,4S)-4-(4-methoxybenzylthio)-2-[4-(2-carbamoyloxyethyl)-1-piperazinylcarbonyl]-1-(4-nitrobenzyloxycarbonyl) pyrrolidine [prepared as described in step (ii) above] in 753 µℓ of anisole, and the resulting mixture was stirred at the same temperature for 1 hour. At the end of this time, the solvent was removed by distillation under reduced pressure, and the residue was repeatedly washed with diethyl ether by decantation. After the residue had been dried in vacuo, 325 mg of the title compound were obtained, as a powder.
Infrared Absorption Spectrum (KBr), νₘₐₓ cm⁻¹:
   1707, 1608, 1524, 1438, 1347, 1280, 1169, 1030.
Nuclear Magnetic Resonance Spectrum (270 MHz, hexadeuterated dimethyl sulphoxide + D₂O) δ ppm:
   1.60 - 1.84 (1H, multiplet);
   2.65 - 2.90 (1H, multiplet);
   2.85 - 4.60 (14H, multiplet);
   4.63 - 5.30 (4H, multiplet);
   7.52 & 7.64 (together 2H, two doublets, J = 8.79 Hz);
   8.23 & 8.24 (together 2H, two doublets, J = 8.79 Hz).

### PREPARATION 54

### (2S,4S)-2-[4-(2-Hydroxyethyl)-1-piperazinylcarbonyl]-4-mercapto-1-(4-nitrobenzyloxycarbonyl)pyrrolidine

### 54(a) (i) trans-4-Methanesulfonyloxy-1-(4-nitrobenzyloxycarbonyl)-L-proline

12.20 ml of triethylamine and 6.81 ml of methanesulphonyl chloride were added, whilst ice-cooling, to a solution of 12.41 g of trans-4-hydroxy-1-(4-nitrobenzyloxycarbonyl)-L-proline in 100 ml of dry tetrahydrofuran, and the resulting mixture was stirred at the same temperature for 40 minutes. At the end of this time, the reaction mixture was concentrated by evaporation under reduced pressure, and the residue was mixed with 350 ml of ethyl acetate and 50 ml of 1N aqueous hydrochloric acid. The mixture was then stirred at room temperature for 2.5 hours, after which the organic layer was separated and washed three times with an aqueous solution of sodium chloride; it was then dried over anhydrous magnesium sulphate. The solvent was removed by distillation under reduced pressure, to give 12.57 g of the title compound, as a powder.
Infrared Absorption Spectrum (KBr), νₘₐₓ cm⁻¹:
   1834, 1753, 1713, 1524, 1346, 1173.
Nuclear Magnetic Resonance Spectrum (CDCℓ₃, 270 MHz), δ ppm:
   2.31 - 2.88 (2H, multiplet);
   3.08 (3H, singlet);
   3.76 - 4.07 (2H, multiplet);
   4.58 (1H, triplet, J = 7.81 Hz);
   5.05 - 5.41 (3H, multiplet);
   7.46 & 7.52 (together 2H, two doublets, J = 8.79 Hz);
   7.50 (1H, broad singlet);
   8.19 & 8.22 (together 2H, two doublets, J = 8.79 Hz).

### 54(a) (ii) (2S,4R)-2-[4-(2-Hydroxyethyl)-1-piperazinylcarbonyl]-4-methanesulfonyloxy-1-(4-nitrobenzyloxycarbonyl)pyrrolidine

1.11 g of 1-(2-hydroxyethyl)piperazine, 1.29 ml of diethyl cyanophosphonate and 1.18 ml of triethylamine were added, in that order, whilst ice-cooling, to a solution of 3.00 g of trans-4-methanesulphonyloxy-1-(4-nitrobenzyloxycarbonyl)-L-proline [prepared as described in step 54(a)(i) above] in 35 ml of dry acetonitrile, and the resulting mixture was stirred at room temperature for 30 minutes. At the end of this time, the reaction mixture was concentrated by evaporation under reduced pressure, and the residue was purified by column chromatography through silica gel, using a 5 : 1 by volume mixture of ethyl acetate and methanol as the eluent, to give 2.60 g of the title compound, as a powder.
Infrared Absorption Spectrum (KBr), νₘₐₓ cm⁻¹:
   1712, 1652, 1523, 1345, 1171.
Nuclear Magnetic Resonance Spectrum (CDCℓ₃, 270 MHz), δ ppm:
   2.23 - 2.80 (9H, multiplet);
   3.06 & 3.07 (together 3H, two singlets);
   3.44 - 4.06 (8H, multiplet);
   4.81 - 4.95 (1H, multiplet);
   5.04 - 5.41 (3H, multiplet);
   7.46 & 7.51 (together 2H, two doublets, J = 8.79 Hz);
   8.21 & 8.22 (together 2H, two doublets, J = 8.79 Hz).

### 54(a) (ii') (2S,4R)-2-[4-(2-Hydroxyethyl)-1-piperazinylcarbonyl]-4-methanesulfonyloxy-1-(4-nitrobenzyloxycarbonyl)pyrrolidine

584 mg of N,N'-carbonyldiimidazole were added to a solution of 1.16 g of trans-4-methanesulphonyloxy-1-(4-nitrobenzyloxycarbonyl)-L-proline in 10 ml of dry acetonitrile, and the resulting mixture was stirred at 40°C for 1 hour. 586 mg of 1-(2-hydroxyethyl)piperazine were then added to the mixture, whilst ice-cooling, and the resulting mixture was stirred at the same temperature for 35 minutes. The solvent was then removed by distillation under reduced pressure, and the residue was purified in a similar manner to that described in step 54(a)(ii), to give 930 mg of the title compound, as a powder. The infrared absorption spectrum and nuclear magnetic resonance spectrum of the product were identical with those of the compound obtained as described in step 54(a) (ii) above.

### 54(a) (ii'') (2S,4R)-2-[4-(2-Hydroxyethyl)-1-piperazinylcarbonyl]-4-methanesulfonyloxy-1-(4-nitrobenzyloxycarbonyl)pyrrolidine

0.21 ml of triethylamine, followed by 0.19 ml of pivaloyl chloride, were added dropwise at -20°C to a solution of 0.5 g of trans-4-methanesulphonyloxy-1-(4-nitrobenzyloxycarbonyl)-L-proline in 5 ml of dry tetrahydrofuran, and the resulting mixture was stirred at the same temperature for 5 minutes. A solution of 0.25 g of 1-(2-hydroxyethyl)piperazine in 3 ml of dry tetrahydrofuran was then added, and the reaction mixture was stirred at the same temperature for 30 minutes, after which the solvent was removed by distillation under reduced pressure. The resulting residue was worked up and purified in a similar manner to that described in step 54(a)(ii), to give 0.42 g of the title compound, as a powder. The infrared absorption spectrum and nuclear magnetic resonance spectrum of the product were identical with those of the compound obtained as described in step 54(a)(ii) above.

### 54(a) (iii) (2S,4S)-4-Acetylthio-2-[4-(2-hydroxyethyl)-1-piperazinylcarbonyl]-1-(4-nitrobenzyloxycarbonyl)pyrrolidine

685 mg of potassium thioacetate were added to a solution of 2.0 g of (2S,4R)-2-[4-(2-hydroxyethyl)-1-piperazinylcarbonyl]-4-methanesulphonyloxy-1-(4-nitrobenzyloxycarbonyl)pyrrolidine [prepared as described in step 54(a)(ii), 54(a)(ii') and 54(a)(ii'') above] in 20 ml of dry acetonitrile, and the resulting mixture was stirred at 80°C for 5 hours. At the end of this time, the reaction mixture was concentrated by evaporation under reduced pressure, the residue was diluted with 200 ml of ethyl acetate, and the dilute solution was washed with an aqueous solution of sodium chloride; it was then dried over anhydrous magnesium sulphate. The solvent was removed by distillation under reduced pressure, and the residue was purified by column chromatography through silica gel, using a gradient elution method with mixtures of ethyl acetate and methanol ranging from 9 : 1 to 4 : 1 by volume as the eluent, to give 1.35 g of the title compound, as a powder.
Infrared Absorption Spectrum (KBr), νₘₐₓ cm⁻¹:
   3437, 1710, 1652, 1522, 1345, 1113.
Nuclear Magnetic Resonance Spectrum (CDCℓ₃, 270 MHz), δ ppm:
   1.82 - 1.98 (1H, multiplet);
   2.34 (3H, singlet);
   2.31 - 2.88 (8H, multiplet);
   3.40 - 4.21 (9H, multiplet);
   4.65 - 4.78 (1H, multiplet);
   5.03 - 5.36 (2H, multiplet);
   7.45 & 7.51 (together 2H, two doublets, J = 8.79 Hz);
   8.17 - 8.24 (2H, multiplet).

### 54(a) (iv) (2S,4S)-2-[4-(2-Hydroxyethyl)-1-piperazinylcarbonyl]-4-mercapto-1-(4-nitrobenzyloxycarbonyl)pyrrolidine

A sodium methoxide solution (prepared by adding 56 mg of metallic sodium to 2.4 ml of methanol) was added to a solution of 1.06 g of (2S,4S)-4-acetylthio-2-[4-(2-hydroxyethyl)-1-piperazinyl]-1-(4-nitrobenzyloxycarbonyl)pyrrolidine [prepared as described in step 54(a)(iii) above] in 10 ml of methanol, and the resulting mixture was stirred at 15°C for 30 minutes. 610 µℓ of a 4N solution of hydrogen chloride in ethyl acetate were then added to the mixture at the same temperature, after which the mixture was stirred for 10 minutes. The reaction mixture was then concentrated by evaporation under reduced pressure, and the residue was purified by column chromatography through silica gel, using a 4 : 1 by volume mixture of ethyl acetate and methanol as the eluent, to give 710 mg of the title compound, as a powder.
Infrared Absorption Spectrum (KBr), νₘₐₓ cm⁻¹:
   3430, 2944, 1700, 1647, 1521, 1439, 1350.
Nuclear Magnetic Resonance Spectrum (CDCℓ₃, 270 MHz), δ ppm:
   1.81 - 1.98 (2H, multiplet);
   2.28 - 2.84 (8H, multiplet);
   3.20 - 3.82 (8H, multiplet);
   4.04 - 4.20 (1H, multiplet);
   4.60 - 4.77 (1H, multiplet);
   5.01 - 5.38 (2H, multiplet);
   7.45 & 7.51 (together 2H, two doublets, J = 8.79 Hz);
   8.15 - 8.25 (2H, multiplet).

### 54(a) (iv') (2S,4S)-2-[4-(2-Hydroxyethyl)-1-piperazinylcarbonyl]-4-mercapto-1-(4-nitrobenzyloxycarbonyl)pyrrolidine

40 ml of a 10 % w/v methanolic solution of hydrogen chloride were added to a solution of 1.0 g of (2S,4S)-4-acetylthio-2-[4-(2-hydroxyethyl)-1-piperazinyl]-1-(4-nitrobenzyloxycarbonyl)pyrrolidine in 10 ml of 1,4-dioxane, and the resulting mixture was stirred at 50°C for 1 hour. At the end of this time, the solvent was removed by distillation under reduced pressure, and the residue was mixed with 40 ml of tetrahydrofuran and 2 ml of a saturated aqueous solution of sodium hydrogencarbonate; it was then dried over anhydrous sodium sulphate. The solvent was removed by distillation under reduced pressure, and the residue was purified by column chromatography through silica gel in a similar manner to that described in step 54(a)(iv) above, to give 712 mg of the title compound, as a powder. The infrared absorption spectrum and nuclear magnetic resonance spectrum of the product were identical with those of the compound obtained as described in step 54(a) (iv) above.

### 54(b) (i) (2S,4S)-2-[4-(2-Hydroxyethyl)-1-piperazinylcarbonyl]-4-mercapto-1-(4-nitrobenzyloxycarbonyl)pyrrolidine bis(trifluoromethanesulfonate)

2.8 ml of trifluoroacetic acid and 91 µℓ of trifluoromethanesulphonic acid were added, whilst ice-cooling, to a solution of 288 mg of (2S,4S)-2-[4-(2-hydroxyethyl)-1-piperazinylcarbonyl]-4-(4-methoxybenzylthio)-1-(4-nitrobenzyloxycarbonyl) pyrrolidine in 580 µℓ of anisole, and the resulting mixture was stirred at room temperature for 1 hour. At the end of this time, the solvent was removed by distillation under reduced pressure, and the resulting residue was washed with diethyl ether by decantation and dried in vacuo, to give 380 mg of the title compound, as a powder.
Infrared Absorption Spectrum (KBr), νₘₐₓ cm⁻¹:
   1795, 1705, 1666, 1609, 1525, 1442, 1408, 1348, 1281, 1226, 1169.
Nuclear Magnetic Resonance Spectrum (270 MHz, D₂O) δ ppm:
   1.54 - 1.63 (1H, multiplet);
   2.61 - 2.72 (1H, multiplet);
   2.90 - 4.46 (14H, multiplet);
   4.64 - 4.96 (2H, multiplet);
   5.08 (2H, singlet);
   7.42 (2H, doublet, J = 8.79 Hz);
   8.08 & 8.10 (together 2H, two doublets, J = 8.79 Hz).

### 54(b) (ii) (2S,4S)-2-[4-(2-Hydroxyethyl)-1-piperazinylcarbonyl]-4-mercapto-1-(4-nitrobenzyloxycarbonyl)pyrrolidine

A solution of 266 mg of (2S,4S)-2-[4-(2-hydroxyethyl)-1-piperazinylcarbonyl]-4-mercapto-1-(4-nitrobenzyloxycarbonyl)pyrrolidine bis(trifluoromethanesulphonate) [prepared as described in step 54(b)(i) above] in a mixture of 5 ml of tetrahydrofuran and 0.2 ml of water was neutralised by adding 76 mg of sodium hydrogencarbonate, after which it was dried over anhydrous magnesium sulphate. The solvent was then removed by distillation under reduced pressure, and the resulting residue was purified by column chromatography through silica gel, using a 4 : 1 by volume mixture of ethyl acetate and methanol as the eluent, to give 188 mg of the title compound, as a powder. The infrared absorption spectrum and nuclear magnetic resonance spectrum of the product were identical with those of the compound obtained as described in step 54(a) (iv) above.

### PREPARATION 55

### (2S,4S)-4-Mercapto-2-(4-nitrobenzyloxycarbonyl-1-piperazinylcarbonyl)-1-methylpyrrolidine trifluoromethanesulfonate

### 55(i) (2S,4S)-4-(4-Methoxybenzylthio)-2-(4-nitrobenzyloxycarbonyl-1-piperazinylcarbonyl)-1-methylpyrrolidine

16.6 g of N,N'-carbonyldiimidazole were added to a suspension of 24 g of (2S,4S)-2-carboxy-4-(4-methoxybenzylthio)-1-methylpyrrolidine in 200 ml of dry acetonitrile, and the resulting mixture was stirred at 35°C for 40 minutes. A solution of 14.7 g of dry piperazine was then added dropwise to the mixture at a temperature of between 30°C and 35°C, after which the mixture was stirred at room temperature for 30 minutes. A solution of 36.8 g of 4-nitrobenzyloxycarbonyl chloride in 100 ml of acetonitrile was then added, whilst ice-cooling, to the reaction mixture, and the mixture thus obtained was stirred at room temperature for 1 hour. At the end of this time, the mixture was concentrated by evaporation under reduced pressure, and the residue was mixed with an aqueous solution of sodium chloride and a 10% aqueous solution of sodium carbonate. The resulting mixture was extracted with ethyl acetate. The extract was washed with an aqueous solution of sodium chloride and dried over anhydrous magnesium sulphate. The solvent was removed by distillation under reduced pressure, and the residue was purified by column chromatography through silica gel, using a 9 : 1 by volume mixture of ethyl acetate and methanol as the eluent, to afford 27.7 g of the title compound.
Infrared Absorption Spectrum (liquid film), νₘₐₓ cm⁻¹:
   1706, 1648, 1513, 1435, 1347, 1248, 1232.
Nuclear Magnetic Resonance Spectrum (CDCℓ₃, 270 MHz), δ ppm:
   1.74 - 1.89 (1H, multiplet);
   2.32 (3H, multiplet);
   2.40 - 2.62 (2H, multiplet);
   2.99 - 3.21 (3H, multiplet);
   3.31 - 4.20 (8H, multiplet);
   3.70 (2H, singlet);
   3.80 (3H, singlet);
   5.24 (2H, singlet);
   6.84 (2H, doublet, J = 8.79 Hz);
   7.20 (2H, doublet, J = 8.79 Hz);
   7.52 (2H, doublet, J = 8.79 Hz);
   8.23 (2H, doublet, J = 8.79 Hz).

### 55(ii) (2S,4S)-4-Mercapto-2-(4-nitrobenzyloxycarbonyl-1-piperazinylcarbonyl)-1-methylpyrrolidine trifluoromethanesulfonate

130 ml of trifluoroacetic acid and 4.6 ml of trifluoromethanesulphonic acid were added dropwise to a solution of 13.8 g of (2S,4S)-4-(4-methoxybenzylthio)-2(4-nitrobenzyloxycarbonyl-1-piperazinylcarbonyl)-1-methylpyrrolidine [prepared as described in step (i) above] in 28.3 ml of anisole, and the resulting mixture was stirred for 30 minutes, whilst ice-cooling. At the end of this time, the reaction mixture was concentrated by evaporation under reduced pressure, and the resulting residue was washed with hexane and ether, in that order, by decantation to give 13.9 g of the title compound as a powder.
Infrared Absorption Spectrum (KBr), νₘₐₓ cm⁻¹:
   1695, 1643, 1518, 1446, 1345, 1251.
Nuclear Magnetic Resonance Spectrum (270 MHz, hexadeuterated dimethyl sulphoxide) δ ppm:
   1.74 - 1.89 (1H, multiplet);
   2.81 & 2.82 (together 3H, two singlets);
   2.92 - 3.08 (1H, multiplet);
   3.17 (1H, singlet);
   3.31 - 3.80 (12H, multiplet);
   4.58 - 4.72 (1H, multiplet);
   5.26 (2H, singlet);
   7.65 (2H, doublet, J = 8.79 Hz);
   8.24 (2H, doublet, J = 8.79 Hz).

### PREPARATION 56

### (2S,4S)-4-Mercapto-2-[4-(4-nitrobenzyloxycarbonylmethyl)-1-piperazinylcarbonyl]-1-(4-nitrobenzyloxycarbonyl)pyrrolidine bis(trifluoromethanesulfonate)

8.0 ml of trifluoroacetic acid and 160 µℓ of trifluoromethanesulphonic acid were added, whilst ice-cooling, to a suspension of 1120 mg of (2S,4S)-4-(4-methoxybenzylthio)-2-[4-(4-nitrobenzyloxycarbonylmethyl)-1-piperazinecarbonyl]-1-(4-nitrobenzyloxycarbonyl)pyrrolidine in 1.75 ml of anisole, and the resulting mixture was stirred at room temperature for 1.5 hours. At the end of this time, the solvent was removed by distillation under reduced pressure, and the resulting residue was repeatedly washed with diethyl ether by decantation and dried in vacuo, to afford 1.58 g of the title compound, as a powder.
Infrared Absorption Spectrum (KBr), νₘₐₓ cm⁻¹:
   1756, 1704, 1667, 1523, 1441, 1348.
Nuclear Magnetic Resonance Spectrum (270 MHz, hexadeuterated dimethyl sulphoxide) δ ppm:
   1.60 - 1.76 (1H, multiplet);
   2.70 - 2.85 (1H, multiplet);
   3.08 - 3.42 (9H, multiplet);
   3.65 - 3.83 (3H, multiplet);
   3.94 & 4.05 (together 1H, two doublets of doublets, J = 9.8 & 6.8 Hz);
   4.72 & 4.81 (1H, two triplets, J = 8.1 Hz);
   5.05 - 5.26 (2H, multiplet);
   5.42 & 5.43 (2H, two singlets);
   7.52, 7.64, 7.69 & 7.70 (together 4H, four doublets, J = 8.8 Hz).
   8.23, 8.24 & 8.28 (together 4H, 3 doublets, J = 8.8 Hz).

### PREPARATION 57

### (2R,4S)-4-Mercapto-2-{4-[2-(4-nitrobenzyloxycarbonyl)oxyethyl]-1-piperazinylcarbonyl}-1-(4-nitrobenzyloxycarbonyl)pyrrolidine

### 57(i) (2R,4R)-4-Hydroxy-2-{4-[2-(4-nitrobenzyloxycarbonyl)oxyethyl]-1-piperazinylcarbonyl}-1-(4-nitrobenzyloxycarbonyl)pyrrolidine

3.34 ml of diethyl cyanophosphate and 8.92 ml of triethylamine were added, whilst ice-cooling, to a suspension of 6.2 g of cis-4-hydroxy-1-(4-nitrobenzyloxycarbonyl)-D-Proline and 8.41 g of 1-[2-(4-nitrobenzyloxycarbonyloxy)ethyl]piperazine dihydrochloride in 62 ml of dry dimethylformamide, and the resulting mixture was stirred at the same temperature for 30 minutes. At the end of this time, the reaction mixture was diluted with 250 ml of ethyl acetate, and the dilute solution was washed with water and then dried over anhydrous magnesium sulphate. The mixture was concentrated by evaporation under reduced pressure, and the resulting residue was purified by column chromatography through silica gel, using a 4 : 1 by volume mixture of ethyl acetate and methanol as the eluent, to give 7.84 g of the title compound, as an oil.
Infrared Absorption Spectrum (liquid film), νₘₐₓ cm⁻¹:
   1748, 1710, 1658, 1624, 1608, 1522, 1439, 1403, 1347, 1262.
Nuclear Magnetic Resonance Spectrum (CDCℓ₃, 270 MHz), δ ppm:
   1.98 - 2.80 (9H, multiplet);
   3.37 - 3.90 (6H, multiplet);
   4.22 - 4.46 (3H, multiplet);
   4.60 - 5.60 (5H, multiplet);
   7.42 - 7.57 (4H, multiplet);
   8.17 - 8.26 (4H, multiplet).

### 57(ii) (2R,4S)-4-Acetylthio-2-{4-[2-(4-nitrobenzyloxycarbonyl)oxyethyl]-1-piperazinylcarbonyl}-1-(4-nitrobenzyloxycarbonyl)pyrrolidine

A solution of 0.73 g of diethyl azodicarboxylate in 2 ml of tetrahydrofuran was added dropwise, whilst ice-cooling, to a solution of 2.1 g of (2R,4R)-4-hydroxy-2-{4-[2-(4-nitrobenzyloxycarbonyl)oxyethyl]-1-piperazinylcarbonyl}-1-(4-nitrobenzyloxycarbonyl) pyrrolidine [prepared as described in step (i) above] and 1.1 g of triphenylphosphine in 14 ml of tetrahydrofuran, and the resulting mixture was stirred at the same temperature for 10 minutes. A solution of 0.32 g of mercaptoacetic acid in 2 ml of tetrahydrofuran was then added dropwise to the mixture, and the mixture was stirred at room temperature for 1 hour. At the end of this time, the solvent was removed by distillation under reduced pressure, and the resulting residue was purified by column chromatography through silica gel, using a 20 : 1 by volume mixture of ethyl acetate and methanol as the eluent, to give 1.2 g of (2R,4S)-4-acetylthio-2-{4-[2-(4-nitrobenzyloxycarbonyl)oxyethyl]-1-piperazinylcarbonyl}-1-(4-nitrobenzyloxycarbonyl) pyrrolidine as a colourless powder.
Infrared Absorption Spectrum (liquid film), νₘₐₓ cm⁻¹:
   1748, 1709, 1654, 1607, 1522, 1439, 1404, 1347, 1263, 1122.
Nuclear Magnetic Resonance Spectrum (CDCℓ₃, 270 MHz), δ ppm:
   2.15 - 2.80 (8H, multiplet);
   2.34 (3H, singlet);
   3.35 - 3.76 (5H, multiplet);
   3.91 - 4.40 (4H, multiplet);
   4.68 - 4.81 (1H, multiplet);
   5.03 - 5.35 (4H, multiplet);
   7.26 - 7.57 (4H, multiplet);
   8.19 - 8.26 (4H, multiplet).

### 57(iii) (2R,4S)-4-Mercapto-2-{4-[2-(4-nitrobenzyloxycarbonyl)oxyethyl]-1-piperazinylcarbonyl}-1-(4-nitrobenzyloxycarbonyl)pyrrolidine

40 ml of a 10% w/v methanolic solution of hydrogen chloride were added to a solution of 1.0 g of (2R,4S)-4-acetylthio-2-{4-[2-(4-nitrobenzyloxycarbonyl)oxyethyl]-1-piperazinylcarbonyl}-1-(4-nitrobenzyloxycarbonyl)-pyrrolidine [prepared as described in step (ii) above] in 10 ml of 1,4-dioxane, and the resulting mixture was stirred at 50°C to 52°C for 1 hour. At the end of this time, the reaction mixture was concentrated by evaporation under reduced pressure, and the concentrate was diluted with 100 ml of ethyl acetate. The dilute solution was neutralised with a saturated aqueous solution of sodium hydrogencarbonate and washed, in turn, with 30 ml of water and with 30 ml of an aqueous solution of sodium chloride. The solvent was removed by distillation under reduced pressure, and the resulting residue was purified by column chromatography through silica gel, using a 20 : 1 by volume mixture of ethyl acetate and methanol as the eluent, to give 566 mg of the title compound, as a colourless powder.
Infrared Absorption Spectrum (liquid film), νₘₐₓ cm⁻¹:
   1748, 1709, 1653, 1607, 1521, 1439, 1404, 1346, 1263.
Nuclear Magnetic Resonance Spectrum (CDCℓ₃, 270 MHz), δ ppm:
   1.74 (1H, doublet, J = 7.32 Hz);
   2.07 - 2.86 (8H, multiplet);
   3.39 - 3.80 (6H, multiplet);
   4.04 - 4.46 (3H, multiplet);
   4.75 - 4.85 (1H, multiplet);
   5.03 - 5.35 (4H, multiplet);
   7.42 - 7.58 (4H, multiplet);
   8.17 - 8.26 (4H, multiplet).

### PREPARATION 58

### (2R,4R)-1-Mercapto-2-{4-[2-(4-nitrobenzyloxycarbonyl)oxyethyl]-1-piperazinylcarbonyl}-1-(4-nitrobenzyloxycarbonyl)pyrrolidine

### 58(i) (2R,4S)-4-Formyloxy-2-{4-[2-(4-nitrobenzyloxycarbonyl)oxyethyl]-1-piperazinylcarbonyl}-1-(4-nitrobenzyloxycarbonyl)pyrrolidine

A solution of 1.31 g of diethyl azodicarboxylate in 5 ml of tetrahydrofuran was added dropwise, whilst ice-cooling, to a solution of 3.0 g of (2R,4R)-4-hydroxy-2-{4-[2-(4-nitrobenzyloxycarbonyl)oxyethyl]-1-piperazinylcarbonyl}-1-(4-nitrobenzyloxycarbonyl) pyrrolidine [prepared as described in Preparation 57(i)] and 1.97 g of triphenylphosphine in 25 ml of tetrahydrofuran, and the resulting mixture was stirred at the same temperature for 10 minutes. 283 µℓ of formic acid were then added dropwise to the mixture, and the mixture was stirred at the same temperature for 5 minutes and at room temperature for 1 hour. At the end of this time, the solvent was removed by distillation under reduced pressure, and the resulting residue was purified by column chromatography through silica gel, using a 20 : 1 by volume mixture of ethyl acetate and methanol as the eluent, to give 1.42 g of the title compound, as a colourless powder.
Infrared Absorption Spectrum (KBr), νₘₐₓ cm⁻¹:
   1749, 1720, 1654, 1606, 1522, 1439, 1405, 1347, 1262.
Nuclear Magnetic Resonance Spectrum (CDCℓ₃, 270 MHz), δ ppm:
   2.20 - 2.95 (8H, multiplet);
   3.35 - 3.95 (6H, multiplet);
   4.15 - 4.55 (2H, multiplet);
   4.79 & 4.85 (together 1H, two triplets, J = 7.81 Hz);
   5.05 - 5.37 (4H, multiplet);
   5.43 - 5.50 (1H, multiplet);
   7.44 - 7.57 (4H, multiplet);
   8.02 (1H, singlet);
   8.19 - 8.26 (4H, multiplet).

### 58(ii) (2R,4S)-4-Hydroxy-2-{4-[2-(4-nitrobenzyloxycarbonyl)oxyethyl]-1-piperazinylcarbonyl}-1-(4-nitrobenzyloxycarbonyl)pyrrolidine

28 ml of a 10% w/v methanolic solution of hydrogen chloride were added dropwise to a solution of 1.42 g of (2R,4S)-4-formyloxy-2-{4-[2-(4-nitrobenzyloxycarbonyl)-oxyethyl]-1-piperazinylcarbonyl}-1-(4-nitrobenzyloxycarbonyl)pyrrolidine [prepared as described in step (i) above] in 14 ml of 1,4-dioxane, and the resulting mixture was stirred at room temperature for 1 hour. At the end of this time, the solvent was removed by distillation under reduced pressure, and the resulting residue was mixed with water. The aqueous mixture thus obtained was made alkaline by the addition of a saturated aqueous solution of sodium hydrogencarbonate, and then the mixture was extracted with ethyl acetate. The extract was washed with an aqueous solution of sodium chloride and dried over anhydrous magnesium sulphate. The solvent was removed by distillation under reduced pressure, and the residue was purified by column chromatography through silica gel, using a 9 : 1 by volume mixture of ethyl acetate and methanol as the eluent to give 1.33 g of the title compound, as a colourless powder.
Infrared Absorption Spectrum (KBr), νₘₐₓ cm⁻¹:
   1750, 1709, 1648, 1608, 1522, 1439, 1406, 1347, 1263.
Nuclear Magnetic Resonance Spectrum (270 MHz, CDCℓ₃ + D₂O) δ ppm:
   2.04 - 2.80 (8H, multiplet);
   3.38 - 3.82 (6H, multiplet);
   4.24 - 4.38 (2H, multiplet);
   4.50 - 4.64 (1H, multiplet);
   4.70 - 4.90 (1H, multiplet);
   5.30 - 5.34 (4H, multiplet);
   7.43 - 7.57 (4H, multiplet);
   8.17 - 8.25 (4H, multiplet).

### 58(iii) (2R,4R)-4-Acetylthio-2-{4-[2-(4-nitrobenzyloxycarbonyl)oxyethyl]-1-piperazinylcarbonyl}-1-(4-nitrobenzyloxycarbonyl)pyrrolidine

A solution of 421 mg of diethyl azodicarboxylate in 1.2 ml of tetrahydrofuran was added dropwise, whilst ice-cooling, to a solution of 1.21 g of (2R,4S)-4-hydroxy-2-{4-[2-(4-nitrobenzyloxycarbonyl)oxyethyl]-1-piperazinylcarbonyl}-1-(4-nitrobenzyloxycarbonyl) pyrrolidine and 634 mg of triphenylphosphine in 8 ml of tetrahydrofuran, and the resulting mixture was stirred at the same temperature for 10 minutes. 171 µℓ of mercaptoacetic acid were then added dropwise to the mixture, and the mixture was stirred at room temperature for 1 hour. Following the same procedure as described in Preparaation 57(ii), the reaction mixture was worked up and purified, to give 1.04 g of the title compound, as a colourless powder.
Infrared Absorption Spectrum (KBr), νₘₐₓ cm⁻¹:
   1750, 1712, 1656, 1607, 1522, 1496, 1438, 1404, 1347, 1263, 1207.
Nuclear Magnetic Resonance Spectrum (CDCℓ₃, 270 MHz), δ ppm:
   1.82 - 1.93 (1H, multiplet);
   2.34 (3H, singlet);
   2.30 - 2.82 (7H, multiplet);
   3.36 - 3.72 (5H; multiplet);
   3.90 - 4.16 (2H, multiplet);
   4.24 - 4.31 (1H, multiplet);
   4.67 & 4.74 (together 1H, two triplets, J = 7.81 Hz);
   5.03 - 5.35 (4H, multiplet);
   7.43 - 7.57 (4H, multiplet);
   8.17 - 8.25 (4H, multiplet).

### 58(iv) (2R,4R)-1-Mercapto-2-{4-[2-(4-nitrobenzyloxycarbonyl)oxyethyl]-1-piperazinylcarbonyl}-1-(4-nitrobenzyloxycarbonyl)pyrrolidine

40 ml of a 10% w/v methanolic solution of hydrogen chloride were added to a solution of 1.0 g of (2R,4R)-4-acetylthio-2-{4-[2-(4-nitrobenzyloxycarbonyl)oxyethyl]-1-piperazinylcarbonyl}-1-(4-nitrobenzyloxycarbonyl)pyrrolidine [prepared as described in step (iii) above] in 10 ml of 1,4-dioxane, and the resulting mixture was stirred at between 50°C and 52°C for 1 hour. Following the same procedure as described in Preparation 57(iii), the reaction mixture was worked up and purified, to afford 648 mg of the title compound, as a colourless powder.
Infrared Absorption Spectrum (KBr), νₘₐₓ cm⁻¹:
   1749, 1710, 1653, 1607, 1522, 1496, 1439, 1404, 1346, 1263, 1206.
Nuclear Magnetic Resonance Spectrum (CDCℓ₃, 270 MHz), δ ppm:
   1.82 - 1.96 (2H, multiplet);
   2.30 - 2.91 (7H, multiplet);
   3.18 - 3.78 (6H, multiplet);
   4.05 - 4.46 (3H, multiplet);
   4.63 & 4.68 (together 1H, two triplets, J = 7.81 Hz);
   5.03 - 5.33 (4H, multiplet);
   7.43 - 7.57 (4H, multiplet);
   8.17 - 8.26 (4H, multiplet).

### PREPARATIONS 59 TO 88

The mercaptans shown in Preparations 59 to 88 were prepared in a similar manner to that described in Preparations 1, 49 and 66, but using (2S,4S)-4-(4-methoxybenzylthio)-1-(4-nitrobenzyloxycarbonyl)-2-pyrrolidinecarboxylic acid, (2S,4S)-2-carboxy-4-(4-methoxybenzylthio)-1-methylpyrrolidine and (2S,4S)-1-(t-butoxycarbonyl)-4-(4-methoxybenzylthio)-2-pyrrolidinecarboxylic acid as starting materials.

### PREPARATION 59

### (2S,4S)-4-Mercapto-2-[(2S)-4-(N-4-nitrobenzyloxycarbonylacetimidoyl)-2-methylpiperazin-1-ylcarbonyl-1-(4-nitrobenzyloxycarbonyl)pyrrolidine

Infrared Absorption Spectrum (Liquid film), νₘₐₓ cm⁻¹:
   1709, 1656, 1606, 1569, 1520, 1430, 1346, 1252.
Nuclear Magnetic Resonance Spectrum (CDCℓ₃, 270 MHz), δ ppm:
   1.12 - 1.38 (3H, multiplet);
   1.75 - 2.10 (2H, multiplet);
   2.12 - 2.38 (3H, multiplet);
   2.55 - 3.93 (8H, multiplet);
   4.01 - 4.89 (4H, multiplet);
   5.04 - 5.30 (4H, multiplet);
   7.42 - 7.59 (4H, multiplet);
   8.17 - 8.23 (4H, multiplet).

### PREPARATION 60

### (2S,4S)-4-Mercapto-2-[(2S)-4-(N-4-nitrobenzyloxycarbonylformimidoyl)-2-methylpiperazin-1-ylcarbonyl]-1-(4-nitrobenzyloxycarbonyl)pyrrolidine trifluoromethanesulfonate

Nuclear Magnetic Resonance Spectrum (hexadeuterated dimethyl sulphoxide + D₂O, 270 MHz), δ ppm:
   1.10 - 1.40 (3H, multiplet);
   1.62 - 1.78 (1H, multiplet);
   2.60 - 3.40 (8H, multiplet);
   3.91 - 4.08 (2H, multiplet);
   4.58 - 4.81 (1H, multiplet);
   5.06 - 5.27 (2H, multiplet);
   5.36 (2H, singlet);
   7.53 - 7.70 (4H, multiplet);
   8.19 - 8.28 (4H, multiplet);
   8.89 (1H, singlet).
Infrared Absorption Spectrum (KBr), νₘₐₓ cm⁻¹:
   1785, 1695, 1609, 1523, 1442, 1349, 1283, 1246, 1031.

### PREPARATION 61

### (2S,4S)-4-Mercapto-1-methyl-2-[(3S)-3-(N-4-nitrobenzyloxycarbonylacetimidoylamino)pyrrolidin-1-ylcarbonyl]pyrrolidine

Infrared Absorption Spectrum (KBr), νₘₐₓ cm⁻¹:
   1522, 1348, 858, 740.
Nuclear Magnetic Resonance Spectrum (270 MHz, D₂O, using sodium tetradeuterated trimethylsilylpropionate as an internal standard), δ ppm:
   1.70 - 2.00 (3H, multiplet);
   2.00 - 2.25 (3H, multiplet);
   2.30 - 3.95 (13H, multiplet);
   3.95 - 4.07 (1H, multiplet);
   4.30 - 4.50 (1H, multiplet);
   7.62 (2H, doublet, J = 8.79 Hz);
   8.23 (2H, doublet, J = 8.79 Hz).

### PREPARATION 62

### (2S,4S)-4-Mercapto-2-[3-(N-4-nitrobenzyloxycarbonylacetimidoylamino)piperidin-1-ylcarbonyl]-1-(4-nitrobenzyloxycarbonyl)pyrrolidine

Infrared Absorption Spectrum (KBr), νₘₐₓ cm⁻¹:
   1705, 1650, 1600, 1550, 1520, 1440, 1340, 1205.

### PREPARATION 63

### (2S,4S)-4-Mercapto-1-methyl-2-[4-(N-4-nitrobenzyloxycarbonylacetimidoylamino)piperidin-1-ylcarbonyl]-pyrrolidine

Infrared Absorption Spectrum (KBr), νₘₐₓ cm⁻¹:
   1710, 1520, 1345, 1210.

### PREPARATION 64

### (2S,4S)-4-Mercapto-1-methyl-2-[4-(N-4-nitrobenzyloxycarbonylformimidoyl)piperazin-1-ylcarbonyl]pyrrolidine bis(trifluoromethanesulfonate),

Nuclear Magnetic Resonance Spectrum (hexadeuterated dimethyl sulphoxide + D₂O, 270 MHz), δ ppm:
   1.79 - 1.91 (1H, multiplet);
   2.83 (3H, singlet);
   2.96 - 3.07 (1H, multiplet);
   3.10 - 3.28 (4H, multiplet);
   3.47 - 3.85 (7H, multiplet);
   4.61 (1H, triplet, J = 9.4 Hz);
   5.36 (2H, singlet);
   7.68 (2H, doublet, J = 8.8 Hz);
   8.26 (2H, doublet, J = 8.8 Hz);
   8.89 (1H, singlet).

### PREPARATION 65

### (2S,4S)-4-Mercapto-2-[(2S)-4-(N-4-nitrobenzyloxycarbonylacetimidoyl)-2-methylpiperazin-1-ylcarbonyl]-1-methyl-pyrrolidine

### PREPARATION 66

### (2S,4S)-4-Mercapto-2-[4-(4-nitrobenzyloxycarbonyl)piperazin-1-ylcarbonyl]-1-(N-4-nitrobenzyloxycarbonylacetimidoyl)pyrrolidine

(2S,4S)-1-(t-Butoxycarbonyl)-4-(4-methoxybenzylthio)-2-[4-(4-nitrobenzyloxycarbonyl)piperazin-1-ylcarbonyl]pyrrolidine was prepared from (2S,4S)-1-(t-butoxycarbonyl)-4-(4-methoxybenzylthio)pyrrolidine-2-carboxylic acid, N,N'-carbonyldiimidazole and 4-(4-nitrobenzyloxycarbonyl)piperazine. This compound was then treated with a 4N solution of hydrogen chloride in ethyl acetate and the product was then subjected to similar reactions to those described in Preparations 1a and 17(iii), to give the title compound, melting at 181.5°C.
Nuclear Magnetic Resonance Spectrum (CDCℓ₃, 270 MHz), δ ppm:
   1.93 (1H, doublet, J = 9.2 Hz);
   1.90 - 2.02 (1H, multiplet);
   2.33 (3H, singlet);
   2.61 - 2.72 (1H, multiplet);
   3.08 - 3.88 (9H; multiplet);
   4.03 (2H, doublet of doublets, J = 10.6 & 7.3 Hz);
   4.89 (1H, triplet, J = 7.3 Hz);
   5.06 - 5.31 (4H, multiplet);
   7.43 - 7.52 (4H, multiplet);
   8.12 - 8.26 (4H, multiplet).

### PREPARATION 67

### (2S,4S)-4-mercapto-2-[4-(4-nitrobenzyloxycarbonyl)piperazin-1-ylcarbonyl]-1-(N-4-nitrobenzyloxycarbonylformimidoyl)pyrrolidine trifluoromethanesulfonate

Infrared Absorption Spectrum (KBr), νₘₐₓ cm⁻¹:
   1783, 1693, 1660, 1608, 1523, 1465, 1441, 1349, 1256, 1228.
Nuclear Magnetic Resonance Spectrum (hexadeuterated dimethyl sulphoxide + D₂O, 270 MHz), δ ppm:
   1.67 - 1.79 (1H, multiplet);
   2.82 - 2.92 (1H, multiplet);
   3.02 - 3.10 (1H, multiplet);
   3.40 - 3.80 (10H, multiplet);
   4.62 (1H, triplet, J = 8.30 Hz);
   5.26 (2H, singlet);
   5.36 (2H, singlet);
   7.65 (2H, doublet, J = 8.79 Hz);
   7.68 (2H, doublet, J = 8.79 Hz);
   8.25 (2H, doublet, J = 8.79 Hz);
   8.26 (2H, doublet, J = 8.79 Hz);
   8.89 (1H, singlet).

### PREPARATION 68

### (2S,4S)-4-Mercapto-1-(N-4-nitrobenzyloxycarbonylacetimidoyl)-2-[4-(N-4-nitrobenzyloxycarbonylacetimidoyl)piperazin-1-ylcarbonyl]pyrrolidine

Infrared Absorption Spectrum (KBr), νₘₐₓ cm⁻¹:
   1782, 1705, 1635, 1522, 1440, 1348, 1280, 1250, 1225.

### PREPARATION 69

### (2S,4S)-4-Mercapto-1-(N-4-nitrobenzyloxycarbonylacetimidoyl)-2-[4-(N-4-nitrobenzyloxycarbonylformimidoyl)piperazin-1-ylcarbonyl]pyrrolidine

### PREPARATION 70

### (2S,4S)-4-Mercapto-1-(N-4-nitrobenzyloxycarbonylformimidoyl)-2-[4-(N-4-nitrobenzyloxycarbonylformimidoyl)piperazin-1-ylcarbonyl]pyrrolidine

### PREPARATION 71

### (2S,4S)-4-Mercapto-1-(N-4-nitrobenzyloxycarbonylformimidoyl)-2-(4-(N-4-nitrobenzyloxycarbonylacetimidoyl)piperazin-1-ylcarbonyl]pyrrolidine

### PREPARATION 72

### (2S,4S)-4-Mercapto-1-(N-4-nitrobenzyloxycarbonylacetimidoyl)-2-[(3S)-3-(N-4-nitrobenzyloxycarbonylacetimidoylamino)pyrrolidin-1-ylcarbonyl]pyrrolidine

Infrared Absorption Spectrum (KBr), νₘₐₓ cm⁻¹:
   1710, 1645, 1522, 1445, 1347.

### PREPARATION 73

### (2S,4S)-4-Mercapto-1-(N-4-nitrobenzyloxycarbonylacetimidoyl)-2-[(3S)-3-(N-4-nitrobenzyloxycarbonylformimidoylamino)pyrrolidin-1-ylcarbonyl]pyrrolidine

### PREPARATION 74

### (2S,4S)-4-Mercapto-1-(N-4-nitrobenzyloxycarbonylacetimidoyl)-2-[(3S)-3-(N-4-nitrobenzyloxycarbonylamino)pyrrolidin-1-ylcarbonyl]pyrrolidine

Infrared Absorption Spectrum (KBr), νₘₐₓ cm⁻¹:
   1708, 1646, 1525, 1442, 1348.

### PREPARATION 75

### (2S,4S)-4-Mercapto-1-(N-4-nitrobenzyloxycarbonylformimidoyl)-2-[(3S)-3-(N-4-nitrobenzyloxycarbonylacetimidoylamino)pyrrolidin-1-ylcarbonyl]pyrrolidine

### PREPARATION 76

### (2S,4S)-4-Mercapto-1-(N-4-nitrobenzyloxycarbonylformimidoyl)-2-[(3S)-3-(N-4-nitrobenzyloxycarbonylformimidoylamino)pyrrolidin-1-ylcarbonyl]pyrrolidine

### PREPARATION 77

### (2S,4S)-4-Mercapto-1-(N-4-nitrobenzyloxycarbonylformimidoyl)-2-[(3S)-3-(N-4-nitrobenzyloxycarbonylamino)pyrrolidin-1-ylcarbonyl]pyrrolidine

### PREPARATION 78

### (2S,4S)-4-Mercapto-1-(N-4-nitrobenzyloxycarbonylacetimidoyl)-2-[4-(4-nitrobenzyloxycarbonyl)homopiperazin-1-ylcarbonyl]pyrrolidine

### PREPARATION 79

### (2S,4S)-4-Mercapto-1-(N-4-nitrobenzyloxycarbonylacetimidoyl)-2-[4-(N-4-nitrobenzyloxycarbonylformimidoyl)homopiperazin-1-ylcarbonyl]pyrrolidine

### PREPARATION 80

### (2S,4S)-4-Mercapto-1-(N-4-nitrobenzyloxycarbonylformimidoyl)-2-[4-(4-nitrobenzyloxycarbonyl)homopiperazin-1-ylcarbonyl]pyrrolidine

### PREPARATION 81

### (2S,4S)-4-Mercapto-1-(N-4-nitrobenzyloxycarbonylformimidoyl)-2-[4-(N-4-nitrobenzyloxycarbonylformimidoyl)homopiperazin-1-ylcarbonyl]pyrrolidine

### PREPARATION 82

### (2S,4S)-4-Mercapto-2-{(3S)-3-[N-methyl-N-(N-4-nitrobenzyloxycarbonylacetimidoyl) amino]pyrrolidin-1-ylcarbonyl}-1-(4-nitrobenzyloxycarbonyl)pyrrolidine

### PREPARATION 83

### (2S,4S)-4-Mercapto-2-[2-(4-nitrobenzyloxycarbonyloxymethyl)-4-(4-nitrobenzyloxycarbonyl)piperazin-1-ylcarbonyl]-1-(4-nitrobenzyloxycarbonyl)pyrrolidine

### PREPARATION 84

### (2S,4S)-4-Mercapto-2-[4-(N-4-nitrobenzyloxycarbonylacetimidoyl)-2-(4-nitrobenzyloxycarbonyloxymethyl)-piperazin-1-ylcarbonyl]-1-(4-nitrobenzyloxycarbonyl) pyrrolidine

### PREPARATION 85

### (2S,4S)-4-Mercapto-2-[4-(4-nitrobenzyloxycarbonyl)-6-(4-nitrobenzyloxycarbonyloxy)homopiperazin-1-ylcarbonyl]-1-(4-nitrobenzyloxycarbonyl)pyrrolidine

### PREPARATION 86

### (2S,4S)-4-Mercapto-2-[4-(N-4-nitrobenzyloxycarbonylformimidoyl)-6-(4-nitrobenzyloxycarbonyloxy)homopiperazin-1-ylcarbonyl]-1-(4-nitrobenzyloxycarbonyl) pyrrolidine

### PREPARATION 87

### (2S,4S)-4-Mercapto-1-(N-4-nitrobenzyloxycarbonylacetimidoyl)-2-[4-(N-4-nitrobenzyloxycarbonylacetimidoylamino)piperidin-1-ylcarbonyl]pyrrolidine

### PREPARATION 88

### (2S,4S)-4-Mercapto-1-methyl-2-[4-(N-4-nitrobenzyloxycarbonylformimidoyl)homopiperazin-1-ylcarbonyl]pyrrolidine

### PREPARATION 89

### (2S,4S)-4-(Methoxybenzylthio)-1-(4-nitrobenzyloxycarbonyl)-2-pyrrolidinecarboxylic acid

### 89(i) (2S,4S)-4-(4-Methoxybenzylthio)-2-pyrrolidinecarboxylic acid

A solution of 4.0 g of (2S,4S)-2-carbamoyl-4-(4-methoxybenzylthio)-2-pyrrolidine hydrochloride dissolved in 40 ml of 2N aqueous hydrochloric acid was stirred in an oil bath kept at 95 - 110°C for 1.5 hours. At the end of this time, the reaction mixture was cooled to room temperature, and its pH was adjusted to a value of from 4 to 6 by the addition of about 40 ml of a 2N aqueous solution of sodium hydroxide, whilst stirring. The crystals which precipitated were collected by filtration, washed with water and subjected to air-drying, to give 3.25 g of the title compound, melting at 198 - 200°C.
Nuclear Magnetic Resonance Spectrum (270 MHz, hexadeuterated dimethyl sulphoxide) δ ppm:
   1.69 (1H, doublet of triplets, J = 13.2 & 8.3 Hz);
   2.44 (1H, doublet of triplets, J = 13.2 & 6.8 Hz);
   2.90 (1H, doublet of doublets, J = 11.2 & 7.8 Hz);
   3.15 - 3.60 (4H, multiplet);
   3.66 (1H, triplet, J = 8.3 Hz);
   3.73 (3H, singlet);
   3.74 (2H, singlet);
   6.88 (2H, doublet, J = 8.8 Hz);
   7.25 (2H, doublet, J = 8.8 Hz).
Infrared Absorption Spectrum (KBr), νₘₐₓ cm⁻¹:
   1610, 1576, 1511, 1445, 1376, 1243.

### 89(ii) (2S,4S)-4-(4-Methoxybenzylthio)-1-(4-nitrobenzyloxycarbonyl)-2-pyrrolidinecarboxylic acid

A suspension of 1.87 g of (2S,4S)-4-(4-methoxybenzylthio)-2-pyrrolidinecarboxylic acid [prepared as described in step (i) above] in 80 ml of a 1 : 1 by volume mixture of tetrahydrofuran and water was transformed to a homogeneous solution by adding 7 ml of a 1N aqueous solution of sodium hydroxide. The solution was ice-cooled and stirred, and, little by little, a solution of 1510 mg of 4-nitrobenzyloxycarbonyl chloride in 10 ml of tetrahydrofuran and 7 ml of a 1N aqueous solution of sodium hydroxide were simultaneously added dropwise. The resulting mixture was stirred at the same temperature for 10 minutes. At the end of this time, the reaction mixture was freed from tetrahydrofuran by distillation under reduced pressure, and its pH was adjusted to a value of between 2 and 3 by the addition of 1N aqueous hydrochloric acid. The crystals which precipitated were collected by filtration, washed well with water and subjected to air-drying. The crystals were further washed with a small amount of diethyl ether and dried, to give 2.42 g of the title compound, melting at 96 - 98°C.
Infrared Absorption Spectrum (KBr), νₘₐₓ cm⁻¹:
   3000, 1746, 1673, 1511, 1341, 1178.
Nuclear Magnetic Resonance Spectrum (CDCℓ₃, 270 MHz), δ ppm:
   2.03 - 2.18 (1H, multiplet);
   2.52 - 2.68 (1H, multiplet);
   3.08 - 3.22 (1H, multiplet);
   3.27 - 3.42 (1H, multiplet);
   3.72 (2H, singlet);
   3.79 (3H, singlet);
   3.77 - 3.98 (1H, multiplet);
   4.38 (1H, triplet, J = 7.3 Hz);
   5.03 - 5.35 (2H, multiplet);
   6.85 (2H, doublet, J = 8.8 Hz);
   7.22 (2H, doublet, J = 8.8 Hz);
   7.42 & 7.48 (together 2H, two doublets, J = 8.3 Hz);
   8.16 & 8.22 (together 2H, two doublets, J = 8.3 Hz);
   5.4 - 6.6 (1H, broad doublet).

### PREPARATION 90

### (2S,4S)-4-Mercapto-2-{4-[2-(4-nitrobenzyloxycarbonyl)oxyethyl]-1-piperazinylcarbonyl}-1-(4-nitrobenzyloxycarbonyl)pyrrolidine

### 90(a) (i) (2S,4S)-2-{4-[2-(4-Nitrobenzyloxycarbonyl)oxyethyl]-1-piperazinylcarbonyl}-4-(4-methoxybenzylthio)-1-(4-nitrobenzyloxycarbonyl) pyrrolidine

A solution of 5.86 g of 4-dimethylaminopyridine and 10.35 g of p-nitrobenzyl chloroformate in 40 ml of dry methylene chloride was added, whilst ice-cooling, to a solution of 22.35 g of (2S,4S)-2-[4-(2-hydroxyethyl)-1-piperazinylcarbonyl]-4-(4-methoxybenzylthio)-1-(4-nitrobenzyloxycarbonyl)pyrrolidine in 160 ml of dry methylene chloride, and the resulting mixture was stirred at room temperature for 1 hour. The reaction mixture was then diluted with 300 ml of ethyl acetate and the dilute solution was washed, in turn, with water (100 ml, once), with an aqueous solution of sodium hydrogencarbonate (100 ml, once) and with an aqueous solution of sodium chloride (100 ml, once). The solvent was then removed by distillation under reduced pressure, and the resulting residue was purified by column chromatography through silica gel, using ethyl acetate as the eluent, to give 26.35 g of the title compound, as a colourless powder.
Infrared Absorption Spectrum (KBr), νₘₐₓ cm⁻¹:
   1748, 1710, 1655, 1608, 1521, 1346, 1251.
Nuclear Magnetic Resonance Spectrum (CDCℓ₃, 270 MHz), δ ppm:
   1.72 - 1.84 (1H, multiplet);
   2.26 - 2.73 (6H, multiplet);
   2.97 - 3.16 (1H, multiplet);
   3.29 - 4.10 (7H, multiplet);
   3.72 (2H, singlet);
   3.79 & 3.80 (together 3H, two singlets);
   4.24 - 4.31 (2H, multiplet);
   4.52 - 4.63 (1H, multiplet);
   5.00 - 5.35 (4H, multiplet);
   6.85 (2H, doublet, J = 8.79 Hz);
   7.23 (2H, doublet, J = 8.79 Hz);
   7.41 - 7.57 (4H, multiplet);
   8.16 - 8.25 (4H, multiplet).

### 90(a) (ii) (2S,4S)-4-Mercapto-2-{4-[2-(4-nitrobenzyloxycarbonyl)oxyethyl]-1-piperazinylcarbonyl}-1-(4-nitrobenzyloxycarbonyl)pyrrolidine bis(trifluoromethanesulfonate)

135.75 mg of trifluoroacetic acid and 6.18 ml of trifluoromethanesulphonic acid were added, whilst ice-cooling, to a solution of 26.00 g of (2S,4S)-2-{4-[2-(4-nitrobenzyloxycarbonyl)oxyethyl]-1-piperazinylcarbonyl}-4-(4-methoxybenzylthio-1-(4-nitrobenzyloxycarbonyl)pyrrolidine in 38.3 ml of anisole, and the resulting mixture was stirred at the same temperature for 1.5 hours. At the end of this time, the solvent was removed by distillation under reduced pressure, and the resulting residue was repeatedly washed with diethyl ether by decantation and dried in vacuo, to afford 32.5 g of the title compound, as a powder.

### 90(a) (iii) (2S,4S)-4-Mercapto-2-{4-[2-(4-nitrobenzyloxycarbonyl)oxyethyl]-1-piperazinylcarbonyl}-1-(4-nitrobenzyloxycarbonyl)pyrrolidine

20 ml of a 5% w/v aqueous solution of sodium hydrogencarbonate was added to 862 mg of (2S,4S)-4-mercapto-2-{4-[2-(4-nitrobenzyloxycarbonyl)oxyethyl]-1-piperazinylcarbonyl}-1-(4-nitrobenzyloxycarbonyl) pyrrolidine bis(trifluoromethanesulphonate), and the mixture was extracted with 50 ml of ethyl acetate. The extract was washed with water and dried over anhydrous magnesium sulphate. The solvent was removed by distillation under reduced pressure, and the resulting residue was purified by column chromatography through silica gel, using a 20 : 1 by volume mixture of ethyl acetate and methanol as the eluent, to afford 514 mg of the title compound, as a powder.
Infrared Absorption Spectrum (KBr), νₘₐₓ cm⁻¹:
   2530, 1748, 1710, 1653, 1521, 1347.
Nuclear Magnetic Resonance Spectrum (CDCℓ₃, 270 MHz), δ ppm:
   1.83 (1H, multiplet);
   2.44 - 2.79 (7H, multiplet);
   3.22 - 3.64 (6H, multiplet);
   4.06 - 4.17 (1H, multiplet);
   4.26 - 4.36 (2H, multiplet);
   4.60 - 4.71 (1H, multiplet);
   5.02 - 5.33 (4H, multiplet);
   7.42 - 7.58 (4H, multiplet);
   8.17 - 8.26 (4H, multiplet).

### 90(b) (i) (2S,4R)-4-Hydroxy-2-{4-[2-(4-nitrobenzyloxycarbonyl)oxyethyl]-1-piperazinylcarbonyl}-1-(4-nitrobenzyloxycarbonyl)pyrrolidine

25.7 ml of diethyl cyanophosphonate and 68.7 ml of triethylamine were added dropwise, whilst ice-cooling, to a suspension of 47.8 g of trans-4-hydroxy-1-(4-nitrobenzyloxycarbonyl)-L-proline and 64.8 g of 1-[2-(4-nitrobenzyloxycarbonyloxy)ethyl]piperazine dihydrochloride in 400 ml of dry dimethylformamide, and the resulting mixture was stirred at the same temperature for 30 minutes. At the end of this time, the reaction mixture was diluted with 1.5 liters of ethyl acetate, and the dilute solution was washed with water and dried over anhydrous magnesium sulphate. The solvent was removed by distillation under reduced pressure, to give 87.6 g of the title compound, as a powder.
Infrared Absorption Spectrum (KBr), νₘₐₓ cm⁻¹:
   1749, 1709, 1650, 1607, 1522, 1499, 1347, 1263.
Nuclear Magnetic Resonance Spectrum (CDCℓ₃, 270 MHz), δ ppm:
   1.63 (1H, singlet);
   1.92 - 2.38 (8H, multiplet);
   3.41 - 3.83 (6H, multiplet);
   4.24 - 4.32 (2H, multiplet);
   4.55 - 4.60 (1H, multiplet);
   4.79 - 4.90 (1H, multiplet);
   5.03 - 5.35 (4H, multiplet);
   7.44 - 7.57 (4H, multiplet);
   8.17 - 8.25 (4H, multiplet).

### 90(b) (i') (2S,4R)-4-Hydroxy-2-{4-[2-(4-nitrobenzyloxycarbonyl)oxyethyl]-1-piperazinylcarbonyl}-1-(4-nitrobenzyloxycarbonyl)pyrrolidine

608 µℓ of chlorotrimethylsilane and 670 µℓ of triethylamine, whilst ice-cooling, were added to a solution of 620 mg of trans-4-hydroxy-1-(4-nitrobenzyloxycarbonyl)-L-proline in 20 ml of dry acetonitrile, and the resulting mixture was stirred at room temperature for 1 hour. At the end of this time, the solvent was removed by distillation under reduced pressure, and the resulting residue was mixed with an aqueous solution of sodium chloride. The mixture was then extracted with ethyl acetate. The extract was washed with an aqueous solution of sodium chloride and dried over anhydrous magnesium sulphate. The solvent was then removed by distillation under reduced pressure, to give 648 mg of trans-1-(4-nitrobenzyloxycarbonyl)-4-trimethylsilyloxy-L-proline as a powder. The whole of this was dissolved in 14 ml of dry acetonitrile, and then 330 mg of N,N'-carbonyldiimidazole were added. The mixture thus obtained was stirred at room temperature for 1 hour. At the end of this time, a solution of 630 mg of 1-[2-(4-nitrobenzyloxycarbonyl)oxyethyl]piperazine in 2 ml of dry acetonitrile was added to the reaction mixture, and the resulting mixture was stirred overnight at room temperature and then at 40°C for a further 1 hour. The reaction mixture was then mixed with 14 ml of 1N aqueous hydrochloric acid and stirred at room temperature for 1 hour. At the end of this time, the mixture was concentrated by evaporation under reduced pressure, and the concentrate was made slightly alkaline by the addition of an aqueous solution of sodium hydrogencarbonate; it was then extracted with ethyl acetate. The extract was washed with water and dried over anhydrous magnesium sulphate. The solvent was removed by distillation under reduced pressure, and the resulting residue was purified by column chromatography through silica gel, using a 9 : 1 by volume mixture of ethyl acetate and methanol as the eluent, to give 589 mg of the title compound, as a powder. The infrared absorption spectrum and nuclear magnetic resonance spectrum of the compound thus obtained were identical with those of the compound prepared as described in step 90(b) (i) above.

### 90(b) (i'') (2S,4R)-4-Hydroxy-2-{4-[2-(4-nitrobenzyloxycarbonyl)oxyethyl-1-piperazinylcarbonyl}-1-(4-nitrobenzyloxycarbonyl)pyrrolidine

343 mg of N,N'-carbonyldiimidazole were added to a solution of 674 mg of trans-1-(4-nitrobenzyloxycarbonyl)-4-trimethylsilyloxy-L-proline in 14 ml of dry acetonitrile, and the resulting mixture was stirred at room temperature for 1 hour. A solution of 275 mg of 1-(2-hydroxyethyl)piperazine in 1 ml of dry acetonitrile was then added to the mixture, and the mixture was stirred overnight at room temperature. At the end of this time, the mixture was concentrated by evaporation under reduced pressure, and the concentrate was mixed with an aqueous solution of sodium chloride. The mixture was then extracted with ethyl acetate. The extract was washed with water and dried over anhydrous magnesium sulphate. The solvent was then removed by distillation under reduced pressure, to give 574 mg of (2S,4R)-2-[4-(2-hydroxyethyl)-1-piperazinylcarbonyl]-4-trimethylsilyloxy-1-(4-nitrobenzyloxycarbonyl)pyrrolidine as an oil. The whole of the compound thus obtained was dissolved in 5.7 ml of methylene chloride, and 170 mg of 4-dimethylaminopyridine and 300 mg of 4-nitrobenzyl chloroformate were added to the solution, whilst ice-cooling. The resulting mixture was stirred at room temperature for 1 hour and then the solvent was removed by distillation under reduced pressure. 15 ml of 1N aqueous hydrochloric acid were added to the residue, and the mixture was stirred at room temperature for 1 hour. The reaction mixture was then made slightly alkaline by the addition of an aqueous solution of sodium hydrogencarbonate, after which it was extracted with ethyl acetate. The extract was washed with water and dried over anhydrous magnesium sulphate. The solvent was then removed by distillation under reduced pressure. Following the procedure described in step 90(b)(i') above, the residue was purified to give 348 mg of the title compound, as a powder. The infrared absorption spectrum and nuclear magnetic resonance spectrum of the compound thus obtained were identical with those of the compound prepared as described in step 90(b) (i) above.

### 90(b) (ii) (2S,4S)-4-Acetylthio-2-{4-[2-(4-nitrobenzyloxycarbonyl)oxyethyl]-1-piperazinylcarbonyl}-1-(4-nitrobenzyloxycarbonyl)pyrrolidine

A solution of 36.5 g of diethyl azodicarboxylate in 100 ml of tetrahydrofuran was added dropwise, whilst ice-cooling, to a solution of 105 g of (2S,4R)-4-hydroxy-2-{4-[2-(4-nitrobenzyloxycarbonyl)oxyethyl]-1-piperazinylcarbonyl}-1-(4-nitrobenzyloxycarbonyl) pyrrolidine [prepared as described in steps 90(b)(i), 90(b)(i') and 90(b)(i'') above] and 55 g of triphenylphosphine in 700 ml of tetrahydrofuran, and the resulting mixture was stirred at the same temperature for 10 minutes. A solution of 15.9 g of mercaptoacetic acid in 100 ml of tetrahydrofuran was then added dropwise to the mixture, and the mixture was stirred at room temperature for 1 hour. At the end of this time, the reaction mixture was concentrated by evaporation under reduced pressure, and the concentrate was dissolved in 1.5 liters of ethyl acetate. The resulting solution was then washed with water and with an aqueous solution of sodium chloride, in that order, after which it was dried over anhydrous sodium sulphate. The solvent was removed by distillation under reduced pressure, and then the residue was mixed with 400 ml of diisopropyl ether. The diisopropyl ether-soluble materials were extracted and discarded. The same extraction operations were repeated four times, and then the resulting residue was purified by column chromatography through 3 kg of silica gel, using a gradient elution method, with mixtures of ethyl acetate and methanol ranging from 1 : 0 to 20 : 1 by volume as the eluent, to give 88.4 g of the title compound, as a colourless powder.
Infrared Absorption Spectrum (KBr), νₘₐₓ cm⁻¹:
   1749, 1711, 1655, 1522, 1347, 1262, 1110.
Nuclear Magnetic Resonance Spectrum (CDCℓ₃, 270 MHz), δ ppm:
   1.82 - 1.93 (1H, multiplet);
   2.34 (3H, singlet);
   2.35 - 2.82 (7H, multiplet);
   3.37 - 3.70 (5H, multiplet);
   3.91 - 4.05 (1H, multiplet);
   4.07 - 4.17 (1H, multiplet);
   4.23 & 4.36 (together 2H, multiplet);
   4.64 - 4.77 (1H, multiplet);
   5.02 - 5.35 (4H, multiplet);
   7.43 - 7.57 (4H, multiplet);
   8.18 - 8.26 (4H, multiplet).

### 90(c) (i) (2S,4R)-4-Methanesulfonyloxy-2-{4-[2-(4-nitrobenzyloxycarbonyl)oxyethyl]-1-piperazinylcarbonyl}-1-(4-nitrobenzyloxycarbonyl)pyrrolidine

89 µℓ of triethylamine and 50 µℓ of methanesulphonyl chloride were added, whilst ice-cooling, to a solution of 321 mg of (2S,4R)-4-hydroxy-2-{4-[2-(4-nitrobenzyloxycarbonyl)oxyethyl]-1-piperazinylcarbonyl}-1-(4-nitrobenzyloxycarbonyl) pyrrolidine in 3.2 ml of dry tetrahydrofuran, and the resulting mixture was stirred at between 0°C and 5°C for 30 minutes and then at room temperature for a further 1 hour. At the end of this time, the reaction mixture was concentrated by evaporation under reduced pressure, and the resulting residue was mixed with an aqueous solution of sodium hydrogencarbonate; the mixture was then extracted with ethyl acetate. The extract was washed with an aqueous solution of sodium chloride and dried over anhydrous magnesium sulphate. The solvent was removed by distillation under reduced pressure, to give 345 mg of the title compound as a powder.
Infrared Absorption Spectrum (KBr), νₘₐₓ cm⁻¹:
   1751, 1710, 1654, 1607, 1523, 1436, 1406.
Nuclear Magnetic Resonance Spectrum (CDCℓ₃, 270 MHz), δ ppm:
   2.22 - 3.01 (8H, multiplet);
   3.06 (3H, singlet);
   3.40 - 4.03 (6H, multiplet);
   4.25 - 4.47 (2H, multiplet);
   4.84 & 4.89 (together 1H, two triplets, J = 7.33 Hz);
   5.04 - 5.37 (5H, multiplet);
   7.46 & 7.50 (together 2H, two doublets, J = 8.79 Hz);
   7.56 (2H, doublet, J = 8.79 Hz);
   8.19 - 8.26 (4H, multiplet).

### 90(c) (ii) (2S,4S)-4-Acetylthio-2-{4-[2-(4-nitrobenzyloxycarbonyl)oxyethyl]-1-piperazinylcarbonyl}-1-(4-nitrobenzyloxycarbonyl)pyrrolidine

51 µℓ of thioacetic acid were added, whilst ice-cooling, to a suspension of 26 mg of sodium hydride (as a 55% w/w dispersion in mineral oil) in 1.4 ml of dry N,N-dimethylformamide, and the resulting mixture was stirred at room temperature for 30 minutes. A solution of 340 mg of (2S,4R)-4-methanesulphonyloxy-2-{4-[2-(4-nitrobenzyloxycarbonyl)oxyethyl]-1-piperazinylcarbonyl}-1-(4-nitrobenzyloxycarbonyl) pyrrolidine [prepared as described in step 90(c)(i) above] in 2 ml of dry N,N-dimethylformamide was then added to the mixture, and the mixture was stirred at between 80°C and 90°C for 4 hours. At the end of this time, the temperature of the reaction mixture was allowed to reduce to room temperature, after which the mixture was poured into an aqueous solution of sodium chloride and extracted with ethyl acetate. The extract was washed with water and dried over anhydrous magnesium sulphate. The solvent was removed by distillation under reduced pressure, and the resulting residue was worked up and purified according to the procedure described in step 90(b)(i) above, to give 166 mg of the title compound, as a powder. The infrared absorption spectrum and nuclear magnetic resonance spectrum of the compound thus obtained were identical with those of the compound prepared as described in step 90(b)(ii) above.

### 90(c) (ii') (2S,4S)-4-Acetylthio-2-{4-[2-(4-nitrobenzyloxycarbonyl)oxyethyl]-1-piperazinylcarbonyl}-1-(4-nitrobenzyloxycarbonyl)pyrrolidine

A solution of 1.10 g of p-nitrobenzyl chloroformate in 10 ml of methylene chloride was added, whilst ice-cooling, to a solution of 1.63 g of (2S,4S)-4-acetylthio-2-[4-(2-hydroxyethyl)-1-piperazinylcarbonyl]-1-(4-nitrobenzyloxycarbonyl)pyrrolidine and 0.62 g of 4-dimethylaminopyridine in 15 ml of methylene chloride and the resulting mixture was stirred at the same temperature for 2 hours. At the end of this time, the reaction mixture was diluted with 100 ml of ethyl acetate, and the dilute solution was washed, in turn, with 100 ml of an aqueous solution of sodium hydrogencarbonate, with 100 ml of water and with 100 ml of an aqueous solution of sodium chloride. The solvent was removed by distillation under reduced pressure, and the resulting residue was purified by column chromatography through silica gel, using a gradient elution method, with mixtures of ethyl acetate and methanol ranging from 30 : 1 to 25 : 1 by volume as the eluent to give 1.86 g of the title compound, as a powder. The infrared absorption spectrum and nuclear magnetic resonance spectrum of the compound thus obtained were identical with those of the compound prepared as described in step 90(b) (ii) above.

### 90(c) (iii) (2S,4S)-4-Mercapto-2-{4-[2-(4-nitrobenzyloxycarbonyl)oxyethyl]-1-piperazinylcarbonyl}-1-(4-nitrobenzyloxycarbonyl)pyrrolidine

600 ml of a 10% w/v methanolic solution of hydrogen chloride were added to a solution of 140 g of (2S,4S)-4-acetylthio-2-{4-[2-(4-nitrobenzyloxycarbonyl)oxyethyl]-1-piperazinylcarbonyl}-1-(4-nitrobenzyloxycarbonyl)pyrrolidine [prepared as described in step 90(b)(ii) or 90(c)(ii) above] in 150 ml of 1,4-dioxane, and the resulting mixture was stirred at 50°C for 1 hour. At the end of this time, the reaction mixture was concentrated by evaporation under reduced pressure, and the concentrate was diluted with 1500 ml of ethyl acetate. The dilute solution was neutralised with an aqueous solution of sodium hydrogencarbonate and washed, in turn, with 300 ml of water and with 300 ml of an aqueous solution of sodium chloride. The solvent was removed by distillation under reduced pressure, and the resulting residue was purified by column chromatography through silica gel, using a gradient elution method, with mixtures of ethyl acetate and methanol ranging from 30 : 1 to 20 : 1 by volume as the eluent, to give 96.44 g of the title compound, as a colourless powder. The infrared absorption spectrum and nuclear magnetic resonance spectrum of the compound thus obtained were identical with those of the compound prepared as described in step 90(a) (iii) above.

### PREPARATION 91

### (2S,4S)-1-(t-Butoxycarbonyl)-4-(4-methoxybenzylthio)-2-pyrrolidinecarboxylic acid

A solution of 0.95 g of di-t-butyldicarbonate in 4 ml of tetrahydrofuran and 4.4 ml of a 1N aqueous solution of sodium hydroxide were simultaneously added dropwise, whilst ice-cooling, to a solution of 0.97 g of (2S,4S)-4-(4-methoxybenzylthio)-2-pyrrolidinecarboxylic acid in 18 ml of tetrahydrofuran and 3.6 ml of a 1N aqueous solution of sodium hydroxide. The mixture was then stirred at room temperature for 1 hour, after which the tetrahydrofuran was removed by evaporation under reduced pressure, and the residue was acidified to a pH value of 2-3 by the addition of 1N aqueous hydrochloric acid, and the resulting mixture was extracted with ethyl acetate. The ethyl acetate solution was washed with water and with an aqueous solution of sodium chloride, after which it was dried over anhydrous magnesium sulphate. The solvent was then removed by distillation under reduced pressure, to give 1.33 g of the title compound.
Infrared Absorption Spectrum (Liquid film), νₘₐₓ cm⁻¹:
   1808, 1732, 1626, 1586, 1552, 1509, 1482, 1436, 1367, 1325, 1315, 1299, 1286, 1246, 1221.
Nuclear Magnetic Resonance Spectrum (CDCℓ₃ + D₂O, 270 MHz), δ ppm:
   1.45 (9H, singlet);
   1.88 - 2.61 (3H, multiplet);
   3.03 - 3.34 (2H, multiplet);
   3.64 - 3.95 (1H, multiplet);
   3.72 (2H, singlet);
   3.80 (3H, singlet);
   4.15 - 4.35 (1H, multiplet);
   6.85 (2H, doublet, J = 8.79 Hz);
   7.23 (2H, doublet, J = 8.79 Hz).

## Claims (Claims for the following Contracting State(s): AT, BE, CH, DE, DK, FR, GB, IT, LI, LU, MC, NL, PT, SE)

1. A compound of formula (I): 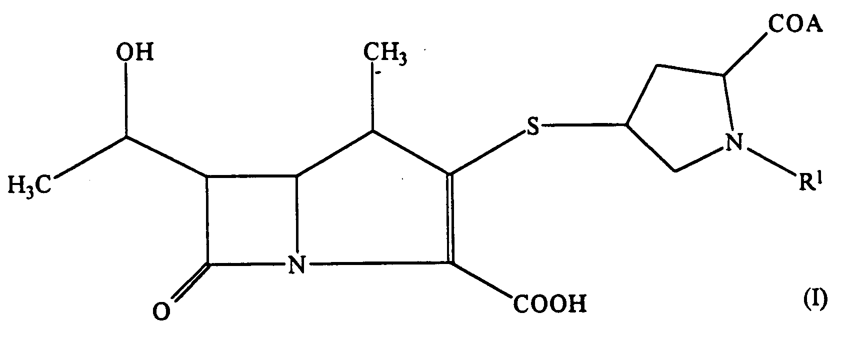 in which:
R¹ represents:
a hydrogen atom,
an unsubstituted alkyl group having from 1 to 6 carbon atoms,
a substituted alkyl group which has from 1 to 6 carbon atoms and which is substituted by at least one of substituents (a), defined below,
an alkenyl group having from 2 to 6 carbon atoms,
an alkynyl group having from 2 to 6 carbon atoms, or
a group of formula -C(=NH)R°, where R° represents a hydrogen atom or an alkyl group having from 1 to 6 carbon atoms; and
A represents a group of formula (A1), (A2), (A3), (A4), (A5), (A6), (A7) or (A8): 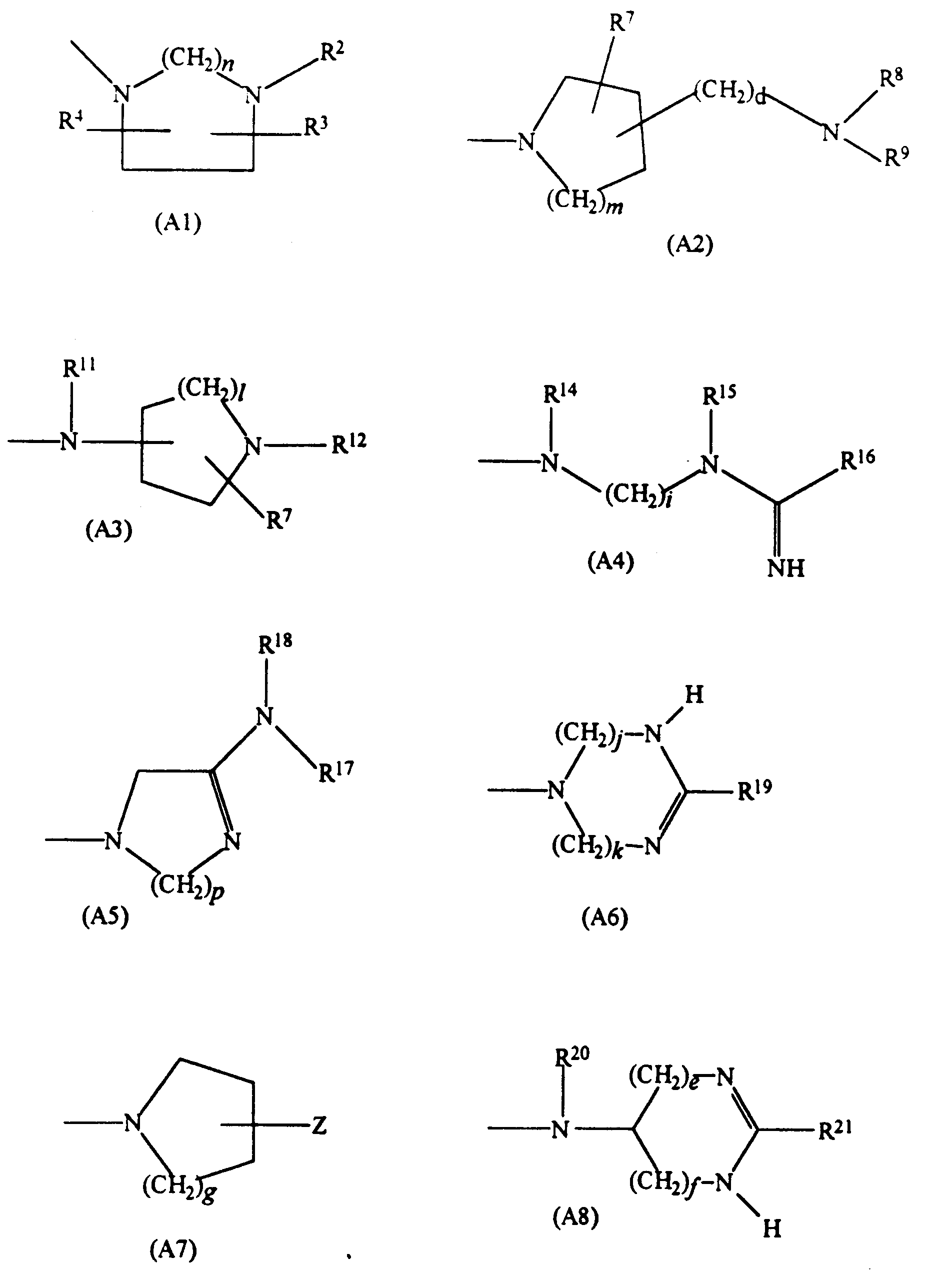 in which:
R² represents:
a hydrogen atom,
an unsubstituted alkyl group having from 1 to 6 carbon atoms,
a substituted alkyl group which has from 1 to 6 carbon atoms and which is substituted by at least one of substituents (b), defined below,
an alkenyl group having from 2 to 6 carbon atoms,
an alkynyl group having from 2 to 6 carbon atoms, or
a group of formula -C(=NH)R⁶,
where R⁶ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
R³, R⁴ and R⁷ are the same or different and each represents:
a hydrogen atom,
an unsubstituted alkyl group having from 1 to 6 carbon atoms,
a substituted alkyl group which has from 1 to 6 carbon atoms and which is substituted by at least one of substituents (d), defined below,
a halogen atom,
a hydroxy group,
a carboxy group,
a group of formula -CO.NR^{a}R^{b}, -OCO.NR^{a}R^{b} or -NR^{a}R^{b},
in which R^{a} and R^{b} are the same or different and each represents a hydrogen atom or an alkyl group having from 1 to 4 carbon atoms, or
a cyano group;
R⁸ represents:
a hydrogen atom,
an unsubstituted alkyl group having from 1 to 6 carbon atoms,
a substituted alkyl group which has from 1 to 6 carbon atoms and which is substituted by at least one of substituents (a), defined below,
an alkenyl group having from 2 to 6 carbon atoms, or
an alkynyl group having from 2 to 6 carbon atoms;
R⁹ represents:
a hydrogen atom,
an unsubstituted alkyl group having from 1 to 6 carbon atoms,
a substituted alkyl group which has from 1 to 6 carbon atoms and which is substituted by at least one of substituents (a), defined below, or
a group of formula -C(=NH)R¹⁰,
where R¹⁰ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
or
R⁸ and R⁹ together represent a group of formula -(CH₂)ₛ-W-(CH₂)ₜ-
in which W represents a carbon-carbon single bond, an oxygen atom, a sulphur atom or a group of formula >NR²², in which R²² represents a hydrogen atom or an alkyl group having from 1 to 6 carbon atoms, and
s and t are the same or different and each is 1, 2 or 3;
R¹¹ represents a hydrogen atom or an alkyl group having from 1 to 6 carbon atoms;
R¹² represents:
a hydrogen atom,
an unsubstituted alkyl group having from 1 to 6 carbon atoms,
a substituted alkyl group which has from 1 to 6 carbon atoms and which is substituted by at least one of substituents (a), defined below,
an alkenyl group having from 2 to 6 carbon atoms,
an alkynyl group having from 2 to 6 carbon atoms, or
a group of formula -C(=NH)R¹³,
where R¹³ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
R¹⁴ and R¹⁵ are the same or different and each represents a hydrogen atom or an alkyl group having from 1 to 6 carbon atoms;
R¹⁶ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
R¹⁷ and R¹⁸ are the same or different and each represents:
a hydrogen atom,
an unsubstituted alkyl group having from 1 to 6 carbon atoms, or
a substituted alkyl group which has from 1 to 6 carbon atoms and which is substituted by at least one of substituents (a), defined below;
or
R¹⁷ and R¹⁸ together represent a group of formula -(CH₂)_{q}-Y-(CH₂)ᵣ-
in which Y represents a carbon-carbon single bond, an oxygen atom, a sulphur atom or a group of formula >NR²³, in which R²³ represents a hydrogen atom or an alkyl group having from 1 to 6 carbon atoms, and
q and r are the same or different and each is 1, 2 or 3;
R¹⁹, R²⁰ and R²¹ are the same or different and each represents a hydrogen atom or an alkyl group having from 1 to 6 carbon atoms;
Z represents an imidazolyl, triazolyl or tetrazolyl group;
d is 0 or 1;
e, f, i, j and k are the same or different and each is 1 or 2;
q, ℓ and m are the same or different and each is 0, 1 or 2; and
n and p are the same or different and each is 1, 2 or 3;
PROVIDED THAT, where A represents a group of formula (A1):
R², R³ and R⁴ do not all represent hydrogen atoms when R¹ represents a hydrogen atom; and
R¹, R³ and R⁴ do not all represent hydrogen atoms when R² represents an alkyl group;
said substituents (a) are selected from:
hydroxy groups,
carboxy groups,
cyano groups,
halogen atoms,
oxygen atoms to form an oxo group,
alkoxy groups having from 1 to 6 carbon atoms, and
groups of formula -CO.NR^{a}R^{b}, -OCO.NR^{a}R^{b} and -NR^{a}R^{b}, in which R^{a} and R^{b} are as defined above;
said substituents (b) are selected from:
hydroxy groups,
carboxy groups,
cyano groups,
halogen atoms,
alkoxy groups having from 1 to 6 carbon atoms,
groups of formula -CO.NR^{a}R^{b}, -OCO.NR^{a}R^{b} and -NR^{a}R^{b}, in which R^{a} and R^{b} are as defined above,
sulphamoyl groups,
ureido groups,
sulpho groups,
alkanoyl groups having from 1 to 6 carbon atoms,
alkanoylamino groups having from 1 to 6 carbon atoms,
alkanoyloxy groups having from 1 to 6 carbon atoms,
alkylthio groups having from 1 to 6 carbon atoms,
alkylsulphinyl groups having from 1 to 6 carbon atoms, and
alkylsulphonyl groups having from 1 to 6 carbon atoms;
said substituents (c) are selected from:
halogen atoms,
alkoxy groups having from 1 to 6 carbon atoms,
cycloalkyl groups having from 3 to 7 ring carbon atoms; and
said substituents (d) are selected from:
hydroxy groups,
cyano groups,
groups of formula -CO.NR^{a}R^{b}, -OCO.NR^{a}R^{b} and -NR^{a}R^{b}, in which R^{a} and R^{b} are as defined above,
carboxy groups,
halogen atoms, and
alkoxy groups having from 1 to 6 carbon atoms;
and pharmaceutically acceptable salts and esters thereof.

2. A compound according to Claim 1, which has the formula (Ia): 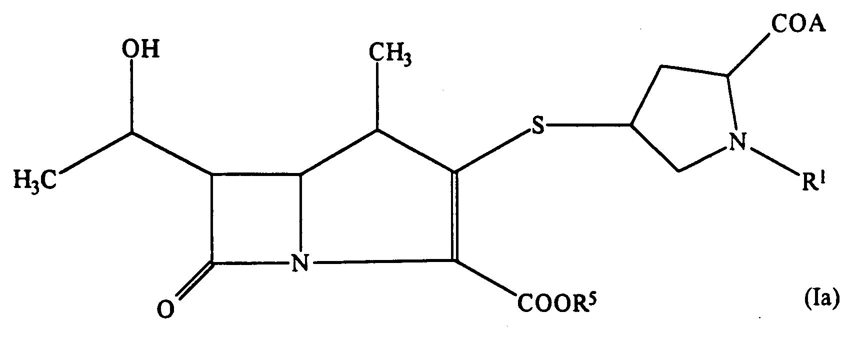 in which R¹ and A are as defined in Claim 1, and R⁵ represents:
a C₁ - C₂₀ alkyl group;
a C₃ - C₇ cycloalkyl group,
an aralkyl group, in which the alkyl part is a C₁ - C₃ alkyl group and the aryl part is a C₆ - C₁₄ carbocyclic aromatic group which may be substituted or unsubstituted and, if substituted, has at least one of substituents (e) defined below, an alkenyl group, which is substituted or unsubstituted and, if substituted, has at least one of substituents (a) defined in Claim 1;
a halogenated C₁ - C₆ alkyl group,
a substituted silylalkyl groups, in which the alkyl part has from 1 to 6 carbon atoms, and the silyl group has up to 3 substituents selected from C₁ - C₆ alkyl groups and phenyl groups which are unsubstituted or have at least one of substituents (e) defined below;
a phenyl group, which is unsubstituted or has at least one of substituents (e) defined below;
a phenacyl group, which is unsubstituted or has at least one of substituents (e) defined below;
a cyclic or acyclic terpenyl group;
an alkoxymethyl group, in which the alkoxy part is C₁ - C₆;
an aliphatic acyloxyalkyl group, in which the acyl group is a C₂ - C₆ alkanoyl group, and the alkyl part is a C₂ - C₆ alkyl group;
a cycloalkyl-substituted aliphatic acyloxyalkyl group, in which the acyl group is a C₂ - C₆ alkanoyl group, the cycloalkyl substituent is C₃ - C₇, and the alkyl part is a C₁ - C₆ alkyl group;
an alkoxycarbonyloxyalkyl group, in which the alkoxy part is C₁ - C₁₀, and the alkyl part is C₁ - C₆;
a cycloalkylcarbonyloxyalkyl or cycloalkyloxycarbonyloxyalkyl group, in which the cycloalkyl group is C₃ - C₁₀, is mono- or poly- cyclic and is unsubstituted or is substituted by at least one C₁ - C₄ alkyl group, and the alkyl group is a C₁ - C₆;
a cycloalkylalkoxycarbonyloxyalkyl group, in which the alkoxy group has a single cycloalkyl substituent, the cycloalkyl substituent being C₃ - C₁₀ and mono- or poly- cyclic;
a terpenylcarbonyloxyalkyl or terpenyloxycarbonyloxyalkyl group, in which the alkyl group has from 1 to 6 carbon atoms;
a 5-alkyl or 5-phenyl (2-oxo-1,3-dioxolen-4-yl)alkyl group in which each alkyl group is C₁ - C₆; or
a phthalidyl, indanyl or 2-oxo-4,5,6,7-tetrahydro-1,3-benzodioxolen-4-yl group; and
substituents (e) are selected from C₁ - C₄ alkyl groups, C₁ - C₄ alkoxy groups, C₁ - C₄ haloalkyl groups, C₁ -C₃ alkylenedioxy groups, halogen atoms, cyano groups and nitro groups.

3. A compound according to Claim 2, in which R⁵ represents a hydrogen atom, a (5-substituted 2-oxo-1,3-dioxolen-4-yl)methyl group, a 1-methylcyclohexylcarbonyloxymethyl group, a 1-isopropoxycarbonyloxyethyl group or a 1-cyclohexylcarbonyloxyethyl group.

4. A compound according to any one of Claims 1 to 3, in which R¹ represents: a hydrogen atom; an alkyl group having from 1 to 3 carbon atoms; a substituted alkyl group having from 1 to 3 carbon atoms, in which the substituent is selected from substituents (a'), defined below; an alkenyl group having 3 or 4 carbon atoms; an alkynyl group having 3 or 4 carbon atoms; a formimidoyl group; or an acetimidoyl group; and
substituents (a') are selected from hydroxy groups, carboxy groups, carbamoyl groups, carbamoyloxy groups, cyano groups, halogen atoms, alkoxy groups having from 1 to 3 carbon atoms, amino groups, and mono- and dialkylamino groups in which the or each alkyl group has from 1 to 3 carbon atoms.

5. A compound according to any one of Claims 1 to 4, in which A represents a group of formula (A1), and n is 2 or 3.

6. A compound according to any one of Claims 1 to 5, in which R² represents:
a hydrogen atom;
an alkyl group having from 1 to 3 carbon atoms;
a substituted alkyl group having from 1 to 3 carbon atoms, in which the substituent is selected from substituents (b'), defined below;
an alkenyl group having 3 or 4 carbon atoms;
an alkynyl group having 3 or 4 carbon atoms; or
a group of formula -C(=NH)R⁶,
where R⁶ represents
a hydrogen atom,
an unsubstituted alkyl group having from 1 to 3 carbon atoms,
a substituted alkyl group which has from 1 to 3 carbon atoms and which is substituted by at least one substituent selected from halogen atoms,
alkoxy groups having from 1 to 3 carbon atoms and cycloalkyl groups having from 3 to 6 carbon atoms, or
a cycloalkyl group having from 3 to 6 ring carbon atoms; and
substituents (b') are selected from: hydroxy groups, carboxy groups, carbamoyl groups, carbamoyloxy groups, cyano groups, sulphamoyl groups, ureido groups, sulpho groups, alkoxy groups having from 1 to 3 carbon atoms, alkoxycarbonyl groups having from 2 to 4 carbon atoms, alkanoyl groups having from 2 to 4 carbon atoms, alkanoylamino groups having from 2 to 4 carbon atoms, alkanoyloxy groups having from 2 to 4 carbon atoms, amino groups, mono- and di- alkylamino groups in which the or each alkyl group has from 1 to 3 carbon atoms, alkylthio groups having from 1 to 3 carbon atoms, alkylsulphinyl groups having from 1 to 3 carbon atoms, alkylsulphonyl groups having from 1 to 3 carbon atoms, mono- and di- alkylcarbamoyl groups in which the or each alkyl group has from 1 to 3 carbon atoms, and mono- and di- alkylcarbamoyloxy groups in which the or each alkyl group has from 1 to 3 carbon atoms.

7. A compound according to any one of Claims 1 to 6, in which A represents a group of formula (A1), and R³ and R⁴ each represents a hydrogen atom, an alkyl group having from 1 to 3 carbon atoms, a hydroxy group, a carboxy group, a carbamoyl group or a substituted alkyl group which has from 1 to 3 carbon atoms and which is substituted by at least one substituent selected from hydroxy groups, alkoxy groups having from 1 to 3 carbon atoms, amino groups, carbamoyl groups and halogen atoms.

8. A compound according to Claim 1 or Claim 2, in which:
A represents a group of formula (A1);
n is 2;
R¹ represents a hydrogen atom, a methyl group, a fluoromethyl group, a formimidoyl group or an acetimidoyl group;
R² represents a hydrogen atom, a 2-hydroxyethyl group, a 2-carbamoylethyl group, a carboxymethyl group, a carbamoylmethyl group, a 2-fluoroethyl group, a formimidoyl group or an acetimidoyl group; and
R³ and R⁴ are the same or different and each represents a hydrogen atom, a methyl group, a carbamoyl group, a cyano group, a carboxy group, a hydroxymethyl group, a fluoromethyl group or an aminomethyl group.

9. A compound according to Claim 1 or Claim 2, in which:
A represents a group of formula (A1);
n is 3;
R¹ represents a hydrogen atom, a methyl group, a fluoromethyl group, a formimidoyl group or an acetimidoyl group;
R² represents a hydrogen atom, a methyl group, a formimidoyl group, an acetimidoyl group, a carboxymethyl group, a carbamoylmethyl group, a 2-hydroxyethyl group or a 2-fluoroethyl group; and
R³ and R⁴ are the same or different and each represents a hydrogen atom, a methyl group, a hydroxy group, an amino group, a cyano group, a carboxy group, a carbamoyl group, a carbamoyloxy group, a hydroxymethyl group, a fluoromethyl group or an aminomethyl group.

10. A compound according to Claim 1 or Claim 2, in which:
A represents a group of formula (A1);
n is 2;
R¹ represents a hydrogen atom, a methyl group, a formimidoyl group or an acetimidoyl group;
R² represents a hydrogen atom, a 2-hydroxyethyl group, a carboxymethyl group, a formimidoyl group or an acetimidoyl group;
R³ represents a hydrogen atom; and
R⁴ represents a methyl group, a carbamoyl group, a cyano group, a hydroxymethyl group, a fluoromethyl group or an aminomethyl group.

11. A compound according to Claim 1 or Claim 2, in which:
A represents a group of formula (A1);
n is 3;
R¹ represents a hydrogen atom, a methyl group, a formimidoyl group or an acetimidoyl group;
R² represents a formimidoyl group, an acetimidoyl group, a carboxymethyl group, a 2-hydroxyethyl group or a 2-fluoroethyl group; and
R³ and R⁴ are the same or different and each represents a hydrogen atom, a hydroxy group, an amino group or a cyano group.

12. A compound according to Claim 1 or Claim 2, in which:
A represents a group of formula (A2), and R⁷ represents: a hydrogen atom; a carboxy group; a carbamoyl group; an alkyl group having from 1 to 3 carbon atoms; or a substituted alkyl group having from 1 to 3 carbon atoms, in which the substituent is selected from hydroxy groups, alkoxy groups having from 1 to 3 carbon atoms, carbamoyl groups, carboxy groups and cyano groups.

13. A compound according to Claim 1, in which A represents a group of formula (A2), and R⁸ represents: a hydrogen atom; an alkyl group having from 1 to 3 carbon atoms; a substituted alkyl group having from 1 to 3 carbon atoms, in which the substituent is selected from hydroxy groups, alkoxy groups having from 1 to 3 carbon atoms, carbamoyl groups, carbamoyloxy groups, carboxy groups, cyano groups, amino groups and halogen atoms; an alkenyl group having 3 or 4 carbon atoms; or an alkynyl group having 3 or 4 carbon atoms.

14. A compound according to Claim 1 or Claim 2, in which A represents a group of formula (A2), and R⁹ represents: a hydrogen atom; an alkyl group having from 1 to 3 carbon atoms; a substituted alkyl group having from 1 to 3 carbon atoms, in which the substituent is selected from hydroxy groups, alkoxy groups having from 1 to 3 carbon atoms, carbamoyl groups, carbamoyloxy groups, carboxy groups, cyano groups, amino groups and halogen atoms; or a group of formula -C(=NH)R¹⁰, in which R¹⁰ represents:
a hydrogen atom;
an alkyl group having from 1 to 3 carbon atoms;
a substituted alkyl group having from 1 to 3 carbon atoms, in which the substituent is selected from alkoxy groups having from 1 to 3 carbon atoms and halogen atoms;
a cycloalkyl group having from 3 to 6 carbon atoms; or
an alkyl group having from 1 to 3 carbon atoms, which is substituted by a single cycloalkyl group having from 3 to 6 carbon atoms.

15. A compound according to Claim 1 or Claim 2, in which A represents a group of formula (A2), and R⁸ and R⁹ together represent a group of formula -(CH₂)ₛ-W-(CH₂)ₜ-, in which W represents a carbon-carbon single bond, an oxygen atom, a sulphur atom or a group of formula >NR²², in which R²² represents a hydrogen atom or an alkyl group having from 1 to 3 carbon atoms, s is 1, 2 or 3 and t is 2.

16. A compound according to Claim 1 or Claim 2, in which:
A represents a group of formula (A2);
d is 0, or 1;
m is 0, 1 or 2;
R¹ represents a hydrogen atom, a methyl group, a fluoromethyl group, a formimidoyl group or an acetimidoyl group;
R⁷ represents a hydrogen atom, an alkyl group having from 1 to 3 carbon atoms, a hydroxy group, an amino group, a cyano group, a halogen atom, a carboxy group, a carbamoyl group or a hydroxymethyl group;
R⁸ represents a hydrogen atom, an alkyl group having from 1 to 3 carbon atoms, a fluoromethyl group, a carbamoylmethyl group, a carboxymethyl group, an alkenyl group having 3 or 4 carbon atoms, an alkynyl group having 3 or 4 carbon atoms, a 2-haloethyl group, a 2-hydroxyethyl group, a 2-alkoxyethyl group, in which the alkoxy part has from 1 to 3 carbon atoms, or a 2-aminoethyl group;
R⁹ represents a hydrogen atom, an alkyl group having from 1 to 3 carbon atoms, a fluoromethyl group, a carbamoylmethyl group, a carboxymethyl group, a formimidoyl group, an acetimidoyl group, a 2-haloethyl group, a 2-hydroxyethyl group, a 2-alkoxyethyl group, in which the alkoxy part has from 1 to 3 carbon atoms, or a 2-aminoethyl group;
or
R⁸ and R⁹ together represent a group of formula
-(CH₂)₄-,
-(CH₂)₅-,
-(CH₂)₂O(CH₂)₂-,
-(CH₂)₂S(CH₂)₂-,
-(CH₂)₂NH(CH₂)₂- or
-(CH₂)₂NCH₃(CH₂)₂-.

17. A compound according to Claim 1 or Claim 2, in which:
A represents a group of formula (A2);
d is 0;
m is 1 or 2;
R¹ represents a hydrogen atom, a methyl group, a fluoromethyl group, a formimidoyl group or an acetimidoyl group;
R⁷ represents a hydrogen atom;
R⁸ represents a hydrogen atom, an alkyl group having from 1 to 3 carbon atoms, a carbamoylmethyl group, a carboxymethyl group, a 2-fluoroethyl group or a 2-hydroxyethyl group; and
R⁹ represents a hydrogen atom, an alkyl group having from 1 to 3 carbon atoms, a formimidoyl group, an acetimidoyl group or a 2-fluoroethyl group.

18. A compound according to Claim 1 or Claim 2, in which A represents a group of formula (A3), ℓ is 0, 1 or 2, and R⁷ represents a hydrogen atom or an alkyl group having from 1 to 3 carbon atoms.

19. A compound according to Claim 1 or Claim 2, in which A represents a group of formula (A3), and R¹¹ represents a hydrogen atom or an alkyl group having from 1 to 3 carbon atoms, such as a methyl, ethyl or propyl group.

20. A compound according to Claim 1 or Claim 2, in which A represents a group of formula (A3), and R¹² represents: a hydrogen atom; an alkyl group having from 1 to 3 carbon atoms; a substituted alkyl group having from 1 to 3 carbon atoms, in which the substituent is selected from hydroxy groups, alkoxy groups having from 1 to 3 carbon atoms, carbamoyl groups, carbamoyloxy groups, carboxy groups, cyano groups, amino groups and halogen atoms; an alkenyl group having 3 or 4 carbon atom; an alkynyl group having 3 or 4 carbon atoms; or a group of formula -C(=NH)R¹³, in which R¹³ represents:
a hydrogen atom;
an alkyl group having from 1 to 3 carbon atoms;
a substituted alkyl group having from 1 to 3 carbon atoms, in which the substituent is selected from alkoxy groups having from 1 to 3 carbon atoms and halogen atoms;
a cycloalkyl group having from 3 to 6 carbon atoms; or
an alkyl group having from 1 to 3 carbon atoms, which is substituted by a single cycloalkyl group having from 3 to 6 carbon atoms.

21. A compound according to Claim 1 or Claim 2, in which:
A represents a group of formula (A3);
ℓ is 0, 1 or 2;
R¹ represents a hydrogen atom, a methyl group, a fluoromethyl group, a formimidoyl group or an acetimidoyl group;
R⁷ represents a hydrogen atom;
R¹¹ represents a hydrogen atom or an alkyl group having from 1 to 3 carbon atoms; and
R¹² represents a hydrogen atom, an alkyl group having from 1 to 3 carbon atoms, a fluoromethyl group, a carbamoylmethyl group, a carboxymethyl group, an alkenyl group having 3 or 4 carbon atoms, an alkynyl group having 3 or 4 carbon atoms, a formimidoyl group, an acetimidoyl group, a 2-haloethyl group, a 2-hydroxyethyl group, a 2-alkoxyethyl group, in which the alkoxy part has from 1 to 3 carbon atoms or a 2-aminoethyl group.

22. A compound according to Claim 1 or Claim 2, in which:
A represents a group of formula (A3);
ℓ is 1 or 2;
R¹ represents a hydrogen atom, a methyl group, a fluoromethyl group, a formimidoyl group or an acetimidoyl group;
R⁷ represents a hydrogen atom;
R¹¹ represents a hydrogen atom or a methyl group; and
R¹² represents a hydrogen atom, an alkyl group having from 1 to 3 carbon atoms, a fluoromethyl group, a carbamoylmethyl group, a carboxymethyl group, a formimidoyl group, an acetimidoyl group, a 2-fluoroethyl group or a 2-hydroxyethyl group.

23. A compound according to Claim 1 or Claim 2, in which A represents a group of formula (A4), and R¹⁴ and R¹⁵ each represents a hydrogen atom or an alkyl group having from 1 to 3 carbon atoms.

24. A compound according to Claim 1 or Claim 2, in which A represents a group of formula (A4), and R¹⁶ represents: a hydrogen atom; an alkyl group having from 1 to 3 carbon atoms; a substituted alkyl group having from 1 to 3 carbon atoms, in which the substituent is selected from alkoxy groups having from 1 to 3 carbon atoms and halogen atoms; a cycloalkyl group having from 3 to 6 carbon atoms; or an alkyl group having from 1 to 3 carbon atoms, which is substituted by a single cycloalkyl group having from 3 to 6 carbon atoms.

25. A compound according to Claim 1 or Claim 2, in which:
A represents a group of formula (A4);
i is 1 or 2; and
R¹, R¹⁴, R¹⁵ and R¹⁶ are the same or different and each represents a hydrogen atom or an alkyl group having from 1 to 3 carbon atoms.

26. A compound according to Claim 1 or Claim 2, in which:
A represents a group of formula (A4);
i is 1; and
R¹, R¹⁴, R¹⁵ and R¹⁶ are the same or different and each represents a hydrogen atom or a methyl group.

27. A compound according to Claim 1 or Claim 2, in which A represents a group of formula (A5), and R¹⁷ and R¹⁸ each represents: a hydrogen atom; an alkyl group having from 1 to 3 carbon atoms; or a substituted alkyl group having from 1 to 3 carbon atoms, in which the substituent is selected from hydroxy groups, alkoxy groups having from 1 to 3 carbon atoms and halogen atoms.

28. A compound according to Claim 1 or Claim 2, in which A represents a group of formula (A5), and R¹⁷ and R¹⁸ together represent a group of formula -(CH₂)_{q}-Y-(CH₂)ᵣ-, in which Y represents a carbon-carbon single bond, an oxygen atom or a group of formula >NR²³, in which R²³ represents a hydrogen atom or an alkyl group having from 1 to 3 carbon atoms, and q and r are each 2 or 3.

29. A compound according to Claim 1 or Claim 2, in which:
A represents a group of formula (A5);
p is 2;
R¹ represents a hydrogen atom, a methyl group, a fluoromethyl group, a formimidoyl group or an acetimidoyl group;
R¹⁷ and R¹⁸ are the same or different and each represents a hydrogen atom, an alkyl group having from 1 to 3 carbon atoms, a 2-haloethyl group or a 2-hydroxyethyl group;
or
R¹⁷ and R¹⁸ together represent a group of formula
-(CH₂)₄-,
-(CH₂)₅-,
-(CH₂)₂O(CH₂)₂-,
-(CH₂)₂S(CH₂)₂,
-(CH₂)₂NH(CH₂)₂- or
-(CH₂)₂NCH₃(CH₂)₂-.

30. A compound according to Claim 1 or Claim 2, in which:
A represents a group of formula (A5);
p is 2;
R¹ represents a hydrogen atom, a methyl group, a fluoromethyl group, a formimidoyl group or an acetimidoyl group; and
R¹⁷ and R¹⁸ are the same or different and each represents a hydrogen atom or a methyl group.

31. A compound according to Claim 1 or Claim 2, in which A represents a group of formula (A6), and R¹⁹ represents a hydrogen atom or an alkyl group having from 1 to 3 carbon atoms.

32. A compound according to Claim 1 or Claim 2, in which:
A represents a group of formula (A6);
j and k are both 2;
R¹ represents a hydrogen atom, a methyl group, a fluoromethyl group, a formimidoyl group or an acetimidoyl group; and
R¹⁹ represents a hydrogen atom or an alkyl group having from 1 to 3 carbon atoms.

33. A compound according to Claim 1 or Claim 2, in which:
A represents a group of formula (A6);
j and k are both 2; and
R¹ and R¹⁹ are the same or different and each represents a hydrogen atom or a methyl group.

34. A compound according to Claim 1 or Claim 2, in which A represents a group of formula (A7), and Z represents a 1-imidazolyl group, a 1,2,4-triazol-1-yl group or a 1,2,3-triazol-1-yl group.

35. A compound according to Claim 1 or Claim 2, in which:
A represents a group of formula (A7);
g is 0, 1 or 2;
R¹ represents a hydrogen atom or a methyl group; and
Z represents a 1-imidazolyl group, a 1,2,4-triazol-1-yl group or a 1,2,3-triazol-1-yl group.

36. A compound according to Claim 1 or Claim 2, in which:
A represents a group of formula (A7);
g is 1 or 2;
R¹ represents a hydrogen atom or a methyl group; and
Z represents a 1-imidazolyl group, a 1,2,4-triazol-1-yl group or a 1,2,3-triazol-1-yl group.

37. A compound according to Claim 1 or Claim 2, in which A represents a group of formula (A8), and R²⁰ and R²¹ each represents a hydrogen atom or an alkyl group having from 1 to 3 carbon atoms.

38. A compound according to Claim 1 or Claim 2, in which:
A represents a group of formula (A8);
e and f are both 1;
R¹ represents a hydrogen atom, a methyl group, a fluoromethyl group, a formimidoyl group or an acetimidoyl group; and
R²⁰ and R²¹ each represents a hydrogen atom or an alkyl group having from 1 to 3 carbon atoms.

39. A compound according to Claim 1 or Claim 2, in which:
A represents a group of formula (A8);
e and f are both 1;
R¹ represents a hydrogen atom or a methyl group;
R²⁰ represents a hydrogen atom; and
R²¹ represents a hydrogen atom or a methyl group.

40. A compound according to any one of the preceding Claims, in which the carbon atoms are in the same configurations as those of thienamycin.

41. The following compounds according to Claim 1:
2-[2-(1-homopiperazinylcarbonyl)-1-methylpyrrolidin-4-ylthio]-6-(1-hydroxyethyl)-1-methyl-1-carbapen-2-em-3-carboxylic acid;
2-[2-(4-carboxymethylhomopiperazin-1-ylcarbonyl)pyrrolidin-4-ylthio]-6-(1-hydroxyethyl)-1-methyl-1-carbapen-2-em-3-carboxylic acid;
2-{2-[4-(2-hydroxyethyl)homopiperazin-1-ylcarbonyl] pyrrolidin-4-ylthio}-6-(1-hydroxyethyl)-1-methyl-1-carbapen-2-em-3-carboxylic acid;
2-[2-(4-acetimidoylhomopiperazin-1-ylcarbonyl)pyrrolidin-4-ylthio]-6-(1-hydroxyethyl)-1-methyl-1-carbapen-2-em-3-carboxylic acid;
2-[2-(4-formimidoylhomopiperazin-1-ylcarbonyl)pyrrolidin-4-ylthio]-6-(1-hydroxyethyl)-1-methyl-1-carbapen-2-em-3-carboxylic acid;
2-[2-(4-formimidoylhomopiperazin-1-ylcarbonyl)-1-methylpyrrolidin-4-ylthio]-6-(1-hydroxyethyl)-1-methyl-1-carbapen-2-em-3-carboxylic acid;
2-[1-methyl-2-(piperazin-1-ylcarbonyl)pyrrolidin-4-ylthio]-6-(1-hydroxyethyl)-1-methyl-1-carbapen-2-em-3-carboxylic acid;
2-{2-[4-(2-hydroxyethyl)piperazin-1-ylcarbonyl]-pyrrolidin-4-ylthio}-6-(1-hydroxyethyl)-1-methyl-1-carbapen-2-em-3-carboxylic acid;
2-[2-(3-methylpiperazin-1-ylcarbonyl)pyrrolidin-4-ylthio]-6-(1-hydroxyethyl)-1-methyl-1-carbapen-2-em-3-carboxylic acid;
2-[2-(4-formimidoylpiperazin-1-ylcarbonyl)pyrrolidin-4-ylthio]-6-(1-hydroxyethyl)-1-methyl-1-carbapen-2-em-3-carboxylic acid;
2-[2-(4-acetimidoylpiperazin-1-ylcarbonyl)pyrrolidin-4-ylthio]-6-(1-hydroxyethyl)-1-methyl-1-carbapen-2-em-3-carboxylic acid;
2-[2-(4-formimidoylpiperazin-1-ylcarbonyl)-1-methylpyrrolidin-4-ylthio]-6-(1-hydroxyethyl)-1-methyl-1-carbapen-2-em-3-carboxylic acid;
2-[2-(4-acetimidoylpiperazin-1-ylcarbonyl)-1-methylpyrrolidin-4-ylthio]-6-(1-hydroxyethyl)-1-methyl-1-carbapen-2-em-3-carboxylic acid;
2-[2-(4-formimidoyl-3-methylpiperazin-1-ylcarbonyl)-pyrrolidin-4-ylthio]-6-(1-hydroxyethyl)-1-methyl-1-carbapen-2-em-3-carboxylic acid;
2-[2-(4-acetimidoyl-3-methylpiperazin-1-ylcarbonyl)pyrrolidin-4-ylthio]-6-(1-hydroxyethyl)-1-methyl-1-carbapen-2-em-3-carboxylic acid;
2-[2-(2-methylpiperazin-1-ylcarbonyl)pyrrolidin-4-ylthio]-6-(1-hydroxyethyl)-1-methyl-1-carbapen-2-em-3-carboxylic acid;
2-[2-(4-formimidoyl-2-methylpiperazin-1-ylcarbonyl)-pyrrolidin-4-ylthio]-6-(1-hydroxyethyl)-1-methyl-1-carbapen-2-em-3-carboxylic acid;
2-[2-(4-acetimidoyl-2-methylpiperazin-1-ylcarbonyl)pyrrolidin-4-ylthio]-6-(1-hydroxyethyl)-1-methyl-1-carbapen-2-em-3-carboxylic acid;
2-[2-(3-hydroxymethylpiperazin-1-ylcarbonyl)pyrrolidin-4-ylthio]-6-(1-hydroxyethyl)-1-methyl-1-carbapen-2-em-3-carboxylic acid;
2-[1-formimidoyl-2-(4-formimidoylpiperazin-1-ylcarbonyl)pyrrolidin-4-ylthio]-6-(1-hydroxyethyl)-1-methyl-1-carbapen-2-em-3-carboxylic acid;
2-[2-(3-acetimidoylaminopyrrolidin-1-ylcarbonyl)pyrrolidin-4-ylthio]-6-(1-hydroxyethyl)-1-methyl-1-carbapen-2-em-3-carboxylic acid;
2-[2-(3-formimidoylaminopyrrolidin-1-ylcarbonyl)pyrrolidin-4-ylthio]-6-(1-hydroxyethyl)-1-methyl-1-carbapen-2-em-3-carboxylic acid;
2-[2-(3-aminopyrrolidin-1-ylcarbonyl)pyrrolidin-4-ylthio]-6-(1-hydroxyethyl)-1-methyl-1-carbapen-2-em-3-carboxylic acid;
2-[2-(4-acetimidoylaminopiperidin-1-ylcarbonyl)pyrrolidin-4-ylthio]-6-(1-hydroxyethyl)-1-methyl-1-carbapen-2-em-3-carboxylic acid;
2-[2-(3-aminopyrrolidin-1-ylcarbonyl)-1-methylpyrrolidin-4-ylthio]-6-(1-hydroxyethyl)-1-methyl-1-carbapen-2-em-3-carboxylic acid;
2-[2-(3-acetimidoylaminopyrrolidin-1-ylcarbonyl)-1-methylpyrrolidin-4-ylthio]-6-(1-hydroxyethyl)-1-methyl-1-carbapen-2-em-3-carboxylic acid;
2-[2-(3-formimidoylaminopyrrolidin-1-ylcarbonyl)-1-methylpyrrolidin-4-ylthio]-6-(1-hydroxyethyl)-1-methyl-1-carbapen-2-em-3-carboxylic acid;
2-[2-(4-acetimidoylaminopiperidin-1-ylcarbonyl)-1-methylpyrrolidin-4-ylthio]-6-(1-hydroxyethyl)-1-methyl-1-carbapen-2-em-3-carboxylic acid;
2-[2-(1-formimidoylpyrrolidin-3-ylcarbamoyl)pyrrolidin-4-ylthio]-6-(1-hydroxyethyl)-1-methyl-1-carbapen-2-em-3-carboxylic acid;
2-[2-(3-dimethylamino-1,2,5,6-tetrahydropyrazin-1-ylcarbonyl)pyrrolidin-4-ylthio]-6-(1-hydroxyethyl)-1-methyl-1-carbapen-2-em-3-carboxylic acid;
and pharmaceutically acceptable salts and esters thereof.

42. A pharmaceutical composition comprising a pharmaceutically acceptable carrier, diluent or adjuvant in admixture with an effective amount of an antibiotic, in which the antibiotic is selected from compounds of formula (I) and pharmaceutically acceptable salts and esters thereof, as claimed in any one of Claims 1 to 41.

43. A process for preparing a compound according to any one of Claims 1 to 41, which comprises the steps:
reacting a compound of formula (II): 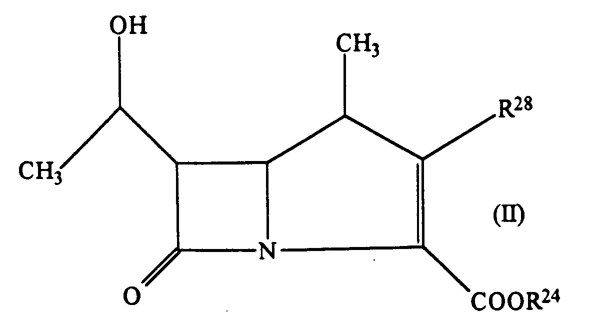 [in which R²⁴ represents a carboxy-protecting group, and R²⁸ represents an alkanesulphonyloxy group, an arylsulphonyloxy group, a dialkylphosphoryloxy group, a diarylphosphoryloxy group or a group of formula -S(→O)R²⁷, where R²⁷ represents an alkyl group, a haloalkyl group, an acetamidoalkyl group, an acetamidoalkenyl group, an aryl group, or an aromatic heterocyclic group] with a compound of formula (III): 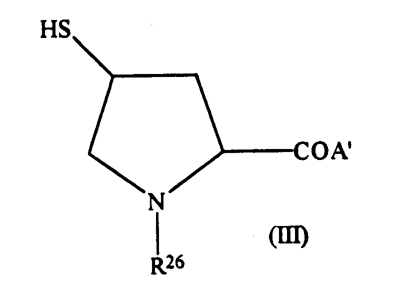 (in which R²⁶ represents any of the groups or atoms represented by R¹ or any such group or atom in which any active group is protected, and A' represents any of the groups or atoms represented by A or any such group or atom in which any active group is protected) and if necessary removing any protecting group.

44. The use of a compound according to any one of Claims 1 to 41 for the manufacture of a medicament for the treatment or prophylaxis of bacterial infections.

## Claims (Claims for the following Contracting State(s): ES, GR)

1. A process for preparing a compound of formula (I): 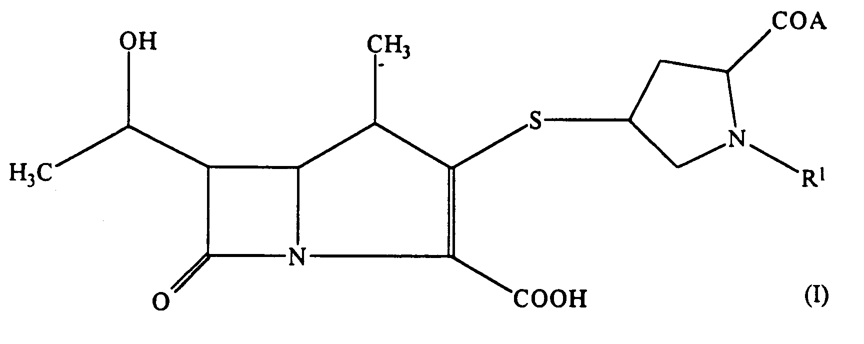 [in which:
R¹ represents:
a hydrogen atom,
an unsubstituted alkyl group having from 1 to 6 carbon atoms,
a substituted alkyl group which has from 1 to 6 carbon atoms and which is substituted by at least one of substituents (a), defined below,
an alkenyl group having from 2 to 6 carbon atoms,
an alkynyl group having from 2 to 6 carbon atoms, or a group of formula -C(=NH)R°, where R° represents a hydrogen atom or an alkyl group having from 1 to 6 carbon atoms; and
A represents a group of formula (A1), (A2), (A3), (A4), (A5), (A6), (A7) or (A8): 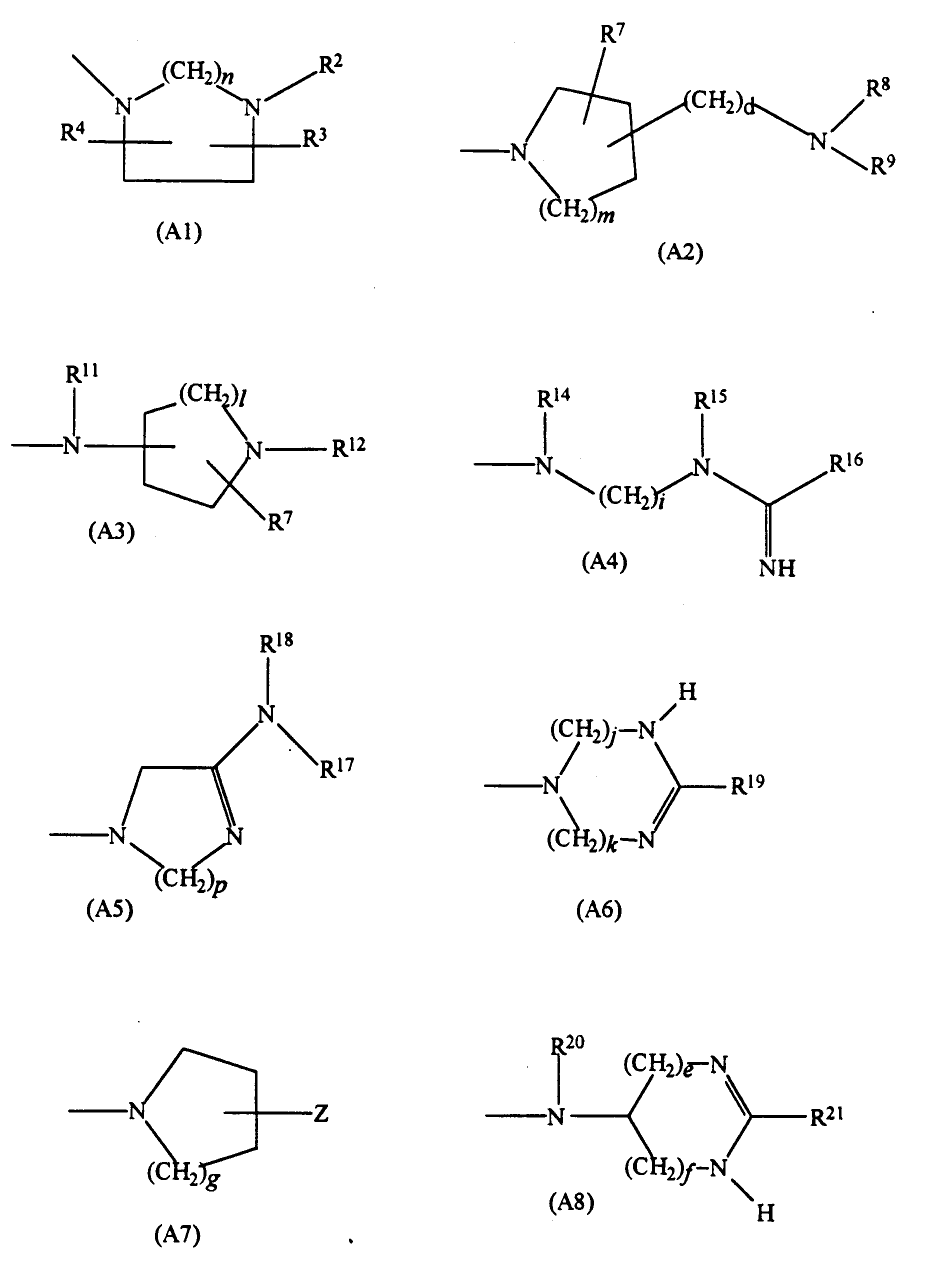 in which:
R² represents**:**
a hydrogen atom,
an unsubstituted alkyl group having from 1 to 6 carbon atoms,
a substituted alkyl group which has from 1 to 6 carbon atoms and which is substituted by at least one of substituents (b), defined below,
an alkenyl group having from 2 to 6 carbon atoms,
an alkynyl group having from 2 to 6 carbon atoms, or
a group of formula -C(=NH)R⁶,
where R⁶ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
R³, R⁴ and R⁷ are the same or different and each represents:
a hydrogen atom,
an unsubstituted alkyl group having from 1 to 6 carbon atoms,
a substituted alkyl group which has from 1 to 6 carbon atoms and which is substituted by at least one of substituents (d), defined below,
a halogen atom,
a hydroxy group,
a carboxy group,
a group of formula -CO.NR^{a}R^{b}, -OCO.NR^{a}R^{b} or -NR^{a}R^{b},
in which R^{a} and R^{b} are the same or different and each represents a hydrogen atom or an alkyl group having from 1 to 4 carbon atoms, or
a cyano group;
R⁸ represents:
a hydrogen atom,
an unsubstituted alkyl group having from 1 to 6 carbon atoms,
a substituted alkyl group which has from 1 to 6 carbon atoms and which is substituted by at least one of substituents (a), defined below,
an alkenyl group having from 2 to 6 carbon atoms, or
an alkynyl group having from 2 to 6 carbon atoms;
R⁹ represents:
a hydrogen atom,
an unsubstituted alkyl group having from 1 to 6 carbon atoms,
a substituted alkyl group which has from 1 to 6 carbon atoms and which is substituted by at least one of substituents (a), defined below, or
a group of formula -C(=NH)R¹⁰,
where R¹⁰ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
or
R⁸ and R⁹ together represent a group of formula -(CH₂)ₛ-W-(CH₂)ₜ-
in which W represents a carbon-carbon single bond, an oxygen atom, a sulphur atom or a group of formula >NR²², in which R²² represents a hydrogen atom or an alkyl group having from 1 to 6 carbon atoms, and
s and t are the same or different and each is 1, 2 or 3;
R¹¹ represents a hydrogen atom or an alkyl group having from 1 to 6 carbon atoms;
R¹² represents:
a hydrogen atom,
an unsubstituted alkyl group having from 1 to 6 carbon atoms,
a substituted alkyl group which has from 1 to 6 carbon atoms and which is substituted by at least one of substituents (a), defined below,
an alkenyl group having from 2 to 6 carbon atoms,
an alkynyl group having from 2 to 6 carbon atoms, or
a group of formula -C(=NH)R¹³,
where R¹³ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
R¹⁴ and R¹⁵ are the same or different and each represents a hydrogen atom or an alkyl group having from 1 to 6 carbon atoms;
R¹⁶ represents a hydrogen atom, an unsubstituted alkyl group having from 1 to 6 carbon atoms, a substituted alkyl group which has from 1 to 6 carbon atoms and which is substituted by at least one of substituents (c), defined below, or a cycloalkyl group having from 3 to 7 ring carbon atoms;
R¹⁷ and R¹⁸ are the same or different and each represents:
a hydrogen atom,
an unsubstituted alkyl group having from 1 to 6 carbon atoms, or
a substituted alkyl group which has from 1 to 6 carbon atoms and which is substituted by at least one of substituents (a), defined below;
or
R¹⁷ and R¹⁸ together represent a group of formula -(CH₂)_{q}-Y-(CH₂)ᵣ-
in which Y represents a carbon-carbon single bond, an oxygen atom, a sulphur atom or a group of formula >NR²³, in which R²³ represents a hydrogen atom or an alkyl group having from 1 to 6 carbon atoms, and
q and r are the same or different and each is 1, 2 or 3;
R¹⁹, R²⁰ and R²¹ are the same or different and each represents a hydrogen atom or an alkyl group having from 1 to 6 carbon atoms;
Z represents an imidazolyl, triazolyl or tetrazolyl group;
d is 0 or 1;
e, f, i, j and k are the same or different and each is 1 or 2;
q, ℓ and m are the same or different and each is 0, 1 or 2; and
n and p are the same or different and each is 1, 2 or 3;
PROVIDED THAT, where A represents a group of formula (A1):
R², R³ and R⁴ do not all represent hydrogen atoms when R¹ represents a hydrogen atom; and
R¹, R³ and R⁴ do not all represent hydrogen atoms when R² represents an alkyl group;
said substituents (a) are selected from:
hydroxy groups,
carboxy groups,
cyano groups,
halogen atoms,
oxygen atoms to form an oxo group,
alkoxy groups having from 1 to 6 carbon atoms, and
groups of formula -CO.NR^{a}R^{b}, -OCO.NR^{a}R^{b} and -NR^{a}R^{b}, in which R^{a} and R^{b} are as defined above;
said substituents (b) are selected from:
hydroxy groups,
carboxy groups,
cyano groups,
halogen atoms,
alkoxy groups having from 1 to 6 carbon atoms,
groups of formula -CO.NR^{a}R^{b}, -OCO.NR^{a}R^{b} and -NR^{a}R^{b}, in which R^{a} and R^{b} are as defined above,
sulphamoyl groups,
ureido groups,
sulpho groups,
alkanoyl groups having from 1 to 6 carbon atoms,
alkanoylamino groups having from 1 to 6 carbon atoms,
alkanoyloxy groups having from 1 to 6 carbon atoms,
alkylthio groups having from 1 to 6 carbon atoms,
alkylsulphinyl groups having from 1 to 6 carbon atoms, and
alkylsulphonyl groups having from 1 to 6 carbon atoms;
said substituents (c) are selected from:
halogen atoms,
alkoxy groups having from 1 to 6 carbon atoms,
cycloalkyl groups having from 3 to 7 ring carbon atoms; and
said substituents (d) are selected from:
hydroxy groups,
cyano groups,
groups of formula -CO.NR^{a}R^{b}, -OCO.R^{a}R^{b} and -NR^{a}R^{b}, in which R^{a} and R^{b} are as defined above,
carboxy groups,
halogen atoms, and
alkoxy groups having from 1 to 6 carbon atoms];
or a pharmaceutically acceptable salt or ester thereof, which process comprises the steps:
reacting a compound of formula (II): 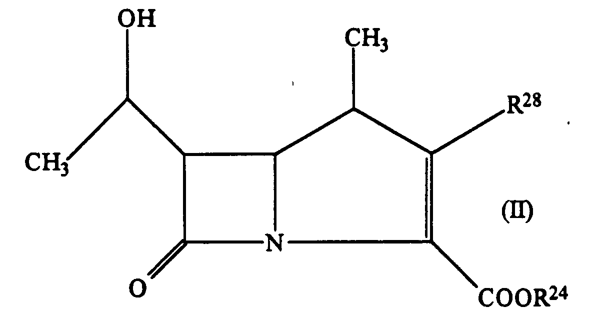 [in which R²⁴ represents a carboxy-protecting group, and R²⁸ represents an alkanesulphonyloxy group, an arylsulphonyloxy group, a dialkylphosphoryloxy group, a diarylphosphoryloxy group or a group of formula -S(→O)R²⁷, where R²⁷ represents an alkyl group, a haloalkyl group, an acetamidoalkyl group, an acetamidoalkenyl group, an aryl group, or an aromatic heterocyclic group] with a compound of formula (III): 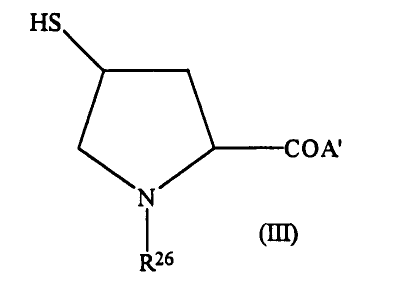 (in which R²⁶ represents any of the groups or atoms represented by R¹ or any such group or atom in which any active group is protected, and A' represents any of the groups or atoms represented by A or any such group or atom in which any active group is protected) and
if necessary removing any protecting group.

2. A process according to Claim 1, in which the reagents and reaction conditions are so chosen as to prepare a compound of formula (I) or a salt or ester thereof which has the formula (Ia): 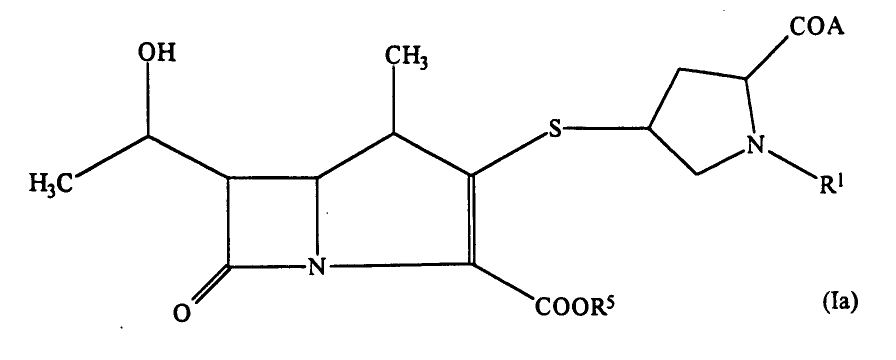 in which R¹ and A are as defined in Claim 1, and R⁵ represents:
a C₁ - C₂₀ alkyl group;
a C₃ - C₇ cycloalkyl group,
an aralkyl group, in which the alkyl part is a C₁ - C₃ alkyl group and the aryl part is a C₆ - C₁₄ carbocyclic aromatic group which may be substituted or unsubstituted and, if substituted, has at least one of substituents (e) defined below, an alkenyl group, which is substituted or unsubstituted and, if substituted, has at least one of substituents (a) defined in Claim 1;
a halogenated C₁ - C₆ alkyl group,
a substituted silylalkyl groups, in which the alkyl part has from 1 to 6 carbon atoms, and the silyl group has up to 3 substituents selected from C₁ - C₆ alkyl groups and phenyl groups which are unsubstituted or have at least one of substituents (e) defined below;
a phenyl group, which is unsubstituted or has at least one of substituents (e) defined below;
a phenacyl group, which is unsubstituted or has at least one of substituents (e) defined below;
a cyclic or acyclic terpenyl group;
an alkoxymethyl group, in which the alkoxy part is C₁ - C₆;
an aliphatic acyloxyalkyl group, in which the acyl group is a C₂ - C₆ alkanoyl group, and the alkyl part is a C₂ - C₆ alkyl group;
a cycloalkyl-substituted aliphatic acyloxyalkyl group, in which the acyl group is a C₂ - C₆ alkanoyl group, the cycloalkyl substituent is C₃ - C₇, and the alkyl part is a C₁ - C₆ alkyl group;
an alkoxycarbonyloxyalkyl group, in which the alkoxy part is C₁ - C₁₀, and the alkyl part is C₁ - C₆;
a cycloalkylcarbonyloxyalkyl or cycloalkyloxycarbonyloxyalkyl group, in which the cycloalkyl group is C₃ - C₁₀, is mono- or poly- cyclic and is unsubstituted or is substituted by at least one C₁ - C₄ alkyl group, and the alkyl group is a C₁ - C₆;
a cycloalkylalkoxycarbonyloxyalkyl group, in which the alkoxy group has a single cycloalkyl substituent, the cycloalkyl substituent being C₃ - C₁₀ and mono- or poly- cyclic;
a terpenylcarbonyloxyalkyl or terpenyloxycarbonyloxyalkyl group, in which the alkyl group has from 1 to 6 carbon atoms;
a 5-alkyl or 5-phenyl (2-oxo-1,3-dioxolen-4-yl)alkyl group in which each alkyl group is C₁ - C₆; or a phthalidyl, indanyl or 2-oxo-4,5,6,7-tetrahydro-1,3-benzodioxolen-4-yl group; and
substituents (e) are selected from C₁ - C₄ alkyl groups, C₁ - C₄ alkoxy groups, C₁ - C₄ haloalkyl groups, C₁ -C₃ alkylenedioxy groups, halogen atoms, cyano groups and nitro groups.

3. A process according to Claim 2, in which the reagents and reaction conditions are so chosen as to prepare a compound of formula (I) or a salt or ester thereof, in which R⁵ represents a hydrogen atom, a (5-substituted 2-oxo-1,3-dioxolen-4-yl)methyl group, a 1-methylcyclohexylcarbonyloxymethyl group, a 1-isopropoxycarbonyloxyethyl group or a 1-cyclohexylcarbonyloxyethyl group.

4. A process according to any one of Claims 1 to 3, in which the reagents and reaction conditions are so chosen as to prepare a compound of formula (I) or a salt or ester thereof, in which R¹ represents: a hydrogen atom; an alkyl group having from 1 to 3 carbon atoms; a substituted alkyl group having from 1 to 3 carbon atoms, in which the substituent is selected from substituents (a'), defined below; an alkenyl group having 3 or 4 carbon atoms; an alkynyl group having 3 or 4 carbon atoms; a formimidoyl group; or an acetimidoyl group; and
substituents (a′) are selected from hydroxy groups, carboxy groups, carbamoyl groups, carbamoyloxy groups, cyano groups, halogen atoms, alkoxy groups having from 1 to 3 carbon atoms, amino groups, and mono- and dialkylamino groups in which the or each alkyl group has from 1 to 3 carbon atoms.

5. A process according to any one of Claims 1 to 4, in which the reagents and reaction conditions are so chosen as to prepare a compound of formula (I) or a salt or ester thereof, in which A represents a group of formula (A1), and n is 2 or 3.

6. A process according to any one of Claims 1 to 5, in which the reagents and reaction conditions are so chosen as to prepare a compound of formula (I) or a salt or ester thereof, in which R² represents:
a hydrogen atom;
an alkyl group having from 1 to 3 carbon atoms;
a substituted alkyl group having from 1 to 3 carbon atoms, in which the substituent is selected from substituents (b'), defined below;
an alkenyl group having 3 or 4 carbon atoms;
an alkynyl group having 3 or 4 carbon atoms; or
a group of formula -C(=NH)R⁶,
where R⁶ represents
a hydrogen atom,
an unsubstituted alkyl group having from 1 to 3 carbon atoms,
a substituted alkyl group which has from 1 to 3 carbon atoms and which is substituted by at least one substituent selected from halogen atoms,
alkoxy groups having from 1 to 3 carbon atoms and cycloalkyl groups having from 3 to 6 carbon atoms, or
a cycloalkyl group having from 3 to 6 ring carbon atoms; and
substituents (b') are selected from: hydroxy groups, carboxy groups, carbamoyl groups, carbamoyloxy groups, cyano groups, sulphamoyl groups, ureido groups, sulpho groups, alkoxy groups having from 1 to 3 carbon atoms, alkoxycarbonyl groups having from 2 to 4 carbon atoms, alkanoyl groups having from 2 to 4 carbon atoms, alkanoylamino groups having from 2 to 4 carbon atoms, alkanoyloxy groups having from 2 to 4 carbon atoms, amino groups, mono- and di- alkylamino groups in which the or each alkyl group has from 1 to 3 carbon atoms, alkylthio groups having from 1 to 3 carbon atoms, alkylsulphinyl groups having from 1 to 3 carbon atoms, alkylsulphonyl groups having from 1 to 3 carbon atoms, mono- and di- alkylcarbamoyl groups in which the or each alkyl group has from 1 to 3 carbon atoms, and mono- and di- alkylcarbamoyloxy groups in which the or each alkyl group has from 1 to 3 carbon atoms.

7. A process according to any one of Claims 1 to 6, in which the reagents and reaction conditions are so chosen as to prepare a compound of formula (I) or a salt or ester thereof, in which A represents a group of formula (A1), and R³ and R⁴ each represents a hydrogen atom, an alkyl group having from 1 to 3 carbon atoms, a hydroxy group, a carboxy group, a carbamoyl group or a substituted alkyl group which has from 1 to 3 carbon atoms and which is substituted by at least one substituent selected from hydroxy groups, alkoxy groups having from 1 to 3 carbon atoms, amino groups, carbamoyl groups and halogen atoms.

8. A process according to Claim 1 or Claim 2, in which the reagents and reaction conditions are so chosen as to prepare a compound of formula (I) or a salt or ester thereof, in which:
A represents a group of formula (A1);
n is 2;
R¹ represents a hydrogen atom, a methyl group, a fluoromethyl group, a formimidoyl group or an acetimidoyl group;
R² represents a hydrogen atom, a 2-hydroxyethyl group, a 2-carbamoylethyl group, a carboxymethyl group, a carbamoylmethyl group, a 2-fluoroethyl group, a formimidoyl group or an acetimidoyl group; and
R³ and R⁴ are the same or different and each represents a hydrogen atom, a methyl group, a carbamoyl group, a cyano group, a carboxy group, a hydroxymethyl group, a fluoromethyl group or an aminomethyl group.

9. A process according to Claim 1 or Claim 2, in which the reagents and reaction conditions are so chosen as to prepare a compound of formula (I) or a salt or ester thereof, in which:
A represents a group of formula (A1);
n is 3;
R¹ represents a hydrogen atom, a methyl group, a fluoromethyl group, a formimidoyl group or an acetimidoyl group;
R² represents a hydrogen atom, a methyl group, a formimidoyl group, an acetimidoyl group, a carboxymethyl group, a carbamoylmethyl group, a 2-hydroxyethyl group or a 2-fluoroethyl group; and
R³ and R⁴ are the same or different and each represents a hydrogen atom, a methyl group, a hydroxy group, an amino group, a cyano group, a carboxy group, a carbamoyl group, a carbamoyloxy group, a hydroxymethyl group, a fluoromethyl group or an aminomethyl group.

10. A process according to Claim 1 or Claim 2, in which the reagents and reaction conditions are so chosen as to prepare a compound of formula (I) or a salt or ester thereof, in which:
A represents a group of formula (A1);
n is 2;
R¹ represents a hydrogen atom, a methyl group, a formimidoyl group or an acetimidoyl group;
R² represents a hydrogen atom, a 2-hydroxyethyl group, a carboxymethyl group, a formimidoyl group or an acetimidoyl group;
R³ represents a hydrogen atom; and
R⁴ represents a methyl group, a carbamoyl group, a cyano group, a hydroxymethyl group, a fluoromethyl group or an aminomethyl group.

11. A process according to Claim 1 or Claim 2, in which the reagents and reaction conditions are so chosen as to prepare a compound of formula (I) or a salt or ester thereof, in which:
A represents a group of formula (A1);
n is 3;
R¹ represents a hydrogen atom, a methyl group, a formimidoyl group or an acetimidoyl group;
R² represents a formimidoyl group, an acetimidoyl group, a carboxymethyl group, a 2-hydroxyethyl group or a 2-fluoroethyl group; and
R³ and R⁴ are the same or different and each represents a hydrogen atom, a hydroxy group, an amino group or a cyano group.

12. A process according to Claim 1 or Claim 2, in which the reagents and reaction conditions are so chosen as to prepare a compound of formula (I) or a salt or ester thereof, in which:
A represents a group of formula (A2), and R⁷ represents: a hydrogen atom; a carboxy group; a carbamoyl group; an alkyl group having from 1 to 3 carbon atoms; or a substituted alkyl group having from 1 to 3 carbon atoms, in which the substituent is selected from hydroxy groups, alkoxy groups having from 1 to 3 carbon atoms, carbamoyl groups, carboxy groups and cyano groups.

13. A process according to Claim 1, in which the reagents and reaction conditions are so chosen as to prepare a compound of formula (I) or a salt or ester thereof, in which A represents a group of formula (A2), and R⁸ represents: a hydrogen atom; an alkyl group having from 1 to 3 carbon atoms; a substituted alkyl group having from 1 to 3 carbon atoms, in which the substituent is selected from hydroxy groups, alkoxy groups having from 1 to 3 carbon atoms, carbamoyl groups, carbamoyloxy groups, carboxy groups, cyano groups, amino groups and halogen atoms; an alkenyl group having 3 or 4 carbon atoms; or an alkynyl group having 3 or 4 carbon atoms.

14. A process according to Claim 1 or Claim 2, in which the reagents and reaction conditions are so chosen as to prepare a compound of formula (I) or a salt or ester thereof, in which A represents a group of formula (A2), and R⁹ represents: a hydrogen atom; an alkyl group having from 1 to 3 carbon atoms; a substituted alkyl group having from 1 to 3 carbon atoms, in which the substituent is selected from hydroxy groups, alkoxy groups having from 1 to 3 carbon atoms, carbamoyl groups, carbamoyloxy groups, carboxy groups, cyano groups, amino groups and halogen atoms; or a group of formula -C(=NH)R¹⁰, in which R¹⁰ represents:
a hydrogen atom;
an alkyl group having from 1 to 3 carbon atoms;
a substituted alkyl group having from 1 to 3 carbon atoms, in which the substituent is selected from alkoxy groups having from 1 to 3 carbon atoms and halogen atoms;
a cycloalkyl group having from 3 to 6 carbon atoms; or
an alkyl group having from 1 to 3 carbon atoms, which is substituted by a single cycloalkyl group having from 3 to 6 carbon atoms.

15. A process according to Claim 1 or Claim 2, in which the reagents and reaction conditions are so chosen as to prepare a compound of formula (I) or a salt or ester thereof, in which A represents a group of formula (A2), and R⁸ and R⁹ together represent a group of formula -(CH₂)ₛ-W-(CH₂)ₜ-, in which W represents a carbon-carbon single bond, an oxygen atom, a sulphur atom or a group of formula >NR²², in which R²² represents a hydrogen atom or an alkyl group having from 1 to 3 carbon atoms, s is 1, 2 or 3 and t is 2.

16. A process according to Claim 1 or Claim 2, in which the reagents and reaction conditions are so chosen as to prepare a compound of formula (I) or a salt or ester thereof, in which:
A represents a group of formula (A2);
d is 0 or 1;
m is 0, 1 or 2;
R¹ represents a hydrogen atom, a methyl group, a fluoromethyl group, a formimidoyl group or an acetimidoyl group;
R⁷ represents a hydrogen atom, an alkyl group having from 1 to 3 carbon atoms, a hydroxy group, an amino group, a cyano group, a halogen atom, a carboxy group, a carbamoyl group or a hydroxymethyl group;
R⁸ represents a hydrogen atom, an alkyl group having from 1 to 3 carbon atoms, a fluoromethyl group, a carbamoylmethyl group, a carboxymethyl group, an alkenyl group having 3 or 4 carbon atoms, an alkynyl group having 3 or 4 carbon atoms, a 2-haloethyl group, a 2-hydroxyethyl group, a 2-alkoxyethyl group, in which the alkoxy part has from 1 to 3 carbon atoms, or a 2-aminoethyl group;
R⁹ represents a hydrogen atom, an alkyl group having from 1 to 3 carbon atoms, a fluoromethyl group, a carbamoylmethyl group, a carboxymethyl group, a formimidoyl group, an acetimidoyl group, a 2-haloethyl group, a 2-hydroxyethyl group, a 2-alkoxyethyl group, in which the alkoxy part has from 1 to 3 carbon atoms, or a 2-aminoethyl group;
or
R⁸ and R⁹ together represent a group of formula
-(CH₂)₄-,
-(CH₂)₅-,
-(CH₂)₂O(CH₂)₂-,
-(CH₂)₂S(CH₂)₂-,
-(CH₂)₂NH(CH₂)₂- or
-(CH₂)₂NCH₃(CH₂)₂-.

17. A process according to Claim 1 or Claim 2, in which the reagents and reaction conditions are so chosen as to prepare a compound of formula (I) or a salt or ester thereof, in which:
A represents a group of formula (A2);
d is 0;
m is 1 or 2;
R¹ represents a hydrogen atom, a methyl group, a fluoromethyl group, a formimidoyl group or an acetimidoyl group;
R⁷ represents a hydrogen atom;
R⁸ represents a hydrogen atom, an alkyl group having from 1 to 3 carbon atoms, a carbamoylmethyl group, a carboxymethyl group, a 2-fluoroethyl group or a 2-hydroxyethyl group; and
R⁹ represents a hydrogen atom, an alkyl group having from 1 to 3 carbon atoms, a formimidoyl group, an acetimidoyl group or a 2-fluoroethyl group.

18. A process according to Claim 1 or Claim 2, in which the reagents and reaction conditions are so chosen as to prepare a compound of formula (I) or a salt or ester thereof, in which A represents a group of formula (A3), ℓ is 0, 1 or 2, and R⁷ represents a hydrogen atom or an alkyl group having from 1 to 3 carbon atoms.

19. A process according to Claim 1 or Claim 2, in which the reagents and reaction conditions are so chosen as to prepare a compound of formula (I) or a salt or ester thereof, in which A represents a group of formula (A3), and R¹¹ represents a hydrogen atom or an alkyl group having from 1 to 3 carbon atoms, such as a methyl, ethyl or propyl group.

20. A process according to Claim 1 or Claim 2, in which the reagents and reaction conditions are so chosen as to prepare a compound of formula (I) or a salt or ester thereof, in which A represents a group of formula (A3), and R¹² represents: a hydrogen atom; an alkyl group having from 1 to 3 carbon atoms; a substituted alkyl group having from 1 to 3 carbon atoms, in which the substituent is selected from hydroxy groups, alkoxy groups having from 1 to 3 carbon atoms, carbamoyl groups, carbamoyloxy groups, carboxy groups, cyano groups, amino groups and halogen atoms; an alkenyl group having 3 or 4 carbon atoms; an alkynyl group having 3 or 4 carbon atoms; or a group of formula -C(=NH)R¹³, in which R¹³ represents:
a hydrogen atom;
an alkyl group having from 1 to 3 carbon atoms;
a substituted alkyl group having from 1 to 3 carbon atoms, in which the substituent is selected from alkoxy groups having from 1 to 3 carbon atoms and halogen atoms;
a cycloalkyl group having from 3 to 6 carbon atoms; or
an alkyl group having from 1 to 3 carbon atoms, which is substituted by a single cycloalkyl group having from 3 to 6 carbon atoms.

21. A process according to Claim 1 or Claim 2, in which the reagents and reaction conditions are so chosen as to prepare a compound of formula (I) or a salt or ester thereof, in which:
A represents a group of formula (A3);
ℓ is 0, 1 or 2;
R¹ represents a hydrogen atom, a methyl group, a fluoromethyl group, a formimidoyl group or an acetimidoyl group;
R⁷ represents a hydrogen atom;
R¹¹ represents a hydrogen atom or an alkyl group having from 1 to 3 carbon atoms; and
R¹² represents a hydrogen atom, an alkyl group having from 1 to 3 carbon atoms, a fluoromethyl group, a carbamoylmethyl group, a carboxymethyl group, an alkenyl group having 3 or 4 carbon atoms, an alkynyl group having 3 or 4 carbon atoms, a formimidoyl group, an acetimidoyl group, a 2-haloethyl group, a 2-hydroxyethyl group, a 2-alkoxyethyl group, in which the alkoxy part has from 1 to 3 carbon atoms or a 2-aminoethyl group.

22. A process according to Claim 1 or Claim 2, in which the reagents and reaction conditions are so chosen as to prepare a compound of formula (I) or a salt or ester thereof, in which:
A represents a group of formula (A3);
ℓ is 1 or 2;
R¹ represents a hydrogen atom, a methyl group, a fluoromethyl group, a formimidoyl group or an acetimidoyl group;
R⁷ represents a hydrogen atom;
R¹¹ represents a hydrogen atom or a methyl group; and
R¹² represents a hydrogen atom, an alkyl group having from 1 to 3 carbon atoms, a fluoromethyl group, a carbamoylmethyl group, a carboxymethyl group, a formimidoyl group, an acetimidoyl group, a 2-fluoroethyl group or a 2-hydroxyethyl group.

23. A process according to Claim 1 or Claim 2, in which the reagents and reaction conditions are so chosen as to prepare a compound of formula (I) or a salt or ester thereof, in which A represents a group of formula (A4), and R¹⁴ and R¹⁵ each represents a hydrogen atom or an alkyl group having from 1 to 3 carbon atoms.

24. A process according to Claim 1 or Claim 2, in which the reagents and reaction conditions are so chosen as to prepare a compound of formula (I) or a salt or ester thereof, in which A represents a group of formula (A4), and R¹⁶ represents: a hydrogen atom; an alkyl group having from 1 to 3 carbon atoms; a substituted alkyl group having from 1 to 3 carbon atoms, in which the substituent is selected from alkoxy groups having from 1 to 3 carbon atoms and halogen atoms; a cycloalkyl group having from 3 to 6 carbon atoms; or an alkyl group having from 1 to 3 carbon atoms, which is substituted by a single cycloalkyl group having from 3 to 6 carbon atoms.

25. A process according to Claim 1 or Claim 2, in which the reagents and reaction conditions are so chosen as to prepare a compound of formula (I) or a salt or ester thereof, in which:
A represents a group of formula (A4);
i is 1 or 2; and
R¹, R¹⁴, R¹⁵ and R¹⁶ are the same or different and each represents a hydrogen atom or an alkyl group having from 1 to 3 carbon atoms.

26. A process according to Claim 1 or Claim 2, in which the reagents and reaction conditions are so chosen as to prepare a compound of formula (I) or a salt or ester thereof, in which:
A represents a group of formula (A4);
i is 1; and
R¹, R¹⁴, R¹⁵ and R¹⁶ are the same or different and each represents a hydrogen atom or a methyl group.

27. A process according to Claim 1 or Claim 2, in which the reagents and reaction conditions are so chosen as to prepare a compound of formula (I) or a salt or ester thereof, in which A represents a group of formula (A5), and R¹⁷ and R¹⁸ each represents: a hydrogen atom; an alkyl group having from 1 to 3 carbon atoms; or a substituted alkyl group having from 1 to 3 carbon atoms, in which the substituent is selected from hydroxy groups, alkoxy groups having from 1 to 3 carbon atoms and halogen atoms.

28. A process according to Claim 1 or Claim 2, in which the reagents and reaction conditions are so chosen as to prepare a compound of formula (I) or a salt or ester thereof, in which A represents a group of formula (A5), and R¹⁷ and R¹⁸ together represent a group of formula -(CH₂)_{q}-Y-(CH₂)ᵣ-, in which Y represents a carbon-carbon single bond, an oxygen atom or a group of formula >NR²³, in which R²³ represents a hydrogen atom or an alkyl group having from 1 to 3 carbon atoms, and q and r are each 2 or 3.

29. A process according to Claim 1 or Claim 2, in which the reagents and reaction conditions are so chosen as to prepare a compound of formula (I) or a salt or ester thereof, in which:
A represents a group of formula (A5);
p is 2;
R¹ represents a hydrogen atom, a methyl group, a fluoromethyl group, a formimidoyl group or an acetimidoyl group;
R¹⁷ and R¹⁸ are the same or different and each represents a hydrogen atom, an alkyl group having from 1 to 3 carbon atoms, a 2-haloethyl group or a 2-hydroxyethyl group;
or
R¹⁷ and R¹⁸ together represent a group of formula
-(CH₂)₄-,
-(CH₂)₅-,
-(CH₂)₂O(CH₂)₂-,
-(CH₂)₂S(CH₂)₂-,
-(CH₂)₂NH(CH₂)₂- or
-(CH₂)₂NCH₃(CH₂)₂-.

30. A process according to Claim 1 or Claim 2, in which the reagents and reaction conditions are so chosen as to prepare a compound of formula (I) or a salt or ester thereof, in which:
A represents a group of formula (A5);
p is 2;
R¹ represents a hydrogen atom, a methyl group, a fluoromethyl group, a formimidoyl group or an acetimidoyl group; and
R¹⁷ and R¹⁸ are the same or different and each represents a hydrogen atom or a methyl group.

31. A process according to Claim 1 or Claim 2, in which the reagents and reaction conditions are so chosen as to prepare a compound of formula (I) or a salt or ester thereof, in which A represents a group of formula (A6), and R¹⁹ represents a hydrogen atom or an alkyl group having from 1 to 3 carbon atoms.

32. A process according to Claim 1 or Claim 2, in which the reagents and reaction conditions are so chosen as to prepare a compound of formula (I) or a salt or ester thereof, in which:
A represents a group of formula (A6);
j and k are both 2;
R¹ represents a hydrogen atom, a methyl group, a fluoromethyl group, a formimidoyl group or an acetimidoyl group; and
R¹⁹ represents a hydrogen atom or an alkyl group having from 1 to 3 carbon atoms.

33. A process according to Claim 1 or Claim 2, in which the reagents and reaction conditions are so chosen as to prepare a compound of formula (I) or a salt or ester thereof, in which:
A represents a group of formula (A6);
j and k are both 2; and
R¹ and R¹⁹ are the same or different and each represents a hydrogen atom or a methyl group.

34. A process according to Claim 1 or Claim 2, in which the reagents and reaction conditions are so chosen as to prepare a compound of formula (I) or a salt or ester thereof, in which A represents a group of formula (A7), and Z represents a 1-imidazolyl group, a 1,2,4-triazol-1-yl group or a 1,2,3-triazol-1-yl group.

35. A process according to Claim 1 or Claim 2, in which the reagents and reaction conditions are so chosen as to prepare a compound of formula (I) or a salt or ester thereof, in which:
A represents a group of formula (A7);
g is 0, 1 or 2;
R¹ represents a hydrogen atom or a methyl group; and
Z represents a 1-imidazolyl group, a 1,2,4-triazol-1-yl group or a 1,2,3-triazol-1-yl group.

36. A process according to Claim 1 or Claim 2, in which the reagents and reaction conditions are so chosen as to prepare a compound of formula (I) or a salt or ester thereof, in which:
A represents a group of formula (A7);
g is 1 or 2;
R¹ represents a hydrogen atom or a methyl group; and
Z represents a 1-imidazolyl group, a 1,2,4-triazol-1-yl group or a 1,2,3-triazol-1-yl group.

37. A process according to Claim 1 or Claim 2, in which the reagents and reaction conditions are so chosen as to prepare a compound of formula (I) or a salt or ester thereof, in which A represents a group of formula (A8), and R²⁰ and R²¹ each represents a hydrogen atom or an alkyl group having from 1 to 3 carbon atoms.

38. A process according to Claim 1 or Claim 2, in which the reagents and reaction conditions are so chosen as to prepare a compound of formula (I) or a salt or ester thereof, in which:
A represents a group of formula (A8);
e and f are both 1;
R¹ represents a hydrogen atom, a methyl group, a fluoromethyl group, a formimidoyl group or an acetimidoyl group; and
R²⁰ and R²¹ each represents a hydrogen atom or an alkyl group having from 1 to 3 carbon atoms.

39. A process according to Claim 1 or Claim 2, in which the reagents and reaction conditions are so chosen as to prepare a compound of formula (I) or a salt or ester thereof, in which:
A represents a group of formula (A8);
e and f are both 1;
R¹ represents a hydrogen atom or a methyl group;
R²⁰ represents a hydrogen atom; and
R²¹ represents a hydrogen atom or a methyl group.

40. A process according to any one of the preceding Claims, in which the reagents and reaction conditions are so chosen as to prepare a compound of formula (I) or a salt or ester thereof, in which the carbon atoms are in the same configurations as those of thienamycin.

41. A process according to Claim 1, in which the reagents and reaction conditions are so chosen as to prepare the following compounds:
2-[2-(1-homopiperazinylcarbonyl)-1-methylpyrrolidin-4-ylthio]-6-(1-hydroxyethyl)-1-methyl-1-carbapen-2-em-3-carboxylic acid;
2-[2-(4-carboxymethylhomopiperazin-1-ylcarbonyl)pyrrolidin-4-ylthio]-6-(1-hydroxyethyl)-1-methyl-1-carbapen-2-em-3-carboxylic acid;
2-{2-[4-(2-hydroxyethyl)homopiperazin-1-ylcarbonyl]pyrrolidin-4-ylthio}-6-(1-hydroxyethyl)-1-methyl-1-carbapen-2-em-3-carboxylic acid;
2-[2-(4-acetimidoylhomopiperazin-1-ylcarbonyl)pyrrolidin-4-ylthio]-6-(1-hydroxyethyl)-1-methyl-1-carbapen-2-em-3-carboxylic acid;
2-[2-(4-formimidoylhomopiperazin-1-ylcarbonyl)pyrrolidin-4-ylthio]-6-(1-hydroxyethyl)-1-methyl-1-carbapen-2-em-3-carboxylic acid;
2-[2-(4-formimidoylhomopiperazin-1-ylcarbonyl)-1-methyl-pyrrolidin-4-ylthio]-6-(1-hydroxyethyl)-1-methyl-1-carbapen-2-em-3-carboxylic acid;
2-[2-(1-methyl-2-piperazinylcarbonyl)pyrrolidin-4-ylthio]-6-(1-hydroxyethyl)-1-methyl-1-carbapen-2-em-3-carboxylic acid;
2-{2-[4-(2-hydroxyethyl)piperazin-1-ylcarbonyl]pyrrolidin-4-ylthio}-6-(1-hydroxyethyl)-1-methyl-1-carbapen-2-em-3-carboxylic acid;
2-[2-(3-methylpiperazin-1-ylcarbonyl)pyrrolidin-4-ylthio]-6-(1-hydroxyethyl)-1-methyl-1-carbapen-2-em-3-carboxylic acid;
2-[2-(4-formimidoylpiperazin-1-ylcarbonyl)pyrrolidin-4-ylthio]-6-(1-hydroxyethyl)-1-methyl-1-carbapen-2-em-3-carboxylic acid;
2-[2-(4-acetimidoylpiperazin-1-ylcarbonyl)pyrrolidin-4-ylthio]-6-(1-hydroxyethyl)-1-methyl-1-carbapen-2-em-3-carboxylic acid;
2-[2-(4-formimidoylpiperazin-1-ylcarbonyl)-1-methyl-pyrrolidin-4-ylthio]-6-(1-hydroxyethyl)-1-methyl-1-carbapen-2-em-3-carboxylic acid;
2-[2-(4-acetimidoylpiperazin-1-ylcarbonyl)-1-methylpyrrolidin-4-ylthio]-6-(1-hydroxyethyl)-1-methyl-1-carbapen-2-em-3-carboxylic acid;
2-[2-(4-formimidoyl-3-methylpiperazin-1-ylcarbonyl)pyrrolidin-4-ylthio]-6-(1-hydroxyethyl)-1-methyl-1-carbapen-2-em-3-carboxylic acid;
2-[2-(4-acetimidoyl-3-methylpiperazin-1-ylcarbonyl)pyrrolidin-4-ylthio]-6-(1-hydroxyethyl)-1-methyl-1-carbapen-2-em-3-carboxylic acid;
2-[2-(2-methylpiperazin-1-ylcarbonyl)pyrrolidin-4-ylthio]-6-(1-hydroxyethyl)-1-methyl-1-carbapen-2-em-3-carboxylic acid;
2-[2-(4-formimidoyl-2-methylpiperazin-1-ylcarbonyl)pyrrolidin-4-ylthio]-6-(1-hydroxyethyl)-1-methyl-1-carbapen-2-em-3-carboxylic acid;
2-[2-(4-acetimidoyl-2-methylpiperazin-1-ylcarbonyl)pyrrolidin-4-ylthio]-6-(1-hydroxyethyl)-1-methyl-1-carbapen-2-em-3-carboxylic acid;
2-[2-(3-hydroxymethylpiperazin-1-ylcarbonyl)pyrrolidin-4-ylthio]-6-(1-hydroxyethyl)-1-methyl-1-carbapen-2-em-3-carboxylic acid;
2-[1-formimidoyl-2-(4-formimidoylpiperazin-1-ylcarbonyl)pyrrolidin-4-ylthio]-6-(1-hydroxyethyl)-1-methyl-1-carbapen-2-em-3-carboxylic acid;
2-[2-(3-acetimidoylaminopyrrolidin-1-ylcarbonyl)pyrrolidin-4-ylthio]-6-(1-hydroxyethyl)-1-methyl-1-carbapen-2-em-3-carboxylic acid;
2-[2-(3-formimidoylaminopyrrolidin-1-ylcarbonyl)pyrrolidin-4-ylthio]-6-(1-hydroxyethyl)-1-methyl-1-carbapen-2-em-3-carboxylic acid;
2-[2-(3-aminopyrrolidin-1-ylcarbonyl)pyrrolidin-4-ylthio]-6-(1-hydroxyethyl)-1-methyl-1-carbapen-2-em-3-carboxylic acid;
2-[2-(4-acetimidoylaminopiperid-1-ylcarbonyl)pyrrolidin-4-ylthio]-6-(1-hydroxyethyl)-1-methyl-1-carbapen-2-em-3-carboxylic acid;
2-[2-(3-aminopyrrolidin-1-ylcarbonyl)-1-methylpyrrolidin-4-ylthio]-6-(1-hydroxyethyl)-1-methyl-1-carbapen-2-em-3-carboxylic acid;
2-[2-(3-acetimidoylaminopyrrolidin-1-ylcarbonyl)-1-methylpyrrolidin-4-ylthio]-6-(1-hydroxyethyl)-1-methyl-1-carbapen-2-em-3-carboxylic acid;
2-[2-(3-formimidoylaminopyrrolidin-1-ylcarbonyl)-1-methylpyrrolidin-4-ylthio]-6-(1-hydroxyethyl)-1-methyl-1-carbapen-2-em-3-carboxylic acid;
2-[2-(4-acetimidoylaminopiperid-1-ylcarbonyl)-1-methylpyrrolidin-4-ylthio]-6-(1-hydroxyethyl)-1-methyl-1-carbapen-2-em-3-carboxylic acid;
2-[2-(1-formimidoylpyrrolidin-3-ylcarbamoyl)pyrrolidin-4-ylthio]-6-(1-hydroxyethyl)-1-methyl-1-carbapen-2-em-3-carboxylic acid;
2-[2-(3-dimethylamino-1,2,5,6-tetrahydropyrazin-1-ylcarbonyl)pyrrolidin-4-ylthio]-6-(1-hydroxyethyl)-1-methyl-1-carbapen-2-em-3-carboxylic acid;
and pharmaceutically acceptable salts and esters thereof.

42. A process for preparing a pharmaceutical composition comprising mixing a pharmaceutically acceptable carrier, diluent or adjuvant with an antibiotic, in which the antibiotic is selected from compounds of formula (I) and pharmaceutically acceptable salts and esters thereof, as defined in any one of Claims 1 to 41.

43. The use of a compound of formula (I) or a pharmaceutically acceptable salt or ester thereof, as defined in any one of Claims 1 to 41 for the manufacture of a medicament for the treatment or prophylaxis of bacterial infections.

## Patentansprüche (Patentansprüche für folgende(n) Vertragsstaat(en): AT, BE, CH, DE, DK, FR, GB, IT, LI, LU, MC, NL, PT, SE)

1. Verbindung der Formel (I): 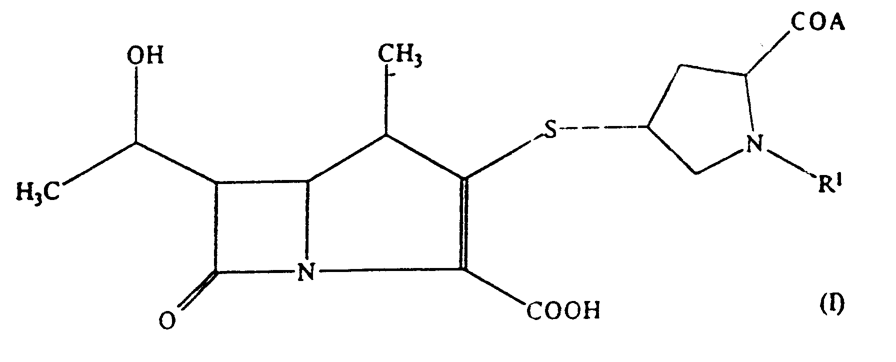 worin
R¹ bedeutet:
ein Wasserstoffatom,
eine unsubstituierte Alkylgruppe mit 1 bis 6 Kohlenstoffatomen,
eine substituierte Alkylgruppe, die 1 bis 6 Kohlenstoffatome aufweist und die mit mindestens einem der nachstehend definierten Substituenten (a) substituiert ist,
eine Alkenylgruppe mit 2 bis 6 Kohlenstoffatomen,
eine Alkinylgruppe mit 2 bis 6 Kohlenstoffatomen oder
eine Gruppe der Formel -C(=NH)R°, worin R° ein Wasserstoffatom oder eine Alkylgruppe mit 1 bis 6 Kohlenstoffatomen bedeutet, und
A eine Gruppe der Formel (A1), (A2), (A3), (A4), (A5), (A6), (A7) oder (A8) bedeutet: 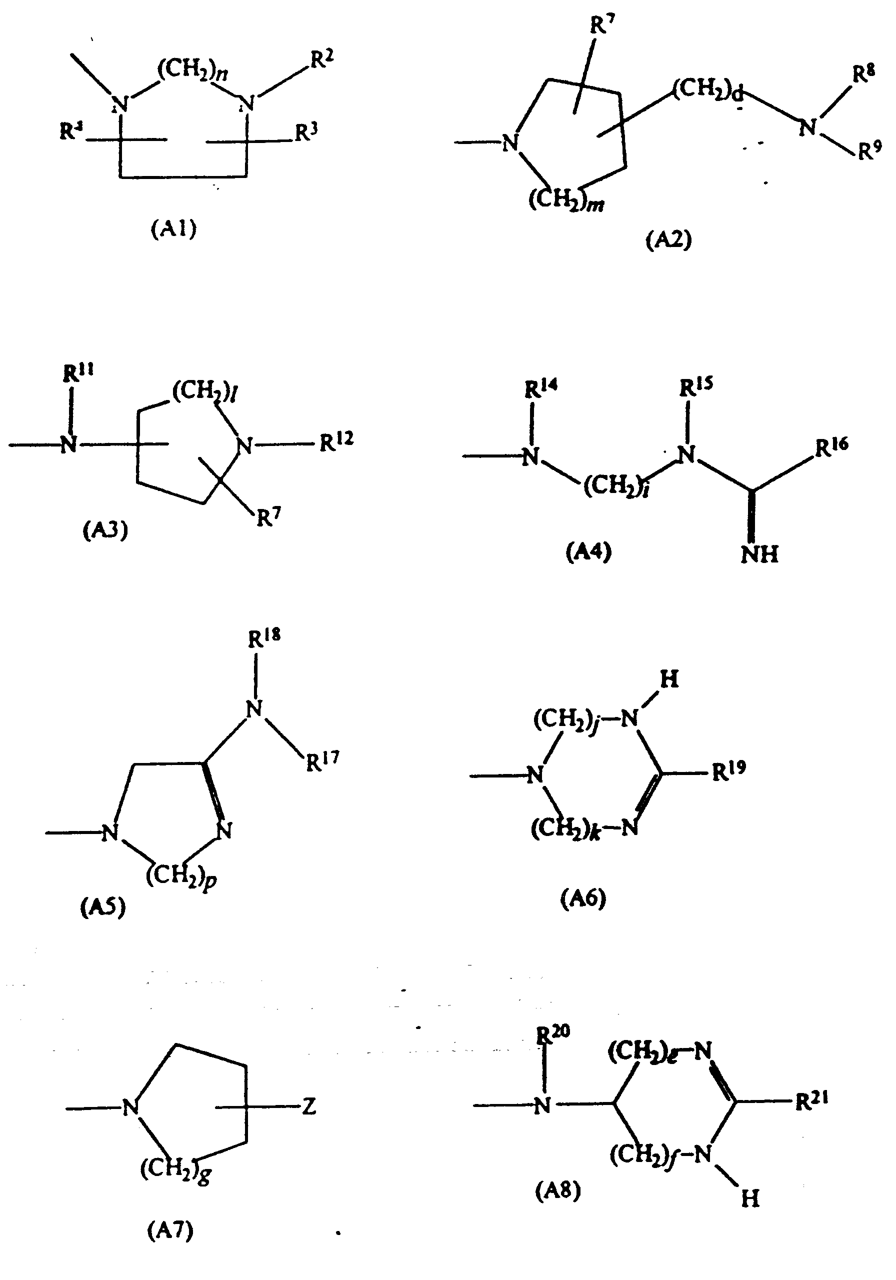 worin
R² bedeutet:
ein Wasserstoffatom,
eine unsubstituierte Alkylgruppe mit 1 bis 6 Kohlenstoffatomen,
eine substituierte Alkylgruppe, die 1 bis 6 Kohlenstoffatome aufweist und die mit mindestens einem der nachstehend definierten Substituenten (b) substituiert ist,
eine Alkenylgruppe mit 2 bis 6 Kohlenstoffatomen,
eine Alkinylgruppe mit 2 bis 6 Kohlenstoffatomen oder
eine Gruppe der Formel -C(=NH)R⁶,
worin R⁶ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet,
R³, R⁴ und R⁷ gleich oder verschieden sind und jeweils bedeuten:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (d) substituiert ist,
ein Halogenatom,
eine Hydroxygruppe,
eine Carboxygruppe,
eine Gruppe der Formel -CO.NR^{a}R^{b}, -OCO.NR^{a}R^{b} oder -NR^{a}R^{b},
worin R^{a} und R^{b} gleich oder verschieden sind und jeweils ein Wasserstoffatom oder eine Alkylgruppe mit 1 bis 4 Kohlenstoffatomen bedeuten,
oder eine Cyangruppe,
R⁸ bedeutet:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (a) substituiert ist,
eine Alkenylgruppe mit 2 bis 6 Kohlenstoffatomen oder
eine Alkinylgruppe mit 2 bis 6 Kohlenstoffatomen,
R⁹ bedeutet:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (a) substituiert ist, oder eine Gruppe der Formel -C(=NH)R¹⁰ ,
worin R¹⁰ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet
oder
R⁸ und R⁹ zusammen eine Gruppe der Formel -(CH₂)ₛ-W-(CH₂)ₜ-darstellen,
in der W eine Kohlenstoff-Kohlenstoff-Einfachbindung, ein Sauerstoffatom, ein Schwefelatom oder eine Gruppe der Formel >NR²² bedeutet, wobei R²² ein Wasserstoffatom oder eine Alkylgruppe mit 1 bis 6 Kohlenstoffatomen darstellt,
und
s und t gleich oder verschieden sind und jeweils 1, 2 oder 3 bedeuten,
R¹¹ ein Wasserstoffatom oder eine Alkylgruppe mit 1 bis 6 Kohlenstoffatomen bedeutet,
R¹² bedeutet:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (a) substituiert ist,
eine Alkenylgruppe mit 2 bis 6 Kohlenstoffatomen,
eine Alkinylgruppe mit 2 bis 6 Kohlenstoffatomen,
oder eine Gruppe der Formel -C(=NH)R¹³,
wobei R¹³ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet,
R¹⁴ und R¹⁵ gleich oder verschieden sind und jeweils ein Wasserstoffatom oder eine Alkylgruppe mit 1 bis 6 Kohlenstoffatomen bedeuten,
R¹⁶ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder
eine Cycloalkylgruppe mit 3 bis 7 Ringkohlenstoffatomen bedeutet,
R¹⁷ oder R¹⁸ gleich oder verschieden sind und jeweils bedeuten:
ein Wasserstoffatom,
eine unsubstituierte Alkylgruppe mit 1 bis 6 Kohlenstoffatomen, oder
eine substituierte Alkylgruppe mit 1 bis 6 Kohlenstoffatomen, die mit mindestens einem der nachstehend definierten Substituenten (a) substituiert ist,
oder
R¹⁷ und R¹⁸ zusammen eine Gruppe der Formel -(CH₂)_{q}-Y-(CH₂)ᵣ-darstellen,
worin Y eine Kohlenstoff-Kohlenstoff-Einfachbindung, ein Sauerstoffatom, ein Schwefelatom oder eine Gruppe der Formel >NR²³ bedeutet, wobei R²³ ein Wasserstoffatom oder eine Alkylgruppe mit 1 bis 6 Kohlenstoffatomen bedeutet, und
q und r gleich oder verschieden sind und jeweils 1, 2 oder 3 bedeuteten,
R¹⁹, R²⁰ und R²¹ gleich oder verschieden sind und jeweils ein Wasserstoffatom oder eine Alkygruppe mit 1 bis 6 Kohlenstoffatomen bedeuten,
Z eine Imidazolyl-, Triazolyl- oder Tetrazolylgruppe darstellt,
d 0 oder 1 ist,
e, f, i, j und k gleich oder verschieden sind und jeweils 1 oder 2 bedeuten,
g, l und m gleich oder verschieden sind und jeweils 0, 1 oder 2 sind und
n und p gleich oder verschieden sind und jeweils 1, 2 oder 3 bedeuten,
mit der Maßgabe daß dann, wenn A eine Gruppe der Formel (A1) darstellt,
nicht alle der Gruppen R² , R³ und R⁴ Wasserstoffatome sind, wenn R¹ ein Wasserstoffatom bedeutet und
nicht alle der Gruppen R¹ , R³ und R⁴ Wasserstoffatome darstellen, wenn R² eine Alkylgruppe bedeutet,
wobei die Substituenten (a) unter
Hydroxygruppen,
Carboxygruppen,
Cyangruppen,
Halogenatomen,
Sauerstoffatomen unter Bildung einer Oxogruppe,
Alkoxygruppen mit 1 bis 6 Kohlenstoffatomen und
Gruppen der Formeln -CO.NR^{a}R^{b}, -OCO.NR^{a}R^{b} und -NR^{a}R^{b} ausgewählt sind, wobei R^{a} und R^{b} wie vorstehend definiert sind,
die Substituenten (b) ausgewählt sind unter
Hydroxygruppen,
Carboxygruppen,
Cyangruppen,
Halogenatomen,
Alkoxygruppen mit 1 bis 6 Kohlenstoffatomen,
Gruppen der Formeln -CO.NR^{a}R^{b}, -OCO.NR^{a}R^{b} und -NR^{a}R^{b}, wobei R^{a} und R^{b} wie vorstehend definiert sind,
Sulfamoylgruppen,
Ureidogruppen,
Sulfogruppen,
Alkanoylgruppen mit 1 bis 6 Kohlenstoffatomen,
Alkanoylaminogruppen mit 1 bis 6 Kohlenstoffatomen,
Alkanoyloxygruppen mit 1 bis 6 Kohlenstoffatomen,
Alkylthiogruppen mit 1 bis 6 Kohlenstoffatomen,
Alkylsulfinylgruppen mit 1 bis 6 Kohlenstoffatomen und
Alkylsulfonylgruppen mit 1 bis 6 Kohlenstoffatomen,
die Substituenten (c) ausgewählt sind unter
Halogenatomen,
Alkoxygruppen mit 1 bis 6 Kohlenstoffatomen,
Cycloalkylgruppen mit 3 bis 7 Ringkohlenstoffatomen und
die Substituenten (d) ausgewählt sind unter
Hydroxygruppen,
Cyangruppen,
Gruppen der Formeln -CO.NR^{a}R^{b}, -OCO.NR^{a}R^{b} und -NR^{a}R^{b} , wobei R^{a} und R^{b} wie vorstehend definiert sind,
Carboxygruppen,
Halogenatomen und
Alkoxygruppen mit 1 bis 6 Kohlenstoffatomen,
und pharmazeutisch geeignete Salze und Ester dieser Verbindung.

2. Verbindung nach Anspruch 1 der Formel (Ia): 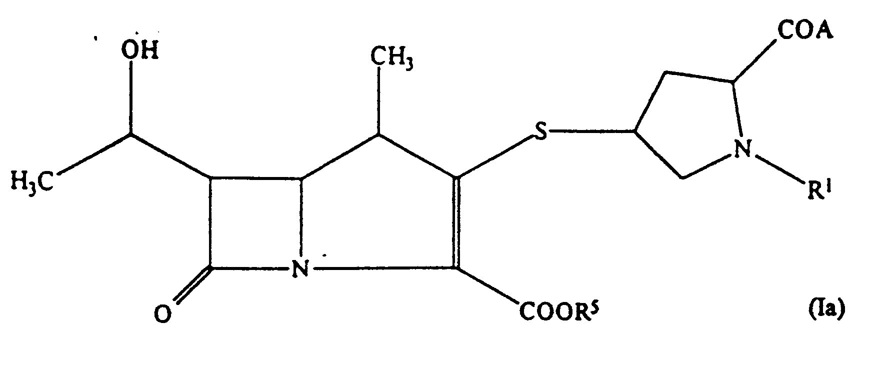 worin R¹ und A wie in Anspruch 1 definiert sind und R⁵ bedeutet:
eine C₁ - C₂₀-Alkylgruppe,
eine C₃ - C₇-Cycloalkylgruppe,
eine Aralkylgruppe, deren Alkylteil eine C₁ - C₃-Alkylgruppe und deren Arylteil eine C₆ - C₁₄-carbocyclische aromatische Gruppe ist, die substituiert oder unsubstituiert sein kann und, falls sie substituiert ist, mindestens einen der nachstehend definierten Substituenten (e) aufweist, eine Alkenylgruppe, die substituiert oder unsubstituiert ist und, falls sie substituiert ist, mindestens einen der in Anspruch 1 definierten Substituenten (a) aufweist,
eine halogenierte C₁ - C₆-Alkylgruppe,
eine substituierte Silylalkylgruppe, deren Alkylteil 1 bis 6 Kohlenstoffatome enthält und deren Silylgruppe bis zu 3 Substituenten aufweist, die unter C₁ - C₆-Alkylgruppen und Phenylgruppen, die unsubstitiuiert sind oder mindestens einen der nachstehend definierten Substituenten (e) haben, ausgewählt sind,
eine Phenylgruppe, die unsubstituiert ist oder die mindestens einen der nachstehend definierten Substituenten (e) aufweist,
eine Phenacylgruppe, die unsubstituiert ist oder die mindestens einen der nachstehend definierten Substituenten (e) aufweist,
eine cyclische oder acyclische Terpenylgruppe,
eine Alkoxymethylgruppe, deren Alkoxyteil C₁ - C₆ umfaßt,
eine aliphatische Acyloxyalkylgruppe, deren Acylgruppe eine C₂ - C₆-Alkanoylgruppe und deren Alkylteil eine C₂ - C₆-Alkylgruppe darstellt,
eine cycloalkylsubstituierte aliphatische Acyloxyalkylgruppe, deren Acylgruppe eine C₂ - C₆-Alkanoylgruppe ist, deren Cycloalkylsubstituent C₃ - C₇ umfaßt und deren Alkylteil eine C₁ - C₆-Alkylgruppe ist,
eine Alkoxycarbonyloxyalkylgruppe, deren Alkoxyteil C₁ - C₁₀ und deren Alkylteil C₁ - C₆ umfaßt,
eine Cycloalkylcarbonyloxyalkyl- oder Cycloalkyloxycarbonyloxyalkylgruppe, deren Cycloalkylgruppe C₃ - C₁₀ umfaßt, mono- oder polycyclisch ist und unsubstituiert oder mit mindestens einer C₁ - C₄-Alkylgruppe substituiert ist und deren Alkylgruppe C₁ - C₆ umfaßt,
eine Cycloalkylalkoxycarbonyloxyalkylgruppe, deren Alkoxygruppe einen einzigen Cycloalkylsubstituenten aufweist, wobei der Cycloalkylsubstituent C₃ - C₁₀ umfaßt und mono- oder polycyclisch ist,
eine Terpenylcarbonyloxyalkyl- oder Terpenyloxycarbonyloxyalkylgruppe, in der die Alkylgruppe 1 bis 6 Kohlenstoffatome aufweist,
eine 5-Alkyl- oder 5-Phenyl(2-oxo-1,3-dioxolen-4-yl)-alkylgruppe, in der jede Alkylgruppe C₁ - C₆ umfaßt oder eine Phthalidyl- Indanyl- oder 2-Oxo-4,5,6,7-tetrahydro-1,3-benzodioxolen-4-yl-gruppe, und
wobei die Substituenten (e) unter C₁ - C₄-Alkylgruppen, C₁ - C₄-Alkoxygruppen, C₁ - C₄-Halogenalkylgruppen, C₁ - C₃-Alkylendioxygruppen, Halogenatomen, Cyangruppen und Nitrogruppen ausgewählt sind.

3. Verbindung nach Anspruch 2, wobei R⁵ ein Wasserstoffatom, eine (5-substituierte 2-Oxo-1,3-dioxolen-4-yl)-methylgruppe, eine 1-Methylcyclohexylcarbonyloxymethyl-Gruppe, eine 1-Isopropoxycarbonyloxyethyl-Gruppe oder eine 1-Cyclohexylcarbonyloxyethyl-Gruppe bedeutet.

4. Verbindung nach einem der Ansprüche 1 bis 3, wobei R¹ bedeutet: ein Wasserstoffatom, eine Alkylgruppe mit 1 bis 3 Kohlenstoffatomen, eine substituierte Alkylgruppe mit 1 bis 3 Kohlenstoffatomen, deren Substituent unter den nachstehend definierten Substituenten (a') ausgewählt ist, eine Alkenylgruppe mit 3 oder 4 Kohlenstoffatomen, eine Alkinylgruppe mit 3 oder 4 Kohlenstoffatomen, eine Formimidoylgruppe oder eine Acetimidoylgruppe, und
wobei die Substituenten (a') unter Hydroxygruppen, Carboxygruppen, Carbamoylgruppen, Carbamoyloxygruppen, Cyangruppen, Halogenatomen, Alkoxygruppen mit 1 bis 3 Kohlenstoffatomen, Aminogruppen und Mono- und Dialkylaminogruppen, in denen die oder jede Alkylgruppe 1 bis 3 Kohlenstoffatome enthält, ausgewählt sind.

5. Verbindung nach einem der Ansprüche 1 bis 4, worin A eine Gruppe der Formel (A1) bedeutet und n 2 oder 3 ist.

6. Verbindung nach einem der Ansprüche 1 bis 5, worin R² bedeutet:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter den nachstehend definierten Substituenten (b') ausgewählt ist,
eine Alkenylgruppe mit 3 oder 4 Kohlenstoffatomen,
eine Alkinylgruppe mit 3 oder 4 Kohlenstoffatomen oder
eine Gruppe der Formel -C(=NH)R⁶,
worin R⁶ ein Wasserstoffatom,
eine unsubstituierte Alkylgruppe mit 1 bis 3 Kohlenstoffatomen,
eine substituierte Alkylgruppe mit 1 bis 3 Kohlenstoffatomen, die mit mindestens einem Substituenten substituiert ist, der ausgewählt ist unter Halogenatomen, Alkoxygruppen mit 1 bis 3 Kohlenstoffatomen und Cycloalkylgruppen mit 3 bis 6 Kohlenstoffatomen, oder
eine Cycloalkylgruppe mit 3 bis 6 Ringkohlenstoffatomen bedeutet, und
wobei die Substituenten (b') ausgewählt sind unter Hydroxygruppen, Carboxygruppen, Carbamoylgruppen, Carbamoyloxygruppen, Cyangruppen, Sulfamoylgruppen, Ureidogruppen, Sulfogruppen, Alkoxygruppen mit 1 bis 3 Kohlenstoffatomen, Alkoxycarbonylgruppen mit 2 bis 4 Kohlenstoffatomen, Alkanoylgruppen mit 2 bis 4 Kohlenstoffatomen, Alkanoylaminogruppen mit 2 bis 4 Kohlenstoffatomen, Alkanoyloxygruppen mit 2 bis 4 Kohlenstoffatomen, Aminogruppen, Mono- und Dialkylaminogruppen, in denen die oder jede Alkylgruppe 1 bis 3 Kohlenstoffatome aufweist,
Alkylthiogruppen mit 1 bis 3 Kohlenstoffatomen, Alkylsulfinylgruppen mit 1 bis 3 Kohlenstoffatomen, Alkylsulfonylgruppen mit 1 bis 3 Kohlenstoffatomen, Mono- und Dialkylcarbamoylgruppen, in denen die oder jede Alkylgruppe 1 bis 3 Kohlenstoffatome aufweist und Mono- und Dialkylcarbamoyloxygruppen, in denen die oder jede Alkylgruppe 1 bis 3 Kohlenstoffatome aufweist.

7. Verbindung nach einem der Ansprüche 1 bis 6, worin A eine Gruppe der Formel (A1) bedeutet und R³ und R⁴ jeweils ein Wasserstoffatom, eine Alkylgruppe mit 1 bis 3 Kohlenstoffatomen, eine Hydroxygruppe, eine Carboxygruppe, eine Carbamoylgruppe oder eine substituierte Alkylgruppe, die 1 bis 3 Kohlenstoffatome aufweist und die mit mindestens einem unter Hydroxygruppen, Alkoxygruppen mit 1 bis 3 Kohlenstoffatomen, Aminogruppen, Carbamoylgruppen und Halogenatomen ausgewählten Substituenten substituiert ist, bedeuten.

8. Verbindung nach Anspruch 1 oder Anspruch 2, worin
A eine Gruppe der Formel (A1) bedeutet,
n 2 ist,
R¹ ein Wasserstoffatom, eine Methylgruppe, eine Fluormethylgruppe, eine Formimidoylgruppe oder eine Acetimidoylgruppe bedeutet;
R² ein Wasserstoffatom, eine 2-Hydroxyethylgruppe, eine 2-Carbamoylethylgruppe, eine Carboxymethylgruppe, eine Carbamoylmethylgruppe, eine 2-Fluorethylgruppe, eine Formimidoylgruppe oder eine Acetimidoylgruppe bedeutet und
R³ und R⁴ gleich oder verschieden sind und jeweils ein Wasserstoffatom, eine Methylgruppe, eine Carbamoylgruppe, eine Cyangruppe, eine Carboxygruppe, eine Hydroxymethylgruppe, eine Fluormethylgruppe oder eine Aminomethylgruppe darstellen.

9. Verbindung nach Anspruch 1 oder Anspruch 2, worin
A eine Gruppe der Formel (A1) bedeutet,
n für 3 steht,
R¹ ein Wasserstoffatom, eine Methylgruppe, eine Fluormethylgruppe, eine Formimidoylgruppe oder eine Acetimidoylgruppe bedeutet,
R² ein Wasserstoffatom, eine Methylgruppe, eine Formimidoylgruppe, eine Acetimidoylgruppe, eine Carboxymethylgruppe, eine Carbamoylmethylgruppe, eine 2-Hydroxyethylgruppe oder eine 2-Fluorethylgruppe bedeutet und
R³ und R⁴ gleich oder verschieden sind und jeweils ein Wasserstoffatom, eine Methylgruppe, eine Hydroxygruppe, eine Aminogruppe, eine Cyangruppe, eine Carboxygruppe, eine Carbamoylgruppe, eine Carbamoyloxygruppe, eine Hydroxymethylgruppe, eine Fluormethylgruppe oder eine Aminomethylgruppe bedeuten.

10. Verbindung nach Anspruch 1 oder Anspruch 2, worin
A eine Gruppe der Formel (A1) bedeutet,
n für 2 steht
R¹ ein Wasserstoffatom, eine Methylgruppe, eine Formimidoylgruppe oder eine Acetimidoylgruppe bedeutet,
R² ein Wasserstoffatom, eine 2-Hydroxyethylgruppe, eine Carboxymethylgruppe, eine Formimidoylgruppe oder eine Acetimidoylgruppe bedeutet,
R³ ein Wasserstoffatom bedeutet, und
R⁴ eine Methylgruppe, eine Carbamoylgruppe, eine Cyangruppe, eine Hydroxymethylgruppe, eine Fluormethylgruppe oder eine Aminomethylgruppe bedeutet.

11. Verbindung nach Anspruch 1 oder Anspruch 2, worin:
A eine Gruppe der Formel (A1) bedeutet,
n für 3 steht
R¹ ein Wasserstoffatom, eine Methylgruppe, eine Formimidoylgruppe oder eine Acetimidoylgruppe bedeutet,
R² eine Formimidoylgruppe, eine Acetimidoylgruppe, eine Carboxymethylgruppe, eine 2-Hydroxyethylgruppe oder eine 2-Fluorethylgruppe bedeutet, und
R³ und R⁴ gleich oder verschieden sind und jeweils ein Wasserstoffatom, eine Hydroxygruppe, eine Aminogruppe oder eine Cyangruppe bedeuten.

12. Verbindung nach Anspruch 1 oder Anspruch 2, worin A eine Gruppe der Formel (A2) bedeutet und R⁷ ein Wasserstoffatom, eine Carboxygruppe, eine Carbamoylgruppe, eine Alkylgruppe mit 1 bis 3 Kohlenstoffatomen oder eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carboxygruppen und Cyangruppen ausgewählt ist, bedeutet.

13. Verbindung nach Anspruch 1, worin A eine Gruppe der Formel (A2) bedeutet und R⁸ ein Wasserstoffatom, eine Alkylgruppe mit 1 bis 3 Kohlenstoffatomen, eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carbamoyloxygruppen, Carboxygruppen, Cyangruppen, Aminogruppen und Halogenatomen ausgewählt ist, eine Alkenylgruppe mit 3 oder 4 Kohlenstoffatomen oder eine Alkinylgruppe mit 3 oder 4 Kohlenstoffatomen bedeutet.

14. Verbindung nach Anspruch 1 oder Anspruch 2, worin A eine Gruppe der Formel (A2) bedeutet und R⁹ bedeutet:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carbamoyloxygruppen, Carboxygruppen, Cyangruppen, Aminogruppen und Halogenatomen ausgewählt ist, oder eine Gruppe der Formel -C(=NH)R¹⁰ , worin R¹⁰ darstellt:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter Alkoxygruppen mit 1 bis 3 Kohlenstoffatomen und Halogenatomen ausgewählt ist,
eine Cycloalkylgruppe mit 3 bis 6 Kohlenstoffatomen
oder eine Alkylgruppe mit 1 bis 3 Kohlenstoffatomen, die mit einer einzigen Cycloalkylgruppe mit 3 bis 6 Kohlenstoffatomen substituiert ist.

15. Verbindung nach Anspruch 1 oder Anspruch 2, worin A eine Gruppe der Formel (A2) bedeutet und R⁸ und R⁹ zusammen eine Gruppe der Formel -(CH₂)ₛ-W-(CH₂)ₜ-, darstellen,
in der W eine Kohlenstoff-Kohlenstoff-Einfachbindung, ein Sauerstoffatom, ein Schwefelatom oder eine Gruppe der Formel >NR²² bedeutet, wobei R²² ein Wasserstoffatom oder eine Alkylgruppe mit 1 bis 3 Kohlenstoffatomen ist, s für 1, 2 oder 3 steht und t 2 ist.

16. Verbindung nach Anspruch 1 oder Anspruch 2, worin
A eine Gruppe der Formel (A2) bedeutet,
d 0 oder 1 ist,
m 0, 1 oder 2 ist,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom, eine Alkylgruppe mit 1 bis 3 Kohlenstoffatomen, eine Hydroxygruppe, eine Aminogruppe, eine Cyangruppe, ein Halogenatom, eine Carboxygruppe, eine Carbamoylgruppe oder eine Hydroxymethylgruppe bedeutet,
R⁸ ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Alkenylgruppe mit 3 oder 4 Kohlenstoffatomen, eine Alkinylgruppe mit 3 oder 4 Kohlenstoffatomen, eine 2-Halogenethylgruppe, eine 2-Hydroxyethylgruppe, eine 2-Alkoxyethylgruppe, deren Alkoxyteil 1 bis 3 Kohlenstoffatome enthält, oder eine 2-Aminoethylgruppe bedeutet,
R⁹ ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Formimidoylgruppe, eine Acetimidoylgruppe, eine 2-Halogenethylgruppe, eine 2-Hydroxyethylgruppe, eine 2-Alkoxyethylgruppe, deren Alkoxyteil 1 bis 3 Kohlenstoffatome aufweist, oder eine 2-Aminoethylgruppe bedeutet,
oder
R⁸ und R⁹ zusammen eine Gruppe der Formel
-(CH₂)₄-,
-(CH₂)₅-,
-(CH₂)₂O(CH₂)₂-,
-(CH₂)₂S(CH₂)₂-,
-(CH₂)₂NH(CH₂)₂- oder
-(CH₂)₂NCH₃(CH₂)₂-
bedeuten.

17. Verbindung nach Anspruch 1 oder Anspruch 2, worin
A eine Gruppe der Formel (A2) bedeutet,
d für 0 steht,
m für 1 oder 2 steht,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom bedeutet,
R⁸ ein Wasserstoffatom, eine Alkylgruppe mit 1 bis 3 Kohlenstoffatomen, eine Carbamoylmethylgruppe, eine Carboxymethylgruppe, eine 2-Fluorethylgruppe oder eine 2-Hydroxyethylgruppe bedeutet und
R⁹ ein Wasserstoffatom, eine Alkylgruppe mit 1 bis 3 Kohlenstoffatomen, eine Formimidoylgruppe, eine Acetimidoylgruppe oder eine 2-Fluorethylgruppe bedeutet.

18. Verbindung nach Anspruch 1 oder Anspruch 2, worin A eine Gruppe der Formel (A3) bedeutet, l für 0, 1 oder 2 steht und R⁷ ein Wasserstoffatom oder eine Alkylgruppe mit 1 bis 3 Kohlenstoffatomen bedeutet.

19. Verbindung nach Anspruch 1 oder Anspruch 2, worin A eine Gruppe der Formel (A3) bedeutet und R¹¹ ein Wasserstoffatom oder eine Alkylgruppe mit 1 bis 3 Kohlenstoffatomen, wie eine Methyl-, Ethyl- oder Propylgruppe, bedeutet.

20. Verbindung nach Anspruch 1 oder Anspruch 2, worin A eine Gruppe der Formel (A3) bedeutet und R¹² darstellt: ein Wasserstoffatom, eine Alkylgruppe mit 1 bis 3 Kohlenstoffatomen, eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carbamoyloxygruppen, Carboxygruppen, Cyangruppen, Aminogruppen und Halogenatomen ausgewählt ist, eine Alkenylgruppe mit 3 oder 4 Kohlenstoffatomen, eine Alkinylgruppe mit 3 oder 4 Kohlenstoffatomen oder eine Gruppe der Formel -C(=NH)R¹³, wobei R¹³ bedeutet:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter Alkoxygruppen mit 1 bis 3 Kohlenstoffatomen und Halogenatomen ausgewählt ist,
eine Cycloalkylgruppe mit 3 bis 6 Kohlenstoffatomen oder
eine Alkylgruppe mit 1 bis 3 Kohlenstoffatomen, die mit einer einzigen Cycloalkylgruppe mit 3 bis 6 Kohlenstoffatomen substituert ist.

21. Verbindung nach Anspruch 1 oder Anspruch 2, worin
A eine Gruppe der Formel (A3) bedeutet,
l für 0, 1 oder 2 steht,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom bedeutet,
R¹¹ ein Wasserstoffatom oder eine Alkylgruppe mit 1 bis 3 Kohlenstoffatomen bedeutet und
R¹² ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Alkenylgruppe mit 3 oder 4 Kohlenstoffatomen, eine Alkinylgruppe mit 3 oder 4 Kohlenstoffatomen, eine Formimidoylgruppe, eine Acetimidoylgruppe, eine 2-Halogenethylgruppe, eine 2-Hydroxyethylgruppe, eine 2-Alkoxyethylgruppe, deren Alkoxyteil 1 bis 3 Kohlenstoffatome aufweist, oder eine Aminoethylgruppe bedeutet.

22. Verbindung nach Anspruch 1 oder Anspruch 2, worin:
A eine Gruppe der Formel (A3) bedeutet,
l für 1 oder 2 steht,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom bedeutet,
R¹¹ ein Wasserstoffatom oder eine Methylgruppe bedeutet und
R¹² ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Formimidoylgruppe, eine Acetimidoylgruppe, eine 2-Fluorethylgruppe oder eine 2-Hydroxyethylgruppe bedeutet.

23. Verbindung nach Anspruch 1 oder Anspruch 2, worin A eine Gruppe der Formel (A4) bedeutet und R¹⁴ und R¹⁵ jeweils ein Wasserstoffatom oder eine Alkylgruppe mit 1 bis 3 Kohlenstoffatomen bedeuten.

24. Verbindung nach Anspruch 1 oder Anspruch 2, worin A eine Gruppe der Formel (A4) bedeutet und R¹⁶ ein Wasserstoffatom, eine Alkylgruppe mit 1 bis 3 Kohlenstoffatomen, eine substituierte Alkylgruppe mit 1 bis 3 Kohlenstoffatomen, deren Substituent unter Alkoxygruppen mit 1 bis 3 Kohlenstoffatomen und Halogenatomen ausgewählt ist, eine Cycloalkylgruppe mit 3 bis 6 Kohlenstoffatomen oder eine Alkylgruppe mit 1 bis 3 Kohlenstoffatomen, die mit einer einzigen Cycloalkylgruppe mit 3 bis 6 Kohlenstoffatomen substituiert ist, bedeutet.

25. Verbindung nach Anspruch 1 oder Anspruch 2, worin
A eine Gruppe der Formel (A4) bedeutet,
i für 1 oder 2 steht und
R¹, R¹⁴, R¹⁵ und R¹⁶ gleich oder verschieden sind und jeweils ein Wasserstoffatom oder eine Alkylgruppe mit 1 bis 3 Kohlenstoffatomen bedeuten.

26. Verbindung nach Anspruch 1 oder Anspruch 2, worin:
A eine Gruppe der Formel (A4) bedeutet,
i für 1 steht und
R¹, R¹⁴, R¹⁵ und R¹⁶ gleich oder verschieden sind und jeweils ein Wasserstoffatom oder eine Methylgruppe bedeuten.

27. Verbindung nach Anspruch 1 oder Anspruch 2, worin A eine Gruppe der Formel (A5) bedeutet und R¹⁷ und R¹⁸ jeweils ein Wasserstoffatom, eine Alkylgruppe mit 1 bis 3 Kohlenstoffatomen oder eine substituierte Alkylgruppe mit 1 bis 3 Kohlenstoffatomen, deren Substituent unter Hydroxygruppen, Alkoxygruppen mit 1 bis 3 Kohlenstoffatomen und Halogenatomen ausgewählt ist, bedeutet.

28. Verbindung nach Anspruch 1 oder Anspruch 2, worin A eine Gruppe der Formel (A5) bedeutet und R¹⁷ und R¹⁸ zusammen eine Gruppe der Formel -(CH₂)_{q}-Y-(CH₂)ᵣ- darstellen, in der Y eine Kohlenstoff-Kohlenstoff-Einfachbindung, ein Sauerstoffatom oder eine Gruppe der Formel >NR²³ darstellt, wobei R²³ ein Wasserstoffatom oder eine Alkylgruppe mit 1 bis 3 Kohlenstoffatomen bedeutet, und q und r jeweils 2 oder 3 bedeuten.

29. Verbindung nach Anspruch 1 oder Anspruch 2, worin
A eine Gruppe der Formel (A5) bedeutet,
p für 2 steht,
R¹ ein Wasserstoffatom, eine Methylgruppe, eine Fluormethylgruppe, eine Formimidoylgruppe oder eine Acetimidoylgruppe bedeutet,
R¹⁷ und R¹⁸ gleich oder verschieden sind und jeweils ein Wasserstoffatom, eine Alkylgruppe mit 1 bis 3 Kohlenstoffatomen, eine 2-Halogenethylgruppe oder eine 2-Hydroxyethylgruppe bedeuten,
oder
R¹⁷ und R¹⁸ zusammen eine Gruppe der Formel
-(CH₂)₄-,
-(CH₂)₅-,
-(CH₂)₂O(CH₂)₂-,
-(CH₂)₂S(CH₂)₂-,
-(CH₂)₂NH(CH₂)₂- oder
-(CH₂)₂NCH₃(CH₂)₂-
darstellen.

30. Verbindung nach Anspruch 1 oder Anspruch 2, worin
A eine Gruppe der Formel (A5) bedeutet,
p für 2 steht,
R¹ ein Wasserstoffatom, eine Methylgruppe, eine Fluormethylgruppe, eine Formimidoylgruppe oder eine Acetimidoylgruppe bedeutet, und
R¹⁷ und R¹⁸ gleich oder verschieden sind und jeweils ein Wasserstoffatom oder eine Methylgruppe bedeuten.

31. Verbindung nach Anspruch 1 oder Anspruch 2, worin A eine Gruppe der Formel (A6) bedeutet und R¹⁹ ein Wasserstoffatom oder eine Alkylgruppe mit 1 bis 3 Kohlenstoffatomen bedeutet.

32. Verbindung nach Anspruch 1 oder Anspruch 2, worin
A eine Gruppe der Formel (A6) bedeutet,
beide Symbole j und k für 2 stehen,
R¹ ein Wasserstoffatom, eine Methylgruppe, eine Fluormethylgruppe, eine Formimidoylgruppe oder eine Acetimidoylgruppe bedeutet, und
R¹⁹ ein Wasserstoffatom oder eine Alkylgruppe mit 1 bis 3 Kohlenstoffatomen bedeutet.

33. Verbindung nach Anspruch 1 oder Anspruch 2, worin
A eine Gruppe der Formel (A6) bedeutet,
beide Symbole j und k für 2 stehen, und
R¹ und R¹⁹ gleich oder verschieden sind und jeweils ein Wasserstoffatom oder eine Methylgruppe bedeuten.

34. Verbindung nach Anspruch 1 oder Anspruch 2, worin A eine Gruppe der Formel (A7) bedeutet und Z eine 1-Imidazolylgruppe, eine 1,2,4-Triazol-1-yl-Gruppe oder eine 1,2,3-Triazol-1-yl-Gruppe bedeutet.

35. Verbindung nach Anspruch 1 oder Anspruch 2, worin
A eine Gruppe der Formel (A7) bedeutet,
g für 0, 1 oder 2 steht,
R¹ ein Wasserstoffatom oder eine Methylgruppe bedeutet und
Z eine 1-Imidazolylgruppe, eine 1,2,4-Triazol-1-yl-Gruppe oder eine 1,2,3-Triazol-1-yl-Gruppe bedeutet.

36. Verbindung nach Anspruch 1 oder Anspruch 2, worin
A eine Gruppe der Formel (A7) bedeutet,
g für 1 oder 2 steht,
R¹ ein Wasserstoffatom oder eine Methylgruppe bedeutet und
Z eine 1-Imidazolylgruppe, eine 1,2,4-Triazol-1-yl-Gruppe oder eine 1,2,3-Triazol-1-yl-Gruppe bedeutet.

37. Verbindung nach Anspruch 1 oder Anspruch 2, worin A eine Gruppe der Formel (A8) bedeutet und R²⁰ und R²¹ jeweils ein Wasserstoffatom oder eine Alkylgruppe mit 1 bis 3 Kohlenstoffatomen bedeuten.

38. Verbindung nach Anspruch 1 oder Anspruch 2, worin
A eine Gruppe der Formel (A8) bedeutet,
beide Symbole e und f für 1 stehen,
R¹ ein Wasserstoffatom, eine Methylgruppe, eine Fluormethylgruppe, eine Formimidoylgruppe oder eine Acetimidoylgruppe bedeutet, und
R²⁰ und R²¹ jeweils ein Wasserstoffatom oder eine Alkylgruppe mit 1 bis 3 Kohlenstoffatomen bedeuten.

39. Verbindung nach Anspruch 1 oder Anspruch 2, worin
A eine Gruppe der Formel (A8) bedeutet,
beide Symbole e und f für 1 stehen,
R¹ ein Wasserstoffatom oder eine Methylgruppe bedeutet,
R²⁰ ein Wasserstoffatom bedeutet und
R²¹ ein Wasserstoffatom oder eine Methylgruppe bedeutet.

40. Verbindung nach einem der vorhergehenden Patentansprüche, in welcher die Kohlenstoffatome in den gleichen Konfigurationen wie die des Thienamycins vorliegen.

41. Folgende Verbindungen nach Anspruch 1:
2-[2-(1-Homopiperazinylcarbonyl)-1-methylpyrrolidin-4-ylthio]-6-(1-hydroxyethyl)-1-methyl-1-carbapen-2-em-3-carbonsäure;
2-[2-(4-Carboxymethylhomopiperazin-1-ylcarbonyl)-pyrrolidin-4-ylthio]-6-(1-hydroxyethyl)-1-methyl-1-carbapen-2-em-3-carbonsäure;
2-{2-[4-(2-Hydroxyethyl)homopiperazin-1-ylcarbonyl]-pyrrolidin-4-ylthio}-6-(1-hydroxyethyl)-1-methyl-1-carbapen-2-em-3-carbonsäure;
2-[2-(4-Acetimidoylhomopiperazin-1-ylcarbonyl)-pyrrolidin-4-ylthio]-6-(1-hydroxyethyl)-1-methyl-1-carbapen-2-em-3-carbonsäure;
2-[2-(4-Formimidoylhomopiperazin-1-ylcarbonyl)-pyrrolidin-4-ylthio]-6-(1-hydroxyethyl)-1-methyl-1-carbapen-2-em-3-carbonsäure;
2-[2-(4-Formimidoylhomopiperazin-1-ylcarbonyl)-1-methylpyrrolidin-4-ylthio]-6-(1-hydroxyethyl)-1-methyl-1-carbapen-2-em-3-carbonsäure;
2-[1-Methyl-2-(piperazin-1-ylcarbonyl)pyrrolidin-4-ylthio]-6-(1-hydroxyethyl)-1-methyl-1-carbapen-2-em-3-carbonsäure;
2-{2-[4-(2-Hydroxyethyl)piperazin-1-ylcarbonyl]-pyrrolidin-4-ylthio}-6-(1-hydroxyethyl)-1-methyl-1-carbapen-2-em-3-carbonsäure;
2-[2-(3-Methylpiperazin-1-ylcarbonyl)-pyrrolidin-4-ylthio]-6-(1-hydroxyethyl)-1-methyl-1-carbapen-2-em-3-carbonsäure;
2-[2-(4-Formimidoylpiperazin-1-ylcarbonyl)-pyrrolidin-4-ylthio]-6-(1-hydroxyethyl)-1-methyl-1-carbapen-2-em-3-carbonsäure;
2-[2-(4-Acetimidoylpiperazin-1-ylcarbonyl)-pyrrolidin-4-ylthio]-6-(1-hydroxyethyl)-1-methyl-1-carbapen-2-em-3-carbonsäure;
2-[2-(4-Formimidoylpiperazin-1-ylcarbonyl)-1-methylpyrrolidin-4-ylthio]-6-(1-hydroxyethyl)-1-methyl-1-carbapen-2-em-3-carbonsäure;
2-[2-(4-Acetimidoylpiperazin-1-ylcarbonyl)-1-methylpyrrolidin-4-ylthio]-6-(1-hydroxyethyl)-1-methyl-1-carbapen-2-em-3-carbonsäure;
2-[2-(4-Formimidoyl-3-methylpiperazin-1-ylcarbonyl)-pyrrolidin-4-ylthio]-6-(1-hydroxyethyl)-1-methyl-1-carbapen-2-em-3-carbonsäure;
2-[2-(4-Acetimidoyl-3-methylpiperazin-1-ylcarbonyl)-pyrrolidin-4-ylthio]-6-(1-hydroxyethyl)-1-methyl-1-carbapen-2-em-3-carbonsäure;
2-[2-(2-Methylpiperazin-1-ylcarbonyl)-pyrrolidin-4-yl thio]-6-(1-hydroxyethyl)-1-methyl-1-carbapen-2-em-3-carbonsäure;
2-[2-(4-Formimidoyl-2-methylpiperazin-1-ylcarbonyl)-pyrrolidin-4-ylthio]-6-(1-hydroxyethyl)-1-methyl-1-carbapen-2-em-3-carbonsäure;
2-[2-(4-Acetimidoyl-2-methylpiperazin-1-ylcarbonyl)-pyrrolidin-4-ylthio]-6-(1-hydroxyethyl)-1-methyl-1-carbapen-2-em-3-carbonsäure;
2-[2-(3-Hydroxymethylpiperazin-1-ylcarbonyl)-pyrrolidin-4-ylthio]-6-(1-hydroxyethyl)-1-methyl-1-carbapen-2-em-3-carbonsäure;
2-[1-Formimidoyl-2-(4-formimidoylpiperazin-1-ylcarbonyl)-pyrrolidin-4-ylthio]-6-(1-hydroxyethyl)-1-methyl-1-carbapen-2-em-3-carbonsäure;
2-[2-(3-Acetimidoylaminopyrrolidin-1-ylcarbonyl)-pyrrolidin-4-ylthio]-6-(1-hydroxyethyl)-1-methyl-1-carbapen-2-em-3-carbonsäure;
2-[2-(3-Formimidoylaminopyrrolidin-1-ylcarbonyl)-pyrrolidin-4-ylthio]-6-(1-hydroxyethyl)-1-methyl-1-carbapen-2-em-3-carbonsäure;
2-[2-(3-Aminopyrrolidin-1-ylcarbonyl)-pyrrolidin-4-ylthio]-6-(1-hydroxyethyl)-1-methyl-1-carbapen-2-em-3-carbonsäure;
2-[2-(4-Acetimidoylaminopiperidin-1-ylcarbonyl)-pyrrolidin-4-ylthio]-6-(1-hydroxyethyl)-1-methyl-1-carbapen-2-em-3-carbonsäure;
2-[2-(3-Aminopyrrolidin-1-ylcarbonyl)-1-methylpyrrolidin-4-ylthio]-6-(1-hydroxyethyl)-1-methyl-1-carbapen-2-em-3-carbonsäure;
2-[2-(3-Acetimidoylaminopyrrolidin-1-ylcarbonyl)-1-methylpyrrolidin-4-ylthio]-6-(1-hydroxyethyl)-1-methyl-1-carbapen-2-em-3-carbonsäure;
2-[2-(3-Formimidoylaminopyrrolidin-1-ylcarbonyl)-1-methylpyrrolidin-4-ylthio]-6-(1-hydroxyethyl)-1-methyl-1-carbapen-2-em-3-carbonsäure;
2-[2-(4-Acetimidoylaminopiperidin-1-ylcarbonyl)-1-methylpyrrolidin-4-ylthio]-6-(1-hydroxyethyl)-1-methyl-1-carbapen-2-em-3-carbonsäure;
2-[2-(1-Formimidoylpyrrolidin-3-ylcarbamoyl)pyrrolidin-4-ylthio]-6-(1-hydroxyethyl)-1-methyl-1-carbapen-2-em-3-carbonsäure;
2-[2-(3-Dimethylamino-1,2,5,6-tetrahydropyrazin-1-ylcarbonyl)pyrrolidin-4-ylthio]-6-(1-hydroxyethyl)-1-methyl-1-carbapen-2-em-3-carbonsäure;
und deren pharmazeutisch geeignete Salze und Ester.

42. Arzneimittelzusammensetzung, die ein pharmazeutisch geeignetes Trägermaterial, Verdünnungsmittel oder Adjuvans im Gemisch mit einer wirksamen Menge eines Antibiotikums enthält, wobei das Antibiotikum unter Verbindungen der Formel (I) und pharmazeutisch geeigneten Salzen und Estern davon gemäß einem der Ansprüche 1 bis 41 ausgewählt ist.

43. Verfahren zur Herstellung einer Verbindung nach einem der Ansprüche 1 bis 41, welches folgende Stufen umfaßt:
Umsetzen einer Verbindung der Formel (II) 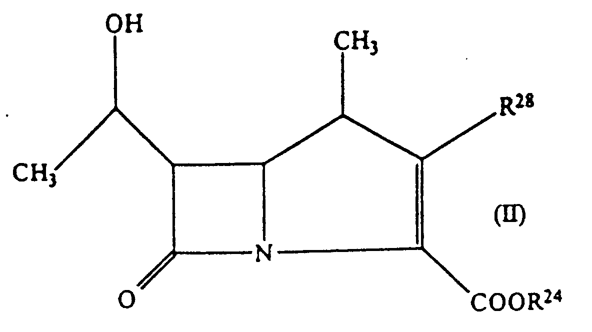 (worin R²⁴ eine Schutzgruppe für die Carboxylgruppe und R²⁸ eine Alkansulfonyloxygruppe, eine Arylsulfonyloxygruppe, eine Dialkylphosphoryloxygruppe, eine Diarylphosphoryloxygruppe oder eine Gruppe der Formel
-S(→O)R²⁷ bedeutet, wobei R²⁷ eine Alkylgruppe, eine Halogenalkylgruppe, eine Acetamidoalkylgruppe, eine Acetamidoalkenylgruppe, eine Arylgruppe oder eine aromatische heterocyclische Gruppe bedeutet) mit einer Verbindung der Formel (III) 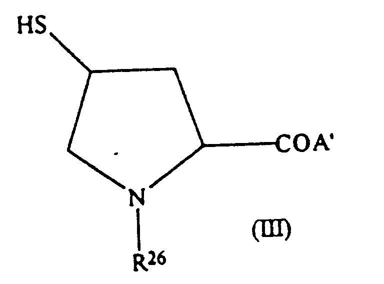 (worin R²⁶ irgendeine der durch R¹ dargestellten Gruppen oder Atome oder eine solche Gruppe oder ein solches Atom bedeutet, worin aktive Gruppen geschützt sind und A' irgendeine der durch A dargestellten Gruppen oder Atome oder irgendeine solche Gruppe oder ein Atom worin aktive Gruppen geschützt sind, bedeutet) und
erforderlichenfalls Entfernen von vorhandenen Schutzgruppen.

44. Verwendung einer Verbindung nach einem der Ansprüche 1 bis 41 zur Herstellung eines Arzneimittels zur Behandlung oder Prophylaxe von bakteriellen Infektionen.

## Patentansprüche (Patentansprüche für folgende(n) Vertragsstaat(en): ES, GR)

1. Verfahren zur Herstellung einer Verbindung der Formel (I): 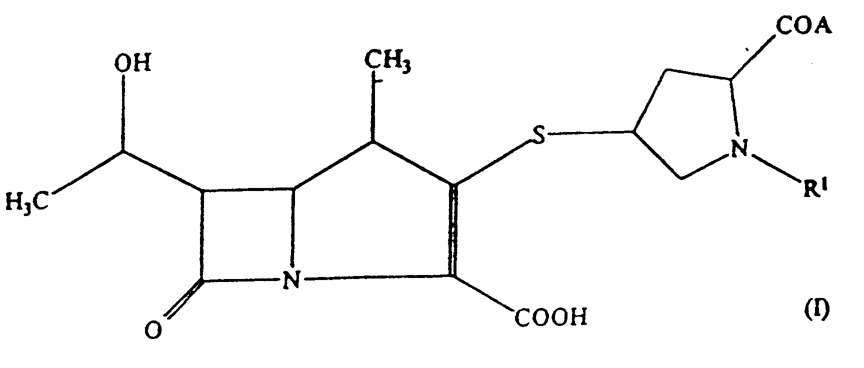 [worin
R¹ bedeutet:
ein Wasserstoffatom,
eine unsubstituierte Alkylgruppe mit 1 bis 6 Kohlenstoffatomen,
eine substituierte Alkylgruppe, die 1 bis 6 Kohlenstoffatome aufweist und die mit mindestens einem der nachstehend definierten Substituenten (a) substituiert ist,
eine Alkenylgruppe mit 2 bis 6 Kohlenstoffatomen,
eine Alkinylgruppe mit 2 bis 6 Kohlenstoffatomen oder
eine Gruppe der Formel -C(=NH)R°, worin R° ein Wasserstoffatom oder eine Alkylgruppe mit 1 bis 6 Kohlenstoffatomen bedeutet, und
A eine Gruppe der Formel (A1), (A2), (A3), (A4), (A5), (A6), (A7) oder (A8) bedeutet: 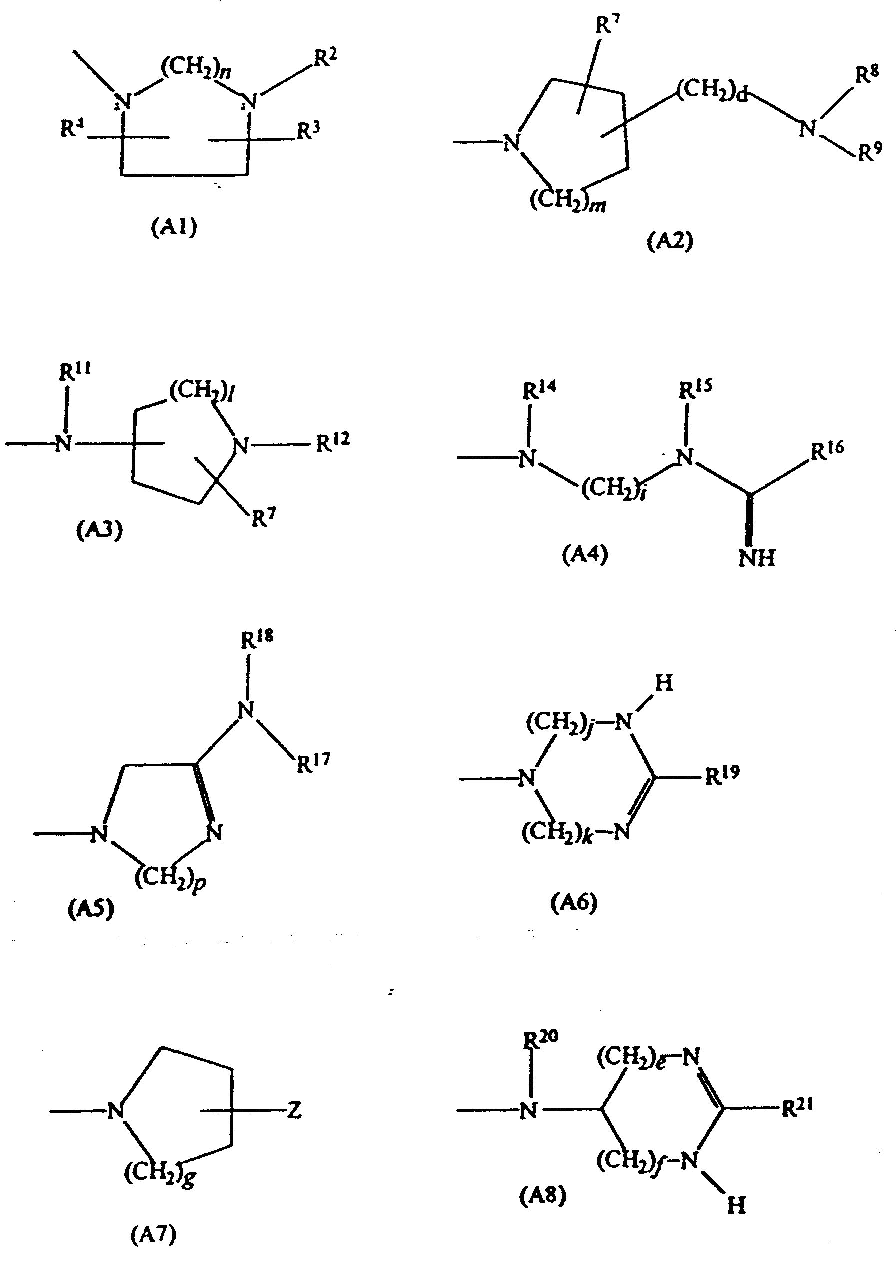 worin
R² bedeutet:
ein Wasserstoffatom,
eine unsubstituierte Alkylgruppe mit 1 bis 6 Kohlenstoffatomen,
eine substituierte Alkylgruppe, die 1 bis 6 Kohlenstoffatome aufweist und die mit mindestens einem der nachstehend definierten Substituenten (b) substituiert ist,
eine Alkenylgruppe mit 2 bis 6 Kohlenstoffatomen,
eine Alkinylgruppe mit 2 bis 6 Kohlenstoffatomen oder
eine Gruppe der Formel -C(=NH)R⁶,
worin R⁶ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet,
R³ , R⁴ und R⁷ gleich oder verschieden sind und jeweils bedeuten:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (d) substituiert ist,
ein Halogenatom,
eine Hydroxygruppe,
eine Carboxygruppe,
eine Gruppe der Formel -CO.NR^{a}R^{b}, -OCO.NR^{a}R^{b} oder -NR^{a}R^{b},
worin R^{a} und R^{b} gleich oder verschieden sind und jeweils ein Wasserstoffatom oder eine Alkylgruppe mit 1 bis 4 Kohlenstoffatomen bedeuten,
oder eine Cyangruppe,
R⁸ bedeutet:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (a) substituiert ist,
eine Alkenylgruppe mit 2 bis 6 Kohlenstoffatomen oder
eine Alkinylgruppe mit 2 bis 6 Kohlenstoffatomen,
R⁹ bedeutet:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (a) substituiert ist,
oder eine Gruppe der Formel -C(=NH)R¹⁰,
worin R¹⁰ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet
oder
R⁸ und R⁹ zusammen eine Gruppe der Formel -(CH₂)ₛ-W-(CH₂)ₜ-darstellen,
in der W eine Kohlenstoff-Kohlenstoff-Einfachbindung, ein Sauerstoffatom, ein Schwefelatom oder eine Gruppe der Formel >NR²² bedeutet, wobei R²² ein Wasserstoffatom oder eine Alkylgruppe mit 1 bis 6 Kohlenstoffatomen darstellt,
und
s und t gleich oder verschieden sind und jeweils 1, 2 oder 3 bedeuten,
R¹¹ ein Wasserstoffatom oder eine Alkylgruppe mit 1 bis 6 Kohlenstoffatomen bedeutet,
R¹² bedeutet:
ein Wasserstoffatom,
eine unsubstituierte Alkylgruppe mit 1 bis 6 Kohlenstoffatomen,
eine substituierte Alkylgruppe mit 1 bis 6 Kohlenstoffatomen, die mit mindestens einem der nachstehend definierten Substituenten (a) substituiert ist,
eine Alkenylgruppe mit 2 bis 6 Kohlenstoffatomen,
eine Alkinylgruppe mit 2 bis 6 Kohlenstoffatomen,
oder eine Gruppe der Formel -C(=NH)R¹³,
wobei R¹³ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet,
R¹⁴ und R¹⁵ gleich oder verschieden sind und jeweils ein Wasserstoffatom oder eine Alkylgruppe mit 1 bis 6 Kohlenstoffatomen bedeuten,
R¹⁶ ein Wasserstoffatom, eine unsubstituierte Alkylgruppe mit 1 bis 6 Kohlenstoffatomen, eine substituierte Alkylgruppe mit 1 bis 6 Kohlenstoffatomen, die mit mindestens einem der nachstehend definierten Substituenten (c) substituiert ist, oder eine Cycloalkylgruppe mit 3 bis 7 Ringkohlenstoffatomen bedeutet,
R¹⁷ oder R¹⁸ gleich oder verschieden sind und jeweils bedeuten:
ein Wasserstoffatom,
eine unsubstituierte Alkylgruppe mit 1 bis 6 Kohlenstoffatomen, oder
eine substituierte Alkylgruppe mit 1 bis 6 Kohlenstoffatomen, die mit mindestens einem der nachstehend definierten Substituenten (a) substituiert ist,
oder
R¹⁷ und R¹⁸ zusammen eine Gruppe der Formel -(CH₂)_{q}-Y-(CH₂)ᵣ-darstellen,
worin Y eine Kohlenstoff-Kohlenstoff-Einfachbindung, ein Sauerstoffatom, ein Schwefelatom oder eine Gruppe der Formel >NR²³ bedeutet, wobei R²³ ein Wasserstoffatom oder eine Alkylgruppe mit 1 bis 6 Kohlenstoffatomen bedeutet, und
q und r gleich oder verschieden sind und jeweils 1, 2 oder 3 bedeuteten,
R¹⁹, R²⁰ und R²¹ gleich oder verschieden sind und jeweils ein Wasserstoffatom oder eine Alkygruppe mit 1 bis 6 Kohlenstoffatomen bedeuten,
Z eine Imidazolyl-, Triazolyl- oder Tetrazolylgruppe darstellt,
d 0 oder 1 ist,
e, f, i, j und k gleich oder verschieden sind und jeweils 1 oder 2 bedeuten,
g, l und m gleich oder verschieden sind und jeweils 0, 1 oder 2 sind und
n und p gleich oder verschieden sind und jeweils 1, 2 oder 3 bedeuten,
mit der Maßgabe daß dann, wenn A eine Gruppe der Formel (A1) darstellt,
nicht alle der Gruppen R² , R³ und R⁴ Wasserstoffatome sind, wenn R¹ ein Wasserstoffatom bedeutet und
nicht alle der Gruppen R¹ , R³ und R⁴ Wasserstoffatome darstellen, wenn R² eine Alkylgruppe bedeutet,
wobei die Substituenten (a) unter
Hydroxygruppen,
Carboxygruppen,
Cyangruppen,
Halogenatomen,
Sauerstoffatomen unter Bildung einer Oxogruppe,
Alkoxygruppen mit 1 bis 6 Kohlenstoffatomen und
Gruppen der Formeln -CO.NR^{a}R^{b}, -OCO.NR^{a}R^{b} und -NR^{a}R^{b} ausgewählt sind, wobei R^{a} und R^{b} wie vorstehend definiert sind,
die Substituenten (b) ausgewählt sind unter
Hydroxygruppen,
Carboxygruppen,
Cyangruppen,
Halogenatomen,
Alkoxygruppen mit 1 bis 6 Kohlenstoffatomen,
Gruppen der Formeln -CO.NR^{a}R^{b}, -OCO.NR^{a}R^{b} und -NR^{a}R^{b}, wobei R^{a} und R^{b} wie vorstehend definiert sind,
Sulfamoylgruppen,
Ureidogruppen,
Sulfogruppen,
Alkanoylgruppen mit 1 bis 6 Kohlenstoffatomen,
Alkanoylaminogruppen mit 1 bis 6 Kohlenstoffatomen,
Alkanoyloxygruppen mit 1 bis 6 Kohlenstoffatomen,
Alkylthiogruppen mit 1 bis 6 Kohlenstoffatomen,
Alkylsulfinylgruppen mit 1 bis 6 Kohlenstoffatomen und
Alkylsulfonylgruppen mit 1 bis 6 Kohlenstoffatomen,
die Substituenten (c) ausgewählt sind unter
Halogenatomen,
Alkoxygruppen mit 1 bis 6 Kohlenstoffatomen,
Cycloalkylgruppen mit 3 bis 7 Ringkohlenstoffatomen und
die Substituenten (d) ausgewählt sind unter
Hydroxygruppen,
Cyangruppen,
Gruppen der Formeln -CO.NR^{a}R^{b}, -OCO.NR^{a}R^{b} und -NR^{a}R^{b} , wobei R^{a} und R^{b} wie vorstehend definiert sind,
Carboxygruppen,
Halogenatomen und
Alkoxygruppen mit 1 bis 6 Kohlenstoffatomen]
oder eines pharmazeutisch geeigneten Salzes oder Esters dieser Verbindung, wobei das Verfahren folgende Stufen umfaßt:
Umsetzen einer Verbindung der Formel (II) 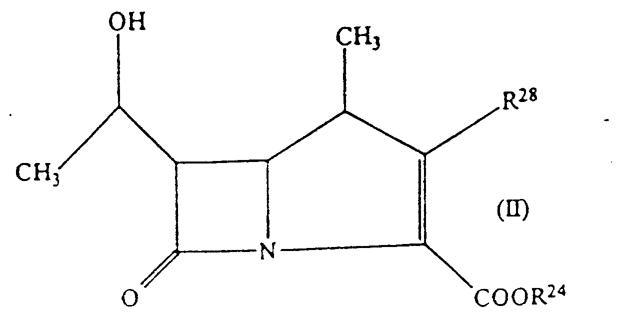 [worin R²⁴ eine Schutzgruppe für die Carboxylgruppe und R²⁸ eine Alkansulfonyloxygruppe, eine Arylsulfonyloxygruppe, eine Dialkylphosphoryloxygruppe, eine Diarylphosphoryloxygruppe oder eine Gruppe der Formel -S(→O)R²⁷ bedeutet, wobei R²⁷ eine Alkylgruppe, eine Halogenalkylgruppe, eine Acetamidoalkylgruppe, eine Acetamidoalkenylgruppe, eine Arylgruppe oder eine aromatische heterocyclische Gruppe bedeutet] mit einer Verbindung der Formel (III) 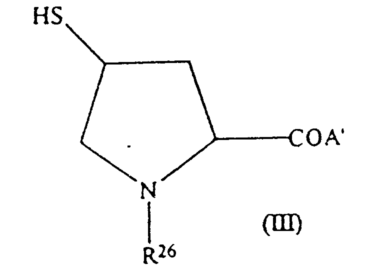 (worin R²⁶ irgendeine der durch R¹ dargestellten Gruppen oder Atome oder eine solche Gruppe oder ein solches Atom bedeutet, worin aktive Gruppen geschützt sind und A' irgendeine der durch A dargestellten Gruppen oder Atome oder irgendeine solche Gruppe oder ein Atom worin aktive Gruppen geschützt sind, bedeutet) und
erforderlichenfalls Entfernen einer vorhandenen Schutzgruppe.

2. Verfahren nach Anspruch 1, bei dem die Reagenzien und die Reaktionsbedingungen so gewählt werden, daß eine Verbindung der Formel (I) oder ein Salz oder Ester davon der der Formel (Ia) hergestellt wird: 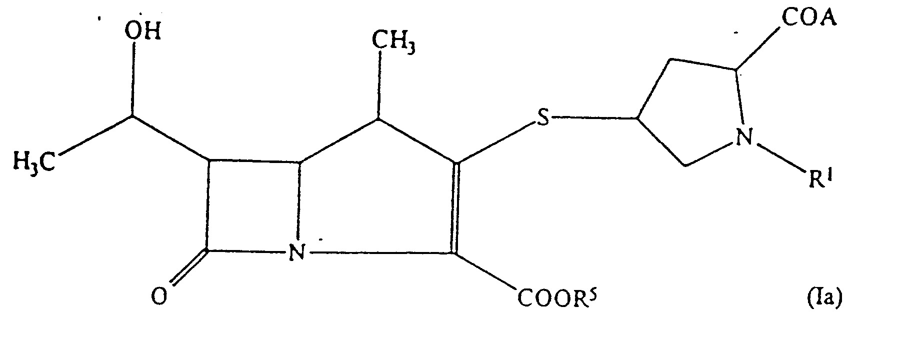 [worin R¹ und A wie in Anspruch 1 definiert sind und R⁵ bedeutet:
eine C₁ - C₂₀-Alkylgruppe,
eine C₃ - C₇-Cycloalkylgruppe,
eine Aralkylgruppe, deren Alkylteil eine C₁ - C₃-Alkylgruppe und deren Arylteil eine C₆ - C₁₄-carbocyclische aromatische Gruppe ist, die substituiert oder unsubstituiert sein kann und, falls sie substituiert ist, mindestens einen der nachstehend definierten Substituenten (e) aufweist, eine Alkenylgruppe, die substituiert oder unsubstituiert ist und, falls sie substituiert ist, mindestens einen der in Anspruch 1 definierten Substituenten (a) aufweist,
eine halogenierte C₁ - C₆-Alkylgruppe,
eine substituierte Silylalkylgruppe, deren Alkylteil 1 bis 6 Kohlenstoffatome enthält und deren Silylgruppe bis zu 3 Substituenten aufweist, die unter C₁ - C₆-Alkylgruppen und Phenylgruppen, die unsubstitiuiert sind oder mindestens einen der nachstehend definierten Substituenten (e) haben, ausgewählt sind,
eine Phenylgruppe, die unsubstituiert ist oder die mindestens einen der nachstehend definierten Substituenten (e) aufweist,
eine Phenacylgruppe, die unsubstituiert ist oder die mindestens einen der nachstehend definierten Substituenten (e) aufweist,
eine cyclische oder acyclische Terpenylgruppe,
eine Alkoxymethylgruppe, deren Alkoxyteil C₁ - C₆ umfaßt,
eine aliphatische Acyloxyalkylgruppe, deren Acylgruppe eine C₂ - C₆-Alkanoylgruppe und deren Alkylteil eine C₂ - C₆-Alkylgruppe darstellt,
eine cycloalkylsubstituierte aliphatische Acyloxyalkylgruppe, deren Acylgruppe eine C₂ - C₆-Alkanoylgruppe ist, deren Cycloalkylsubstituent C₃ - C₇ umfaßt und deren Alkylteil eine C₁ - C₆-Alkylgruppe ist,
eine Alkoxycarbonyloxyalkylgruppe, deren Alkoxyteil C₁ - C₁₀ und deren Alkylteil C₁ - C₆ umfaßt,
eine Cycloalkylcarbonyloxyalkyl- oder Cycloalkyloxycarbonyloxyalkylgruppe, deren Cycloalkylgruppe C₃ - C₁₀ umfaßt, mono- oder polycyclisch ist und unsubstituiert oder mit mindestens einer C₁ - C₄-Alkylgruppe substituiert ist und deren Alkylgruppe C₁ - C₆ umfaßt,
eine Cycloalkylalkoxycarbonyloxyalkylgruppe, deren Alkoxygruppe einen einzigen Cycloalkylsubstituenten aufweist, wobei der Cycloalkylsubstituent C₃ - C₁₀ umfaßt und mono- oder polycyclisch ist,
eine Terpenylcarbonyloxyalkyl- oder Terpenyloxycarbonyloxyalkylgruppe, in der die Alkylgruppe 1 bis 6 Kohlenstoffatome aufweist,
eine 5-Alkyl- oder 5-Phenyl(2-oxo-1,3-dioxolen-4-yl)-alkylgruppe, in der jede Alkylgruppe C₁ - C₆ umfaßt oder
eine Phthalidyl- Indanyl- oder 2-Oxo-4,5,6,7-tetrahydro-1,3-benzodioxolen-4-yl-gruppe, und
wobei die Substituenten (e) unter C₁ - C₄-Alkylgruppen, C₁ - C₄-Alkoxygruppen, C₁ - C₄-Halogenalkylgruppen, C₁ - C₃-Alkylendioxygruppen, Halogenatomen, Cyangruppen und Nitrogruppen ausgewählt sind.]

3. Verfahren nach Anspruch 2, bei dem die Reagenzien und die Reaktionsbedingungen so gewählt werden, daß eine Verbindung der Formel (I) oder ein Salz oder Ester davon hergestellt wird, worin R⁵ ein Wasserstoffatom, eine (5-substituierte 2-Oxo-1,3-dioxolen-4-yl)-methylgruppe, eine 1-Methylcyclohexylcarbonyloxymethyl-Gruppe, eine 1-Isopropoxycarbonyloxyethyl-Gruppe oder eine 1-Cyclohexylcarbonyloxyethyl-Gruppe bedeutet.

4. Verfahren nach einem der Ansprüche 1 bis 3, bei dem die Reagenzien und die Reaktionsbedingungen so gewählt werden, daß eine Verbindung der Formel (I) oder ein Salz oder Ester davon hergestellt wird, worin R¹ bedeutet: ein Wasserstoffatom, eine Alkylgruppe mit 1 bis 3 Kohlenstoffatomen, eine substituierte Alkylgruppe mit 1 bis 3 Kohlenstoffatomen, deren Substituent unter den nachstehend definierten Substituenten (a') ausgewählt ist, eine Alkenylgruppe mit 3 oder 4 Kohlenstoffatomen, eine Alkinylgruppe mit 3 oder 4 Kohlenstoffatomen, eine Formimidoylgruppe oder eine Acetimidoylgruppe, und
wobei die Substituenten (a') unter Hydroxygruppen, Carboxygruppen, Carbamoylgruppen, Carbamoyloxygruppen, Cyangruppen, Halogenatomen, Alkoxygruppen mit 1 bis 3 Kohlenstoffatomen, Aminogruppen und Mono- und Dialkylaminogruppen, in denen die oder jede Alkylgruppe 1 bis 3 Kohlenstoffatome enthält, ausgewählt sind.

5. Verfahren nach einem der Ansprüche 1 bis 4, bei dem die Reagenzien und die Reaktionsbedingungen so gewählt werden, daß eine Verbindung der Formel (I) oder ein Salz oder Ester davon hergestellt wird, worin A eine Gruppe der Formel (A1) bedeutet und n 2 oder 3 ist.

6. Verfahren nach einem der Ansprüche 1 bis 5, bei dem die Reagenzien und die Reaktionsbedingungen so gewählt werden, daß eine Verbindung der Formel (I) oder ein Salz oder Ester davon hergestellt wird, worin R² bedeutet:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter den nachstehend definierten Substituenten (b') ausgewählt ist,
eine Alkenylgruppe mit 3 oder 4 Kohlenstoffatomen,
eine Alkinylgruppe mit 3 oder 4 Kohlenstoffatomen oder
eine Gruppe der Formel -C(=NH)R⁶,
worin R⁶ ein Wasserstoffatom,
eine unsubstituierte Alkylgruppe mit 1 bis 3 Kohlenstoffatomen,
eine substituierte Alkylgruppe mit 1 bis 3 Kohlenstoffatomen, die mit mindestens einem Substituenten substituiert ist, der ausgewählt ist unter Halogenatomen, Alkoxygruppen mit 1 bis 3 Kohlenstoffatomen und Cycloalkylgruppen mit 3 bis 6 Kohlenstoffatomen, oder
eine Cycloalkylgruppe mit 3 bis 6 Ringkohlenstoffatomen bedeutet, und
wobei die Substituenten (b') ausgewählt sind unter Hydroxygruppen, Carboxygruppen, Carbamoylgruppen, Carbamoyloxygruppen, Cyangruppen, Sulfamoylgruppen, Ureidogruppen, Sulfogruppen, Alkoxygruppen mit 1 bis 3 Kohlenstoffatomen, Alkoxycarbonylgruppen mit 2 bis 4 Kohlenstoffatomen, Alkanoylgruppen mit 2 bis 4 Kohlenstoffatomen, Alkanoylaminogruppen mit 2 bis 4 Kohlenstoffatomen, Alkanoyloxygruppen mit 2 bis 4 Kohlenstoffatomen, Aminogruppen, Mono- und Dialkylaminogruppen, in denen die oder jede Alkylgruppe 1 bis 3 Kohlenstoffatome aufweist,
Alkylthiogruppen mit 1 bis 3 Kohlenstoffatomen, Alkylsulfinylgruppen mit 1 bis 3 Kohlenstoffatomen, Alkylsulfonylgruppen mit 1 bis 3 Kohlenstoffatomen, Mono- und Dialkylcarbamoylgruppen, in denen die oder jede Alkylgruppe 1 bis 3 Kohlenstoffatome aufweist und Mono- und Dialkylcarbamoyloxygruppen, in denen die oder jede Alkylgruppe 1 bis 3 Kohlenstoffatome aufweist.

7. Verfahren nach einem der Ansprüche 1 bis 6, bei dem die Reagenzien und die Reaktionsbedingungen so gewählt werden, daß eine Verbindung der Formel (I) oder ein Salz oder Ester davon hergestellt wird, worin A eine Gruppe der Formel (A1) bedeutet und R³ und R⁴ jeweils ein Wasserstoffatom, eine Alkylgruppe mit 1 bis 3 Kohlenstoffatomen, eine Hydroxygruppe, eine Carboxygruppe, eine Carbamoylgruppe oder eine substituierte Alkylgruppe, die 1 bis 3 Kohlenstoffatome aufweist und die mit mindestens einem unter Hydroxygruppen, Alkoxygruppen mit 1 bis 3 Kohlenstoffatomen, Aminogruppen, Carbamoylgruppen und Halogenatomen ausgewählten Substituenten substituiert ist, bedeuten.

8. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A1) bedeutet,
n 2 ist,
R¹ ein Wasserstoffatom, eine Methylgruppe, eine Fluormethylgruppe, eine Formimidoylgruppe oder eine Acetimidoylgruppe bedeutet;
R² ein Wasserstoffatom, eine 2-Hydroxyethylgruppe, eine 2-Carbamoylethylgruppe, eine Carboxymethylgruppe, eine Carbamoylmethylgruppe, eine 2-Fluorethylgruppe, eine Formimidoylgruppe oder eine Acetimidoylgruppe bedeutet und
R³ und R⁴ gleich oder verschieden sind und jeweils ein Wasserstoffatom, eine Methylgruppe, eine Carbamoylgruppe, eine Cyangruppe, eine Carboxygruppe, eine Hydroxymethylgruppe, eine Fluormethylgruppe oder eine Aminomethylgruppe darstellen.

9. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A1) bedeutet,
n für 3 steht,
R¹ ein Wasserstoffatom, eine Methylgruppe, eine Fluormethylgruppe, eine Formimidoylgruppe oder eine Acetimidoylgruppe bedeutet,
R² ein Wasserstoffatom, eine Methylgruppe, eine Formimidoylgruppe, eine Acetimidoylgruppe, eine Carboxymethylgruppe, eine Carbamoylmethylgruppe, eine 2-Hydroxyethylgruppe oder eine 2-Fluorethylgruppe bedeutet und
R³ und R⁴ gleich oder verschieden sind und jeweils ein Wasserstoffatom, eine Methylgruppe, eine Hydroxygruppe, eine Aminogruppe, eine Cyangruppe, eine Carboxygruppe, eine Carbamoylgruppe, eine Carbamoyloxygruppe, eine Hydroxymethylgruppe, eine Fluormethylgruppe oder eine Aminomethylgruppe bedeuten.

10. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A1) bedeutet,
n für 2 steht
R¹ ein Wasserstoffatom, eine Methylgruppe, eine Formimidoylgruppe oder eine Acetimidoylgruppe bedeutet,
R² ein Wasserstoffatom, eine 2-Hydroxyethylgruppe, eine Carboxymethylgruppe, eine Formimidoylgruppe oder eine Acetimidoylgruppe bedeutet,
R³ ein Wasserstoffatom bedeutet, und
R⁴ eine Methylgruppe, eine Carbamoylgruppe, eine Cyangruppe, eine Hydroxymethylgruppe, eine Fluormethylgruppe oder eine Aminomethylgruppe bedeutet.

11. Verfahren mach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A1) bedeutet,
n für 3 steht
R¹ ein Wasserstoffatom, eine Methylgruppe, eine Formimidoylgruppe oder eine Acetimidoylgruppe bedeutet,
R² eine Formimidoylgruppe, eine Acetimidoylgruppe, eine Carboxymethylgruppe, eine 2-Hydroxyethylgruppe oder eine 2-Fluorethylgruppe bedeutet, und
R³ und R⁴ gleich oder verschieden sind und jeweils ein Wasserstoffatom, eine Hydroxygruppe, eine Aminogruppe oder eine Cyangruppe bedeuten.

12. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A2) bedeutet und R⁷ ein Wasserstoffatom, eine Carboxygruppe, eine Carbamoylgruppe, eine Alkylgruppe mit 1 bis 3 Kohlenstoffatomen oder eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carboxygruppen und Cyangruppen ausgewählt ist, bedeutet.

13. Verfahren nach Anspruch 1, bei dem die Reagenzien und die Reaktionsbedingungen so gewählt werden, daß eine Verbindung der Formel (I) oder ein Salz oder Ester davon hergestellt wird, worin A eine Gruppe der Formel (A2) bedeutet und R⁸ ein Wasserstoffatom, eine Alkylgruppe mit 1 bis 3 Kohlenstoffatomen, eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carbamoyloxygruppen, Carboxygruppen, Cyangruppen, Aminogruppen und Halogenatomen ausgewählt ist, eine Alkenylgruppe mit 3 oder 4 Kohlenstoffatomen oder eine Alkinylgruppe mit 3 oder 4 Kohlenstoffatomen bedeutet.

14. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A2) bedeutet und R⁹ bedeutet:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carbamoyloxygruppen, Carboxygruppen, Cyangruppen, Aminogruppen und Halogenatomen ausgewählt ist, oder eine Gruppe der Formel -C(=NH)R¹⁰ , worin R¹⁰ darstellt:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter Alkoxygruppen mit 1 bis 3 Kohlenstoffatomen und Halogenatomen ausgewählt ist,
eine Cycloalkylgruppe mit 3 bis 6 Kohlenstoffatomen
oder eine Alkylgruppe mit 1 bis 3 Kohlenstoffatomen, die mit einer einzigen Cycloalkylgruppe mit 3 bis 6 Kohlenstoffatomen substituiert ist.

15. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A2) bedeutet und R⁸ und R⁹ zusammen eine Gruppe der Formel -(CH₂)ₛ-W-(CH₂)ₜ-, darstellen,
in der W eine Kohlenstoff-Kohlenstoff-Einfachbindung, ein Sauerstoffatom, ein Schwefelatom oder eine Gruppe der Formel >NR²² bedeutet, wobei R²² ein Wasserstoffatom oder eine Alkylgruppe mit 1 bis 3 Kohlenstoffatomen ist, s für 1, 2 oder 3 steht und t 2 ist.

16. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A2) bedeutet,
d 0 oder 1 ist,
m 0, 1 oder 2 ist,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom, eine Alkylgruppe mit 1 bis 3 Kohlenstoffatomen, eine Hydroxygruppe, eine Aminogruppe, eine Cyangruppe, ein Halogenatom, eine Carboxygruppe, eine Carbamoylgruppe oder eine Hydroxymethylgruppe bedeutet,
R⁸ ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Alkenylgruppe mit 3 oder 4 Kohlenstoffatomen, eine Alkinylgruppe mit 3 oder 4 Kohlenstoffatomen, eine 2-Halogenethylgruppe, eine 2-Hydroxyethylgruppe, eine 2-Alkoxyethylgruppe, deren Alkoxyteil 1 bis 3 Kohlenstoffatome enthält, oder eine 2-Aminoethylgruppe bedeutet,
R⁹ ein Wasserstoffatom,
eine Alkylgruppe mit 1 bis 3 Kohlenstoffatomen,
eine Fluormethylgruppe, eine Carbamoylmethylgruppe, eine Carboxymethylgruppe, eine Formimidoylgruppe, eine Acetimidoylgruppe, eine 2-Halogenethylgruppe, eine 2-Hydroxyethylgruppe, eine 2-Alkoxyethylgruppe, deren Alkoxyteil 1 bis 3 Kohlenstoffatome aufweist, oder eine 2-Aminoethylgruppe bedeutet,
oder
R⁸ und R⁹ zusammen eine Gruppe der Formel
-(CH₂)₄-,
-(CH₂)₅-,
-(CH₂)₂O(CH₂)₂-,
-(CH₂)₂S(CH₂)₂-,
-(CH₂)₂NH(CH₂)₂- oder
-(CH₂)₂NCH₃(CH₂)₂-
bedeuten.

17. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A2) bedeutet,
d für 0 steht,
m für 1 oder 2 steht,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom bedeutet,
R⁸ ein Wasserstoffatom, eine Alkylgruppe mit 1 bis 3 Kohlenstoffatomen, eine Carbamoylmethylgruppe, eine Carboxymethylgruppe, eine 2-Fluorethylgruppe oder eine 2-Hydroxyethylgruppe bedeutet und
R⁹ ein Wasserstoffatom, eine Alkylgruppe mit 1 bis 3 Kohlenstoffatomen, eine Formimidoylgruppe, eine Acetimidoylgruppe oder eine 2-Fluorethylgruppe bedeutet.

18. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A3) bedeutet, l für 0, 1 oder 2 steht und R⁷ ein Wasserstoffatom oder eine Alkylgruppe mit 1 bis 3 Kohlenstoffatomen bedeutet.

19. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A3) bedeutet und R¹¹ ein Wasserstoffatom oder eine Alkylgruppe mit 1 bis 3 Kohlenstoffatomen, wie eine Methyl-, Ethyl- oder Propylgruppe, bedeutet.

20. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A3) bedeutet und R¹² darstellt:
ein Wasserstoffatom, eine Alkylgruppe mit 1 bis 3 Kohlenstoffatomen, eine substituierte Alkylgruppe mit 1 bis 3 Kohlenstoffatomen, deren Substituent unter Hydroxygruppen, Alkoxygruppen mit 1 bis 3 Kohlenstoffatomen, Carbamoylgruppen, Carbamoyloxygruppen, Carboxygruppen, Cyangruppen, Aminogruppen und Halogenatomen ausgewählt ist, eine Alkenylgruppe mit 3 oder 4 Kohlenstoffatomen, eine Alkinylgruppe mit 3 oder 4 Kohlenstoffatomen oder eine Gruppe der Formel -C(=NH)R¹³, wobei R¹³ bedeutet:
ein Wasserstoffatom,
eine Alkylgruppe mit 1 bis 3 Kohlenstoffatomen,
eine substituierte Alkylgruppe mit 1 bis 3 Kohlenstoffatomen, deren Substituent unter Alkoxygruppen mit 1 bis 3 Kohlenstoffatomen und Halogenatomen ausgewählt ist,
eine Cycloalkylgruppe mit 3 bis 6 Kohlenstoffatomen oder
eine Alkylgruppe mit 1 bis 3 Kohlenstoffatomen, die mit einer einzigen Cycloalkylgruppe mit 3 bis 6 Kohlenstoffatomen substituert ist.

21. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A3) bedeutet,
l für 0, 1 oder 2 steht,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom bedeutet,
R¹¹ ein Wasserstoffatom oder eine Alkylgruppe mit 1 bis 3 Kohlenstoffatomen bedeutet und
R¹² ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Alkenylgruppe mit 3 oder 4 Kohlenstoffatomen, eine Alkinylgruppe mit 3 oder 4 Kohlenstoffatomen, eine Formimidoylgruppe, eine Acetimidoylgruppe, eine 2-Halogenethylgruppe, eine 2-Hydroxyethylgruppe, eine 2-Alkoxyethylgruppe, deren Alkoxyteil 1 bis 3 Kohlenstoffatome aufweist, oder eine Aminoethylgruppe bedeutet.

22. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin:
A eine Gruppe der Formel (A3) bedeutet,
l für 1 oder 2 steht,
R¹ ein Wasserstoffatom, eine Methylgruppe, eine Fluormethylgruppe, eine Formimidoylgruppe oder eine Acetimidoylgruppe bedeutet,
R⁷ ein Wasserstoffatom bedeutet,
R¹¹ ein Wasserstoffatom oder eine Methylgruppe bedeutet und
R¹² ein Wasserstoffatom, eine Alkylgruppe mit 1 bis 3 Kohlenstoffatomen, eine Fluormethylgruppe, eine Carbamoylmethylgruppe, eine Carboxymethylgruppe, eine Formimidoylgruppe, eine Acetimidoylgruppe, eine 2-Fluorethylgruppe oder eine 2-Hydroxyethylgruppe bedeutet.

23. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A4) bedeutet und R¹⁴ und R¹⁵ jeweils ein Wasserstoffatom oder eine Alkylgruppe mit 1 bis 3 Kohlenstoffatomen bedeuten.

24. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A4) bedeutet und R¹⁶ ein Wasserstoffatom, eine Alkylgruppe mit 1 bis 3 Kohlenstoffatomen, eine substituierte Alkylgruppe mit 1 bis 3 Kohlenstoffatomen, deren Substituent unter Alkoxygruppen mit 1 bis 3 Kohlenstoffatomen und Halogenatomen ausgewählt ist, eine Cycloalkylgruppe mit 3 bis 6 Kohlenstoffatomen oder eine Alkylgruppe mit 1 bis 3 Kohlenstoffatomen, die mit einer einzigen Cycloalkylgruppe mit 3 bis 6 Kohlenstoffatomen substituiert ist, bedeutet.

25. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A4) bedeutet,
i für 1 oder 2 steht und
R¹, R¹⁴, R¹⁵ und R¹⁶ gleich oder verschieden sind und jeweils ein Wasserstoffatom oder eine Alkylgruppe mit 1 bis 3 Kohlenstoffatomen bedeuten.

26. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin:
A eine Gruppe der Formel (A4) bedeutet,
i für 1 steht und
R¹, R¹⁴, R¹⁵ und R¹⁶ gleich oder verschieden sind und jeweils ein Wasserstoffatom oder eine Methylgruppe bedeuten.

27. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A5) bedeutet und R¹⁷ und R¹⁸ jeweils ein Wasserstoffatom, eine Alkylgruppe mit 1 bis 3 Kohlenstoffatomen oder eine substituierte Alkylgruppe mit 1 bis 3 Kohlenstoffatomen, deren Substituent unter Hydroxygruppen, Alkoxygruppen mit 1 bis 3 Kohlenstoffatomen und Halogenatomen ausgewählt ist, bedeutet.

28. Verbindung nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A5) bedeutet und R¹⁷ und R¹⁸ zusammen eine Gruppe der Formel -(CH₂)_{q}-Y-(CH₂)ᵣ- darstellen, in der Y eine KohlenstoffKohlenstoff-Einfachbindung, ein Sauerstoffatom oder eine Gruppe der Formel >NR²³ darstellt, wobei R²³ ein Wasserstoffatom oder eine Alkylgruppe mit 1 bis 3 Kohlenstoffatomen bedeutet, und q und r jeweils 2 oder 3 bedeuten.

29. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A5) bedeutet,
p für 2 steht,
R¹ ein Wasserstoffatom, eine Methylgruppe, eine Fluormethylgruppe, eine Formimidoylgruppe oder eine Acetimidoylgruppe bedeutet,
R¹⁷ und R¹⁸ gleich oder verschieden sind und jeweils ein Wasserstoffatom, eine Alkylgruppe mit 1 bis 3 Kohlenstoffatomen, eine 2-Halogenethylgruppe oder eine 2-Hydroxyethylgruppe bedeuten,
oder
R¹⁷ und R¹⁸ zusammen eine Gruppe der Formel
-(CH₂)₄-,
-(CH₂)₅-,
-(CH₂)₂O(CH₂)₂-,
-(CH₂)₂S(CH₂)₂-,
-(CH₂)₂NH(CH₂)₂- oder
-(CH₂)₂NCH₃(CH₂)₂-
darstellen.

30. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A5) bedeutet,
p für 2 steht,
R¹ ein Wasserstoffatom, eine Methylgruppe, eine Fluormethylgruppe, eine Formimidoylgruppe oder eine Acetimidoylgruppe bedeutet, und
R¹⁷ und R¹⁸ gleich oder verschieden sind und jeweils ein Wasserstoffatom oder eine Methylgruppe bedeuten.

31. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A6) bedeutet und R¹⁹ ein Wasserstoffatom oder eine Alkylgruppe mit 1 bis 3 Kohlenstoffatomen bedeutet.

32. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A6) bedeutet,
beide Symbole j und k für 2 stehen,
R¹ ein Wasserstoffatom, eine Methylgruppe, eine Fluormethylgruppe, eine Formimidoylgruppe oder eine Acetimidoylgruppe bedeutet, und
R¹⁹ ein Wasserstoffatom oder eine Alkylgruppe mit 1 bis 3 Kohlenstoffatomen bedeutet.

33. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A6) bedeutet,
beide Symbole j und k für 2 stehen, und
R¹ und R¹⁹ gleich oder verschieden sind und jeweils ein Wasserstoffatom oder eine Methylgruppe bedeuten.

34. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A7) bedeutet und Z eine 1-Imidazolylgruppe, eine 1,2,4-Triazol-1-yl-Gruppe oder eine 1,2,3-Triazol-1-yl-Gruppe bedeutet.

35. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A7) bedeutet,
g für 0, 1 oder 2 steht,
R¹ ein Wasserstoffatom oder eine Methylgruppe bedeutet und
Z eine 1-Imidazolylgruppe, eine 1,2,4-Triazol-1-yl-Gruppe oder eine 1,2,3-Triazol-1-yl-Gruppe bedeutet.

36. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A7) bedeutet,
g für 1 oder 2 steht,
R¹ ein Wasserstoffatom oder eine Methylgruppe bedeutet und
Z eine 1-Imidazolylgruppe, eine 1,2,4-Triazol-1-yl-Gruppe oder eine 1,2,3-Triazol-1-yl-Gruppe bedeutet.

37. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin A eine Gruppe der Formel (A8) bedeutet und R²⁰ und R²¹ jeweils ein Wasserstoffatom oder eine Alkylgruppe mit 1 bis 3 Kohlenstoffatomen bedeuten.

38. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A8) bedeutet,
beide Symbole e und f für 1 stehen,
R¹ ein Wasserstoffatom, eine Methylgruppe, eine Fluormethylgruppe, eine Formimidoylgruppe oder eine Acetimidoylgruppe bedeutet, und
R²⁰ und R²¹ jeweils ein Wasserstoffatom oder eine Alkylgruppe mit 1 bis 3 Kohlenstoffatomen bedeuten.

39. Verfahren nach Anspruch 1 oder Anspruch 2, bei dem die Reagenzien und die Reaktionsbedingungen so gewählt werden, daß eine Verbindung der Formel (I) oder ein Salz oder Ester davon hergestellt wird, worin
A eine Gruppe der Formel (A8) bedeutet,
beide Symbole e und f für 1 stehen,
R¹ ein Wasserstoffatom oder eine Methylgruppe bedeutet,
R²⁰ ein Wasserstoffatom bedeutet und
R²¹ ein Wasserstoffatom oder eine Methylgruppe bedeutet.

40. Verfahren nach einem der vorhergehenden Patentansprüche, bei dem die Reagenzien und die Reaktionsbedingungen so gewählt werden, daß eine Verbindung der Formel (I) oder ein Salz oder Ester davon hergestellt wird, worin die Kohlenstoffatome in den gleichen Konfigurationen wie die des Thienamycins vorliegen.

41. Verfahren nach Anspruch 1, bei dem die Reagenzien und die Reaktionsbedingungen so gewählt werden, daß die folgenden Verbindungen entstehen:
2-[2-(1-Homopiperazinylcarbonyl)-1-methylpyrrolidin-4-ylthio]-6-(1-hydroxyethyl)-1-methyl-1-carbapen-2-em-3-carbonsäure;
2-[2-(4-Carboxymethylhomopiperazin-1-ylcarbonyl)-pyrrolidin-4-ylthio]-6-(1-hydroxyethyl)-1-methyl-1-carbapen-2-em-3-carbonsäure;
2-{2-[4-(2-Hydroxyethyl)homopiperazin-1-ylcarbonyl]-pyrrolidin-4-ylthio}-6-(1-hydroxyethyl)-1-methyl-1-carbapen-2-em-3-carbonsäure;
2-[2-(4-Acetimidoylhomopiperazin-1-ylcarbonyl)-pyrrolidin-4-ylthio]-6-(1-hydroxyethyl)-1-methyl-1-carbapen-2-em-3-carbonsäure;
2-[2-(4-Formimidoylhomopiperazin-1-ylcarbonyl)-pyrrolidin-4-ylthio]-6-(1-hydroxyethyl)-1-methyl-1-carbapen-2-em-3-carbonsäure;
2-[2-(4-Formimidoylhomopiperazin-1-ylcarbonyl)-1-methylpyrrolidin-4-ylthio]-6-(1-hydroxyethyl)-1-methyl-1-carbapen-2-em-3-carbonsäure;
2-[1-Methyl-2-(piperazin-1-ylcarbonyl)pyrrolidin-4-ylthio]-6-(1-hydroxyethyl)-1-methyl-1-carbapen-2-em-3-carbonsäure;
2-{2-[4-(2-Hydroxyethyl)piperazin-1-ylcarbonyl]-pyrrolidin-4-ylthio}-6-(1-hydroxyethyl)-1-methyl-1-carbapen-2-em-3-carbonsäure;
2-[2-(3-Methylpiperazin-1-ylcarbonyl)-pyrrolidin-4-ylthio]-6-(1-hydroxyethyl)-1-methyl-1-carbapen-2-em-3-carbonsäure;
2-[2-(4-Formimidoylpiperazin-1-ylcarbonyl)-pyrrolidin-4-ylthio]-6-(1-hydroxyethyl)-1-methyl-1-carbapen-2-em-3-carbonsäure;
2-[2-(4-Acetimidoylpiperazin-1-ylcarbonyl)-pyrrolidin-4-ylthio]-6-(1-hydroxyethyl)-1-methyl-1-carbapen-2-em-3-carbonsäure;
2-[2-(4-Formimidoylpiperazin-1-ylcarbonyl)-1-methylpyrrolidin-4-ylthio]-6-(1-hydroxyethyl)-1-methyl-1-carbapen-2-em-3-carbonsäure;
2-[2-(4-Acetimidoylpiperazin-1-ylcarbonyl)-1-methylpyrrolidin-4-ylthio]-6-(1-hydroxyethyl)-1-methyl-1-carbapen-2-em-3-carbonsäure;
2-[2-(4-Formimidoyl-3-methylpiperazin-1-ylcarbonyl)-pyrrolidin-4-ylthio]-6-(1-hydroxyethyl)-1-methyl-1-carbapen-2-em-3-carbonsäure;
2-[2-(4-Acetimidoyl-3-methylpiperazin-1-ylcarbonyl)-pyrrolidin-4-ylthio]-6-(1-hydroxyethyl)-1-methyl-1- carbapen-2-em-3-carbonsäure;
2-[2-(2-Methylpiperazin-1-ylcarbonyl)-pyrrolidin-4-yl thio]-6-(1-hydroxyethyl)-1-methyl-1-carbapen-2-em-3-carbonsäure;
2-[2-(4-Formimidoyl-2-methylpiperazin-1-ylcarbonyl)-pyrrolidin-4-ylthio]-6-(1-hydroxyethyl)-1-methyl-1-carbapen-2-em-3-carbonsäure;
2-[2-(4-Acetimidoyl-2-methylpiperazin-1-ylcarbonyl)-pyrrolidin-4-ylthio]-6-(1-hydroxyethyl)-1-methyl-1-carbapen-2-em-3-carbonsäure;
2-[2-(3-Hydroxymethylpiperazin-1-ylcarbonyl)-pyrrolidin-4-ylthio]-6-(1-hydroxyethyl)-1-methyl-1-carbapen-2-em-3-carbonsäure;
2-[1-Formimidoyl-2-(4-formimidoylpiperazin-1-ylcarbonyl)-pyrrolidin-4-ylthio]-6-(1-hydroxyethyl)-1-methyl-1-carbapen-2-em-3-carbonsäure;
2-[2-(3-Acetimidoylaminopyrrolidin-1-ylcarbonyl)-pyrrolidin-4-ylthio]-6-(1-hydroxyethyl)-1-methyl-1-carbapen-2-em-3-carbonsäure;
2-[2-(3-Formimidoylaminopyrrolidin-1-ylcarbonyl)-pyrrolidin-4-ylthio]-6-(1-hydroxyethyl)-1-methyl-1-carbapen-2-em-3-carbonsäure;
2-[2-(3-Aminopyrrolidin-1-ylcarbonyl)-pyrrolidin-4-ylthio]-6-(1-hydroxyethyl)-1-methyl-1-carbapen-2-em-3-carbonsäure;
2-[2-(4-Acetimidoylaminopiperidin-1-ylcarbonyl)-pyrrolidin-4-ylthio]-6-(1-hydroxyethyl)-1-methyl-1-carbapen-2-em-3-carbonsäure;
2-[2-(3-Aminopyrrolidin-1-ylcarbonyl)-1-methylpyrrolidin-4-ylthio]-6-(1-hydroxyethyl)-1-methyl-1-carbapen-2-em-3-carbonsäure;
2-[2-(3-Acetimidoylaminopyrrolidin-1-ylcarbonyl)-1-methylpyrrolidin-4-ylthio]-6-(1-hydroxyethyl)-1-methyl-1-carbapen-2-em-3-carbonsäure;
2-[2-(3-Formimidoylaminopyrrolidin-1-ylcarbonyl)-1-methylpyrrolidin-4-ylthio]-6-(1-hydroxyethyl)-1-methyl-1-carbapen-2-em-3-carbonsäure;
2-[2-(4-Acetimidoylaminopiperidin-1-ylcarbonyl)-1-methylpyrrolidin-4-ylthio]-6-(1-hydroxyethyl)-1-methyl-1-carbapen-2-em-3-carbonsäure;
2-[2-(1-Formimidoylpyrrolidin-3-ylcarbamoyl)pyrrolidin-4-ylthio]-6-(1-hydroxyethyl)-1-methyl-1-carbapen-2-em-3-carbonsäure;
2-[2-(3-Dimethylamino-1,2,5,6-tetrahydropyrazin-1-ylcarbonyl)pyrrolidin-4-ylthio]-6-(1-hydroxyethyl)-1-methyl-1-carbapen-2-em-3-carbonsäure;
und deren pharmazeutisch geeignete Salze und Ester.

42. Verfahren zur Herstellung einer Arzneimittelzusammensetzung, welches das Vermischen eines pharmazeutisch geeigneten Trägermaterials, Verdünnungsmittels oder Adjuvans mit einem Antibiotikum umfaßt, wobei das Antibiotikum unter Verbindungen der Formel (I) und pharmazeutisch geeigneten Salzen und Estern davon gemäß einem der Ansprüche 1 bis 41 ausgewählt ist.

43. Verwendung einer Verbindung der Formel (I) oder eines pharmazeutisch geeigneten Salzes oder Esters davon nach einem der Ansprüche 1 bis 41 zur Herstellung eines Arzneimittels zur Behandlung oder Prophylaxe von bakteriellen Infektionen.

## Revendications (Revendications pour l'(les) Etat(s) contractant(s) suivant(s): AT, BE, CH, DE, DK, FR, GB, LI, LU, MC, NL, PT, SE)

1. Composé de formule (I): 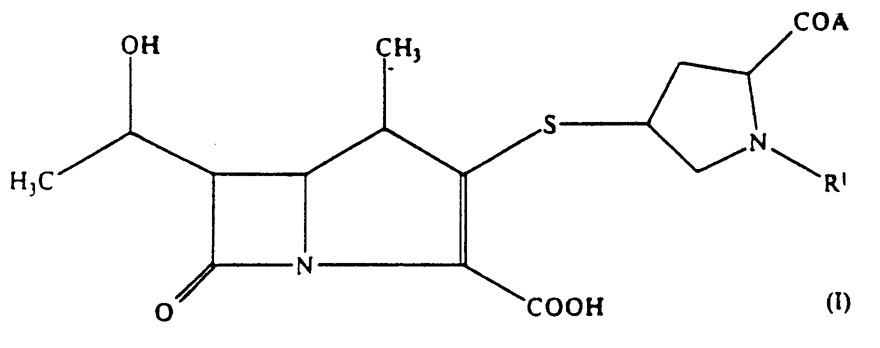 dans laquelle
R¹ représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous,
un groupe alcényle ayant de 2 à 6 atomes de carbone,
un groupe alcynyle ayant de 2 à 6 atomes de carbone, ou
un groupe de formule -C(=NH)R°, dans laquelle R° représente un atome d'hydrogène ou un groupe alkyle ayant de 1 à 6 atomes de carbone; et
A représente un groupe de formule (A1), (A2), (A3), (A4), (A5), (A6), (A7) ou (A8): 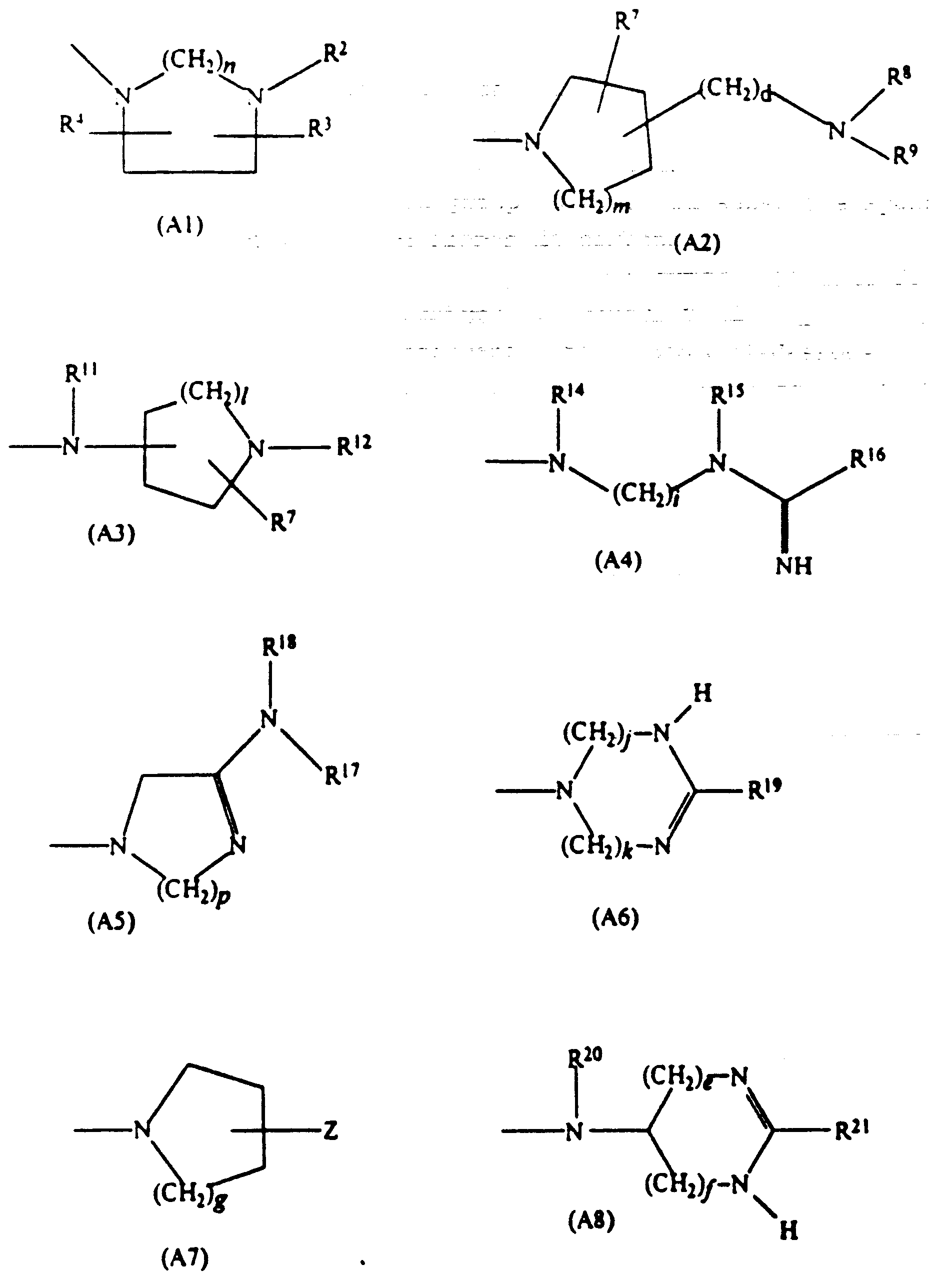 formules dans lesquelles:
R² représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (b) définis ci-dessous,
un groupe alcényle ayant de 2 à 6 atomes de carbone,
un groupe alcynyle ayant de 2 à 6 atomes de carbone, ou
un groupe de formule -C(=NH)R⁶,
dans laquelle R⁶ représente un atome d'hydrogène, un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
R³, R⁴ et R⁷ sont identiques ou différents et représentent chacun
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (d) définis ci-dessous,
un atome d'halogène,
le groupe hydroxyle,
le groupe carboxyle,
un groupe de formule -CO.NR^{a}R^{b}, -OCO.NR^{a}R^{b} ou -NR^{a}R^{b},
formules dans lesquelles R^{a} et R^{b} sont identiques ou différents et représentent chacun un atome d'hydrogène ou un groupe alkyle ayant de 1 à 4 atomes de carbone, ou
le groupe cyano;
R⁸ représente
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous,
un groupe alcényle ayant de 2 à 6 atomes de carbone, ou
un groupe alcynyle ayant de 2 à 6 atomes de carbone;
R⁹ représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous, ou
un groupe de formule -C(=NH)R¹⁰,
dans laquelle R¹⁰ représente un atome d'hydrogène ou un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
ou
R⁸ et R⁹ représentent ensemble un groupe de formule -(CH₂)ₛ-W-(CH₂)ₜ-
dans laquelle W représente une liaison carbone-carbone simple, un atome d'oxygène ou de soufre, ou un groupe de formule 〉NR²², dans laquelle R²² représente un atome d'hydrogène ou un groupe alkyle ayant de 1 à 6 atomes de carbone, et
s et t sont identiques ou différents et représentent chacun 1, 2 ou 3;
R¹¹ représente un atome d'hydrogène ou un groupe alkyle ayant de 1 à 6 atomes de carbone;
R¹² représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous,
un groupe alcényle ayant de 2 à 6 atomes de carbone,
un groupe alcynyle ayant de 2 à 6 atomes de carbone, ou
un groupe de formule -C(=NH)R¹³,
dans laquelle R¹³ représente un atome d'hydrogène ou un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
R¹⁴ et R¹⁵ sont identiques ou différents et représentent chacun un atome d'hydrogène ou un groupe alkyle ayant de 1 à 6 atomes de carbone;
R¹⁶ représente un atome d'hydrogène ou un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
R¹⁷ et R¹⁸ sont identiques ou différents et représentent chacun
un atome d'hydrogène,
un groupe alkyle non substitué ayant de 1 à 6 atomes de carbone, ou
un groupe alkyle substitué ayant de 1 à 6 atomes de carbone et étant substitué par au moins un des substituants (a) définis ci-dessous;
ou
R¹⁷ et R¹⁸ représentent ensemble un groupe de formule -(CH₂)_{q}-Y-(CH₂)ᵣ-,
dans laquelle Y représente une liaison carbone-carbone simple, un atome d'oxygène ou de soufre, ou un groupe de formule 〉NR²³, dans laquelle R²³ représente un atome d'hydrogène ou un groupe alkyle ayant de 1 à 6 atomes de carbone, et
q et r sont identiques ou différents et représentent chacun 1, 2 ou 3;
R¹⁹, R²⁰ et R²¹ sont identiques ou différents et représentent chacun un atome d'hydrogène ou un groupe alkyle ayant de 1 à 6 atomes de carbone;
Z représente le groupe imidazolyle, triazolyle ou tétrazolyle;
d est 0 ou 1;
e, f, i, j et k sont identiques ou différents et représentent chacun 1 ou 2;
g, ℓ et m sont identiques ou différents et représentent chacun 0, 1 ou 2; et
n et p sont identiques ou différents et représentent chacun 1, 2 ou 3;
étant entendu que lorsque A représente un groupe de formule (A1):
R², R³ et R⁴ ne représentent pas tous des atomes d'hydrogène lorsque R¹ représente un atome d'hydrogène; et
R¹, R³ et R⁴ ne représentent pas tous des atomes d'hydrogène lorsque R² représente un groupe alkyle;
lesdits substituants (a) sont choisis parmi:
le groupe hydroxyle,
le groupe carboxyle,
le groupe cyano,
des atomes d'halogène,
des atomes d'oxygène pour la formation d'un groupe oxo,
des groupes alcoxy ayant de 1 à 6 atomes de carbone, et
des groupes de formule -CO.NR^{a}R^{b}, -OCO.NR^{a}R^{b} et -NR^{a}R^{b}, R^{a} et R^{b} étant tels que définis plus haut;
lesdits substituants (b) sont choisis parmi:
le groupe hydroxyle,
le groupe carboxyle,
le groupe cyano,
des atomes d'halogène,
des groupes alcoxy ayant de 1 à 6 atomes de carbone,
des groupes de formule -CO.NR^{a}R^{b}, -OCO.NR^{a}R^{b} et -NR^{a}R^{b}, R^{a} et R^{b} étant tels que définis plus haut,
les groupes sulfamoyle,
uréido,
sulfo,
des groupes alcanoyle ayant de 1 à 6 atomes de carbone,
des groupes alcanoylamino ayant de 1 à 6 atomes de carbone,
des groupes alcanoyloxy ayant de 1 à 6 atomes de carbone,
des groupes alkylthio ayant de 1 à 6 atomes de carbone,
des groupes alkylsulfinyle ayant de 1 à 6 atomes de carbone, et
des groupes alkylsulfonyle ayant de 1 à 6 atomes de carbone;
lesdits substituants (c) sont choisis parmi:
des atomes d'halogène,
des groupes alcoxy ayant de 1 à 6 atomes de carbone,
des groupes cycloalkyle ayant de 3 à 7 atomes de carbone formant le cycle; et
lesdits substituants (d) sont choisis parmi:
le groupe hydroxyle,
le groupe cyano,
des groupes de formule -CO.NR^{a}R^{b}, -OCO.NR^{a}R^{b} et -NR^{a}R^{b}, R^{a} et R^{b} étant tels que définis plus haut;
le groupe carboxyle,
des atomes d'halogène, et
des groupes alcoxy ayant de 1 à 6 atomes de carbone;
et ses sels et esters pharmaceutiquement acceptables.

2. Composé selon la revendication 1, correspondant à la formule (Ia): 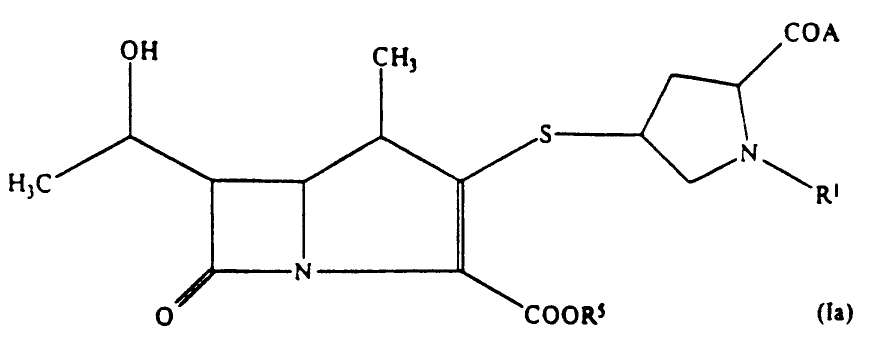 dans laquelle R¹ et A sont tels que définis dans la revendication 1, et R⁵ représente:
un groupe alkyle en C₁-C₂₀,
un groupe cycloalkyle en C₃-C₇,
un groupe aralkyle dans lequel le fragment alkyle est
un groupe alkyle en C₁-C₃ et le fragment aryle est un groupe aromatique carbocyclique en C₆-C₁₄ qui peut être substitué ou non substitué, et, s'il est substitué, comporte au moins un des substituants (e) définis ci-dessous,
un groupe alcényle qui est substitué ou non substitué, et, s'il est substitué, comporte au moins un des substituants (a) définis dans la revendication 1,
un groupe alkyle en C₁-C₆ halogéné,
des groupes silylalkyle subtitués, dans lesquels le fragment alkyle a de 1 à 6 atomes de carbone et le fragment silyle porte jusqu'à 3 substituants choisis parmi des groupes alkyle en C₁-C₆ et des groupes phényle qui ne sont pas substitués ou portent au moins un des substituants (e) définis ci-dessous;
un groupe phényle qui n'est pas substitué ou porte au moins un des substituants (e) définis ci-dessous;
un groupe phénacyle qui n'est pas substitué ou porte au moins un des substituants (e) définis ci-dessous;
un groupe terpényle cyclique ou acyclique;
un groupe alcoxyméthyle dans lequel le fragment alcoxy est en C₁-C₆;
un groupe acyloxyalkyle aliphatique dans lequel le fragment acyle est un groupe alcanoyle en C₂-C₆ et le fragment alkyle est un groupe alkyle en C₂-C₆;
un groupe acyloxyalkyle aliphatique substitué par un radical cycloalkyle, dans lequel le fragment acyle est un groupe alcanoyle en C₂-C₆, le substituant cycloalkyle est en C₃-C₇, et le fragment alkyle est un groupe alkyle en C₁-C₆;
un groupe alcoxycarbonyloxyalkyle dans lequel le fragment alcoxy est en C₁-C₁₀ et le fragment alkyle est en C₁-C₆;
un groupe cycloalkylcarbonyloxyalkyle ou cycloalkyloxycarbonyloxyalkyle, dans lequel le fragment cycloalkyle est en C₃-C₁₀, est mono- ou polycyclique et n'est pas substitué ou est substitué par au moins un groupe alkyle en C₁-C₄, et le fragment alkyle est en C₁-C₆;
un groupe cycloalkylalcoxycarbonyloxyalkyle dans lequel le fragment alcoxy porte un seul substituant cycloalkyle, le substituant cycloalkyle étant en C₃-C₁₀ et mono- ou polycyclique;
un groupe terpénylcarbonyloxyalkyle ou terpényloxycarbonyloxyalkyle dans lequel le groupe alkyle a de 1 à 6 atomes de carbone;
un groupe 5-alkyl- ou 5-phényl-(2-oxo-1,3-dioxolène-4-yl)alkyle dans lequel chaque radical alkyle est en C₁-C₆; ou
le groupe phtalidyle, indanyle ou 2-oxo-4,5,6,7-tétrahydro-1,3-benzodioxolène-4-yle; et
les substituants (e) sont choisis parmi des groupes alkyle en C₁-C₄, alcoxy en C₁-C₄, halogénoalkyle en C₁-C₄, alkylènedioxy en C₁-C₃, des atomes d'halogène, les groupes cyano et nitro.

3. Composé selon la revendication 2, dans lequel R⁵ représente un atome d'hydrogène ou un groupe (5-substitué-2-oxo-1,3-dioxolène-4-yl)méthyle, 1-méthylcyclohexylcarbonyloxyméthyle, 1-isopropoxycarbonyloxyéthyle ou 1-cyclohexylcarbonyloxyéthyle.

4. Composé selon l'une quelconque des revendications 1 à 3, dans lequel R¹ représente un atome d'hydrogène ou un groupe alkyle ayant de 1 à 3 atomes de carbone; un groupe alkyle substitué ayant de 1 à 3 atomes de carbone, dans lequel le substituant est choisi parmi les substituants (a') définis ci-dessous; un groupe alcényle ayant 3 ou 4 atomes de carbone; un groupe alcynyle ayant 3 ou 4 atomes de carbone; le groupe formimidoyle ou acétimidoyle; et
les substituants (a') sont choisis parmi les groupes hydroxyle, carboxyle, carbamoyle, carbamoyloxy, cyano, des atomes d'halogène, des groupes alcoxy ayant de 1 à 3 atomes de carbone, le groupe amino et des groupes mono- et dialkylamino dans lesquels le ou chaque groupe alkyle a de 1 à 3 atomes de carbone.

5. Composé selon l'un quelconque des revendications 1 à 4, dans lequel A représente un groupe de formule (A1) et n est égal à 2 ou 3.

6. Composé selon l'une quelconque des revendications 1 à 5, dans lequel R² représente:
un atome d'hydrogène;
un groupe alkyle ayant de 1 à 3 atomes de carbone;
un groupe alkyle substitué ayant de 1 à 3 atomes de carbone, dans lequel le substituant est choisi parmi les substituants (b') définis ci-dessous;
un groupe alcényle ayant 3 ou 4 atomes de carbone;
un groupe alcynyle ayant 3 ou 4 atomes de carbone; ou
un groupe de formule -C(=NH)R⁶,
dans laquelle R⁶ représente:
un atome d'hydrogène,
un groupe alkyle non substitué ayant de 1 à 3 atomes de carbone,
un groupe alkyle substitué ayant de 1 à 3 atomes de carbone et étant substitué par au moins un substituant choisi parmi des atomes d'halogène et des groupes alcoxy ayant de 1 à 3 atomes de carbone, et des groupes cycloalkyle ayant de 3 à 6 atomes de carbone, ou
un groupe cycloalkyle ayant de 3 à 6 atomes de carbone formant le cycle; et
les substituants (b') sont choisis parmi les groupes hydroxyle, carboxyle, carbamoyle, carbamoyloxy, cyano, sulfamoyle, uréido, sulfo, des groupes alcoxy ayant de 1 à 3 atomes de carbone, alcoxycarbonyle ayant de 2 à 4 atomes de carbone, alcanoyle ayant de 2 à 4 atomes de carbone, alcanoylamino ayant de 2 à 4 atomes de carbone, alcanoyloxy ayant de 2 à 4 atomes de carbone, amino, mono- et dialkylamino dans lesquels le ou chaque fragment alkyle a de 1 à 3 atomes de carbone, des groupes alkylthio ayant de 1 à 3 atomes de carbone, alkylsulfinyle ayant de 1 à 3 atomes de carbone, alkylsulfonyle ayant de 1 à 3 atomes de carbone, des groupes mono- et dialkylcarbamoyle dans lesquels le ou chaque groupe alkyle a de 1 à 3 atomes de carbone, et des groupes mono- et dialkylcarbamoyloxy dans lesquels le ou chaque groupe alkyle a de 1 à 3 atomes de carbone.

7. Composé selon l'une quelconque des revendications 1 à 6, dans lequel A représente un groupe de formule (A1), et R³ et R⁴ représentent chacun un atome d'hydrogène ou un groupe alkyle ayant de 1 à 3 atomes de carbone, le groupe hydroxyle, carboxyle, carbamoyle ou un groupe alkyle substitué ayant de 1 à 3 atomes de carbone et portant au moins un substituant choisi parmi des groupes hydroxyle, alcoxy ayant de 1 à 3 atomes de carbone, amino, carbamoyle et des atomes d'halogène.

8. Composé selon la revendication 1 ou 2, dans lequel:
A représente un groupe de formule (A1);
n est égal à 2;
R¹ représente un atome d'hydrogène ou le groupe méthyle, fluorométhyle, formimidoyle ou acétimidoyle;
R² représente un atome d'hydrogène ou le groupe 2-hydroxyéthyle, 2-carbamoyléthyle, carboxyméthyle, carbamoylméthyle, 2-fluoroéthyle, formimidoyle ou acétimidoyle; et
R³ et R⁴ sont identiques ou différents et représentent chacun un atome d'hydrogène ou le groupe méthyle, carbamoyle, cyano, carboxyle, hydroxyméthyle, fluorométhyle ou aminométhyle.

9. Composé selon la revendication 1 ou 2, dans lequel:
A représente un groupe de formule (A1);
n est égal à 3;
R¹ représente un atome d'hydrogène ou le groupe méthyle, fluorométhyle, formimidoyle ou acétimidoyle;
R² représente un atome d'hydrogène ou le groupe méthyle, formimidoyle, acétimidoyle, carboxyméthyle, carbamoylméthyle, 2-hydroxyéthyle ou 2-fluoroéthyle; et
R³ et R⁴ sont identiques ou différents et représentent chacun un atome d'hydrogène ou le groupe méthyle, hydroxyle amino, cyano, carboxyle, carbamoyle, carbamoyloxy, hydroxyméthyle, fluorométhyle ou aminométhyle.

10. Composé selon la revendication 1 ou 2, dans lequel:
A représente un groupe de formule (A1);
n est égal à 2;
R¹ représente un atome d'hydrogène ou le groupe méthyle, formimidoyle ou acétimidoyle;
R² représente un atome d'hydrogène ou le groupe 2-hydroxyéthyle, carboxyméthyle, formimidoyle ou acétimidoyle;
R³ représente un atome d'hydrogène; et
R⁴ représente le groupe méthyle, carbamoyle, cyano, hydroxyméthyle, fluorométhyle ou aminométhyle.

11. Composé selon la revendication 1 ou 2, dans lequel:
A représente un groupe de formule (A1);
n est égal à 3;
R¹ représente un atome d'hydrogène ou le groupe méthyle, formimidoyle ou acétimidoyle;
R² représente le groupe formimidoyle, acétimidoyle, carboxyméthyle, 2-hydroxyéthyle ou 2-fluoroéthyle; et
R³ et R⁴ sont identiques ou différents et représentent chacun un atome d'hydrogène ou le groupe hydroxyle, amino ou cyano.

12. Composé selon la revendication 1 ou 2, dans lequel:
A représente un groupe de formule (A2) et R⁷ représente un atome d'hydrogène ou le groupe carboxyle ou carbamoyle, ou un groupe alkyle ayant de 1 à 3 atomes de carbone, ou un groupe alkyle substitué ayant de 1 à 3 atomes de carbone, dans lequel le substituant est choisi parmi des groupes hydroxyle, alcoxy ayant de 1 à 3 atomes de carbone, carbamoyle, carboxyle et cyano.

13. Composé selon la revendication 1, dans lequel A représente un groupe de formule (A2), et R⁸ représente un atome d'hydrogène ou un groupe alkyle ayant de 1 à 3 atomes de carbone, un groupe alkyle substitué ayant de 1 à 3 atomes de carbone, dans lequel le substituant est choisi parmi des groupes hydroxyle, alcoxy ayant de 1 à 3 atomes de carbone, carbamoyle, carbamoyloxy, carboxyle, cyano, amino et des atomes d'halogène; un groupe alcényle ayant 3 ou 4 atomes de carbone ou un groupe alcynyle ayant 3 ou 4 atomes de carbone.

14. Composé selon la revendication 1 ou 2, dans lequel A représente un groupe de formule (A2) et R⁹ représente un atome d'hydrogène ou un groupe alkyle ayant de 1 à 3 atomes de carbone, un groupe alkyle substitué ayant de 1 à 3 atomes de carbone, dans lequel le substituant est choisi parmi des groupes hydroxyle, alcoxy ayant de 1 à 3 atomes de carbone, carbamoyle, carbamoyloxy, carboxyle, cyano, amino et des atomes d'halogène; ou un groupe de formule -C(=NH)R¹⁰ dans laquelle R¹⁰ représente:
un atome d'hydrogène,
un groupe alkyle ayant de 1 à 3 atomes de carbone,
un groupe alkyle substitué ayant de 1 à 3 atomes de carbone, dans lequel le substituant est choisi parmi des groupes alcoxy ayant de 1 à 3 atomes de carbone et des atomes d'halogène,
un groupe cycloalkyle ayant de 3 à 6 atomes de carbone, ou
un groupe alkyle ayant de 1 à 3 atomes de carbone, qui est substitué par un seul groupe cycloalkyle ayant de 3 à 6 atomes de carbone.

15. Composé selon la revendication 1 ou 2, dans lequel A représente un groupe de formule (A2), et R⁸ et R⁹ forment ensemble un groupe de formule -(CH₂)ₛ-W-(CH₂)ₜ-, dans laquelle W représente une liaison carbone-carbone simple ou un atome d'oxygène ou de soufre ou un groupe de formule 〉NR²², dans laquelle R²² représente un atome d'hydrogène ou un groupe alkyle ayant de 1 à 3 atomes de carbone, s est 1, 2 ou 3, et t est égal à 2.

16. Composé selon la revendication 1 ou 2, dans lequel:
A représente un groupe de formule (A2);
d est 0 ou 1;
m est 0, 1 ou 2;
R¹ représente un atome d'hydrogène ou le groupe méthyle, fluorométhyle, formimidoyle ou acétimidoyle;
R⁷ représente un atome d'hydrogène ou d'halogène ou un groupe alkyle ayant de 1 à 3 atomes de carbone, hydroxyle, amino, cyano, carboxyle, carbamoyle ou hydroxyméthyle;
R⁸ représente un atome d'hydrogène ou un groupe alkyle ayant de 1 à 3 atomes de carbone, fluorométhyle, carbamoylméthyle, carboxyméthyle, alcényle ayant 3 ou 4 atomes de carbone, alcynyle ayant 3 ou 4 atomes de carbone, 2-halogénoéthyle, 2-hydroxyéthyle, un groupe 2-alcoxyéthyle dans lequel le fragment alcoxy a de 1 à 3 atomes de carbone, ou le groupe 2-aminoéthyle;
R⁹ représente un atome d'hydrogène ou un groupe alkyle ayant de 1 à 3 atomes de carbone, fluorométhyle, carbamoylméthyle, carboxyméthyle, formimidoyle, acétimidoyle, 2-halogénoéthyle, 2-hydroxyéthyle ou un groupe 2-alcoxyéthyle dans lequel le fragment alcoxy a de 1 à 3 atomes de carbone, ou le groupe 2-aminoéthyle, ou
R⁸ et R⁹ forment ensemble un groupe de formule
-(CH₂)₄-,
-(CH₂)₅-,
-(CH₂)₂O(CH₂)₂-,
-(CH₂)₂S(CH₂)₂-,
-(CH₂)₂NH(CH₂)₂- ou
-(CH₂)₂NCH₃(CH₂)₂-.

17. Composé selon la revendication 1 ou 2, dans lequel:
A représente un groupe de formule (A2);
d est 0;
m est 1 ou 2;
R¹ représente un atome d'hydrogène ou le groupe méthyle, fluorométhyle, formimidoyle ou acétimidoyle;
R⁷ représente un atome d'hydrogène;
R⁸ représente un atome d'hydrogène ou un groupe alkyle ayant de 1 à 3 atomes de carbone, carbamoylméthyle, carboxyméthyle, 2-fluoroéthyle ou 2-hydroxyéthyle; et
R⁹ représente un atome d'hydrogène ou un groupe alkyle ayant de 1 à 3 atomes de carbone, formimidoyle, acétimidoyle ou 2-fluoroéthyle.

18. Composé selon la revendication 1 ou 2, dans lequel A représente un groupe de formule (A3), ℓ est 0, 1 ou 2, et R⁷ représente un atome d'hydrogène ou un groupe alkyle ayant de 1 à 3 atomes de carbone.

19. Composé selon la revendication 1 ou 2, dans lequel A représente un groupe de formule (A3) et R¹¹ représente un atome d'hydrogène ou un groupe alkyle ayant de 1 à 3 atomes de carbone, tel que le groupe méthyle, éthyle ou propyle.

20. Composé selon la revendication 1 ou 2, dans lequel A représente un groupe de formule (A3), et R¹² représente un atome d'hydrogène; un groupe alkyle ayant de 1 à 3 atomes de carbone; un groupe alkyle substitué ayant de 1 à 3 atomes de carbone, dans lequel le substituant est choisi parmi des groupes hydroxyle, alcoxy ayant de 1 à 3 atomes de carbone, carbamoyle, carbamoyloxy, carboxyle, cyano, amino et des atomes d'halogène; un groupe alcényle ayant 3 ou 4 atomes de carbone; un groupe alcynyle ayant 3 ou 4 atomes de carbone; ou un groupe de formule -C(=NH)R¹³, dans laquelle R¹³ représente:
un atome d'hydrogène;
un groupe alkyle ayant de 1 à 3 atomes de carbone;
un groupe alkyle substitué ayant de 1 à 3 atomes de carbone, dans lequel le substituant est choisi parmi des groupes alcoxy ayant de 1 à 3 atomes de carbone et des atomes d'halogène;
un groupe cycloalkyle ayant de 3 à 6 atomes de carbone; ou
un groupe alkyle ayant de 1 à 3 atomes de carbone, qui est substitué par un seul groupe cycloalkyle ayant de 3 à 6 atomes de carbone.

21. Composé selon la revendication 1 ou 2, dans lequel:
A représente un groupe de formule (A3);
ℓ est 0, 1 ou 2;
R¹ représente un atome d'hydrogène ou le groupe méthyle, fluorométhyle, formimidoyle ou acétimidoyle;
R⁷ représente un atome d'hydrogène;
R¹¹ représente un atome d'hydrogène ou un groupe alkyle ayant de 1 à 3 atomes de carbone; et
R¹² représente un atome d'hydrogène ou un groupe alkyle ayant de 1 à 3 atomes de carbone, fluorométhyle, carbamoylméthyle, carboxyméthyle, alcényle ayant 3 ou 4 atomes de carbone, alcynyle ayant 3 ou 4 atomes de carbone, formimidoyle, acétimidoyle, 2-halogénoéthyle, 2-hydroxyéthyle, un groupe 2-alcoxyéthyle dans lequel le fragment alcoxy a de 1 à 3 atomes de carbone, ou le groupe 2-aminoéthyle.

22. Composé selon la revendication 1 ou 2, dans lequel:
A représente un groupe de formule (A3);
ℓ est 1 ou 2;
R¹ représente un atome d'hydrogène ou le groupe méthyle, fluorométhyle, formimidoyle ou acétimidoyle;
R⁷ représente un atome d'hydrogène;
R¹¹ représente un atome d'hydrogène ou le groupe méthyle; et
R¹² représente un atome d'hydrogène ou un groupe alkyle ayant de 1 à 3 atomes de carbone, fluorométhyle, carbamoylméthyle, carboxyméthyle, formimidoyle, acétimidoyle, 2-fluoroéthyle ou 2-hydroxyéthyle.

23. Composé selon la revendication 1 ou 2, dans lequel A représente un groupe de formule (A4), et R¹⁴ et R¹⁵ représentent chacun un atome d'hydrogène ou un groupe alkyle ayant de 1 à 3 atomes de carbone.

24. Composé selon la revendication 1 ou 2, dans lequel A représente un groupe de formule (A4), et R¹⁶ représente un atome d'hydrogène ou un groupe alkyle ayant de 1 à 3 atomes de carbone; un groupe alkyle substitué ayant de 1 à 3 atomes de carbone, dans lequel le substituant est choisi parmi des groupes alcoxy ayant de 1 à 3 atomes de carbone et des atomes d'halogène; un groupe cycloalkyle ayant de 3 à 6 atomes de carbone; ou un groupe alkyle ayant de 1 à 3 atomes de carbone, qui est substitué par un seul groupe cycloalkyle ayant de 3 à 6 atomes de carbone.

25. Composé selon la revendication 1 ou 2, dans lequel:
A représente un groupe de formule (A4);
i est 1 ou 2; et
R¹, R¹⁴, R¹⁵ et R¹⁶ sont identiques ou différents et représentent chacun un atome d'hydrogène ou un groupe alkyle ayant de 1 à 3 atomes de carbone.

26. Composé selon la revendication 1 ou 2, dans lequel:
A représente un groupe de formule (A5);
i est 1; et
R¹, R¹⁴, R¹⁵ et R¹⁶ sont identiques ou différents et représentent chacun un atome d'hydrogène ou le groupe méthyle.

27. Composé selon la revendication 1 ou 2, dans lequel A représente un groupe de formule (A5), et R¹⁷ et R¹⁸ représentent chacun un atome d'hydrogène; un groupe alkyle ayant de 1 à 3 atomes de carbone ou un groupe alkyle substitué ayant de 1 à 3 atomes de carbone, dans lequel le substituant est choisi parmi des groupes hydroxyle, alcoxy ayant de 1 à 3 atomes de carbone et des atomes d'halogène.

28. Composé selon la revendication 1 ou 2, dans lequel A représente un groupe de formule (A5), et R¹⁷ et R¹⁸ représentent ensemble un groupe de formule -(CH₂)_{q}-Y-(CH₂)ᵣ-, dans laquelle Y représente une liaison carbone-carbone simple, un atome d'oxygène ou un groupe de formule 〉NR²³, dans laquelle R²³ représente un atome d'hydrogène ou un groupe alkyle ayant de 1 à 3 atomes de carbone, et q et r représentent chacun 2 ou 3.

29. Composé selon la revendication 1 ou 2, dans lequel:
A représente un groupe de formule (A5);
p est égal à 2;
R¹ représente un atome d'hydrogène ou le groupe méthyle, fluorométhyle, formimidoyle ou acétimidoyle;
R¹⁷ et R¹⁸ sont identiques ou différents et représentent chacun un atome d'hydrogène ou un groupe alkyle ayant de 1 à 3 atomes de carbone, 2-halogénoéthyle ou 2-hydroxyéthyle; ou
R¹⁷ et R¹⁸ représentent ensemble un groupe de formule
-(CH₂)₄-,
-(CH₂)₅-,
-(CH₂)₂O(CH₂)₂-,
-(CH₂)₂S(CH₂)₂-,
-(CH₂)₂NH(CH₂)₂- ou
-(CH₂)₂NCH₃(CH₂)₂-.

30. Composé selon la revendication 1 ou 2, dans lequel:
A représente un groupe de formule (A5);
p est égal à 2;
R¹ représente un atome d'hydrogène ou le groupe méthyle, fluorométhyle, formimidoyle ou acétimidoyle; et
R¹⁷ et R¹⁸ sont identiques ou différents et représentent chacun un atome d'hydrogène ou le groupe méthyle.

31. Composé selon la revendication 1 ou 2, dans lequel A représente un groupe de formule (A6), et R¹⁹ représente un atome d'hydrogène ou un groupe alkyle ayant de 1 à 3 atomes de carbone.

32. Composé selon la revendication 1 ou 2, dans lequel:
A représente un groupe de formule (A6);
j et k représentent chacun 2;
R¹ représente un atome d'hydrogène ou le groupe méthyle, fluorométhyle, formimidoyle ou acétimidoyle; et
R¹⁹ représente un atome d'hydrogène ou un groupe alkyle ayant de 1 à 3 atomes de carbone.

33. Composé selon la revendication 1 ou 2, dans lequel:
A représente un groupe de formule (A6);
j et k représentent chacun 2; et
R¹ et R¹⁹ sont identiques ou différents et représentent chacun un atome d'hydrogène ou le groupe méthyle.

34. Composé selon la revendication 1 ou 2, dans lequel A représente un groupe de formule (A7), et Z représente le groupe 1-imidazolyle, 1,2,4-triazole-1-yle ou 1,2,3-triazole-1-yle.

35. Composé selon la revendication 1 ou 2, dans lequel:
A représente un groupe de formule (A7);
g est égal à 0, 1 ou 2;
R¹ représente un atome d'hydrogène ou le groupe méthyle; et
Z représente le groupe 1-imidazolyle, 1,2,4-triazole-1-yle ou 1,2,3-triazole-1-yle.

36. Composé selon la revendication 1 ou 2, dans lequel:
A représente un groupe de formule (A7);
g est égal à 1 ou 2;
R¹ représente un atome d'hydrogène ou le groupe méthyle; et
Z représente le groupe 1-imidazolyle, 1,2,4-triazole-1-yle ou 1,2,3-triazole-1-yle.

37. Composé selon la revendication 1 ou 2, dans lequel A représente un groupe de formule (A8), et R²⁰ et R²¹ représentent chacun un atome d'hydrogène ou un groupe alkyle ayant de 1 à 3 atomes de carbone.

38. Composé selon la revendication 1 ou 2, dans lequel:
A représente un groupe de formule (A8);
e et f sont chacun égal à 1;
R¹ représente un atome d'hydrogène ou le groupe méthyle, fluorométhyle, formimidoyle ou acétimidoyle; et
R²⁰ et R²¹ représentent chacun un atome d'hydrogène ou un groupe alkyle ayant de 1 à 3 atomes de carbone.

39. Composé selon la revendication 1 ou 2, dans lequel:
A représente un groupe de formule (A8);
e et f sont chacun égal à 1;
R¹ représente un atome d'hydrogène ou le groupe méthyle;
R²⁰ représente un atome d'hydrogène; et
R²¹ représente un atome d'hydrogène ou le groupe méthyle.

40. Composé selon l'une quelconque des revendications précédentes, dans lequel les atomes de carbone sont dans les mêmes configurations que ceux de la thiénamycine.

41. Composés suivants selon la revendication 1:
acide 2-[2-(1-homopipérazinylcarbonyl)-1-méthylpyrrolidine-4-ylthio]-6-(1-hydroxyéthyl)-1-méthyl-1-carbapén-2-ème-3-carboxylique,
acide 2-[2-(4-carboxyméthylhomopipérazine-1-ylcarbonyl)pyrrolidine-4-ylthio]-6-(1-hydroxyéthyl)-1-méthyl-1-carbapén-2-ème-3-carboxylique,
acide 2-{2-[4-(2-hydroxyéthyl)homopipérazine-1-ylcarbonyl]pyrrolidine-4-ylthio}-6-(1-hydroxyéthyl)-1-méthyl-1-carbapén-2-ème-3-carboxylique,
acide 2-[2-(4-acétimidoylhomopipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylhomopipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylhomopipérazine-1-ylcarbonyl)-1-méthylpyrrolidine-4-ylthio]-6-(1-hydroxyéthyl)-1-méthyl-1-carbapén-2-ème-3-carboxylique,
acide 2-[1-méthyl-2-(pipérazine-1-ylcarbonyl)pyrrolidine-4-ylthio]-6-(1-hydroxyéthyl)-1-méthyl-1-carbapén-2-ème-3-carboxylique,
acide 2-{2-[4-(2-hydroxyéthyl)pipérazine-1-ylcarbonyl]pyrrolidine-4-ylthio}-6-(1-hydroxyéthyl)-1-méthyl-1-carbapén-2-ème-3-carboxylique,
acide 2-[2-(3-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylpipérazine-1-ylcarbonyl)pyrrolidine-4-ylthio]-6-(1-hydroxyéthyl)-1-méthyl-1-carbapén-2-ème-3-carboxylique,
acide 2-[2-(4-acétimidoylpipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylpipérazine-1-ylcarbonyl)-1-méthylpyrrolidine-4-ylthio]-6-(1-hydroxyéthyl)-1-méthyl-1-carbapén-2-ème-3-carboxylique,
acide 2-[2-(4-acétimidoylpipérazine-1-ylcarbonyl)-1-méthylpyrrolidine-4-ylthio]-6-(1-hydroxyéthyl)-1-méthyl-1-carbapén-2-ème-3-carboxylique,
acide 2-[2-(4-formimidoyl-3-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-acétimidoyl-3-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(2-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoyl-2-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-acétimidoyl-2-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(3-hydroxyméthylpipérazine-1-ylcarbonyl)pyrrolidine-4-ylthio]-6-(1-hydroxyéthyl)-1-méthyl-1-carbapén-2-ème-3-carboxylique,
acide 2-[1-formimidoyl-2-(4-formimidoylpipérazine-1-ylcarbonyl)pyrrolidine-4-ylthio]-6-(1-hydroxyéthyl)-1-méthyl-1-carbapén-2-ème-3-carboxylique,
acide 2-[2-(3-acétimidoylaminopyrrolidine-1-ylcarbonyl)pyrrolidine-4-ylthio]-6-(1-hydroxyéthyl)-1-méthyl-1-carbapén-2-ème-3-carboxylique,
acide 2-[2-(3-formimidoylaminopyrrolidine-1-ylcarbonyl)pyrrolidine-4-ylthio]-6-(1-hydroxyéthyl)-1-méthyl-1-carbapén-2-ème-3-carboxylique,
acide 2-[2-(3-aminopyrrolidine-1-ylcarbonyl)pyrrolidine-4-ylthio]-6-(1-hydroxyéthyl)-1-méthyl-1-carbapén-2- ème-3-carboxylique,
acide 2-[2-(4-acétimidoylaminopipéridine-1-ylcarbonyl)pyrrolidine-4-ylthio]-6-(1-hydroxyéthyl)-1-méthyl-1-carbapén-2-ème-3-carboxylique,
acide 2-[2-(3-aminopyrrolidine-1-ylcarbonyl)-1-méthylpyrrolidine-4-ylthio]-6-(1-hydroxyéthyl)-1-méthyl-1-carbapén-2-ème-3-carboxylique,
acide 2-[2-(3-acétimidoylaminopyrrolidine-1-ylcarbonyl)-1-méthylpyrrolidine-4-ylthio]-6-(1-hydroxyéthyl)-1-méthyl-1-carbapén-2-ème-3-carboxylique,
acide 2-[2-(3-formimidoylaminopyrrolidine-1-ylcarbonyl)-1-méthylpyrrolidine-4-ylthio]-6-(1-hydroxyéthyl)-1-méthyl-1-carbapén-2-ème-3-carboxylique,
acide 2-[2-(4-acétimidoylaminopipéridine-1-ylcarbonyl)-1-méthylpyrrolidine-4-ylthio]-6-(1-hydroxyéthyl)-1-méthyl-1-carbapén-2-ème-3-carboxylique,
acide 2-[2-(1-formimidoylpyrrolidine-3-ylcarbamoyl)pyrrolidine-4-ylthio]-6-(1-hydroxyéthyl)-1-méthyl-1-carbapén-2-ème-3-carboxylique,
acide 2-[2-(3-diméthylamino-1,2,5,6-tétrahydropyrazine-1-ylcarbonyl)pyrrolidine-4-ylthio]-6-(1-hydroxyéthyl)-1-méthyl-1-carbapén-2-ème-3-carboxylique,
et leurs sels et esters pharmaceutiquement acceptables.

42. Composition pharmaceutique comprenant un véhicule, diluant ou adjuvant pharmaceutiquement acceptable, en mélange avec une quantité efficace d'un antibiotique, dans laquelle l'antibiotique est choisi parmi les composés de formule (I) et leurs sels et esters pharmaceutiquement acceptables, tels que revendiqués dans l'une quelconque des revendications 1 à 41.

43. Procédé pour la préparation d'un composé selon l'une quelconque des revendications 1 à 41, comprenant les étapes suivantes:
mise en réaction d'un composé de formule (II): 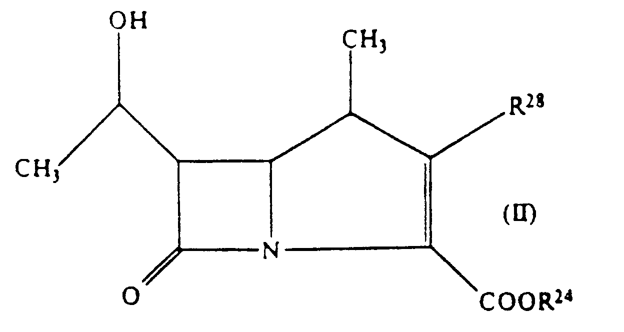 [dans laquelle R²⁴ représente un groupe protecteur de fonction carboxyle, et R²⁸ représente un groupe alcanesulfonyloxy, arylsulfonyloxy, dialkylphosphoryloxy, diarylphosphoryloxy ou un groupe de formule -S(-O)R²⁷, dans laquelle R²⁷ représente un groupe alkyle, halogénoalkyle, acétamidoalkyle, acétamidoalcényle, aryle ou aromatique hétérocyclique] avec un composé de formule (III): 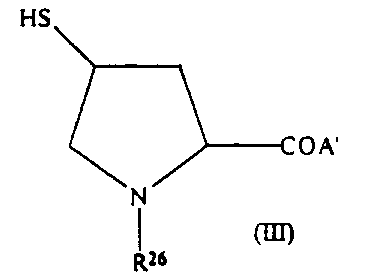 (dans laquelle R²⁶ représente l'un quelconque des groupes ou atomes représentés par R¹ ou tout autre groupe ou atome dans lequel un groupe actif quelconque est protégé, et A' représente l'un quelconque des groupes ou atomes représentés par A ou l'un quelconque de ces groupes ou atomes dans lequel un groupe actif quelconque est protégé) et si nécessaire l'élimination de tout groupe protecteur.

44. Utilisation d'un composé selon l'une quelconque des revendications 1 à 41, pour la fabrication d'un médicament destiné au traitement ou à la prophylaxie d'infections bactériennes.

## Revendications (Revendications pour l'(les) Etat(s) contractant(s) suivant(s): ES, GR)

1. Procédé pour la préparation d'un composé de formule (I): 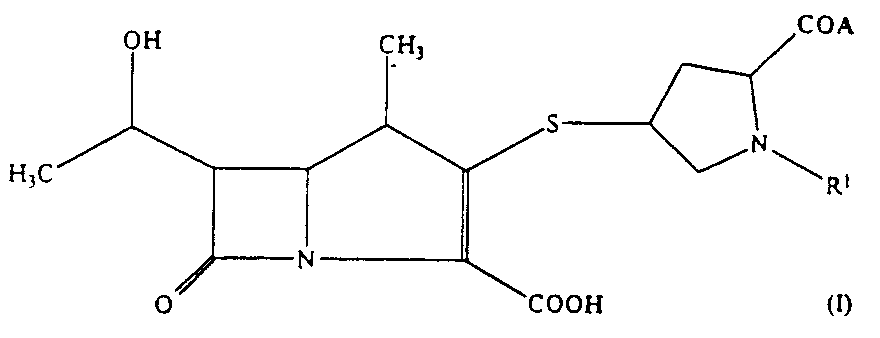 dans laquelle
R¹ représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous,
un groupe alcényle ayant de 2 à 6 atomes de carbone,
un groupe alcynyle ayant de 2 à 6 atomes de carbone, ou
un groupe de formule -C(=NH)R°, dans laquelle R° représente un atome d'hydrogène ou un groupe alkyle ayant de 1 à 6 atomes de carbone; et
A représente un groupe de formule (A1), (A2), (A3), (A4), (A5), (A6), (A7) ou (A8): 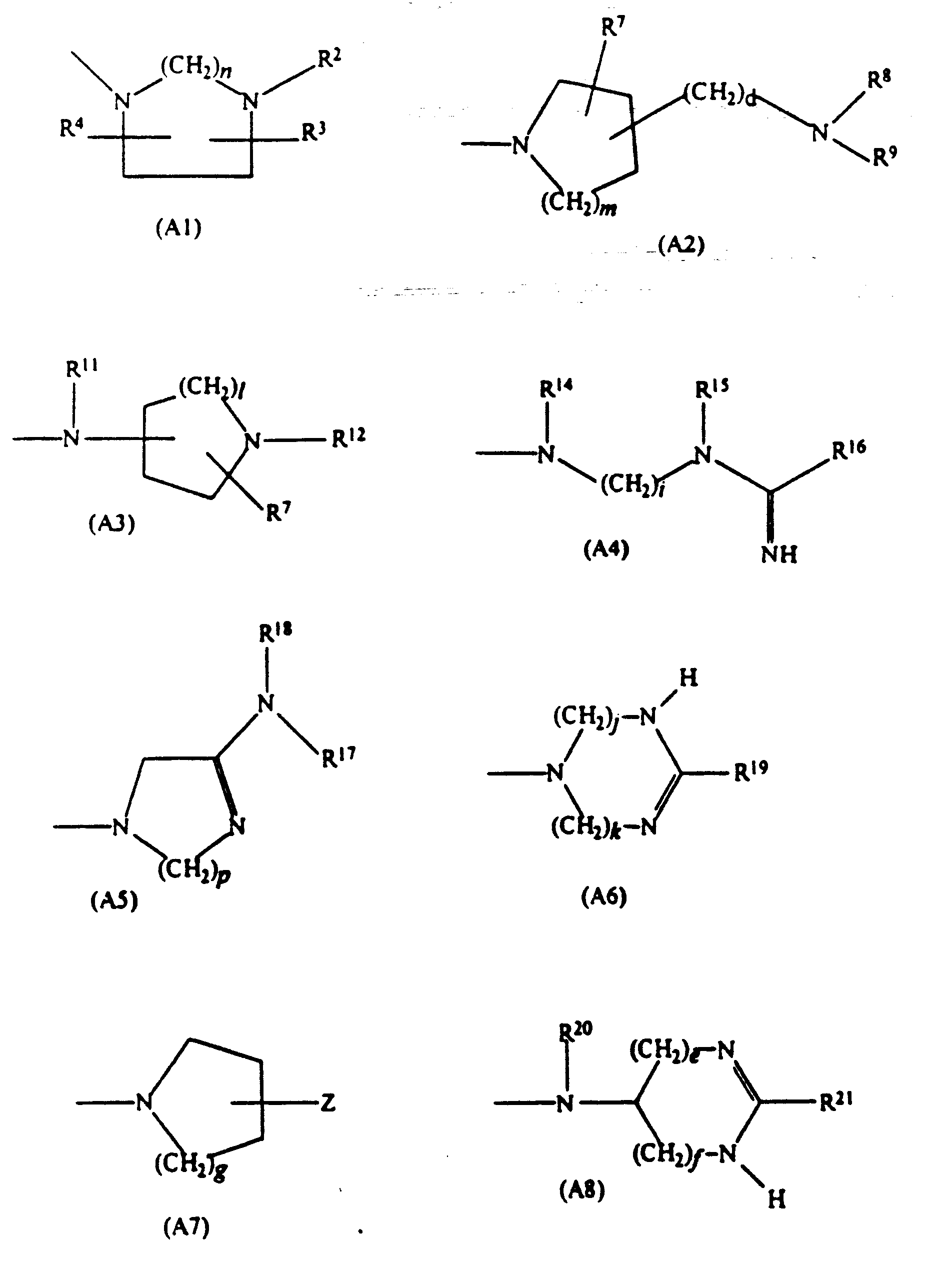 formules dans lesquelles:
R² représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (b) définis ci-dessous,
un groupe alcényle ayant de 2 à 6 atomes de carbone,
un groupe alcynyle ayant de 2 à 6 atomes de carbone, ou
un groupe de formule -C(=NH)R⁶,
dans laquelle R⁶ représente un atome d'hydrogène, un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
R³, R⁴ et R⁷ sont identiques ou différents et représentent chacun
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (d) définis ci-dessous,
un atome d'halogène,
le groupe hydroxyle,
le groupe carboxyle,
un groupe de formule -CO.NR^{a}R^{b}, -OCO.NR^{a}R^{b} ou -NR^{a}R^{b},
formules dans lesquelles R^{a} et R^{b} sont identiques ou différents et représentent chacun un atome d'hydrogène ou un groupe alkyle ayant de 1 à 4 atomes de carbone, ou
le groupe cyano;
R⁸ représente
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous,
un groupe alcényle ayant de 2 à 6 atomes de carbone, ou
un groupe alcynyle ayant de 2 à 6 atomes de carbone;
R⁹ représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous, ou
un groupe de formule -C(=NH)R¹⁰,
dans laquelle R¹⁰ représente un atome d'hydrogène ou un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
ou
R⁸ et R⁹ représentent ensemble un groupe de formule -(CH₂)ₛ-W-(CH₂)ₜ-
dans laquelle W représente une liaison carbone-carbone simple, un atome d'oxygène ou de soufre, ou un groupe de formule 〉NR²², dans laquelle R²² représente un atome d'hydrogène ou un groupe alkyle ayant de 1 à 6 atomes de carbone, et
s et t sont identiques ou différents et représentent chacun 1, 2 ou 3;
R¹¹ représente un atome d'hydrogène ou un groupe alkyle ayant de 1 à 6 atomes de carbone;
R¹² représente:
un atome d'hydrogène,
un groupe alkyle non substitué ayant de 1 à 6 atomes de carbone,
un groupe alkyle substitué ayant de 1 à 6 atomes de carbone et étant substitué par au moins un des substituants (a) définis ci-dessous,
un groupe alcényle ayant de 2 à 6 atomes de carbone,
un groupe alcynyle ayant de 2 à 6 atomes de carbone, ou
un groupe de formule -C(=NH)R¹³,
dans laquelle R¹³ représente un atome d'hydrogène ou un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
R¹⁴ et R¹⁵ sont identiques ou différents et représentent chacun un atome d'hydrogène ou un groupe alkyle ayant de 1 à 6 atomes de carbone;
R¹⁶ représente un atome d'hydrogène ou un groupe alkyle non substitué ayant de 1 à 6 atomes de carbone, un groupe alkyle substitué ayant de 1 à 6 atomes de carbone et étant substitué par au moins un des substituants (c) définis ci-dessous, ou un groupe cycloalkyle ayant de 3 à 7 atomes de carbone formant le cycle;
R¹⁷ et R¹⁸ sont identiques ou différents et représentent chacun
un atome d'hydrogène,
un groupe alkyle non substitué ayant de 1 à 6 atomes de carbone, ou
un groupe alkyle substitué ayant de 1 à 6 atomes de carbone et étant substitué par au moins un des substituants (a) définis ci-dessous;
ou
R¹⁷ et R¹⁸ représentent ensemble un groupe de formule -(CH₂)_{q}-Y-(CH₂)ᵣ-,
dans laquelle Y représente une liaison carbone-carbone simple, un atome d'oxygène ou de soufre, ou un groupe de formule 〉NR²³, dans laquelle R²³ représente un atome d'hydrogène ou un groupe alkyle ayant de 1 à 6 atomes de carbone, et
q et r sont identiques ou différents et représentent chacun 1, 2 ou 3;
R¹⁹, R²⁰ et R²¹ sont identiques ou différents et représentent chacun un atome d'hydrogène ou un groupe alkyle ayant de 1 à 6 atomes de carbone;
Z représente le groupe imidazolyle, triazolyle ou tétrazolyle;
d est 0 ou 1;
e, f, i, j et k sont identiques ou différents et représentent chacun 1 ou 2;
g, ℓ et m sont identiques ou différents et représentent chacun 0, 1 ou 2; et
n et p sont identiques ou différents et représentent chacun 1, 2 ou 3;
étant entendu que lorsque A représente un groupe de formule (A1):
R², R³ et R⁴ ne représentent pas tous des atomes d'hydrogène lorsque R¹ représente un atome d'hydrogène; et
R¹, R³ et R⁴ ne représentent pas tous des atomes d'hydrogène lorsque R² représente un groupe alkyle;
lesdits substituants (a) sont choisis parmi:
le groupe hydroxyle,
le groupe carboxyle,
le groupe cyano,
des atomes d'halogène,
des atomes d'oxygène pour la formation d'un groupe oxo,
des groupes alcoxy ayant de 1 à 6 atomes de carbone, et
des groupes de formule -CO.NR^{a}R^{b}, -OCO.NR^{a}R^{b} et -NR^{a}R^{b}, R^{a} et R^{b} étant tels que définis plus haut;
lesdits substituants (b) sont choisis parmi:
le groupe hydroxyle,
le groupe carboxyle,
le groupe cyano,
des atomes d'halogène,
des groupes alcoxy ayant de 1 à 6 atomes de carbone,
des groupes de formule -CO.NR^{a}R^{b}, -OCO.NR^{a}R^{b} et -NR^{a}R^{b}, R^{a} et R^{b} étant tels que définis plus haut,
les groupes sulfamoyle,
uréido,
sulfo,
des groupes alcanoyle ayant de 1 à 6 atomes de carbone,
des groupes alcanoylamino ayant de 1 à 6 atomes de carbone,
des groupes alcanoyloxy ayant de 1 à 6 atomes de carbone,
des groupes alkylthio ayant de 1 à 6 atomes de carbone,
des groupes alkylsulfinyle ayant de 1 à 6 atomes de carbone, et
des groupes alkylsulfonyle ayant de 1 à 6 atomes de carbone;
lesdits substituants (c) sont choisis parmi:
des atomes d'halogène,
des groupes alcoxy ayant de 1 à 6 atomes de carbone,
des groupes cycloalkyle ayant de 3 à 7 atomes de carbone formant le cycle; et
lesdits substituants (d) sont choisis parmi:
le groupe hydroxyle,
le groupe cyano,
des groupes de formule -CO.NR^{a}R^{b}, -OCO.NR^{a}R^{b} et -NR^{a}R^{b}, R^{a} et R^{b} étant tels que définis plus haut;
le groupe carboxyle,
des atomes d'halogène, et
des groupes alcoxy ayant de 1 à 6 atomes de carbone;
ou d'un sel ou ester pharmaceutiquement acceptable de celui-ci, lequel procédé comprend les étapes suivantes:
mise en réaction d'un composé de formule (II): 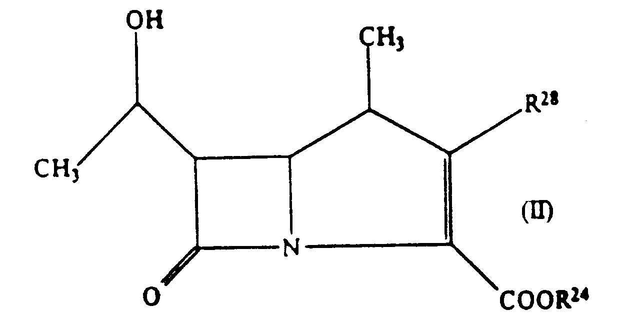 [dans laquelle R²⁴ représente un groupe protecteur de fonction carboxyle, et R²⁸ représente un groupe alcanesulfonyloxy, arylsulfonyloxy, dialkylphosphoryloxy, diarylphosphoryloxy ou un groupe de formule -S(-O)R²⁷, dans laquelle R²⁷ représente un groupe alkyle, halogénoalkyle, acétamidoalkyle, acétamidoalcényle, aryle ou aromatique hétérocyclique] avec un composé de formule (III): 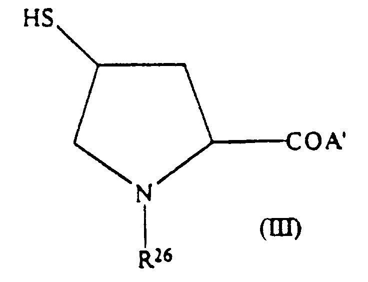 (dans laquelle R²⁶ représente l'un quelconque des groupes ou atomes représentés par R¹ ou tout autre groupe ou atome dans lequel un groupe actif quelconque est protégé, et A' représente l'un quelconque des groupes ou atomes représentés par A ou l'un quelconque de ces groupes ou atomes dans lequel un groupe actif quelconque est protégé) et
si nécessaire l'élimination de tout groupe protecteur.

2. Procédé selon la revendication 1, dans lequel on choisit les réactifs et conditions réactionnelles de manière à préparer un composé de formule (I) ou un sel ou ester de celui-ci, correspondant à la formule Ia): 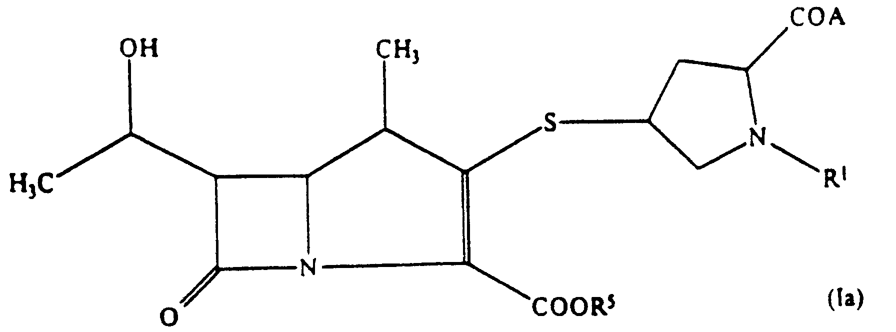 dans laquelle R¹ et A sont tels que définis dans la revendication 1, et R⁵ représente:
un groupe alkyle en C₁-C₂₀,
un groupe cycloalkyle en C₃-C₇,
un groupe aralkyle dans lequel le fragment alkyle est un groupe alkyle en C₁-C₃ et le fragment aryle est un groupe aromatique carbocyclique en C₆-C₁₄ qui peut être substitué ou non substitué, et, s'il est substitué, comporte au moins un des substituants (e) définis ci-dessous,
un groupe alcényle qui est substitué ou non substitué, et, s'il est substitué, comporte au moins un des substituants (a) définis dans la revendication 1,
un groupe alkyle en C₁-C₆ halogéné,
des groupes silylalkyle subtitués, dans lesquels le fragment alkyle a de 1 à 6 atomes de carbone et le fragment silyle porte jusqu'à 3 substituants choisis parmi des groupes alkyle en C₁-C₆ et des groupes phényle qui ne sont pas substitués ou portent au moins un des substituants (e) définis ci-dessous;
un groupe phényle qui n'est pas substitué ou porte au moins un des substituants (e) définis ci-dessous;
un groupe phénacyle qui n'est pas substitué ou porte au moins un des substituants (e) définis ci-dessous;
un groupe terpényle cyclique ou acyclique;
un groupe alcoxyméthyle dans lequel le fragment alcoxy est en C₁-C₆;
un groupe acyloxyalkyle aliphatique dans lequel le fragment acyle est un groupe alcanoyle en C₂-C₆ et le fragment alkyle est un groupe alkyle en C₂-C₆;
un groupe acyloxyalkyle aliphatique substitué par un radical cycloalkyle, dans lequel le fragment acyle est un groupe alcanoyle en C₂-C₆, le substituant cycloalkyle est en C₃-C₇, et le fragment alkyle est un groupe alkyle en C₁-C₆;
un groupe alcoxycarbonyloxyalkyle dans lequel le fragment alcoxy est en C₁-C₁₀ et le fragment alkyle est en C₁-C₆;
un groupe cycloalkylcarbonyloxyalkyle ou cycloalkyloxycarbonyloxyalkyle, dans lequel le fragment cycloalkyle est en C₃-C₁₀, est mono- ou polycyclique et n'est pas substitué ou est substitué par au moins un groupe alkyle en C₁-C₄, et le fragment alkyle est en C₁-C₆;
un groupe cycloalkylalcoxycarbonyloxyalkyle dans lequel le fragment alcoxy porte un seul substituant cycloalkyle, le substituant cycloalkyle étant en C₃-C₁₀ et mono- ou polycyclique;
un groupe terpénylcarbonyloxyalkylé ou terpényloxycarbonyloxyalkyle dans lequel le groupe alkyle a de 1 à 6 atomes de carbone;
un groupe 5-alkyl- ou 5-phényl-(2-oxo-1,3-dioxolène-4-yl)alkyle dans lequel chaque radical alkyle est en C₁-C₆; ou
le groupe phtalidyle, indanyle ou 2-oxo-4,5,6,7-tétrahydro-1,3-benzodioxolène-4-yle; et
les substituants (e) sont choisis parmi des groupes alkyle en C₁-C₄, alcoxy en C₁-C₄, halogénoalkyle en C₁-C₄, alkylènedioxy en C₁-C₃, des atomes d'halogène, les groupes cyano et nitro.

3. Procédé selon la revendication 2, dans lequel on choisit les réactifs et conditions réactionnelles de manière à préparer un composé de formule (I) ou un sel ou ester de celui-ci, dans lequel R⁵ représente un atome d'hydrogène ou un groupe (5-substitué-2-oxo-1,3-dioxolène-4-yl)méthyle, 1-méthylcyclohexylcarbonyloxyméthyle, 1-isopropoxycarbonyloxyéthyle ou 1-cyclohexylcarbonyloxyéthyle.

4. Procédé selon l'une quelconque des revendications 1 à 3, dans lequel on choisit les réactifs et conditions réactionnelles de manière à préparer un composé de formule (I) ou un sel ou ester de celui-ci, dans lequel R¹ représente un atome d'hydrogène ou un groupe alkyle ayant de 1 à 3 atomes de carbone; un groupe alkyle substitué ayant de 1 à 3 atomes de carbone, dans lequel le substituant est choisi parmi les substituants (a') définis ci-dessous; un groupe alcényle ayant 3 ou 4 atomes de carbone; un groupe alcynyle ayant 3 ou 4 atomes de carbone; le groupe formimidoyle ou acétimidoyle; et
les substituants (a') sont choisis parmi les groupes hydroxyle, carboxyle, carbamoyle, carbamoyloxy, cyano, des atomes d'halogène, des groupes alcoxy ayant de 1 à 3 atomes de carbone, le groupe amino et des groupes mono- et dialkylamino dans lesquels le ou chaque groupe alkyle a de 1 à 3 atomes de carbone.

5. Procédé selon l'une quelconque des revendications 1 à 4, dans lequel on choisit les réactifs et conditions réactionnelles de manière à préparer un composé de formule (I) ou un sel ou ester de celui-ci, dans lequel A représente un groupe de formule (A1) et n est égal à 2 ou 3.

6. Procédé selon l'une quelconque des revendications 1 à 5, dans lequel on choisit les réactifs et conditions réactionnelles de manière à préparer un composé de formule (I) ou un sel ou ester de celui-ci, dans lequel R² représente:
un atome d'hydrogène;
un groupe alkyle ayant de 1 à 3 atomes de carbone;
un groupe alkyle substitué ayant de 1 à 3 atomes de carbone, dans lequel le substituant est choisi parmi les substituants (b') définis ci-dessous;
un groupe alcényle ayant 3 ou 4 atomes de carbone;
un groupe alcynyle ayant 3 ou 4 atomes de carbone; ou
un groupe de formule -C(=NH)R⁶,
dans laquelle R⁶ représente:
un atome d'hydrogène,
un groupe alkyle non substitué ayant de 1 à 3 atomes de carbone,
un groupe alkyle substitué ayant de 1 à 3 atomes de carbone et étant substitué par au moins un substituant choisi parmi des atomes d'halogène et des groupes alcoxy ayant de 1 à 3 atomes de carbone, et des groupes cycloalkyle ayant de 3 à 6 atomes de carbone, ou
un groupe cycloalkyle ayant de 3 à 6 atomes de carbone formant le cycle; et
les substituants (b') sont choisis parmi les groupes hydroxyle, carboxyle, carbamoyle, carbamoyloxy, cyano, sulfamoyle, uréido, sulfo, des groupes alcoxy ayant de 1 à 3 atomes de carbone, alcoxycarbonyle ayant de 2 à 4 atomes de carbone, alcanoyle ayant de 2 à 4 atomes de carbone, alcanoylamino ayant de 2 à 4 atomes de carbone, alcanoyloxy ayant de 2 à 4 atomes de carbone, amino, mono- et dialkylamino dans lesquels le ou chaque fragment alkyle a de 1 à 3 atomes de carbone, des groupes alkylthio ayant de 1 à 3 atomes de carbone, alkylsulfinyle ayant de 1 à 3 atomes de carbone, alkylsulfonyle ayant de 1 à 3 atomes de carbone, des groupes mono- et dialkylcarbamoyle dans lesquels le ou chaque groupe alkyle a de 1 à 3 atomes de carbone, et des groupes mono- et dialkylcarbamoyloxy dans lesquels le ou chaque groupe alkyle a de 1 à 3 atomes de carbone.

7. Procédé selon l'une quelconque des revendications 1 à 6, dans lequel on choisit les réactifs et conditions réactionnelles de manière à préparer un composé de formule (I) ou un sel ou ester de celui-ci, dans lequel A représente un groupe de formule (A1), et R³ et R⁴ représentent chacun un atome d'hydrogène ou un groupe alkyle ayant de 1 à 3 atomes de carbone, le groupe hydroxyle, carboxyle, carbamoyle ou un groupe alkyle substitué ayant de 1 à 3 atomes de carbone et portant au moins un substituant choisi parmi des groupes hydroxyle, alcoxy ayant de 1 à 3 atomes de carbone, amino, carbamoyle et des atomes d'halogène.

8. Procédé selon la revendication 1 ou 2, dans lequel on choisit les réactifs et conditions réactionnelles de manière à préparer un composé de formule (I) ou un sel ou ester de celui-ci, dans lequel
A représente un groupe de formule (A1);
n est égal à 2;
R¹ représente un atome d'hydrogène ou le groupe méthyle, fluorométhyle, formimidoyle ou acétimidoyle;
R² représente un atome d'hydrogène ou le groupe 2-hydroxyéthyle, 2-carbamoyléthyle, carboxyméthyle, carbamoylméthyle, 2-fluoroéthyle, formimidoyle ou acétimidoyle; et
R³ et R⁴ sont identiques ou différents et représentent chacun un atome d'hydrogène ou le groupe méthyle, carbamoyle, cyano, carboxyle, hydroxyméthyle, fluorométhyle ou aminométhyle.

9. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A1);
n est égal à 3;
R¹ représente un atome d'hydrogène ou le groupe méthyle, fluorométhyle, formimidoyle ou acétimidoyle;
R² représente un atome d'hydrogène ou le groupe méthyle, formimidoyle, acétimidoyle, carboxyméthyle, carbamoylméthyle, 2-hydroxyéthyle ou 2-fluoroéthyle; et
R³ et R⁴ sont identiques ou différents et représentent chacun un atome d'hydrogène ou le groupe méthyle, hydroxyle, amino, cyano, carboxyle, carbamoyle, carbamoyloxy, hydroxyméthyle, fluorométhyle ou aminométhyle.

10. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A1);
n est égal à 2;
R¹ représente un atome d'hydrogène ou le groupe méthyle, formimidoyle ou acétimidoyle;
R² représente un atome d'hydrogène ou le groupe 2-hydroxyéthyle, carboxyméthyle, formimidoyle ou acétimidoyle;
R³ représente un atome d'hydrogène; et
R⁴ représente le groupe méthyle, carbamoyle, cyano, hydroxyméthyle, fluorométhyle ou aminométhyle.

11. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A1);
n est égal à 3;
R¹ représente un atome d'hydrogène ou le groupe méthyle, formimidoyle ou acétimidoyle;
R² représente le groupe formimidoyle, acétimidoyle, carboxyméthyle, 2-hydroxyéthyle ou 2-fluoroéthyle; et
R³ et R⁴ sont identiques ou différents et représentent chacun un atome d'hydrogène ou le groupe hydroxyle, amino ou cyano.

12. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A2) et R⁷ représente un atome d'hydrogène ou le groupe carboxyle ou carbamoyle, ou un groupe alkyle ayant de 1 à 3 atomes de carbone, ou un groupe alkyle substitué ayant de 1 à 3 atomes de carbone, dans lequel le substituant est choisi parmi des groupes hydroxyle, alcoxy ayant de 1 à 3 atomes de carbone, carbamoyle, carboxyle et cyano.

13. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A2), et R⁸ représente un atome d'hydrogène ou un groupe alkyle ayant de 1 à 3 atomes de carbone, un groupe alkyle substitué ayant de 1 à 3 atomes de carbone, dans lequel le substituant est choisi parmi des groupes hydroxyle, alcoxy ayant de 1 à 3 atomes de carbone, carbamoyle, carbamoyloxy, carboxyle, cyano, amino et des atomes d'halogène; un groupe alcényle ayant 3 ou 4 atomes de carbone ou un groupe alcynyle ayant 3 ou 4 atomes de carbone.

14. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A2) et R⁹ représente un atome d'hydrogène ou un groupe alkyle ayant de 1 à 3 atomes de carbone, un groupe alkyle substitué ayant de 1 à 3 atomes de carbone, dans lequel le substituant est choisi parmi des groupes hydroxyle, alcoxy ayant de 1 à 3 atomes de carbone, carbamoyle, carbamoyloxy, carboxyle, cyano, amino et des atomes d'halogène; ou un groupe de formule -C(=NH)R¹⁰ dans laquelle R¹⁰ représente:
un atome d'hydrogène,
un groupe alkyle ayant de 1 à 3 atomes de carbone,
un groupe alkyle substitué ayant de 1 à 3 atomes de carbone, dans lequel le substituant est choisi parmi des groupes alcoxy ayant de 1 à 3 atomes de carbone et des atomes d'halogène,
un groupe cycloalkyle ayant de 3 à 6 atomes de carbone, ou
un groupe alkyle ayant de 1 à 3 atomes de carbone, qui est substitué par un seul groupe cycloalkyle ayant de 3 à 6 atomes de carbone.

15. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A2), et R⁸ et R⁹ forment ensemble un groupe de formule -(CH₂)ₛ-W-(CH₂)ₜ-, dans laquelle W représente une liaison carbone-carbone simple ou un atome d'oxygène ou de soufre ou un groupe de formule 〉NR²², dans laquelle R²² représente un atome d'hydrogène ou un groupe alkyle ayant de 1 à 3 atomes de carbone, s est 1, 2 ou 3, et t est égal à 2.

16. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A2);
d est 0 ou 1;
m est 0, 1 ou 2;
R¹ représente un atome d'hydrogène ou le groupe méthyle, fluorométhyle, formimidoyle ou acétimidoyle;
R⁷ représente un atome d'hydrogène ou d'halogène ou un groupe alkyle ayant de 1 à 3 atomes de carbone, hydroxyle, amino, cyano, carboxyle, carbamoyle ou hydroxyméthyle;
R⁸ représente un atome d'hydrogène ou un groupe alkyle ayant de 1 à 3 atomes de carbone, fluorométhyle, carbamoylméthyle, carboxyméthyle, alcényle ayant 3 ou 4 atomes de carbone, alcynyle ayant 3 ou 4 atomes de carbone, 2-halogénoéthyle, 2-hydroxyéthyle, un groupe 2-alcoxyéthyle dans lequel le fragment alcoxy a de 1 à 3 atomes de carbone, ou le groupe 2-aminoéthyle;
R⁹ représente un atome d'hydrogène ou un groupe alkyle ayant de 1 à 3 atomes de carbone, fluorométhyle, carbamoylméthyle, carboxyméthyle, formimidoyle, acétimidoyle, 2-halogénoéthyle, 2-hydroxyéthyle ou un groupe 2-alcoxyéthyle dans lequel le fragment alcoxy a de 1 à 3 atomes de carbone, ou le groupe 2-aminoéthyle, ou
R⁸ et R⁹ forment ensemble un groupe de formule
-(CH₂)₄-,
-(CH₂)₅-,
-(CH₂)₂O(CH₂)₂-,
-(CH₂)₂S(CH₂)₂-,
-(CH₂)₂NH(CH₂)₂- ou
-(CH₂)₂NCH₃(CH₂)₂-.

17. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A2);
d est 0;
m est 1 ou 2;
R¹ représente un atome d'hydrogène ou le groupe méthyle, fluorométhyle, formimidoyle ou acétimidoyle;
R⁷ représente un atome d'hydrogène;
R⁸ représente un atome d'hydrogène ou un groupe alkyle ayant de 1 à 3 atomes de carbone, carbamoylméthyle, carboxyméthyle, 2-fluoroéthyle ou 2-hydroxyéthyle; et
R⁹ représente un atome d'hydrogène ou un groupe alkyle ayant de 1 à 3 atomes de carbone, formimidoyle, acétimidoyle ou 2-fluoroéthyle.

18. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A3), ℓ est 0, 1 ou 2, et R⁷ représente un atome d'hydrogène ou un groupe alkyle ayant de 1 à 3 atomes de carbone.

19. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A3) et R¹¹ représente un atome d'hydrogène ou un groupe alkyle ayant de 1 à 3 atomes de carbone, tel que le groupe méthyle, éthyle ou propyle.

20. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A3), et R¹² représente un atome d'hydrogène; un groupe alkyle ayant de 1 à 3 atomes de carbone; un groupe alkyle substitué ayant de 1 à 3 atomes de carbone, dans lequel le substituant est choisi parmi des groupes hydroxyle, alcoxy ayant de 1 à 3 atomes de carbone, carbamoyle, carbamoyloxy, carboxyle, cyano, amino et des atomes d'halogène; un groupe alcényle ayant 3 ou 4 atomes de carbone; un groupe alcynyle ayant 3 ou 4 atomes de carbone; ou un groupe de formule -C(=NH)R¹³, dans laquelle R¹³ représente:
un atome d'hydrogène;
un groupe alkyle ayant de 1 à 3 atomes de carbone;
un groupe alkyle substitué ayant de 1 à 3 atomes de carbone, dans lequel le substituant est choisi parmi des groupes alcoxy ayant de 1 à 3 atomes de carbone et des atomes d'halogène;
un groupe cycloalkyle ayant de 3 à 6 atomes de carbone; ou
un groupe alkyle ayant de 1 à 3 atomes de carbone, qui est substitué par un seul groupe cycloalkyle ayant de 3 à 6 atomes de carbone.

21. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A3);
ℓ est 0, 1 ou 2;
R¹ représente un atome d'hydrogène ou le groupe méthyle, fluorométhyle, formimidoyle ou acétimidoyle;
R⁷ représente un atome d'hydrogène;
R¹¹ représente un atome d'hydrogène ou un groupe alkyle ayant de 1 à 3 atomes de carbone; et
R¹² représente un atome d'hydrogène ou un groupe alkyle ayant de 1 à 3 atomes de carbone, fluorométhyle, carbamoylméthyle, carboxyméthyle, alcényle ayant 3 ou 4 atomes de carbone, alcynyle ayant 3 ou 4 atomes de carbone, formimidoyle, acétimidoyle, 2-halogénoéthyle, 2-hydroxyéthyle, un groupe 2-alcoxyéthyle dans lequel le fragment alcoxy a de 1 à 3 atomes de carbone, ou le groupe 2-aminoéthyle.

22. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A3);
ℓ est 1 ou 2;
R¹ représente un atome d'hydrogène ou le groupe méthyle, fluorométhyle, formimidoyle ou acétimidoyle;
R⁷ représente un atome d'hydrogène;
R¹¹ représente un atome d'hydrogène ou le groupe méthyle; et
R¹² représente un atome d'hydrogène ou un groupe alkyle ayant de 1 à 3 atomes de carbone, fluorométhyle, carbamoylméthyle, carboxyméthyle, formimidoyle, acétimidoyle, 2-fluoroéthyle ou 2-hydroxyéthyle.

23. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A4), et R¹⁴ et R¹⁵ représentent chacun un atome d'hydrogène ou un groupe alkyle ayant de 1 à 3 atomes de carbone.

24. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A4), et R¹⁶ représente un atome d'hydrogène ou un groupe alkyle ayant de 1 à 3 atomes de carbone; un groupe alkyle substitué ayant de 1 à 3 atomes de carbone, dans lequel le substituant est choisi parmi des groupes alcoxy ayant de 1 à 3 atomes de carbone et des atomes d'halogène; un groupe cycloalkyle ayant de 3 à 6 atomes de carbone; ou un groupe alkyle ayant de 1 à 3 atomes de carbone, qui est substitué par un seul groupe cycloalkyle ayant de 3 à 6 atomes de carbone.

25. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A4);
i est 1 ou 2; et
R¹, R¹⁴, R¹⁵ et R¹⁶ sont identiques ou différents et représentent chacun un atome d'hydrogène ou un groupe alkyle ayant de 1 à 3 atomes de carbone.

26. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A5);
i est 1; et
R¹, R¹⁴, R¹⁵ et R¹⁶ sont identiques ou différents et représentent chacun un atome d'hydrogène ou le groupe méthyle.

27. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A5), et R¹⁷ et R¹⁸ représentent chacun un atome d'hydrogène; un groupe alkyle ayant de 1 à 3 atomes de carbone ou un groupe alkyle substitué ayant de 1 à 3 atomes de carbone, dans lequel le substituant est choisi parmi des groupes hydroxyle, alcoxy ayant de 1 à 3 atomes de carbone et des atomes d'halogène.

28. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A5), et R¹⁷ et R¹⁸ représentent ensemble un groupe de formule -(CH₂)_{q}-Y-(CH₂)ᵣ-, dans laquelle Y représente une liaison carbone-carbone simple, un atome d'oxygène ou un groupe de formule 〉NR²³, dans laquelle R²³ représente un atome d'hydrogène ou un groupe alkyle ayant de 1 à 3 atomes de carbone, et q et r représentent chacun 2 ou 3.

29. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A5);
p est égal à 2;
R¹ représente un atome d'hydrogène ou le groupe méthyle, fluorométhyle, formimidoyle ou acétimidoyle;
R¹⁷ et R¹⁸ sont identiques ou différents et représentent chacun un atome d'hydrogène ou un groupe alkyle ayant de 1 à 3 atomes de carbone, 2-halogénoéthyle ou 2-hydroxyéthyle; ou
R¹⁷ et R¹⁸ représentent ensemble un groupe de formule
-(CH₂)₄-,
-(CH₂)₅-,
-(CH₂)₂O(CH₂)₂-,
-(CH₂)₂S(CH₂)₂-,
-(CH₂)₂NH(CH₂)₂- ou
-(CH₂)₂NCH₃(CH₂)₂-.

30. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A5);
p est égal à 2;
R¹ représente un atome d'hydrogène ou le groupe méthyle, fluorométhyle, formimidoyle ou acétimidoyle; et
R¹⁷ et R¹⁸ sont identiques ou différents et représentent chacun un atome d'hydrogène ou le groupe méthyle.

31. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A6), et R¹⁹ représente un atome d'hydrogène ou un groupe alkyle ayant de 1 à 3 atomes de carbone.

32. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A6);
j et k représentent chacun 2;
R¹ représente un atome d'hydrogène ou le groupe méthyle, fluorométhyle, formimidoyle ou acétimidoyle; et
R¹⁹ représente un atome d'hydrogène ou un groupe alkyle ayant de 1 à 3 atomes de carbone.

33. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A6);
j et k représentent chacun 2; et
R¹ et R¹⁹ sont identiques ou différents et représentent chacun un atome d'hydrogène ou le groupe méthyle.

34. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A7), et Z représente le groupe 1-imidazolyle, 1,2,4-triazole-1-yle ou 1,2,3-triazole-1-yle.

35. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A7);
g est égal à 0, 1 ou 2;
R¹ représente un atome d'hydrogène ou le groupe méthyle; et
Z représente le groupe 1-imidazolyle, 1,2,4-triazole-1-yle ou 1,2,3-triazole-1-yle.

36. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A7);
g est égal à 1 ou 2;
R¹ représente un atome d'hydrogène ou le groupe méthyle; et
Z représente le groupe 1-imidazolyle, 1,2,4-triazole-1-yle ou 1,2,3-triazole-1-yle.

37. Procédé selon la revendication 1 ou 2, dans lequel on choisit les réactifs et conditions réactionnelles de manière à préparer un composé de formule (I) ou un sel ou ester de celui-ci, dans lequel A représente un groupe de formule (A8), et R²⁰ et R²¹ représentent chacun un atome d'hydrogène ou un groupe alkyle ayant de 1 à 3 atomes de carbone.

38. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A8);
e et f sont chacun égal à 1;
R¹ représente un atome d'hydrogène ou le groupe méthyle, fluorométhyle, formimidoyle ou acétimidoyle; et
R²⁰ et R²¹ représentent chacun un atome d'hydrogène ou un groupe alkyle ayant de 1 à 3 atomes de carbone.

39. Procédé selon la revendication 1 ou 2, dans lequel on choisit les réactifs et conditions réactionnelles de manière à préparer un composé de formule (I) ou un sel ou ester de celui-ci, dans lequel:
A représente un groupe de formule (A8);
e et f sont chacun égal à 1;
R¹ représente un atome d'hydrogène ou le groupe méthyle;
R²⁰ représente un atome d'hydrogène; et
R²¹ représente un atome d'hydrogène ou le groupe méthyle.

40. Procédé selon l'une quelconque des revendications précédentes, dans lequel on choisit les réactifs et conditions réactionnelles de manière à préparer un composé de formule (I) ou un sel ou ester de celui-ci, dans lequel les atomes de carbone sont dans les mêmes configurations que ceux de la thiénamycine.

41. Procédé selon la revendication 1 ou 2, dans lequel on choisit les réactifs et conditions réactionnelles de manière à préparer les composés suivants:
acide 2-[2-(1-homopipérazinylcarbonyl)-1-méthylpyrrolidine-4-ylthio]-6-(1-hydroxyéthyl)-1-méthyl-1-carbapén-2-ème-3-carboxylique,
acide 2-[2-(4-carboxyméthylhomopipérazine-1-ylcarbonyl)pyrrolidine-4-ylthio]-6-(1-hydroxyéthyl)-1-méthyl-1-carbapén-2-ème-3-carboxylique,
acide 2-{2-[4-(2-hydroxyéthyl)homopipérazine-1-ylcarbonyl]pyrrolidine-4-ylthio}-6-(1-hydroxyéthyl)-1-méthyl-1-carbapén-2-ème-3-carboxylique,
acide 2-[2-(4-acétimidoylhomopipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylhomopipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylhomopipérazine-1-ylcarbonyl)-1-méthylpyrrolidine-4-ylthio]-6-(1-hydroxyéthyl)-1-méthyl-1-carbapén-2-ème-3-carboxylique,
acide 2-[1-méthyl-2-(pipérazine-1-ylcarbonyl)pyrrolidine-4-ylthio]-6-(1-hydroxyéthyl)-1-méthyl-1-carbapén-2-ème-3-carboxylique,
acide 2-{2-[4-(2-hydroxyéthyl)pipérazine-1-ylcarbonyl]pyrrolidine-4-ylthio}-6-(1-hydroxyéthyl)-1-méthyl-1-carbapén-2-ème-3-carboxylique,
acide 2-[2-(3-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylpipérazine-1-ylcarbonyl)pyrrolidine-4-ylthio]-6-(1-hydroxyéthyl)-1-méthyl-1-carbapén-2-ème-3-carboxylique,
acide 2-[2-(4-acétimidoylpipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoylpipérazine-1-ylcarbonyl)-1-méthylpyrrolidine-4-ylthio]-6-(1-hydroxyéthyl)-1-méthyl-1-carbapén-2-ème-3-carboxylique,
acide 2-[2-(4-acétimidoylpipérazine-1-ylcarbonyl)-1-méthylpyrrolidine-4-ylthio]-6-(1-hydroxyéthyl)-1-méthyl-1-carbapén-2-ème-3-carboxylique,
acide 2-[2-(4-formimidoyl-3-méthylpipérazine-1-ylcarbonyl)pyrrolidine-4-ylthio]-6-(1-hydroxyéthyl)-1- méthyl-1-carbapén-2-ème-3-carboxylique,
acide 2-[2-(4-acétimidoyl-3-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(2-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-formimidoyl-2-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(4-acétimidoyl-2-méthylpipérazine-1-ylcarbonyl)pyrrolidine-4-ylthio]-6-(1-hydroxyéthyl)-1-méthyl-1-carbapén-2-ème-3-carboxylique,
acide 2-[2-(3-hydroxyméthylpipérazine-1-ylcarbonyl)pyrrolidine-4-ylthio]-6-(1-hydroxyéthyl)-1-méthyl-1-carbapén-2-ème-3-carboxylique,
acide 2-[1-formimidoyl-2-(4-formimidoylpipérazine-1-ylcarbonyl)pyrrolidine-4-ylthio]-6-(1-hydroxyéthyl)-1-méthyl-1-carbapén-2-ème-3-carboxylique,
acide 2-[2-(3-acétimidoylaminopyrrolidine-1-ylcarbonyl)pyrrolidine-4-ylthio]-6-(1-hydroxyéthyl)-1-méthyl-1-carbapén-2-ème-3-carboxylique,
acide 2-[2-(3-formimidoylaminopyrrolidine-1-ylcarbonyl)pyrrolidine-4-ylthio]-6-(1-hydroxyéthyl)-1-méthyl-1-carbapén-2-ème-3-carboxylique,
acide 2-[2-(3-aminopyrrolidine-1-ylcarbonyl)pyrrolidine-4-ylthio]-6-(1-hydroxyéthyl)-1-méthyl-1-carbapén-2-ème-3-carboxylique,
acide 2-[2-(4-acétimidoylaminopipéridine-1-ylcarbonyl)pyrrolidine-4-ylthio]-6-(1-hydroxyéthyl)-1-méthyl-1-carbapén-2-ème-3-carboxylique,
acide 2-[2-(3-aminopyrrolidine-1-ylcarbonyl)-1-méthylpyrrolidine-4-ylthio]-6-(1-hydroxyéthyl)-1-méthyl-1-carbapén-2-ème-3-carboxylique,
acide 2-[2-(3-acétimidoylaminopyrrolidine-1-ylcarbonyl)-1-méthylpyrrolidine-4-ylthio]-6-(1-hydroxyéthyl)-1-méthyl-1-carbapén-2-ème-3-carboxylique,
acide 2-[2-(3-formimidoylaminopyrrolidine-1-ylcarbonyl)-1-méthylpyrrolidine-4-ylthio]-6-(1-hydroxyéthyl)-1-méthyl-1-carbapén-2-ème-3-carboxylique,
acide 2-[2-(4-acétimidoylaminopipéridine-1-ylcarbonyl)-1-méthylpyrrolidine-4-ylthio]-6-(1-hydroxyéthyl)-1-méthyl-1-carbapén-2-ème-3-carboxylique,
acide 2-[2-(1-formimidoylpyrrolidine-3-ylcarbamoyl)pyrrolidine-4-ylthio]-6-(1-hydroxyéthyl)-1-méthyl-1-carbapén-2-ème-3-carboxylique,
acide 2-[2-(3-diméthylamino-1,2,5,6-tétrahydropyrazine-1-ylcarbonyl)pyrrolidine-4-ylthio]-6-(1-hydroxyéthyl)-1-méthyl-1-carbapén-2-ème-3-carboxylique,
et leurs sels et esters pharmaceutiquement acceptables.

42. Procédé pour la préparation d'une composition pharmaceutique, comprenant le mélange d'un véhicule, diluant ou adjuvant pharmaceutiquement acceptable, avec un antibiotique, dans lequel l'antibiotique est choisi parmi les composés de formule (I) et leurs sels et esters pharmaceutiquement acceptables, tels que définis dans l'une quelconque des revendications 1 à 41.

43. Utilisation d'un composé de formule (I) ou d'un sel ou ester pharmaceutiquement acceptable de celui-ci, tel que défini dans l'une quelconque des revendications 1 à 41, pour la fabrication d'un médicament destiné au traitement ou à la prophylaxie d'infections bactériennes.
